(19) Europäisches Patentamt
European Patent Office
Office européen des brevets

(11) EP 1 754 795 A1

(12) EUROPEAN PATENT APPLICATION

(43) Date of publication:
**21.02.2007 Bulletin 2007/08**

(51) Int Cl.:
***C12Q 1/68*** *(2006.01)*

(21) Application number: **06254064.6**

(22) Date of filing: **02.08.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **02.08.2005 US 704740 P**

(71) Applicant: **Veridex, LLC**
**Warren, NJ 07059 (US)**

(72) Inventors:
- **Wang, Yixin**
  **San Diego, CA 92130 (US)**
- **Zhang, Yi**
  **San Diego, CA 92127 (US)**
- **Atkins, David**
  **Toronto, ONT M4W 1Y3 (CA)**
- **Sieuwerts, Anieta M.,**
  **c/o Dept. of Med. Oncology**
  **Rotterdam (NL)**
- **Smid, Marcel,**
  **c/o Dept. of Medical Oncology**
  **Rotterdam (NL)**
- **Klijin, Jan G. M.,**
  **c/o Dept. of Medical Oncology**
  **Rotterdam (NL)**
- **Martens, John W. M.,**
  **c/o Dept. of Medical Oncology**
  **Rotterdam (NL)**
- **Foekens, John A.,**
  **c/o Dept. of Medical Oncology**
  **Rotterdam (NL)**

(74) Representative: **Mercer, Christopher Paul et al**
**Carpmaels & Ransford,**
**43-45 Bloomsbury Square**
**London WC1A 2RA (GB)**

# (54) Predicting bone relapse of breast cancer

(57) A method of providing predicting relapse of breast cancer in bone is conducted by analyzing the expression of a group of genes. Gene expression profiles in a variety of medium such as microarrays are included as are kits that contain them.

EP 1 754 795 A1

## Description

## BACKGROUND

This invention relates to breast cancer patient prognosis with respect to relapse to bone and is based on the gene expression profiles of patient biological samples.

The most abundant site of a distant relapse in breast cancer is the bone. Many factors have been implicated in facilitating bone relapse including blood flow in red bone marrow, adhesive molecules in the tumor cells, and immobilized growth factors in the bone matrix such as transforming growth factors-β, bone morphogenetic proteins, platelet derived growth factor, insulin-like growth factors, and fibroblast growth factors. However, gene-based relationships involving the promotion of interactions with bone and cancer cells derived from breast cancers have been largely unknown.

A breast cancer prognostic was recently described for predicting distant recurrence in lymph node negative patients. Wang et. al, PCT/CTS2005/005711 filed Feb. 18, 2005. Gene expression patterns have also been used to classify breast tumors into different clinically relevant subtypes. Perou et al. (2000); Sørlie et al. (2001); Sørlie et al. (2003); Gruvberger et al. (2001); van't Veer et al. (2002); van de Vijver et al. (2002); Ahr et al. (2002); Huang et al. (2003); Sotiriou et al. (2003); Woelfle et al. (2003); Ma et al. (2003); Ramaswamy et al. (2003); Chang et al. (2003); Sotiriou et al. (2003); and Hedenfalk et al. (2001). Currently, however, there are few diagnostic tools available to identify patients specifically at risk for relapse to bone. There is a need to specifically identify a patient's risk of disease relapse to bone to ensure she receives appropriate therapy.

## SUMMARY OF THE INVENTION

The invention encompasses a method of assessing breast cancer status by obtaining a biological sample from a breast cancer patient and measuring the expression levels of genes via Markers where the gene expression levels above or below pre-determined cut-off levels are indicative of breast cancer status with respect to bone metastasis.

The invention encompasses a method of staging breast cancer by obtaining a biological sample from a breast cancer patient and measuring the expression levels in the sample of genes via Markers where the gene expression levels above or below pre-determined cut-off levels are indicative of the breast cancer stage.

The invention encompasses a method of monitoring breast cancer patient treatment by obtaining a biological sample from a breast cancer patient and measuring the expression levels in the sample of genes via Markers where the gene expression levels above or below pre-determined cut-off levels (as set forth in an algorithm) are sufficiently indicative of risk of metastasis to bone to enable a physician to determine the degree and type of therapy recommended to prevent such metastasis.

The invention encompasses a method of treating a breast cancer patient by obtaining a biological sample from a breast cancer patient; and measuring the expression levels in the sample of genes via Markers where the gene expression levels above or below pre-determined cut-off levels indicate a high risk of bone metastasis and; treating the patient with adjuvant therapy if they are a high risk patient.

The invention encompasses a method of generating a bone relapse probability score to enable prognosis of breast cancer patients by obtaining gene expression data from a statistically significant number of patient biological samples, applying univariate Cox's regression analysis to the data to obtain selected genes; applying weighted expression levels to the selected genes with standard Cox's coefficients to obtain a prediction model that can be applied as a bone relapse probability score.

The invention encompasses a method of generating a breast cancer prognostic patient report by obtaining a biological sample from the patient; measuring gene expression of the sample; applying a bone relapse probability score; and using the results obtained to generate the report and patient reports generated thereby.

The invention encompasses a composition containing Markers.

The invention encompasses a kit for conducting an assay to determine breast cancer prognosis using a biological sample obtained from the patient. The kit contains materials for detecting Markers. Preferably, the kit includes instructions for its use.

The invention encompasses articles for assessing breast cancer status containing Markers.

The invention encompasses a diagnostic/prognostic portfolio containing Markers where the combination is sufficient to characterize breast cancer status or risk of relapse in bone in a biological sample.

The inventive methods can be advantageously used in conjunction with other breast prognostics. This can be done reflexively so that first the prognosis of any relapse is determined followed by the application of the PAM approach presented in this application. Alternatively, these methods can be conducted simultaneously or near-simultaneously to provide the physician and/or patient with information concerning the likelihood of relapse anywhere and, more specifically, relapse to bone.

## DETAILED DESCRIPTION

The invention encompassing a method of assessing breast cancer status determines whether a patient is at high risk of a recurrence of the disease in bone. References to prognosis and prediction throughout this application are drawn to predictions relating to the relapse of breast cancer with its appearance in bone. These methods involve obtaining a biological sample from a breast cancer patient and measuring the expression levels in the sample of certain genes where the gene expression levels above or below pre-determined cut-off levels are indicative of breast cancer status with respect to its relapse in bone.

The inventive methods, compositions, articles, and kits described and claimed in this specification include one or more Markers. "Marker" is used throughout this specification to refer to:

a) genes and gene expression products such as RNA, mRNA and corresponding cDNA, peptides, proteins, fragments and complements of each of the foregoing, and

b) compositions such as probes, antibodies, ligands, haptens, and labels that, through physical or chemical interaction with a) indicate the expression of the gene or presence of the gene expression product and wherein the gene, gene expression product or compositions correspond with:

i) SEQ ID NO 112,

ii) a combination of SEQ ID NO 112 and a member of the group consisting of SEQ ID NO 113, SEQ ID NO 114, SEQ ID NO 115, SEQ ID NO 116,

iii) a combination of SEQ ID NO 112 and all of SEQ ID NO 113, SEQ ID NO 114, SEQ ID NO 115, and SEQ ID NO 116,

iv) one or more of SEQ ID NO 112- SEQ ID NO 116 and one or more of SEQ ID NO 117-198, or

v) all of SEQ ID NO 112- SEQ ID NO 198.

A gene corresponds to the sequence designated by a SEQ ID NO when it contains that sequence. A gene segment or fragment corresponds to the sequence of such gene when it contains a portion of the referenced sequence or its complement sufficient to distinguish it as being the sequence of the gene. A gene expression product corresponds to such sequence when its RNA, mRNA, or cDNA hybridizes to the composition having such sequence (e.g. a probe) or, in the case of a peptide or protein, it is encoded by such mRNA. A segment or fragment of a gene expression product corresponds to the sequence of such gene or gene expression product when it contains a portion of the referenced gene expression product or its complement sufficient to distinguish it as being the sequence of the gene or gene expression product.

Markers corresponding to ii and iii are preferred. Markers corresponding to iv and v are most preferred.

While the mere presence or absence of particular nucleic acid sequences (e.g., genes containing SNPs) in a tissue sample has only rarely been found to have diagnostic or prognostic value, information about the expression of various proteins, peptides or mRNA is increasingly viewed as important. The mere presence of nucleic acid sequences having the potential to express proteins, peptides, or mRNA (such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. However, relative indications of the degree to which genes are active or inactive can be found in gene expression profiles. Here it is reported that assaying gene expression is useful in identifying and reporting whether a breast cancer patient is likely to experience a relapse to bone. This is important for a number of reasons including insuring that the patient can receive the most beneficial treatment.

Sample preparation is an important aspect of practicing the methods and using the kits and articles of the invention. Sample preparation requires the collection of patient samples. Patient samples used in the inventive method are those that are suspected of containing diseased cells such as epithelial cells taken from the primary tumor in a breast sample. The sample can be any sample that is suspected of having cancer cells present including, without limitation, primary tumor tissue, aspirates of tissue or fluid, ductal fluids, prepared by any method known in the art including bulk tissue preparation and laser capture microdissection. Bulk tissue preparations can be obtained from a biopsy or a surgical specimen. Fluids can be readily obtained with fine needle aspirates, lavages, and other methods of extraction known in the medical arts. Most preferably, the sample is obtained from a primary tumor. Samples taken from surgical margins are also preferred. Laser Capture Microdissection (LCM) technology is one way to select the cells to be studied, minimizing variability caused by cell type heterogeneity. Samples can also comprise circulating epithelial cells extracted from peripheral blood. These can be obtained according to a number of methods but the most preferred method is the magnetic separation technique described in U.S. Patent 6,136,182, incorporated in its entirety in this specification. Once the sample containing the cells of interest has been obtained, genetic material is extracted and used in the methods or with the kits or articles of the inventions. Preferably, RNA is extracted and amplified and a gene expression profile is obtained, preferably via micro-array, for genes in the appropriate portfolios.

Using gene expression microarray data (Affymetrix U133A Chips) of 107 primary breast tumors that were all lymph-node negative at the time of diagnosis and that all had relapsed, panels of genes were found significantly differentially expressed between patients who relapsed to bone versus those who relapsed elsewhere in the body. This panel was arrived at using the SAM approach that is described in this application. The most differentially expressed gene in that panel, TFF1, was confirmed by quantitative RT-PCR in an independent cohort (n=122, p=0.0015). Additionally, a classifier was developed that accurately predicts bone relapse in general. This classifier/panel is referred to as the PAM panel in this application. This classifier can be used as tool to recommend adjuvant therapy particularly suited for treatment of bone metastasis including without limitation, bisphosphonate treatment. These treatments can be recommended in addition to endocrine, chemotherapy, radiation, or other treatments.

The inventive methods of staging breast cancer involve obtaining a biological sample from a breast cancer patient and measuring the expression levels in the sample of genes via Markers where the gene expression levels above or below pre-determined cut-off levels are used as input to indicate breast cancer stage. The information is utilized in any classification known in the art including the TNM system American Joint Committee on Cancer www.cancerstaging.org and comparison to stages corresponding to patients with similar gene expression profiles.

The methods of determining breast cancer patient treatment involve obtaining a biological sample from a breast cancer patient and measuring the expression levels in the sample of genes via Markers where the gene expression levels above or below pre-determined cut-off levels are sufficiently indicative of risk of relapse to bone to enable a physician to determine the degree and type of therapy recommended to prevent such relapse.

The method of treating a breast cancer patient involve obtaining a biological sample from a breast cancer patient; and measuring the expression levels in the sample of genes via Markers where the gene expression levels above or below pre-determined cut-off levels indicate a high risk of relapse to bone and treating the patient with adjuvant therapy if they are a high risk patient.

The above methods can further include measuring the expression level of at least one gene constitutively expressed in the sample.

The above methods preferably have a specificity of at least 40% and a sensitivity of at least at least 80%.

The above methods can be used where the expression pattern of the genes is compared to an expression pattern indicative of a breast cancer patient who has relapsed in bone. The comparison can be by any method known in the art including comparison of expression patterns is conducted with pattern recognition methods. Pattern recognition methods can be any known in the art including PAM analysis and, alternatively, Cox's proportional hazards analysis.

Preferably, levels of up- and down-regulation of the gene markers used in the invention are distinguished based on fold changes of the intensity measurements of hybridized microarray probes. In any event, the inventive methods, kits, portfolios, and measurements and analyses undertaken in them employ pre-determined cut-off levels indicative of at least 1.7-fold over- or under-expression in the sample relative to samples from patients without bone relapse. Preferably, the pre-determined cut-off levels have at least a statistically significant p-value for over-expression in the sample from patients having relapse to bone relative to non-bone relapse patients. More preferably, the p-value is less than 0.05. A 2.0 fold difference is more preferred for making such distinctions. That is, before a gene is said to be differentially expressed in samples from relapsing versus non-relapsing patients, the samples from the relapsing patients are found to yield at least 2 times more, or 2 times less intensity than the those of the non-relapsing patient. The greater the fold difference, the more preferred is use of the gene as a diagnostic or prognostic tool provided that the p-value of the gene is acceptable from a clinical point of view (i.e., closely associated with relapse to bone). Genes selected for the gene expression profiles of the instant invention have expression levels that result in the generation of a signal that is distinguishable from those of the non-relapsing or non-modulated genes by an amount that exceeds background using clinical laboratory instrumentation.

The above methods can be used where gene expression is measured on a microarray or gene chip. Gene chips and microarrays suitable for use herein are also included in the invention. The microarray can be a cDNA array or an oligonucleotide array and can further contain one or more internal control reagents.

The above methods can likewise be used where gene expression is determined by nucleic acid amplification and detection methods. Preferably, such methods include the polymerase chain reaction (PCR) of RNA extracted from the sample. The PCR can be reverse transcription polymerase chain reaction (RT-PCR). The RT-PCR can further contain one or more internal control reagents.

The above methods can be used where gene expression is detected by measuring or detecting a protein encoded by the gene. Any method known in the art can be used including detection by an antibody specific to the protein and measuring a characteristic of the gene. Suitable characteristics include, without limitation, DNA amplification, methylation, mutation and allelic variation.

A method of the invention encompasses generating a bone relapse probability score to enable prediction of relapse to bone. This method can be conducted by obtaining gene expression data from a statistically significant number of patient biological samples and applying the PAM analysis as described below. In another embodiment of the invention, the bone relapse probability score can be obtained by application of the Cox regression formula using standardized Cox regression coefficients.

The inventive method of generating a breast cancer prognostic patient report (for relapse to bone) is conducted by obtaining a biological sample from the patient, measuring gene expression of the sample; applying a bone relapse probability score to the results and using the results obtained to generate the report. The report may contain an assessment of patient outcome and/or probability of risk relative to the patient population.

The inventive compositions include at least one probe set of Markers. The composition can further contain reagents for conducting a microarray, amplification or probe-based analysis, and a medium through which the nucleic acid sequences, their complements, or portions thereof are assayed.

The inventive kit for conducting an assay to determine breast cancer prognosis in a biological sample include materials for detecting Markers. The kit can further contain reagents for conducting a microarray, amplification or probe-based analysis, and a medium through which the nucleic acid sequences, their complements, or portions thereof are assayed.

The inventive articles for assessing breast cancer status include materials for detecting Markers. The articles can further contain reagents for conducting a microarray, amplification or probe-based analysis, and a medium through which said nucleic acid sequences, their complements, or portions thereof are assayed.

The microarrays useful in the inventive methods, articles, and kits can contain Markers where the combination is sufficient to characterize breast cancer status or risk of relapse in bone.

The preferred kits, articles, and microarrays include substrates to which probes are fixed or bind and to which target Markers bind or associate so they can be detected. It is most preferred that these substrates are suitable only for conducting the assay or described in this specification or that are suitable for conducting a discrete number of related assays (i.e., contain a small number of panels).

The invention encompasses a diagnostic/prognostic portfolio of Markers where the combination is sufficient to characterize breast cancer status or risk of relapse to bone in a biological sample.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complementary DNA (cDNA) or complementary RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002; 6,218,122; 6,218,114; and 6,004,755.

Analysis of expression levels is conducted by comparing signal intensities and subjecting these measurements to statistical algorithms. Generating a ratio matrix of the expression intensities of genes in a test sample versus those in a control sample is one such method. For instance, the gene expression intensities from a test tissue can be compared with the expression intensities generated from tissue of the same type from a patient with the condition of interest (e.g., tumor tissue from a patient who relapsed to bone vs. one who did not). A ratio of these expression intensities indicates the fold-change in gene expression between the test and control samples.

Gene expression profiles can also be displayed in a number of ways. The most common method is to arrange raw fluorescence intensities or ratio matrix into a graphical dendogram where columns indicate test samples and rows indicate genes. The data are arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including GeneSpring from Agilent Technologies and Partek Discover™ and Partek Infer™ software from Partek®.

Modulated genes used in the methods of the invention are described in the Examples. Differentially expressed genes are either up- or down-regulated in patients with a relapse to bone of breast cancer relative to those without such a relapse. Up regulation and down regulation are relative terms meaning that a detectable difference (beyond the contribution of noise in the system used to measure it) is found in the amount of expression of the genes relative to some baseline. In this case, the baseline is the measured gene expression of a non-relapsing patient. The genes of interest in the diseased cells (from the relapsing patients) are then either up- or down-regulated relative to the baseline level (from the non-bone relapsing patients) using the same measurement method. A patient with a gene expression pattern consistent with that of the condition of interest (likelihood of relapse to bone) is assessed as having such condition and treated accordingly. In therapy monitoring, clinical judgments are made regarding the effect of a given course of therapy by comparing the expression of genes over time to determine whether the gene expression profiles have changed or are changing to patterns more consistent with tissue of non-relapsing patients.

Statistical values can be used to confidently distinguish modulated from non-modulated genes and noise and establish expression profiles. One such statistical test that finds the genes most significantly different between diverse groups of samples is based on a Student's T-test. P-values are obtained relating to the inclusion of particular genes to a class of genes. The lower the p-value, the more compelling the evidence that the gene is showing a difference between the different groups. Since microarrays measure more than one gene at a time, tens of thousands of statistical tests may be performed at one time so one is unlikely to see small p-values just by chance. Adjustments for using a Sidak correction as well as a randomization/permutation experiment can be made. A p-value less than 0.05 by the T-test is evidence that the gene is significantly different. More compelling evidence is a p-value less then 0.05 after the Sidak correction is factored in. For a large number of samples in each group, a p-value less than 0.05 after the randomization/permutation test is the most compelling evidence of a significant difference.

Another parameter that can be used to select genes that generate a signal that is greater than that of the non-modulated gene or noise is the use of a measurement of absolute signal difference. Preferably, the signal generated by the modulated gene expression is at least 20% different than those of the non-modulated gene or the genes of the non-relapsing patient (on an absolute basis). It is even more preferred that such genes produce expression patterns that are at least 30% different than those of non-modulated genes.

The genes that are grouped so that information obtained about the set of genes in the group provides a sound basis for making a clinically relevant judgment such as a diagnosis, prognosis, or treatment choice make up the portfolios of the invention. In this case, the judgments supported by the portfolios involve breast cancer and its chance of relapse to bone.

Preferably, portfolios are established such that the combination of genes in the portfolio exhibit improved sensitivity and specificity relative to individual genes or randomly selected combinations of genes. In the context of the instant invention, the sensitivity of the portfolio can be reflected in the fold differences exhibited by a gene's expression in the state of a patient that relapses to bone relative to those without such relapse. Specificity can be reflected in statistical measurements of the correlation of the signaling of gene expression with the condition of interest. For example, standard deviation can be a used as such a measurement. In considering a group of genes for inclusion in a portfolio, a small standard deviation in expression measurements correlates with greater specificity. Other measurements of variation such as correlation coefficients can also be used.

The portfolios of genes of this invention were determined through SAM analysis. Gene expression patterns are analyzed using PAM analysis. SAM (Significance Analysis of Microarrays) is a statistical approach to identify genes whose expression patterns are significantly associated with specific characteristics of sample sets. This method is embodied in software developed at Stanford University and it is publicly available. SAM identifies genes with statistically significant changes in expression by assimilating a set of gene specific T-tests. The method is described in US Patent Application 20020019704 to Tusher et. al., filed March 19, 2001 and incorporated in its entirety in this specification. It is also described in Significance Analysis of Microarrays Applied to the Ionizing Radiation Response; Tusher, Tibshirani, and Chu, 5116-5121_ PNAS _ April 24, 2001 _ vol. 98 _ no. 9.

In a SAM analysis, each gene assayed is assigned a score on the basis of its change in gene expression relative to the standard deviation of repeated measurements for that gene. Genes with scores greater than a threshold are deemed potentially significant. The percentage of such genes identified by chance is the false discovery rate (FDR). To estimate the FDR, nonsense genes are identified by analyzing permutations of the measurements. The threshold can be adjusted to identify smaller or larger sets of genes, and FDRs are calculated for each set.

A value referred to as the "relative difference" or d(i) in gene expression is based on the ratio of change in gene expression to standard deviation in the data for that gene. The "gene-specific scatter" s(i)is the standard deviation of repeated expression measurements. The coefficient of variation of *d(i)* is computed as a function of *s(i).* To find significant changes in gene expression, genes are ranked by magnitude of their *d*(*i*)value s, so that *d*(1)is the largest relative difference, *d(2)* is the second largest relative difference, and *d(i)* is the ith largest relative difference. For each of the permutations, relative differences *dp*(*i*) are also calculated, and the genes are again ranked such that *dp*(*i*) is the ith largest relative difference for permutation *p*. The expected relative difference, *dE*(*i*), is defined as the average over the balanced permutations.

To identify potentially significant changes in expression, a scatter plot of the observed relative difference *d*(*i*) versus the expected relative difference *dE*(*i*) can be used. For the vast majority of genes, *d*(*i*) is approximately equal to *dE*(*i*), but some genes are represented by points displaced from the *d*(*i*) = *dE*(*i*) line by a distance greater than a threshold. To determine the number of falsely significant genes generated by SAM, horizontal cutoffs are defined as the smallest *d*(*i*) among the genes called significantly induced and the least negative *d*(*i*) among the genes called significantly repressed. The number of falsely significant genes corresponding to each permutation is computed by counting the number of genes that exceed the horizontal cutoffs for induced and repressed genes. The estimated number of falsely significant genes is the average of the number of genes called significant from all permutations. This method for setting thresholds provides asymmetric cutoffs for induced and repressed genes. An alternative is the standard *t* test, which imposes a symmetric horizontal cutoff, with *d*(*i*) greater than c for induced genes and *d(i)* less than c for repressed genes. However, the asymmetric cutoff is preferred because it allows for the possibility that d(i)for induced and repressed genes may behave differently in some biological experiments.

PAM (Predictive Analysis of Microarrays) analysis is a modified version of the nearest-centroid method. The method was developed at Stanford University Labs and is typically carried out using the Statistical package R. It provides a list of significant genes whose expression characterizes each diagnostic class and estimates prediction error via cross-validation. The method is a nearest shrunken centroid methodology. It is described in Diagnosis of Multiple Cancer Types by Shrunken Centroids of Gene Expression; Narashiman and Chu, PNAS 2002 99:6567-6572 (May 14, 2002).

In this method, a standardized centroid is computed for each class. This is the average gene expression for each gene in each class divided by the within-class standard deviation for that gene. Nearest centroid classification takes the gene expression profile of a new sample, and compares it to each of these class centroids. The class whose centroid that it is closest to, in squared distance, is the predicted class for that new sample. Nearest shrunken centroid classification "shrinks" each of the class centroids toward the overall centroid for all classes by an amount called the threshold. This shrinkage consists of moving the centroid towards zero by threshold, setting it equal to zero if it hits zero. For example if threshold was 2.0, a centroid of 3.2 would be shrunk to 1.2, a centroid of -3.4 would be shrunk to -1.4, and a centroid of 1.2 would be shrunk to zero. After shrinking the centroids, the new sample is classified by the usual nearest centroid rule, but using the shrunken class centroids. This shrinkage can make the classifier more accurate by reducing the effect of noisy genes and provides an automatic gene selection. In particular, if a gene is shrunk to zero for all classes, then it is eliminated from the prediction rule. Alternatively, it may be set to zero for all classes except one, and it can be learned that the high or low expression for that gene characterizes that class. The user decides on the value to use for threshold. Typically one examines a number of different choices. To guide in this choice, PAM does K-fold cross-validation for a range of threshold values. The samples are divided up at random into K roughly equally sized parts. For each part in turn, the classifier is built on the other K-1 parts then tested on the remaining part. This is done for a range of threshold values, and the cross-validated misclassification error rate is reported for each threshold value. Typically, the user would choose the threshold value giving the minimum cross-validated misclassification error rate.

Alternatively, gene expression portfolios can be established through the use of optimization algorithms such as the mean variance algorithm widely used in establishing stock portfolios. This method is described in detail in US patent publication number 20030194734. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application," referred to as "Wagner Software" throughout this specification, is preferred. This software uses functions from the "Wagner Associates Mean-Variance Optimization Library" to determine an efficient frontier and optimal portfolios in the Markowitz sense is preferred. Use of this type of software requires that microarray data be transformed so that it can be treated as an input in the way stock return and risk measurements are used when the software is used for its intended financial analysis purposes.

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with breast cancer. Output from the method would be an optimized set of genes that could include some genes that are expressed in peripheral blood as well as in diseased tissue. If samples used in the testing method are obtained from peripheral blood and certain genes differentially expressed in instances of breast cancer are differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply a rule that only a prescribed percentage of the portfolio can be represented by a particular gene or group of genes. Commercially available software such as the Wagner Software readily accommodates these types of heuristics.

This can be useful, for example, when factors other than accuracy and precision (e.g., anticipated licensing fees) have an impact on the desirability of including one or more genes.

One method of the invention involves comparing gene expression profiles for various genes (or portfolios) to ascribe prognoses. The gene expression profiles of each of the genes comprising the portfolio are fixed in a medium such as a computer readable medium. This can take a number of forms. For example, a table can be established into which the range of signals (e.g., intensity measurements) indicative of the condition of interest (e.g., high probability of relapse to bone) is input. Actual patient data can then be compared to the values in the table to determine the likelihood of relapse to bone from the patient samples. In a more sophisticated embodiment, patterns of the expression signals (e.g., fluorescent intensity) are recorded digitally or graphically.

The gene expression patterns from the gene portfolios used in conjunction with patient samples are then compared to the expression patterns. Pattern comparison software can then be used to determine whether the patient samples have a pattern indicative of relapse to bone. Of course, these comparisons can also be used to determine whether the patient is not likely to experience relapse to bone. The expression profiles of the samples are then compared to the portfolio of a control cell. If the sample expression patterns are consistent with the expression pattern for relapse to bone of a breast cancer then (in the absence of countervailing medical considerations) the patient is treated as one would treat such a relapsing patient. If the sample expression patterns are consistent with the expression pattern from the normal/control cell then the patient is diagnosed negative for breast cancer.

The gene expression pattern of a patient can be used to determine prognosis of breast cancer (with respect to its relapse in bone) through the use of a Cox's hazard analysis program. Such analyses are preferably conducted using S-Plus software (commercially available from Insightful Corporation). Using such methods, a gene expression profile is compared to that of a profile that confidently represents bone relapse (i.e., expression levels for the combination of genes in the profile is indicative of bone relapse). The Cox's hazard model with the established threshold is used to compare the similarity of the two profiles (known relapse to bone versus patient) and then determines whether the patient profile exceeds the threshold. If it does, then the patient is classified as one who will relapse to bone and is accorded treatment such as adjuvant therapy, bisphosphonate therapy, or other appropriate therapy. If the patient profile does not exceed the threshold then they are classified as a patient without bone relapse. Other analytical tools can also be used to answer the same question such as, linear discriminate analysis, logistic regression and neural network approaches.

Numerous other well-known methods of pattern recognition are available. The following references provide some examples:

Weighted Voting: Golub et al. (1999).
Support Vector Machines: Su et al. (2001); and Ramaswamy et al. (2001).
K-nearest Neighbors: Ramaswamy (2001).
Correlation Coefficients: van 't Veer et al. (2002).

The gene expression profiles of this invention can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., Cancer Antigen 27.29 ("CA 27.29")). A range of such markers exists including such analytes as CA 27.29. In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Where a suspicious mass exists, a fine needle aspirate (FNA) is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous results.

Articles of this invention include representations of the gene expression profiles useful for treating, diagnosing, prognosticating, and otherwise assessing whether it is likely that a breast cancer patient will experience relapse in bone. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in Partek Discover™ and Partek Infer™ software from Partek® mentioned above can best assist in the visualization of such data.

Different types of articles of manufacture according to the invention are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the invention can be fashioned into reagent kits for conducting hybridization, amplification, and signal generation indicative of the level of expression of the genes of interest for detecting breast cancer.

Kits made according to the invention include formatted assays for determining the gene expression profiles. These can include all or some of the materials needed to conduct the assays such as reagents and instructions.

The invention is further illustrated by the following non-limiting examples. All references cited herein are hereby incorporated by reference herein.

## Examples

Genes analyzed according to this invention are typically related to full-length nucleic acid sequences that code for the production of a protein or peptide. One skilled in the art will recognize that identification of full-length sequences is not necessary from an analytical point of view. That is, portions of the sequences or ESTs can be selected according to well-known principles for which probes can be designed to assess gene expression for the corresponding gene.

### Example 1

### Sample Handling and Microarray Work for Previously Established Distant Relapse Profile

This example describes the establishment of a portfolio of genes for the identification of breast cancer patients at high risk of a relapse generally (i.e., not restricted to bone relapse).

Frozen tumor specimens from lymph node negative patients treated during 1980-1995, but untreated with systemic neoadjuvant therapy, were selected from the tumor bank at the Erasmus Medical Center (Rotterdam, Netherlands). All tumor samples were submitted to a reference laboratory from 25 regional hospitals for steroid hormone receptor measurements. The guidelines for primary treatment were similar for all hospitals. Tumors were selected in a manner to avoid bias. On the assumption of a 25-30% in 5 years, and a substantial loss of tumors because of quality control reasons, 436 invasive tumor samples were processed. Patients with a poor, intermediate, and good clinical outcome were included. Samples were rejected based on insufficient tumor content (53), poor RNA quality (77) or poor chip quality (20) leaving 286 samples eligible for further analysis.

Median age of patients at the time of surgery (breast conserving surgery: 219 patients; modified radical mastectomy: 67 patients) was 52 years (range, 26-83 years). Radiotherapy was given to 248 patients (87%) according to institutional protocol. Patients were included regardless of radiotherapy status, as this study was not aimed to investigate the potential effects of a specific type of surgery or adjuvant radiotherapy. Furthermore, studies have shown that radiotherapy has no clear effect on distant disease relapse. Early Breast Cancer Trialists (1995). Lymph node negativity was based on pathological examination by regional pathologists. Foekens et al. (1989a).

Prior to inclusion, all 286 tumor samples were confirmed to have sufficient (>70%) tumor and uniform involvement of tumor in H&E stained 5μm frozen sections. ER (and PgR) levels were measured by ligand binding assay or enzyme immunoassay (EIA) (Foekens et al. (1989b)) or by immunohistochemistry (in 9 tumors). The cutoff values used to classify patients as positive or negative for ER and PR was 10fmol/mg protein or 10% positive tumor cells. Postoperative follow-up involved examination every 3 months during the first 2 years, every 6 months for years 3 to 5, and every 12 months from year 5. The date of diagnosis of metastasis was defined as the date of confirmation of metastasis after symptoms reported by the patient, detection of clinical signs, or at regular follow-up. The median follow-up period of surviving patients (n=198) was 101 months (range, 20-171). Of the 286 patients included, 93 (33%) showed evidence of distant metastasis within 5 years and were counted as failures in the analysis of distant metastasis-free survival (DMFS). Five patients (2%) died without evidence of disease and were censored at last follow-up. Eighty-three patients (29%) died after a previous relapse. Therefore, a total of 88 patients (31%) were failures in the analysis of overall survival (OS).

### Example 2

### Gene Expression Analysis of data obtained in Example 1

Total RNA was isolated from 20 to 40 cryostat sections of 30 μm thickness (50-100 mg) with RNAzol B (Campro Scientific, Veenendaal, Netherlands). Biotinylated targets were prepared using published methods (Affymetrix, CA, Lipshutz et al. (1999)) and hybridized to the Affymetrix oligonucleotide microarray U133a GeneChip. Arrays were scanned using standard Affymetrix protocols. Each probe set was treated as a separate gene. Expression values were calculated using Affymetrix GeneChip analysis software MAS 5.0. Chips were rejected if average intensity was <40 or if the background signal >100. To normalize the chip signals, probe sets were scaled to a target intensity of 600, and scale mask files were not selected.

**Example 3**

**Statistical Analysis of genes identified in Example 2**

Gene expression data was filtered to include genes called "present" in two or more samples. 17,819 genes passed this filter and were used for hierarchical clustering. Before clustering, the expression level of each gene was divided by its median expression level in the patients. This standardization step limited the effect of the magnitude of expression of genes, and grouped together genes with similar patterns of expression in the clustering analysis. To identify patient subgroups, we carried out average linkage hierarchical clustering on both the genes and the samples using GeneSpring 6-0.

To identify genes that discriminate patients who developed distant metastases from those who remained metastasis-free for 5 years, two supervised class prediction approaches were used. In the first approach, 286 patients were randomly assigned to training and testing sets of 80 and 206 patients, respectively. Kaplan-Meier survival curves (Kaplan et al. (1958)) for the two sets were examined to ensure that there was no significant difference and no bias was introduced by the random selection of the training and testing sets. In the second approach, the patients were allocated to one of two subgroups stratified by ER status.

Each patient subgroup was analyzed separately in order to select markers. The patients in the ER-positive subgroup were randomly allocated into training and testing sets of 80 and 129 patients, respectively. The patients in the ER-negative subgroup were randomly divided into training and testing sets of 35 and 42 patients, respectively. The markers selected from each subgroup training set were combined to form a single signature to predict tumor metastasis for both ER-positive and ER-negative patients in a subsequent independent validation.

The sample size of the training set was determined by a resampling method to ensure its statistical confidence level. Briefly, the number of patients in the training set started at 15 patients and was increased by steps of 5. For a given sample size, 10 training sets with randomly selected patients were made. A gene signature was constructed from each of training sets and then tested in a designated testing set of patients by analysis of receiver operating characteristic (ROC) curve with distant metastasis within 5 years as the defining point. The mean and the coefficient of variation (CV) of the area under the curve (AUC) for a given sample size were calculated. A minimum number of patients required for the training set were chosen at the point that the average AUC reached a plateau and the CV of the 10 AUC was below 5%.

Genes were selected as follows. First, univariate Cox's proportional hazards regression was used to identify genes for which expression (on $\log_2$ scale) was correlated with the length of DMFS. To reduce the effect of multiple testing and to test the robustness of the selected genes, the Cox's model was constructed with bootstrapping of the patients in the training set. Efron et al. (1981). Briefly, 400 bootstrap samples of the training set were constructed, each with 80 patients randomly chosen with replacement. A Cox's model was run on each of the bootstrap samples. A bootstrap score was created for each gene by removing the top and bottom 5% p-values and then averaging the inverses of the remaining bootstrap p-values. This score was used to rank the genes. To construct a multiple gene signature, combinations of gene markers were tested by adding one gene at a time according to the rank order. ROC analysis using distant metastasis within 5 years as the defining point was performed to calculate the area under AUC for each signature with increasing number of genes until a maximum AUC value was reached.

The Relapse Score (RS) was used to calculate each patient's risk of distant metastasis. The score was defined as the linear combination of weighted expression signals with the standardized Cox's regression coefficient as the weight.

$$\text{Relapse Score} = A \cdot I + \sum_{i=1}^{60} I \cdot w_i x_i + B \cdot (1 - I) + \sum_{j=1}^{16} (1 - I) \cdot w_j x_j$$

where

$$I = \begin{cases} 1 & \text{if ER level} > 10 \text{ fmol per mg protein} \\ 0 & \text{if ER level} \leq 10 \text{ fmol per mg protein} \end{cases}$$

A and B are constants
$w_i$ is the standardized Cox's regression coefficient for ER + marker

$x_i$ is the expression value of ER + marker on a log2 scale
$w_j$ is the standardized Cox's regression coefficient for ER - marker
$x_j$ is the expression value of ER - marker on a log2 scale

The threshold was determined from the ROC curve of the training set to ensure 100% sensitivity and the highest specificity. The values of constants A of 313.5 and B of 280 were chosen to center the threshold of RS to zero for both ER-positive and ER-negative patients. Patients with positive RS scores were classified into the poor prognosis group and patients with negative RS scores were classified into the good prognosis group. The gene signature and the cutoff were validated in the testing set. Kaplan-Meier survival plots and log-rank tests were used to assess the differences in time to distant metastasis of the predicted high and low risk groups. Odds ratios (OR) were calculated as the ratio of the odds of distant metastasis between the patients predicted to relapse and those predicted to remain relapse-free.
Univariate and multivariable analyses with Cox's proportional hazards regression were done on the individual clinical variables with and without the gene signature. The HR and its 95% confidence interval (CI) were derived from these results. All statistical analyses were performed using S-Plus 6.1 software (Insightful, VA).

**Example 4**

**Pathway Analysis of genes identified in Example 3**

A functional class was assigned to each of the genes in the prognostic signature gene described in Examples 1-3 (non-bone specific relapse). Pathway analysis was done with Ingenuity 1.0 software (Ingenuity Systems, CA). Affymetrix probes were used as input to search for biological networks built by the software. Biological networks identified by the program were assessed in the context of general functional classes by GO ontology classification. Pathways with two or more genes in the prognostic signature were selected and evaluated.

**Example 5**

**Results for Examples 1-4**

Patient and Tumor Characteristics

Clinical and pathological features of the 286 patients of examples 1-3 are summarized in Table 1.

**Table 1. Clinical and Pathological Characteristics of Patients and Their Tumors**

| Characteristics | | All patients (%) | ER-positive training set (%) | ER-negative training set (%) | Validation set (%) |
|---|---|---|---|---|---|
| Number | | 286 | 80 | 35 | 171 |
| Age (mean±SD) | | 54±12 | 54±13 | 54±13 | 54±12 |
| | ≤40 yr | 36 (13) | 12 (15) | 3 (9) | 21 (12) |
| | 41-55 yr | 129 (45) | 30 (38) | 17 (49) | 82 (48) |
| | 56-70 yr | 89 (31) | 28 (35) | 11 (31) | 50 (29) |
| | >70 yr | 32 (11) | 10 (13) | 4 (11) | 18 (11) |
| Menopausal status | | | | | |
| | Premenopausal | 139 (49) | 39 (49) | 16 (46) | 84 (49) |
| | Postmenopausal | 147 (51) | 41 (51) | 19 (54) | 87 (51) |
| T stage | | | | | |
| | T1 | 146 (51) | 38 (48) | 14 (40) | 94 (55) |
| | T2 | 132 (46) | 41 (51) | 19 (54) | 72 (42) |
| | T3/4 | 8 (3) | 1 (1) | 2 (6) | 5 (3) |
| Grade | | | | | |
| | Poor | 148(52) | 37 (46) | 24(69) | 87 (51) |
| | Moderate | 42 (15) | 12(15) | 3 (9) | 27 (16) |
| | Good | 7 (2) | 2 (3) | 2 (6) | 3 (2) |
| | Unknown | 89 (31) | 29 (36) | 6 (17) | 54 (32) |
| ER* | | | | | |

(continued)

| Characteristics | | All patients (%) | ER-positive training set (%) | ER-negative training set (%) | Validation set (%) |
|---|---|---|---|---|---|
| | Positive | 209 (73) | 80 (100) | 0 (0) | 129 (75) |
| | Negative | 77 (27) | 0 (0) | 35(100) | 42 (25) |
| PgR* | | | | | |
| | Positive | 165(58) | 59(74) | 5 (14) | 101(59) |
| | Negative | 111 (39) | 19 (24) | 29 (83) | 63 (37) |
| | Unknown | 10 (3) | 2 (2) | 1 (3) | 7 (4) |
| Metastasis <5 years | | | | | |
| | Yes | 93 (33) | 24 (30) | 13 (37) | 56 (33) |
| | No | 183 (64) | 51 (64) | 17 (49) | 115 (67) |
| | Censored if <5 yr | 10 (3) | 5 (6) | 5 (14) | 0 (0) |

*ER-positive and PgR positive: >10 fmol/mg protein or >10% positive tumor cells.

There were no differences in age or menopausal status. The ER-negative training group had a slightly higher proportion of larger tumors and, as expected, more poor grade tumors than the ER-positive training group. The validation group of 171 patients (129 ER-positive, 42 ER-negative) did not differ from the total group of 286 patients with respect to any of the patients or tumor characteristics.

Two approaches were used to identify markers predictive of disease relapse. First, the data was divided randomly so that all the 286 patients (ER-positive and ER-negative combined) were put into a training set and a testing set. Thirty-five genes were selected from 80 patients in the training set and a Cox's model to predict the occurrence of distant metastasis was built. A moderate prognostic value was observed. Table 2. Unsupervised clustering analysis showed two distinct subgroups highly correlated with the tumor ER status (chi square test p <0.0001).

**Table 2**

| SEQ ID NO: | Cox's coefficient | p-value |
|---|---|---|
| 1 | 4.008 | 0.00006 |
| 2 | -3.649 | 0.00026 |
| 3 | 4.005 | 0.00006 |
| 4 | -3.885 | 0.00010 |
| 5 | -3.508 | 0.00045 |
| 6 | -3.176 | 0.00150 |
| 7 | 3.781 | 0.00016 |
| 8 | 3.727 | 0.00019 |
| 9 | -3.570 | 0.00036 |
| 10 | -3.477 | 0.00051 |
| 11 | 3.555 | 0.00038 |
| 12 | -3.238 | 0.00120 |
| 13 | -3.238 | 0.00120 |
| 14 | 3.405 | 0.00066 |
| 15 | 3.590 | 0.00033 |
| 16 | -3.157 | 0.00160 |
| 17 | -3.622 | 0.00029 |
| 18 | -3.698 | 0.00022 |
| 19 | 3.323 | 0.00089 |
| 20 | -3.556 | 0.00038 |
| 21 | -3.317 | 0.00091 |
| 22 | -2.903 | 0.00370 |
| 23 | -3.338 | 0.00085 |
| 24 | -3.339 | 0.00084 |
| 25 | -3.355 | 0.00079 |

(continued)

| SEQ ID NO: | Cox's coefficient | p-value |
|---|---|---|
| 26 | 3.713 | 0.00021 |
| 27 | -3.325 | 0.00088 |
| 28 | -2.984 | 0.00284 |
| 29 | 3.527 | 0.00042 |
| 30 | -3.249 | 0.00116 |
| 31 | -2.912 | 0.00360 |
| 32 | 3.118 | 0.00182 |
| 33 | 3.435 | 0.00059 |
| 34 | -2.971 | 0.00297 |
| 35 | 3.282 | 0.00103 |

Each subgroup was analyzed in order to select markers. Seventy-six genes were selected from patients in the training sets (60 for the ER-positive group, 16 for the ER-negative group). With the selected genes and ER status taken together, a Cox's model to predict relapse of cancer (not specific to bone) was built. Validation of the 76-gene predictor in the 171 patient testing set produced an ROC with an AUC value of 0.694, sensitivity of 93% (52/56), and specificity of 48% (55/115). Patients with a relapse score above the threshold of the prognostic signature have an 11·9-fold OR (95% CI: 4.04-35.1; p<0.0001) to develop distant metastasis within 5 years. As a control, randomly selected 76-gene sets were generated. These produced ROC with an average AUC value of 0.515, sensitivity of 91 %, and specificity of 12% in the testing group. Patients stratified by such a gene set would have an odds ratio of 1.3 (0.50-3.90; p=0.8) for development of metastases, indicating a random classification. In addition, the Kaplan-Meier analyses for distant metastasis free survival (DMFS) and overall survival (OS) as a function of the 76-gene signature showed highly significant differences in time to metastasis between the groups predicted to have good and poor prognosis. At 60 and 80 months, the respective absolute differences in DMFS between the groups with predicted good and poor prognosis were 40% (93% vs. 53%) and 39% (88% vs. 49%) and those in OS were 27% (97% vs. 70%) and 32% (95% vs. 63%), respectively.

The 76-gene profile also represented a strong prognostic factor for the development of distant metastasis in the subgroups of 84 premenopausal patients (HR: 9.60), 87 postmenopausal patients (HR: 4.04) and 79 patients with tumor sizes of 10 to 20 mm (HR: 14.1).

Univariate and multivariable Cox's regression analyses are summarized in Table 3.

**Table 3: Uni- and multivariable analyses for DMFS in the testing set of 171 relapse patients**

| | Univariate analysis | | | Multivariable analysis* | | |
|---|---|---|---|---|---|---|
| | HR† | (95%CI)† | p-value | HR† | (95%CI)† | p-value |
| Age‡ | | | | | | |
| Age2 vs. Age1 | 1.16 | (0.51 - 2.65) | 0.7180 | 1.14 | (0.45 - 2.91) | 0.7809 |
| Age3 vs. Age1 | 1.32 | (0.56 - 3.10) | 0.5280 | 0.87 | (0.26 - 2.93) | 0.8232 |
| Age4 vs. Age1 | 0.95 | (0.32 - 2.82) | 0.9225 | 0.61 | (0.15 - 2.60) | 0.5072 |
| Menopausal status§ | 1.24 | (0.76 - 2.03) | 0.3909 | 1.53 | (0.68 - 3.44) | 0.3056 |
| Stage‖ | 1.08 | (0.66-1.77) | 0.7619 | 2.57 | (0.23 - 29.4) | 0.4468 |
| Differentiation¶ | 0.38 | (0.16 - 0.90) | 0.0281 | 0.60 | (0.24-1.46) | 0.2590 |
| Tumor size** | 1.06 | (0.65-1.74) | 0.8158 | 0.34 | (0.03-3.90) | 0.3849 |
| ER†† | 1.09 | (0.61 - 1.98) | 0.7649 | 1.05 | (0.54-2.04) | 0.8935 |
| PR†† | 0.83 | (0.51 - 1.38) | 0.4777 | 0.85 | (0.47-1.53) | 0.5882 |

(continued)

| | Univariate analysis | | | Multivariable analysis* | | |
|---|---|---|---|---|---|---|
| | HR† | (95%CI)† | p-value | HR† | (95%CI)† | p-value |
| 76-gene signature | 5.67 | (2.59 - 12.4) | $1.5 \times 10^{-5}$ | 5.55 | (2.46 - 12.5) | $3.6 \times 10^{-5}$ |

*The multivariable model included 162 patients, due to missing values in 9 patients
† Hazard ratio and 95% confidence interval
‡ Agel is ≤40 yr, Age2 is 41 to 55 yr, Age3 is 56 to 70 yr, Age4 is >70 yr
§Post-menopausal vs. pre-menopausal
‖ Stage: II & III vs. I
¶Grade: moderate/good vs. poor, unknown grade was included as a separate group
**Tumor size: >20 mm vs. ≤20 mm
†† Positive vs. negative

Other than the 76-gene signature, only grade was significant in univariate analysis and moderate/good differentiation was associated with favorable DMFS. Multivariable regression estimation of HR for the occurrence of tumor metastasis within 5 years was 5.55 (p<0.0001), indicating that the 76-gene set represents an independent prognostic signature strongly associated with a higher risk of tumor metastasis. Univariate and multivariable analyses were also done separately for ER-positive and ER-negative patients the 76-gene signature was also an independent prognostic variable in the subgroups stratified by ER status.

The function of the 76 genes (Table 4) in the non-bone specific prognostic signature was analyzed to relate the genes to biological pathways.

**Table 4**

| ER Status | SEQ ID NO. | Std. Cox's coefficient | Cox's p-value |
|---|---|---|---|
| + | 36 | -3.83 | 0.00005 |
| + | 37 | -3.865 | 0.00001 |
| + | 38 | 3.63 | 0.00002 |
| + | 39 | -3.471 | 0.00016 |
| + | 40 | 3.506 | 0.00008 |
| + | 41 | -3.476 | 0.00001 |
| + | 42 | 3.392 | 0.00006 |
| + | 43 | -3.353 | 0.00080 |
| + | 44 | -3.301 | 0.00038 |
| + | 45 | 3.101 | 0.00033 |
| + | 46 | -3.174 | 0.00128 |
| + | 47 | 3.083 | 0.00020 |
| + | 48 | 3.336 | 0.00005 |
| + | 49 | -3.054 | 0.00063 |
| + | 50 | -3.025 | 0.00332 |
| + | 51 | 3.095 | 0.00044 |
| + | 52 | -3.175 | 0.00031 |
| + | 53 | -3.082 | 0.00086 |
| + | 54 | 3.058 | 0.00016 |
| + | 55 | 3.085 | 0.00009 |
| + | 56 | -2.992 | 0.00040 |
| + | 57 | -2.791 | 0.00020 |
| + | 58 | -2.948 | 0.00039 |
| + | 59 | 2.931 | 0.00020 |
| + | 60 | -2.896 | 0.00052 |
| + | 61 | 2.924 | 0.00050 |
| + | 62 | 2.915 | 0.00055 |
| + | 63 | -2.968 | 0.00099 |

(continued)

| ER Status | SEQ ID NO. | Std. Cox's coefficient | Cox's p-value |
|---|---|---|---|
| + | 64 | 2.824 | 0.00086 |
| + | 65 | -2.777 | 0.00398 |
| + | 66 | -2.635 | 0.00160 |
| + | 67 | -2.854 | 0.00053 |
| + | 68 | 2.842 | 0.00051 |
| + | 69 | -2.835 | 0.00033 |
| + | 70 | 2.777 | 0.00164 |
| + | 71 | -2.759 | 0.00222 |
| + | 72 | -2.745 | 0.00086 |
| + | 73 | 2.79 | 0.00049 |
| + | 74 | 2.883 | 0.00031 |
| + | 75 | -2.794 | 0.00139 |
| + | 76 | -2.743 | 0.00088 |
| + | 77 | -2.761 | 0.00164 |
| + | 78 | -2.831 | 0.00535 |
| + | 79 | 2.659 | 0.00073 |
| + | 80 | -2.715 | 0.00376 |
| + | 81 | 2.836 | 0.00029 |
| + | 82 | -2.687 | 0.00438 |
| + | 83 | -2.631 | 0.00226 |
| + | 84 | -2.716 | 0.00089 |
| + | 85 | 2.703 | 0.00232 |
| + | 86 | -2.641 | 0.00537 |
| + | 87 | -2.686 | 0.00479 |
| + | 88 | -2.654 | 0.00363 |
| + | 89 | 2.695 | 0.00095 |
| + | 90 | -2.758 | 0.00222 |
| + | 91 | 2.702 | 0.00084 |
| + | 92 | -2.694 | 0.00518 |
| + | 93 | 2.711 | 0.00049 |
| + | 94 | -2.771 | 0.00156 |
| + | 95 | 2.604 | 0.00285 |
| - | 96 | -3.495 | 0.00011 |
| - | 97 | 3.224 | 0.00036 |
| - | 98 | -3.225 | 0.00041 |
| - | 99 | -3.145 | 0.00057 |
| - | 100 | -3.055 | 0.00075 |
| - | 101 | -3.037 | 0.00091 |
| - | 102 | -3.066 | 0.00072 |
| - | 103 | 3.06 | 0.00077 |
| - | 104 | -2.985 | 0.00081 |
| - | 105 | -2.983 | 0.00104 |
| - | 106 | -3.022 | 0.00095 |
| - | 107 | -3.054 | 0.00082 |
| - | 108 | -3.006 | 0.00098 |
| - | 109 | -2.917 | 0.00134 |
| - | 110 | -2.924 | 0.00149 |
| - | 111 | -2.882 | 0.0017 |

Although 18 of the 76 genes have unknown function, several pathways or biochemical activities were identified that were well represented such as cell death, cell cycle and proliferation, DNA replication and repair and immune response (Table 5).

**Table 5. Pathway analysis of the 76 genes from the prognostic signature**

| Functional Class | 76-gene signature |
|---|---|
| Cell death | TNFSF10, TNFSF 13, MAP4, CD44, IL 18, GAS2, NEFL, EEF1A2, BCLG, C3 |
| Cell cycle | CCNE2, CD44, MAP4, SMC4L1, TNFSF10, AP2A2, FEN1, KPNA2, ORC3L, PLK1 |
| Proliferation DNA replication, recombination / repair | CD44, IL18, TNFSF10, TNFSF13, PPP1CC, CAPN2, PLK1, SAT TNFSF10, SMC4L1, FEN1, ORC3L, KPNA2, SUPT16H, POLQ, ADPRTL1 |
| Immune response | TNFSF10, CD44, IL18, TNFSF13, ARHGDIB, C3 |
| Growth | PPP1CC, CD44, IL 18, TNFSF10, SAT, HDGFRP3 |
| Cellular assembly and organization | MAP4, NEFL, TNFSF10, PLK1, AP2A2, SMC4L1 |
| Transcription | KPNA2, DUSP4, SUPT16H, DKFZP434E2220, PHF11, ETV2 |
| Cell-to-cell signaling and interaction | CD44, IL 18, TNFSF10, TNFSF 13, C3 |
| Survival | TNFSF10, TNFSF13, CD44, NEFL |
| Development | IL18, TNFSF10, COL2A1 |
| Cell morphology | CAPN2, CD44, TACC2 |
| Protein synthesis | IL18, TNFSF10, EEF1A2 |
| ATP binding | PRO2000, URKL1, ACACB |
| DNA binding | HIST1H4H, DKFZP434E2220, PHF11 |
| Colony formation | CD44, TNFSF10 |
| Adhesion | CD44, TMEM8 |
| Neurogenesis | CLN8, NEURL |
| Golgi apparatus | GOLPH2, BICD1 |
| Kinase activity | CNK1, URKL 1 |
| Transferase activity | FUT3, ADPRTL 1 |

Genes implicated in disease progression were found including calpain2, origin recognition protein, dual specificity phosphatases, Rho-GDP dissociation inhibitor, TNF superfamily protein, complement component 3, microtubule-associated protein, protein phosphatase 1 and apoptosis regulator BCL-G. Furthermore, previously characterized prognostic genes such as cyclin E2 (Keyomarsi et al. (2002)) and CD44 (Herrera-Gayol et al. (1999)) were in the gene signature.

The patients providing the samples had not received adjuvant systemic therapy, so the multigene assessment of prognosis was not subject to potentially confounding contributions by predictive factors related to systemic treatment. From this analysis a 76-gene signature that accurately predicts distant tumor relapse that is not specifically prognostic of bone relapse. This signature is applicable to all relapsing breast cancer patients independently of age, tumor size and grade and ER status. In Cox's multivariable analysis for DMFS the 76-gene signature was the only significant variable, superseding the clinical variables, including grade. After 5 years, absolute differences in DMFS and OS between the patients with the good and poor 76-gene signatures were 40% and 27%, respectively. Of the patients with a good prognosis signature, 7% developed distant metastases and 3% died within 5 years. If further validated, this prognostic signature will yield a positive predictive value of 37% and a negative predictive value of 95%, on the assumption of a 25% rate of disease relapse in breast cancer patients. In particular, this signature can be valuable for defining the risk of relapse for the increasing proportion of T1 tumors (<2 cm). Comparison with the St Gallen and NIH guidelines was instructive. Although ensuring the same number of the high-risk patients would receive the necessary treatment, the 76-gene signature would recommend systemic adjuvant chemotherapy to only 52% of the low-risk patients, as compared to 90% and 89% by the St. Gallen and NIH guidelines, respectively (Table 6).

**Table 6. Comparison of the 76-gene signature and the current conventional consensus on treatment of breast cancer**

| Method | Patients guided to receive adjuvant chemotherapy in the testing set | |
|---|---|---|
| | Metastatic disease at 5 years (%) | Metastatic disease free at 5 years (%) |
| St Gallen | 52/55 (95) | 104/115 (90) |
| NIH | 52/55 (95) | 101/114 (89) |
| 76-gene signature | 52/56 (93) | 60/115 (52) |

The conventional consensus criteria. St. Gallen: tumor ≥ 2cm, ER-negative, grade 2-3, patient <35 yr (either one of these criteria); NIH: tumor>1cm.

The 76-gene signature can thus result in a reduction of the number of low-risk relapse patients who would be recommended to have unnecessary adjuvant systemic therapy.

The 76-genes in the prognostic signature belong to many functional classes, suggesting that different paths could lead to disease progression. The signature included well-characterized genes and 18 unknown genes. This finding could explain the superior performance of this signature as compared to other prognostic factors. Although genes involved in cell death, cell proliferation, and transcriptional regulation were found in both patient groups stratified by ER status, the 60 genes selected for the ER-positive group and the 16 genes selected for the ER-negative group had no overlap. This result supports the idea that the extent of heterogeneity and the underlying mechanisms for disease progression could differ for the two ER-based subgroups of breast cancer patients.

Comparison of these results with those of the study by van de Vijver et al. (2002) is difficult because of differences in patients, techniques and materials used. van de Vijver et al. included both node-negative and node-positive patients, who had or had not received adjuvant systemic therapy, and only women younger than 53 years. Furthermore, the microarray platforms used in the studies are different, Affymetrix vs. Agilent. Of the 70 genes of the van't Veer (2002) study, only 48 are present on the Affymetrix U133a array, while of the 76 genes of this profile only 38 are present on the Agilent array. There is a 3-gene overlap between the two signatures (cyclin E2, origin recognition complex, and TNF superfamily protein). Despite the apparent difference, both signatures included genes that identified several common pathways that might be involved in tumor relapse. This finding supports the idea that while there might be redundancy in gene members, effective signatures could be required to include representation of specific pathways.

The strengths of the study described above compared with the study of van de Vijver et al. (2002) are the larger number of untreated relapse patients (286 vs. 141), and the independence of the 76-gene signature with respect to age, menopausal status, and tumor size. The validation set of patients in this approach is completely without overlap with the training set in contrast to 90% of other reports. Ransohoff (2004).

In conclusion, as only approximately 30-40% of the untreated patients develop tumor relapse, the prognostic signature could provide a powerful tool to identify those patients at low risk preventing over treatment in substantial numbers of patients. The recommendation of adjuvant systemic therapy in patients with primary breast cancer could be guided in the future by this prognostic signature. The preferred profiles described in Examples 1-5 (for risk of relapse generally) are the 35-gene portfolio made up of the genes of SEQ ID NOs: 1-35, the 60-gene portfolio made up of the genes of SEQ ID NOs: 36-95 which is best used to prognosticate ER-positive patients, and the 16-gene portfolio made up of genes of SEQ ID NOs: 96-111 which is best used to prognosticate ER-negative patients.

## Example 6

### Comparison of Breast Tumor Gene Profile Generated From Laser Capture Microdissection and Bulk Tissue In Stage I/II Breast Cancer

Gene-expression profiling has been shown to be a powerful diagnostic and prognostic tool for a variety of cancer types. Almost exclusively in all cases bulk tumor RNA was used for hybridization on the chip. Estrogens play important roles in the development and growth of hormone-dependent tumors.

About 75% of breast cancers express estrogen receptor (ER), which is an indicator for (adjuvant) tamoxifen treatment and is associated with patient outcomes.

To gain insights into the mechanisms trigged by estrogen in breast epithelia cells and their association with tumorigenesis, laser capture microdissection (LCM) was used to procure histologically homogenous population of tumor cells from 29 early stage primary breast tumors, in combination with GeneChip expression analysis. Of these 29 patients, 11 were ER-negative and 17 were ER-positive based on quantitative ligand binding or enzyme immunoassays on tumor cytosols. For comparison, gene expression profiling was also obtained using bulk tissue RNA isolated from the same group of 29 patients.

Fresh frozen tissue samples were collected from 29 lymph-node-negative breast cancer patients who had been surgically treated for a breast tumor and had not received neoadjuvant systemic therapy. For each patient tissue sample, an H&E slide was first used to evaluate the cell morphology. RNA was isolated from both tumor cells obtained by LCM (PALM) performed on cryostat sections and from whole cryostat sections, i.e., bulk tissue of the same tumor. RNA sample quality was analyzed by an Agilent BioAnalyzer. The RNA samples were hybridized to Affymetrix human U133A chip that contains approximately 22,000 probe sets. The fluorescence was quantified and the intensities were normalized. Clustering Analysis and Principal Component Analysis were used to group patients with similar gene expression profiles. Genes that are differentially expressed between ER-positive and ER-negative samples were selected.

Total RNA isolated from LCM procured breast cancer cells was subjected to two-round T7 based amplification in target preparation, versus one round amplification with bulk tissue RNA. Expression levels of 21 control genes (Table 7) were compared between LCM data set and bulk tissue set to demonstrate the fidelity of linear amplification.

**Table 7: Control gene list**

| SEQ ID NO: | Name |
|---|---|
| 112 | protein phosphatase 2, regulatory subunit B (B56), delta isoform |
| 113 | CCCTC-binding factor (zinc finger protein) |
| 114 | solute carrier family 4 (anion exchanger), member 1, adaptor protein |
| 115 | ribonuclease P |
| 116 | hypothetical protein FLJ20188 |
| 117 | KIAA0323 protein |
| 118 | cDNA FLJ12469 |
| 119 | translation initiation factor eIF-2b delta subunit |
| 120 | heterogeneous nuclear ribonucleoprotein K |
| 121 | hydroxymethylbilane synthase |
| 122 | cDNA DKFZp586O0222 |
| 123 | chromosome 20 open reading frame 4 |
| 124 | thyroid hormone receptor interactor 4 |
| 125 | hypoxanthine phosphoribosyltransferase 1 (Lesch-Nyhan syndrome) |
| 126 | DnaJ (Hsp40) homolog, subfamily C, member 8 |
| 127 | dual specificity phosphatase 11 (RNA/RNP complex 1-interacting) |
| 128 | calcium binding atopy-related autoantigen 1 |
| 129 | stromal cell-derived factor 2 |
| 130 | Ewing sarcoma breakpoint region 1 |
| 131 | CCR4-NOT transcription complex, subunit 2 |
| 132 | F-box only protein 7 |

The results obtained are depicted in Table 8.

**Table 8 Clinical characteristics of patients**

| Characteristic | | No. of patients (%) |
|---|---|---|
| Age in years | | |
| | < 40 | 1 (3) |
| | 40-44 | 5(17) |
| | 45-49 | 8(28) |
| | ≥ 50 | 15 (52) |

(continued)

| | | |
|---|---|---|
| Tumor diameter in mm | | |
| | ≤ 20 | 11 (40) |
| | > 20 | 17 (59) |
| Histologic grade | | |
| | II (intermediate) | 5 (17) |
| | III (poor) | 12 (41) |
| Estrogen-receptor status | | |
| | Negative | 11 (40) |
| | Positive | 17 (59) |
| Surgery | | |
| | Breast-conserving therapy | 26 (90) |
| | Mastectomy | 3(10) |
| Chemotherapy | | |
| | No | 29(100) |
| Hormonal therapy | | |
| | No | 29 (100) |
| Disease-free survival in months | | |
| | ≤48 | 13 (45) |
| | > 48 | 16(55) |

A hierarchical clustering based on 5121 genes showed that LCM and bulk tissue samples are completely separated based on global RNA expression profiles. The expression levels of 21 control genes in RNA isolates from LCM samples and bulk tissues subjected to an additional round of linear amplification used for RNA obtained by LCM did not cause differential expression of the control genes. Differentially expressed genes between ER-positive and ER-negative sub-clusters in both LCM and bulk tissue samples were defined by Student T-test pathway analysis by Gene Ontology for genes exclusively associated with ER in LCM samples, exclusively in bulk tissues, and for those that are common in both LCM and bulk tissue were conducted.

The results obtained show several important conclusions. First, genes related to cell proliferation and energy metabolism were seen differentially expressed in ER-/ER+ patients both in bulk tissue data set and LCM data set. Second, due to the enrichment of breast cancer cells via LCM, genes involved in cell surface receptor linked signal transduction, RAS signal transduction, JAK-STAT signal transduction and apoptosis were found associate to ER status. These genes were not identified in bulk data set. Third, microdissection provides a sensitive approach to studying epithelial tumor cells and an insight into signaling pathway associated with estrogen receptors. Therefore, it is clear that the application of the gene expression profile described herein to LCM isolated tumor cells is commensurate with results obtained in heterogeneous bulk tissue.

**Example 7**

**Validation and pathway analysis of the 76-gene prognostic signature in breast cancer**

This Example reports the results of a validation study in which the 76-gene signature was used to predict outcomes of 132 patients obtained from 4 independent sources.

In addition, in order to evaluate the robustness of this gene signature, this Example further provides identification of substitutable components of the signature and describes how the substitutions lead to the identification of key pathways in an effective signature.

Fresh frozen tissue samples were collected from 132 patients who had been surgically treated for a breast tumor and had not received adjuvant systemic therapy. The patient samples used were collected between 1980 and 1996. For each patient tissue sample, an H&E slide was used to evaluate the cell morphology. Then total RNA samples were prepared and the sample quality was analyzed by Agilent BioAnalyzer. The RNA samples were analyzed by microarray analysis. The fluorescence was quantified and the intensities were normalized. A relapse hazard score was calculated for each patient based on the expression levels of the 76-gene signature. The patients were classified into good and poor outcome groups.

In order to evaluate the robustness of this gene signature, two statistical analyses were designed and used. First, gene selection and signature construction procedures that were used to discover the 76-gene signature were repeated. As shown in Table 8, ten training sets of 115 patients each were randomly selected from the total of 286 patients. The remaining patients were served as the testing set.

Second, the number of patients in a training set was increased to 80% of the 286 patients and used the remaining 20% of the patients as the testing set. This selection procedure was also repeated 10 times. In both procedures, Kaplan-Meier survival curves were used to ensure no significant difference in disease free survival between the training and the testing pair. Genes were selected and a signature was built from each of the training sets using Cox's proportional-hazards regression. Each signature was validated in the corresponding testing set. Furthermore, the 76-gene prognostic signature was assigned into functional groups using GO ontology classification. Pathways that cover significant numbers of genes in the signature were selected (p-value <0.05 and >2 hits). The selected pathways were also evaluated in all the prognostic signatures derived from different training sets.

**Table 9A: Results from 10 signatures using training sets of 115 patients, Table 9B: Results from 10 signatures using training sets of 80% of the patients.**

| A | | B | |
|---|---|---|---|
| AUC of ROC | 0.62 (0.55-0.70) | AUC of ROC | 0.62 (0.53-0.72) |
| Sensitivity | 86% (0.84 - 0.88) | Sensitivity | 83% (0.81 - 0.85) |
| Specificity | 34% (0.21 - 0.56) | Specificity | 46% (0.28 - 0.62) |
| Freq. of Relapse | 33% | Freq. of Relapse | 33% |
| PPV | 40% (0.35-0.49) | PPV | 47% (0.32-0.58) |
| NPV | 81 % (0.75-0.89) | NPV | 82% (0.78-0.89) |
| Odds Ratio | 3.5 (1.7-7.9) | Odds Ratio | 5.6 (1.7-15) |

The results obtained in this Example show that:

- The 76-gene signature is successfully validated in 132 independent patients, giving an AUC value of 0.757 in the 132 relapse breast cancer patients from 4 independent sources. The signature shows 88% sensitivity and 41% specificity.
- The average AUC for the substitute signatures is 0.64 (95% CI: 0.53 -0.72). This result is consistent with that of the 76-gene predictor (AUC of 0.69). Twenty-one pathways over-represented in the 76-gene signature were also found in all the other prognostic signatures, suggesting that common biological pathways are involved in tumor relapse.
- These results suggest that gene expression profiles provide a powerful approach to perform risk assessment of patient outcome. The data highlight the feasibility of a molecular prognostic assay that provides patients with a quantitative measurement of tumor relapse.

**Example 8**

**Bone Relapse Signatures**

From the sample set used to establish the 76-gene profile for predicting distant relapse, 107 samples were selected to further study bone relapse. These samples were all selected because the site of relapse was known and the samples could be grouped into bone and non-bone distant relapse sets. Those classified as bone relapse samples included those that had bone relapse and also possibly relapsed in other parts of the body. The remaining relapse patient samples were labeled non-bone.

The information relating to the samples used in these analyses are shown in Table 10.

Two different analyses were performed. First, Significance Analysis of Microarrays (SAM) analysis was used to identify differentially expressed genes in the case of relapse in bone relative to relapse elsewhere (i.e., non-bone). In the second analysis a bone relapse predictor was established to determine the likelihood of a patient for relapsing in bone. This signature is referred to as a Prediction Analysis of Microarrays (PAM).

In the case of the SAM analysis, 300 permutations of the data were used to calculate a false discovery rate (FDR). Genes were considered significant when the FDR was below 5% and when a minimum 1.7 fold difference in expression level was observed. To construct a diagnostic profile that would be useful in distinguishing those who (from among those likely to relapse) would be likely to relapse in bone, samples were divided into a training set (n=72, 46 with a relapse in bone and 26 with a non-bone relapse) and a testing set (n=35, 23 bone and 12 non-bone relapses) stratified by site of relapse, ER protein level and metastasis-free interval. A gene selection step using an optimal cut-off procedure was performed in the samples of the training set. All measured expression levels of a gene were used as the cut point to assign the gene being "high" or "low" in a particular sample, keeping a minimum of 20 samples in one of the groups. Knowing the site of relapse of these samples, the frequencies for the categories high/Bone, low/Bone, high/Non-bone and low/Non-bone were counted for each cut-off. The optimal cut-off was determined by using the $\chi^2$ distribution. Genes were included if the maximal $\chi^2$ score was 10.827 or higher (p <0.001) for analysis in a Prediction Analysis of Microarrays (PAM).

TFF1 was the most significant gene in the gene profiles established through this procedure (from a statistical point of view). Further experiments to determine TFF 1 mRNA levels by quantitative RT-PCR were performed using the following primer pairs (TGGAGCAGAGAGGAGGCAAT and ACGAACGGTGTCGTCGAAAC). The samples selected for the RT-PCR study were matched for patient and tumor characteristics listed in Table 10. Gene expression levels were expressed relative to a panel of housekeeper genes and were $^2$log transformed. The difference in gene expression levels of TFF 1 was correlated to the two relapse groups and p-values were calculated using Kruskal-Wallis anova, $\chi^2$ approximates and corrected for ties. Statistical analyses were performed using Analyse-it software (Analyse-it Software Ltd, Leeds, United Kingdom).

**Results**

**SAM Analysis**

The samples described above were classified according to the site of relapse, 69 samples were labeled as bone and 38 as non-bone. Using SAM, 73 probe-sets representing 69 unique genes were seen as significantly differentially expressed between the bone and non-bone samples. The 5 highest ranking genes were TFF1, TFF3, AGR2, NAT1, and CRIP1 all of which are higher expressed in the bone relapse samples. The highest ranked gene, TFF1, was studied in 122 independent breast tumors by quantitative RT-PCR. TFF1 expression was significantly associated with the site of relapse (p = 0.0015) with relative median expression level and 95% CI for TFF1 of 3.02 (1.41 to 4.66) and -1.63 (-5.44 to 2.49) for the bone and non-bone relapse group, respectively. Genes corresponding to SEQ ID No.s 112-147 were higher expressed in bone relapse samples. The remainder were lower expressed.

**PAM Analysis**

The samples were divided into a training set (n=72) and a testing set (n=35) stratified by site of relapse, ER protein level and metastasis-free interval. Using the optimal cut-off procedure, 588 informative genes were selected for input in the PAM analysis. A 31-gene predictor was selected after 10-fold cross-validation of the training set that could identify the bone relapse samples in the testing set with 100% sensitivity and 50% specificity. The predictor showed a 79.3% positive predictive value and misclassified 17% of the samples. 17 genes in the profile, including TFF1, were also present in the SAM gene list (all 31 genes are referenced in the "PAM"-column in Table 11).

To ascertain the validity of the gene set, 50 sets of 100 randomly chosen genes were also analyzed. These random gene sets were used for input in a PAM analysis using the same training and testing set. The mean percentage of misclassified samples was 28.5% (SD 4.3%). This indicates that the 17% misclassified samples found by the actual PAM gene list is significantly lower (z-value 2.67, two-tailed p=0.008) than the random data sets.

**Table 10. Clinical and tumor characteristics of patients for SAM and PAM analyses.**

| Characteristics | All patients | Bone relapse | Non-bone relapse |
|---|---|---|---|
| Number | 107 | 69 | 38 |
| Age (mean±SD) | 53±12 | 52±12 | 54±11 |
| ≤40 yr | 16(15%) | 12(17%) | 4 (11%) |
| 41-55 yr | 49(46%) | 32(46%) | 17 (45%) |
| 56-70 yr | 34(32%) | 20(29%) | 14 (37%) |

(continued)

| Characteristics | | All patients | Bone relapse | Non-bone relapse |
|---|---|---|---|---|
| | >70 yr | 8 (7%) | 5 (7%) | 3 (8%) |
| Menopausal status | | | | |
| | Premenopausal | 51 (48%) | 33 (48%) | 18 (47%) |
| | Postmenopausal | 56 (52%) | 36 (52%) | 20 (53%) |
| T stage | | | | |
| | T1 | 54(50%) | 38 (55%) | 16(42%) |
| | T2 | 50(47%) | 31 (45%) | 19(50%) |
| | T3/4 | 3 (3%) | 0 (0%) | 3 (8%) |
| Grade | | | | |
| | Poor | 61 (57%) | 39(57%) | 22(58%) |
| | Good-Moderate | 10 (9%) | 9 (13%) | 1 (3%) |
| | Unknown | 36 (34%) | 21 (30%) | 15 (39%) |
| ER*† | | | | |
| | Positive | 80 (75%) | 57 (83%) | 23 (61%) |
| | Negative | 27 (25%) | 12 (17%) | 15 (39%) |
| PgR* | | | | |
| | Positive | 56 (52%) | 38 (55%) | 18 (47%) |
| | Negative | 48 (45%) | 28 (41%) | 20 (52%) |
| | Unknown | 3 (3%) | 3 (4%) | 0 (0%) |

* ER and PgR are defined positive when tumors contain > 10 fmol/mg protein or > 10% positive tumor cells.

† Patient characteristics are equally distributed between the bone or non-bone relapses, except for ER status (p-value = 0.02), calculated using the $\chi^2$ distribution.

**Table 11: Genes involved in bone matastasis of breast cancer.**

| SEQ ID NO | Probe-id | Gene Symbol | SAM Score | Fold Change | FDR (%) | PAM† | Gene Title |
|---|---|---|---|---|---|---|---|
| 112 | 205009_at | TFF1 | -4.92 | 3.1 | 1.9 | yes | trefoil factor 1 |
| 113 | 204623_at | TFF3 | -4.23 | 2.6 | 1.9 | yes | trefoil factor 3 (intestinal) |
| 114 | 209173_at | AGR2 | -4.06 | 1.9 | 1.9 | | anterior gradient 2 homolog |
| 115 | 214440_at | NAT1 | -4.04 | 2.5 | 1.9 | yes | N-acetyltransferase 1 |
| 116 | 205081_at | CRIP1 | -3.80 | 1.9 | 1.9 | yes | cysteine-rich protein 1 (intestinal) |
| 117 | 214774_x_at | TNRC9 | -3.72 | 1.9 | 1.9 | yes | trinucleotide repeat containing 9 |
| 118 | 214858_at | -- | -3.60 | 2.0 | 1.9 | yes | Pp14571 |
| 119 | 219197_s_at | SCUBE2 | -3.59 | 2.1 | 1.9 | | signal peptide, CUB domain, EGF-like 2 |

(continued)

| SEQ ID NO | Probe-id | Gene Symbol | SAM Score | Fold Change | FDR (%) | PAM† | Gene Title |
|---|---|---|---|---|---|---|---|
| 120 | 215108_x_at | TNRC9 | -3.57 | 1.9 | 1.9 | yes | trinucleotide repeat containing 9 |
| 121 | 206754_s_at | CYP2B6 | -3.57 | 2.1 | 1.9 | | cytochrome P450, family 2, subfamily B, polypeptide 6 |
| 122 | 210056_at | RND1 | -3.48 | 1.7 | 1.9 | yes | Rho family GTPase 1 |
| 123 | 205186_at | DNALI1 | -3.45 | 2.0 | 1.9 | | dynein, axonemal, light intermediate polypeptide 1 |
| 124 | 203130_s_at | KIF5C | -3.42 | 2.0 | 1.9 | | kinesin family member 5C |
| 125 | 216623_x_at | TNRC9 | -3.32 | 1.9 | 1.9 | yes | trinucleotide repeat containing 9 |
| 126 | 222256_s_at | PLA2G4B | -3.31 | 1.8 | 1.9 | | phospholipase A2, group IVB (cytosolic) |
| 127 | 210021_s_at | UNG2 | -3.29 | 1.7 | 1.9 | yes | uracil-DNA glycosylase 2 |
| 128 | 204607_at | HMGCS2 | -3.22 | 2.3 | 1.9 | yes | 3-hydroxy-3-methylglutaryl-Coenzyme A synthase 2 |
| 129 | 213664_at | SLC1A1 | -3.17 | 2.7 | 1.9 | yes | solute carrier family 1 member 1 |
| 130 | 211657_at | CEACAM6 | -3.16 | 2.3 | 1.9 | | carcinoembryonic antigen-related cell adhesion molecule 6 |
| 131 | 222348_at --- | | -3.01 | 1.7 | 1.9 | | --- |
| 132 | 209114_at | TSPAN-1 | -2.93 | 1.8 | 1.9 | | tetraspan 1 |
| 133 | 205645_at | REPS2 | -2.84 | 1.8 | 1.9 | | RALBP1 associated Eps domain containing 2 |
| 134 | 39763_at | HPX | -2.84 | 1.7 | 1.9 | | hemopexin |
| 135 | 214099_s_at | PDE4DIP | -2.83 | 2.2 | 1.9 | | phosphodiesterase 4D interacting protein |
| 136 | 203757_s_at | CEACAM6 | -2.77 | 2.8 | 1.9 | | carcinoembryonic antigen-related cell adhesion molecule 6 |
| 137 | 204485_s_at | TOM1L1 | -2.76 | 1.8 | 1.9 | | target of myb1-like 1 |
| 138 | 206378_at | SCGB2A2 | -2.76 | 1.9 | 1.9 | | secretoglobin, family 2A, member 2 |
| 139 | 211712_s_at | ANXA9 | -2.76 | 1.7 | 1.9 | | annexin A9 |

(continued)

| SEQ ID NO | Probe-id | Gene Symbol | SAM Score | Fold Change | FDR (%) | PAM† | Gene Title |
|---|---|---|---|---|---|---|---|
| 140 | 204378_at | BCAS1 | -2.73 | 1.8 | 1.9 | | breast carcinoma amplified sequence 1 |
| 141 | 206243_at | TIMP4 | -2.67 | 1.8 | 3.3 | | tissue inhibitor of metalloproteinase 4 |
| 142 | 210272_at | CYP2B6 | -2.58 | 1.8 | 4.4 | | cytochrome P450, family 2, subfamily B, polypeptide 6 |
| 143 | 205597_at | C6orf29 | -2.56 | 1.7 | 4.4 | | chromosome 6 open reading frame 29 |
| 144 | 221946_at | C9orf116 | -2.50 | 1.9 | 4.4 | | chromosome 9 open reading frame 116 |
| 145 | 210297_s_at | MSMB | -2.49 | 3.0 | 4.4 | | microseminoprotein, beta- |
| 146 | 204014_at | DUSP4 | -2.48 | 1.9 | 4.4 | | dual specificity phosphatase 4 |
| 147 | 204379_s_at | FGFR3 | -2.47 | 1.8 | 4.4 | | fibroblast growth factor receptor 3 |
| 148 | 205014_at | FGFBP1 | 3.66 | 1.9 | 1.9 | | fibroblast growth factor binding protein 1 |
| 149 | 209406_at | BAG2 | 3.65 | 1.8 | 1.9 | | BCL2-associated athanogene 2 |
| 150 | 210655_s_at | FOXO3A | 3.56 | 2.6 | 1.9 | yes | forkhead box 03A |
| 151 | 220559_at | EN1 | 3.53 | 2.5 | 1.9 | | engrailed homolog 1 |
| 152 | 209800_at | KRT16 | 3.46 | 12.7 | 1.9 | | keratin 16 |
| 153 | 209373_at | BENE | 3.39 | 1.9 | 1.9 | | BENE protein |
| 154 | 214595_at | KCNG1 | 3.33 | 3.4 | 1.9 | | potassium voltage-gated channel, subfamily G, member 1 |
| 155 | 216365_x_at | IGLC2 /// IGLJ3 | 3.28 | 2.7 | 1.9 | | Immunoglobulin lambda constant 2 /// Immunoglobulin lambda joining 3 |
| 156 | 206125_s_at | KLK8 | 3.22 | 1.8 | 1.9 | | kallikrein 8 |
| 157 | 211637_x_at | | 3.22 | 2.4 | 1.9 | | Immunoglobulin heavy chain V region (Humha448) /// Similar to lg heavy chain V-I region HG3 precursor |
| 158 | 209126_x_at | KRT6B | 3.20 | 2.5 | 1.9 | yes | keratin 6B |

(continued)

| SEQ ID NO | Probe-id | Gene Symbol | SAM Score | Fold Change | FDR (%) | PAM† | Gene Title |
|---|---|---|---|---|---|---|---|
| 159 | 219480_at | SNAI1 | 3.15 | 1.8 | 3.3 | | snail homolog 1 |
| 160 | 205347_s_at | TMSNB | 3.10 | 2.3 | 3.3 | | thymosin, beta, identified in neuroblastoma cells |
| 161 | 210683_at | NRTN | 3.02 | 2.7 | 3.3 | yes | neurturin |
| 162 | 1438_at | EPHB3 | 2.99 | 2.0 | 3.3 | yes | EPH receptor B3 |
| 163 | 217294_s_at | ENO1 | 2.87 | 2.3 | 4.4 | yes | enolase 1 |
| 164 | 215223_s_at | SOD2 | 2.86 | 1.9 | 4.4 | | superoxide dismutase 2, mitochondrial |
| 165 | 211908_x_at | IGHG1 | 2.85 | 1.9 | 4.4 | | immunoglobulin heavy constant gamma 1 (G1m marker) |
| 166 | 222242_s_at | KLK5 | 2.80 | 2.2 | 4.4 | | kallikrein 5 |
| 167 | 206391_at | RARRES1 | 2.79 | 2.2 | 4.4 | | retinoic acid receptor responder 1 |
| 168 | 219415_at | TTYH1 | 2.78 | 5.0 | 4.4 | | tweety homolog 1 |
| 169 | 209772_s_at | CD24 | 2.78 | 2.1 | 4.4 | | CD24 antigen |
| 170 | 217281_x_ | at MGC27165 /// IGHG1 | 2.77 | 2.1 | 4.4 | | hypothetical protein MGC27165 /// immunoglobulin heavy constant gamma 1 (G1m marker) |
| 171 | 204855_at | SERPINB5 | 2.75 | 2.5 | 4.4 | | serine (or cysteine) proteinase inhibitor, clade B member 5 |
| 172 | 201387_s_at | UCHL1 | 2.73 | 2.6 | 4.4 | | ubiquitin carboxyl-terminal esterase L1 |
| 173 | 220425_x_at | ROPN1 | 2.73 | 2.6 | 4.4 | | ropporin, rhophilin associated protein 1 |
| 174 | 218484_at | LOC56901 | 2.72 | 2.4 | 4.4 | | NADH:ubiquinone oxidoreductase MLRQ subunit homolog |
| 175 | 215177_s_at | ITGA6 | 2.70 | 1.9 | 4.4 | | integrin, alpha 6 |
| 176 | 209372_x_at | TUBB /// MGC8685 | 2.70 | 2.1 | 4.4 | | tubulin, beta polypeptide |
| 177 | 202316_x_at | UBE4B | 2.67 | 1.9 | 4.4 | | ubiquitination factor E4B |

(continued)

| SEQ ID NO | Probe-id | Gene Symbol | SAM Score | Fold Change | FDR (%) | PAM† | Gene Title |
|---|---|---|---|---|---|---|---|
| 178 | 211641_x_ at | IGHM | 2.67 | 2.0 | 4.4 | | Immunoglobulin heavy chain VH3 (H11) |
| 179 | 217404_s_ at | COL2A1 | 2.65 | 4.4 | 4.4 | | collagen, type II, alpha 1 |
| 180 | 203126_at | IMPA2 | 2.65 | 1.9 | 4.4 | | inositol(myo)-1(or 4)-monophosphatase 2 |
| 181 | 221986_s_ at | DRE1 | 2.64 | 1.8 | 4.4 | | DRE1 protein |
| 182 | 205778_at | KLK7 | 2.64 | 4.0 | 4.4 | | kallikrein 7 |
| 183 | 202134_s_ at | TAZ | 2.60 | 2.0 | 4.4 | | transcriptional co-activator with PDZ-binding motif (TAZ) |
| 184 | 212065_s_ at | USP34 | 2.60 | 1.8 | 4.4 | | ubiquitin specific protease 34 |
| 185 | 201952_at | --- | | | | yes | |
| 186 | 202987_at | C60RF4 | | | | yes | Chromosome 6 open reading frame 4 |
| 187 | 218489_s_ at | ALAD | | | | yes | aminolevulinate, delta-, dehydratase |
| 188 | 213606_s_ at | ARHGDIA | | | | yes | Rho GDP dissociation inhibitor (GDI) alpha |
| 189 | 201679_at | ARS2 | | | | yes | arsenate resistance protein |
| 190 | 217528_at | CLCA2 | | | | yes | chloride channel, calcium activated, family member 2 |
| 191 | 205830_at | CLGN | | | | yes | calmegin |
| 192 | 220363_s_ at | ELM02 | | | | yes | engulfment and cell motility 2 (ced-12 homolog, C. elegans) |
| 193 | 220622_at | LRRC31 | | | | yes | leucine rich repeat containing 31 |
| 194 | 202601_s_ at | HTATSF1 | | | | yes | HIV TAT specific factor 1 |
| 195 | 206638_at | HTR2B | | | | yes | 5-hydroxytryptamine (serotonin) receptor 2B |
| 196 | 218211_s_ at | MLPH | | | | yes | melanophilin |
| 197 | 218985_at | SLC2A8 | | | | yes | solute carrier family 2, member 8 |

(continued)

| SEQ ID NO | Probe-id | Gene Symbol | SAM Score | Fold Change | FDR (%) | PAM† | Gene Title |
|---|---|---|---|---|---|---|---|
| 198 | 209278_s_at | TFPI2 | | | | yes | tissue factor pathway inhibitor 2 |
| † Genes identified by the PAM analysis; the genes from this analysis that were not identified by SAM are appended after the SAM-identified genes. | | | | | | | |

**Pathway Analysis for Bone Relapse Signature**

Differentially expressed genes were compared to those found in the Gene Ontology and KEGG databases. As there were only 8 genes from the SAM list annotated in the KEGG database, that list was merged with a recently published bone metastasis profile. In that study, Kang et al. generated gene expression profiles from sub clones of the ER-negative breast cancer cell line MDA-MB-231 that when injected into mice, poorly or efficiently relapsed to bone. Differentially expressed genes between those two subtypes were considered as the bone relapse signature. Since Kang et al. used the same microarrays as those used in the examples above, it was convenient to merge their 127 probe-set list (122 unique genes) with the SAM gene list (n=69). Although the two profiles share only one gene (BENE), it is likely they address common pathways. To this end, both lists were mapped on the KEGG database. The in total 20 KEGG-annotated genes revealed that 5 of the 20 genes (FGF5, SOS1 and DUSP1 (Kang list) and FGFR3 and DUSP4 (SAM)) were located in the FGFR-p42/44 MAP-kinase pathway; this number of genes is statistically different from a random dataset ($p<0.0001$). All 5 genes were up-regulated in the bone metastasizing cells/tumors. The 142 genes from the combined list that were annotated in Gene Ontology database were studied. Determinations were made as to whether the Gene Ontology descriptions were over-represented in the merged SAM/Kang list compared with all genes printed on the U133a chip. Over-represented annotations point to biological processes, which are possibly linked to the site of relapse. For example, the description "extracellular" was linked to 21 of the 142 (14.8%) genes from the bone marker list, whereas 1350 out of 16367 genes (8.2%) of the U133a chip were annotated to this description. This means "extracellular" is 1.8 times over-represented ($p=0.006$, $\chi^2$-distribution) in the bone relapse list. Other examples are "cell adhesion" (17 genes, $p=0.0007$) and "cell organization and biogenesis" (22 genes, $p=2.3\ 10^{-5}$) found 2.2 and 2.4 times over-represented, respectively. Additional, "immune response" was significant ($p=8.7\ 10^{-5}$), but in contrast to the above-mentioned descriptions the genes linked to "immune response" originated predominantly from the Kang list.

Table 12 identifies the sequences referred to in this specification.

**Table 12: Sequence identification**

| SEQ ID NO: | psid | Gene Name | Accession # | Gene description |
|---|---|---|---|---|
| 1 | 213165_at | CDABP0086 | A1041204 | |
| 2 | 217432_s_at | | AF179281 | iduronate 2-sulfatase (Hunter syndrome) |
| 3 | 221500_s_at | | BE782754 | syntaxin 16 / |
| 4 | 208452_x_at | MYO9B | NM_004145 | myosin IXB |
| 5 | 220234_at | CA8 | NM_004056 | carbonic anhydrase VIII |
| 6 | 207865_s_at | BMP8 | NM_001720 | bone morphogenetic protein 8 (osteogenic protein 2) |
| 7 | 201769_at | KIAA0171 | NM_014666 | KIAA0171 gene product |
| 8 | 218940_at | FLJ13920 | NM_024558 | hypothetical protein FLJ13920 |
| 9 | 209018_s_at | BRPK | BF432478 | protein kinase BRPK |
| 10 | 216647 at | DKFZp586L1 824 | AL117663 | from clone DKFZp586L1824 |
| 11 | 213405 at | DKFZp564E1 22 | N95443 | from clone DKFZp564E122 |
| 12 | 202921_s_at | ANK2 | NM_001148 | ankyrin 2, neuronal, transcript variant 1 |

(continued)

| SEQ ID NO: | psid | Gene Name | Accession # | Gene description |
|---|---|---|---|---|
| 13 | 208401_s_at | | U01157 | glucagon-like peptide-1 receptor with CA dinucleotide repeat |
| 14 | 218090_s_at | WDR11 | NM_018117 | WD40 repeat domain 11 protein |
| 15 | 218139_s_at | FLJ10813 | NM_018229 | hypothetical protein FLJ10813 |
| 16 | 202485_s_at | MBD2 | NM_003927 | methyl-CpG binding domain protein 2, transcript variant 1 |
| 17 | 201357_s_at | SF3A1 | NM_005877 | splicing factor 3a, subunit 1, 120kD |
| 18 | 214616_at | H3FD | NM_003532 | H3 histone family, member D |
| 19 | 207719_x_at | KIAA0470 | NM_014812 | KIAA0470 gene product |
| 20 | 202734_at | TRIP10 | NM_004240 | thyroid hormone receptor interactor 10 |
| 21 | 202175_at | FLJ22678 | NM_024536 | hypothetical protein FLJ22678 |
| 22 | 213870_at | | AL031228 | clone 1033B10 on chromosome 6p21.2-21.31 |
| 23 | 208967_s_at | adk2 | U39945 | adenylate kinase 2 |
| 24 | 204312_x_at | | AI655737 | cAMP responsive element binding protein 1 |
| 25 | 203815_at | GSTT1 | NM_000853 | glutathione S-transferase ζ1 |
| 26 | 207996_s_at | C180RF1 | NM_004338 | chromosome 18 open reading frame 1 |
| 27 | 221435_x_at | HT036 | NM_031207 | hypothetical protein HT036 |
| 28 | 219987_at | FLJ12684 | NM_024534 | hypothetical protein FLJ12684 |
| 29 | 221559_s_at | MGC:2488 | BC000229 | clone MGC:2488 |
| 30 | 207007_at | NR113 | NM_005122 | nuclear receptor subfamily 1, group I, mem 3 |
| 31 | 219265_at | FLJ13204 | NM_024761 | hypothetical protein FLJ13204 |
| 32 | 40420_at | | AB015718 | lok mRNA for protein kinase |
| 33 | 202266_at | AD022 | NM_016614 | TRAF and TNF receptor-associated protein |
| 34 | 219522_at | FJX1 | NM_014344 | putative secreted ligand homologous to fjx1 |
| 35 | 212334_at | AKAP350C | BE880245 | AKAP350C, alternatively spliced |
| 36 | 219340_s_at | CLN8 | AF123759 | Putative transmembrane protein |
| 37 | 217771_at | GP73 | NM_016548 | Golgi membrane protein (LOC51280) |
| 38 | 202418_at yeast | Yif1p | NM_020470 | Putative transmembrane protein; homolog of Golgi membrane protein |
| 39 | 206295_at | IL-18 | NM_001562 | Interleukin 18 |
| 40 | 201091_s_at | | BE748755 | Heterochromatin-like protein |
| 41 | 204015_s_at | DUSP4 | BC002671 | Dual specificity phosphatase 4 |
| 42 | 200726_at | PPP1CC | NM_002710 | Protein phosphatase 1, catalytic subunit, γ isoform |
| 43 | 200965_s_at | ABLIM-s | NM_006720 | Actin binding LIM protein 1, transcript variant |

(continued)

| SEQ ID NO: | psid | Gene Name | Accession # | Gene description |
|---|---|---|---|---|
| 44 | 210314_x_at | TRDL-1 | AF114013 | Tumor necrosis factor-related death ligand 1 γ |
| 45 | 221882_s_at | M83 | AI636233 | Five-span transmembrane protein |
| 46 | 217767_at | C3 | NM_000064 | Complement component 3 |
| 47 | 219588_s_at | FLJ20311 | NM_017760 | hypothetical protein |
| 48 | 204073_s_at | C11ORF9 | NM_013279 | chromosome 11 open reading frame 9 |
| 49 | 212567_s_at | | AL523310 | Putative translation initiation factor |
| 50 | 211382_s_at | TACC2 | AF220152 | |
| 51 | 201663_s_at | CAP-C | NM_005496 | chromosome-associated polypeptide C |
| 52 | 221344 at | OR12D2 | NM_013936 | Olfactory receptor, family 12, subfamily D, member 2 |
| 53 | 210028_s_at | ORC3 | AF125507 | Origin recognition complex subunit 3 |
| 54 | 218782_s_at | PRO2000 | NM_014109 | PRO2000 protein |
| 55 | 201664_at | SMC4 | AL136877 | (Structural maintenance of chromosome 4, yeast)-like |
| 56 | 219724_s_at | KIAA0748 | NM_014796 | KIAA0748 gene product |
| 57 | 204014_at | DUSP4 | NM_001394 | Dual specificity phosphatase 4 |
| 58 | 212014_x_at | CD44 | AI493245 | CD44 |
| 59 | 202240_at | PLK1 | NM_005030 | Polo (Drosophila)-like kinase 1 |
| 60 | 204740_at | CNK1 | NM_006314 | connector enhancer of KSR-like (Drosophila kinase suppressor of ras) |
| 61 | 208180_s_at | H4FH | NM_003543 | H4 histone family, member H |
| 62 | 204768_s_at | FEN1 | NM_004111 | Flap structure-specific endonuclease |
| 63 | 203391_at | FKBP2 | NM_004470 | FK506-binding protein 2 |
| 64 | 211762_s_at | KPNA2 | BC005978 | Karyopherin α 2 (RAG cohort 1, importin α 1) |
| 65 | 218914_at | CGI-41 | NM_015997 | CGI-41 protein |
| 66 | 221028_s_at | MGC11335 | NM_030819 | hypothetical protein MGC11335 |
| 67 | 211779_x_at | MGC13188 | BC006155 | Clone MGC:13188 |
| 68 | 218883_s_at | FLJ23468 | NM_024629 | hypothetical protein FLJ23468 |
| 69 | 204888_s_at | | AA772093 | Neuralized (Drosophila)-like |
| 70 | 217815_at | FACTP140 | NM_007192 | Chromatin-specific transcription elongation factor, 140 kD subunit |
| 71 | 201368_at | Tis11d | U07802 | |
| 72 | 201288_at | ARHGDIB | NM_001175 | Rho GDP dissociation inhibitor (GDI) β |
| 73 | 201068_s_at | PSMC2 | NM_002803 | Proteasome (prosome, macropain) 26S subunit, ATPase, 2 |
| 74 | 218478 s at | DKFZP434E 2220 | NM 017612 | hypothetical protein DKFZP434E2220 |
| 75 | 214919_s_at | KIAA1085 | R39094 | |

(continued)

| SEQ ID NO: | psid | Gene Name | Accession # | Gene description |
|---|---|---|---|---|
| 76 | 209835_x_at | | BC004372 | Similar to CD44 |
| 77 | 217471_at | | AL117652 | |
| 78 | 203306 s at | SLC35A1 | NM_006416 | Solute carrier family 35 (CMP-sialic acid transporter), member 1 |
| 79 | 205034_at | CCNE2 | NM_004702 | Cyclin E2 |
| 80 | 221816_s_at | | BF055474 | Putative zinc finger protein NY-REN-34 antigen |
| 81 | 219510_at | POLQ | NM_006596 | Polymerase (DNA directed) ζ |
| 82 | 217102_at | | AF041410 | Malignancy-associated protein |
| 83 | 208683_at | CANP | M23254 | Ca2-activated neutral protease large subunit |
| 84 | 215510_at | | AV693985 | ets variant gene 2 |
| 85 | 218533_s_at | FLJ20517 | NM_017859 | hypothetical protein FLJ20517 |
| 86 | 215633_x_at | LST-1N | AV713720 | mRNA for LST-1N protein |
| 87 | 221928_at | | A1057637 | Hs234898 ESTs, weakly similar to 2109260A B-cell growth factor |
| 88 | 214806_at | BICD | U90030 | Bicaudal-D |
| 89 | 204540_at | EEF1A2 | NM_001958 | eukaryotic translation elongation factor 1 α 2 |
| 90 | 221916_at | | BF055311 | hypothetical protein |
| 91 | 216693_x_at | DKFZp434C1 722 | AL133102 | |
| 92 | 209500_x_at | | AF114012 | tumor necrosis factor-related death ligand-1β |
| 93 | 209534_at | FLJ10418 | AK001280 | moderately similar to Hepatoma-derived growth factor |
| 94 | 207118_s_at | MMP23A | NM_004659 | matrix metalloproteinase 23A |
| 95 | 211040_x_at | | BC006325 | G-2 and S-phase expressed 1 |
| 96 | 218430_s_at | FLJ12994 | NM_022841 | hypothetical protein FLJ12994 |
| 97 | 217404_s_at | | X16468 | α-1 type II collagen. |
| 98 | 205848_at | GAS2 | NM_005256 | growth arrest-specific 2 |
| 99 | 214915_at | FLJ11780 | AK021842 | clone HEMBA1005931, weakly similar to zinc finger protein 83 |
| 100 | 216010_x_at | | D89324 | α(1,31,4) fucosyltransferase |
| 101 | 204631_at | MYH2 | NM_017534 | myosin heavy polypep 2 skeletal muscle adult |
| 102 | 202687_s_at | | U57059 | Apo-2 ligand mRNA |
| 103 | 221634_at | | BC000596 | Similar to ribosomal protein L23a, clone MGC:2597 |
| 104 | 220886_at | GABRQ | NM_018558 | γ-aminobutyric acid (GABA) receptor, ζ |
| 105 | 202237_at | ADPRTL1 | NM_006437 | ADP-ribosyltransferase (NAD+; poly (ADP-ribose) polymerase)-like 1 |

(continued)

| SEQ ID NO: | psid | Gene Name | Accession # | Gene description |
|---|---|---|---|---|
| 106 | 204218_at | DKFZP564M 082 | NM_014042 | protein DKFZP564M082 |
| 107 | 221241_s_at | BCLG | NM_030766 | apoptosis regulator BCL-G |
| 108 | 209862 s at | | BC001233 | Similar to KIAA0092 gene product, clone MGC:4896 |
| 109 | 217019_at | RPS4X | AL137162 | Contains novel gene and 5 part of gene for novel protein similar to X-linked ribosomal protein 4 |
| 110 | 210593_at | | M55580 | spermidinespermine N1-acetyltransferase |
| 111 | 216103_at | KIAA0707 | AB014607 | KIAA0707 |
| 112 | 205009_at | TFF1 | NM 003225 | trefoil factor 1 |
| 113 | 204623_at | TFF3 | NM 003226 | trefoil factor 3 (intestinal) |
| 114 | 209173_at | AGR2 | AF088867 | anterior gradient 2 homolog |
| 115 | 214440_at | NAT1 | NM_000662 | N-acetyltransferase 1 |
| 116 | 205081_at | CRIP1 | NM_001311 | cysteine-rich protein 1 (intestinal) |
| 117 | 214774_x_at | TNRC9 | AK027006 | trinucleotide repeat containing 9 |
| 118 | 214858_at | --- | AF070536 | Pp14571 |
| 119 | 219197_s_at | SCUBE2 | AI424243 | signal peptide, CUB domain, EGF-like 2 |
| 120 | 215108_x_at | TNRC9 | U80736 | trinucleotide repeat containing 9 |
| 121 | 206754_s_at | CYP2B6 | NM_000767 | cytochrome P450, family 2, subfamily B, polypeptide 6 |
| 122 | 210056_at | RND1 | U69563 | Rho family GTPase 1 |
| 123 | 205186_at | DNALI1 | NM_003462 | dynein, axonemal, light intermediate polypeptide 1 |
| 124 | 203130_s_at | KIF5C | NM_004522 | kinesin family member 5C |
| 125 | 216623_x_at | TNRC9 | AK025084 | trinucleotide repeat containing 9 |
| 126 | 222256_s_at | PLA2G4B | AK000550 | phospholipase A2, group IVB (cytosolic) |
| 127 | 210021_s_at | UNG2 | BC004877 | uracil-DNA glycosylase 2 |
| 128 | 204607_at | HMGCS2 | NM_005518 | 3-hydroxy-3-methylglutaryl-Coenzyme A synthase 2 |
| 129 | 213664_at | SLC1A1 | AW235061 | solute carrier family 1 member 1 |
| 130 | 211657_at | CEACAM6 | M18728 | carcinoembryonic antigen-related cell adhesion molecule 6 |
| 131 | 222348_at | --- | AW971134 | --- |
| 132 | 209114_at | TSPAN-1 | AF133425 | tetraspan 1 |
| 133 | 205645_at | REPS2 | NM_004726 | RALBP1 associated Eps domain containing 2 |
| 134 | 39763_at | HPX | M36803 | hemopexin |

(continued)

| SEQ ID NO: | psid | Gene Name | Accession # | Gene description |
|---|---|---|---|---|
| 135 | 214099_s_at | PDE4DIP | AK001619 | phosphodiesterase 4D interacting protein |
| 136 | 203757_s_at | CEACAM6 | BC005008 | carcinoembryonic antigen-related cell adhesion molecule 6 |
| 137 | 204485_s_at | TOM1L1 | NM_005486 | target of myb1-like 1 |
| 138 | 206378_at | SCGB2A2 | NM_002411 | secretoglobin, family 2A, member 2 |
| 139 | 211712_s_at | ANXA9 | BC005830 | annexin A9 |
| 140 | 204378_at | BCAS1 | NM_003657 | breast carcinoma amplified sequence 1 |
| 141 | 206243_at | TIMP4 | NM_003256 | tissue inhibitor of metalloproteinase 4 |
| 142 | 210272_at | CYP2B6 | M29873 | cytochrome P450, family 2, subfamily B, polypeptide 6 |
| 143 | 205597_at | C6orf29 | NM_025257 | chromosome 6 open reading frame 29 |
| 144 | 221946_at | C9orf116 | AU160041 | chromosome 9 open reading frame 116 |
| 145 | 210297_s_at | MSMB | U22178 | microseminoprotein, beta- |
| 146 | 204014_at | DUSP4 | NM_001394 | dual specificity phosphatase 4 |
| 147 | 204379_s_at | FGFR3 | NM_000142 | fibroblast growth factor receptor 3 |
| 148 | 205014_at | FGFBP1 | NM_005130 | fibroblast growth factor binding protein 1 |
| 149 | 209406_at | BAG2 | AF095192 | BCL2-associated athanogene 2 |
| 150 | 210655_s_at | FOXO3A | AF041336 | forkhead box 03A |
| 151 | 220559_at | EN1 | NM_001426 | engrailed homolog 1 |
| 152 | 209800_at | KRT16 | AF061812 | keratin 16 |
| 153 | 209373_at | BENE | BC003179 | BENE protein |
| 154 | 214595_at | KCNG1 | AI332979 | potassium voltage-gated channel, subfamily G, member 1 |
| 155 | 216365_x_at | IGLC2///IGLJ3 | AF047245 | Immunoglobulin lambda constant 2 /// Immunoglobulin lambda joining 3 |
| 156 | 206125_s_at | KLK8 | NM 007196 | kallikrein 8 |
| 157 | 211637_x_at | --- | L23516 | Immunoglobulin heavy chain V region (Humha448) /// Similar to Ig heavy chain V-I region HG3 precursor |
| 158 | 209126_x_at | KRT6B | L42612 | keratin 6B |
| 159 | 219480_at | SNAI1 | NM 005985 | snail homolog 1 |
| 160 | 205347_s_at | TMSNB | NM 021992 | thymosin, beta, identified in neuroblastoma cells |
| 161 | 210683_at | NRTN | AL161995 | neurturin |
| 162 | 1438_at | EPHB3 | X75208 | EPH receptor B3 |
| 163 | 217294_s_at | ENO1 | U88968 | enolase 1 |
| 164 | 215223_s_at | SOD2 | W46388 | superoxide dismutase 2, mitochondrial |
| 165 | 211908_x_at | IGHG1 | M87268 | immunoglobulin heavy constant gamma 1 (G1m marker) |

(continued)

| SEQ ID NO: | psid | Gene Name | Accession # | Gene description |
|---|---|---|---|---|
| 166 | 222242_s_at | KLK5 | AF243527 | kallikrein 5 |
| 167 | 206391_at | RARRES1 | NM 002888 | retinoic acid receptor responder 1 |
| 168 | 219415_at | TTYH1 | NM 020659 | tweety homolog 1 |
| 169 | 209772_s_at | CD24 | X69397 | CD24 antigen |
| 170 | 217281_x_at | MGC27165/// IGHG1 | AJ239383 | hypothetical protein MGC27165 /// immunoglobulin heavy constant gamma 1 (G1m marker) |
| 171 | 204855_at | SERPINB5 | NM 002639 | serine (or cysteine) proteinase inhibitor, clade B member 5 |
| 172 | 201387_s_at | UCHL1 | NM_004181 | ubiquitin carboxyl-terminal esterase L1 |
| 173 | 220425_x_at | ROPN1 | NM 017578 | ropporin, rhophilin associated protein 1 |
| 174 | 218484_at | LOC56901 | NM_020142 | NADH:ubiquinone oxidoreductase MLRQ subunit homolog |
| 175 | 215177_s_at | ITGA6 | AV733308 | integrin, alpha 6 |
| 176 | 209372_x_at | TUBB /// MGC8685 | BF971587 | tubulin, beta polypeptide |
| 177 | 202316_x_at | UBE4B | AW241715 | ubiquitination factor E4B |
| 178 | 211641_x_at | IGHM | L06101 | Immunoglobulin heavy chain VH3 (H11) |
| 179 | 217404_s_at | COL2A1 | X16468 | collagen, type II, alpha 1 |
| 180 | 203126_at | IMPA2 | NM_014214 | inositol(myo)-1 (or4)-monophosphatase2 |
| 181 | 221986_s_at | DRE1 | AW006750 | DRE1 protein |
| 182 | 205778_at | KLK7 | NM_005046 | kallikrein 7 |
| 183 | 202134_s_at | TAZ | NM 015472 | transcriptional co-activator with PDZ-binding motif (TAZ) |
| 184 | 212065_s_at | USP34 | AB018272 | ubiquitin specific protease 34 |
| 185 | 201952_at | --- | NM_001627 | |
| 186 | 202987_at | C6ORF4 | AW296296 | Chromosome 6 open reading frame 4 |
| 187 | 218489_s_at | ALAD | NM_000031 | aminolevulinate, delta-, dehydratase |
| 188 | 213606_s_at | ARHGDIA | AI571798 | Rho GDP dissociation inhibitor (GDI) alpha |
| 189 | 201679_at | ARS2 | NM_015908 | arsenate resistance protein |
| 190 | 217528_at | CLCA2 | BF003134 | chloride channel, calcium activated, family member 2 |
| 191 | 205830_at | CLGN | NM_004362 | calmegin |
| 192 | 220363_s_at | ELMO2 | NM_022086 | engulfment and cell motility 2 (ced-12 homolog, C. elegans) |
| 193 | 220622_at | LRRC31 | NM_024727 | leucine rich repeat containing 31 |
| 194 | 202601_s_at | HTATSF1 | A1373539 | HIV TAT specific factor 1 |
| 195 | 206638_at | HTR2B | NM_000867 | 5-hydroxytryptamine (serotonin) receptor 2B |

(continued)

| SEQ ID NO: | psid | Gene Name | Accession # | Gene description |
|---|---|---|---|---|
| 196 | 218211_s_at | MLPH | NM_024101 | melanophilin |
| 197 | 218985_at | SLC2A8 | NM_014580 | solute carrier family 2, member 8 |
| 198 | 209278_s_at | TFPI2 | L27624 | tissue factor pathway inhibitor 2 |

Ahr et al. (2002) "Identification of high risk breast-cancer patients by gene-expression profiling" Lancet 359: 131-132

Chang et al. (2003) "Gene expression profiling for the prediction of therapeutic response to docetaxel in patients with breast cancer" Lancet 362:362-9

Early Breast Cancer Trialists' Collaborative Group (1995) "Effects of radiotherapy and surgery in early breast cancer. An overview of the randomized trials" N Engl J Med 333:1444-1455

Early Breast Cancer Trialists' Collaborative Group (1998a) "Polychemotherapy for early breast cancer: an overview of the randomized trials" Lancet 352:930-942

Early Breast Cancer Trialists' Collaborative Group (1998b) "Tamoxifen for early breast cancer: an overview of randomized trials" Lancet 351:1451-1467

Efron (1981) "Censored data and the bootstrap" J Am Stat Assoc 76:312-319

Eifel et al. (2001) "National Institutes of Health Consensus Development Conference Statement: adjuvant therapy for breast cancer, November 1-3, 2000" J Natl Cancer Inst93:979-989

Foekens et al. (1989b) "Prognostic value of estrogen and progesterone receptors measured by enzyme immunoassays in human breast tumor cytosols" Cancer Res 49:5823-5828

Foekens et al. (1989a) "Prognostic value of receptors for insulin-like growth factor 1, somatostatin, and epidermal growth factor in human breast cancer" Cancer Res 49:7002-7009

Goldhirsch et al. (2003) "Meeting highlights: Updated International Expert Consensus on the Primary Therapy of Early Breast Cancer" J Clin Oncol 21:3357-3365

Golub et al. (1999) "Molecular classification of cancer: class discovery and class prediction by gene expression monitoring" Science 286:531-537

Gruvberger et al. (2001) "Estrogen receptor status in breast cancer is associated with remarkably distinct gene expression patterns" Cancer Res 61:5979-5984

Hedenfalk et al. (2001) "Gene-expression profiles in hereditary breast cancer" N Engl J Med 344:539-548

Herrera-Gayol et al. (1999) "Adhesion proteins in the biology of breast cancer: contribution of CD44" Exp Mol Pathol 66:149-156

Huang et al. (2003) "Gene expression predictors of breast cancer outcomes" Lancet 361:1590-1596

Kaplan et al. (1958) "Non-parametric estimation of incomplete observations" J Am Stat Assoc 53:457-481

Keyomarsi et al. (2002) "Cyclin E and survival in patients with breast cancer" N Engl J Med 347:1566-1575

Lipshutz et al. (1999) "High density synthetic oligonucleotide arrays" Nat Genet 21:20-24

Ma et al. (2003) "Gene expression profiles of human breast cancer progression" Proc Natl Acad Sci USA 100: 5974-5979

Ntzani et al. (2003) "Predictive ability of DNA microarrays for cancer outcomes and correlates: an empirical assessment" Lancet 362:1439-1444

Perou et al. (2000) "Molecular portraits of human breast tumors" Nature 406:747-752

Ramaswamy et al. (2001) "Multiclass cancer diagnosis using tumor gene expression signatures" Proc Natl Acad Sci USA 98:15149-15154

Ramaswamy et al. (2003) "A molecular signature of metastasis in primary solid tumors" Nat Genet 33:1-6

Ransohoff (2004) "Rules of evidence for cancer molecular-marker discovery and validation" Nat Rev Cancer 4:309-314

Sørlie et al. (2001) "Gene expression patterns of breast carcinomas distinguish tumor subclasses with clinical implications" Proc Natl Acad Sci USA 98:10869-10874

Sørlie et al. (2003) "Repeated observation of breast tumor subtypes in independent gene expression data sets" Proc Natl Acad Sci USA 100:8418-8423

Sotiriou et al. (2003) "Gene expression profiles derived from fine needle aspiration correlate with response to systemic chemotherapy in breast cancer" Breast Cancer Res 4:R3

Sotiriou et al. (2003) "Breast cancer classification and prognosis based on gene expression profiles from a population-based study" Proc Natl Acad Sci USA 100:10393-10398

Su et al. (2001) "Molecular classification of human carcinomas by use of gene expression signatures" Cancer Res 61:7388-7393

van de Vijver et al. (2002) "A gene expression signature as a predictor of survival in breast cancer" N Engl J Med 347:1999-2009

van't Veer et al. (2002) "Gene expression profiling predicts clinical outcome of breast cancer" Nature 415: 530-536

Wang et al. (2004) "Gene expression profiles and molecular markers to predict relapse of Dukes' B colon cancer" J Clin Oncol 22:1564-1571

Woelfle et al. (2003) "Molecular signature associated with bone marrow micrometastasis in human breast cancer" Cancer Res 63:5679-5684

## Claims

**1.** A method of assessing breast cancer status comprising the steps of

a. obtaining a biological sample from a breast cancer patient; and
b. measuring the expression levels in the sample of genes via a Marker

wherein the gene expression levels above or below pre-determined cut-off levels are indicative of the likelihood of relapse in bone.

**2.** A method of staging breast cancer patients comprising the steps of

a. obtaining a biological sample from a breast cancer patient; and
b. measuring the expression levels in the sample of genes via a Marker

wherein the gene expression levels above or below pre-determined cut-off levels are indicative of the breast cancer stage.

**3.** The method of claim 2 wherein the stage corresponds to classification by the TNM system.

**4.** The method of claim 2 wherein the stage corresponds to patients with similar gene expression profiles.

**5.** A method of determining breast cancer patient treatment protocol comprising the steps of

a. obtaining a biological sample from a breast cancer patient; and
b. measuring the expression levels in the sample of genes via a Marker

wherein the gene expression levels above or below pre-determined cut-off levels are sufficiently indicative of risk of relapse in bone to enable a physician to determine the degree and type of therapy recommended to prevent or treat relapse in bone.

**6.** A method of treating a breast cancer patient comprising the steps of:

a. obtaining a biological sample from a breast cancer patient; and
b. measuring the expression levels in the sample of genes via a Marker
wherein the gene expression levels above or below pre-determined cut-off levels are indicate a high risk of relapse in bone and;
c. treating the patient with adjuvant therapy, bisphosphonate therapy, or other relevant therapy if they are a high risk patient.

**7.** The method of claim 1 wherein the bulk tissue preparation is obtained from a biopsy or a surgical specimen.

8. The method of claim 1 wherein the Markers include all of those corresponding to SEQ ID NOs: 112-198.

9. The method of claim 1, 2, 5 or 6 further comprising measuring the expression level of at least one gene constitutively expressed in the sample.

10. The method of claim 1, 2, 5 or 6 further comprising determining the estrogen receptor (ER) status of the sample.

11. The method of claim 10 wherein the ER status is determined by measuring the expression level of at least one gene indicative of ER status.

12. The method of claim 11 wherein the ER status is determined by measuring the presence of ER in the sample.

13. The method of claim 12 wherein the presence of ER is measured immunohistochemically.

14. The method of claim 1, 2, 5 or 6 wherein the sample is obtained from a primary tumor.

15. The method of claim 1, 2, 5 or 6 wherein the specificity is at least about 40%.

16. The method of claim 1, 2, 5 or 6 wherein the sensitivity is at least at least about 90%.

17. The method of claim 1, 2, 5 or 6 wherein the expression pattern of the genes is compared to an expression pattern indicative of a breast cancer patient that relapses to bone.

18. The method of claim 17 wherein the comparison of expression patterns is conducted with pattern recognition methods.

19. The method of claim 18 wherein the pattern recognition methods include the use of a bone relapse predictor score.

20. The method of claim 1, 2, 5 or 6 wherein the pre-determined cut-off levels are at least 1.7-fold over- or under-expression in the sample relative to cells or tissue from non-bone relapsing patients.

21. The method of claim 1, 2, 5 or 6 wherein the pre-determined cut-off levels have at least a statistically significant p-value over-expression in the sample having metastatic cells relative to cells or tissue from non-bone relapsing patients.

22. The method of claim 21 wherein the p-value is less than 0.05.

23. The method of claim 1, 2, 5 or 6 wherein gene expression is measured on a microarray or gene chip.

24. The method of claim 23 wherein the microarray is a cDNA array or an oligonucleotide array.

25. The method of claim 23 wherein the microarray or gene chip further comprises one or more internal control reagents.

26. The method of claim 1, 2, 5 or 6 wherein gene expression is determined by nucleic acid amplification conducted by polymerase chain reaction (PCR) of RNA extracted from the sample.

27. The method of claim 26 wherein said PCR is reverse transcription polymerase chain reaction (RT-PCR).

28. The method of claim 27, wherein the RT-PCR further comprises one or more internal control reagents.

29. The method of claim 1, 2, 5 or 6 wherein gene expression is detected by measuring or detecting a protein encoded by the gene.

30. The method of claim 29 wherein the protein is detected by an antibody specific to the protein.

31. The method of claim 1, 2, 5 or 6 wherein gene expression is detected by measuring a characteristic of the gene.

32. The method of claim 31 wherein the characteristic measured is selected from the group consisting of DNA amplification, methylation, mutation and allelic variation.

**33.** A method of assessing breast cancer status comprising the steps of

a. obtaining a biological sample from a breast cancer patient; and
b. measuring the expression levels in the sample of genes via a Marker

wherein the gene expression levels above or below pre-determined cut-off levels are indicative of the likelihood of relapse in bone.

**34.** A kit for conducting an assay to determine breast cancer prognosis in a biological sample comprising a Marker..

**35.** The kit of claim 34 wherein the Marker corresponds to any of SEQ ID NO 112-116.

**36.** The kit of claim 34 wherein the Marker corresponds to all of SEQ ID NO 112-116.

**37.** The kit of claim 35 including Markers corresponding to one or more of SEQ ID NOs. 117-198.

**38.** The kit of claim 34 including Markers corresponding to all of SEQ ID NOs. 112-198.

**39.** The kit of claim 34 further comprising reagents for conducting a microarray analysis.

**40.** The kit of claim 34 further comprising a medium through which said nucleic acid sequences, their complements, or portions thereof are assayed.

**41.** Articles for assessing breast cancer status comprising Markers.

**42.** The articles of claim 41 wherein the Marker corresponds to any of SEQ ID NO 112-116.

**43.** The articles of claim 41 wherein the Marker corresponds to all of SEQ ID NO 112-116.

**44.** The articles of claim 41 including Markers corresponding to one or more of SEQ ID NOs. 117-198.

**45.** The articles of claim 41 further comprising reagents for conducting a microarray analysis.

**46.** The articles of claim 41 further comprising a medium through which said nucleic acid sequences, their complements, or portions thereof are assayed.

**47.** A microarray or gene chip for performing the method of claim 1, 2, 5, or 6.

**48.** The microarray of claim 47 comprising a Maker sufficient to characterize breast cancer status or risk of relapse in bone from a biological sample.

**49.** The microarray of claim 47 wherein the measurement or characterization is at least 1.7-fold over- or under-expression.

**50.** The microarray of claim 47 wherein the measurement provides a statistically significant p-value over- or under-expression.

**51.** The microarray of claim 47 wherein the p-value is less than 0.05.

**52.** The microarray of claim 47 comprising a cDNA array or an oligonucleotide array.

**53.** The microarray of claim 47 further comprising or more internal control reagents.

**54.** A diagnostic/prognostic portfolio comprising a Marker sufficient to characterize breast cancer status or risk of relapse in bone in a biological sample.

**55.** The portfolio of claim 54 wherein the measurement or characterization is at least 1.7-fold over- or under-expression.

**56.** The portfolio of claim 54 wherein the measurement provides a statistically significant p-value over- or under-expression.

**57.** The portfolio of claim 54 wherein the p-value is less than 0.05.

European Patent Office

## PARTIAL EUROPEAN SEARCH REPORT

which under Rule 45 of the European Patent Convention shall be considered, for the purposes of subsequent proceedings, as the European search report

Application Number
EP 06 25 4064

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/073137 A1 (CHEN LAN BO [US] ET AL) 17 April 2003 (2003-04-17) * claim 24 * ----- | 1-5,7, 9-47 | INV. C12Q1/68 |
| X | KANG Y ET AL: "A MULTIGENIC PROGRAM MEDIATING BREAST CANCER METASTASIS TO BONE" CANCER CELL, XX, US, vol. 3, no. 6, June 2003 (2003-06), pages 537-549, XP008062522 ISSN: 1535-6108 * the whole document * ----- | 1-5,7, 9-47 | |
| X | US 6 207 375 B1 (SUBRAMANIAM MALAYANNAN [US] ET AL) 27 March 2001 (2001-03-27) * column 22, lines 8-18 * ----- | 1-5,7, 9-47 | |
| X | WO 99/50666 A (SEIBEL MARKUS [DE]; DIEL INGO J [DE]) 7 October 1999 (1999-10-07) * the whole document * ----- | 1-5,7, 9-47 | TECHNICAL FIELDS SEARCHED (IPC) |
| X | WO 00/22130 A2 (CHIRON CORP [US]) 20 April 2000 (2000-04-20) * claim 18 * ----- -/-- | 1-5,7, 9-47 | C12Q |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

6

| Place of search | Date of completion of the search | Examiner |
|---|---|---|
| The Hague | 11 January 2007 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C07)

European Patent Office

# PARTIAL EUROPEAN SEARCH REPORT

Application Number
EP 06 25 4064

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| P,X | WO 2006/061430 A2 (PROSKELIA [FR]; EDICALE INSERM INST NAT DE LA [FR]; CLEMENT-LACROIX PH) 15 June 2006 (2006-06-15) * the whole document * ----- | 1-5,7, 9-47 | |
| P,X | WO 2005/078136 A (HEALTH RESEARCH INC [US]; COIGNET LIONEL J [US]) 25 August 2005 (2005-08-25) * the whole document * ----- | 1-5,7, 9-47 | |
| X | WO 2004/022750 A (GARVAN INST MED RES [AU]; WATTS COLIN [AU]; SAUNDERS DARREN [AU]; HEND) 18 March 2004 (2004-03-18) u133a gen chip ----- | 34-47 | TECHNICAL FIELDS SEARCHED (IPC) |

6

EPO FORM 1503 03.82 (P04C10)

European Patent Office

INCOMPLETE SEARCH SHEET C

Application Number
EP 06 25 4064

Claim(s) not searched:
6

Reason for the limitation of the search (non-patentable invention(s)):

Article 52 (4) EPC - Method for treatment of the human or animal body by therapy

## ANNEX TO THE EUROPEAN SEARCH REPORT ON EUROPEAN PATENT APPLICATION NO. EP 06 25 4064

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2007

| Patent document cited in search report | | Publication date | Patent family member(s) | | | Publication date |
|---|---|---|---|---|---|---|
| US 2003073137 | A1 | 17-04-2003 | US | 2006228763 | A1 | 12-10-2006 |
| US 6207375 | B1 | 27-03-2001 | NONE | | | |
| WO 9950666 | A | 07-10-1999 | AU | 3035699 | A | 18-10-1999 |
| | | | EP | 1064554 | A1 | 03-01-2001 |
| WO 0022130 | A2 | 20-04-2000 | AU | 1316200 | A | 01-05-2000 |
| | | | EP | 1121437 | A2 | 08-08-2001 |
| | | | JP | 2002527066 | T | 27-08-2002 |
| | | | US | 6468790 | B1 | 22-10-2002 |
| | | | US | 2002009739 | A1 | 24-01-2002 |
| WO 2006061430 | A2 | 15-06-2006 | NONE | | | |
| WO 2005078136 | A | 25-08-2005 | AU | 2005212401 | A1 | 25-08-2005 |
| | | | CA | 2550429 | A1 | 25-08-2005 |
| | | | EP | 1723258 | A1 | 22-11-2006 |
| WO 2004022750 | A | 18-03-2004 | CA | 2497873 | A1 | 18-03-2004 |
| | | | EP | 1539957 | A1 | 15-06-2005 |
| | | | JP | 2005537790 | T | 15-12-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO CTS2005005711 A, Wang
- US 6136182 A
- US 6271002 B
- US 6218122 B
- US 6218114 B
- US 6004755 A
- US 20020019704 A, Tusher
- US 20030194734 A


### Non-patent literature cited in the description


- **TUSHER ; TIBSHIRANI ; CHU.** Significance Analysis of Microarrays Applied to the Ionizing Radiation Response. *PNAS,* 24 April 2001, vol. 98 (9), 5116-5121
- **NARASHIMAN ; CHU.** Diagnosis of Multiple Cancer Types by Shrunken Centroids of Gene Expression. *PNAS 2002,* 14 May 2002, vol. 99, 6567-6572
- **AHR et al.** Identification of high risk breast-cancer patients by gene-expression profiling. *Lancet,* 2002, vol. 359, 131-132
- **CHANG et al.** Gene expression profiling for the prediction of therapeutic response to docetaxel in patients with breast cancer. *Lancet,* 2003, vol. 362, 362-9
- Effects of radiotherapy and surgery in early breast cancer. An overview of the randomized trials. *N Engl J Med,* 1995, vol. 333, 1444-1455
- Polychemotherapy for early breast cancer: an overview of the randomized trials. *Lancet,* 1998, vol. 352, 930-942
- Tamoxifen for early breast cancer: an overview of randomized trials. *Lancet,* 1998, vol. 351, 1451-1467
- **EFRON.** Censored data and the bootstrap. *J Am Stat Assoc,* 1981, vol. 76, 312-319
- **EIFEL et al.** National Institutes of Health Consensus Development Conference Statement: adjuvant therapy for breast cancer. *J Natl Cancer Inst,* 01 November 2000, vol. 93, 979-989
- **FOEKENS et al.** Prognostic value of estrogen and progesterone receptors measured by enzyme immunoassays in human breast tumor cytosols. *Cancer Res,* 1989, vol. 49, 5823-5828
- **FOEKENS et al.** Prognostic value of receptors for insulin-like growth factor 1, somatostatin, and epidermal growth factor in human breast cancer. *Cancer Res,* 1989, vol. 49, 7002-7009
- **GOLDHIRSCH et al.** Meeting highlights: Updated International Expert Consensus on the Primary Therapy of Early Breast Cancer. *J Clin Oncol,* 2003, vol. 21, 3357-3365
- **GOLUB et al.** Molecular classification of cancer: class discovery and class prediction by gene expression monitoring. *Science,* 1999, vol. 286, 531-537
- **GRUVBERGER et al.** Estrogen receptor status in breast cancer is associated with remarkably distinct gene expression patterns. *Cancer Res,* 2001, vol. 61, 5979-5984
- **HEDENFALK et al.** Gene-expression profiles in hereditary breast cancer. *N Engl J Med,* 2001, vol. 344, 539-548
- **HERRERA-GAYOL et al.** Adhesion proteins in the biology of breast cancer: contribution of CD44. *Exp Mol Pathol,* 1999, vol. 66, 149-156
- **HUANG et al.** Gene expression predictors of breast cancer outcomes. *Lancet,* 2003, vol. 361, 1590-1596
- **KAPLAN et al.** Non-parametric estimation of incomplete observations. *J Am Stat Assoc,* 1958, vol. 53, 457-481
- **KEYOMARSI et al.** Cyclin E and survival in patients with breast cancer. *N Engl J Med,* 2002, vol. 347, 1566-1575
- **LIPSHUTZ et al.** High density synthetic oligonucleotide arrays. *Nat Genet,* 1999, vol. 21, 20-24
- **MA et al.** Gene expression profiles of human breast cancer progression. *Proc Natl Acad Sci USA,* 2003, vol. 100, 5974-5979
- **NTZANI et al.** Predictive ability of DNA microarrays for cancer outcomes and correlates: an empirical assessment. *Lancet,* 2003, vol. 362, 1439-1444
- **PEROU et al.** Molecular portraits of human breast tumors. *Nature,* 2000, vol. 406, 747-752
- **RAMASWAMY et al.** Multiclass cancer diagnosis using tumor gene expression signatures. *Proc Natl Acad Sci USA,* 2001, vol. 98, 15149-15154
- **RAMASWAMY et al.** A molecular signature of metastasis in primary solid tumors. *Nat Genet,* 2003, vol. 33, 1-6

- **RANSOHOFF.** Rules of evidence for cancer molecular-marker discovery and validation. *Nat Rev Cancer,* 2004, vol. 4, 309-314
- **SØRLIE et al.** Gene expression patterns of breast carcinomas distinguish tumor subclasses with clinical implications. *Proc Natl Acad Sci USA,* 2001, vol. 98, 10869-10874
- **SØRLIE et al.** Repeated observation of breast tumor subtypes in independent gene expression data sets. *Proc Natl Acad Sci USA,* 2003, vol. 100, 8418-8423
- **SOTIRIOU et al.** Gene expression profiles derived from fine needle aspiration correlate with response to systemic chemotherapy in breast cancer. *Breast Cancer Res,* 2003, vol. 4, R3
- **SOTIRIOU et al.** Breast cancer classification and prognosis based on gene expression profiles from a population-based study. *Proc Natl Acad Sci USA,* 2003, vol. 100, 10393-10398
- **SU et al.** Molecular classification of human carcinomas by use of gene expression signatures. *Cancer Res,* 2001, vol. 61, 7388-7393
- **VAN DE VIJVER et al.** A gene expression signature as a predictor of survival in breast cancer. *N Engl J Med,* 2002, vol. 347, 1999-2009
- **VAN'T VEER et al.** Gene expression profiling predicts clinical outcome of breast cancer. *Nature,* 2002, vol. 415, 530-536
- **WANG et al.** Gene expression profiles and molecular markers to predict relapse of Dukes' B colon cancer. *J Clin Oncol,* 2004, vol. 22, 1564-1571
- **WOELFLE et al.** Molecular signature associated with bone marrow micrometastasis in human breast cancer. *Cancer Res,* 2003, vol. 63, 5679-5684

SEQUENCE LISTING

<110> Wang, Yixin
Zhang, Yi
Atkins, David
Smid, Marcel
Klijn, Jan G.M.
Martens, John W.M.
Foekens, John A.
Sieuwerts, Anieta M.

<120> Predicting Bone Relapse of Breast Cancer

<130> P044554EP

<140> not yet assigned
<141> 2006-08-02

<150> 60/704740
<151> 2005-08-02

<160> 198

<170> PatentIn version 3.3

<210> 1
<211> 1044
<212> DNA
<213> human

<400> 1
gccaggcccc ctcctgaaag aggctccttg aagataattc taacatcttt gtcatcagtg 60

ttgacatctc ttgattgtat cctgtcattc catttgagat cttcctggtt ctggctggca 120

tcctctgaca tcactccagc agaagaaagg gaaggaacac gctgcattac tgcgtgggat 180

atcttctaac catactgatg attctccgta tagctggtct tttccacctt atcatccttc 240

ctctgagaag tatgaaaaca ctaagacatg tcgagggcca gatggagaac tccatgccaa 300

cctgctttgc cctgtggatg tgctggatgt tcccgagggc accttgcctg acaaacagag 360

cactgagcaa gccatacagt tgttggaaaa gatgaaaacg tcagccagtc ctttcttcct 420

ggccgttggg tatcataagc cacacatccc cttcagatac cccaaggaat ttcagaagtt 480

gtatcccttg gagaacatca ccctggcccc cgatcccgag gtccctgatg gcctaccccc 540

tgtggcctac aacccctgga tggacatcag gcaacgggaa gacgtccaag ccttaaacat 600

cagtgtgccg tatggtccaa ttcctgtgga ctttcagcgg aaaatccgcc agagctactt 660

tgcctctgtg tcatatttgg atacacaggt cggccgcctc ttgagtgctt tggacgatct 720

tcagctggcc aacagcacca tcattgcatt tacctcggat catgggtggg ctctaggtga 780

```
acatggagaa tgggccaaat acagcaattt tgatgttgct acccatgttc ccctgatatt     840

ctatgttcct ggaaggacgg cttcacttcc ggaggcaggc gagaagcttt tcccttacct     900

cgacccttttt gattccgcct cacagttgat ggagccaggc aggcaatcca tggaccttgt    960

ggaacttgtg tctctttttc ccacgctggc tggacttgca ggactgcagg ttccacctcg    1020

ctgccccgtt ccttcatttc acgt                                           1044


<210>  2
<211>  1152
<212>  DNA
<213>  human

<400>  2
tctttttggc ttgagcctga ggccttgtcg agaagcttcc gtgaaagggt gggccagccg      60

ggccacgaga aagaaagtga ataaatcagg aatataagtg ggcggggggc ccctgagagg     120

ggggtcgcaa agggtgagac atggccacca ggcgtttaac cgacgctttc ttgttgttgc     180

ggaataattc catccaaaac cggcagctgt tagccgagca agtgagtagt cacatcacct     240

ccagccctct gcattcacgt agcattgctg cggagctgga cgagcttgct gatgaccgta     300

tggcactggt gtcaggcatc agcttagatc cagaagcagc gattggtgtg acaaaacggc     360

cacctcctaa gtgggtggat ggagtggatg aaattcagta tgatgttggc cggattaagc     420

agaagatgaa agaatcggcc agccttcatg acaagcattt aaacagaccc accctggatg     480

acagcagcga agaggaacat gccattgaga taactaccca agagatcact cagctcttcc     540

acaggtgcca gcgtgccgtg cagccctgcc gagccgggcc cgggcctgct ccgagcagga     600

ggggcggctg cttgggaacg tggtgcctcg tggcgcaggc cctgcaggaa ctctccacca     660

gcttccggca cgcacaatca ggctacctca aacgcatgaa gaatcgagag gaaagatccc     720

agcatttttt cgacacatca gtaccactaa tggatgatgg agacgataac actctttacc     780

atcggggttt tacagaggac cagttagttc tggtggagca gaacacactg atggtggaag     840

agcgggaacg agagattcgc cagatggtac agtccatttc tgacctgaat gaaatattca     900

gggacttagg ggcgatgatt gtagaacagg gtacagtcct tgacagaatt gactataacg     960

ttgaacagtc ctgtatcaaa actgaagatg gtttgaaaca gcttcacaag gcagaacagt    1020

atcaaaagaa gaatcggaag atgcttgtga ttttaatatt atttgtcatc atcattgtgc    1080

tcattgttgt cctcgttggc gtgaagtctc gataagtggc attgggtttt cgtgtgtgcc    1140

gcgcgtgtgg at                                                        1152
```

<210> 3
<211> 7488
<212> DNA
<213> human

<400> 3
atgagtgtga aagaggcagg cagctcgggc cgccgggagc aggcggccta ccacctgcac 60

atctacccccc agctgtccac caccgagagc caggcctcgt gccgcgtgac tgccaccaag 120

gacagcacca cctcggacgt catcaaggac gccattgcca gcctgcggct ggacggcacc 180

aaatgttatg tgctggtgga ggtcaaagag tcgggaggcg aggaatgggt gctggacgcc 240

aacgactcgc ctgtgcaccg ggtgctgcta tggccccggc gggcacagga cgagcaccct 300

caggaggatg gctactactt cctgctgcag gagcgcaacg cagatggaac catcaagtac 360

gtgcatatgc agctggtggc gcaggccaca gccacccggc gcctagtgga gcgtggcctc 420

ctgccacggc agcaggcgga ctttgatgac ctgtgtaacc tccccgagct aaccgagggc 480

aacctcctga agaacctcaa gcaccgcttc ctgcaacaaa agatctacac gtacgcgggg 540

agcatcctgg tggccatcaa cccctttaag ttcctgccca tctacaaccc caagtacgtg 600

aagatgtatg agaaccagca gctgggcaag ctggagccac acgtcttcgc gctggccgac 660

gtggcctact acaccatgct caggaagcgc gtgaaccagt gcatcgtgat ctcgggtgag 720

agcggctccg gcaagaccca gagcaccaac ttcctcatcc actgcctcac cgccctcagc 780

cagaagggct acgccagcgg cgtcgagagg accatcctgg gtgctggccc tgtgctggag 840

gcttttggaa atgccaagac agcccacaac aacaactcca gccggtttgg gaaattcatc 900

caagtcagct acctagagag tggcatcgtg agaggagctg tcgtcgagaa atatctgctt 960

gaaaagtctc gcctggtgtc tcaggagaag gatgagagga actaccatgt gttttattat 1020

ttgttacttg ggtcagcga ggaagagcgc caagaatttc agctcaagca gcctgaagat 1080

tatttctacc tcaaccagca taacttgaag attgaagatg ggaggacct gaagcatgac 1140

tttgagaggc tcaagcaggc catggagatg gtgggcttcc tccccgccac caagaagcag 1200

atttttgccg tcctctcggc catcctgtac ctgggcaacg tcacttataa gaagagagct 1260

acaggccgag aggaagggtt ggaggtcggg ccacccgagg tgctggacac cctgtcgcag 1320

cttctgaagg tgaagcgaga aatcttggtg gaggttctga ccaaaagaaa aacggtgacc 1380

gtcaacgaca agcttatcct tccctacagc ctcagcgagg ccatcactgc ccgcgactcc 1440

atggccaagt ctctgtacag cgccctgttc gactggattg tgctgcggat caaccacgca 1500

ctcctcaaca agaaggacgt ggaagaggca gtctcgtgcc tgtccattgg ggtcctggac 1560
atcttcgggt ttgaagactt cgagaggaac agctttgagc agttctgcat caactacgcc 1620
aatgagcagc tgcagtatta cttcaaccag cacatcttca agctggagca ggaggaatat 1680
cagggcgagg ggatcacgtg gcacaacatc ggctacacag acaatgtcgg ctgcatccat 1740
ctcatcagca agaaacccac gggcctcttc tacctgctgg acgaggagag caacttcccc 1800
cacgccacga gccagaccct gctggccaag ttcaaacagc aacatgagga caataagtac 1860
ttcctgggca ccccggtcat ggagccagct ttcatcatcc agcacttcgc agggaaggtg 1920
aaatatcaga tcaaggactt ccgggagaag aacatggact acatgcggcc agacatcgtg 1980
gccctgctgc ggggcagtga cagctcctac gtgcgggagc tcatcggcat ggaccccgtg 2040
gccgtgttcc gctgggccgt gctccgggct gctatccggg ccatggcagt gcttcgggag 2100
gccggacgcc tgcgggccga gagggccgaa aaggctgcag gtatgagcag ccctggtgcc 2160
caaagtcacc cagaagagct gccaagagga gccagcaccc cttcggaaaa actttaccgc 2220
gatttgcata accaaatgat caagagcatc aaaggattgc cctggcaggg cgaggacccc 2280
cgtagccttc tccagtccct cagtcggctc cagaaacccc gcgccttcat cctgaaaagt 2340
aaaggtatca aacaaaagca gatcattcca aagaacctac tggactccaa gtccctgaaa 2400
ctcatcatca gcatgactct gcacgaccgc accaccaagt ccctactgca cctgcacaag 2460
aagaaaaagc caccaagcat cagcgcccag ttccagacat cccttaacaa gctcttggag 2520
gcactgggga aggcggagcc cttctttatc cgctgcatcc gttccaatgc tgaaaagaaa 2580
gagctgtgct ttgacgacga gctggtcctg cagcagctgc gctacaccgg catgctggag 2640
accgtgcgca tccggaggtc agggtacagc gccaagtaca cgttccagga tttcaccgag 2700
cagttccagg tgctcctgcc caaggatgcc cagccctgca gggaggtcat ctccaccctc 2760
ctggagaaaa tgaagataga caagaggaac taccagatcg ggaagaccaa ggtcttcctg 2820
aaggagacgg agcggcaagc cctgcaggag acgctgcacc gggaggtggt gcggaaaatc 2880
ctgctgctgc agagctggtt ccggatggtg ctggagcgtc ggcacttcct gcagatgaag 2940
cgggccgccg tcaccatcca ggcctgctgg cggtcctacc gggtccggag ggcgctggag 3000
aggacgcagg ctgccgtgta cctccaggcc tcatggaggg gctactggca gcggaagctc 3060
taccggcacc agaaacagag catcatccgc ctgcagagcc tgtgtcgggg gcacctgcag 3120
cgcaagagct tcagccagat gatctcggag aagcagaagg cagaagagaa ggagagggaa 3180
gccctggaag ccgcaagagc aggtgctgag gagggcggac agggtcaggc ggctggaggg 3240

cagcaggtag ctgagcaggg gccggagcca gcggaggatg gcgggcacct ggcatcggag 3300
cctgaggtgc agccaagtga caggtccccc ctagagcact cctcacctga gaaggaggcc 3360
ccaagcccag agaagactct cccaccccag aaaaccgtgg cggctgaaag tcacgagaaa 3420
gtccccagca gccgggagaa gcgtgagtcg cgtcggcaaa gagggctgga gcacgtcaag 3480
ttccagaaca aacacatcca gtcctgcaag gaggagagtg ccctcagaga accttccaga 3540
agggtcaccc aggagcaagg ggtgagtctc ctggaagaca aaaaggagag cagagaagat 3600
gaaacccttc tagtcgtaga gacggaggct gagaacacat ctcaaaagca gcccacagag 3660
caaccccagg ccatggcagt tggcaaggtc tctgaagaaa ctgagaagac gctgcccagt 3720
gggagcccca ggcctggcca gttggagcgg ccgaccagcc tggccctgga cagcagggtc 3780
agcccaccgg cccctggcag cgcccccgag acccccgagg acaagagcaa accatgtggc 3840
agcccaaggg ttcaggaaaa gcccgacagc cccggaggct ccacgcagat ccagcggtac 3900
ctggacgccg agcggctggc cagcgccgtg gaactgtggc ggggcaagaa gctggtggcc 3960
gccgccagcc ctagtgccat gctcagccag tccctggacc tcagcgacag acaccgggcc 4020
acaggggccg ccctcacgcc cacagaggag aggcgcacct ccttctccac gagcgacgtc 4080
tccaagctcc tcccgtccct ggccaaggct cagcctgcag cagaaaccac ggacggagag 4140
cgaagtgcga aaaagccagc tgtccagaag aagaagccag gcgacgcatc ctccctccca 4200
gacgcagggc tgtccccggg ctctcaggtc gactctaaat ccacgtttaa gaggcttttt 4260
ctgcataaaa ccaaggataa aaaatacagc ctggagggcg cagaggagct ggagaatgca 4320
gtgtccgggc acgtggtgct ggaagccacc accatgaaga agggcctgga agcccccctcc 4380
ggacagcagc atcgccacgc tgcaggtgag aagcgcacca aggaaccagg aggcaaaggg 4440
aagaagaacc gaaatgtcaa gattgggaag atcacagtgt cagagaagtg gcgggaatcg 4500
gtgttccgcc agatcaccaa cgccaatgag ctcaagtacc tggacgagtt cctgctcaac 4560
aagataaatg acctccgttc ccagaagacg cccattgaga gcttgtttat cgaagccacc 4620
gagaagttca ggagcaacat caaaacgatg tactctgtcc cgaacgggaa gatccacgtg 4680
ggctacaagg atctgatgga gaactaccag atcgtcgtca gcaacctggc cactgagcgt 4740
ggccagaagg acaccaacct ggtcctcaac ctcttccagt cactgctaga tgagttcacc 4800
cgtggctaca ccaagaacga cttcgagcca gtgaagcaga gcaaagctca gaagaagaag 4860
cggaagcagg agcgtgctgt ccaggagcac aacgggcacg tgttcgccag ctaccaggtt 4920

agcatcccgc agtcgtgcga gcagtgcctc tcctatatct ggctcatgga caaggccctg 4980
ctctgcagcg tgtgcaagat gacctgccac aagaagtgcg tgcacaagat tcagagccac 5040
tgctcctaca cctacgggag gaagggcgag ccaggcgttg agcctggcca cttcggcgtg 5100
tgcgtagaca gcctgaccag cgacaaggcc tcggtgccca tcgtgctgga gaagctcctg 5160
gaacacgtgg agatgcacgg cctgtacacc gagggcctct accgcaagtc gggtgctgcc 5220
aaccgcactc gggagctccg gcaggcgctg cagacagacc ccgcagcagt caagctggag 5280
aacttcccca tccacgccat cacaggggtg ctgaagcagt ggctgcggga gctgcccgag 5340
cccctcatga ccttcgcaca gtacggcgac ttcctccgag ccgtcgagct gccggagaag 5400
caggagcagc tggctgccat ctatgccgtc ctggagcacc ttccagaagc caaccacaac 5460
tccctggaga gactcatctt ccaccttgtc aaggtggccc tgctcgagga tgtcaaccgc 5520
atgtcacctg gggcgctggc cattatcttc gcaccctgcc tcctgcgctg ccctgacaac 5580
tcggacccgc tgaccagcat gaaggacgtc ctcaagatca ccacgtgcgt ggagatgctg 5640
atcaaggagc agatgaggaa atacaaagtg aagatggagg agatcagcca actggaggct 5700
gcagagagta tcgccttccg caggctttcg ctcctgcgac aaaatgctcc atggcctctc 5760
aaactggggt tttcgtctcc ctatgagggg gtcctgaaca agagccccaa gacccgggac 5820
atccaggagg aggagctgga ggtgctgctg gaggaggagg cagccggcgg cgatgaggac 5880
cgggaaaagg agattctcat tgaacggatc cagtccatca aggaggagaa ggaggacatc 5940
acctaccggc tgccggagct ggacccaagg ggctcggacg aggagaacct ggactcggag 6000
acgtcggcca gcaccgagag cctgctggag gagcgggccg ggcgggggc ctcggaaggg 6060
ccccctgcgc ctgctctccc ttgccccggc gcgcccaccc cgagccccct ccccaccgtg 6120
gccgcccctc cacgacgaag gccgtcgtcc ttcgtaacgg tcagagtgaa gacccccegg 6180
cggaccccca tcatgcccac ggccaacatc aagctcccac caggcctgcc ctcccacctg 6240
cctcgctggg caccgggtgc ccgggaggcg gctgccccag tgcggcgccg ggagccacct 6300
gcccgccgcc cggaccagat acattccgtg tacatcacgc ccggggcaga cctgccagtg 6360
cagggcgccc tggagcccct agaagaggat ggccagccac ctggggccaa gcggaggtac 6420
tcggatcccc caacgtactg cctgcccccc gcctcgggcc agaccaatgg ctgagagcca 6480
cagctgacaa agtctgcatg tccgaggacg gcccctgcac tggagctggg cgccagagct 6540
gcagagctag tgttcggccc tcagagaagg atccagaatc aaaagctcaa gagtgacgtg 6600
aggtgggcac cggccccaag tgcagagtca aggcagggag aggccggctg gagccaggcc 6660

```
ccctcgcacg cagcccccaa atcatggacg cacctgtggg gagcaccaca tctccacctg   6720
cggcctcaca tctccccact cccctttttg tacgtttaac tgtttctttg tacgtggttt   6780
acgtaacttt aaactgtaac agccttaatg gaagaccaaa tggtttttta tatgtgtatg   6840
tacaaagttt tctattaacg ctgcccgtct cccttataac ctggacgtga gctgtcagag   6900
cagaagccac taggccactg cgcgtctgag gctcagaccc tgctgtggtt ggcttggggt   6960
ggccaatggg ctgggaccct ccatgagagt tttggacact tgggtcacct gacccgtgcc   7020
tctctgacac atgtctccgg ggggcagcca cctggccaat gtgcattttt gcacatgctg   7080
gaaccttcca tgggggtctg ggctattggc tggagccagg acatgagtca ggggcaccat   7140
ggacctcacg tgccagggag acttgaatgt ggctgtcact cttccggacg ccaagggctg   7200
caggaggctg cttttggcac tacccacccc gtgtgacaga ataggagcca gcgactcagg   7260
actgctcacg ggtcaggagg gcaacgcctg aagtcagacc tccctatagg tcaacaggga   7320
caacctgggg atctctggag cagggccctc ctctctcagg cttggcccac tcccccagac   7380
acctggacac gtggccacaa atctgggaca aggggccccc gcacagcatg aaataaaaag   7440
tgcctgagaa gtgtgtgcaa aaaaaaaaaa aaaaaaaaaa aaaaaaa                 7488
```

<210> 4
<211> 2172
<212> DNA
<213> human

<400> 4

```
acagccgctc cctcgctctg ctggggcctc cggacgcgct tcccacgcgg gtctctggaa     60
cactcggtcc gaacgcacgc ctgcttgcac tcacactgcg gttcacaccc gcaggcgctc    120
tcgcacccac actgccgctc acgcgcgctc acactccccc acgcgcgctc cgctccggct    180
ccagccccgc gccccgcgaa ggcgcaggca ctgctgccga gagcgccgag gggccccgcg    240
gccttcccat ggcggacctg agcttcatcg aagataccgt cgccttcccc gagaaggaag    300
aggatgagga ggaagaagag gagggtgtgg agtggggcta cgaggaaggt gttgagtggg    360
gtctggtgtt tcctgatgct aatggggaat accagtctcc tattaaccta aactcaagag    420
aggctaggta tgacccctcg ctgttggatg tccgcctctc cccaaattat gtggtgtgcc    480
gagactgtga agtcaccaat gatggacata ccattcaggt tatcctgaag tcaaaatcag    540
ttctttcggg aggaccattg cctcaagggc atgagtttga actgtacgaa gtgagatttc    600
actggggaag agaaaaccag cgtggttctg agcacacggt taatttcaaa gcttttccca    660
```

tggagctcca tctgatccac tggaactcca ctctgtttgg cagcattgat gaggctgtgg 720
ggaagccgca cggaatcgcc atcattgctc tgtttgttca gataggaaag gaacatgttg 780
gcttgaaggc tgtgactgaa atcctccaag atattcagta taaggggaag tccaaaacaa 840
taccttgctt taatcctaac actttattac cagaccctct gctgcgggat tactgggtgt 900
atgaaggctc tctcaccatc ccaccttgca gtgaaggtgt cacctggata ttattccgat 960
accctttaac tatatcccag ctacagatag aagaatttcg aaggctgagg acacatgtta 1020
agggggcaga acttgtggaa ggctgtgatg ggattttggg agacaacttt cggcccactc 1080
agcctcttag tgacagagtc attagagctg catttcagta gccaaagagg acaggaacaa 1140
gtctgtcttc atgagggagg aagacaatgg tcctataatg cccttggata agaaaaggaa 1200
acttttgagc tgcaccttca gtttatcctc aaagcctgcg ttgtttgtct tcatctaatc 1260
cagctttgat ggacatctgt gatggttgcc tgtacacttg ctgaaatgaa atattagaaa 1320
tggctgtata ttccaaagaa accctatatt atatatccac attactgctg ctaggattca 1380
tagttgcaca tactgtttat tgcttatgtg tagaaggaat gaaactagtt tccagagttg 1440
ttattaatat gaatatatat catgtgttaa tattgagaaa ggaaaaatac attcccggtg 1500
ttagtagttc ttcatttcct gtctccaaca gaaaattcac tcattttaga actagtgtaa 1560
ttcttgataa taaaataaga gttttgatta agaacagcat agagcttcaa aatgcaaagt 1620
gaatgattag taaaattatg tctcatttta ttttttcagc acccatacca caattaatat 1680
taggctggat tgccatggga aacatttttt ggcattaatg cagcaacata atactcactt 1740
taggtattac tacatagttg aaggatttaa ctgaatgtat ggatcaaatt tatttatttg 1800
acatattcga agctgtggtt taataggaat ttgagaaagg tgtaagaaat aggataaaaa 1860
gaaggtcagc accatgtacc aggaatagct ttactttcca tacatagaaa tataaattta 1920
gtggtatcct atattacttt agtgtcgtac gctttgtaag acttaaatat tttattctat 1980
tgattccact actttggtat gttaagacat ttctttaaag atgaccaaca atatccttat 2040
tttaggtgcc actagcagat gtaagcgtat acttagttgc cgttagatgt gacagaatga 2100
gataatttat gtaaagcagt agagtacctg gcacaaagca aacaataaat attattgtta 2160
ttgttgttat aa 2172

<210> 5
<211> 3536
<212> DNA

<213> human

<400> 5
ccgcccgtcc cgccccgccc cgccgcccgc cgcccgccga gcccagcctc cttgccgtcg 60
gggcgtcccc aggccctggg tcggccgcgg agccgatgcg cgcccgctga gcgccccagc 120
tgagcgcccc cggcctgcca tgaccgcgct ccccggcccg ctctggctcc tgggcctggc 180
gctatgcgcg ctgggcgggg gcggccccgg cctgcgaccc ccgcccggct gtccccagcg 240
acgtctgggc gcgcgcgagc gccgggacgt gcagcgcgag atcctggcgg tgctcgggct 300
gcctgggcgg ccccggcccc gcgcgccacc cgccgcctcc cggctgcccg cgtccgcgcc 360
gctcttcatg ctggacctgt accacgccat ggccggcgac gacgacgagg acggcgcgcc 420
cgcggagcgg cgcctgggcc gcgccgacct ggtcatgagc ttcgttaaca tggtggagcg 480
agaccgtgcc ctgggccacc aggagcccca ttggaaggag ttccgctttg acctgaccca 540
gatcccggct ggggaggcgg tcacagctgc ggagttccgg atttacaagg tgcccagcat 600
ccacctgctc aacaggaccc tccacgtcag catgttccag gtggtccagg agcagtccaa 660
cagggagtct gacttgttct tttggatct tcagacgctc cgagctggag acgagggctg 720
gctggtgctg gatgtcacag cagccagtga ctgctggttg ctgaagcgtc acaaggacct 780
gggactccgc ctctatgtgg agactgagga cgggcacagc gtggatcctg gcctggccgg 840
cctgctgggt caacgggccc cacgctccca acagcctttc gtggtcactt tcttcagggc 900
cagtccgagt cccatccgca cccctcgggc agtgaggcca ctgaggagga ggcagccgaa 960
gaaaagcaac gagctgccgc aggccaaccg actcccaggg atctttgatg acgtccacgg 1020
ctcccacggc cggcaggtct gccgtcggca cgagctctac gtcagcttcc aggacctcgg 1080
ctggctggac tgggtcatcg ctccccaagg ctactcggcc tattactgtg aggggggagtg 1140
ctccttccca ctggactcct gcatgaatgc caccaaccac gccatcctgc agtccctggt 1200
gcacctgatg atgccagacg cagtccccaa ggcgtgctgt gcacccacca agctgagcgc 1260
cacctctgtg ctctactatg acagcagcaa caatgtcatc ctgcgcaagc accgcaacat 1320
ggtggtcaag gcctgcggct gccactgagt ccacccgccc ggcccagctg cagccaccct 1380
tctcatctgg atcgggcccc tcagaagcag gaaaccctca aacccagcca gaccccaggc 1440
cggggcattg ccagggagga ccctcacaac cacgtacatg accctttctc cttcatgcca 1500
ggctcctatg ctccccttgc cctgccaggc atttgtgtga ctgtcctgtt tccagcccag 1560
gtggtctcaa tcatcaggca gtgttctacc caaatgcaaa cgcctctccc ggaggcatgt 1620

cctggctggt tctttggggt tggcacagaa gtcctgtctg aggtcctatc catgcccctt 1680
actggctcag gtcgtgagat agatgtggaa tgacctgaga ggcacctgga gcccactgtt 1740
ggccaccttg agctcttcac catccatcac agggtgtggt gtgtgtagtc agggtctggt 1800
tggctcccca ttgcctgccc gaggtgcaag gtggggtata aaactggata acccctgaag 1860
tattgtatat tcatggatct gaagcactga tccactggtc acaggtagac atgtggagtc 1920
aactcaagaa aaagctgagt gaacagcatg atttagggct aaagccaatg gcatttatct 1980
tcccttgtct tcctgctttg catttgcctc tgccatctag gaaagacatg taagagcatg 2040
gacattttac tttggagaaa cagaaaaatc ttggggcttc caattgaccc atctatctgc 2100
caccatgttg ccccaccagg agctcagctc tgtggagttt tccctttgct gagcaagcat 2160
gtggttgcat tgggtggccc aggatgacaa tgcacagcac agatgccatc atttcccttt 2220
cccctctgaa tggcagacat cagtaatcaa tctggaatgt ttttcttcca aatctgagtg 2280
gaattttcaa atgatcagca cagccactgc caacagatat gatgtaaagt gaaacctggt 2340
tgccatcttc tgccatgctg aggagcagtc catccctgcc cgagcatgta tcggcaacat 2400
gggcagcctg tgaccgggtc tggggcgagg ccaggggcca tcaaaaacag gctgatcacc 2460
aaagtcagtg tcaccctgga tgcccagcag ccctgtcctg tgtcttgggc ctgtgagtca 2520
aagaaaaggt ccttttcagg gagtgacaag tagtaattag gctgagttgg gtggagaggt 2580
ttgtctcagc ctctgctgtt ctcggaaact gctgttctcc ttggagcagc cactgggagt 2640
tggagtgttt atttgatttc tgacttgcta agcctgtaat ttacctgctg gaatagacag 2700
agtccagctg cccaaaccgt gtcattaaaa gcagatcctg cgcccgcccc atccacaggc 2760
acagcccggc agagtggttc cacctcccca tgggcccaag gatgcgcctc tctggagttc 2820
acgtgctgca cccccaggga ggggcctggg gaaagctggt ccagcagcag gggtggaggc 2880
tggggccaca ctgcgggaca gcagcccctc cacctggacc agggagggcc tccatgtgca 2940
agcgcagagg aagagaccct cccatgtacg caaagggcag ccccaggctg tctggaagtt 3000
ggagaattcc ctatcagcac agggatctca gctctggcct ggaggtgaag agacctgcct 3060
tgtaggtggc ttccttatct gcgcctccat tttctatctg cactttttga tctccaaaca 3120
accttcagcc aaagaatctg tctaccaact cctcatagtg agccagaagc agcctcataa 3180
ccctgaatgt ggggctctgg tggctgtcac gaagcagagt tggcacataa catggaacct 3240
ggccaggcat ggtggctcac acctataacc ccagcacttt gggaggccaa ggcaggcaga 3300
tcacctgaag tcaggagttc aagaccatcc tggccaacac agtgaaaccc catctgtact 3360

aaaaatacaa gattacctgg gcatggtggt gcatgcctat aatcccagct actcaggagg 3420

ctgaggcaga attgcttgaa cctgggaggt ggaggttgca gtgagcagag atcacaacat 3480

tgcacttcag cctggtgaca tgagcaaaac tgttgtctca acaaaatgaa attatg 3536

<210> 6
<211> 3425
<212> DNA
<213> human

<400> 6
agcgcctgtg ctgccgcgtc tcgacgtgtc accggcggcg ccgctgctgt ggcaaaggaa 60

ggagctgact gggggagttc gaggcggcgg gcggcggtga ccccggcctg gaactgcccc 120

ggtacggaag tgttccgggg tccgtgggga gcaggagagg gaggcggcgg accgtcccgc 180

gcggggcacg atgttgaaca tgtggaaggt gcgcgagctg gtggacaaag ccaccaatgt 240

tgttatgaat tattcagaga tcgagtctaa ggttcgagag gcaacgaacg atgatccttg 300

gggaccttct gggcaactca tgggagagat tgccaaggct acatttatgt atgaacaatt 360

tccagaactt atgaacatgc tttggtcacg aatgttaaaa gacaacaaaa agaattggag 420

aagagtttat aagtcgttgc tgctcctagc ttacctcata aggaatggat cagagcgtgt 480

tgttacaagt gccagagaac acatttatga tttacgatcc ctggaaaatt accactttgt 540

agatgagcat ggtaaggatc aaggtataaa tattcgacag aaggtgaagg aattggttga 600

atttgcccag gatgacgaca ggcttcgtga agagcgaaag aaagcaaaga agaacaaaga 660

caagtatgtt ggggtttcct cagacagtgt tggaggattc agatacagtg aaagatatga 720

tcctgagccc aaatcaaaat gggatgagga gtgggataaa aacaagagtg cttttccatt 780

cagtgataaa ttaggtgagc tgagtgataa aattggaagc acaattgatg acaccatcag 840

caagttccgg aggaaagata gagaagactc tccagaaaga tgcagcgaca gcgatgagga 900

aaagaaagcg agaagaggca gatctcccaa aggtgaattc aaagatgaag aggagactgt 960

gacgacaaag catattcata tcacacaggc cacagagacc accacaacca gacacaagcg 1020

cacagcaaat ccttccaaaa ccattgatct tggagcagca gcacattaca caggggacaa 1080

agcaagtcca gatcagaatg cttcaaccca cacacctcag tcttcagtta agacttcagt 1140

gcctagcagc aagtcatctg gtgaccttgt tgatctgttt gatggcacca gccagtcaac 1200

aggaggatca gctgatttat tcggaggatt tgctgacttt ggctcagctg ctgcatcagg 1260

cagtttccct tcccaagtaa cagcaacaag tgggaatgga gactttggtg actggagtgc 1320

```
cttcaaccaa gccccatcag gccctgttgc ttccagtggc gagttctttg gcagtgcctc   1380
acagccagcg gtagaacttg ttagtggctc acaatcagct ctaggcccac ctcctgctgc   1440
ctcaaattct tcagacctgt ttgatcttat gggctcgtcc caggcaacca tgacatcttc   1500
ccagagtatg aatttctcta tgatgagcac taacactgtg ggacttggtt tgcctatgtc   1560
aagatcacag aatacagata tggtccagaa atcagtcagc aaaaccttgc cctctacttg   1620
gtctgacccc agtgtaaaca tcagcctaga caacttacta cctggtatgc agccttccaa   1680
accccagcag ccatcactga atacaatgat tcagcaacag aatatgcagc agcctatgaa   1740
tgtgatgact caaagttttg gagctgtgaa cctcagttct ccatcgaaca tgcttcctgt   1800
ccggccccaa actaatgctt tgataggggg acccatgcct atgagcatgc ccaatgtgat   1860
gactggcacc atgggaatgg cccctcttgg aaatactccg atgatgaacc agagcatgat   1920
gggcatgaac atgaacatag ggatgtccgc tgctgggatg ggcttgacag gcacaatggg   1980
aatgggcatg cccaacatag ccatgacttc tggaactgtg caacccaagc aagatgcctt   2040
tgcaaatttc gccaatttta gcaaataaga gattgtaaaa gaagcagatt gaatgaagaa   2100
tttttagctg tgcagatagg tgatgttggg atggaaaatg ctaatcaact accctttctt   2160
ttatcaagta attaaaataa atctacataa agaaccaaaa aggctgtttt ataaaagtga   2220
aatatccagt atttcagagg gccaggcaag agcacttcag atgaggcagt caaaatcatt   2280
tttttccagt gaggatagac cacaagtggg tggtgagacc attgaaagcc tttatcaact   2340
gaagagtcca tttaacagca taatttgtgg gaagactgga atagggctga ataaatgtgt   2400
ttgaatctct aattttatac tttcttttcc tgaggaactt gatttttctg tccctggatc   2460
gccttgtcat aattgggtct gttcctttta ctaccactct tgagtccata tatgaaatca   2520
ttaaagttgg atgatcagtt ttttataaaa atatatattt ttgtccaaga aaaaaaaaag   2580
catacatatg tgattatggc taaatcaaag gtaactggaa tgtatatact tttgctaatg   2640
ttccagcaac actgctatta tactatccaa atttttattg taacaaaacc tctttaagca   2700
attggtgatt gccatgggac ttttcccatg tcttctgctg taattatcct gtgcagaact   2760
aggaagaaat ttttttcagg actgctctat ggtttccttt aaaagaaaaa aacttctgtt   2820
tgtttttagc agtcattatt tacaatttgc agtgattaac ttggcaaggc ttccttccgt   2880
gtttatccct gtagccatca tttaagtcag gaacagtcag aaaaatattt attttatttt   2940
tttttgggt gtctgcaaag gtaaaaatcc attaaaacct taagttaaat ataaatgtta   3000
```

caactcaatg tttgctttta gattttatac agtatttgtt ttgttttggt tttgagtgta 3060

tataatgcag cattagcaat atggttccaa tagaggagtt aaatatatat tgttaaagga 3120

gacctgtagc agtcaaagat tttattgatt taatgacaaa ggaaattaat gaaaatgttt 3180

ttgtttttct gctgtaattc tgcattaagc tcacatgaaa atcatgattc tagagtttgg 3240

aatgcaaaat taattgtttt accctcaagc tgggaatatt tttcaaaata aatactataa 3300

tatagatatc aaattattac ctccccatgt tatgttgaaa atttttttat taaattgata 3360

aaactttatt tccattatat tcataatgtt ctgttataca taacattaaa atgttcatta 3420

aaatc 3425

<210> 7
<211> 1767
<212> DNA
<213> human

<400> 7
gcgcgccgct ttctgttgcc gggcgcaatg gcggatacgc tggagtcctc gctggaggac 60

ccactgcgga gctttgtgcg agttttggag aagcgggatg gtacagtgct acgactacag 120

cagtatagct ccggtggcgt gggttgcgtt gtgtgggacg ctgccattgt cctttctaaa 180

tacctggaaa cgcccgagtt ttctggcgac ggggcccacg cgctgagccg gcggtcggtg 240

ctggagctgg gttcgggcac cggggccgtg ggctcatgg ctgctaccct cggggctgat 300

gttgtagtca ccgatcttga ggaattgcaa gacttgctga agatgaatat taatatgaac 360

aagcatcttg tcactggttc tgttcaagcc aaggggggaa gaaatagaag gctttccttc 420

tccacccgac ttcatactga tggccgactg catatactat gaagagtctt tggagccatt 480

gctgaaaact ctaaaagata tcagcggatt tgaaacttgt attatatgtt gttatgaaca 540

acgaacaatg gggaaaaatt cagaaattga gaaaaaatat tttgagctcc ttcagctaga 600

ttttgacttt gaaaaaattc ctttggaaaa acgtgatgaa gagtatcgaa gtgaagatat 660

tcatattata tacatcagaa agaaaaaatc gaaatttcca tcgtgaagcc tttaatcatc 720

ttacccaagg ctctaacaac ctgggtaaac taaagatgtg aatagagcat gtgaatacag 780

catgggaaga ttgtgttcac agattttttt ttccggcacg tccttagaga tccgatgtat 840

agatgatgac caccaggggc tgtctgcaat acgaaaaatt cctgcttgcc tgcctgcctg 900

ccaatggccc tgaatccagc ttaggttact tagttcagca tcaagttctt cttaaatgtt 960

gggaatcagt tattcaaata aaaattggta ttgaggcgag gctgaaatct gccaaaaaca 1020

gccaacatga tttgactgga ccttttacag gaagctagag ataaatttct gaagagaact 1080

atctgctatt atataataat gttttaaatt caaactagta aattttagtt tgtcttcaga 1140

gtttaaaagg ttttcatttt gtacataatt atacaatatt tatcatttgt attttccact 1200

tcatttcttt taaaatatct ttaatactga aatgttcttt taattttaaa aagaactgag 1260

atattgtctt gtatacttat attggccaaa gttttctttt cctccaccat acatgtctat 1320

gtgatttaat cagtaaattg tactgtaatg agttgctaag aagacaaatc agaagattgg 1380

aggaacaaaa gatatcttta cagatttttc atttgagcat ggagtgagaa tagaaagacc 1440

agtttcaggc ttatgcactt acgtggctca tgcactttat gtatatgttc caggaaaatc 1500

tggcttaaaa atactggtat tgtttacatg aagcagtgaa aggtttttga ataactacaa 1560

atgtagttct atatgtatat accaaatgaa tttctgttct gtgtctctct gttttatgtt 1620

atgaagccat tcgctcatat acaataatct gtcaaggact ttaatcatac ttgttccaaa 1680

gagtagtaac cggatggaat tctggtattt acaggcattg gtgctagatg gtacatttta 1740

tgtgttaaaa taaacattgt ttttgag 1767

<210> 8
<211> 2008
<212> DNA
<213> human

<400> 8
cagcaactcc gtggccaagc tgaggaggcc cagggcccgc tcctgcttct ccacgggtgt 60

gctctgtggc ccccgatgga agggggaagg atccaagcag gccctccctg cccagggcag 120

aagattctgg agtcctgggc gttgcctgtg agtgttctga gagataaggg ccccgcccgc 180

cccgccccgg gaccaaggtc ctcgtcccgt cccttgctgc gccgggaact cctgccccac 240

gcaggcttct ggtctccaca ctgggcttgt tcctgggtga tgagattcgg ctccaccctc 300

acgggctgcc agcctcctgc ccgatgggtg agcctctgct gctgcttcag caaagggcgg 360

ccccacttcc cacactggca ggtggggaca gggagaccct gggcctcccc agagctcatg 420

ggttagcttc cacaccctca tgctgtcatg agcctttctc acagggtcag aagagtctgc 480

atctgacaca gagaccccag tccacatagt cagggctggg ccagagggag ggacttgggg 540

ctgatgggga ggaggctcgg cgtgagagtg aggtctttgc ccaaggaggg tatgaaagct 600

ggggctttag tgggtgggta ggagttggcc agcagagagc cgacggaaga gttaatttaa 660

gtttatggac ctgtaagtgc tccgagtccc agggtgtctg ggtgttagct gctgactggt 720

gtcctcatga gtcgggctgg gtgaggctgg gttaggaagg gttccacaag ccccccgggt 780
ccactgaggt ttgcccctga gatgatgaac cgaagctttt agagctgggc aggcgtgagg 840
gtgacccata actctctctg tcctgcctgc aggtcttgag gaccggccca gctcaggctc 900
ctggggcagc ggcgaccaga gcagctcctc ctttgacccc agccgggtag gagtccccgt 960
gaggcctgag accctgagtt tcctgtcccc agagttgggg gcgaggggtg tggggaaact 1020
gaggctgaga gaaggggcca gccttccagg ggtttggact gggggacatg ggtttgagtc 1080
ctgcctctcc agctgtaatg cggggtggag ggggggcctg tgaagcccca gcacctctca 1140
tccccacccc atctccccta gaccttcagc gagggcaccc acttcactga gtcgcacagc 1200
agcctctctt catccacatt cctgggaccg ggactcggag gtgagtgcct ggcctggtgc 1260
ggtccctctg ctgtgcacag atgtgcagac gtgggccttg gcatggtcag tgcacgcaca 1320
gcgccaggca agtccccagc gttcacccag agggtgtgga cagccgaagc ctctgcccgt 1380
cctgggagca gctgcgggga ggtgcagggg acgtggcccg gactccctgg cacggcactc 1440
tgcaactagg cttccccgcc tgagcgtaga ggcagggagg ccgttccaga ccccacttcc 1500
tccctgggat ttctgcaggg ctcctcctgc ttcccgggga ggggtcagcc gggctggcct 1560
gtgcccgctg ggaaggaggc gtaggagttc tggagactcg ggagggcggc cagagtgtgc 1620
ggtggtcaga gcccatgccc ttccgcagac cgcccgcccg ccctctttcc ctgggggccc 1680
ctcctgttaa ccgagagaat cgtgactcag gttgggctcg gtggctcacg cctgtaatac 1740
cagcactttg ggagagcgag gtgggtggat cacctgaggt caggagtttg agaccagcct 1800
ggccaacatg gtgaaacccc gcttctacca aaaacacaaa aattagccgg tcatggtgtt 1860
ggatgcttat agtgccaact actcaggagg ctgaggcagg agaattgctt gaacccagga 1920
ggtggaggct gcagtgagcc gagatcacgc cattgccctc cagcctgggt gatggagcga 1980
gattccatct taaaaaaaaa aaaaaaaa 2008

<210> 9
<211> 12533
<212> DNA
<213> human

<400> 9
tccaaactgt tcaaaatgat gaacgaagat gcagctcaga aaagcgacag tggagagaag 60
ttcaacggca gtagtcagag gagaaaaaga cccaagaagt ctgacagcaa tgcaagcttc 120
ctccgtgctg ccagagcagg caacctggac aaagttgtgg aatatctgaa gggggcata 180

gacatcaata cctgcaatca gaatggactc aacgctctcc atctggctgc caaggaaggc 240
cacgtggggc tggtgcagga gctgctggga agagggtcct ctgtggattc tgccactaag 300
aagggaaata ccgctcttca cattgcatct ttggctggac aagcagaagt tgtcaaagtt 360
cttgttaagg aaggagccaa tattaatgca cagtctcaga atggctttac tcctttatac 420
atggctgccc aagagaatca cattgatgtt gtaaaatatt tgctggaaaa tggagctaat 480
cagagcactg ctacagagga tggctttact cctctagctg tggcactcca gcaaggacac 540
aaccaggcgg tggccatcct cttggagaat gacaccaaag ggaaagtgag gctgccagct 600
ctgcatattg ccgctaggaa agacgacacc aaatctgccg cacttctgct tcagaatgac 660
cacaatgctg acgtacaatc caagatgatg gtgaatagga caactgagag tggttttacc 720
cctttgcaca tagctgcaca ttacggaaat gtcaacgtgg caactcttct tctaaaccgg 780
ggagctgctg tggacttcac agccaggaat ggaatcactc ctctgcatgt ggcttccaaa 840
agaggaaata caaacatggt gaagctctta ctggatcgag gcggtcagat cgatgccaaa 900
actagggatg ggttgacacc acttcactgt gctgcacgaa gtgggcatga ccaagtggtg 960
gaacttctgt tggaacgggg tgcccccttg ctggcaagga ctaagaatgg gctgtctcca 1020
ctacacatgg ctgcccaggg agaccacgtg gaatgtgtga agcacctgtt acagcacaag 1080
gcacctgttg atgatgtcac cctagactac ctgacagccc tccacgttgc tgcgcactgt 1140
ggccactacc gtgtaaccaa actcctttta gacaagagag ccaatccgaa cgccagagcc 1200
ctgaatggtt ttactccact gcacattgcc tgcaagaaaa accgcatcaa agtcatggaa 1260
ctgctggtga aatatggggc ttcaatccaa gctataacag agtctggcct cacaccaata 1320
catgtggctg ccttcatggg ccacttgaac attgtcctcc ttctgctgca gaacggagcc 1380
tctccagatg tcactaacat tcgtggtgag acggcactac acatggcagc ccgagccggg 1440
caggtggaag tggtccgatg cctcctgaga aatggtgccc ttgttgatgc cagagccagg 1500
gaggaacaga cacctttaca tattgcctcc cgcctgggta agacagaaat tgtccagctg 1560
cttctacaac atatggctca tccagatgcg gccactacaa atgggtacac accactgcac 1620
atctctgccc gggagggcca ggtggatgtg gcatcagtcc tattggaagc aggagcagcc 1680
cactccttag ctaccaagaa gggttttact cccctgcatg tagcagccaa gtatggaagc 1740
ctggatgtgg caaaacttct cttgcaacgc cgtgctgccg cagattctgc agggaagaac 1800
ggccttaccc cgctccatgt tgctgctcat tatgacaacc agaaggtggc gctgctgtta 1860
ctggagaagg gtgcttcccc tcatgccact gccaagaatg gctatactcc gttacatatt 1920

```
gctgccaaga agaatcaaat gcagatagct tccacactcc tgaactatgg agcagagaca   1980
aacattgtga caaagcaagg agtaactcca ctccatctgg cctcgcagga ggggcacaca   2040
gatatggtta ccttgcttct ggataaggga gccaatatcc acatgtcaac taagagtgga   2100
ctcacatcct tacaccttgc agcccaggaa gataaagtga atgttgctga tattctcacc   2160
aagcatggag ctgatcagga tgctcataca aagcttggtt acacaccttt aattgtggcc   2220
tgtcactatg gaaatgtgaa aatggtcaac tttcttctga agcagggagc aaatgttaac   2280
gcaaaaacca agaacggcta cacgcctttg caccaggccg ctcagcaggg tcacacgcac   2340
atcatcaacg tcctgctcca gcatggggcc aagcccaacg ccaccactgc gaatggcaac   2400
actgccttgg cgattgctaa gcgtctgggc tacatctccg tggtcgacac cctgaaggtt   2460
gtgactgagg aggtcaccac caccaccaca actattacag aaaaacacaa actaaatgta   2520
cctgagacga tgactgaggt tcttgatgtt tctgatgaag agggtgatga cacaatgact   2580
ggtgatgggg gagaatacct taggcctgag gacctaaaag aactgggtga tgactcacta   2640
cccagcagtc agttcctgga tggtatgaat tacctgcgat acagcttgga gggaggacga   2700
tctgacagcc ttcgatcctt cagttccgac aggtctcaca ctctgagcca tgcctcctac   2760
ctgagggaca gtgccgtgat ggatgactca gttgtgattc ccagtcacca ggtgtcaact   2820
ctagccaagg aggcagaaag gaattcttat cgcctaagct ggggcactga gaacttagac   2880
aacgtggctc tttcttctag tcctattcat tcaggtttcc tggttagttt tatggtggat   2940
gcccgaggtg gtgctatgcg aggatgcaga cacaatgggc tccgaatcat tattccacct   3000
cggaaatgta ctgctccaac gcgagtcacc tgccgactgg tcaagcgcca cagactggca   3060
acaatgcctc caatggtgga aggagaaggc ctggccagtc gcctgatcga agttggacct   3120
tctggtgctc agttccttgg taaacttcac ctgccaacgg ctcctccccc acttaatgag   3180
ggagaaagtt tggtcagccg cattcttcag ctggggcctc ctggaaccaa attccttggg   3240
cctgtgatcg tggagatccc tcactttgcg gcccttcgag gaaaggaaag ggaactggtg   3300
gtcctgcgca gtgagaatgg ggacagctgg aaagagcatt tctgtgacta cactgaagat   3360
gaattgaatg aaattcttaa cggcatggat gaagtactgg atagcccaga agacctagaa   3420
aagaaacgaa tctgccgcat catcacccga gacttcccac agtactttgc agtggtgtct   3480
cgtatcaaac aggacagcaa tctgattggc ccagaaggag gtgtactgag cagcacagtg   3540
gtgccccagg tgcaggccgt cttcccagag gggcactca ccaagcggat ccgtgtaggc   3600
```

ctgcaggctc aacctatgca cagtgagctg gttaagaaga tcctaggcaa caaagctacc 3660
ttcagcccta tagtcacttt ggaacctaga agaagaaaat tccacaaacc aattaccatg 3720
accattcctg tccccaaagc ttcaagtgat gtcatgttga atggttttgg gggagatgca 3780
ccaaccttaa gattactatg cagcataaca ggtggaacca cccctgccca gtgggaagat 3840
attacaggaa ctacgccatt aacatttgtc aatgaatgtg tttcctttac aacaaacgtg 3900
tctgccaggt tctggctgat agattgtcga cagatccagg aatccgttac ttttgcatca 3960
caagtataca gagaaattat ctgcgtacct tatatggcca aatttgtagt gtttgccaaa 4020
tcacatgacc ccattgaagc caggttgagg tgtttctgca tgactgatga taaagtggat 4080
aagacccttg aacaacaaga aaattttgct gaggtggcca gaagcaggga tgtggaggtg 4140
ttagaaggaa aacccatcta cgttgattgt ttcggcaact tggtaccatt aactaaaagt 4200
ggccagcatc atatattcag ttttttttgcc ttcaaagaaa atagacttcc tctatttgtc 4260
aaggtacgcg atacgactca ggaaccttgc ggacgactat catttatgaa ggagccaaaa 4320
tccacgagag gcctggtgca tcaagctatt tgcaacttaa acatcacttt gccgatttat 4380
acaaaggaat cagagtcaga tcaagaacag gaggaagaga tcgatatgac atcagaaaaa 4440
aatgatgaga cagaatctac agaaacatct gtcctgaaaa gtcacctggt taatgaagtt 4500
cctgtcctag caagtccgga cttgctctct gaagtttctg agatgaaaca agatttgatc 4560
aaaatgaccg ccatcttgac cacagatgtg tctgataagg caggttctat taaagtgaag 4620
gagctggtga aggctgctga ggaagagcca ggagagcctt ttgaaatcgt tgaaagagtt 4680
aaagaggact tagagaaagt gaatgaaatc ctgagaagtg gaacctgcac aagagatgaa 4740
agcagtgtgc agagctctcg gtctgagaga ggattagttg aagaggaatg ggttattgtc 4800
agtgatgagg aaatagaaga ggctaggcaa aaagcacctt tagaaatcac tgaatatcca 4860
tgtgtagaag ttagaataga taaagagatc aaaggaaaag tagagaaaga ctcaactggg 4920
ctagtgaact accttactga tgatctgaat acctgtgtgc ctcttcccaa agagcagctg 4980
cagacagttc aagataaggc agggaagaaa tgtgaggctc tggctgttgg caggagctct 5040
gaaaaggaag ggaaagacat acccccagat gagacacaga gtacacagaa acagcacaaa 5100
ccaagcttgg gaataaagaa gccagtaaga aggaaattaa aagaaaagca gaaacaaaaa 5160
gaggaaggtt tacaagctag tgcagagaaa gctgaactta aaaaaggtag ttcagaagag 5220
tcattaggtg aagacccagg tttagcccct gaacccccttc ccactgtcaa ggccacatct 5280
cctttgatag aagaaactcc cattggttcc ataaaggaca aagtaaaggc ccttcagaag 5340

```
cgagtggaag atgaacagaa aggtcgaagc aagttgccca tcagagtcaa aggcaaggag   5400
gacgtgccaa aaaagaccac ccacaggcca catccagctg cgtcaccctc tctgaagtca   5460
gagagacatg cgccagggtc tccctcccct aaaacagaaa gacactctac tctttcctct   5520
tccgcaaaaa ctgaaaggca ccctccagta tcaccatcaa gtaaaactga gaaacactca   5580
cctgtgtcac cctctgcaaa aacggaaaga cattcacctg cgtcatcatc gagtaaaact   5640
gagaaacact cacctgtatc accctcgaca aaaactgaaa ggcactctcc tgtgtcatct   5700
acaaaaacag aaagacaccc acctgtttcg ccttcaggca aaacagacaa acgtccacct   5760
gtatcgccct ccgggaggac agaaaaacac ccgccagtat cgcctgggag aacagaaaaa   5820
cgcttgcctg tttcaccctc cggaagaacg gacaagcacc aacctgtatc aacagctggg   5880
aaaactgaga agcacctgcc tgtgtcacct tctggcaaaa cagaaaagca accacctgta   5940
tcccccactt caaaaacaga gaggattgag gaaaccatgt ctgttcggga gctgatgaag   6000
gctttccagt caggtcagga cccttctaaa cataaaactg gactctttga gcacaaatca   6060
gcaaaacaaa agcagccaca agagaaaggt aaagttcggg tagaaaaaga aaaggggccg   6120
atactaaccc agagagaagc tcagaaaaca gagaatcaga caatcaaacg aggccagaga   6180
ctcccggtaa cgggcacagc agaatccaaa agaggagttc gtgtttcctc cataggagtt   6240
aagaaagaag atgcagctgg aggaaaggag aaagttctca gccacaaaat acctgaacct   6300
gttcagtcag tgcctgaaga agaaagccac agagagagcg aagtgcccaa agaaaagatg   6360
gctgatgagc agggagacat ggatctacag atcagcccag ataggaaaac ctccactgac   6420
ttctctgagg tcattaagca agagttggaa gacaatgaca aataccaaca attccgcctg   6480
agtgaggaga cagaaaaggc acagcttcac ttagaccaag tactcactag tcctttcaac   6540
acaacatttc cactcgacta catgaaagat gagttccttc cagctctgtc tttacaaagc   6600
ggtgctttag atggcagttc tgaaagccta aagaatgagg gggtagccgg ctctccgtgt   6660
ggcagcctga tggaggggac ccctcagatt agttcagaag aaagctataa gcatgaaggc   6720
ctagcagaga cccctgagac gagcccagaa agcctttctt tctcaccaaa gaaaagtgag   6780
gagcaaactg ggaaacaaa ggaaagcacc aagacagaaa ccaccacaga aattcgttca   6840
gaaaaagagc atcccacgac caaagacatt actggtggct ctgaagagcg aggtgccaca   6900
gtcactgagg actcagagac ctctactgag agttttcaga aagaggccac tctaggctct   6960
cccaaagaca caagccctaa aagacaagat gattgcacag gcagctgtag tgtagcatta   7020
```

gctaaagaga cacctacagg actgactgag gaggcagcct gtgatgaagg tcaacgtacc 7080
tttggtagtt cagcccacaa gacacaaact gatagtgagg ctcaggaatc cacagccacc 7140
tcagacgaga caaaggcctt gccgctgcct gaggcttctg taaagacaga tacaggaact 7200
gaatcaaaac ctcagggagt cattagaagt ccccaagggt tagaacttgc actccctagc 7260
cgagatagcg aagtcctcag cgctgtggct gatgactcat tagcagtgag ccacaaagac 7320
tctctggaag ccagccctgt gctagaagat aactcttcac acaaaacccc tgattctctg 7380
gagccaagtc ctctgaaaga atccccttgc cgtgactctc tggaaagcag ccctgttgaa 7440
ccaaagatga aggctggaat tttccaagt cactttcctc ttcctgcagc tgttgccaaa 7500
acagaactct tgacggaagt ggcctctgtg cggtcccggc tactccgaga ccctgatggc 7560
agtgctgagg atgacagtct tgagcagaca tcgctcatgg agagctcagg gaagagcccc 7620
ctttctcctg acacccccag ctctgaagaa gtcagctatg aggttacacc caaaaccaca 7680
gatgtaagta caccaaaacc agctgtgatt catgaatgtg cagaggagga tgattcagaa 7740
aacggggaga aaaagaggtt cacacctgaa gaggagatgt ttaaaatggt aaccaaaatc 7800
aaaatgtttg atgaacttga acaagaagca aagcagaaaa gggactacaa aaaagaaccc 7860
aaacaagaag aatcttcttc atcttctgac ccagatgctg actgttcagt agatgtggat 7920
gaaccaaaac atacaggcag tggggaggat gaaagtggtg tccctgtgtt agtaacttcg 7980
gagagcagga aggtgtcttc ctcctcagaa agtgaacctg agttggcaca gcttaaaaaa 8040
ggtgctgact caggcctttt accagaacca gtgattcgag tacaacctcc ttctccactt 8100
ccatcaagca tggactccaa ttccagtcca gaagaagtac aattccagcc tgtcgtttcc 8160
aaacaatata ctttcaagat gaatgaagat actcaggaag agccaggcaa atcagaagaa 8220
gaaaaagatt ctgaatccca tttagctgaa gaccgtcatg ctgtttccac tgaggctgaa 8280
gacaggtctt atgataagct aaacagagac actgatcagc caaaaatctg tgatggccat 8340
ggatgtgagg ccatgagtcc tagcagctca gctcgtcctg tctcttcagg tctacagagt 8400
ccgactggtg atgatgttga tgaacagcca gtcatctata aagaatcatt agctctccaa 8460
ggcactcatg aaaaagacac agagggagaa gagcttgatg tttctagagc agaatctcca 8520
caagcagatt gccccagtga aagcttttca tcttcatcct ctttgcctca ttgtttggta 8580
tctgaaggaa aagaattaga tgaagacata tctgccacat cttctattca aaaaacagag 8640
gtcacaaaaa ctgatgaaac atttgagaac ttaccaaagg actgcccctc tcaagactca 8700
tccattacta ctcaaacaga tagattttcc atggatgttc ccgtgtctga cctagctgag 8760

```
aatgatgaaa tctatgatcc acaaatcact agcccttatg aaaatgtccc ttcccaatct    8820
tttttctcta gtgaagaaag caaaacccaa acagatgcaa atcacaccac aagttttcac    8880
tcttctgaag tgtattctgt taccatcaca tcccctgttg aagacgttgt agtggcaagc    8940
tcctctagtg gaactgtttt aagcaaagaa tctaattttg agggccagga cataaaaatg    9000
gaatcccaac tggaaagtac cttgtgggaa atgcaatcag acagtgtctc ttcatctttc    9060
gagcctacta tgtccgctac aacaacagtt gttggtgaac aaataagcaa agtcatcatc    9120
acaaaaactg atgtggattc tgattcttgg agtgaaattc gggaagacga tgaagccttt    9180
gaggctcgtg tgaaagagga agaacaaaag atatttggtt tgatggtaga cagacaatca    9240
cagggtacca cccctgacac cactcctgct aggaccccaa ctgaagaggg gaccccaaca    9300
agtgagcaaa acccatttct gtttcaggaa ggaaaattgt ttgaaatgac ccgaagtggt    9360
gccattgata tgaccaaaag gtcctatgca gatgaaagtt ttcacttttt ccaaattggt    9420
caagaatcca gggaagagac tctctctgaa gatgtgaaag aaggggctac tggggctgat    9480
cccctaccgc tggagacatc agctgaatca ctagcacttt cagaatcaaa agaaacagtg    9540
gatgatgagg cagacttact tccagatagc gtgagtgagg aagtagagga aatacctgct    9600
tcggatgctc aacttaactc ccaaatgggg atttcagcct ccactgaaac acctacaaaa    9660
gaggctgtta gtgtagggac caaggacctc cccaccgtgc aaacgggtga tatacctcct    9720
ctctctggtg taaagcagat atcctgcccc gactcttctg aaccagctgt acaagtccag    9780
ttagattttt ccacactcac caggtctgtt tattcagata ggggtgatga ttctcccgat    9840
tcttccccag aagaacagaa atcagtaatc gagattccta ctgcacccat ggagaatgtg    9900
ccttttactg aaagcaaatc caaaattcct gtaaggacta tgcccacttc caccccagca    9960
cctccatctg cagagtatga gagttcagtt tctgaagatt ttctatccag tgtagatgag    10020
gaaaataagg cggatgaagc aaaaccaaag tccaaactcc ctgtcaaagt acccctccaa    10080
agagttgaac agcagctctc agatctagac acctctgtcc agaagacagt ggctcctcag    10140
ggacaggaca tggcaagcat cgcaccagat aatagaagca aatctgaatc tgatgctagt    10200
tctttggatt caaagaccaa atgcccagta aaaacccgaa gttacactga gacagaaaca    10260
gagagcagag agagggccga ggaacttgag ttagaatcag aagaaggggc cacaagacca    10320
aagatactta catcccgatt gccagttaag agcagaagca ctacatcttc ctgcaggggg    10380
ggcacgagcc ccacaaaaga aagtaaggag catttctttg acctttacag aaattccata    10440
```

gaattctttg aggagattag tgatgaggct tccaaattag tggataggct gacacagtca 10500
gagagggagc aggaaatagt ttcagacgat gaaagtagta gtgccctgga agtatcagta 10560
attgaaaatc tgccacctgt tgagaccgag cactcagttc ctgaggacat ctttgacaca 10620
aggcccattt gggatgagtc tattgagact ctgattgaac gcatccctga tgaaaatggc 10680
catgaccatg ctgaagaccc acaggatgag caggaacgga tcgaggaaag gctggcttat 10740
attgctgatc accttggctt cagctggaca gaattagcaa gagaactgga tttcactgag 10800
gagcaaattc atcaaattcg aattgaaaat cccaactctc ttcaagacca gagtcatgca 10860
ctgttgaagt actggctaga gagggatggg aaacatgcta cagataccaa cctcgttgaa 10920
tgtctcacca agatcaaccg aatggatatt gttcatctca tggagaccaa cacagaacct 10980
ctccaggagc gcatcagtca tagttatgca gaaattgaac agaccattac actggatcat 11040
agtgaagggt tctcggtact tcaagaggag ttatgcactg cacagcacaa gcagaaagag 11100
gagcaagctg tttctaaaga aagtgagacc tgcgatcacc ctcctatcgt ctcagaggaa 11160
gacatttctg ttggttattc cacttttcag gatggcgtcc ccaaaactga gggggacagc 11220
tcatcaacag cactctttcc ccaaactcac aaggagcaag ttcaacagga tttctcaggg 11280
aaaatgcaag acctgcctga agagtcatct ctggaatatc agcaggaata ttttgtgaca 11340
actccaggaa cagaaacatc agagactcag aaggctatga tagtacccag ctctcccagc 11400
aagacacctg aggaagttag cacccctgca gaggaggaga agctgtacct ccagacccca 11460
acatccagcg agcggggagg ctctcccatc atacaagaac ccgaagagcc ctcagagcac 11520
agagaggaga gctctccgcg gaaaaccagc ctcgtaatag tggagtctgc cgataaccag 11580
cctgagacct gtgaaagact cgatgaagat gcagcttttg aaaagggaga cgatatgcct 11640
gaaatacccc cagaaacagt cacagaagaa gaatacattg atgagcatgg acacaccgtg 11700
gtaaagaagg ttactaggaa aatcattagg cggtatgtat cctctgaagg cacagagaaa 11760
gaagagatta tggtgcaggg aatgccacag gaacctgtca acatcgagga aggggatggc 11820
tattccaaag ttataaagcg tgttgtattg aagagtgaca ccgagcagtc agaggacaac 11880
aatgagtaaa gccatcacac agaagagggc tgtggtgaag gaccagcatg gaaaacgcat 11940
tgacttggag cacctggagg atgtaccaga agcactagac caggacgacc tccagcgcga 12000
tctccagcag ctccttcggc atttctgcaa ggaggacttg aagcaagagg ccaagtgagg 12060
gactgcccag ttctcacacc agaaaccaca cattcactca atatgcagct tcctgtttca 12120
gtaggggagt gacctaactg gcctaattaa tgggataccc cgacatttcc actgttagca 12180

aatatacggc attttgcttt agttttcccc catcctcttt aactataaag ctaatttgtg 12240

accaaagatg gcatccttca tactggatgc tgtatccaat actttgttgt gtctgtgcta 12300

acctgggaac tggccacctc cattgttctt tgcttctgca caagatccat gaaaatccat 12360

tgatcagaag aacttcacct gcagacctct tcaagtgaca ctatgtagga atccttccaa 12420

ggaatatcta tgtacaatgt atatagctga aatgctcaga tgaacaacat attaaaatta 12480

aaaccactgc ctattgtaac tacactgggc atcagaataa aaggcctcta aaa 12533

<210> 10
<211> 3071
<212> DNA
<213> human

<400> 10
atggccggcg cccccggccc gctgcgcctt gcgctgctgc tgctcgggat ggtgggcagg 60

gccggccccc gcccccaggg tgccactgtg tccctctggg agacggtgca gaaatggcga 120

gaataccgac gccagtgcca gcgctccctg actgaggatc cacctcctgc cacagacttg 180

ttctgcaacc ggaccttcga tgaatacgcc tgctggccag atggggagcc aggctcgttc 240

gtgaatgtca gctgcccctg gtacctgccc tgggccagca gtgtgccgca gggccacgtg 300

taccggttct gcacagctga aggcctctgg ctgcagaagg acaactccag cctgccctgg 360

agggacttgt cggagtgcga ggagtccaag cgaggggaga gaagctcccc ggaggagcag 420

ctcctgttcc tctacatcat ctacacggtg ggctacgcac tctccttctc tgctctggtt 480

atcgcctctg cgatcctcct cggcttcaga cacctgcact gcaccaggaa ctacatccac 540

ctgaacctgt ttgcatcctt catcctgcga gcattgtccg tcttcatcaa ggacgcagcc 600

ctgaagtgga tgtatagcac agccgcccag cagcaccagt gggatgggct cctctcctac 660

caggactctc tgagctgccg cctggtgttt ctgctcatgc agtactgtgt ggcggccaat 720

tactactggc tcttggtgga gggcgtgtac ctgtacacac tgctggcctt ctcggtcttc 780

tctgagcaat ggatcttcag gctctacgtg agcataggct ggggtgttcc cctgctgttt 840

gttgtcccct ggggcattgt caagtacctc tatgaggacg agggctgctg gaccaggaac 900

tccaacatga actactggct cattatccgg ctgcccattc tctttggcat tggggtgaac 960

ttcctcatct ttgttcgggt catctgcatc gtggtatcca aactgaaggc caatctcatg 1020

tgcaagacag acatcaaatg cagacttgcc aagtccacgc tgacactcat ccccctgctg 1080

gggactcatg aggtcatctt tgcctttgtg atggacgagc acgcccgggg gaccctgcgc 1140

ttcatcaagc tgtttacaga gctctccttc acctccttcc aggggctgat ggtggccatc 1200
ttatactgct ttgtcaacaa tgaggtccag ctggaatttc ggaagagctg ggagcgctgg 1260
cggcttgagc acttgcacat ccagagggac agcagcatga agcccctcaa gtgtcccacc 1320
agcagcctga gcagtggagc cacggcgggc agcagcatgt acacagccac ttgccaggcc 1380
tcctgcagct gagactccag cgcctgccct ccctggggtc cttgctgcag gccgggtggc 1440
caatccaggt gggagagaca ctcccaggga caagggaagg aagggacaca cacacacaca 1500
cacacacaca cacacacaca tacatcctgc tttccctccc caaacccatc agacaggtaa 1560
atgggcagtg cctcctggga ccatggacac attttctcct aggagaagca gcctcctaat 1620
ttgatcacag tggcgagagg agaggaaaaa cgatcgctgt gaaaatgagg aggattgctt 1680
cttgtgaaac cacaggccct tggggttccc ccagacagag ccgcaaatca accccagact 1740
caaactcaag gtcaacggct tattagtgaa actggggctt gcaagaggag gtggttctga 1800
aagtggctct tctaacctca gccaaacaca gagcgggagt gacgggagcc tcctctgctt 1860
gcatcacttg gggtcaccac cctcccctgt cttctctcaa agggaagctg tttgtgtgtc 1920
tgggttgctt atttccctca tcttgccccc tcatctcact gcccagtttc tttttgaggg 1980
gctttgtttg ggccactgcc agcagctgtt tctggaaatg gctgtaggtg gtgttgagaa 2040
agaatgagca ttgagacggt gctcgcttct cctccaggta tttgagttgt tttggtgcct 2100
gcctctgcca tgcccagaga atcagggcag gcttgccacc ggggaaccca gccctggggt 2160
atgagctgcc aagtctattt taaagacgct caagaatcct ctggggttca tctagggaca 2220
cgttaggaat gtccagactg tgggtgtaga ttacctgcca cttccaggag cccagagggc 2280
caagagagac attgcctcca cctctccttg gaaatacttt atctgtgacc acacgctgtc 2340
tcttgagaat ttggatacac tctctagctt taggggacca tgaagagact ctcttaggga 2400
aaccaatagt ccccatcagc accatggagg caggctcccc ctgcctttga aattccccca 2460
cttgggagct tgtatatact tcactcactt ttctttattg ctgtgaatag tctgtgtgca 2520
caatgggcaa ttctgacttc tcccatctag tggaaatgag cgaaatcatg gttgtagtga 2580
tgttgtttgg gagagtgcag tagtaattga tttgacccac tcacacttgg agctaattaa 2640
ggtttgccct gcctgcagcc tcccccacaa ataatgaaca gcagaaagac tggacgggga 2700
aacctatcaa tcctgccccc agccatggtg aggaagcccc aagccatggt gacacacagc 2760
agcactgcag atagccagac acatggctat cctagagagg ctggcaagga gttcgtggct 2820

gcaaaagaag tttctggagc aagagagagc tcgctcttgg gagtcaggac ctccggggag 2880

agcagagggt tccgacggat tcctttatga gtcagtctct ctctcccttt taaatggtgg 2940

gaaccctccc caaaaccttt ccccagacac attctcctgt gcccctcaga gaggcatgtg 3000

atgtgcaagg aaaataatag gatgtaaaac acatcaagta gaaaatttct tatacttcca 3060

aaaaaaaaag c 3071

<210> 11
<211> 3088
<212> DNA
<213> human

<400> 11
cgcgccaggg aggttgtcgg gaggggccgg cgaataaaac gagcggcgaa agaaccgaaa 60

aaaggctcga cgctaccgtg tatgaggaac tttgatcctt gcgggccacc attccggaag 120

tagaatttag aggaagaaaa taccggagtt gcagggtata ggtaaatttc tcaaggttat 180

aggttggggt tcttagaact ttttgtggtg tgtgttggcc tagagcgact cagaagcgtt 240

agtgacttca cctaaaaaag ctaacctctc tgctgagcgc gaccggtatg cggcgcagga 300

tgagcctcag ggcttctgtt aagagtctgt ctgagaaagc cggtctgcgc tgttcctcgg 360

tggcgacctt aattatgaga tgagctaatg ctttactgac ttaaccatgg cgcagcgggc 420

agtgtggctc ataagccacg aaccgggaac tccactttgt ggcaccgtga gattctccag 480

acggtatcca actgttgaaa aacgagccag agtcttcaat ggagcaagtt atgtgcctgt 540

tcctgaagat ggtccctttc ttaaagcact gctctttgaa cttagattat tggatgatga 600

taaagacttc gttgagagtc gtgatagctg ttcacgcatc aataaaacat ccatttatgg 660

actcctgata ggaggtgaag aactctggcc agttgttgct tttctgaaga atgacatgat 720

atatgcttgt gttccactag ttgaacaaac tctgtcccct cgtccgccac taattagtgt 780

cagtggagtt tcacaaggct ttgaatttct ttttgggata caggattttc tttattcagg 840

tcaaaaaaat gactctgagc tgaatacaaa attgagccag ttgcctgact tgcttctgca 900

ggcttgtcca tttggtactt tattagatgc caacttacag aattcattag ataataccaa 960

ttttgcatct gtgactcagc cacagaaaca gccagcttgg aaaactggga cgtacaaagg 1020

aaaaccacaa gtttctattt ctatcactga aaaggtaaaa tccatgcaat atgataaaca 1080

gggtatagca gatacatggc aagttgttgg aacagtgact tgcaagtgtg atttggaagg 1140

aatcatgcca aatgttacca tcagcttgag tctccccacc aatggatctc cacttcagga 1200

```
tattctagtt caccccttgtg taacttctct tgactctgca attctgactt ctagtagtat   1260
tgatgcaatg gatgactctg catttagtgg gccttacaaa tttccattca ctccaccttt   1320
agagtcattc aacttatgct tctacacttc ccaggtccct gccccaccaa ttttgggttt   1380
ttatcaaatg aaggaggaag aagtacaact aagaataacc attaatttaa aacttcatga   1440
aagtgtgaaa aataattttg aattctgtga agcccatata cctttttaca atagaggtcc   1500
aattacacat ttggaataca aaactagttt tggccagctt gaagtatttc gagagaaaag   1560
cttattgatc tggattattg gccagaagtt cccaaaatca atggaaatta gtctttctgg   1620
aactgtaact tttggagcca agagccatga gaagcagcca tttgacccaa tttgtactgg   1680
agaaacagca tatttaaagc ttcattttag gatcttagat tacacactta ctggatgtta   1740
tgcagatcag cattcagttc aagtttttgc atcaggaaaa ccaaaaataa gtgcacaccg   1800
gaaactaatt tcttctgatt attacatctg gaattctaaa gcccctgctc cagtaacata   1860
tggatcatta ttattgtaat agtctcatgt ttaaatggga ttatataatg ataacagttt   1920
aaagaaaatc ataatcttat atttttaatg tggatgcata taacctgtga gtgaaaaatc   1980
actgaatgat ttaattgtaa aagtagtctt atgtggtgtt tgtagtctga tagagcttga   2040
aaggacattt taaaagctaa tgtctccaat tttgtcaacc ttcgatttta tgccagtata   2100
attcagaaca tagaaaagta atgattcact tgggctcatt ttagactggt cctgggtcac   2160
cctgccacac ttgtttccta gtgtttctgt ggcagacatt gctaatcaat tacagccctt   2220
ttctgtactg agccttggat aaagggtcag gctccttttt agttcagaga ttcaggcagc   2280
cactcccagt gggttgtaga taatgtgcaa gataaaaact attttctctt ccaaatctaa   2340
gtactaagct cctagtataa ggtgttgtta cagaatacca gagaccatgt tagagacaac   2400
tacatctctt caaaaaacag ccaacagaga caaaggaaaa gtgtttaaat agtaagctgt   2460
tcttcttaat cagaactatc ctattgacta ataaataatc tgcataattc tacttaaggt   2520
gtgtaatctc tgttctagag ttagttttta agtaagcttg ttaatctgcc actttgacat   2580
tttgcttagg atgtcagtag ccatattaag atgtgtagaa taccttcaga agatgatcat   2640
agtgttttgt aatcatttaa tgtctgcagc caaattttta aaggtaattt agacctaata   2700
ctgctcttgc tgtgtcttat taagttaaaa ttaatgaatg aattctggta aaaattcaaa   2760
aggcactctg tgagtagaga gtatcattta agcttatttt agtcacatgt agtatatatc   2820
tccttaaagc tgtcactctc actttcttac cattctcttg atttcttcag aaaccatcta   2880
gtcatcatct ttatactcta cctgcttctg caattatata tcatattatg ttttcagagc   2940
```

agttcattgt caagttggac tttaagtgac cattcaagaa aagatgaaat ctcacgaacc 3000

tcaaaacttc attcatgtct ttttacaaat gagaaaaaaa aatgcattaa agattaatac 3060

tcaatttgaa aaaaaaaaaa aaaaaaaa 3088

<210> 12
<211> 2584
<212> DNA
<213> human

<400> 12
gggggcgtgg ccccgagaag gcggagacaa gatggccgcc catagcgctt ggaggaccta 60

agaggcggtg gccggggcca cgccccgggc aggagggccg ctctgtgcgc gcccgctcta 120

tgatgcttgc gcgcgtcccc cgcgcgccgc gctgcgggcg gggcgggtct ccgggattcc 180

aagggctcgg ttacggaaga agcgcagcgc cggctgggga gggggctgga tgcgcgcgca 240

cccgggggga ggccgctgct gcccggagca ggaggagggg gagagtgcgg cgggcggcag 300

cggcgctggc ggcgactccg ccatagagca gggggccag ggcagcgcgc tcgccccgtc 360

cccggtgagc ggcgtgcgca gggaaggcgc tcggggcggc ggccgtggcc gggggcggtg 420

gaagcaggcg ggccggggcg gcggcgtctg tggccgtggc cggggccggg gccgtggccg 480

gggacgggga cggggccggg gccggggccg cggccgtccc ccgagtggcg gcagcggcct 540

tggcggcgac ggcggcggct gcggcggcgg cggcagcggt ggcggcggcg ccccccggcg 600

ggagccggtc cctttcccgt cggggagcgc ggggccgggg cccaggggac cccgggccac 660

ggagagcggg aagaggatgg attgcccggc cctccccccc ggatggaaga aggaggaagt 720

gatccgaaaa tctgggctaa gtgctggcaa gagcgatgtc tactacttca gtccaagtgg 780

taagaagttc agaagcaagc ctcagttggc aaggtacctg ggaaatactg ttgatctcag 840

cagttttgac ttcagaactg gaaagatgat gcctagtaaa ttacagaaga acaaacagag 900

actgcgaaac gatcctctca atcaaaataa gggtaaacca gacttgaata caacattgcc 960

aattagacaa acagcatcaa ttttcaaaca accggtaacc aaagtcacaa atcatcctag 1020

taataaagtg aaatcagacc cacaacgaat gaatgaacag ccacgtcagc ttttctggga 1080

gaagaggcta caaggactta gtgcatcaga tgtaacagaa caaattataa aaaccatgga 1140

actacccaaa ggtcttcaag gagttggtcc aggtagcaat gatgagaccc ttttatctgc 1200

tgttgccagt gctttgcaca caagctctgc gccaatcaca gggcaagtct ccgctgctgt 1260

ggaaaagaac cctgctgttt ggcttaacac atctcaaccc ctctgcaaag cttttattgt 1320

cacagatgaa gacatcagga aacaggaaga gcgagtacag caagtacgca agaaattgga 1380

agaagcactg atggcagaca tcttgtcgcg agctgctgat acagaagaga tggatattga 1440

aatggacagt ggagatgaag cctaagaata tgatcaggta actttcgacc gactttcccc 1500

aagagaaaat tcctagaaat tgaacaaaaa tgtttccact ggcttttgcc tgtaagaaaa 1560

aaaatgtacc cgagcacata gagcttttta atagcactaa ccaatgcctt tttagatgta 1620

tttttgatgt atatatctat tattcaaaaa atcatgttta ttttgagtcc taggacttaa 1680

aattagtctt ttgtaatatc aagcaggacc ctaagatgaa gctgagcttt tgatgccagg 1740

tgcaatctac tggaaatgta gcacttacgt aaaacatttg tttcccccac agttttaata 1800

agaacagatc aggaattcta aataaatttc ccagttaaag attattgtga cttcactgta 1860

tataaacata tttttatact ttattgaaag gggacacctg tacattcttc catcatcact 1920

gtaaagacaa ataaatgatt atattcacag actgattgga attctttctg ttgaaaagca 1980

cacacaataa agaacccctc gttagccttc ctctgattta cattcaactc tgatccctgg 2040

gccttaggtt tgacatggag gtggaggaag atagcgcata tatttgcagt atgaactatt 2100

gcctctggac gttgtgagaa ttgtgctttc accagaattt ctaagaattt ctgctaaata 2160

tcacctagca tgtgtaattt tttttccttg cctgtgactt ggacttttga tagttctata 2220

agaataaggc tttttcttcc cttgggcatg agtcagatac acaaggaccc ttcaggtgtt 2280

actagaaggc gtccatgttt attgtttttt aaagaatgtt tggcactctc taacgtccac 2340

tagcttactg agttatcagg tgcaggtcag actcttggct acagtgagag gcagcttcta 2400

ggcagagttg cttaatgaaa gggtttgtaa tactttacaa accattacct gtacctggcc 2460

tggcctccaa aatattaaca ttcttttttct gttgaaactc gcgagtgtaa ctttcatacc 2520

acttgaattt attgatattt aattatgaaa actagcatta cattattaaa cgatttctaa 2580

aatc 2584

<210> 13
<211> 2935
<212> DNA
<213> human

<400> 13
ccgcaactcg ctcggccgcc gccatcttgc gagctcgtcg tactgaccga gcggggaggc 60

tgtcttgagg cggcaccgct caccgacacc gaggcggact ggcagccctg agcgtcgcag 120

tcatgccggc cggacccgtg caggcggtgc cccgccgcc gcccgtgccc acggagccca 180

```
aacagcccac agaagaagaa gcatcttcaa aggaggattc tgcaccttct aagccagttg      240
tggggattat ttaccctcct ccagaggtca gaaatattgt tgacaagact gccagctttg      300
tggccagaaa cgggcctgaa tttgaagcta ggatccgaca gaacgagatc aacaacccca      360
agttcaactt tctgaacccc aatgaccctt accatgccta ctaccgccac aaggtcagcg      420
agttcaagga agggaaggct caggagccgt ccgccgccat ccccaaggtc atgcagcagc      480
agcagcagac cacccagcag cagctgcccc agaaggtcca agcccaagta atccaagaga      540
ccatcgtgcc caaagagcct cctcctgagt ttgagttcat tgctgatcct ccctctatct      600
cagccttcga cttggatgtg gtgaagctga cggctcagtt tgtggccagg aatgggcgcc      660
agtttctgac ccagctgatg cagaaagagc agcgcaacta ccagtttgac tttctccgcc      720
cacagcacag cctcttcaac tacttcacga agctagtgga acagtacacc aagatcttga      780
ttccacccaa aggtttattt tcaaagctca agaaagaggc tgaaaacccc cgagaagttt      840
tggatcaggt gtgttaccga gtggaatggg ccaaattcca ggaacgtgag aggaagaagg      900
aagaagagga gaaggagaag gagcgggtgg cctatgctca gatcgactgg catgattttg      960
tggtggtgga aacagtggac ttccaaccca atgagcaagg gaacttccct cccccccacca    1020
cgccagagga gctgggggcc cgaatcctca ttcaggagcg ctatgaaaag tttggggaga     1080
gtgaggaagt tgagatggag gtcgagtctg atgaggagga tgacaaacag gagaaggcgg     1140
aggagcctcc ttcccagctg gaccaggaca cccaagtaca agatatggat gagggttcag     1200
atgatgaaga agaagggcag aaagtgcccc cacccccaga gacacccatg cctccacctc     1260
tgcccccaac tccagaccaa gtcattgtcc gcaaggatta tgatcccaaa gcctccaagc     1320
ccttgcctcc agcccctgct ccagatgagt atcttgtgtc cccccattact ggggagaaga     1380
tccccgccag caaaatgcag gaacacatgc gcattggact tcttgaccct cgctggctgg     1440
agcagcggga tcgctccatc cgtgagaagc agagcgatga tgaggtgtac gcaccaggtc     1500
tggatattga gagcagcttg aagcagttgg ctgagcggcg tactgacatc ttcggtgtag     1560
aggaaacagc cattggtaag aagatcggtg aggaggagat ccagaagcca gaggaaaagg     1620
tgacctggga tggccactca ggcagcatgg cccggaccca gcaggctgcc caggccaaca     1680
tcaccctcca ggagcagatt gaggccattc acaaggccaa aggcctggtg ccagaggatg     1740
acactaaaga gaagattggc cccagcaagc ccaatgaaat ccctcaacag ccaccgccac     1800
catcttcagc caccaacatc cccagctcgg ctccacccat cacttcagtg ccccgaccac     1860
```

ccacaatgcc acctccagtt cgtactacag ttgtctccgc agtacccgtc atgccccggc 1920

ccccaatggc atctgtggtc cggctgcccc caggctcagt gatcgccccc atgccgccca 1980

tcatccacgc gcccagaatc aacgtggtgc ccatgcctcc ctcggcccct cctattatgg 2040

ccccccgccc accccccatg attgtgccaa cagcctttgt gcctgctcca cctgtggcac 2100

ctgtcccagc tccagcccca atgccccctg tgcatccccc acctcccatg gaagatgagc 2160

ccacctccaa aaaactgaag acagaggaca gcctcatgcc agaggaggag ttcctgcgca 2220

gaaacaaggg tccagtgtcc atcaaagtcc aggtgcccaa catgcaggat aagacggaat 2280

ggaaactgaa tgggcaggtg ctggtcttca ccctcccact cacggaccag gtctctgtca 2340

ttaaggtgaa gattcatgaa gccacaggca tgcctgcagg gaaacagaag ctacagtatg 2400

agggtatctt catcaaagat tccaactcac tggcttacta caacatggcc aatggcgcag 2460

tcatccacct ggccctcaag gagagaggcg ggaggaagaa gtagacaaga ggaacctgct 2520

gtcaagtccc tgccattttg cctctcctgt ctcccacccc ctgccccaga cccaggagcc 2580

cccctgaggc tttgccttgc ctgcatattt gtttcgctct tactcagttt gggaattcaa 2640

attgtcctgc agaggttcat tcccctgacc ctttccccac attggtaaga gtagctgggt 2700

tttctaagcc actctctgga atctctttgt gttagggtct cgatttgagg acattcattt 2760

cttcagcagc ccattagcaa ctgagagccc agggatgtcc tacaggatag tttcatagtg 2820

acaggtggca cttggctaat agaatatggc tgatattgtc attaatcatt ttgtaccttg 2880

acatgggttg tctaataaaa ctcggaccct tcttgtgaaa aaaaaaaaaa aaaaa 2935

<210> 14
<211> 462
<212> DNA
<213> human

<400> 14
atggcgcgta ctaagcagac ggctcgtaaa tccacaggcg gtaaagcacc gcgcaaacag 60

ctggccacta aggcagctcg caagagcgct ccggccacgg gcggcgtgaa gaagccccat 120

cgctaccgcc ctggcaccgt ggctctgcgc gagatccgtc gctaccagaa gtctaccgag 180

cttctaatcc ggaagctgcc gtttcagcgc ctggtgcgag aaatagctca ggacttcaag 240

accgacctgc gcttccagag ttccgcggtg atggcgctgc aggaggcctg cgaggcctac 300

ttggtggggc ttttcgagga caccaacctg tgcgctattc atgccaaacg cgtgaccatc 360

atgcctaaag acatccagct tgcccgccgc attcgtgggg agagggcgtg aattgttttg 420

agtacaaacc ttaaatccaa aggctcttct cagagccaac ca 462

<210> 15
<211> 6830
<212> DNA
<213> human

<400> 15
ccacggtaag gggatgacgt agctttgcca aagacttaga agctaagcag aaaatgagct 60

taacatcctg gtttttggtg agcagtggag gcactcgcca caggctgcca cgagaaatga 120

tttttgttgg aagagatgac tgtgagctca tgttgcagtc tcgtagtgtg gataagcaac 180

acgctgtcat caactatgat gcgtctacgg atgagcattt agtgaaggat ttgggcagcc 240

tcaatgggac ttttgtgaat gatgtaagga ttccggaaca gacttatatc accttgaaac 300

ttgaagataa gctgagattt ggatatgata caaatctttt cactgtagta caaggagaaa 360

tgagggtccc tgaagaagct cttaagcatg agaagtttac cattcagctt cagttgtccc 420

aaaaatcttc agaatcagaa ttatccaaat ctgcaagtgc caaaagcata gattcaaagg 480

tagcagacgc tgctactgaa gtgcagcaca aaactactga agcactgaaa tccgaggaaa 540

aagccatgga tatttctgct atgccccgtg gtactccatt atatgggcag ccgtcatggt 600

ggggggatga tgaggtggat gaaaaaagag ctttcaagac aaatggcaaa cctgaagaaa 660

aaaaccatga agctggaaca tcagggtgca gcatagatgc caagcaagtt gaggaacaat 720

ctgcagctgc aaatgaagaa gtactttttc ctttctgtag ggaaccaagt tattttgaaa 780

tccctacaaa agaattccag caaccatcac aaataacaga aagcactatt catgaaatcc 840

caacaaaaga cacgccaagt tcccatataa caggtgcagg gcatgcttca tttaccattg 900

aatttgatga cagtacccca gggaaggtaa ctattagaga ccatgtgaca aagtttactt 960

ctgatcagcg ccacaagtcc aagaagtctt ctcctggaac tcaagacttg ctggggattc 1020

aaacaggaat gatggcaccc gaaaacaaag ttgctgactg gctagcacaa aacaaccctc 1080

ctcaaatgct atgggaaaga acagaagagg attctaaaag cattaaaagt gatgttccag 1140

tgtacttgaa aaggttgaaa ggaaataaac atgatgatgg tacgcaaagt gattcagaga 1200

acgctggggc tcacaggcgc tgtagcaaac gtgcaactct tgaggaacac ttaagacgcc 1260

accattcaga acacaaaaag ctacagaagg tccaggctac tgaaaagcat caagaccaag 1320

ctgttactag ctctgcgcat cacagagggg ggcatggtgt tccacatggg aaattgttaa 1380

aacagaaatc agaggagcca tcggtgtcaa tacccttcct acaaactgca ttattaagaa 1440

gttcagggag tcttgggcac agaccaagcc aggagatgga taaaatgtta aaaaatcaag 1500
caacttctgc tacttctgaa aaggataatg atgatgacca aagtgacaag ggtacttata 1560
ccattgagtt agagaatccc aacagtgagg aagtggaagc aagaaaaatg attgacaagg 1620
tgtttggagt agatgacaat caggattata ataggcctgt tatcaacgaa aaacataaag 1680
atctaataaa agattgggct ctcagttctg ctgcagcagt aatggaagaa agaaaaccac 1740
tgactacatc tggatttcac cactcagagg aaggcacatc ttcatctgga agcaaacgtt 1800
gggtttcaca gtgggctagt ttggctgcca atcatacaag gcatgatcaa gaagaaagga 1860
taatggaatt ttctgcacct cttcctttag agaatgagac agagatcagt gagtctggca 1920
tgacagtgag aagtactggc tctgcaactt ccttggctag ccagggagag agaaggagac 1980
gaactcttcc ccagcttcca aatgaagaaa agtctcttga gagccacaga gcaaaggttg 2040
taacacagag gtcagagata ggagaaaaac aagacacaga acttcaggag aaagaaacac 2100
ctacacaggt ataccagaaa gataaacaag atgctgacag acccttgagt aaaatgaaca 2160
gggcagtaaa tggagagact ctcaaaactg gtggagataa taaaacccta cttcacttag 2220
gcagctctgc tcctggaaaa gagaaaagtg aaactgataa ggaaacttct ttggtaaagc 2280
aaacattagc aaaacttcaa caacaagaac aaagggagga ggctcagtgg acacctacta 2340
aattgtcttc caaaaatgtt tcaggtcaga cagataaatg tagggaggaa acttttaaac 2400
aagaatcaca acctccagaa aaaaattcag gacattctac aagcaaagga gacagagtgg 2460
cacaaagtga gagcaagaga agaaaagctg aggaaattct gaaaagtcag actccaaagg 2520
gaggagacaa gaaggaatcc tccaagtcat tagtgcgaca agggagcttc actatagaaa 2580
aacccagccc aaacataccc atagaactta ttccccatat aaataaacag acttcctcta 2640
ctccttcttc tttagcatta acatctgcaa gtagaatacg agaaagaagt gagtctttgg 2700
atcctgattc tagtatggac acaaccctta ttctaaaaga cacagaagca gtaatggctt 2760
ttctagaagc taaactacgt gaagataata aaactgatga aggaccagat actcccagtt 2820
ataatagaga caattctatt tcaccagaat ctgatgtaga tacagctagt acaatcagtc 2880
tggttactgg agaaactgaa agaaagtcaa cccaaaagcg aaagagtttc actagcctct 2940
ataaagatag gtgttccaca ggttctcctt ccaaagatgt tacaaaatca tcatcttcag 3000
gtgctaggga aaaaatggaa aagaaaacaa aaagtcgttc cacagatgtg ggttcaagag 3060
cagatggtcg taaatttgtt cagtccagtg ggagaataag acagccctca gtagacttaa 3120
cagatgatga ccaaacctct agtgtacctc attctgccat ctctgatatt atgtcatctg 3180

atcaagaaac ttactcttgt aaacctcatg gacggactcc acttacctca gctgatgagc 3240
atgtacattc caaactggaa ggaagtaaag taacgaaatc taagacttct ccggtggtat 3300
ctggttcatc tagtaaatca accacccttc caaggccacg acctaccagg acttccctct 3360
tgcgcagagc acgacttggt gaagcttcag acagtgaact tgctgatgct gacaaagcat 3420
ctgttgcttc tgaagtatcc acaacaagtt ctacatcaaa acctcccaca ggaaggcgta 3480
acatctctcg gattgattta ttggctcagc ctcgtagaac acgacttggc tcactgtcag 3540
ctcgtagtga ctctgaagca acaatttcta gaagtagtgc ctcttcgagg accgcagaag 3600
ccatcattag aagtggagcc agactagtac catcagataa attttctcct agaattagag 3660
ctaacagtat ctctcgactc tcagactcca aggtcaaaag tatgacctca gctcatggct 3720
ctgcttcagt aaattcaaga tggaggcgct ttcctactga ttatgcttcc acctcagaag 3780
atgaatttgg atcaaaccgt aattccccta aacatacccg tctacgtact tctccagccc 3840
tgaaaaccac tcgcttgcag agcgctggat cagcaatgcc tactagttct tcattcaaac 3900
accggattaa agagcaggaa gactacatcc gagattggac tgctcatcga gaagagatag 3960
ccaggatcag ccaagatctt gctctcattg ctcgggagat caacgatgta gcaggagaga 4020
tagattcagt gacttcatca ggcactgccc ctagtaccac agtaagcact gctgccacca 4080
cccctggctc tgccatagac actagagaag agttggttga tcgtgttttt gatgaaagcc 4140
tcaacttccg aaagattcct ccattagttc attccaaaac accagaagga aacaacggtc 4200
gatctggtga tccaagacct caagcagcag agcctcccga tcacttaaca attacaaggc 4260
ggagaacctg gagcagggat gaagtcatgg gagataatct gctgctgtca tccgtctttc 4320
agttctctaa gaagataaga caatctatag ataagacagc tggaaagatc agaatattat 4380
ttaaagacaa agatcggaat tgggatgaca tagaaagcaa attaagagcc gaaagtgaag 4440
tccctattgt gaaaacctca agcatggaga tttcttctat cttacaggaa ctgaaaagag 4500
tagaaaagca gctgcaagca atcaatgcta tgattgatcc tgatggaact ttggaggctc 4560
tgaacaacat gggatttccc agtgctatgt tgccatctcc accgaaacag aagtccagcc 4620
ctgtgaataa ccaccacagc ccgggtcaga caccaacact tggccaacca gaagctaggg 4680
ctcttcatcc tgctgctgtt tcagccgcag ctgaatttga gaatgctgaa tctgaggctg 4740
atttcagtat acatttcaat agattcaacc ccgatgggga agaggaagat gttacagtac 4800
aagaatgact ttctcttgat tgttgaaaaa tcattacctg tggaatggct aggaatattg 4860

gaagcagcat agtgttgatg tacgcaaaac aagacagctt ggtcagctac aatcttggaa 4920
tccctgtctt cttaatttta tttatttatt tttgacgtat aatgtagtat atcaatcctt 4980
tcaaactatt tagataacca cttgatgcac aaataggaaa aagcagattg tggcagtgtc 5040
gccttttgtg gttttatgat tttcaaattg aatttaatga ttacaccctt tcccttcata 5100
gatctttttt cttttttta agccatgctg tgacctacaa gcaaactaaa tagccaacat 5160
ttctgaaccc ctaagtctcc tgtgccaagc tgctccctga aatggacttc ttcatctgta 5220
cagatttgtt aaaccattct atttgcttct taataatagg atttatatta gtactcatta 5280
ccattggaca caatgacata agtactctcc acagtaaagc agacctttca caacagtcac 5340
tctgtgtcct aaaattttcc aacatagatg tgatttatat aactttgttg atacgtaaat 5400
tgtcttgggg tttacggaaa ttaactatta tgtttgcact aagatttgct gggagtggta 5460
ggtggacata tctatatatc aataaggact aaccgtcttt tttgtacata ggggattgat 5520
aatactgtat ttgttttaag cccacagtgt ttttactcca ctttcaaaaa gtcaatttgg 5580
gcactttttt tcattttttt ttaatgggaa taagatttgg tctctcattt taggttaaat 5640
gataactaga aagattaaac tagacagata gtttaggtgg agtatatttt taaaactcag 5700
aacatgtata ttggtcctgt gttaccaagt ttatatgtga cagttgaaaa agaaattccc 5760
ttgaaatgat catgaggtta aaatttctt cattagggga cttggagaac cagtagtcgt 5820
aagattagtt gatagtttca ctcccaagca atgaattgct tctgtgtgtt tccctgtagg 5880
actcaatagt aaatgctgtc tgtcttacac atttataagg accctgcaag acgacgacaa 5940
aggcctttgg cctgtgctac taaacaagaa gcctatgaaa aatttcttct ttaaacttgt 6000
tttttctctt tccagtaagt tcacatttgg ataattttaa aaagaaaagt aattaccttt 6060
gtgtttccag aacactataa ttggggtgta tcttaattca gttaaatatt attagtagac 6120
ctggattttc ccccttgacc ccatcagtct ataaaggtta aactgcaact tttatgaaat 6180
ggtctttaat atttccacaa taatcctgtg ctatatttgt tttaagaaac aaagtaactc 6240
tatacacttc aagactttac aggatttttt aaatcctgta ttgttggatc aattaataaa 6300
gatgcaaaaa aactttatag agatgtaaaa acaaaactat aatggatctc ctatttttct 6360
ttaaatacaa aaaaaaaaag gtaaatgaac tattctcctt gtaaagctaa attccccatt 6420
ctgtctaata aaggaagact gaaaaaaggt tttaaagaac ataaatggaa agatacaaat 6480
gctttgaagg aataaacgaa atgttaaaac agggtcaatc catttgaaga aaaagttgga 6540
caaaataatc agcattgctc cctcttttat ttaatatttg ggtactgatt atatccacat 6600

ggaagtagaa ggtaaggagt ttaggaaaat actagaatct actctgctta cattcttgtt 6660

taagtgttta cacagtttgg ttcaattata aacatttggc cttttactat gttattttta 6720

ttttgtatga atacattatc ctccttattt atttttgtta catttattac ttatgttatt 6780

ttgttatgca tttacaactc catttttaaa taaagtgttt ctggaacttt 6830

<210> 16
<211> 2001
<212> DNA
<213> human

<400> 16
ggccgcggtg gtggctgcgg cggcggcggc gggagcagca tggattgggg cactgagctg 60

tgggatcagt tcgaggtgct cgagcgccac acgcagtggg ggctggacct gttggacaga 120

tatgtaaagt tcgtgaaaga acgcaccgaa gtggaacagg cttacgccaa acaactgcgg 180

agcctggtga aaaaatatct gcccaagaga cctgccaagg atgatcctga gtccaaattc 240

agccagcaac agtccttcgt acagattctc caggaggtga atgactttgc aggccagcgg 300

gagctggtgg ctgagaacct cagtgtccgt gtatgtcttg agctgaccaa gtactcacaa 360

gagatgaaac aggagaggaa gatgcacttc caagaagggc ggcgggccca gcagcagctg 420

gaaaatggct ttaaacagct ggagaatagt aagcgtaaat ttgagcggga ctgccgggag 480

gcagagaagg cagcccagac tgctgaacgg ctagaccagg atatcaacgc caccaaggct 540

gatgtggaga aggccaagca gcaagcccac cttcggagtc acatggccga agaaagcaaa 600

aacgaatatg cggctcaact gcagcgcttc aaccgagacc aagcccactt ctatttttca 660

cagatgcccc agatattcga taagctccaa gacatggatg aacgcagggc cacccgcctg 720

ggtgccgggt atgggctcct gtcggaggcc gagctggagg tggtgcccat aatagccaag 780

tgcttggagg gcatgaaggt ggctgcaaat gctgtggatc ccaagaacga ctcccacgtc 840

cttatagagc tgcacaagtc aggttttgcc cgcccgggcg acgtggaatt cgaggacttc 900

agccagccca tgaaccgtgc accctccgac agcagtctgg gcaccccctc ggatggacgg 960

cctgaactcc gaggcccggg tcgcagccgc accaagcgct ggccttttgg caagaagaac 1020

aagacagtgg tgaccgagga ttttagccac ttgcccccag agcagcagcg aaaacggctt 1080

caacagcagt tggaagaacg cagtcgtgaa cttcagaagg aggttgacca gagggaagcc 1140

ctaaagaaaa tgaaggatgt ctatgagaag acacctcaga tgggggaccc cgccagcttg 1200

gagccccaga tcgctgaaac cctgagcaac attgaacggc tgaaattgga agtgcagaag 1260

tatgaggcgt ggctggcaga agctgaaagt cgagtcctta gcaaccgggg agacagcctg 1320

agccggcacg cccggcctcc cgaccccccc gctagcgccc cgccagacag cagcagcaac 1380

agcgcatcac aggacaccaa ggagagctct gaagagcctc cctcagaaga gagccaggac 1440

acccccattt acacggagtt tgatgaggat ttcgaggagg aacccacatc cccccatagg t 1500

cactgtgtgg ccatctacca ctttgaaggg tccagcgagg gcactatctc tatggccgag 1560

ggtgaagacc tcagtcttat ggaagaagac aaaggggacg gctggacccg ggtcaggcgg 1620

aaagagggag gcgagggcta cgtgcccacc tcctacctcc gagtcacgct caattgaacc 1680

ctgccagaga cgggaagagg ggggctgtcg gctgctgctt ctgggccacg gggagcccca 1740

ggacctatgc actttatttc tgaccccgtg gcttcggctg agacctgtgt aacctgctgc 1800

cccctccacc cccaacccag tcctacctgt cacaccggac ggacccgctg tgccttctac 1860

catcgttcca ccattgatgt acatactcat gttttacatc ttttctttct gcgctcggct 1920

ccggccattt tgttttatac aaaaatgggt tttttttttt tctttaatat atttcaagag 1980

attttttttt tttttttttt t 2001

<210> 17
<211> 2956
<212> DNA
<213> human

<400> 17
cgaggcgcgg ctccggggat tcggctcggg ccgctggctc tgctctgcgg ggagggagcg 60

ggcccgcccg cggggcccga gccctccgga tccgccccct ccccggtccc gccccctcgg 120

agactcctct ggctgctctg ggggttcgcc ggggccgggg acccgcggtc cgggcgccat 180

gcgggcatcg ctgctgctgt cggtgctgcg gcccgcaggg cccgtggccg tgggcatctc 240

cctgggcttc accctgagcc tgctcagcgt cacctgggtg gaggagccgt gcggcccagg 300

cccgccccaa cctggagact ctgagctgcc gccgcgcggc aacaccaacg cggcgcgccg 360

gcccaactcg gtgcagcccg gagcggagcg cgagaagccc ggggccggcg aaggcgccgg 420

ggagaattgg gagccgcgcg tcttgcccta ccaccctgca cagcccggcc aggccgccaa 480

aaaggccgtc aggacccgct acatcagcac ggagctgggc atcaggcaga ggctgctggt 540

ggcggtgctg acctctcaga ccacgctgcc cacgctgggc gtggccgtga accgcacgct 600

ggggcaccgg ctggagcgtg tggtgttcct gacgggcgca cggggccgcc gggccccacc 660

tggcatggca gtggtgacgc tgggcgagga gcgacccatt ggacacctgc acctggcgct 720

```
gcgccacctg ctggagcagc acggcgacga ctttgactgg ttcttcctgg tgcctgacac     780
cacctacacc gaggcgcacg gcctggcacg cctaactggc cacctcagtc tggcctccgc     840
cgcccacctg tacctgggcc ggccccagga cttcatcggc ggagagccca cccccggccg     900
ctactgccac ggaggctttg gggtgctgct gtcgcgcatg ctgctgcaac aactgcgccc     960
ccacctggaa ggctgccgca acgacatcgt cagtgcgcgc cctgacgagt ggctgggtcg    1020
ctgcattctc gatgccaccg ggggtgggctg cactggtgac cacgaggggg tgcactatag    1080
ccatctggag ctgagccctg gggagccagt gcaggagggg gaccctcatt tccgaagtgc    1140
cctgacagcc caccctgtgc gtgaccctgt gcacatgtac cagctgcaca aagctttcgc    1200
ccgagctgaa ctggaacgca cgtaccagga gatccaggag ttacagtggg agatccagaa    1260
taccagccat ctggccgttg atggggaccg ggcagctgct tggcccgtgg gtattccagc    1320
accatcccgc ccggcctccc gctttgaggt gctgcgctgg gactacttca cggagcagca    1380
cgctttctcc tgcgccgatg gctcaccccg ctgcccactg cgtggggctg accgggctga    1440
tgtggccgat gttctgggga cagctctaga ggagctgaac cgccgctacc acccggcctt    1500
gcggctccag aagcagcagc tggtgaatgg ctaccgacgc tttgatccgg cccggggtat    1560
ggaatacacg ctggacttgc agctggaggc actgaccccc cagggaggcc gccggcccct    1620
cactcgccga gtgcagctgc tccggccgct gagccgcgtg gagatcttgc ctgtgcccta    1680
tgtcactgag gcctcacgtc tcactgtgct gctgcctcta gctgcggctg agcgtgacct    1740
ggcccctggc ttcttggagg cctttgccac tgcagcactg gagcctggtg atgctgcggc    1800
agccctgacc ctgctgctac tgtatgagcc gcgccaggcc cagcgcgtgg cccatgcaga    1860
tgtcttcgca cctgtcaagg cccacgtggc agagctggag cggcgtttcc ccggtgcccg    1920
ggtgccatgg ctcagtgtgc agacagccgc accctcacca ctgcgcctca tggatctact    1980
ctccaagaag cacccgctgg acacactgtt cctgctggcc gggccagaca cggtgctcac    2040
gcctgacttc ctgaaccgct gccgcatgca tgccatctcc ggctggcagg ccttctttcc    2100
catgcatttc caagccttcc acccagctgt ggccccacca caagggcctg ggcccccaga    2160
gctgggccgt gacactggcc gctttgatcg ccaggcagcc agcgaggcct gcttctacaa    2220
ctccgactac gtggcagccc gtgggcgcct ggcggcagcc tcagaacaag aagaggagct    2280
gctggagagc ctggatgtgt acgagctgtt cctccacttc tccagtctgc atgtgctgcg    2340
ggcggtggag ccggcgctgc tgcagcgcta ccgggcccag acgtgcagcg cgaggctcag    2400
```

tgaggacctg taccaccgct gcctccagag cgtgcttgag ggcctcggct cccgaaccca 2460

gctggccatg ctactctttg aacaggagca gggcaacagc acctgacccc accctgtccc 2520

cgtgggccgt ggcatggcca caccccaccc cacttctccc ccaaaaccag agccacctgc 2580

cagcctcgct gggcagggct ggccgtagcc agaccccaag ctggcccact ggtcccctct 2640

ctggctctgt gggtccctgg gctctggaca agcactgggg gacgtgcccc cagagccacc 2700

cacttctcat cccaaaccca gtttccctgc cccctgacgc tgctgattcg ggctgtggcc 2760

tccacgtatt tatgcagtac agtctgcctg acgccagccc tgcctctggg ccctgggggc 2820

tgggctgtag aagagttgtt ggggaaggag ggagctgagg agggggcatc tcccaacttc 2880

tccctttttgg accctgccga agctccctgc ctttaataaa ctggccaagt gtgaaaaaaa 2940

aaaaaaaaaa aaaaaa 2956

<210> 18
<211> 889
<212> DNA
<213> human

<400> 18
gcgaactggt ggcagtgaga gacttcggcg gacatggctc ccagcgtgcc agcggcagaa 60

cccgagtatc ctaaaggcat ccgggccgtg ctgctggggc ctcccggggc cggtaaaggg 120

acccaggcac ccagattggc tgaaaacttc tgtgtctgcc atttagctac tggggacatg 180

ctgagggcca tggtggcttc tggctcagag ctaggaaaaa agctgaaggc aactatggat 240

gctgggaaac tggtgagtga tgaaatggta gtggagctca ttgagaagaa tttggagacc 300

cccttgtgca aaaatggttt tcttctggat ggcttccctc ggactgtgag gcaggcagaa 360

atgctcgatg acctcatgga gaagaggaaa gagaagcttg attctgtgat tgaattcagc 420

atcccagact ctctgctgat ccgaagaatc acaggaaggc tgattcaccc caagagtggc 480

cgttcctacc acgaggagtt caaccctcca aaagagccca tgaaagatga catcaccggg 540

gaacccttga tccgtcgatc agatgataat gaaaaggcct tgaaaatccg cctgcaagcc 600

taccacactc aaaccacccc actcatagag tactacagga aacgggggat ccactccgcc 660

atcgatgcat cccagacccc cgatgtcgtg ttcgcaagca tcctagcagc cttctccaaa 720

gccacatgta aagacttggt tatgtttatc taatgttggg tccaagaagg aatttctttc 780

catccctgtg aggcaatggg tgggaatgat aggacaggca aagagaagct tcctcaggct 840

agcaaaaata tcatttgatg tattgattaa aaaagcactt gcttgatgt 889

<210> 19
<211> 2964
<212> DNA
<213> human

<400> 19
agtcggcggc ggctgctgct gcctgtggcc cgggcggctg ggagaagcgg agtgttggtg 60

agtgacgcgg cggaggtgta gtttgacgcg gtgtgttacg tgggggagag aataaaactc 120

cagcgagatc cgggccgtga acgaaagcag tgacggagga gcttgtacca ccggtaacta 180

aatgaccatg gaatctggag ccgagaacca gcagagtgga gatgcagctg taacagaagc 240

tgaaaaccaa caaatgacag ttcaagccca gccacagatt gccacattag cccaggtatc 300

tatgccagca gctcatgcaa catcatctgc tcccaccgta actctagtac agctgcccaa 360

tgggcagaca gttcaagtcc atggagtcat tcaggcggcc cagccatcag ttattcagtc 420

tccacaagtc caaacagttc agatttcaac tattgcagaa agtgaagatt cacaggagtc 480

agtggatagt gtaactgatt cccaaaagcg aagggaaatt ctttcaagga ggccttccta 540

caggaaaatt ttgaatgact tatcttctga tgcaccagga gtgccaagga ttgaagaaga 600

gaagtctgaa gaggagactt cagcacctgc catcaccact gtaacggtgc caactccaat 660

ttaccaaact agcagtggac agtatattgc cattacccag ggaggagcaa tacagctggc 720

taacaatggt accgatgggg tacagggcct gcaaacatta accatgacca atgcagcagc 780

cactcagccg ggtactacca ttctacagta tgcacagacc actgatggac agcagatctt 840

agtgcccagc aaccaagttg ttgttcaagc tgcctctgga gacgtacaaa cataccagat 900

tcgcacagca cccactagca ctattgcccc tggagttgtt atggcatcct ccccagcact 960

tcctacacag cctgctgaag aagcagcacg aaagagagag gtccgtctaa tgaagaacag 1020

ggaagcagct cgagagtgtc gtagaaagaa gaaagaatat gtgaaatgtt tagaaaacag 1080

agtggcagtg cttgaaaatc aaaacaagac attgattgag gagctaaaag cacttaagga 1140

cctttactgc cacaaatcag attaatttgg gatttaaatt ttcacctgtt aaggtggaaa 1200

atggactggc ttggccacaa cctgaaagac aaaataaaca ttttattttc taaacatttc 1260

tttttttcta tgcgcaaaac tgcctgaaag caactacaga atttcattca tttgtgcttt 1320

tgcattaaac tgtgaatgtt ccaacacctg cctccacttc tcccctcaag aaattttcaa 1380

cgccaggaat catgaagaga cttctgcttt tcaaccccca ccctcctcaa gaagtaataa 1440

tttgtttact tgtaaattga tgggagaaat gaggaaaaga aaatcttttt aaaaatgatt 1500

```
tcaaggtttg tgctgagctc cttgattgcc ttagggacag aattacccca gcctcttgag   1560
ctgaagtaat gtgtgggccg catgcataaa gtaagtaagg tgcaatgaag aagtgttgat   1620
tgccaaattg acatgttgtc acattctcat tgtgaattat gtaaagttgt taagagacat   1680
accctctaaa aaagaacttt agcatggtat tgaaggaatt agaaatgaat ttggagtgct   1740
ttttatgtat gttgtcttct tcaatactga aaatttgtcc ttggttctta aaagcattct   1800
gtactaatac agctcttcca tagggcagtt gttgcttctt aattcagttc tgtatgtgtt   1860
caacattttt gaatacatta aaagaagtaa ccaactgaac gacaaagcat ggtatttgaa   1920
ttttaaatta aagcaaagta aataaaagta caaagcatat tttagttagt actaaattct   1980
tagtaaaatg ctgatcagta aaccaatccc ttgagttata taacaagatt tttaaataaa   2040
tgttattgtc ctcaccttca aaaatattta tattgtcact catttacgta aaaagatatt   2100
tctaatttac tgttgcccat tgcacttaca taccaccacc aagaaagcct tcaagatgtc   2160
aaataaagca aagtgatata tatttgttta tgaaatgtta catgtagaaa aatactgatt   2220
ttaaatattt tccatattaa caatttaaca gagaatctct agtgaatttt ttaaatgaaa   2280
gaagttgtaa ggatataaaa agtacagtgt tagatgtgca caaggaaagt tattttcaga   2340
catatttgaa tgactgctgt actgcaatat ttggattgtc attcttacaa aacatttttt   2400
tgttctcttg taaaaagagt agttattagt tctgctttag ctttccaata tgctgtatag   2460
cctttgtcat tttataattt taattcctga ttaaaacagt ctgtatttgt gtatatcata   2520
cattgttttc aataccactt ttaattgtta ctcattttat tcactaagct cgataaatct   2580
aacagttact cttaaaaaaa aaaaaaagac taaggtggat tttaaaaatt ggaaactgac   2640
ataatgttag gttataattt ctcatttgga gccgggcgca gtggctcacg cctgtaatcc   2700
cagcactttg ggaggccaag gtgggtggat cacctgtggt caagagttca agaccagcct   2760
ggccatcatg gtgaaacccc atctctacta aaaatacaaa aattagccag gcgtggtggc   2820
tggcgcctgt aatcccagct actcaggagg ttgaggcagc agaattgctt gaacccagga   2880
ggcagagggt tgcagtgagc cgagatagca ccattgcact ccagcctggg cgactccatc   2940
tcaaaaaata aaaaaaaaaa aaaa                                          2964
```

<210> 20
<211> 1004
<212> DNA
<213> human

<400> 20

atgggtctgg agctctacct ggacctgctg tcccagccct gccgcgctgt ttacatcttt 60

gccaagaaga acgacattcc cttcgagctg cgcatcgtgg atctgattaa aggtcagcac 120

ttaagcgatg cctttgccca ggtgaacccc ctcaagaagg tgccggcctt gaaggacggg 180

gacttcacct tgacggagag tgtggccatc ctgctctacc tgacgcgcaa atataaggtc 240

cctgactact ggtaccctca ggacctgcag gcccgtgccc gtgtggatga gtacctggca 300

tggcagcaca cgactctgcg gagaagctgc ctccgggcct tgtggcataa ggtgatgttc 360

cctgtgttcc tgggtgggcc agtatctccc cagacactgg cagccaccct ggcagagttg 420

gatgtgaccc tgcagttgct cgaggacaag ttcctccaga acaaggcctt ccttactggt 480

cctcacatct ccttagctga cctcgtagcc atcacggagc tgatgcatcc cgtgggtgct 540

ggctgccaag tcttcgaagg ccgacccaag ctggccacat ggcggcagcg cgtggaggca 600

gcagtggggg aggacctctt ccaggaggcc catgaggtca ttctgaaggc caaggacttc 660

ccacctgcag accccaccat aaagcagaag ctgatgccct gggtgctggc catgatccgg 720

tgagctggga aacctcaccc ttgcaccgtc ctcagcagtc cacaaagcat tttcatttct 780

aatggcccat gggagccagg cccagaaagc aggaatggct tgcttaagac ttgcccaagt 840

cccagagcac ctcacctccc gaagccacca tccccaccct gtcttccaca gccgcctgaa 900

agccacaatg agaatgatgc acactgaggc cttgtgtcct ttaatcactg catttcattt 960

tgattttgga taataaacct ggctcagcct gagcctctgc ttct 1004

<210> 21
<211> 8093
<212> DNA
<213> human

<400> 21
ctcagctccc tgtggtgggg gatggcagag gcttcccgct ccccgcagct ggtttgcaca 60

gcctcccgac tgtgccgtgg tgtctccttc cctaaagtgg gacagtcgtt tctgtgtcct 120

gctggcctct gaggaacaga cgtgtgtgag aggccttcag ggcctgatgg ctggggtggt 180

gacagtgcct ggagaatggg tggggactgg aggggccagg tggctaacca ctctcctctt 240

ccatggcagc ggagctggag ttcgcccaaa tcatcatcat cgtcgtggtg gtcacggtga 300

tggtggtggt catcgtctgc ctgctgaacc actacaaagt ctccacgcgg tccttcatca 360

accgcccgaa ccagagccgg aggcgggagg acgggctgcc gcaggaaggg tgcctgtggc 420

cttcagacag cgccgcaccg cggctgggcg cctcggagat catgcatgcc ccgcggtcca 480

```
gggacaggtt cacagcgccg tccttcatcc agagggatcg cttcagccgc ttccagccca    540
cctaccccta tgtgcagcac gagattgatc ttcctcccac catctccctg tccgacggtg    600
aagagccacc tccttaccag ggccctgca ccctgcagct ccgggaccct gaacagcaga    660
tggaactcaa ccgagagtcc gtgagggccc cacccaaccg aaccatattt gacagtgatt    720
taatagacat tgctatgtat agcgggggtc catgcccacc cagcagcaac tcgggcatca    780
gtgcaagcac ctgcagcagt aacgggagga tggaggggcc accccccaca tacagcgagg    840
tgatgggcca ccacccaggc gcctctttcc tccatcacca gcgcagcaac gcacacaggg    900
gcagcagact gcagtttcag cagaacaatg cagagagcac aatagtaccc atcaaaggca    960
aagataggaa gcctgggaac ctggtctgat tccttccaac gtgcacttca gctggagaaa   1020
gaaaccaaga agggaagcgg ccgctgggcc cctcctgcgc acagtgttgt tcagtttcac   1080
atggtacaaa taagtaaaac caaatgagca aacacggtct ttgtttctga ttccttttag   1140
gggaattgca tgcaaactag actgaaatga tacaaacttc catctggtct gaccgcaaac   1200
agtgtttatt tggggacagg ggttgggatg ggggtgtggg caggggaaaa cagagaacgg   1260
gatgctttga agataccatg aaataaaacc cacagaggta tttgatgtat ttaattgtga   1320
aaggagactt tgcagataaa tgaggccaga atggcatgtt ttataattaa ctgaataaag   1380
aaggaagcat tattatatat tattgtgggg aagaaccagc cagttcgctt tttctcctaa   1440
ggtgtggact tttattttgt tttaaaaata tgaatcaaaa ttcctgtgtt gtgtgccaag   1500
gtataaagtg gagaagttag atgagtgcaa ggagctcctt tgtgttgtga tgatgtgttt   1560
taaaagttgc actatcttaa tgttgaaaat atttacaagg gaactgtttt acgtgaagtt   1620
ctgtatgttg tcttttcacc tgtggattgt aatcaggccc aaggaatatc ctggagtggt   1680
ccccagaagc atccaagaaa agatatttgg ggacgtagcc taacatttta ccaacttacg   1740
taaatcaaaa aagtcattat tgttgcagga gtttgcatca aatagcagtg catcgctgaa   1800
gcttttggag acttttggat ggaagataag atagggaaga ttaagttcca gcatttctga   1860
cttgttattt tgagttactc tgctactctt aggctgcata gtttatgaga aaatgaacac   1920
atgcatttat ggatccagta tcatgcagtg ctgccctcat cctccagcag tgcaatttct   1980
tcagtaattt agattttttt cactatagca tgaaatatat tcaaatacat accttatttt   2040
atgcaataaa ttgtttaaaa tgcaaggtgg ttattctgca tactgttgaa atatgtgact   2100
cctcagtata ttcccattgc ctctccccct ttcctcgaca gcttagttca gttctgcagg   2160
gctgctcagt tcacaggagg ctcccagcag ccaccccaca tccagcctac acagaacttt   2220
```

cgtgtgggag tggtgtgggt ggtggttttc ttatgctttg gaagcccccta gaaataatga 2280

cggaagaatg ccatgttgct gatcgtggta ataagccatt gtgggttatt gtatgtcact 2340

agtattagca tagcattctt aaaggaatgc agtgttcaaa acctacccaa attccccgca 2400

ggattttacc aaacccttcc ccaggccagt tttgtactga aggcaagaac tggacagtca 2460

gagaacagtg gagggggcaa gtgactgaag agcaccgggt aaaaagcaca acatgcagtt 2520

aaaatgcaaa ctagaaaact aattttaaat attgttagtt ttaatatttc ctgatattta 2580

caaatattca ttcttatata caatgaaaaa aataactttc ttctgcagat gtaagcactg 2640

gcttttataa gagcagcagc caacacgttt agcagacact gcgcgtggag aagggcttat 2700

ctgcagtaca ctctgccatg tggagggtgg gcctctgtgg cctcttcaca taacaagatg 2760

agctggaatg atgattccat gactcccacc tatgcagcct taaagccaaa tccgcgtgtg 2820

tgtgtttgtg tctgtctgtg ggtctcgaag gtgatccgtc ggtgcggtgg ctctgtgctg 2880

taactggaga gactgttcca aaccccaaga gttgtctgat cctagtctgt tcccttctgc 2940

ttcttacctc tgtagatagg tcactggttt ttgtttgttt gttttgagga ttggaatttc 3000

cattacattc atcctttgca cacagtaaca tccacagaac tagtccaact cttaaaagga 3060

gagaggaaaa acacaggcac cagttgtcag ctcatgctta caacctgtgt ggaagtatat 3120

acagttgaga gtcacagtgg aggttctgag actggattca gtcttgttcc agtgacagtt 3180

ggaaggcctc tgctggagag acaccagctc tcagggcaga gattggcttg gggccagaag 3240

gaccctcccc aaccctggag acaccctgaa ggttcactgg ctctccagat tagcctctct 3300

tcctctgtca ggcaaagatg aggagcccgt gttcccatcg ggccctgctg gcagggactt 3360

gcagtggatt cttggtcagg tgtgcccaca gatgcggagg cgaggtgagt gattccatca 3420

tttcagttct cacctgcagt tttggtgaag caggagatgc accccacagc tctagctctc 3480

aaatggcttc acagtcctta cttctctacc tgcctcaaga aggggctcag agcagagact 3540

tgtgaattcc ttagtaactg tgagtatatg aatgtgttgc acatgtccac agtattggcg 3600

agataattac ataattcaga tacctttaat catctttcaa gaaagaggct cctcccattc 3660

aaccacccta gagaactgcc tttgttaaat agttatttaa agactcatac atatcaaacc 3720

atgactttga aaggtcttcg aggctggggc tctgtaatga attagtttaa aagccaaggt 3780

cataacatga attgatggtc aatttccctt cagcagaagg aaaaggtgat ttagatcagt 3840

agctcttttg aaggttgtgg ctgacctgtt cataccgtgt cgcctcatgg ctagtgtggc 3900

```
gttgaaagag tagcgactgg gaagatacaa cttacacagt ggggcctatt gttctttcaa   3960
gaacccttt tttagcttat agaacccatg ggtccagttt agtaacgagt gatttaggca   4020
atcaatgata ggtttataat cttagattat tccagcaaag tgtggattgc attgttagga   4080
agaacatttg gtgggaatga acactcctgg gcataccgct gacttttgtc ccttgttccc   4140
ggtgtaggag acccaaggca tcttgaatcc catctataag aacacaatct tccagcatac   4200
gtttgctttt tcagaaactc tagcattctc tttaaatact gacgcaatcc ttaatggaaa   4260
agagatttca tgaagcaaat tatgtatttc aatagttctt ctatttttag tgtccaaaat   4320
ttactaatac agaagcttga caagcatgtc ctcaccctcc ccaccacata aacacatgga   4380
cacacaccca agccacaaga aatcccaaga gagcagaagc gaatttttaa aagatttatc   4440
gtgaggactg catttccatt cactaatttt ggctcaaact tatgaggcag gaaatagggg   4500
ccaacagtaa atgggggagg cctcctgaca ccagcagagg aattttgtac ccaggcgagg   4560
acttcttgaa cttctgcgta tctccgtttg atctctttca cctttatttc atcttcataa   4620
gaatgagaaa ggctcaaaag gaagcacttt tagaaatctt ctctgaccta gaagaatcca   4680
tccaaatccc tgccttcctc tctgaaccaa cagttccctt ctctgacagg gggccatcct   4740
ctatcttcca tccagcggct cttcctttta ggaaggctct ggtgcagagc acttcaaata   4800
tgtcctcagg ccagatactg attgctagta gagagacacc cggcacccag tccgaagccc   4860
tccctcaaag gaccggctta tggcgttggt cactggcagg ctcagagaca ttctactgtg   4920
ggcgcaggga gcccggcccc ccatgcagcc atgactggat gcgcccccat ctcgggggct   4980
tgctgcactg cttgtttatt gaattttgct acttagaatg gcaacattaa ctttgtgtac   5040
cattcatttt ttaaaaattt tccaaagctc ggcagtgtat gaaagaaaaa actgggaaag   5100
atacttggtt tctgttaact tttgtgttgc ttgcttaagt gattaaagcc agtgcttgga   5160
gccaagcctt catgccacga acatgctcca cagcctgccc tttgctctcc tgctcacact   5220
gaccaagaat gccgcgtgct tggcctactg aggtgaaagg acaattgaat gacaggtggg   5280
caaagggaga acttcccctt cttggtgcga ggaaagtcac aaatttaaaa atgttgcttc   5340
cagcccagat cctaaatgct agttctcagc agctgcgtgg cttaccgttc gccatttcca   5400
ccaccgccag ctgccagcac cgctacagat cacagagatg tgaacagaca atggaaagca   5460
ctcttagcct tgcagtggtc tacatttttt aggaaccaat atttcagcat tctttattac   5520
ccggcacgct gtgtcctttg cagagttcaa gtttatgtta ctgccagggt cagacagtca   5580
tttgctgctg ctgctgctgc tgctgctgct tctcgaactg gatgcattag gaagctgctg   5640
```

tctgagtgta ggaatgtctt gctaagaaag caatgtcttc cttcatcctt ttctttcttc 5700
cctctgcgtg tccttgtttt tgtgtaatgc gggagagggt tagagctata gagattatat 5760
atacactatc cgtgcacatt atatatatgt agatataccc ctatcatgtc agagatctgc 5820
atgtcagttt ttcagcaact aaggtgcctc atgttctgag ttcagcagat ataggaacca 5880
agccgccccc tcctgcactt gatgctccca cctttgttgt gcctcactta aaatggtgct 5940
tttttcagtt gtctgtcttt tcttatgttt ttatttgtaa ggtgctgtat ataagttgaa 6000
tatattatgc acatatccta cccaatgggt agaacaaaaa gttgttaata ctgtaatata 6060
atgtatagat gataccaatt ttaacagaaa tggcatagaa tttgtgaatg cctatgtgct 6120
ttgtcctctt ttgtaaggaa atttgcaaat ggatgcatac agattaaagt ctatgtagtt 6180
tattttccta ttaaatatca atattataac acaagagaaa gaagtgtgaa caaacaagca 6240
acagtttatg accagcgtat atatagcaat ggaaagttgc atctttgctg tgaaaacact 6300
ttaaagaaaa tactttttaa aaaatcccac agctttttgg ttgccactag acgcttctta 6360
ttttaatcat tttagtaatg ctcagctgga ccagtgttag ttatatttga gtcagaaaaa 6420
tgttgttttt caacttgctt tataatctcc tgcatctatc tcctgctgta gcatcaygaa 6480
ggtgtcaggc aacagtgaaa agtgcacatt tttgttgttg cagaaactgt gtcagaggaa 6540
taagtaaatc agcctgcagc agaagacttt gttcagctcc agaggcatct gtgaccgtct 6600
gtgtccaagt ctctctgtgc ctttttcttt tacaaactga agctgtggag ccaatgaagt 6660
aacagtagag attgtaggga aagaatacct caggaaaaac aaatacactt acaagaagac 6720
cctgttctta gaaaatgtgt ttagttatgg gttagcacta gaagagactt ggctgtcagc 6780
cagccaagtg aaggacctct catccattcc cattcatgtc ccatcataat acggacmcaa 6840
aaagcaaact cggttttgcc atcagttaga aattacgttt tggattgtat attgttacat 6900
ctctcttcca gcttagtttt tagtgtctga ttgtgacctc tgcatttatc ttcaaatacc 6960
ctaattttaa aacaaaagaa caagaaaagt ttataacacc atgttcacta aaaccacggt 7020
tgaatcttgg gtgtgggcat cctttcgagt gttgtccata agagcagttc gtggaatttt 7080
gcccatctga cccatattat cagcttattc tgccaccaga gtagagtcta ataaattcca 7140
aagtttttat ttgctccatg gtgtatgttc tgactttgaa aatgtcagat tctataatca 7200
tacccctaac atccaggaga caaatgacag attatcttta aactgaaatt gactctacaa 7260
tgcaaccctt aatgctgaat ggattaaaaa agtcagccct tttagtatct gtttgaaagg 7320

gccgtaaaaa gttgacactt ttgttgttgt ggatcctgcg tgtctagacc cacgtgttgt 7380

ttccatcgta tactgtaggg tgcacccctt gggattcatc attaagaact gaggctcact 7440

gttgtcagaa acaaagctcc caccccccag gttcaacctt gtgggagaac tgttgagcat 7500

gagaatgttc tagactcaga ggtactaaaa tttgttacca catcattgct tcctttctac 7560

aggacgaatt gaggcttaaa ctttactgtt aatgatactg gttcatttta atgtgcttgt 7620

tggtatgttg ctatttttca tttcatagct ttcaaaaatc atgctaattg tatacttgtc 7680

tagtttaagg ctattttaaa atatgtacaa tactattcac agcatttagt tcgtttaatt 7740

tttattataa agcaatctac taaaaaagta caactgtatt tgaacttttc aatagttgtt 7800

tgtgagctat gataatcaaa agtcattaaa gtctttttta acaaacattc gtgcttactt 7860

ttcaacataa ttcccagtta tatacagaaa aagatttcca cctgtcacgt atctgcctct 7920

tttacctgag caatggtgta gttcttagac ctaaggtctg taattgcaat acttttaaag 7980

aaagatgttg ctctaagtgc tgtttgttag ttatgaaatc agatttttct gcttgttctt 8040

aatgctgtgg tcaaaccata gcacaaaatc attaaaaata atcagcggca tac 8093

<210> 22
<211> 999
<212> DNA
<213> human

<400> 22
ttttttggc ggtataaaag tgcttcttta tttggtcata cagctttcaa atgttgctta 60

acgaacagat agcattttta taatcagagg aaagactcag tattaaagtg cgcgtcccat 120

cattgcaacc gggccctcgg gcagccagct ctctcttgct tcgcgtccgg ctctcaggcc 180

ctggagctcg gcctccctac cttctccagg cgcgggtcct gctccttttc cggccgccct 240

tcctcagccc ccggctcgga cactctgcag gagaccaaga gaagggggaa atggggctgg 300

gggccgtccc cgggagacag gcggccttcc gagagggact ggagcaggcc gtgcggtatg 360

ccaaagccct gggctgtccc aggatccacc tgatggctgg ccgagtaccc cagggagctg 420

atcgaatagc agtcaaggct gagatggagg ccgtttttct ggagaacctg aggcatgcag 480

ctggggtttt ggctcaggag gacctcgtgg gactgctgga gcccatcaac acccgcatca 540

ctgaccccca gtacttcctg gacacgcccc agcaggcggc agccatctta cagaaggtag 600

gaagacccaa cctccaatta caaatggaca tattccactg gcagatcatg gatgggaacc 660

tgacaggaaa catccgggag ttcctgccca ttgttgggca tgtgcaggtg gcacaggtcc 720

caggccgagg ggagcccagc agccccggag agctgaattt cccctatctg tttcaactgc 780

tggaagatga aggctacaaa ggcttcgtgg gctgtgagta tcagcctcga ggagacacag 840

tagagggctt gagttggcta cgttcatact gggataggcg gggccaccca gaggctggcc 900

agtgagggcc cgcacaccac ccacgtgcct ccagacagcg agtgacatcc catctcctcc 960

tctgcattaa agatgacctg ctgaaaaaaa aaaaaaaaa 999

<210> 23
<211> 2316
<212> DNA
<213> human

<400> 23
tcaaaataaa attcctgaat ttgtacaagc cacaggaagc tagattgaga tcattatatg 60

acaactggaa ggccaaggct atgggttacc tcaaattgag gaattttggc acctactcac 120

aggctccatg agcagatgaa gtagacagct ttactcagta tctcagacca agaacttcat 180

ctccatctcc aactagctga aacatcttcc ctcctcaacc tggaaaattc tctgacttag 240

aaatttaaac aaaaccctcc cctttcattg aatctccatt gtctggagtt tgcttgtttt 300

aatctagcct gttcctccac tatgggctcc ctttcaaact atgccctgct tcaactaacc 360

cttactgctt ttttgacaat tctagtacaa cctcagcacc tgcttgctcc agttttccgg 420

acactatcta tcttgactaa tcagtctaat tgctggttat gtgaacatct agataatgca 480

gaacaacccg aactagtttt tgttcctgcc agtgcaagca cctggtggac ctattctgga 540

caatggatgt atgaaagggt gtggtatcca caagcagaag tacagaatca ctctacttcc 600

tcctatcgta aagtgacttg gcactgggaa gcctccatgg aagctcaagg tctatccttt 660

gctcaagtaa ggttattgga gggaaatttt tctctttgcg tagaaaataa aaatggcagt 720

ggacccttcc taggtaatat acctaaacaa tactgtaatc aaatactatg gtttgattct 780

acagatggca ccttcatgcc ctctatagat gttacaaatg aatccaggaa cgatgatgat 840

gatacaagtg tttgcctagg cactagacaa tgttcctggt ttgcaggttg cacaaaccgg 900

acctggaaca gctcagctgt tcccttgatt ggtctgccca atacccaaga ctacaaatgg 960

gtagatcgaa attctggatt gacctggtca ggtaatgaca cctgtctcta tagctgccaa 1020

aaccaaacca aaggccttct gtaccagcta tttcgcaacc tattttgctc ttatggcctg 1080

acagaggcac atgggaaatg gagatgtgca gatgccagca taactaatga caaaggtcat 1140

gatggacacc ggacccccac ctggtggctc acaggttcca atctgacctt gtctgtgaac 1200

```
aactctggcc tcttttttttt gtgcggcaat ggggtgtaca aagggtttcc acctaaatgg   1260
tctgggcgat gtggacttgg gtatcttgta ccttccctca ccagatacct caccttaaat   1320
gctagccaaa ttacaaacct gagatccttc attcataaag taacaccgca tagatgcacc   1380
caaggagaca cagacaatcc acctctgtat tgcaacccca aggacaattc aacaataagg   1440
gcccttttc caagtttggg aacttatgat ttagaaaagg caattctaaa catttccaaa   1500
gcaatggaac aggaattcag tgccactaag cagaccttgg aagcacacca atcaaaagtt   1560
agcagtttag cctctgcatc ccgaaaggat catgtcttgg atataccgac cacccaacga   1620
caaacggctt gtggaactgt tggcaaacag tgttgcctct atataaatta ttcggaagaa   1680
ataaagtcta atatacagcg tctccacgaa gcatccgaga acctgaagaa tgtaccgtta   1740
cttgattggc aaggcatatt tgcaaaagtg ggagactggt tcagatcatg gggctatgtg   1800
cttttaattg ttcttttctg cttattcatc tttgttttaa tctatgttcg tgtctttcgc   1860
aaatctcgca gatcccttaa ctcccaacct ctgaacctag ccttatctcc acagcaatca   1920
gcacagctcc ttgtcagtga aacttcatgt caagtttcaa atagggcaat gaagggacta   1980
acaacccatc aatatgatac aagtctactt tgagaatatc tgaacaaaca gcagctgcag   2040
acaaaaagcc ttagctaaac tttgatgagt aaagcaggtc ttaccgagaa ttcagctgcc   2100
aaaaccctcc tctgagtgtt cctcttataa gggcacttag cactaggacc tcccaaggta   2160
ttgtaaataa gccttatcag aactttttgt agtttcattc tgaagcctta agacacacac   2220
cataaagctg atctgtaaac ccttacccct tgctgttcag agagctactc tttgtagtgt   2280
tcttgcatgc atatataata aatgtttttt ctattg                              2316
```

<210> 24
<211> 2133
<212> DNA
<213> human

<400> 24

```
caaccgcgcc gtctgtccct ggcaagccag cggcggttta aaggaggtgg cgggaagcct     60
gtgtgtgctt caaatcgtca ccctcatggt cgctccggtt tttcacgact gaaaataaca    120
tagcaaaata agccaagatg tctgtggatc caatgaccta cgaggcccag ttctttggct    180
tcacgccaca aacgtgcatg cttcggatct acattgcatt tcaagactac ctatttgaag    240
tgatgcaggc cgttgaacag gttattctga agaagctgga tggcatccca gactgtgaca    300
ttagcccagt gcagattcgc aaatgcacag agaagtttct ttgcttcatg aaaggacatt    360
```

```
ttgataacct ttttagcaaa atggagcaac tgtttttgca gctgatttta cgtattccct   420
caaacatctt gcttcctgaa gataaatgta aggagacacc ttatagtgag gaagattttc   480
agcatctcca gaaagaaatt gaacagttac aggagaagta caagactgaa ttatgtacta   540
agcaggccct tcttgcagaa ttagaagagc aaaaaattgt tcaggccaaa ctcaaacaga   600
cgttgacttt ctttgatgag cttcataatg ttggcagaga tcatgggact agtgatttta   660
gggagagttt agtatccctg gttcagaact ccagaaaact acagaacatt agagacaatg   720
tggaaaagga atcgaaacga ctgaaaatat cttaattgct cagtagtcaa aaggaggagc   780
ctgtcaaaaa gtagaatcat aaggactgtt caaaccataa ggactgttca aatcatacca   840
gtgactgttc aaaccaacca tactttttat tagatttgct ttgtcaactc tttcttgtat   900
tctgtgtttt cctctttttt ggtccacttt gctgaggtat gaagtgtact actttgaact   960
aggctgaagc atctgagtct tctaataagt gggaagggat ccaacaaaga agccatgacc  1020
agttaaagat atttgcagag ttacaccttg gtcataagtc ctttgtgacc ttgattattt  1080
tggcttactc tttggatgag accagacaag aaaaggatta aacgggtggc tcctttaata  1140
ttattattat tgtttttgag acaaggtccc tttctgtcac ccaggttaga gtagatttca  1200
gtggcacaat cttggctcac tgcaacctct gtgtcctggg ctcaagtgat cctcctgcct  1260
cagcctccca agtagctagg accacaggtg cgtgtcacca tgcttggcta atttttttgc  1320
agaaacgagg cctcactata ttgtccaggc tgagtggctc ttttattaac cagtcattac  1380
actgcggaac agccaacata gagtacttgc tctcgccctg tgaattttct ttcatgaggg  1440
agtcaatatg tagtggaaag aagcatgtag caaaaaagac aaccttgatc tttaataaaa  1500
aagaagttgg tttatttcca aaataaatcc cctgacaaaa aacctggtga tgttaagcaa  1560
ttgactgtct tagagtccag cagaagacct tagacaaaaa aagcagaacc cactggagta  1620
gaaaaggaag catgtagcat atactcagta gtgaaattta attttactga ctgttaggta  1680
tctatgccaa tttgttttca tacttcagtt ggttttggaa tctgccttat acctaatatt  1740
taattattta ttcacactca taagcatcaa atatttaatg ccctcagtgg gaaaattgtg  1800
tttaaactca atggaatcta atatttcttt atgtcgttag tccctgtaaa atgttaggtc  1860
acccaaggaa aggggagaaa tagcaatggt tgttcctaag gtattgcttg ccctccatgt  1920
cttcctaaag agcagaactt ggagtttctc ctttatgtag agaagaagta acttagggtg  1980
tatttgcaat gaaatattca tagatattga aagcttgtgt ttacatgaaa tatgtttatt  2040
atcaagaatt ccttttttcca attctgtaca ttaaatatat gtgttttaaa ggaaaaaaaa  2100
```

aaaaaaaaaa aaaaaaaaaa aaaaaaaaaa aaa 2133

<210> 25
<211> 1336
<212> DNA
<213> human

<400> 25
cctaactcca atcactggca actcctgaga tcagaggaaa accagcaaca gcgtgggagt 60

ttggggagag gcattccata ccagattctg tggcctgcag gtgacatgct gcctaagaga 120

agcaggagtc tgtgacagcc accccaacac gtgacgtcat ggccagtagg gaagatgagc 180

tgaggaactg tgtggtatgt ggggaccaag ccacaggcta ccactttaat gcgctgactt 240

gtgagggctg caagggtttc ttcaggagaa cagtcagcaa aagcattggt cccacctgcc 300

cctttgctgg aagctgtgaa gtcagcaaga ctcagaggcg ccactgccca gcctgcaggt 360

tgcagaagtg cttagatgct ggcatgagga aagacatgat actgtcggca gaagccctgg 420

cattgcggcg agcaaagcag gcccagcggc gggcacagca aacacctgtg caactgagta 480

aggagcaaga agagctgatc cggacactcc tgggggccca cacccgccac atgggcacca 540

tgtttgaaca gtttgtgcag tttaggcctc cagctcatct gttcatccat caccagccct 600

tgcccaccct ggcccctgtg ctgcctctgg tcacacactt cgcagacatc aacactttca 660

tggtactgca agtcatcaag tttactaagg acctgcccgt cttccgttcc ctgcccattg 720

aagaccagat ctcccttctc aagggagcag ctgtggaaat ctgtcacatc gtactcaata 780

ccactttctg tctccaaaca caaaacttcc tctgcgggcc tcttcgctac acaattgaag 840

atggagcccg tgtggggttc caggtagagt ttttggagtt gctctttcac ttccatggaa 900

cactacgaaa actgcagctc caagagcctg agtatgtgct cttggctgcc atggccctct 960

tctctcctga ccgacctgga gttacccaga gagatgagat tgatcagctg caagaggaga 1020

tggcactgac tctgcaaagc tacatcaagg gccagcagcg aaggccccgg gatcggtttc 1080

tgtatgcgaa gttgctaggc ctgctggctg agctccggag cattaatgag gcctacgggt 1140

accaaatcca gcacatccag ggcctgtctg ccatgatgcc gctgctccag gagatctgca 1200

gctgaggcca tgctcacttc cttccccagc tcacctggaa caccctggat acactggagt 1260

gggaaaatgc tgggaccaaa gattgggccg ggttcaaagg gagcccagtg gttgcaatga 1320

aagactaaag caaaac 1336

<210> 26
<211> 5638
<212> DNA
<213> human

<400> 26
gcagctggag tcaggcgctc gcccgtccgc cggttggctc gccgggacct cgcgcaccgg 60

cggcagagtc ccttgcgtgg attggcaagc gacgccccac ctgccccgag ctcaccattt 120

tctttcgcgc tggctgcagc tgacccggcg aagggagccg accgggccct gggctggagg 180

taaaacccca cggaaagaac atgaggttcc cttggaaatc attcaagagg aagatggaag 240

gggctgttta gaagagctta aaagctacag gctgtaagac tggggcctga gtgctggcag 300

tggaaaacac tgttgggctg tgatctctcc ctgaaaagtt ccaggtgcct ttttgcttcc 360

tgcaaaagaa aggaagcgaa agagaagacc atgtccatag ccctgaagca ggtattcaac 420

aaggacaaga ccttccgacc caagaggaaa tttgaacctg gcacacagag gtttgagctg 480

cacaaacggg ctcaggcatc cctcaactcg ggtgtggacc tgaaggcggc tgtgcagttg 540

cccagtgggg aggaccagaa tgactgggtg gcagtacatg tggtggacct cttcaatcgg 600

atcaacctca tctatggcac catctgtgag ttctgcaccg agcggacctg tcctgtgatg 660

tcagggggcc ccaaatatga gtatcggtgg caggatgatc tcaagtataa gaagccaaca 720

gcgctgccag ctccccagta catgaacctt cttatggatt ggattgaggt tcagatcaac 780

aacgaggaaa tatttccaac atgcgtgggt gttcccttcc caaagaactt ccttcagatc 840

tgcaagaaga tcctgtgccg ccttttccgg gtctttgtcc acgtctatat ccaccacttc 900

gaccgggtca ttgtgatggg tgcagaggcc catgtcaaca cctgctacaa acacttctat 960

tactttgtca cagagatgaa cctcatagac cgcaaggagc tagagccttt gaaagaaatg 1020

acgagcagga tgtgtcacta atgctccacc tcacccttttg gaagaaagga aagctgtttc 1080

ctcctggtgc cctgagcggg caggaggtgg accaccctgg ctgaaatgac acacctactc 1140

ccaggaacag cagaggtgga ggcaagcagt gactcctgag agacattccc cactcacttt 1200

gtgtgctctt aaccttctga gtgctgctag cccagacctg tggacgaggc agaccacaac 1260

gtgaaagaag gaccagcccc ttgaccgttc tggctgggga attgtccacg aggaagcctc 1320

tgcacttcca cacatggcac agttctgcct gtgacctgcc gcctaagctt tactggaatt 1380

cagggttttg agactgagat gcgtgtcgta tttttcactt atctgtcttg tcagctggcc 1440

gacttctctg tgattggttt tttaagtgcc gagtgaattt tggacctctg gatgtgcagc 1500

aagtttttat gcaataagcc ttcctttcag gtctctaaaa gctcctgctc tgatctgtgg 1560

tttaacactg tgcagggctg tggagctctg agagacctga acccctaccc atcccctgca 1620
cctccctact ctccctgccg aggcgtccat agcatttccc taataaatag ttttatcagg 1680
gacactccat ggggtggctt gtctctgccc caaacagggt cttcacgttc taactgcagg 1740
gaagagactg gttactagcg taaagctgac aagttaccag ttcacctgat cagaatgtat 1800
tttttataac caccatcatt ccttgtctct tcagtggaag atattctttc ctgtttccca 1860
gaagagagaa ataaaagtcc taaataacta agaatgatca gcgggagcgt tgggcatgat 1920
tactgcagtg tttgttcttt attaaagaca gggagtcgtg gctgtctcta cataatacta 1980
acatttcagt gtaaaaagta caattggtta tttggtacgt gtagatttaa cacccagacg 2040
gctgacttgt aaccctcccc actagccttc tgagcattta aacccagccg tcattctctc 2100
cccactccac gctccccact cctctgccac gttatgttac ggcacaaaat taatttctgc 2160
ccctgtgatt gggccacggt tgccaaggta acagtggagc tggagctaac ttcctccttt 2220
catcctttcc aattttctat tccagaagac agtcagaata atgcatctgt actacatcct 2280
gccttttgaa cctaaatgag tttcgttgat gaaatgtgcc tctctgattc atcacaaact 2340
gtggtgttct gaccacctgt gacgaggggg tcataatact tccagtgatc cttttaattt 2400
agcaaaatat ttgtcggtgg agggaagtag ataagaatgt attagtgtat tttaaagtaa 2460
taatgattaa agaataacta aatgactctg attttgtttt atcaatcata atgtgttaaa 2520
gccctctgtg gctgccacaa aagactagaa tcatgaaatc tttgtccaag ctaagaaagg 2580
gctgtctctg tgggtacagt ggcattctct gttgacctaa ccaagcagcc tgaaccttgc 2640
tttgtgtctc gtaaaggtca tctcacggaa tccaattgct gctacctcac catgttgctg 2700
tgccctggtc aacaataaac atactttttt ccccccttcct aagtaacttt gagaggacaa 2760
gataggcaaa gtttttttccc tacatctctt ttggccaaaa gaatatctga ccatcacaga 2820
gatccagaga acagaaacaa gagcccctaa gtcccagcct ctgcaataca cagttaccag 2880
atgggaatgc agcacagtgg cacagagagg taactacgga gcccatgccc tggaggaact 2940
ctgtagctgt gcatcatgga acgccacagg atttttacat taaaaagttg aatatgttcc 3000
tgcatttcag cagtgcataa tcgagcagga ggctggaatc tggggacctt tgttattcat 3060
tctttttaag gttatttttt attaactgtg catatgaaat ttccactcaa tttaaaaatc 3120
caaatctttt taagcttctt ggaagaattt cctcctgctt atattgttaa ttgcactaat 3180
gaactcttct agggtaaatt ctggcaaact acccttttt tttttttaat cacagaatat 3240

```
tcctgttccc acagaacttt ggcacattct gaagaaaatc tctgcagtca atttttatct   3300
cctctgcttg actgcatcta actgttcatc tctgttataa atggagcttc agccctctgg   3360
gtcagagact gacaaccctc cttcttcttc ctaacctgtc ctactctcct tctttttctg   3420
ctttcatttt aattttggga aattttaaag taaaagtttc aaagttgaaa gattatagtg   3480
agtaccctta tgtcctttag tgcatcagtt gttaatgttt tgccttgttt tcttttcttc   3540
cttttttttt ttaagagaag gggtctctcc ctattttacc caagctggcc ttgaacccct   3600
tgcctcaagt gagcttcaac cttagcctcc tgagtagcca ggactatggc atgtaccacc   3660
gcacctgcct cacattttct ttctcactgt aaatacacat tcttattctt gaaccttctg   3720
aaaataagtt gcagacatca tgaaaatgta aaatacttca tcatattctt tcttttaata   3780
agagcattgt cctataaatc tgcaatattt tcacgcccat gaaactgaac actgatactg   3840
cataaatcta atatatgatc catattcaaa attctccagt tgtccccaaa atatccttta   3900
tagctgttta tttttaaatc caggatccaa tcaagaatta tacgttatat gtagttgtca   3960
ttctcttttg tttcctttaa ttgattagag ttgccctttt gtgtgtgtct gagtggtggg   4020
tggtggggtg gtgtttgtgt atgatgtgca gtgtgtgtgt gtgtggtgtg tctttcatga   4080
cactgacatt attaaagagt tcaggcaagt ttgtggaatg tcccacaatc tagacttttt   4140
tactgattgt ttcctcatca cgagatacag gttacatttt ttttcccaag actgctacat   4200
gtgtgatgtt gtttccttac tactgcctca cctcaggaag cacataatgt ctacttgtct   4260
cttttctgat attaggactg atcaggtgtt gtctgcctga tccaatcatt atcaagttcc   4320
ataattcatt attaagctct gaaattcagg acacatacat ttatagagtg tgaggctgtg   4380
catacgtaca ttcatccctt aatgttctca aggaggccca ggatccccaa aatattataa   4440
gcattgcagt cagatgatat agcactagca aagcattcat ccctttccat ataacaggag   4500
ggaggggaag aaggaagttt ccatgtgcag gcaatacgtg agagcatggg caagtgagga   4560
tttgtatgct ccctgtagcc agaggtgaaa tattcaacgc ttttattttt tctcccgtct   4620
tcaatgacct cccatttgga cccatagttt atttatagag agcacacact ccctaattgt   4680
cagatggttt ttttgggaaa actcttagat agtacagtga gttgtgaccc tccaaaggat   4740
cacaagtaca taaacagata caaagtattt atgtggtatt aaaaattcat ggtgatggtg   4800
caagggagag atgattagga agaaaatgtc ttaaacggct gtaattgaga aatactgatc   4860
tagagtcatt tcctgcttct taaattgttc gttttctctt ccctgggtat tttctcattt   4920
ccaaagaccc agagtctcag agccagttta agtggaagaa actttgagag gtttcttaat   4980
```

caattccttc tatctctcaa accatgccag taacttcctt ggtgaaaaaa tctatactcc 5040

tctcacagtt cttcgggcat ggaaattcta tagcattctt tggtataacc aacccactga 5100

accctccaca acatcttgaa ttccattcaa ctcgagctga agtattcttt ttgagtttca 5160

gtatatcttt tctagtgtcc cacttttaga aaggtccaaa gatgatatat aataagtgaa 5220

aatacaatta atcagagctt tatttgcata tccattaaat atctgaaggc tattaccagt 5280

tgtgcttcag cctcctttcc cttgcaattc tcccagggtg gcatcctcta cggagtcttg 5340

ttgctccatt gttgaccact gatgtaactc aacgtgaaac cttcccaaag ctctccacat 5400

cactctctgt tatggctgtg accagcagtg aacatgaaaa gaacttattt catgccttct 5460

ccaaactata ttgtttcttt gctaagtcca gacctgcttt ttctacattt ataggttttc 5520

tattttaatc gccatgcaaa attctttatc atactcaatc attttggttt atcttcctga 5580

cttcttacct ttaacatccc tgttaaagat tacactgttg ttgtttcaga aaaaaaa 5638

<210> 27
<211> 4221
<212> DNA
<213> human

<400> 27
cagcccgagc ccgagcccga gcccgagccg gcgccaccgc gcccccggcc atggcttttg 60

ccaatttccg ccgcatcctg cgcctgtcta ccttcgagaa gagaaagtcc cgcgaatatg 120

agcacgtccg ccgcgacctg gaccccaacg aggtgtggga gatcgtgggc gagctgggcg 180

acggcgcctt cggcaaggtt tacaaggcca agaataagga gacgggtgct ttggctgcgg 240

ccaaagtcat tgaaaccaag agtgaggagg agctggagga ctacatcgtg gagattgaga 300

tcctggccac ctgcgaccac ccctacattg tgaagctcct gggagcctac tatcacgacg 360

ggaagctgtg gatcatgatt gagttctgtc caggggggagc cgtggacgcc atcatgctgg 420

agctggacag aggcctcacg gagccccaga tacaggtggt ttgccgccag atgctagaag 480

ccctcaactt cctgcacagc aagaggatca tccaccgaga tctgaaagct ggcaacgtgc 540

tgatgaccct cgagggagac atcaggctgg ctgactttgg tgtgtctgcc aagaatctga 600

agactctaca gaaacgagat tccttcatcg gcacgcctta ctggatggcc cccgaggtgg 660

tcatgtgtga gaccatgaaa gacacgccct acgactacaa agccgacatc tggtccctgg 720

gcatcacgct gattgagatg gcccagatcg agccgccaca ccacgagctc aaccccatgc 780

gggtcctgct aaagatcgcc aagtcagacc ctcccacgct gctcacgccc tccaagtggt 840

ctgtagagtt ccgtgacttc ctgaagatag ccctggataa gaacccagaa acccgaccca 900

gtgccgcgca gctgctggag catcccttcg tcagcagcat caccagtaac aaggctctgc 960

gggagctggt ggctgaggcc aaggccgagg tgatggaaga gatcgaagac ggccgggatg 1020

agggggaaga ggaggacgcc gtggatgccg cctccaccct ggagaaccat actcagaact 1080

cctctgaggt gagtccgcca agcctcaatg ctgacaagcc tctcgaggag tcaccttcca 1140

ccccgctggc acccagccag tctcaggaca gtgtgaatga gccctgcagc cagccctctg 1200

gggacagatc cctccaaacc accagtcccc cagtcgtggc ccctggaaat gagaacggcc 1260

tggcagtgcc tgtgcccctg cggaagtccc gacccgtgtc aatggatgcc agaattcagg 1320

tagcccagga gaagcaagtt gctgagcagg gtggggacct cagcccagca gccaacagat 1380

ctcaaaaggc cagccagagc cggcccaaca gcagcgccct ggagaccttg ggtggggaga 1440

agctggccaa tggcagcctg gagccacctg cccaggcagc tccagggcct tccaagaggg 1500

actcggactg cagcagcctc tgcacctctg agagcatgga ctatggtacc aatctctcca 1560

ctgacctgtc gctgaacaaa gagatgggct ctctgtccat caaggacccg aaactgtaca 1620

aaaaaaccct caagcggaca cgcaaatttg tggtggatgg tgtggaggtg agcatcacca 1680

cctccaagat catcagcgaa gatgagaaga aggatgagga gatgagattt ctcaggcgcc 1740

aggaactccg agagcttcgg ctgctccaga aagaagagca tcggaaccag acccagctga 1800

gtaacaagca tgagctgcag ctggagcaaa tgcataaacg ttttgaacag gaaatcaacg 1860

ccaagaagaa gttctttgac acggaattag agaacctgga gcgtcagcaa aagcagcaag 1920

tggagaagat ggagcaagac catgccgtgc gccgccggga ggaggccagg cggatccgcc 1980

tggagcagga tcgggactac accaggttcc aagagcagct caaactgatg aagaaagagg 2040

tgaagaacga ggtggagaag ctcccccgac agcagcggaa ggaaagcatg aagcagaaga 2100

tggaggagca cacgcagaaa aagcagcttc ttgaccggga ctttgtagcc aagcagaagg 2160

aggacctgga gctggccatg aagaggctca ccaccgacaa caggcgggag atctgtgaca 2220

aggagcgcga gtgcctcatg aagaagcagg agctccttcg agaccgggaa gcagccctgt 2280

gggagatgga agagcaccag ctgcaggaga ggcaccagct ggtgaagcag cagctcaaag 2340

accagtactt cctccagcgg cacgagctgc tgcgcaagca tgagaaggag cgggagcaga 2400

tgcagcgcta caaccagcgc atgatagagc agctgaaggt gcggcagcaa caggaaaagg 2460

cgcggctgcc caagatccag aggagtgagg gcaagacgcg catggccatg tacaagaaga 2520

```
gcctccacat caacggcggg ggcagcgcag ctgagcagcg tgagaagatc aagcagttct   2580
cccagcagga ggagaagagg cagaagtcgg agcggctgca gcaacagcag aaacacgaga   2640
accagatgcg ggacatgctg gcgcagtgtg agagcaacat gagcgagctg cagcagctgc   2700
agaatgaaaa gtgccacctc ctggtagagc acgaaaccca gaaactgaag gccctggatg   2760
agagccataa ccagaacctg aaggaatggc gggacaagct tcggccgcgc aagaaggctc   2820
tggaagagga tctgaaccag aagaagcggg agcaggagat gttcttcaag ctgagcgagg   2880
aggcggagtg cccaaacccc tccaccccaa gcaaggccgc caagttcttc ccctacagtt   2940
ctgcggatgc ttcttaacaa ccgcccgggg ctgtggctgg cagcttggtg ggccccaggg   3000
ccttctcctt cattctctgt gaacatgtaa ctcaggaccc cttttccctc ttgcgtctgt   3060
gccagctcaa atccagcccc tcgccctgtg ccaccccaac tgtgcctgat agacctgccc   3120
cagcgttcct gacttcttgc tggcctgtgg agggtgaggt gtaattattt gtcacctgaa   3180
cctaatgtat attctccttg agccccagat cccttcaagc tggaagggat ggggctgttg   3240
gtggggtcag ggtccaagag gaatgggtgt tctgtggcct cgagtcctct cctgtttgcg   3300
aaaataccag ttttgctctc tgtgggacaa agcactgctg atgaagtccc cgtgggctca   3360
tccgggctgg aattcttggt tttcagcca ttccctgcag agtcactcaa tcatcaagtc   3420
cctcacagcc atttctgttc ccagaggagc caggcctgca gctggctgct caggagatgg   3480
cctcattcct cctgttctcc cagtttgctt tccacttaag acaaagcctt gtctatgtgg   3540
ggggcgggga ccggggaaag agggaggctg aaatgtttat tctgcttctc ccgtgtttca   3600
tgccatctcg cgtcccccctt cctgcacatg ggtgtgaatg cacacacata cgcgtacaca   3660
caggacttgg tctgctggcc tggcctcttc tgcccaggtg ggttggaaca cgtttgctgc   3720
ctgagcctgt gccactgagc atgttaggtg gagcagttgg tgtggcacgt gcggggtgtt   3780
ggcaccggag gcatggaaaa gcacaggctg tactgccagg ctgcgatgcg tgctggcccc   3840
cgcacaggct cctgtgtgca gggactgatt cctcagcaca cgaggcttcc acaacccagt   3900
ctgctccata gcactctggc ccaccctgtc tgcaggtgaa acaggagggc tgtttgcctc   3960
tgccccatcc ccccgactgt gttcaggagt cccaccttgc atttcagacc tgggctggca   4020
gtctgttgga cttctcttca ggaagaaaaa gcatcagggg gaaatggaat gcccctgccc   4080
caggaacatg gcagaagcac aggttctgta cctcagatgg actcctgctg ggccttcggg   4140
gtctcagttg gcttccccca gattctgatt ctacagctgc agaatgtata taacacaata   4200
aaagcaaatg tttgaaccag t                                             4221
```

<210> 28
<211> 1940
<212> DNA
<213> human

<400> 28
ggggcggtgc agaggcggca ggaagatgga gttggggagt tgcctggagg gcgggaggga 60

ggcggcggag gaagagggcg agcctgaggt gaaaaagcgg cgacttctgt gtgtggagtt 120

tgcctcggtc gcaagctgcg atgccgcagt ggctcagtgc ttcctggccg agaacgactg 180

ggagatggaa agggctctga actcctactt cgagcctccg gtggaggaga gcgccttgga 240

acgccgacct gaaaccatct ctgagcccaa gacctatgtt gacctaacca atgaagaaac 300

aactgattcc accacttcta aaatcagccc atctgaagat actcagcaag aaaatggcag 360

catgttctct ctcattacct ggaatattga tggattagat ctaaacaatc tgtcagagag 420

ggctcgaggg gtgtgttcct acttagcttt gtacagccca gatgtgatat ttctacagga 480

agttattccc ccatattata gctacctaaa gaagagatca agtaattatg agattattac 540

aggtcatgaa gaaggatatt tcacagctat aatgttgaag aaatcaagag tgaaattaaa 600

aagccaagag attattcctt ttccaagtac caaaatgatg agaaaccttt tatgtgtgca 660

tgtgaacgtg tcaggaaatg agctttgcct tatgacatcc catttggaga gcaccagagg 720

gcatgctgcg gaacgaatga atcagttaaa aatggtttta aagaaaatgc aagaggctcc 780

agagtcagct acagttatat ttgcaggaga tacaaatcta agggatcgag aggttaccag 840

atgtggtggt ttacccaaca acattgtgga tgtctgggag tttttgggca aacctaaaca 900

ttgccagtat acatgggata cacaaatgaa ctctaatctt ggaataactg ctgcttgtaa 960

acttcgtttt gatcgaatat ttttcagagc agcagcagaa gagggacaca ttattccccg 1020

aagtttggac cttcttggat tagaaaaact ggactgtggt agatttccta gtgatcactg 1080

gggtcttctg tgcaacttag atataatatt gtaaaatgct tttcaagtgt gggttttgcc 1140

ctgattgttg caaatacaat ttccaccttc tggaaaggta ggtttgctgt ggaggaaata 1200

atgtactaga tcattgtcac agaaaaacca actatgattt atggttgtgt tttcagaatt 1260

caacattaaa gattaatgtt tatttaaacg aacacattcc tgcattcagg atgtgaggcc 1320

atttaataaa aagggcacaa agcctgtcag agttttcaac ggtgcttata gctgccagct 1380

ggattccaaa caggtaccac attgtctctg agctaatgtt tatatttttc cattcaggca 1440

ccgaaatagt taatatttga aataagtctt caaaagaaaa cataagagat tattgagttc 1500

```
ttgggactgg atcctttatt tcataagttc agatcatctt aaatgaaaat gccatgatta   1560
tctgcagtta agtagatgac agctattcta catcagactt gatttttgtc agctaattac   1620
ataattggta agctataatt gaaaccttat ggcttaaaat tccttaactc ctttttgatt   1680
catgtttgta gtcatgttgt caacagaggc aaagttaagc ttgatgatgg ttaaaatcgg   1740
tttgatagca ccatgggaca tttttctaac aaaaataaat gcatgaagag acatagcctt   1800
ttagttttgc taattgtgaa atggaaatgc tttacaggaa gtaaatgcaa attactttta   1860
agtgtgcttt aaagaaaaat attttcccca caagagaaat ttaaataaag aattttattt   1920
gtttaaaaaa aaaaaaaaaa                                                1940

<210>  29
<211>  2367
<212>  DNA
<213>  human

<400>  29
gcggccgcga tggggccgaa gcgcccgaag ccccggagcc cacaaactgc cgggcccgcc     60
tcgccgccgg gacccgggtg cctgggctcg gcttgaagcg gcggcggcgc accggcacag    120
ccgcgggagc atgggcagga ggatgcgggg cgccgccgcc accgcggggc tctggctgct    180
ggcgctgggc tcgctgctgg cgctgtgggg agggctcctg ccgccgcgga ccgagctgcc    240
cgcctcccgg ccgcccgaag accgactccc acggcgcccg gcccggagcg gcggccccgc    300
gcccgcgcct cgcttccctc tgcccccgcc cctggcgtgg gacgcccgcg gcggctccct    360
gaaaactttc cgggcgctgc tcaccctggc ggccggcgcg gacggcccgc cccggcagtc    420
ccggagcgag cccaggtggc acgtgtcagc caggcagccc cggccggagg agagcgccgc    480
ggtgcacggg ggcgtcttct ggagccgcgg cctggaggag caggtgcccc cgggcttttc    540
ggaggcccag gcggcggcgt ggctggaggc ggctcgcggc gcccggatgg tggccctgga    600
gcgcgggggt tgcgggcgca gctccaaccg actggcccgt tttgccgacg gcacccgcgc    660
ctgcgtgcgc tacggcatca acccggagca gattcagggc gaggccctgt cttactatct    720
ggcgcgcctg ctgggcctcc agcgccacgt gccgccgctg gcactggctc gggtggaggc    780
tcggggcgcg cagtgggcgc aggtgcagga ggagctgcgc gctgcgcact ggaccgaggg    840
cagcgtggtg agcctgacac gctggctgcc caacctcacg gacgtggtgg tgcccgcgcc    900
ctggcgctcg gaggacggcc gtctgcgccc cctccgggat gccgggggtg agctggccaa    960
cctcagccag gcggagctgg tggacctagt acaatggacc gacttaatcc ttttcgacta   1020
```

cctgacggcc aacttcgacc ggctcgtaag caacctcttc agcctgcagt gggacccgcg 1080

cgtcatgcag cgtgccacca gcaacctgca ccgcggtccg ggcggggcgc tggtctttct 1140

ggacaatgag gcgggcttgg tgcacggcta ccgggtagca ggcatgtggg acaagtataa 1200

cgagccgctg ttgcagtcag tgtgcgtgtt ccgcgagcgg accgcgcggc gcgtcctgga 1260

gctgcaccgc ggacaggacg ccgcggcccg gctgctgcgc ctctaccggc gccacgagcc 1320

tcgcttcccc gagctggccg cccttgcaga cccccacgct cagctgctac agcgccgcct 1380

cgacttcctc gccaagcaca ttttgcactg taaggccaag tacggccgcc ggtctgggac 1440

ttagtgtcac cgggaggaaa agagagagat ctggggctgg ggtatggatg atggggggaa 1500

gggcggtcgc ctctgccact gtcagggacc agccggccaa cgcccacccg caaaggtgtc 1560

taaaaacttc agcttttcac ccacctgccc ctttctttca atcccacgct gtttcctttc 1620

aaagttctgg gaggacgaac tcaccgaggc gagaagtgta acattctctc cacccagctt 1680

ataaaaggat tctttactgt gccagcacgg ggattggatc cgaagaaact ggctactggg 1740

gtttggcccc cgagtggccg tccctgtggg agatgcaccc cattcttggg ccccccctcat 1800

tccctttccg aaaaaggaaa acttgcgttt gagccgttga gctaattctg caattttcta 1860

ccaaacagag cgctggtggc cccggagcag ggctgtgaca ttggctggtg gagccccttc 1920

ctgtgttctc cctttgttcc agcgccgcga tggtgagatc actgttccaa gcagggggac 1980

ggctcgcgat aggacaaaga gagcaggacc tccagactct ggggagccct gcagaccttg 2040

acaatttgcc tgactcattc ctgacctctt gtcattttgg cctgaaggct acaaattcag 2100

ggtcagctgt atgcactaag tcaaataatg aatttcttcc tccctctcgc aaccgaccaa 2160

aattttgaca acgatgatgt tcaccagaag gaaaaaaaaa tcagttttat gcactttatt 2220

ttgttttgat tttcattttt tattaagaaa aaattttatt ttacagaatt taccttctct 2280

gtatatatgt gcataaagtg tggtgtaaat atactaaaca aacttatatt tcaataaaag 2340

ggagtttaaa atttaaaaaa aaaaaaa 2367

<210> 30
<211> 548
<212> DNA
<213> human

<400> 30
tttaatttat aaatcagttt atttgcagat tttatctttc ttaaagtata tttcagttaa 60

caaatctatt tacacaggta tacatgggaa ttcatcccag ttattttaca gatcgtactt 120

acaaaccaca cccttaagtg ttcggtgcaa aattgcactg gatgtgacta ttggctgaaa 180

atggcctgat gaccaggcta tttacacgca cacagtaaac gggtttattt tttcatgtct 240

gcacagtaca gtacacacaa ggaggctgaa cctcccagag aatgctttgc aatgtgtttg 300

ccaagaaggc tttgcaaagt ccagaaggaa aagctattaa acataggtta attaaaatga 360

cagtacctcc tttacatcag tttacatttt ttcttcacat ttttataata caaagatatt 420

ttattgaatt gctgcacaac cagttgaaga tgatttgtaa acatgtaaat tcctacaatt 480

tgcattaaat gtcattgtag tttctataat ttgctctttg aattaatgtt ttattcacta 540

gtattctt 548

<210> 31
<211> 654
<212> DNA
<213> human

<400> 31
ggatgccaag ttcttttagt aattcaccag ctccatgcag ggacatcaca gggctgccct 60

ccatgagcag aggaggaggg ctgcctggca gagcgtttca cactccaggt tagccagaaa 120

gagcatcttc atttttgttt ccacacaaca cttctctgtg agcctgttgg ccaacaaagt 180

ggcggccgat tgttggagga gccacccaac catcttggct aaccttaaat tcttcagggc 240

tagaatatgt tcaccccaga ggcttagatg aagcacattt gcggctactc gggcagatgg 300

tctcttgctg gcctctcgct ggagcagtgc cctcaccaac tgtctcacgt ctggaggcac 360

tgactcgggc agtgcaggta gctgagcctc ttggtagctg cggctttcaa ggtgggcctt 420

gccctggccg tagaagggat tgacaagccc gaagatttca taggcgatgg ctcccactgc 480

ccaggcatca gccttgctgt agtcaatcac tgccctgggg ccaggacggg ccgtggacac 540

ctctggggcc atcaaacagc cgttttcgcc ccgatccacg taccagctgc tgaaaggcaa 600

ctgcatgccg atgctctcat cagccagaca gcaacccaaa tctgcgatca ccag 654

<210> 32
<211> 610
<212> DNA
<213> human

<220>
<221> misc_feature
<222> (448)..(448)
<223> n is a, c, g, or t

<220>
<221> misc_feature
<222> (479)..(479)
<223> n is a, c, g, or t

<220>
<221> misc_feature
<222> (528)..(528)
<223> n is a, c, g, or t

<220>
<221> misc_feature
<222> (548)..(548)
<223> n is a, c, g, or t

<220>
<221> misc_feature
<222> (556)..(556)
<223> n is a, c, g, or t

<220>
<221> misc_feature
<222> (592)..(592)
<223> n is a, c, g, or t

<400> 32
tttttatatt cgtaaacata gcattttatt aacaaaatgt ttacaccttt cttacaacaa 60

aacagtgatt tagcatgtta tactctataa aagtgtatta tagaatattc aagctaaaac 120

aatattattt aaaagtatgg caaatcaact ttcatttgca aactatctac tatgagcata 180

ggcatgaaaa caaaaatctt acaacaaaaa acaaaaggtc tagttttctt ttgtcagcat 240

ctataaacaa tacagacatc atatggttta catacactta ggtccagccc aaggttaaga 300

ctgcaaaaat aaagtcttca aacatccaca gaagcttggc aaaccaccac tgcagcattc 360

catgacaggt aaccaggcca tgtgtcatgc agtaagtcca aggccataaa tttgggctgt 420

ttataatagt gctccaaagc ggccaaantg taagcactga attttcttcc gtaatagant 480

attataaagg cccaatactt attcttcaca ggcttgagaa gctgcatntt ggagaagaga 540

gatctggnag cgcttncttt ttttgaaagc taggcatgca agtgttctca gnttaattgg 600

cttccgtaca 610

<210> 33
<211> 4515
<212> DNA
<213> human

<400> 33
ggtcagcgcc caggtcctgg gctggccgcc gggatgttgc cctacacagt gaacttcaag 60

gtgtcggcgc gcaccctcac gggggccctc aacgcccaca acaaggcggc ggtggactgg 120
ggctggcaag gtttaattgc ttatggatgt cattcacttg tggtagtgat tgattccatt 180
actgcccaaa ctcttcaagt tttagaaaag cataaagctg atgttgtaaa ggttaaatgg 240
gccagggaaa actatcacca taacattggc tcaccatatt gcttacggtt agcttctgct 300
gatgtcaatg ggaagatcat cgtctgggat gtagcagcag gagtagctca gtgtgagatc 360
caagagcatg ccaagcctat ccaggatgtt cagtggttgt ggaatcaaga tgcttcccgc 420
gatttactgc ttgctatcca cccgccaaat tacattgtgc tctggaatgc cgacactggc 480
accaaactat ggaagaagag ctatgcagat aacattcttt ctttttcttt tgaccctttt 540
gatccctcac atttaacttt gcttaccagc gagggtattg ttttcatctc agacttctcc 600
ccatccaagc ctccctcagg ccctgggaaa aaagtttaca tatccagccc acactctagc 660
ccagctcata acaagctggc cacagccaca ggtgccaaga aagctctaaa taaagtaaaa 720
attttaatca ctcaagagaa acctagtgct gaattcataa ctctcaatga ttgccttcag 780
ttggcatacc tgccttcaaa aaggaatcac atgttgttgc tctatcctcg agagatttta 840
atccttgacc ttgaggtgaa tcagacggtg ggtgtgattg caatagaacg cacaggagtt 900
ccatttttac aggtaatacc ctgctttcag cgtgatggtt tattttgtct acatgaaaat 960
ggttgtataa ctttacgtgt tcgaagatct tataataaca tttttaccac ttcaaatgag 1020
gaaccagatc cagatccagt tcaggagctt acctatgatt tacgaagcca gtgtgatgca 1080
atcagggtga caaaaaccgt ccgtcccttc agtatggtgt gctgtcctgt caatgagaat 1140
gcagccgccc tcgtagtgag tgatggcagg gtcatgatat gggaactcaa gtctgcagtt 1200
tgtaatcgaa attcacggaa cagtagttct ggtgtgtcac ctttatattc accagtgtct 1260
ttctgtggaa ttcctgtagg agtgctacag aataaactcc cagacctttc cttagataac 1320
atgattgggc aaagtgcaat tgctggggaa gaacatccca gaggttcaat tctgcgggaa 1380
gtgcacctca agttcctgct gacgggactg ctttcaggac tgcccgcacc acagtttgct 1440
attcgtatgt gtccaccgtt gaccacaaaa aacatcaaga tgtatcagcc actgctggct 1500
gttggtacaa gtaatggttc tgtcctggtg taccatctca ccagtggtct gctacacaaa 1560
gagttaagca tccactcatg tgaagtcaag ggtattgaat ggacaagttt gactagtttt 1620
ctttcttttg ctacctcaac accaaacaat atgggattag tgagaaatga acttcaactg 1680
gttgatcttc caacaggtag gagcattgct tttcgtggtg aaagaggcaa tgatgaatct 1740

```
gccatcgaaa tgattaaagt atctcatttg aagcagtatt tggcagtcgt attcagagat   1800
aaacccctgg agctatggga tgttaggact tgtacccttc ttagagagat gtccaaaaac   1860
ttccctacaa taactgcttt ggagtggtca ccatctcaca acttgaagag cctgagaaag   1920
aagcaacttg ctactcgaga ggccatggcc cgccagaccg tagtctcaga cacagagctg   1980
agtattgttg aatcatctgt gatcagcttg ctgcaggagg cagaaagtaa atctgaactt   2040
agtcagaaca tctctgcccg ggaacatttt gtatttaccg atattgatgg ccaagtgtat   2100
catctcactg ttgaaggaaa ctcagtaaaa gacagtgctc ggattccacc agatggaagt   2160
atgggtagta ttacctgcat cgcttggaaa ggtgatacat tagtgcttgg agatatggat   2220
ggaaatttaa atttctggga cttgaaaggc agagtatcca gaggaatacc cacacaccga   2280
agttgggtga ggaagattcg ttttgctcct ggtaaaggaa atcaaaaatt aatagcaatg   2340
tacaatgatg gagctgaagt gtgggatact aaagaggttc agatggtgag cagtttaaga   2400
agtggcagaa atgtgacctt tcgtatattg gatgtggact ggtgtacgtc agataaagtg   2460
atcttggcct cagatgatgg gtgcatcaga gtcctagaga tgtctatgaa gtctgcgtgc   2520
tttagaatgg atgaacaaga gttaaccgag cctgtgtggt gcccctatct ccttgttcca   2580
agggcctctc ttgccttgaa agccttctta ttacaccagc cttggaatgg acagtattct   2640
ttggacattt ctcatgttga ctatccagaa aatgaagaaa taaagaatct cctccaagaa   2700
cagttgaatt cattgtctaa tgacataaag aaactgttgc ttgatccaga attcactctc   2760
ttgcagaggt gcctgcttgt ttcaaggctc tatggtgatg aatcggagct gcacttctgg   2820
actgtcgctg cccactacct gcacagctta tcccaggaaa agtcagccag cacaacagct   2880
cctaaagaag ctgctcctcg agacaaactg agcaacccac tggatatatg ctatgacgtg   2940
ctctgtgaaa atgcctactt tcagaaattt cagctagaaa gggttaatct acaggaagtg   3000
aaacggtcaa cttatgatca tacaaggaaa tgtacagacc agctactgct cttgggtcaa   3060
acagacagag ctgtgcagtt gctgttggaa acaagtgcag ataaccagca ttattactgt   3120
gattcactga aagcctgttt agtcactact gtcacctcgt caggcccctc tcagagcacc   3180
attaagttgg tggcaacgaa tatgattgcc aatggcaaat tggcagaggg cgttcagttg   3240
ctctgcctga tagataaggc tgcagacgcc tgccgctacc tgcagacata cggcgagtgg   3300
aatcgggctg catggctggc aaaagtccgt ttgaatcctg aggagtgtgc cgatgtttta   3360
aggcggtggg ttgaccacct ttgttctcca caagtcaatc agaaatcaaa ggctctcctg   3420
gttctcctct ctctgggctg ctttttagc gtggcagaga cgcttcacag catgagatac   3480
```

tttgatagag cagccttatt tgtggaagct tgcctcaagt atggagcatt tgaagtcact 3540

gaggacacag agaaactcat cactgctata tatgcagatt atgcccggag tttgaagaac 3600

ctcggtttta agcagggagc agttctcttt gcttcaaaag ccggagcagc tggcaaagac 3660

ttattgaatg agcttgagtc cccaaggaa gaacccattg aagagtgaca gcttaataaa 3720

tgccagggaa tctgacctgg aaggcagatg ggaggggct ggtctggctg tggccaccgt 3780

cacagtccag gatgaagagg agtacagggt cctgtgagct gtttgaccac tgttctaaga 3840

ctatgtgtgc ccaaaagcac ataagcatct atgttgagag taagtttgta tcctgcgttg 3900

gtctcagaaa gaacgtgaat gcttaagatt ttgaaagtac ataatatttt atactttggg 3960

agagagcttt aagagtccct ggaaatactt tttaattttt ttaacttaaa attcaagaga 4020

ctgaatcact tttctcattg attaaatgta aagattattg agaaacctat agtaaatgaa 4080

atttgtaaga tgttttctca aatatatgct gtgcctgtac ttatatacag tctttcaaga 4140

gagatacaaa caaggcagaa acatttaaac tagtattaaa ggtagtttac caaagcattt 4200

tttgttttct taccttgaaa acacagaacc gttaattcct tggtttaagc agttgctaag 4260

ttttttaatt ttaggctcag aggcccatag gaggttttaa gatttatgtt tagtccgata 4320

ggtgaggtct ttgatatttt gaattttaac tccttttatg atacatcaca gtaacctcat 4380

ttttgaagtc tttctttgta ctttaatgtt ctctctgttc taatagttga agtatgagat 4440

gtaactatta taaactgttg ctgaaaacat aaatgtctgt aacttacaaa catgataaat 4500

aaattaaaaa ttcca 4515

<210> 34
<211> 175737
<212> DNA
<213> human

<400> 34
gatctgcccg cctcagcctc ccaaagtgct gggattatag gcgtgagcca ctgcgcctga 60

cctttttttt tttaaatctt ttgagagaga cgtagtcttg ctctgtctcc caggttggag 120

tgcagtggcg tgatctcggc tcactgcaac ctccgcctcc cagattcaag cgattctcct 180

gcctcagtct cccaagtagc tgggattaca ggcacctgcc atcatgccca gctaattttg 240

tattttttttg tagagacggg gttttactgt gttggccagg ctggtcttga actcctgacc 300

tcagatgatc tgcccgcctc ggcctcccaa agtgttggga ttacaggcgt gagccactgc 360

gcctggccca cacattttta ggttataaaa ttaaacgtaa tatggccagg tgcggtggct 420

```
cacgcctgta atcccagcac tttggaggcc aaggcgggtg gatcgcctga ggtcaggagt     480
ttgagaccag cctggccaac atggcgaaac tctgtctcta ctaaaaattc acaaaattag     540
ccgggtgtcg tggcgggggc ctgtaattcc ggcaacttgg gaggctgagg caggagaatt     600
gcttgaacct gggaggcagg ggttgcagtg agccaagact gtgccattgc actccaacct     660
gggcaacaag agcaaaactc cgtctcaaaa acaaacaaac aaacaaaaca taatatgaga     720
ctggacacag tggctcatgc ctgtaatctt aacagtttgg taggctgagg tgggcagatc     780
acttgagccc agaagttcga aacaagccat gtcacccatg acatggcaaa actctgtctc     840
tacagaagat agaaaaatta gccgggtgtg gtggtgcatg cctgtagtcc cagctactca     900
ggaggctgag gtgatcctcc cacctcagcc caggaggtta aggctgcagt gagctgtgat     960
catgccactg cactctagca tgggcaacag agtgagaccc ggtctcagaa aaaaaaaata    1020
ataataatca aatatatttg tgtaatacag atctactaat gggaagaact gaatttctct    1080
ttttgaggtt aacattttgc ctaattgatg tacaaagtta gtgttccaga tggtcaaatt    1140
tgactgtaga tattcatgtt catgctgatc tgtagagatt gcaagtattt catctttgaa    1200
aacatctttt cacacaggta atgataggtg atatgtgagg tgcttgaaat gctgtgaagc    1260
acttacgact gtgtcactgt gacttgtagt gtacaaagca gcagtgcaaa tcaggatgct    1320
gtagtcgctg tcgtgaccac tcggctgtgt gttgtagagc aaaagcagcc acagtattaa    1380
gtaaatagtg tggccccgtt ccaataaaac tttatttgtt ggatattgga atttgaattt    1440
catacggttt tcacagtctc aaaatattct tttgattttt tttcaaccac ttaaaaatgt    1500
aaaaaccatt cttggcttgt gggctataca gaactagatg atgggccagg tttggcccat    1560
gggcagtagt ttaccaagcc ttggtttaaa gccctcatat aagctgttgt agacattaag    1620
atgagttaag gcatatagtt tagcacagcg cttagcaaaa tagggagcgc tgtgttcatc    1680
attgtcattc aagatgatcg ttctctccag gtctggctga tgtgaggggt ggaggtggtg    1740
tctgctttgg atttctgctg ttcctgaggg agtatctgca ttttccacag cttttctgtc    1800
tgatttgtat tttcctctga ttccttttgt catcaggtat tcactgggca cctgctgtgg    1860
gcagggctct gcgctgaggt tctggagaca aaaggatgaa tcgttgagcc tgccctggtg    1920
tggtgctccc gttatccttt aggtataaaa acttgtggct attttttttt tttttttgag    1980
acagagtctt gatctgtcac ccaggctgga gtgcagtggc acaatctcag ctcaccacaa    2040
cctctgcccc ccggggttca agcgattctc ctgcctcagc ctcctgagta gctgggatta    2100
```

caggcgccca cgaaccacgc ccagttaatt ttttaatgtt tagtagagat ggggtttcac 2160
catcttggcc aggctgatct tgaactcctg acatcgtgat ccacctgctt cggcctccca 2220
aagtgcaagt gttgggatta caggcgtgag ccactgcacc cggccatggc tatggttttt 2280
gagaatgatt ggccaggtga tgcatttatt tattttatta ctatttttcg agacggagtc 2340
ttgatctatc acccaggctg gagtgcagtg gcgcgatctc ggttcattac aacctccgcc 2400
tcctaggctc aagtgattgt tctgcctcag cctccaagta gctgggagta caagtgcatg 2460
ccactgcatg cgctaatttt tgtattttta gtagagatgg ggttttgcca tgttggcttg 2520
gctggtctca aactcctgac ctcaggtgat ccacccacct cggcctccca aagtgctggg 2580
attacaggca tgagccacca cgcctagctc aggtgatgcc attagtttct acacagttat 2640
cctctgtcat cccagactca atgtgtccat cactggatag gtcaccgcct cctaaagttt 2700
ctcttgacct gctctcatct cccagaattt tcctgtcacc cagaatttga ctcaggtaca 2760
caccaccaca cctggataat ttttctattt tttgtagaga tggggtttca ccatgtagcc 2820
caggctggtc tctgtcttga actcctgggc tcaagcggtc ctcctacctc aacctcccaa 2880
agtactggga ttacagagta attactgtga gccaccacat ccagcttggc caccagctta 2940
ttcaaagtct ccaggatgct gggaccaccc cccccaccac ccgctccctg tttttgcata 3000
attgtatctt ttttttttga gacggagtct cactctgtca cccaggctgg agtgcaatgg 3060
tgtggtctcg gctcactgca acctccacct cctgggttca agcgattctc ctgcctcagc 3120
ctcctgagaa gctgggacta caggtgtgtg ccaccacacc cggctaattt ttgtatttgt 3180
agtacagatg gaatttcacc atgttggcca ggctggtctt gaactcctga ccttgtgatc 3240
cacccgcctg tcctcccaaa gtgctgggat tataggcgtg agccaccgtg tctggccttt 3300
gtaattgtat ctttgtgaat gagtgatttg gttctgccct ttttactcca tatttatacc 3360
agcctggctc taggagagtc agaaggcctg cccaagggtt tctgccctct ctgggccact 3420
gggccaaagc tgtagcttgc cctccgtggg ctacctgggt cagccactcc tgtgcttagg 3480
gcttaatcac tagttcgctg aggcctgaag tttaatcaac accttgaggc taaacagctc 3540
tggtccttgt gatcttcagc ccatccctgc ttttctctgg tcctcctcag gagcttgtca 3600
ggccacgggg gctccagtga tgaggctgac catctttctt aagaggtgtt ctggtagctt 3660
gtattatgat tggattgcgt tgacttctca aagccgaact gctgcttact agtagtacag 3720
tagactccca tttggcactg ggctggtctt tacccagggg tcccatagat gggtgtgtgg 3780
aggggagaag agccagcatg catccttgag ttgttagttc accaaaggat gtgaccctgt 3840

gttccacgag cgctcggtaa ttcctggttt gcactgatgg cctttctctt tggtaaagtc 3900
agggcgctga ttatcttgtc ggaagtatcc agctggtcct ctcttgggct ccatcactgt 3960
gcaagtctct gctcataaga actcttcagt tcccttctcc tgggttcaca gccaaggcaa 4020
aaaagagact ccttggcctc ttggcaaaca tacatcccct acctggctgg tccccccttca 4080
gcttccctct ctaaggaaca acttgaacat agaaactcca acctcacctg taatcccagc 4140
actctgggag gccaaggtgg gcaggtcatt tgaggccagg agttcaagac cagcgtggtc 4200
aacatggcga aaccctggct ctactaaaaa ttagcggtgg tggcgcatgc ctgtaatccc 4260
agctacttgg gaggctgagg cacgagaatc acttgaacca gggaggcaga ggctgcagtg 4320
agccagggtc atgccactgc actccagctt gggcaacaga gagaccctgt ctcaaaaaaa 4380
gaaaaggaac tccaacttct gaaagtaaac aacttatgca gtacatgatt cctggagagg 4440
atttggtcac caaccttccc tccttccttc cagggaaagc acacagaggg gcagggcaag 4500
ggcctccagt gcattacttc accacttccc tgagtaggtc ttctcaagtc cttggagcct 4560
tcactttgtg atttgtcact gccagagagc aatgttggga tcatgagctg tgggtcctca 4620
gtctgacttt aaagaaacct cacaaattgt ctgctcctga ttttcctgac catgtttccc 4680
accaatccta acaaacctct gcttccctct cagtctcaac actctggtgt taatgcattt 4740
gaacctgctg tgtatctact tcctgtaatt ttttgagaat tatctagaat ggctcaaatc 4800
ccacctttcc catgaagcct ttctgaccac tgcacatgct ccttcccctt gctgggctgt 4860
gtgctgcatg tgtgtacagg gggacatcac atttgggtgc agcctgtcgt gtgggcgttc 4920
cttttgacgc ccgtgtcttg agagggctgt gtggtgttag caggttagag gcagatgcta 4980
aagagacagg cctgaccatt cactgaccta gctttccttt ccctgagctt cagtttcctc 5040
acctcttaaa gggaaataaa aatagtatga gttgtgtgca gcttaactga gaggatgcct 5100
gtggagtgtt cagggtgatg gcagtggtta ttactattaa tctttttctc cccagccaga 5160
cttgatgcct ggacttggcc acagcttgct tggtaaaccc atttactgac atattaattg 5220
atccctcttc tctcaccagg tacatggcca cctctggcct agaccaccag ctgaagatct 5280
ttgacttgcg agggacgtac cagcctctga gcactcggac cctgccccat ggagcagggc 5340
acctggcctt ctcccagagg ggactgctgg tggcgggaat gggtgacgtt gtcaacatct 5400
gggcagggca gggcaaggcc agcccaccct cccttgaaca gccctacctc acccaccggc 5460
tctcaggccc tgtgcatggc cttcagttct gccccttga agatgtgctg gggtggggc 5520

```
acactggggg catcaccagc atgctggtcc ctggtgagtg ggccagggga tggggcttga    5580
agtgaatgaa ctctgggtgg aagtttgggc cagagacatc caggaactgg gggcttagtt    5640
gggctggagc tgttggactg aggttcttcc ttatggctcc atgcttctcc ctccctccct    5700
tcaggggccg gtgagcccaa cttcgatggc ctggagagta atccatacag aagccggaag    5760
cagcgccagg agtgggaggt gaaggccctg ctagagaagg tgaggctccc ctgctggaga    5820
gggtgagatc ccccccattg ctggagaggg tcaggttccc ccgtcctgga gagggtgagg    5880
ttcctccttc ctggagaagg tcaggttccc ccctgctgga gaaggtcagg ttccccactg    5940
ctggagaagg tcaggttccc ccctgctgga gaaggtaagg tttcctcttc ctggagaggg    6000
tgagattccc ctctcctgga cggggtcagg tttccctctg ctggagaggg tgagattccc    6060
cctgctggag agggtcaggt ttccctctgc tggagagggt gaaattccct ctcctggaga    6120
gggtcaggtt ccccccctgct ggagagggtg agattgcccc tgttggaggt gaggttcccc    6180
tgctgcagag agtgaggccc ctgctgctgg ggaaggtgtg gtttggggtt ggaggtagag    6240
aaagctctcc tgatgtctca tgctgggggt cttgggtaga ggggttcagc agcctccctg    6300
gcttctcttt ttggtgctac cctcctgtgg ctccttgctg agtcctgtcc ctctgtgaca    6360
cccccaggta cctgcagagc ttatttgtct ggacccacga gccctggccg aggtggatgt    6420
catctccctg gagcagggaa agaaggagca gatagagagg ctggtatgga gcaggcccct    6480
gaatgcccag gcccgtcttc tcccccacat cctctcccca ccatggcttg tccccagaag    6540
tgcggccagc aggtgggtgc tgccagttcc ctgactccga ggctgtaaca gatatcactc    6600
ctgtcccctc cctcagggct atgacccgca ggctaaggct cccttccagc caaagccaaa    6660
gcagaagggc cgcagctcca cggcaagcct ggtgaagagg aagaggaagg tcatggatga    6720
ggaacacagg gtaaatgagc attggcatgg gccgggcctt ccacaggctg caccctcctg    6780
cttgtgcctc tgcccttgtc agcctgccac ttctcactct gtgcctgtgt cctccccgtc    6840
tccggtcccc aggacaaggt ccggcagagc cttcagcagc agcatcataa ggaggcgaag    6900
gccaagccca cgggggcccg gccatctgcc ctggacagat ttgtgcgctg agccagactc    6960
cagggttgcc tgggaacagt ctctccccaa gatcacctgt agggaaatga gtgttccctg    7020
gaacaaggag gtgggggcag tgtggcccct tccccaactg ggggtggaca gctgtctcct    7080
ggggtgggtt ggtattaaag aggaaagcga ttttttggat aatgtgtctg gatcatcttg    7140
tggacctgtt cccctccccc ttgccaagca tgaggccatt ctaggatcgg aggggtcagt    7200
gtcctgcctc tacccccacc ccataccttg tggctgctgt ggccttagct ttgcctttta    7260
```

ggggccttcc tcctcagaga ggctgtcctc tctcccatgt ccctggagat gtccttccca 7320
tgcagcaggg tctggcagcc tctgggctct ccaggtttgt ggtgttctgg gggtcctggt 7380
gaagaccaca ctgcactcga agaagtggag acaagtttat tgaggagctt gacacccctc 7440
ttctgcccta gcttgagaga acaactgcag catttttttc tttttctctt cccgatgacc 7500
atcttttggg ctggcgggcc aggcccctgg gtgtctccca tatcgctgtc tttagtgaga 7560
ctgaggatct ggtataagga aacagatcag ctgcagccag tgagggcccc tgcgttgggc 7620
ctgggagaga gaaggtcctg ttcctgcctt tcctgtggcc ccaggcactc tcagctctga 7680
agccaggcta ttgctcgaca ccgcaggatg cccaggactc cctggaacca ggagcaaaag 7740
ggcgcagtcc tttccccgtc agagccaccc accagcttcc aggcttcctg catcttccag 7800
gggtccagcc tgtgggacgt cagcaggaat ttcccatagc agcaccggtc tagcgggtag 7860
tgggtggcac cagccagctt gacacactgt gttggggcga ggcctcctcg tcgggcactt 7920
acccccacaa agacatcctc taatgggaga aggggtgccc ggctggccac cttgagaatg 7980
agctgcacag cagacgctga cagcacatac cccgtgcctg aggcataggg tggaaagggg 8040
ccccaggtgt gaggccactg ctcctctgat acgcggtgcc tgcccccccgg tgtccgagag 8100
gggttcacgc gccagtgcac ccggcccaag tacagaagag gcacttcctc gctgtgcaaa 8160
acctggcctc cttcctgctc agcctctctc tggggttccg tgcttctctc ccattgcccc 8220
caacggcccc ctcgcaagac cagctctgat accagttcag ggacgttgac atacacatca 8280
tcgtccgtct tgaggacgta tcgggccatg gggcagtgtt tctcagccca gttcagcccg 8340
ctgagggtct ttagggtgag gttgcggtag gagtcctgga aggcggcctg caagatatcc 8400
ccctgggctg ctgactccga ggccaggtca ctcccctggg aaccccacac ggggtgctgt 8460
gcgttcggct ctcccagcaa gaatagcgtc tgtaccctga gcccccgggc ctcgcgcagc 8520
ccgccccacg aagcccgaat ggcgtttctc tggttcaggt tctccggagc cgtgcacacc 8580
aggatgagca ggaagggagg ggccccggga ccactgcaag cttcctggtt ggggatcaag 8640
aggcggggca gggccagggg cggccccggt gaggcggggg ctgggagcag ggaggctagt 8700
gagaggctca gcagctcctc ccccaacccc gaaggcccga agagggtcca gacgatcacc 8760
agcagcaaag cggcgagaag gaggcgccgg aagagcctga gctgcatggt gcggcgtact 8820
ccgaggggat ccgtaagagc gaagggcgga gactagctcc ccgggcctgg gtcacggccg 8880
gggacttaac cgaccacgcc cgggatgcgg aggtctgagc gcgcgaccgg gaccagcgca 8940

ccctgggggg cggggagagg cggtgcagct gcggaccgcg gtcgaggcat gctgcggggg 9000
tggctacctt gtcgccgcgg cctcgccgcc agcaagctcc gctccatggc tggccgactg 9060
cggcactcac ggcccctccc ccgcctcgcc ccggcggccg gtctgccgcc cgcttccgcg 9120
ttgcgcgctc ccatccccgc cggaagcggc gtcggagcag cgcggcaggg cgcggaaacc 9180
cgggcgggtg gagtggcacc aaacggagtg agaccggtct gtccagccgc tccccatccc 9240
aacccaccga cgtactccac cagggagacc acacccttca taaccaacgt ccgcgttttt 9300
gcctttgttg cttcggtcac agcccgaagg attttccctt atgactagat gaccccttaa 9360
aagaaaacaa aaacaaaaac aaaaaaaccc agagtgacca agacctgcaa actcacctta 9420
actgcaactg gctaggctca gctaacccac tccacaatga tgctgcacac caaccccagg 9480
gtgttggtct ctaaactggt ctgggtcagc ccctcaaggc tgaactgttt taccctcaca 9540
tttctcttta cagagaggga catcactagc tacatatagg gagactgggg ggtctgcagc 9600
atcaccactg ctaaaactaa tagcactgtg tgggcaactg agtagggcag ctctaaatgg 9660
aaaaaggacc cagcacttta acattcccta ttagaatgga cagaaaggac aggaagccat 9720
aggtatataa actatttatt aacagacaag gcctacagac ttatttcttc ttggacacac 9780
ccacggtgcg gccacggcgg ccagtggtct tggtgtgctg gcctcggaca cgaaggctgc 9840
aggtaaaaag agaagagtca caggtcatgc acagcagaaa caaaaggttg gggaaaaaga 9900
agagcgagta ggaggaatgc tgagtcgagg taggcaggga gatgagaccc cccactcacc 9960
cccagaagtg acgcagccct ctatgggccc gaatcttctt cagtcgctcc aggtcttcac 10020
ggagcttgtt gtccagacca ttggctagga cctggattca gaggaggggg caagggatgt 10080
tttatcccct gacctcctat gcccaattct gttaggaccc tccacccttc ctctaatcct 10140
gccctcattt cagtacacac ctggctgtat tttccatcct ttacatcctt ctgtctgttc 10200
aagaaccagt ctgggatctt gtactggcgt ggattctgca taatggtgat cacacgttcc 10260
acctggcagg cagaccaagg tcagatctag atggacccct ttctgaggct gaccccaccc 10320
ccagcccccct tccttgtcct cacctcatcc tcagtgagtt ctcccgccct cttggtgagg 10380
tcaatgtctg ctttcctcaa caccacatga gcatatcttc ggcccacacc ctgaatgaag 10440
tgaggggttt cggaattagt gttgaatctg atttttaaat tcctttatag cctcattcaa 10500
aaagctcaca aaggtaggat ctgcaataat ctattttgca atcccctagg tcaccctttc 10560
ctcacccacc tttccagata taaaccgttc gacagaaggt taactttggg ccggtcgcag 10620
tggctcacgc ctgtaatccc agcactttgg gaggcccaag tgggcagatg acattgaggg 10680

caggagttca aaaccagcct ggccaacatg gtgaaaccct gtctctacta aaaatacaag 10740
aaaagttgct gagcatggtg gcaggtgcct gtaatcccag ctacttggga ggctgcagca 10800
gaatagcttg aacctaggag gtggaggttg cagcaagcca agatcacgca accacactct 10860
aacctgggcg acagtgagac cccgtctcaa aaaaagaaaa aagtaacttt gttcaggggt 10920
atgactgaat catgaacaca gaatttagta ttacagacaa ccctttgaaa gcaggcagta 10980
tctttttttt cccccattgc tcagcatatg tagttgtcac taagtgcctt ctgattcttg 11040
catttaggag aacaaggggc ctgccactcc aagctgatgc agtaaaccag ggtcttatga 11100
gttacaggct ttgattaagg tattgggctg tcagttcctc agaatctcta agggtcacaa 11160
ttatttaggc aaatcactgt atccccataa tcaaagtatt ccaagatcac aaactcaatt 11220
ttgtcagtgc ccagaactga ctgcccaagt tattataaaa agaaactaat gcttacattg 11280
gtagcatcac atcaacacca gcactagcac ccatgccatc tgaactcatg aaccaaagtg 11340
atttggagag tgaaggagga actgaatact gagccctgcc cctcattttg tccaactaga 11400
acatcagaat gtggccctat ttggaaataa ggtcgttacg gaaataatta ggtaaaatga 11460
cgttatattt gattagggtg ggctctagat ccaatgacaa atcatgagag agacacgaag 11520
aggaccatga gatgacagca acagattgga gtaatgcagc tacaagccaa ggaacactaa 11580
ggattactga caaccaccag aagacaggaa gaggcaaggt aggattctta agtgcctcca 11640
gcgccaagag taattttctg cttagtttat atgaacttgt tacagtaggc tccaggaaac 11700
taaaacaaat ggcagttcct atctgcaaga aagagatcaa gggttggctt caccgtggct 11760
catgcctata tgcctgtaat cccagcactt taggaggctg aggctggagg atcactgagc 11820
ccaggagttc aagaccagcc tggacaacac agcaagatcc agattagcca gatgtggtgg 11880
tgcacacctg tagccccaac tacttggaaa gttgaggtag gaggattgac ttgagccctg 11940
gaggtcgagg ctgcagtgaa gagtgattcc gccactccag cctgggcgac agtctcaaaa 12000
aataaatgtg gctgggtgca gtggctcacg cctataatcc cagcactttg ggaggctgag 12060
gtgggtggat cacctgaggt cagaagttta agaccagttt gaccaacatg gtgaaacccc 12120
atctcttcta aaaatacaaa aattagctgg gcgtgtgcct gtaatctcag ctacttggga 12180
ggctgaggtt gcagtgagat aagattgcac cattgcactc cggcctgggt gacggggtga 12240
gactctgtct cgggataaat aaataaacat gaccaagggt caaattatta ggttgattca 12300
gaagagagca gggtctttca aatttattaa ttaaatgacg aggtttcacc atgttgacca 12360

ggctggtctc gaactcctga cctcagggga tccgcccact tcagcctccg agtgagccac 12420
tgcactaagc ctcaaattta attatatgtt tacaagtgaa aagatttact tcaaaatggg 12480
tgtgggaaga gatatagatg aaataagatt tctaattttt agttagatac atccatgaag 12540
gttattctct attacactta tgaatgctgc acctctactt aagcctcaca gccggtctct 12600
gaaataagta cagtatttca cactgcctta ttagtttcac agagcaccca acagaaggaa 12660
tttccctcta caaattcctc aacagagcca acagtatccc tactactatc cattccctaa 12720
cgtcgcctaa gtacctacca tgcaacagaa gccaagaaga caggcctagc cctgcacgca 12780
tggacacata ctggtaggct atttccacaa ctaactacac attctgactt aagggaaaaa 12840
aaaagaacgt tatgtatcta gaaagccaag aattcattag gtttgtggag agaaatactc 12900
aagtgaaata atattcttgc attcattgat tcgattctta aaagccaaag aaagatggat 12960
gtactccttc acacttcgac aggaaaggag tgtcaattat ggctcatctg agtaaatgtc 13020
ccactcagac ttcttccacc ctccatctac aggcttcttc acacttttta tctgttggta 13080
tttctactta tccctcaaga tgcccaataa tcctccaatt ctcagggaat ataaatttcg 13140
caggaagggt tgctgttatg atggtgggat gggtaaagaa accgaatcta gaagaaatct 13200
cacaagctcg tggttcagga aaatcttagc gaattcgggt gagcaaccat acctcctccc 13260
cacttcccca ggtgaaatgt tttggcttga ccttagacca ctatcttggc tttaatcact 13320
tattttctgt ggtaaatgct aaaggggtca gtctctccct aaaccacttt cttttcttaa 13380
tccagcagtc aaggtgatct ggatgagact caagtctccc cagccttgct tcatttttgc 13440
ttatgtcttc acaacccaat tctcatttac attccctatt ccttacccta cttcacttac 13500
cttaatggca gtgatggcaa aggctatttt ccgccgccca tcgatgttgg tgttgagtac 13560
tcgcaaaata tgctggaact tttcagggat cactagagac tagttgaaaa agtacggggg 13620
aagaaatcag acacagtaaa ctatcaaaca gtaaggccga taaggcaaac gaacttcata 13680
caaaaaggtt aagcctgtca cacaagtggc aagacacccg ccccccattc cttaggtttt 13740
cacaccacag agcaaactcc tcagtcctgc agagtaggca catgaagcgt tcaagcttgg 13800
gaaaatgcaa cctctggaga gaggaagcag gtctcctgcc cattcccaaa agtgcgtggc 13860
aaaagccaga gttccccata tacgcccaag ccttagacag ctttcgtgcg cccgagcagt 13920
gacccttcct cgggcccgct ctccggaatt ggaaatcttt ggtgtccctt tccagggacg 13980
cgatccaagg ctccatacag aaagtcgcag gccctcaggg caggacgttt ccagaaccct 14040
gacagccggc tttccttgcc cgagctgcga tgccgctgga tcacggcacg gattccagtc 14100

ttaccatggc tgcagcacaa gcggcggcgt gtaggcctcc tgtggaagag agagcggaag 14160
tgacgcgata tcattatata gcttccagga ggaagaggcg gtgcgatcta ggagaagtgc 14220
ttccggaaac tgtttcagcg cgacctggct ggatttatga gagtatggct tgggtaccac 14280
aacttccggt gcgcctttct ttacagttcg taaggttcat aggtaagaga acagcgaagg 14340
ttccggggct agtttgtgtt tcagacttcc atatttccat ttgcagttgg gtgaagtggt 14400
tagagctgaa aggggacagc tggagtcaaa caacttaact caaatatctg ggaacgattt 14460
cgcgctgagg aagacccttt gggacttgta gctccactcc ggggaacgga ctcgccggga 14520
ctgacagttg ccggaagtga ggctgcgggg aatggccgcc gctgcgacca tggcggctgc 14580
ggcccgggaa ctggtgttgc gggctgggac ctcagatatg gaggaggaag agggcccgct 14640
ggtgagagcg cggagctgtt tctcctccgt agttccggtg ctcctagctt cagaaagacc 14700
tgagctgggt ttaggcacaa gggtgggaac ttggctgcta cccgtgatat tacctgtttg 14760
gctccccgta cccttccctg tgatcagacc cgaccctgag cgggacccta gtggctgaaa 14820
ctcatgttct ggggcacaaa atttcaactt cactccacat tttggggtag agtcacgaag 14880
tagtatagtg taaaggttaa gaacatggcc tctgaagcca gactgcctga gttctatctc 14940
tgccacttac acgctgtgtg cccttgggca agttaaccag cttctctgtg ccatcatttc 15000
tcacaccttt aaggtgagga agatagaacc tattgtatac atcgtcgtta ggattagatg 15060
aatatgtgta agattgcacc tggcatgcag gtaaacacta tgtgttttga catttctaga 15120
aagcctgggg ttccccagag gtgtgataga acacccaaga tggcaggagt ctgagggtct 15180
taaatttaag tgcaagttct gctctatcct ctgcaattcc tagctttcct cattcggtca 15240
tctccactgc actttttttt tttttttttt ttttttgaga cggagtcttg ctctgtcgcc 15300
caggctggag tgcagtggcg cgatcttggc tcactgcaag ctccaccttc cgggttcacg 15360
ccattctcct gcctcaacct cccaagtagc tgggactaca ggcgcccgcc accgcgcccg 15420
gctaattttt tgtatttttt agtagagacg gggtttcact gtgttagcca ggatggtctc 15480
gatctcctga cctcgtgatc ttcccacctc ggcctcccaa agtgctggga ttacaggcgt 15540
gagccactgc gtccggcctc cactgcactg tcttgtcttt tacctttttt tgttttcttt 15600
agacgggttt ccctgtgtta cccaggttga ccttgaactc ctgatctcct caaacaatct 15660
tcccgtctca gcttctaagt agttgggact acaaggagcc caccaccatg ctgggctacc 15720
tccactgtct cactggatcg tctcactcct tgtcgtattc cttacccatc agcacaaaat 15780

```
aggagagtgg gtacaacgtg agactcttag aatttttcaa agcagtacct gtggctccca 15840
gtaaaagcag ctccttctca cttgaaggac tggggatatc caattatatt ttttctttct 15900
gtctttcacc gtaccctact cccattaact tatccctttc caggcgggtg gtcctgggct 15960
ccaggaacca ctgcaacttg gggagttgga tatcacttct gatgaattca tcctggatga 16020
agtggatggt aagcgatggc atggggtggg tgcagtacct gtaatctgag aggcatgggg 16080
gctggaagga ataaggtagg gattggtgtg cagatactca atgtggggag agggagctgt 16140
ggagctggac ccttatgata aacttctatt tccagttcac attcaggcaa atctggagga 16200
tgagttagta aaggaagctc ttaaaacggt gaggctttcc cctgtactaa tctgcccttc 16260
tatctattct accccaaaag gaaaaacaac agtctcttgc ttttgttgta tcagcctggt 16320
atctgaccct ttagtccttc actgattctg ttacagtgtc cctttactga gacttccttc 16380
tgacctgtca tctttatgtt ctcccagggt gtagatctcc gtcactattc aaagcaagtt 16440
gagctggagc tacagcagat tgaacagaaa tccattcggg attgtatcct ccagcagagg 16500
gaaggggtgg tgctattgac atggggattt tgtggcgtgt ggtacagatg tcccagctgg 16560
ggtcaacatc tctgaggttt ctatcacttc aaagagaaat tggagaggta ctacaaatct 16620
gaggatctcc catcttcacc atgaggcccc ttgacttttt gtggatcaga tattcaagag 16680
agtgagaata tagcatctct acacaaccag atcacagcct gtgatgctgt cctggaggtt 16740
agtaatcatg acctgtgacc cctaatagct actctgaacc agatcatgga ctctggtgcc 16800
atttttaggt catcagcctc aagatgggga caggttaggg tcattttcaa gatgacttca 16860
ggagcctttt ttgttattac tgccatattt aatgttattt cctgataatc tggggtgtct 16920
catcatctgc caccctcaag gactgataaa tactgaagac ttcacaaaga gtctttggtg 16980
gcccacttat cccctacaaa ccctatagca ctccggcaac atctaccctc cctacagcga 17040
atggagcaga tgttgggagc ttttcagagt gacctcagct ccatcagctc tgagatccgg 17100
acactgcagg aacagtcagg agccatgaac attcgacttc gaaatcgcca ggcagttcgg 17160
gggaaacttg gggagcttgt tgatggtctg gtggtgcctt ctgctctggt cacgtgagtt 17220
accagtttga aggttataa tgtccatagc aggtggatgg gctggggtga cccaccctat 17280
ttaccatatg ggtctcctgg ctccaggtta agtgcttttt agaaattagt agccaggtta 17340
accaccattt ctttaggtat gcattaggca tcaaagatta agattaagaa tgatgtaaga 17400
tcaggtgtat agtggggagg tccaggaata gaaaactttg tacatgtgtc ctgactatat 17460
ggtacaatgc aaggtggtag agatgacagg ggctggaggt aaggagtgtg gtctgggtct 17520
```

ggtttaggga tgtctgggtc tcccttgtaa ctcatcccac ccctccctgc taaccagggc 17580
aattctggag gctccagtga cagagcccag gttcttggag cagctacagg agctggatgc 17640
caaggcagcc gcagtcagag agcaggaagc tagaggcaca gcagcctgcg cagatgtcag 17700
aggcgtgctc gatcggctcc gggtcaaggt gggaagtagg gatggatacc ctggaggcta 17760
ctggaagcag ccttcccaag atcttcacta tggatttcct tggcagagct cactgtagcc 17820
tgtctccagg gacttcaccc tgccttttct gggcagcccc attcttgtct ctctgggttt 17880
ccctgaaaat cttgagaaat accaaagaga tattcaccac ccttctgcct atgtttacca 17940
tttgttgctt cccccagttc ctctcataca gcaaaagggt tgcagcttca tgccctgtgt 18000
ttggttcccc acatctaggc agtgacgaag atccgagagt ttatcctcca gaagatttat 18060
tccttcagga aacccatgac caactatcag atcccccaga cggccctgct gaagtacagg 18120
tcacaacccc aaggaagtgc atgggatggc ctttgagttt ttgagcggcc tagtgtgggc 18180
atctggtttc ttgggagaca ggtagactga catgttcctg acccccctagg ttcttctatc 18240
agtttctgct gggcaatgaa cgagcaacag caaaggagat cagggatgaa tatgtggaga 18300
cgctgagcaa gatttacctg tcttactacc gctcttacct ggggcggctc atgaaggtgc 18360
aggtaaggtc aggagggaga ggtattcctg ggcacatgga ctgggaggag gggacatccc 18420
tgggcaagga atattttatt gtgttgtggg aagagacggt tatcagttct cttttctctt 18480
tccttagtat gaggaagtcg ctgagaaaga tgatctaatg ggtgtggaag atacagcaaa 18540
gaaagatatc ctagtactgg ggaaccctca tgccaggcct tgagagtggg aggactttcg 18600
actccagcca ccaatgcact gctccttacc ttttcctatg ggactgaaac cttttagaat 18660
gtgatgaaag ctatagcctc cctccccaga aagatgcaca cattattttg acacatagtt 18720
ttgcatatga ttgcaaggga gggagagaca ccctgaagcc cattcacaga tgtcagcacg 18780
tggggcccta gtactgggaa agagggctgg ctaggccacg tctgcctgtc tggggtcccc 18840
acagatagtg agtctttggg cccagttatg gtctcattgt tttccctgac tctgacccat 18900
cttacgattc ttctcaaagc catcgctccg cagcaggaac accattttca ccctaggaac 18960
ccgcggctct gtcatctccc ccactgaact tgaggccccc atcctggtgc ctcacacagc 19020
gcagcgcgga gagcagaggg tatgaggagg aacaagcttc agtcacgaaa gaggctctga 19080
gcacggtggg gagggaaggc cagacctcag ggcagagctt cccaacctgc aggccctgtc 19140
ttaatcccct tagacttggg agtggccttc tccaatcact ccagcctgct gtgatgtgaa 19200

aagagctggg aaccattgac ttaagggcat gcagagggga gagactggat gaggggaatg 19260
acagtcagag gcaggtgttg aggtggatgc aggagtagca acagcaggtg ggtgtaggcc 19320
ctttttgtac ccctatgttc ccccaccttt tcagtgccca ttcagcttgc tcttagacag 19380
cccggaccta gatagatgcg aagggtctgc ctagctctta ttactcaggt ttactgattt 19440
tccagccata tccatgggaa gtcagtgttg gggtccatcc cttctgtttt tcttctttac 19500
tcctcctctc ccagtatcca tttgaggccc tcttccgcag ccagcactac gccctcctag 19560
acaattcctg ccgcgaatac cttttcatct gtgaattttt tgttgtgtct ggcccagctg 19620
cacacgacct gttccatgct gtcatgggcc gtacactcag catgaccctg gtaagactcc 19680
tgtttcctgt accattcaac acctttgtcc ccagtcttca tcagcagcat agtggggtca 19740
tgactctggt agaactgaaa tcaggatcgc ctcattctaa aatgtctaag ccttggctgg 19800
gcgtggtagt gcacacccat aatcccagca ctttgggagt ccaaggcagt aggattgcct 19860
gagctcagga gtttgagacc agcctgggca acaacctcat ctctacaaaa aatgaaaaat 19920
tagccgggtg tgtgttgatg tgtgcctgtg gtcacagcta ctaaagatgc tgaggtggaa 19980
ggattgcttg agcccaggag gtcaaggctg cagtgagcca tgcactccag agtgagacct 20040
gtctcaaaaa aaaaaaaaaa aaaaaatagc caggtgcagt ggcttacgcc tgtaatccca 20100
gcactttggg aggctgaggc gggaggatca caaggtcagg agtttgagac cagcctgacc 20160
aacatgatga aaccctgtct ctactaacag tacaaaaatt agccaggtgt gcgcggcctg 20220
taatcccagc tactcgggag gctgaggcag gaggattgct tgaacctggg aggaggagat 20280
tgcagtgagc caagattgcg ccattgcact ccagcctggg tgacagagcg agactccatc 20340
tcaaaaaaaa aacttgttac ttagacttgt cacagtgtta ctcccccatg aaatcctcag 20400
tgcgtagggt ccctgagact acccctactg ctgacccaca atcaggcaat agcacgtgaa 20460
aactgagggt cccccaaatc ttggtgctca aacccctgac ccaagctcag ccctgaactc 20520
tgctgtgata ggcttgtgat catagatcct ggtgtggcca ggtgcagtgg cttatgcctg 20580
caatcccagt actttgggag accaaggcag gaaggttgct tgaggccagg agttcgagac 20640
cagcctgggc aatgcggcaa gaccttgtct ctacaaaaaa tttaaaaata aacttaacca 20700
ggcatggtgg tacacaccca tggtcccagt tgcttggcag gctgaggtgg gaacatcact 20760
tgagcccaga tgtttgaggc tgcagtgaac tatgattgta ccattacaat ccagcctagg 20820
tgacaaaaca acctgtctct aaagccgaaa acaccgggtg cagtggctca cacctgtaat 20880
cccagcactt taggaggctg aggtgggcgg atcgcctgag gtcaggagtt cgagaccagc 20940

ttgaccaaca tgatgaaacc ccatctctac taaaatacaa aaattagtcg ggcgtggtgg 21000
caggcgccta taattccagc tactcaggag gctgaggcag gagaattgct tgaacctggg 21060
aggcaggcgg aggttgcagt gagccgagat tgtgccactg cattccagct tgggtggcaa 21120
gagtgaaact ctatctcaaa aaacaaaaaa aaagccaaaa acatagatcc tggacaggaa 21180
agcagggggc agttccattt tacgtctctc ccttctgtcc tgtccagtgc atcatcccca 21240
aagaaacaaa tgcgtttgtt catagtcact gaggacaaca aactgacatt ctcttttaat 21300
cctctgtttg ttccccaatc acacagaaac acctggattc ttatctagct gactgctacg 21360
atgccattgc tgtttttctc tgtatccaca ttgttctccg gttccgtaac attgcagcaa 21420
agagggatgt tcctgccctg gacaggtcac tgaattctgg tccttgatgc ccaacaggcc 21480
acgaactctc ttggtcttgc atgactctgc cctgacctta gactcctact gacctggtat 21540
catcaacctt tattcccatt catttgctcc ggaggcagta gtgctgcttt ttgactcacc 21600
tttctccaac cactgccccc caacccccac aactagctat tgaccattaa actgaatgat 21660
gccccactcc ctcccatccc cctccaacat tccttgctgg ctgacctctg attctgatta 21720
agcccacagg tactgggaac aggtgcttgc cttgctatgg ccacggtttg aactgatcct 21780
ggagatgaat gttcagagcg tccgaagcac tgacccccag cgcctagggg ggttggatac 21840
tcggccccac tatgtgaggg agggcaaggg taacaaaggg ttcttgaaag gcagggctct 21900
aagcatcatt gtgggggttg gccaaggtca cagttgtgtc atgggccacg ctatgagcag 21960
gtgttctggg agggtggtga aactcatggt gagtggtgca ctgtgggtag gagggactga 22020
aggtagagtc acatcacatg gaggtgggtt ggctaaggaa gttgggaggt tgtgttggtt 22080
gggccaggag gtgggtgtga ttttccttcc cactctctcc tagatcacac gccgctatgc 22140
agagttctcc tccgctcttg tcagtatcaa ccagacaatt cctaatgaac ggaccatgca 22200
attgctggga cagctgcagg tgagggtcgg acaggagaca cttcccagca gcagggctgc 22260
ttccagtggg tggagtctga ggaggagcca gatggggccc agatgacctt gctttctggt 22320
gtcacctctt cctctctgcc ttttcaggtg gaggtggaga attttgtcct ccgagtggca 22380
gctgagttct cctcaaggaa ggagcagctt gtgtttctga tcaacaacta tgacatgatg 22440
ctgggtgtgc tgatggtaat aggtgctcct tctccttgcc ttcctaccca gaaaattcgg 22500
tttcccccac tgctgttagc tctgtgaggg cagagatgat gcctgtcttg ttcagtgttt 22560
tactcttgga gtctggcaca gtgtccagct tagtacaata agtgtggaat gaaggggaca 22620

tcctgaagtg aaggactgct ttccccaaac tagacatttc cctttgtctt cccttaggag 22680
cgggctgcag atgacagcaa agaggttgag agcttccagc agctgctcaa tgctcggaca 22740
caggtagggg tgtggggaaa agggaagaga cagccttctg tgctgttttc agctctctct 22800
ctctctctct ctgtgtgtgt gtgtgtgtgt gtgtgtgtgt gtagggagtg gcataatgga 22860
tctcaggtat gcggaattac tgtaactctt gattcacaac atactgtcat tattggtcta 22920
ttttatttta ttttgtcttt ggtttttttt tttttttttt tttgaggcaa agtgtcactc 22980
tgtcacccag gctggagtgc agtggcatga acatggctca ctgcagcctc aacagcctgg 23040
gcttaagtga tccttctacc ttagcccccct gggtagctgg gaccacaggt gcgagccacc 23100
atgcctagct aattttttat ttttagaatg aattttattt ttttctttat ctatcctctc 23160
aagcatttat cctttgtgtt ataaacaatc cgattacact aagttatttt aaaatgtaca 23220
attaagttat aattgactat agtcaccctg ttgtgctgtc aaatagtagg tcttatttat 23280
tcttcctatt tttgtttgta cccattaatc atccccacct tctccccagc cttccactag 23340
ccttcccagc ctctggtagc catccttcta ctctcttcgt acatgagtac atgagttcaa 23400
ttgttttgat ttttagattc cacgaatcag tgagaacaca tgacgtttgt ctttctgtgc 23460
ctggcttatt tcacttaata taataatttc caattctatg catgttgttg caaatgactg 23520
agtctcattc ttttttatgg ctgagtagta ctccattgtg tatatatccc acattttctt 23580
tacccattca tctgttgatg gacacttagg ttgcttccaa atcttagcta ttgtgaacaa 23640
tactgcaaca aacatgggag tgcaaatatc tcttcaatgt actgatttcc tttcttttgg 23700
gtatataccc agcagtggga ttgctgaatc atatggtagc tctactttta gtttttttgag 23760
caaccttcaa actgttctcc aaagtgtttg tactaatcta cattcccacc aacagtgtat 23820
aagggttccc ttttctccac atccttgcca gcattgctat tgcctgtctt ttggataaaa 23880
gccattctga ctggagtgag atgagatctc attgtagttt tgatttgtat ttctctgata 23940
atagtgatgg tgagcccctt ttcatatatg tctgtttgcc atttgtattc ttttgaaaaa 24000
tatctattca aatcttttgc ccattttttta ataggattat tagactgtca tgtagagttg 24060
tttgagcccc ttatatattc tggttattaa tcccttggca gatgggatgt gcaaatattc 24120
tttgtctctt cactttattg tttctttagc tgtgcagaag cttttttttt tttttttttt 24180
tttttgaga tggagttttg ttcttgttgt ccaggctgga gtgcgatggt gcgatctcag 24240
ctcaccgcaa cctccgcctc ccaggttcaa gcaattttcc tgcctcagcc tcctgagtag 24300
ctgtgattac aggcatgtgc caccatgcct ggctaatttt gtatttttag tagagatggg 24360

gtttcaccat gttggtcagg ctggtctcaa actcctgacc tcaggtgatc tgcccagctc 24420
tgtctcccaa agtgctggga ttacaggcgt gagccaccgt gcccagctgt gcagaagctt 24480
tgtaacttga cgtgatccca tttggccatt tttgctttgg ttgcctgtgc ttgtggggta 24540
tggctcaaga aatttttgcc cagacccgtg tcttggagat tttccccagt gttttcttgt 24600
agtagtttca taatttggtc ttagatttaa gtctttaatc catttgattt ggttttcata 24660
tgtggcgaga gataggggta tagtttcatt cttctgtgta atggatagcc agttttccca 24720
gcaccattat tgaagagact atctctcttt tttttttttt cagacggagt cttgctctgt 24780
cacctaggct ggagtgcagt ggtgcagtat tggctcactg caacctctgc ctccttggtt 24840
caagtgattc tcctgcctca gcctcctgaa tagttgggat tactgatgcc tgccaccacg 24900
ccgggctagt tttgtgtttt ttagtagaga cgggatttca ccatgttagc caggcttgtc 24960
ttgaactcct gacttcaagt gatcctccca cctgggcctc ccaagtgctg ggattacagg 25020
cgtgagctac cgtgcctgac caagagacta tcttttcccc agtgtatgtt cttggcactt 25080
ttaccaaaaa tgagtttact gtaggtgtgt gccagctaat tttttaattt tgtaaagaca 25140
gttgtcttta caaaagacaa ctgttgcctg ggctggtctt gaattactgc cctcaagtga 25200
aaaatcttat gattcttttt ttttttaaa gacggagtct cactctgact cccacactgg 25260
agtgcagcga tacgatgtca gctcactgca acgtccgcct cccaggttaa gtgattctcc 25320
tgcctcagcc tctgagtagc tgggactgca ggcacgtgcc accatgtcca gctaattttt 25380
gtatttttag tagagatggg gtttcaccat gttggcccag ctggtcttaa aactcctgac 25440
ctcaggtgat tcacccgcct cagcctccca aagtgctggg attataggca tgagtcactg 25500
cacccggcct agattctgtt tttaatgaca ctggaaaaca acgaattttt gttttggcat 25560
atatatagct ttacttttaa aagcaagaac acacacaaaa aaagggggaa aaaagcaaga 25620
acacaataaa tgcaacattt agatcaacga gtacctgtgc acgagaagca cagggatggt 25680
ttcagtagga tttcatagtt tgatggtgac ttttgccaag ctgctgctac ttggattgtg 25740
tggtgtgttt gtgagtgttt atatcattaa aaaacaaaac agccactgca ctgggttgtt 25800
ttatttttta atgatataaa cactgatgag acaaggcaag aaaacagcaa ggcaaaaaag 25860
aaaaaaaaaa aagccaggca tggtggtatg cacctgtagt tctagctatt tgggagacaa 25920
aggcgagagg accactttga aatccaggag ttcgagatta cagcgagcca tgatcatgac 25980
attgtagtct acccttggca acacagcaag accctgtctc aaacagcaaa acaaaacaaa 26040

```
aaaccccaga atcacaggat ttttttcatg cataattttt ctaatattgt catgttattg  26100
catatggcta tagctcattc cttttcactg ccatatatga cttgtgtgat taaacatatt  26160
ctataaatgg attgtttcca gtttcttgct tctgtgagca ttgttttatg aataatcatg  26220
tcttttagca cacatagata ttgattatct tcatcaaaag tttctcttgg gtataaacct  26280
aggacagtag tcttgctgct aataagatat gcagtattag tctttatgag acaatgataa  26340
attgttttcc taagtagttc cttctcacca gcaatatgta agaattccta ctgagccaca  26400
ttttctccaa catgtggtat tatcaggtct ttgccatttt ttttcttttt ttttttagag  26460
accaaggatg ctgctaaaga tgctcgggat cagaaatgtt tcagattttg atttttttcg  26520
gattttggat tccttgcatt atttttactg gttagcatct gtagtctgaa aatctgaaat  26580
ctgaaatgct ccaaagagca ttacctttga gcatcatgtt gatactcaaa aacttccgga  26640
ttttggagca ttttggattt cagattattg gattagggat actcaacctg tatttcagtg  26700
ggttttattt gtaattttct gattactaat aacattgagc atcatttcaa atgtttagta  26760
actatatgta ggggttcagt caggctggtg ggaaaaatat tagtgatgat agccacaaac  26820
cctcttggaa ggcctaagag tttgcataac ttcagtaata gatatggttg aaggcgacct  26880
gatctttacc tttagttaaa taaattagag taataacaaa ggaatgtggg gaagttatct  26940
aggtagcttg tttactcata tggtcttaag actaatcttt gatgtaccgc aggtgcttaa  27000
ctgctttcta ctcaggaagt ccacaatgtc agttaccccg tagtggtgtt gactcaagtc  27060
tttgtcaatt aatctttact gaataaatgc gagtctcact agctggtcag ggccgccatt  27120
gcaactgttt acagtactct tcagggagtc tgtaaacagc ctggacacac tcagctggac  27180
tggcaaagca gagtatctgt gtgtcagtgt acctcattca tccgttgccg ggtcaggggt  27240
ctgcaaggga cagactccct gaagctggtg ctctgtgtga ggagcgtcac cacaactata  27300
caattttttt ttttcctgta aaatggctgt catagcattt gaccttttaa aaatcggctt  27360
gtggctgggc agggtgtaca cctgtaatcc cagcactttg ggaggctgag gcggatggat  27420
cagttgaggc cagtaattca agaccagcct ggccagcatg gcagaaccct gtctctacta  27480
gaagtacaaa aattaaccag gcatagtggc gcatgcctgt agtcccagct cctcaggggc  27540
gctgaacctg ggaggcggag gttgcagtga gccaagatta tgccactgca ctccagcctc  27600
agttacagag cgtgactctc tcaaaaaata aagaaaaaaa taagtctgct agtatttttt  27660
atttataaga gttctttgca tcatctacat actaatcttg ttgattatac gtaggttaca  27720
gattatcttt tagtatgtat ctttttactt tttttttttt ttttggtgag atgagggtct  27780
```

ttctgtgttg cccaggctcc tgggctcaag aaatccttgc accttttttt tttttttttt 27840
gagatggagt ttcactcatg ttgccgaggc tggagtgcaa tggtgtgatc tcagctcact 27900
gcacctctgc ctcccgggtt caagcactta tcctttctca gcctcctgag tagctgggat 27960
tacaggcgtg tgctaccacg cccggctaat ttttgtatat ttagtagaga tgggttttgc 28020
catgttggcc aggccagtct tgaacgcctg acctcaggtg atccatccgc ctcagcctcc 28080
caaaatgctg ggattacagg catgagccac cgcgtccagc ctttgttttt ttaatgttgc 28140
tttttgatta aagaaatttg taattttaat atgattgaat ttatctgtcc ttcattttta 28200
tgtctttgtt tttagtaaca gtcttgctct gtcacccaag ctggaatgca gtagtgtgat 28260
catggctcac tgtagcctca acctcctggg ctcaggcaac cctcctccct cagcctccca 28320
agtaactagg actacaggtg tgtgccatat gcccagctaa tttttgttgt tgttgttgtt 28380
ttgtagagac agagtctcac tatgttgccc aggctggtct caaactcctg gcctcaagtg 28440
atcctcccgc atcagcctcc caaagtgctg ggcttacagc caccatctac cacgcctgac 28500
tatttttgtc ctgtttaaga aattcctggg ctgggtgtgg tggctcacgc ctgtaatccc 28560
agtactttgg gaggctgagg cgggtggatt acgaggtcac gggatcgaga ccatcctggc 28620
caacatggtg aaacctcgtc tctactaaaa atacaaaaaa attagctggg cgtggtgatg 28680
cgtacctgta gtcctagcta ctcatgaggc tgaggcagga gaatcacttg aaccagggag 28740
gtggaggatg cagtgagcca agatcgtgcc actgcactcc agactggtga tagagtgaga 28800
ctccgtttaa aaaaaaaaaa aatgaaattc cttattactc caaggccaga aaaatattat 28860
gctacatttt cttcttaata ttgtagaatt ttggccaggt gcagtggcta aagcctgtaa 28920
tcccagcact ttgggaggcc aaggcaggaa gattgcttga agccaggagt ttgagaccag 28980
cctaggcagt atagtgagac ctcatctcta caaaaaatta aaaaaatata ttagccaagc 29040
atagtggcac acacctgtag tctctgctac tcaggtagct gaggtgggag gatcacttga 29100
gcctgggagg tcgaggctgc agtgagccct gtttgtgctg ctgcactcca gcctgggcaa 29160
cagagtgaga ccctgtctca aaaaataata ataaaattaa agaataaaca aatatataga 29220
gagaattttt ttgttacctt taagtctcaa atttacttgg aattaatttt tgtgtgtgat 29280
gtgaggtagg gatctatttc tttttttat gtggatggtt ggcccagaac catttataga 29340
aaatcttttt cttttctttt tttttctttt cttttttttt ttttttagac agagtctcac 29400
tcttgtcacc caggctggag tgcagggcca caatctcagc gcactgcaac ctctgcctcc 29460

tgggttcaag tgattctcct gcctcagcct cctgagtagc taggattaca ggcatgtgcc 29520
accacaccca gctaattttt gtatttttag tagagatggg gtttcaccgt gttggctagg 29580
ctggtctcga aaagttttca cattttaaaa agtctttaag gattttgatt agaattacat 29640
tgtctataga ctaatttgga agaattgata ctttatgata ttgaaacttc ctatccatga 29700
tcatgatcat gggctcccta tccttttttt tttttttttt tttttttttt tttttttttt 29760
tttgagacag gttttgctct gtcactcagg ttggagtgca gtggcacaat catagctcac 29820
tgcagccttg aattcctggg ttcaagtgct tctcttgtct cagcctcccg agtagctagg 29880
actagaggca cacagcacca cacttggctt tttttttct gtagagaata caaaatttaa 29940
aaatttaatt ttttgtagtg tctcactaaa ctgggattac agatgtgagc cactccacct 30000
ggcctcctcc tccaattttt aagagagtct tttaatgtct tttgataacc tttttaaatt 30060
ttcagcacaa agacactgta agtcgtttgc catattttta ggtgggtata atacgtatgt 30120
ttagttctac taggaataaa taaatctttt ctagtttttt gtctttaaca tcttatgttt 30180
ttgatttctt attctactgg ctaacactcc caggatgatg tttagaagtt gaacagaaag 30240
gtgatagtga gaacccttttt catattcctg attttgaaag aattgttgct ggtgctgtgg 30300
ctcatgcctg taatcccagc actttgggat gctgagatgg gagaatctct tgagcccagg 30360
agtttgagag tttgagggga tctgcctggg caacatagtg agactccatt tctttttttt 30420
tttttttttt ttagacagag tctccctctg tctcccaggc tggagtggtg cagtggtgca 30480
atctcagctc actgcaagct ccgcctcccg ggttcatgcc attcttcctg cctcagcctc 30540
ccgagtagct gagactacag gtgcccagca ccatgcccgg tatttttttt gtattttgta 30600
tttttagtag agacgggggg tttcaccatg ttagccagga tggtcttgat ctgacctcgt 30660
catctgcccg ccttggcctc ccaaagtgct gggattacag gtgttagcca ccatgcccgg 30720
ctaagacccc atttctttaa aaaaaaaaaa aaaaaaaaaa aaaagccagg cgtagtggca 30780
tgcatttgta gttctagctg cttgggaggc tgaggcggga tcattgcagg agattaaggc 30840
acccacaaag atgcacttaa accagttgtt tctgctgaga gcagctggga tttgtcactg 30900
gatttgccag gtatagagac tcgccagctg ccatcaatgc ttctggtcct ttttggtcag 30960
ctgctgcttc atactacgta ggaaagattg ttaaagccca gtgcactgtt tcctctggtc 31020
actcctctgc tatcaaggtg ttctcttttg cgttatagaa agcttaaagt tcatctaagt 31080
ccagaccagt actcctatag gaatataatg catgccacaa atgtgagcca cttaaataat 31140
tttaaatttt ctagaagcta cattaaaaag taaggagaaa cagatgaaat aattttttaa 31200

accactacat ccaaaatatt attatttcaa catataatca ataaaaaata gtactgaggc 31260
cagcacggtg gctcacacct gtaatcccag cacaggccaa ggcagggagg atcgcttgag 31320
cccaggagtt tgagaccagc ctggacaaca tggtgaaacc ccatctctac aaaaaataaa 31380
aaaaaaatga gtgggacatg ttggcacatg cctgttagtc ccagctactc aggaggctga 31440
ggtgggataa tcactgagcc aggaaagttg aggttgtagt gagctgtgat tgtgccactg 31500
cactccagcg tgggtgatgg agcaagaccc tgtttccgaa aaaaaaaaag aaaaaaaaga 31560
tatacttaaa atttattcta agatgaagtc ttcaaaatca ggtgtatata tcatgcttac 31620
aacacattat ataatttatg gctcctgcct agcaggggtc atcactgtta gttcaacttt 31680
tctttggcaa gtctactccc tgtgccccac cgtacactgc tacattattg tttgtatatg 31740
tgtgtgtgag actgcattta ggcaactgta ataggttgaa tttggttatt taatctctgt 31800
ccaaaatcac atcacaaaga tgccttgatt atagaaagat ccttccttgc agcatattag 31860
ttatttgaca ccctcagtgg atataagtga atattttgag ggaagaatag aaaagataca 31920
atttttattt atttatttat ttatttattt actgagacaa gttgctgctc tgtcgcccag 31980
gctggagtgc agtgacatga tcttggctca ctacaacctc tgcctcccgg ttcaagtgat 32040
tcttctgctt cagtctccct gagtagctgg ggttacaggc atgcaccacc acacctggct 32100
aattttagta ttaataattt atttttaaat ggaataataa tttcactttc ttcttaccag 32160
atctttctgt ctgggttgtt tgcttttatg agtttttttg tttgaatctc ccttttacat 32220
taaacaaaaa ttaacatttt attatgggaa atttcactat gctattagaa aagcatagtg 32280
aacccccagg catcatcagt gatattcggt gtatagcagt tcacggacag ttttgtttca 32340
tctataccaa cacctattgc ttatcctcct tctgtgtccc ccagttactt tgaattaaat 32400
cttatacata tacagatgaa tattatcact tcatctgtat cagaatgtta tttttattaa 32460
gaaaattatt tttgagatag ggccttgctg tagtgcccag gctggagtgt agtagcatga 32520
tataactccc tgcagcctcg aactcctggg ctcaagtgat cctcccacct cagcctccta 32580
agtaactggg actacaggtg tgtgccacca cacctggtaa cagatggttt taaaaactac 32640
aatacattat cacacttaaa aaaattatct tatctaaaat aactattcag attttcccag 32700
ttatttcata agtgattttt tttttttttt ttttgagaca cagtttcact cttgttgcct 32760
ccaggctgga gtgcaatggc ccgatcttgg cacactccaa catctgcctc ccgggttcaa 32820
gcaattctcc tgcctcagtc tcctgagtag ctgggattat agacacccac caccacgccc 32880

agctaatttt tgtattttta gtagagacag gatttcacca tgttggccag gctggtctca 32940
aactcctgac ctcaggtgat ctacccgcct cagcctccca aaatgctggg attacaggca 33000
tgagccactg tgcctggcct tcataaatga tttttatggt tcaaatcagg atccatataa 33060
agtccatata tgtgtctttt tttagtctat agatttccct tccatctcct ctgcaattta 33120
tttttggaag aaactgagtt attgagtcat ttgtcatcta gaatgatgta cagtttagat 33180
tttgctgact gcattccatg gtgtggccaa acttcctctg tctggtgtat ttctcataag 33240
tgtgtaatta gatggaaagg cttggtcaga tttaaattag tttttttttt tctacaggga 33300
tatttgagag gtagtggtat gtatttccat caggaagcac ataatgtctt cttggctctc 33360
tttgtgatgt tagtagccat caatttcagt gcctagatac attaattaga tttctgtatc 33420
tcctactatc tcaaaatttt ccatcttctg tctgtgctac attctgagtc ctttcttcag 33480
ttataaattc taattcattg tataattctt gttaatctgt tgactttcta aaactggctt 33540
ttcatatcta aaaaaatttc ctttttcaa gtctgttcat tcctcatagt tcttactaat 33600
tggtcatttt tacatccttt tttttttttt ttttttttga gacggagtct tgctctgtca 33660
tccaggctgg agtgcagtgg catgatctct gctcactgca acctccgcct cccaagttca 33720
agtgattctc ttgcctcagt ctcccgagta gctgggatta taggcatcca ccatcatgcc 33780
cagctaattt ttgtattttt agtagagatg gggtttcatc atgttggcca ggctggtctt 33840
gaactcctga cctcaggtga tctgcccacc tcagcctccc aaagtgctgg gattacaggc 33900
gtgagccact gtgcccggcc tatccttaac tttagacatt gcacacagta caactgctat 33960
ccgtatcgga tagttcttaa ggtcagcagc tattgcttat tgtgtctgct gactctcttg 34020
gtagctgccc ttcttttgtg tctagtgatc tttgagttca ttgtttgatc ttaaccaggg 34080
gaactgtatg ggccaaaatt aggattgagg atatttttct ccatagagga ttttccttaa 34140
cttttgcagc agctgaaaga tgccattcag atggatctac attagtcatg atgccagaat 34200
tgggcaaatc tgctgacacc aggacacctg tgtatatgtg tttgtgtctt tccaggaatt 34260
cattgaagag ttgctgtctc cccctttgg gggtttagtg gcatttgtga aggaggctga 34320
ggctttgatt gagcgtggac aggctgagcg acttcgaggg gaagaaggta tgaggaaaat 34380
atggtaatga tgggatcagt ggtaagggaa gtggaaaaga aaaatgaaag gatgcagatt 34440
acatggtggg ggaatagagt atgaaagact ggattgagag aataccagaa aagagggttt 34500
gatgataagg atggctatac cttggggaga acttagtgga gttgaagatc agccagatcc 34560
ctctctgaca ctgtttcctc ctgctattag cccgggtaac tcagctgatc cgtggctttg 34620

gtagttcctg gaaatcatca gtggaatctc tgagtcagga tgtaatgcgg agtttcacca 34680
acttcagaaa tggcaccagt atcattcagg tgacctgcaa gtcccaggcc ccactcagat 34740
cccccatcat taattatttt ccccatcttt ttgctgggct cagcatcatc taagtgaccc 34800
ttggtcttca gttactagct gtgcccaaag ttctgtatga gccctaacct gattcttttt 34860
tttttgtttg ttttgttttt tttttgagac agagtctagc tctgttgtcc aggctggagt 34920
gcagtggcac agtctcggct cactgcaacc tctgcctccc aggttcaagt gattttcctg 34980
cctcagcctc ccaagtagct gggattacag gtgcctgcca ccacaaccag ctaattttcg 35040
tatttgtagt agagatgggg tttcactgtg ttggccaggc tggtctcgaa ttcctgacct 35100
cgtgatctgc ccgccatggc ctcccacagt gctgggatta caagtgtgag ccactgcgcc 35160
tggccgccta acctgattct taatcatcac tattagcacc attttacggt ttgatccctc 35220
aatgactttc tttgaccagg gagcgctgac ccagctgatc cagctctatc atcgcttcca 35280
ccgggtgctg tcccagccgc agctccgagc cctccctgcc cgggctgagc tcatcaacat 35340
tcaccacctt atggtggagc tcaagaagca taagcccaac ttctgatgtg ccagaaaccg 35400
ccctgagatc tgccggtcat ctccatggac ttctgcaccc cattccatac ccttcttcac 35460
ctggggtacc ccttccagtt ttccccttgc ttcccaggcc cttgacatgg cttacctgcc 35520
ttcactccca gcaccttgcc caacaggata agctggatcc ccttggcctt ctgaatatcc 35580
cagtgtcttc aggtttccca agaccacttc cctgtgggct tccaaaatgg cctttatcat 35640
ttctccagtc tgtcaccctc ctttcctgct cccatacacc caaggcttgt ttcttcccct 35700
gtaaaaacca ctgcctcaat ctctggttca ctcaactagt caccatgtcc tgaggcatga 35760
agcctcctca gctcttggaa ttgctggcaa ggggtgactg cctctgagtc attgtgtttt 35820
tcaaagtgat ttcttttctg tagctttttg acctaagatc tcagcaattt gaacactaac 35880
ctctcccctc ctggctcaag aattactccg aagtcagtct gcagaaaata aatatttagt 35940
atgacatgac acttatccca tttcctttct ccttcctcct gaaggttttt caggtggccc 36000
catctctaga atgacctttc ctttctgact acttcctggg cttacatccc ttctggtggg 36060
tgtaacctct aacctctagc tactcccagg ccattggagg aaggtggccc ctggctaaaa 36120
tagcagatgc tgcaagggat gagaaccagg ccgcgcagcc ccagacactg tcccaagggc 36180
tcccttggga caagcccaag ctgactctgg aatccctcct ccgtagcagc tccatagctc 36240
ctctggttgt ggctgtgaca gccccttgct cgggggctgc agcacccaac ccacacccac 36300

accgttgggg cagggctaga ccaggcgaaa ttcgtaccat ccgtttgttt gacttggagc 36360
cttcctgctg tcctcgccta tacgcctcgt ttgaacttag gctctagact ggagagacac 36420
ggggacccct ttaaggccta agaagagagg cgcagttaag gaaaaatgtt acgtttcctg 36480
tgtcctccac ccctacggcc taaacattcc ctccccaggt ccctggagag tggtggaaag 36540
cggttcctcc ccgcttaggc ccttcggatg caggtctagc ccgtcggcaa cgggaggtgt 36600
cctgagtggg tctgtgactg ccgagcacac ccgccgcgga gcggaggctg ctgcttcctg 36660
aggctgagag tggatccggc tccgggctct cctaattggc ggacgctggg gggcggcgtg 36720
gtaaggcaga acggagcggc atctcagctc tcgccttttc agggttccgc cccatatccg 36780
agagccgctc tctaattggc ttgggaaacc gtatctcagc gctttggcct agcgactttg 36840
aacgtgtttg cgcctgagac cgaagtgcag aagagggcga gcgcaggagg agaggcttgg 36900
tgaatcagcg attcctgatt ggccaggcgt gccttgaggg cggggccaga actgcgtcct 36960
taactgacct cgcccttgcc cagatcaccg cctccgcgtt gctccgggtt taccccgcct 37020
gactcgctgc gctatgcgtt ccctcacgcc tgccggatgc caggcggcga tgtgccaggc 37080
tctgaggggc cgcgagctca ccccagacgc cggccccggg aatcctctgc tcctccactt 37140
tcccttcccg ctactggttt cttgcccacc cactcccagg tgtcactctt gggacatcca 37200
gatgttctga catttgacct gactccagcc tcagcgcgag gacgggagaa gggccaaggg 37260
tatgagattt tggacaggag aggcattggc tactctgaca aagagcgtgg attcccaaag 37320
aaagggtccc cagatacccc gcaggggaga ctgtcgagac aggcgacttt agccaactaa 37380
tgctcgcacg cgaggagggc tgtgcaggca ggcaccacgg ttcttcacag ccttccttct 37440
ttccttcccc tgtccgtcgc agaacccaaa tcctcagagc tgatcgagaa gcgcgtgttg 37500
ttgcagaagt cactgagggg cagacctgga atgtgagccg tggggcgaag ggacagctct 37560
ggagactcgt gattccggaa cccggtgggc attcaagtga tactggagca cgcagttttc 37620
ggggacatta agctcgcccc aggggtccac aggaaccctc tgacccaacg gtctctctgg 37680
acaggatgat ccagaacaga cggtgaagag acaaaacagc gtgaaaggga gcggcgatac 37740
ctaaactgac ctccggaggg cagcatgatc aagggaagga gctgttctcg ctccgctcaa 37800
acccccgcgc agcctctgca gcttactcct gcttcggaag gcggcgaggt tccaccccca 37860
ccaccccggt ccccgccgcc ctcttcgcgc tgaagctgcg gagggtcttt ttcttcagcc 37920
cccaaatcct ctgctctgtg gcttaagatt cccaggctta aacccataac tgctagggtc 37980
atcactcctc aaacttctcc ctcacttgtt ttgctgatga cggacacagg ggcctcacag 38040

actcagaagc atctggagtc atttcgaagg acagaactgt ggcaatatcc tctttccccc 38100
tcctaatctc tgagaacagt gtctgtatat ggcacggggg tccctagtgt catatagaag 38160
gactcggaga tgtcctcatg ggctgtaacg gcccccggtg ctattcaggg atctctgttc 38220
tttaaagaaa tatcatttcc ccttatttct ctacctttgt gccctcacct cgccctgaat 38280
tgtttcttaa actgggcttt ctgggaaacg tttacttcgt gttcaacagc aaagtctcgg 38340
cataaggcga gggcggcagg gggtgggggg cgggcgtttg ctcgccttgg cattaatttc 38400
agagctgctg aacgtggaca aaagagaggg aacatcctcc tttcttccat tcctgtaaat 38460
atggacccac ccaccctcca cttaagatgg gtggcttttc tgattttaag atatcaagca 38520
gcctggcgag gtggctgaca cctgggatcc cagaactttg ggaagccgag gcgggaagat 38580
cccttaaggc caggagttct agaccagcct ggccaatatg gcgaaaaccc atctctacta 38640
aaaatacaaa aattagcctg gcgtggtggt gcctgcctat aatcccagct actagggtgg 38700
ctgagtcagg agagtcgctt gaacctggga ggcggaggtt gtaatgagcc gagatggcca 38760
ctgcactcca gcctgggtga cagtgagact ctgtctcaaa aataataata ataataataa 38820
taaaaagata taaattggct gggctttaaa ataaagtagt cagaggccca gggtggtgac 38880
tcatgcctgt aatccttttg gaaggcagag gccagaggat cttgagcaca ggagtttgag 38940
actagcctgg gcaacatagg gagacccatc tctaccaaaa aaaaaaaaaa aaaaaagcaa 39000
aaagccaagc atggtggccc atgcctgtgg tcccagctag ttgtgaggct gaggcaggag 39060
gattgcttga gaacaggagt tagagattgc agtgagctat gattgcacta ctgcactcca 39120
gcctgggtga cagagtgaga ccctatctct ttttaaaaaa ttaaaaaaca aagtagattt 39180
agtgtgaaga ttcaaggaaa gaaagagaaa gcaagacaaa actttaagga gaggagaatc 39240
gaaattggct gacttcaccc tggacataat ggacacagct attcatgtta cgtactagcg 39300
ttaacaaagt gttttcagaa gcagttgtca ttaaaccaac acatttattg aagctcttct 39360
ctgtgttctg tgataggcac tgcggattca gcagagacta aatcaaagtc cttgatctcg 39420
gagagcacat atttcatcta attgaattca cataccagcc ttatgaggag gtgttcttcc 39480
tgtttcaggt aatgagcttg aatttcaaat aatgagttca aaaccatata ggcactcagt 39540
ggctgaacct tctgtgccca cattcagctt ttgttctatg actccaggct gggagtcatg 39600
gagctgagga ggcagccggt tgtagagaag caacagatta ataagtaata gtaaattatt 39660
gaaaaaatat ttacagaact gaaggaaaag ctgaacaggc ggtctttgga aaggacagaa 39720

gccaggatag ttccagcatc caggcagcag acaccaatgg tcagtgcttc tactttggat 39780
caaaaagaga gaactccaat aggcttaggg tgggtcaggt gctcacactc atctgaggaa 39840
ggggagggtc ccttgaggaa tagtaccata gactacccaa tgttggagga taatggcccc 39900
aacgcaaaaa tgggatcttg ccaccagaca aatgggatat agatgccagg ctgcaaaatc 39960
caacaaatgt acacgtgccc aaatcccgac tccatcacta atagccatgc aatcctctga 40020
ctatcaatta gattaattga cactcttttt tctctcgcta ctgtggtaca tgcagtagtt 40080
acctttattc ttcactttgt tttctcataa gactaacaag cagtttttta aaataaagaa 40140
ataaaagcaa aattgcttat tgtcttagtc tattcattgg caaactagta acattaaata 40200
aactccagac agagccactg gagaagaatc aagtacaact tagtagaaat aacaccgtat 40260
gtgtgaaggt gagtgtgtgt gtgtgtgtgt gtgtgtgtgt gtgtgtgtgt gtgatggaaa 40320
atactttgtc ctgcccaaac tgtaagtcat gaccatgccc ctctcagcag aagtttatct 40380
tcatctcatt tggaagttta taagaacaca gcttaaaaaa aacctgtgat tttggagcaa 40440
ggtcaagcct ttgctattgc ttccacattt tgttttgttt tatttattta tttacttttc 40500
agttttttgg agttttcttt atttgtttgt gtttttgaga cagggtctcg ctctgttgcc 40560
caggctggag tgcagaggca ccatctcggc tcactgcaac ctctgcctcc cgggttcaag 40620
cgattcccct ggcttagcct actgagtagc tgggattact ggcacgcacc accacatcca 40680
gctaattttt gtattttcag tagacactga aatacatgtt tgccatgttg gctgggctgg 40740
tctcaaactc ctggcctcat gtgatctgcc tgcctcggcc tcccaaagtg ctgggattac 40800
aggcatgagc caccacgcct gcctagtttt tagtttttttg agacagagtc ttgctcagtt 40860
acccaggctg gaatgcagtg gcatgatcac agtttactgc agcctcgacc tcatggattt 40920
aagcaatcct cccacctcag cctctgggat agctgggact acagtcatgt gccaccacac 40980
ctggctaatt tttgtatttt ttggtaaacg aggtttcacc atgttgccca ggctggtctt 41040
gaactcctga gctcaagtga tctcctacct cggcctccca aagtgttgga attacaggca 41100
tgagccactg ccccagctgt tttattaatt tagttaaaaa tatatgaaaa gaggccctgc 41160
gcggtggctc acgcctgtaa tcccagcact ttgggaggct gaggtgggcg gatcacctgc 41220
ggttttggag ttcaagacca gcctgaccaa catggagaaa ccccatctct actaaaaata 41280
taaaaaatca gctaggcatg gtggcacata cctgtaatct cagctactcg agaggctgag 41340
gcaggagaat tgccctaacc cagaggaggt tgtggtgagc caagattgcg ccattgcact 41400
ccagcctggg caacaagagc gaaactctgt ctcaaaaaaa aaagtatata tatatatata 41460

tatatatgaa aaggctgggt gcggtggctc acgcctgtaa tcctaacact ttgggaggct 41520
gaggcaggtg gatcacctga ggtcaggaat ttgagaccag cctggccaac atggcaaaac 41580
cgtgtctcta ctaaaaataa aaaaattagc caggcatggt ggcaggcgcc tgtaatccca 41640
actacttggg aggctgaggc aggtggatca cctgaggtca ggaatttgag accagcctgg 41700
ccaacatggc aaaaccctgt ccctactaaa aatacaaaaa ttagccaggc atggtggcag 41760
gtgcctgtaa tcccaactac ttgggaggct gaggcaggag aattgcttga actcaggggg 41820
cagaagttgg cagtgagcca agatcacacc atttcactcc agtctgggca aaagagcaag 41880
attctgtctc aaaaaaaaaa aaaaaaaaaa aaaaaaaaat atatatatat atatatatat 41940
atatatatat atatatatat atatatatat acatacatac acacacacat acacacatac 42000
aggcactagc caggcgtggt ggctcatgcc tgtaatccca gcactttggg aggccgaggc 42060
gggcagatca caaaacaggc agggcatggt ggtccacgcc tgtaatccta gcactttggg 42120
aggctgaggt gggcagatca cttgagcaca ggagttcgag actagcctga gcaacatggc 42180
gaaactctgt ctctaccaaa aatacaaaaa ttagctgggt gtggtggtgc atgccagtaa 42240
tcccagctac tccggaggct gagaggcagg aggattgctt ggggaggcag aggttgcagt 42300
gagccgagat ggcaccacta gactccagcc caggtgacag agggagaccc tgtctcaaaa 42360
aaaaaaaaaa aaaagaaaga aaagaagaaa agaaaaacaa atcacctgcc ttatgaagaa 42420
acagtttgta atagatataa gacacagaat gtgcagcccc tggtatggaa taagtcctta 42480
gaaaatgtta gtttcctctc tgtccctgtg tgacatgaag ggaattccag ggccagtgcc 42540
acttcctggt ggtttccctc acttaagtgc tgtgtacccc attgcaaaag catgtcttcc 42600
aggttcctga taccacatcc catgtcctct gcctttgggg ctctcatagg attaagtgtc 42660
attttcccta tgaactagaa aagaatcttc tgaacgtctg ccttgggact gatgatatag 42720
ctgagctgaa aggacaatta gttttcctcc ccagaccaac ccaaccttca aggctgggga 42780
ctcatttgat gctcccattc aaacatttgg agaaaattgt ctctgagtac caggtctttt 42840
tagcatattt tctccaaatg ttacatcctt aggggctttg aatagtagaa gagccttcaa 42900
cattggttca gatggaagca tgcaggcatg tggagagttg ccactccctg atccctgaaa 42960
caaggcttag gatggaaaag agcctcctgg ggaggttggc tctgaatgta aagctgggag 43020
aggcacctcc tgtcaaccta agggcttgtt ggtaggggca acatgttgac ctggcttcct 43080
tcccactctg agacatcact ggctctcatg cagagctatt ttccttcttt ctttctttct 43140

ttctttcttt cttctttctt tctttctttc tttctttctt tctttctttc tttctttctt 43200
tctttctttc ttttcttttc ttttctttct ttcttttttt ttttgagacg gagtttcact 43260
cttgttgccc aggctagagt gcaacggcac gatctcagct caccacaact gccacctccc 43320
tgggccaagc aattctcgtg cctcagcctc ccgagtagct gggattacag gcatgtgcca 43380
ccatgcccag ctaattttgt atttttagta gagatggggt ttctccttgt tggtcaggct 43440
cgtctcaaac tcccaacctc aggtgatcca cctgccttgg cctcccaaag tgctgggatt 43500
acaggagtaa gctgccgtgc ctggcctttc attcttttct ttccttcttt ccttctttcc 43560
ttccttcctt ctttccttct ttccttcctt ccttcctccc tccttccctc cctccctcct 43620
ttcttctttc ttctctttct ttctccttcc ttccttctct ctctcttttc ttttctttct 43680
tctttctggg tttcgctcta ttgccgaggc tggagtgcag tgagtggcac gatcacggct 43740
cactgtagcc tcaacttcct aggctccagc gatcctccta cctcagcctc cttagtagct 43800
gggattgcag gcatgagcca ctgtgcccag tgtaatgcag ggctcttcat ccaggatccc 43860
aaagggcaaa caaaaatagg agaaaattac atttcatttt cactaatctc taactgaaat 43920
ttagcatttt ctccaatgat aaatgtaggc aataaaccta caccggtatt tgtagtacat 43980
gtgactgttg ttaatagaat ttacacattt gtgtcatctt ccagtagttg cagaaatttc 44040
aaaatgtcat gtatgctcac ggttactttg tagttagtgg tagtggttag actcaccact 44100
agatcttctt cttacacatt ttgtttcatt ttgacaactg tacttcaata tgattgtttt 44160
cctttgtaat cccatttatt ttactttatg cagttgaaga ttattctgac actccacaga 44220
cttcaccaga cttccaaaac ctgtcctaga cgtaaaaagt taagaaacct taaatctcta 44280
atgatgctgt catttcctgt tttgtattat cttacaacca gggtaattag gaatgccttc 44340
ttcttcttct tttttttttt tttttttga gacggagtct tgccctgtca cccaggctgg 44400
agtgcagtgg cacaatcttg gctcactgca acctctgcct cccgggttca agtgattctc 44460
ctgcctcagc ctcccaagta ggtgggattg caggcatcca ccaccatgcc cagctatttt 44520
tttgtatatt tagtagagat ggggtttcac catgttggcc aggctggtct tgaactcctg 44580
acatcaggtg atccacccac ctcagcctcc caaagtgctg ggattacagg catgagccac 44640
cgtgcctggc ttgctttctt cttgtttaaa ctaaaggtct tcttgtaaaa ttcctgctta 44700
tattttcttc attattactc aaattcaatt attcatttat ttgccaaata tttagaaaac 44760
acttctcttg tacaaaacca tgctgcataa aactgttatt atgcgaaaaa taatccttac 44820
tcgccatgcc ctctaccccg accccccaac cccatttaca atctggtaga gctggactgt 44880

ttacccacat aggacacaca ggctgcagct taccatgggc agggagtggg gatggttgtt 44940
ccaggtgcag gtaataagaa ggtgtattgt ctgtggagaa tttaaaaata attctaaaac 45000
caactttttt tttttttttt tttttgagat ggagtctgac tctttggccc aggctggagt 45060
gcagtggcac catcttggct cactgcagcc tctgcctccc gggttccagt gattctcctg 45120
cctcagcctc ccaggtagct ggcattacag gcacacacca ccacgccagg ctaatttttg 45180
tgtttttagt agagacgggg ttttaccacg ttggccaggc tggtcttgaa ctcctgacct 45240
caggtgatct gcccacctca ccctcccaaa gtgctaggat tacaggtgtg agccactgcg 45300
cccagcctga agccaacttt ttattgatac tgcgcaccag caattataaa aaaatgttag 45360
tgacaaaata ccccttgcag tgtagcctcc tccccccact ccaccctgct tggtatgcca 45420
ctgcacacag gggagtttaa ccatttccag tttacacaca cacacacaga cacacacaca 45480
cacacacaca cacacacaca cacaaacaca cagagccttt gagacttcct tttctcctca 45540
actcccccag cccagttctc atgtcactct actcagggta ggatctgcag cactgctggg 45600
ccagtatccc caaaggtgag ccctgtaagc tccttggcct ctggatggtc tcattaatag 45660
tccaggatga gccctgcaat cttggcatct gtaggcaggt ctcagctctt cttcacaccc 45720
ctgggcttga accccttggc aactgcgttt gacagtacat gattcccatt cttcatctcc 45780
agattttcca tccttcaaga aaacactgga tttcctggtc aagccaaggc ttaatgaact 45840
ggcctggcag cttctctggt cctcaagttt ccttttcttg ccttactttc tgaagacttc 45900
tagtagtttt acttggaagt gtcactgtct ccatttttat tcctgagttt tctgcagttc 45960
ttttttctgt ggccccacgg actcatccag agcctgcagg gagctgacac ctcatctgag 46020
ctgctagaat tgaggtgaga aaggaggttc ctaagtgcag aaatgtgttt tctcctctcc 46080
agccaccact gaaggctttg gatgtgattg agtcagacct ctcctgcaca gtctcccttt 46140
ggattacctc aagataaacc aatttgtgac tttaattaca tccgcgaagt cccttcccag 46200
ctgcacctaa gctaaagtgt gattgaatga atgaaagagg gtgtgctggc gatcagctag 46260
ggaagtgatt ccaagggtcc ctagactcca cattaccaac tgcaaaatgt gacgttctta 46320
cgttctgaca agcgaactct cctcccaaat tcattgcctg agttgatgca aatgaattca 46380
gccacgcctt ttgggtttgg catctgagaa gtgtctgtga cttgggaaga gtggtcagaa 46440
tggcgataat gggaagagac cacatttcag ctggtggaga gtgaaaggac agccaacatc 46500
ttgccctttc tgaagcatac acacattgga gattgaaagt gtctgttggc ctcacaggca 46560

ctgaggaaag cctcccatcc ccttagattt tcctcctttt tcctgaggtc ccctgaacct 46620
aatagtctgt aaccttattc catgtctggg tcaacaaacc accttccaag gaggacttga 46680
atataataat ccgtttcctc tgcattcctc cagaacacag agtccacacc atccatgagt 46740
ctgtcaaaca aaacattcct cattggtctc aatgttgagc actgaaagtt ctctgagctc 46800
atttagcacc aactcatcat ccttaaaatc cacaggatgg gccgggtgca gtgacccatg 46860
cctgtgacta caacaccttg ggaggccaag gggcagatct cttgagccca ggagttcaag 46920
accagcctgg gaaacatggt gagacacctt ctctacaaaa aaaaattttt ttttttgaga 46980
cggaatctca ctctgtcgcc caggctggag tgcagtggtg caatctcggc tcgctgcaac 47040
ctccgcctcc agggttcaag cgactctctg cctcagctcc cgagtagctg ggattgcagg 47100
catccaccac catgcccagc taatttttgt atttttagta gagacagggt tttaccacgt 47160
tggccaggct ggtctcgaac tcctgacctc gtgatctacc cgcctcagcc tcccaaagtg 47220
ctgggattac aggtgtgagc caccatgccc ggcctacaaa aatttttta aaaaaattag 47280
ccaggaatgg tggtgtacac ctgtggtccc agctacttgg gaagctgaga taggaggatt 47340
ccttgagccc aggaggttga ggctgcacag tgagctgtga tcacgccact gtactccagc 47400
ctgagcaata gtgagacctt atctcagaaa acaaacaaaa caaccaaaaa tatccatagg 47460
atgaaacata tgtttgatta tgacaacttg cttatggtac cttctggatg gcccagctcc 47520
cttgcagatc cccagaccaa cagcttttgt tgtagtgaca gcttcttctt gtaacctctt 47580
tttttttttt tttgagatgg aatttcactc ttgttgccca ggctggagtg caatgacatg 47640
atctcggctc actgcagcct ctgcctccag ggttcaagcg attctgctgc ctcagcctcc 47700
tgagtagctg gaactacagg tatgcaccac catgcccggc taatgtttgt atttttaata 47760
gggacggtgt tttgccatgt tggccaggct ggtctcgaac tcctggcctc aggtgatcca 47820
cctgcctcag cttcccaaag tgcttggatt acaggcgtga gccactgcac ctggcctaca 47880
gcctctaatt tcattttcat ccaaaagctt taatggaagg cccacagatt ccctcttcaa 47940
gtaacagcaa agcgcatctc ctttaagatt tctttgatca tcttcataaa gcttgacttt 48000
gaagtcttct gaggatttct cctggaggca aaccagatat atagacattt gtatttctgt 48060
cttcttcaac atgaaattat cctcattcca tttttctttt atctcccctg tttttaggat 48120
cagtgggttt ggggggtttt ctttgcagaa attctgcatt cctagcattg acattttgta 48180
catttgcagg agagctagat gccccaccat tagaaaagcc acacttggct gggtatgtag 48240
caatgaaacc ttcagtggcc ttgggaaacc acgccttctt gtccagatcc cactcattgt 48300

gggagtcagt tggctgctgc cctaaataac ccgtttgtca ctgagatctt cttgtctgtt 48360
gcagtcctta gtgtctccat ataatttatg cattcacaac tgctcatcaa gtcatcatta 48420
tcatccactt catgtttcct acctagccag gggcagggtt caggtaatca aagaggctgc 48480
tctgctgggc ctgcaaggct ccaccaagtg tgtctgaggt gacagcccaa gcactgttat 48540
aaatatttgc gcactgttgt aaacatttgc attagtccct tctcgcactg ctataaagaa 48600
atacctgaaa ctgggtaact tataaagaaa agaggttgcc gggcgcggtg gctcatgcct 48660
gtagtcccag cactttgggt ggctgaggca ggtggatcac ttgaggtcag gagtttgaga 48720
ccagcctggc caatatggtg aaaccccgtt tctactaaga atacaaaaat tagccgggca 48780
tggtggccag cacctgtaat cccagctact tgggaggctg aagcaggaga atcacttgat 48840
ccggggaggc agaggttgca gtgagccaag atcatgccac tgcactccag cctgggtgac 48900
agagcaagac tccgtctcgg aaaaaaagaa aagaaaagat gtttgctggg cacagtggct 48960
catgcttgta atcctagcag cactttggga ggctgaggcc ggagggttaa tcacttgagc 49020
tcaagagttt gagaccagcc tgggcaacat ggcaaaaacc cgtctctata aaaaattcaa 49080
aacttagctg ggtatggtgg catgtgcttg tggttctagc tacttgggga gctgaggtgg 49140
gagaattgct tcagaccaga aggtagaggc tacagtgagc tgtgattgcg ccactgcctg 49200
ggcgacagag caagacccta tctcaaaaaa aaaaagaggt ttaattggct catggttctg 49260
tgggctctac aggaagcatg atgctggcat ctgcttgcct tctggggagg tctccagaaa 49320
cttacaatta tggcagaagg caaaggagga gcgagccttc tcacatggca ggagccgaga 49380
gagagggagg aggtgcggca cacttttaaa caaccagagc ttgccagaac tctctatcat 49440
gagaacagca ccaggaggat ggtgctaaac cattcgtgag aatccactcc cataatccaa 49500
ccacctctca ccaggctcca cctccaacac tgggactaca tgagatttgg tggggacaca 49560
gatccaaacc atatcaacat ttaacacatt gcatctttga attgtcacag caaccctgtg 49620
aggtgggtac tagtgttatc cccattttac agagaggaaa ttgatgccca gagaccttgc 49680
caaggttata gacaggacag ttagtgcatg acaaagctgg aattggaatg cagccagttc 49740
tgctttagaa gccactttct tgcctgcctt tctttcttta tttctttatc ttttctttct 49800
ttctctctct ctccttcctt ccttccttcc ttttctttct tttcttttct tttttttttt 49860
tttttttgag acagggtctg gctttgttgc tcaggctgga gtgtggtggc accatcttgg 49920
ctcactgcaa cctccacctt ctgggctcaa gtcatcctcc tacctaagct tcccaagtag 49980

ctgggattac aggtgcatac caccacacct ggctaatgtt tgtatttttt ttagtagaga 50040
tggggtttca ccatgtttgc taggctggtc ttgtactcct ggcctcccaa agtgctggga 50100
ttagaggcgt gagctattgc accccgcctg aagctacatt cttttttttt tttttttttt 50160
aatgagacga gtcttgctct gtcccccagg ctggagtggt gcagtggcgt gatcttggct 50220
cactggaacc tccgcctccc gggttcaagc aattctcctg cctcagcctc tcgagtagct 50280
gggattacag gcacctgcca ccactcctgg ctgtttttc catttttaat agagacgggg 50340
cttcaccata ttggccatgc tggtctcaaa ctcctgacct caagcgatcc gcctgccttg 50400
gccttccaaa gtgctgggat acaggcataa gccatcgtgc ctggccaagg ctacattctt 50460
aaacgccact tttacagct tccaagaaac taactgggtg aaatccagcc tgcctcctgt 50520
ccttgttttt atcatttcat agtgaaaaca taaactccca agcccaccta ctctattgca 50580
ctgtgtctcc atgacactct gtaccttctg gctctgactc agccctccac tttccccctc 50640
cctaacctgc ctgtgccctc taataaactg tagcccccct catacccctta gcttctcctt 50700
ggagcattcc ctccacttcc tggcgttaag tgaaatttaa ctctcccttg agggcactac 50760
ctcctttgca ggcttctgaa atgagagctg ttgattttct tacagctgag ggtgctctcc 50820
tggcttcaga ttgctgcctc cggactactc ctcccccagc ccccaactct agtttaaaat 50880
aacccgcggc cttccaggca ctgcaattag gctaaacttt cctcttcctt tctcactgct 50940
actgtctgcc agacccctcc acctcactca ggggaacgct ggcccagctg accgtctgtc 51000
tttgcattga gtcctccctc tgggagactt cattgtccac ctggaggatc cattccctac 51060
ttgacctctc aataacctgg ccacttaatc cccaagaatc ttctcctctc caactcggcc 51120
acacattctc attgtcatac cctggacctt atcacctgaa agagcccctc cttgagaatc 51180
agtcaagcag cctgctcttg gcccccacct cctaaccgtc tgtccatcag tctgtctgcc 51240
ctcctcgcaa ccaattttca gtctcctagg acctttggat caatgatgcc ttccgtcttc 51300
cagcttatga gcccctccct attcagctca actccacaac ccttcatttc aacacctctc 51360
tggacaatgt cgccaactcc ccttcccctc tgtattttca tcctttgtac ccaagtggca 51420
aaaccccaac cctggatgag cccagtgatc tgttttcttc aggattttac tctgatagcc 51480
aagaactgtt ggtggtggtg gggaaatca cacaatcagg aaatggttcc cactcctcac 51540
tcatgatcat gaactctaag gaggctccca gaattgttta gtggcccaac cacatcactc 51600
tggtcaatgg ctacgttaaa cctccctgat ccttgaacct ctgaggctct tctgtctctc 51660
acctcatgct cacttgatga cctccctctt atgtcacaga gaaaatgaaa gtcatcagag 51720

gtgagctccc tcaaccatct gtcaccagat ccacacccct ccaagaggaa cacaatgcat 51780
gcagcgcatg taccttgatt tgatcctgat ttgaaatata tgcataggct ggatgcagtg 51840
gctcacgcct gtaattccag cactttgctt gagctcagga gttcaaggca ggtggattac 51900
ttaagctcag gagttcaaga cccacctggg caacagagaa aaaccctgtc tctacaaaaa 51960
aaaaaaaata caaaaattag tcaggtgtgg tgggtcacac ctaaatagta gttccagcta 52020
cttaggaggc tgaggtggga ggatcacttg agcctgggag gtggaggttg tagtgagctg 52080
tgattgcact gctgcactct agcctgggtg acagtgagac ccagtcaaaa aagaaagaag 52140
gaaaggaagg aaggaaaggg aggagagaga gaaaggaagg aaggaaggaa ggaaaaacag 52200
aagaaaggaa ggaaagaaat taaaaagaaa gatatatata tatataaata tatatataaa 52260
agacattttg aggctaactg gggaatattt gagtatattc tgctatcaga tgaagtgaag 52320
aaatgatcgt tattttctaa ttttgttttt ttttttgaga ctgagtcttg ctctgtcacc 52380
aggctggagg tcagtggcgc aatctcagct cactgcaacc tgtccctact gggttcaagc 52440
aattctcctg cctcagcctc ccaagtagct gggattatag gtatgcacca ccacgcccgg 52500
ctaatttttg tatttttagt agagacgggg tttcaccata ttggccagga tggtctcaat 52560
ctgttgacct tgtgatcctc ctgcctcagc ctcccaaaat gctgggatta caggtgtgag 52620
ccaccgtgcc tggccatgat cattattttc ttagctgtga ttacgtattg tggttatgga 52680
atctccttat ttttgggaga tgcatgctga ggtacttagg tgtgaagcac atgctttcct 52740
atattttcat atggtccaag aatgttttag tagtcactca acaatttcta tgtttaaaaa 52800
aaaaaaaaag ggctgggcac agtggctcat gcccgtaatc aatccgagca ctttgggagg 52860
ctgaggcagg cggatcacct gaggtcggaa gttcaagacc agcctgacca acatggagaa 52920
accccatcta tagtaaaaat acaaaattag cttggcgtgg tggcgcatgc ctgtaatcac 52980
agctactcgg gagtctgagg caggagaatt gcttgaaccc agtaggcgga ggttgcgtac 53040
agccgagatc gcgccattgc actccagctt gggcaacaag agcgaaattc catctcaaaa 53100
caaaaaacta aaaacaagcc cacaaagacg tatataggcc ttggcactgt ggctcatgtc 53160
tgtaatccca gaactttggg aggccaaagc aagaggatca cctgagtcca gtttgagacc 53220
agctgggcaa caaggcgata ccttgtctct acaaaaaatt taaaaattag ccgggcatgg 53280
tgccatgcac ctgtggcccc agttacttga gaggctgagg tgggaggatc gcttgagccc 53340
aaaagtttga agctgagatg agctatgatc aggtcactgc actccaccct gtgcgatgaa 53400

gtgagaccct gtcttggaaa aaaaaaaaaa gaaccatata tatatgtgtg tgtatatata 53460
tatgtttgta tatatataat atataaatat ttgtatatat gtataaataa caattgttaa 53520
atctaagtaa aatgatcgac atacaaacaa tattgtatta tattttcaag ttttctgtat 53580
gtttgaaatt tttcataata aaaaaactgg gcaaaattct ctcacttcca ctcactgctc 53640
gaccctttca ggctggtgtt tatctctacc ttgctgctac tcaaatgttt ttttgcaatg 53700
aactttatgt cattaagtcc attgtttcgt tttcttctta cttgaactgc cactagtagc 53760
tgacgttgct ggccagttct ttcttgaaac actgtcgttt tttggcctcc aggatttggc 53820
ctcttctggc ttcctcccat ccctccatcg gctgctcttc cacccctcag ccggctcact 53880
ccgcctttga ctgaaatcac ctggtcatat tggagttcat caagagaggt cggtcctgga 53940
ccctcctctt cttatgctac cctttccttc taggggatca cacctgtcct gtggcttcag 54000
ttcccatcag ctggttactc tcaaatttat ctcttgcctc aaatctccag atttgaccac 54060
cttcctgcct ggcatcccta cttggatgtc ccaaaggtat tactaactta gctttttttt 54120
tttttttttt gggagacaga gtttcgctct tgttggccag gctggggtgc agtggcaaga 54180
ccttggctca ctgcaacctc cgcctcctgg gttcaagtga ttctcctacc tcagcctccg 54240
gagtagctgg gattacaggc atccgccacg acacccggca aactttttgt attttcagta 54300
gagatggggt ttcaccatgt tggccaggct ggtctcaaac tcctgacctc aggtgatcca 54360
cctgcctcag cttcccaaag tgctgggatt acaggcatga gccacggtgc ccagcttaac 54420
ttagcatatt tgaaacagaa gtcatgatct tcccacagaa gcagtatcat cctcagatct 54480
gaaccacagg ggccctgccc tggcccacat actttagggg gattcaccta tcacaaacaa 54540
cttatcacac acatttatta catgacacct gggcacctct gtcactgatc tgctgtcagt 54600
gctggaacag cacaacctga aatcttacac atctgctctt gtaatcaacg ccgttcaagc 54660
cctggggaat ggtgacttct gtaatctctt gccctgtatc ctggaaacaa aagatgacta 54720
tagccaattc tgtgaaaatc tgaggttgtg cctctgcctg gattctgaag catgtactgc 54780
tttggagtgt agggaggatt taagcagtgg aaacatgtaa gtgaaatcac tttaaccagc 54840
ttgttaaata ttacctctca aatagcatga caaagtatca tttcccaata gtgttgagtg 54900
tggtttttct tgtttctctt tgttttagag gtatggatgt atgttgtgct gcatttacaa 54960
cagttgcaaa ggatagctga ggaacatgtt gctgtattaa acaatcctta ttactgttaa 55020
atttatatgc atgtggtggg aggggtggtg agggataaaa aactacacat ttacagtgta 55080
cactcctcct gtgatggggg caccaaaatc tcagaaatca ccactaaata acttattcat 55140

gtaactgaac accacctgtt ccccaaaagc ctattgaaaa aaaaattgaa tacatgtagg 55200
tctagatata actcttgtga agtatgatat tgattcttta tgttgtaata gatggacatt 55260
gaatacgaga ttatgaatac aatcatcctc agtattcacc agggattgat tccaaaaccc 55320
ccactgatgt tcaaacccct tgtataacat ggcatagtat ttgcatataa cctatgcaca 55380
ttcttccaca tactttaaat catccctagg ttatctatgg tgcctatcgc aatgtaaatg 55440
ctgtataaat atatgttata ctgtattttt tatttgtatt attttttctt tctttccttc 55500
cttccttccc ttcccttcct tccttccttc cttccttcct tccttccttt ctttctttct 55560
gacagtcttg ctgtgtcacc caggctggag tgctatggca ggatatcagc tcactgcaaa 55620
ctctgcctcc caggttcacg ccattctcct acctcagcct tccaagtagc tgggattaca 55680
ggtgcccgcc accatgcccg gctaattttt ttgcattttt agtagagatg gggtttcacc 55740
gcgttagcca ggatggtctt gatctcctga ctttgtgatc cacccgcctt gacctcccaa 55800
agtgctggga ttacaggcgt gagccaccgc gcccggactg gccatatata ttttaaaaat 55860
atttttattc ttcagttggt tgaatccata ggtgtggacc tacagataca aaatgccaac 55920
tgtagtttaa tttttataaa actatctgtc ttagtccatt cagactgcta taacaaaata 55980
ccttacactg agcaatttat aaataataaa cactgcttac agttctggag gctagaatgt 56040
ccaagcaggt ggtgccagca gatttggtag ccagtgaggg cacactttgc ttcatagatg 56100
gcgccttctt gctgcatctt cacgggtgga aggggcaaac aagctccttt gggcctcttt 56160
tttttaagac agggtctcgc tgtgtcacct aggctagagt gcagtggcgt gatctcggtt 56220
tactgcaagc tctgcctcca gggttcaagt gattctcttg cctcagcctc ctgaatagct 56280
gggattacaa atgcctgcca ccatgactgg ctaatttttg tgtttttagt agagatgggg 56340
tttctccatg ttgaccaggc ttgtcttgaa ctaccgacct caaatgatta gcccgcctga 56400
gcctcccaaa gtgctaggat tacaggcatg agccaccgca cccagcctgg gcttctttta 56460
taacggcatt aatcccattc atgaggacag gcccctcatg acctaatcac cttctaaagg 56520
ccccatctct taatatcacc acattgggga ttaggtttca atttatgaat tttagggggg 56580
cacaaacatt tatgccatag caatacataa taaaaattaa taaatatgta tatatttttt 56640
tgagacaggg tctcactctg tcaccaggct ggagtgcagt ggcacaatca tggctcacta 56700
cagcctcaac ctcccaggct caggcaatcc ttctacctca gcctccagag tagctgaggc 56760
cacatatgtg tgccaccatg cccggctaag ttttctattt tttgtagaga tggcatttca 56820

ccatgttgcc cagggggtc tcaaaacact gggctcaagc agtctgccca ccttgacctc 56880
ccaaaacact gggatttcag gtgtgagcta ctgggcccag cccaaaattt caaaaaaaag 56940
caggcaaaaa gtcagcttta tctaaatgtg tttgatttaa aatattgatg tgtattcatt 57000
atcatttata ttttgtccag atgccgtgtc tcatggctga gattccagca ctttgggagg 57060
ctgaggtggg aggatcactc gagctcagaa atttgagacc agcctggaca acatagtgag 57120
accatctctc tacaaaaact aaaaaaatta gctgggtgcg atggctcatg cctgtaatcc 57180
cagcactttg ggaggccaag gcgggtggat cacttgaggt caggagttcg agaccagcct 57240
ggccaacatg gttaaacccc acgtctacta aaaatacaaa aaattagcag aagaatgctt 57300
gaacccatat gtatacatgt atatatacat atgtgtatgt gtatgtttat atatatatgt 57360
atatgataca aaattagcca ggtgtggcag cacacacctg tagtcccagc tatttgggag 57420
gctgagatgg gaggatcact tggacctggg aggtcaaagc tgcagtgaac tgggatcatg 57480
gcactgtact ccagcctggg tgacaacgtg agatcctgcc aaaaaaaaaa aggaaaaata 57540
aaaagagtga ttttacagaa aaattgagat tttcaattta tcacggcagt gccagctctg 57600
aattcctatg cggcaacgct ggaccaggag ctgctgcccc tctaggaggg gagggtgccc 57660
tccagcctgt tatagccttc ccttggcact tggcacatta cttgcctggc ccttacagga 57720
atttgaggtg caaattgatc tagatcattg aaggaagagt ctgggtcttc tcagttttgg 57780
ggcactttcg ttgccaagta aggttcttat gagattttta gaagttgaga ttaaaaaaga 57840
gtttatatgc agctggaggt gatacctaac aacaaccata ataacagcat caacaacaaa 57900
aacagtagcc tagagcagtt actctaagcc aggtaagtgt tctaaagcac ttcacacaac 57960
ttatttactc tcacgacaat gctattaggt agcactttga atacacacat tttgtaggtg 58020
aggaaattgg ggcatgtagc tgttaagatt tatatcagcc gggcgtggtc actcatgcct 58080
gttcccagca ctttgggagg ccaaagcggg cggatcacga ggtcaggagt ttgagaccag 58140
cctgaccaac atggtgaaac cccgtctcta ctaaaaatac aaaaattagc tggacatggt 58200
ggcggctgcc tgtgatccca gctattcagg aggctgaggc aggagaatcg ctttaatctg 58260
ggaggcggag gttgcagtga gctgagatca tgccactgcg ctccagcctg agtgacagtg 58320
agactctgtc tcaaaaaaaa aaaaaaaaaa aagatttata ccaatttata ccgtccaata 58380
ggaggcgtga gactcaacac agggtggagg agagaatagg tcaggaaatc acatctctcc 58440
acctgcacag ccaccatcat tgtacccttc taaagagact ggcaacaact ttcattcagc 58500
actgtcccat tgattggctc atttcatgat cacagcaacc cagttaagca aatcctcacc 58560

tttgcatcgc ctgctttgta tcacttttct ttaaaattaa aaataaaaaa aatggattac 58620
aaattcggtt aacgcccatc ccatttctct ccttccttgt agtaattatc attctaaagt 58680
tgttgggaat tattcccatg cgtactttgc acttttaaaa aatgtttgct ttcctaaaca 58740
tataccactg ttttgtgtat tttaaaactc acacaaataa tatcatacaa catccatttt 58800
tgcaagtttc ttactcatta cattgctaaa tatgtctata attcatttaa ctgctgtata 58860
gtatcccatt ataagaacaa accatacttt atttatccat tttacttaac aacagttagc 58920
ttatttccac ttcttcttga caattaactg atactgcaat gaccattttt atcctcgcct 58980
ttttgtgcat gtgtagaaat gtttttctaa ggaatagatc tagaagcaag attgctgggt 59040
catagggaat attcattttc agcattaact ggtgccaaaa ttctcttcaa atttgttgta 59100
tctgtttaca cttcaacaca aaatatatgc attcctattg tcccacattt ttgtcaacag 59160
tgttagaata attttaattt tggtgatttt gtgggtgtaa aatggtaact cgcgctgtga 59220
tttgcatttc tacattactg gttaaattga gcatcttttc ttatgtttat tgaccattag 59280
gtttctcttt tgtgactcac ctattcacag cttttgctca tattctcttg cgttgtcctt 59340
ttcatatgga tctgcaactt ctttatgaat cctggatata atcctttgtt gtttatgtag 59400
attgtgaata ttctctaagt ctgtggcttg tcttttactt tacgtgattt cttttttttt 59460
tttttttgag acggaatcct gctctgtcgc ccaggctgga gtgcagtggc acaatctcgg 59520
ctcgctgcaa gctccgcctc atgggttcac gccattctcc tacctaagcc tcccgagtag 59580
ctgggactac aggcacccac taccacgtcc agctaatttt tttgtatttt tggtagagac 59640
ggggtttcac catgttggcc aggatggtct caatctcctg accttgtgat ccacccgcct 59700
tggcctccca aagtgctggg attacaggct tgagccatcg cgcccggcct atgtgatttc 59760
ttataaaagc ttaaaaatgt tttaacgtgg tcatatgtat caatattttc ctttattgct 59820
tgtgcattta tttatagtcg acaaataaaa attgtatcta tggtgtaaca acatgatgtg 59880
tgtatatata tgtgtgtgtg tgtgtgtgta atggctagag taattactgt atctgttatc 59940
tcacatacta tcattttaaa cgtgatattt gaaatgtact ctcctagcaa ttatttttat 60000
ttttattttt tttttgagac aggatcttgc tctgtcgccc aggctagagt gcagtggcaa 60060
gattatagct cactgcagcc tggacctccc aggctcaagt gattttctct tctcagcctc 60120
ccaagtagct gggactactg gcacgtgcca ccacatcctg ctaatttttt caattttttg 60180
tagagacagg gtctcactat gttacctagg ctagtcttga attcttgggg tcaagcaatc 60240

aagcaatcgg catcccgaaa tgctaggatt acaggcatga gccactgcgc ccagctaatt 60300
tttctatttt ttttagagat ggggtctcac catgttgccc aagctggtgt cgaactggtc 60360
tcaagcgatc cacccacctc agcctcccaa agtgctgggt ttacagacat aagccactgc 60420
agctggccag caattttcaa gtatacaata cattgttatt aactctagtc atcatgttat 60480
acaatagatc tcctgaattt attctaccta actgaaattt tgtatccttt gactaaagct 60540
ctccaatccc tgcccacacc cgcccccgc cccctagccc ctgtgcagcc accattctat 60600
tctctgcttc tgcaatttta ttttatttat ttattttttt aaatttattt tgagacaggg 60660
tctcactctg ttgtctgggt tggagtccag aggtctgatc acagctcact gcagcctcaa 60720
actcttggat tcaagagatc ctcctgcctc agcctcccga gtagttggga ctacaggcac 60780
gtgccaccat gcctggctga gtttgactct ttaggttcca catgtaaatg aaatcatggt 60840
atttgtcttt ctgtgcctgg tttatttcat ttagcataat atcctctagt tttatccatg 60900
ttgtcacaga tgacagaatt tccttctttt taaagactga atagtattcc attgtgtata 60960
tgttccataa tctctctatt tattcattga tggacactta ggttgtttcc atgtcttggc 61020
tattgtgaat aatggtgcag tgaacttggg aatacagata tctcttcaac atactgagtt 61080
catttctttc gaaatatacc tgataatgga actactggat cacatggtag ttttaatttt 61140
ttgaggaagc tccattctgt tttccaaaat aggtgtacaa atttacattc ctaccaacag 61200
tttgtgaggg ttcctttttc tccacatcct tgccaccact tgtaatctct tggtttattt 61260
ttattttttt tatcagagcc atcttagcag gtatgaggtg atatctcact gtggtttaaa 61320
tgttcatttc cttgataatt agcaatattg agcatttgtt tttcatatac ctgttggcca 61380
tttatgtgtc ttcttttgag aaatgttttt tcagatcatt tgcccattgt aaaatcagat 61440
ttttggctgg atgcagtggc ttatgtttgt aattccagca ctttgggagg ccaaggcagg 61500
aggatcactt gagcctagga gttcaagata aatctgggca acatagggaa accctgtctc 61560
tacaaaaaaa ttttaaaaat tagcagggca cggtggggtg tgcctgtggt cccagctact 61620
tgggaagctg aggtgggagg attgcttgag cctgggagtt gaaggctgca gtgagttatt 61680
attgcggcac tgtattccag cctgagcaac agggtgagac cccaacttaa agaaaaaaaa 61740
tcagattttt ggttttcaac aactcttgct attaagtttc ttataaaaat attcacccct 61800
tatcagatgt atagtttaca aatattttgt ccctttccgt aggttgtctg ttgattgttt 61860
cctttgctgt tcagaagctt tcatttgatg caatctcatt tgtccaaaaa aattattgcc 61920
cagaccaatg tcaagaagcc aagatcatgc cactgcactc cagcccagca acagagcgag 61980

actctcatctc aaaaaaaaga tctgaaattg taaaaatact agaagaaaat ataggagaaa 62040
agcaattctc ctgcctcagc ctcctgagtg gctgggacta caggtgtgca ccaccacacc 62100
cagctgattt ttgtattttt agtagagatg gggcttcacc atgttggcca ggatggtctc 62160
aatctcttga tcttatgatc cacctgcctt ggcctcccaa agtgctggga ttataggtgt 62220
aagccaccgc accccgccat ttcagatctt atgtttaagt ctttaattca ttttgagtag 62280
tttttttttt tttttttttt tttggaggca gtctctctct gttcttcagg ctggagtgca 62340
gtggtgtggt cttggcaacc tctgcctccc agattcaagt aattcttgtg ccttagcctc 62400
ctgagtagct gggactatag gtgtgtgcca ccacccctgg ctaatttttg tgtttttggt 62460
aggtttcacc atgttggcca ggctggtttc gaactcctgg cctcaagtaa cccattggcc 62520
ttagcctccc aaagtgctgg gattacagat gtgagccact atgcctggcc tgagttgatt 62580
tttgtatatg aggtgagata tggtcaaatt ttattcttct acatgtggat atccagtttc 62640
cccaggactg tttattgagg agactgtcct ttccccattg tatgttcttg gcatatttgt 62700
aaaggatcag ttggccataa aatgtgtgga tttatttttg gcctctattc tggtcttata 62760
tgtctgtttt tgttccagaa ccatgctgtt tttattacta tagctttgtg gcagcttctg 62820
aaattaggta gtgtgatggc tctagctttg ttccttttgt tcaagattgc tttggccatt 62880
ttggtccttg tcttttgtgg ttccatatga atcttaggat ttttttttcta tttctgtgaa 62940
aatgtcattg gaattttgat agaggttgga tggaatctac agtttggggt agtgtggaca 63000
ttttaacaat attaatcttt ccaatccatt aacatggaat atctttccat ttatttgtgt 63060
ctttaatttc tttcttcaat gctttatggt tttcagtgta cagatttctc acctctttga 63120
ttaaatttat tcctaagtat atatttttga tgctattata aatgtgattg ctttcttgat 63180
ttcttttttg gttggtgcat tattagtata cagtaatgct actgactttt gaatattgat 63240
tttgtatcct gcaactttac tgaatttatt agttctaata gtttttttgg tggtatcttt 63300
agggatttct ctatataaga tcatgtcatc tacaaagtga caatttaact tctttctttc 63360
caatttggat gcctagggag catcagtggc tcaagccagc tgtcctggtg cacagggagg 63420
cgaggctatg agttcgaggc caacctggtc aacattgacc aatttggatg tcttgtattt 63480
ctttctcttg cctatctgtt ctgactagaa cttctagtac tatattgaat agaaatggtg 63540
agaatggtta tccttgtctt gttcctgatc ttagaggaaa atcttttaac ttttcatgat 63600
tatgtatgat gttagctgtg ggtttgtcat atatgggctt tattgtgttg agatacactt 63660

tttctatacc taatttgcta agaatttgta tcatgaaagg atgttaaatt ttgtcaaacg 63720
cctttccccc atctattgat ataatcatat agtgttcttc attttgttaa ctgcttgtac 63780
ttttgtactt ttaaaatttt gtataagaaa tccttctcta ttctgatatc atcaatatgt 63840
gatcttatat tttctttgaa tagttttcag ttttgctttt tactctagct acctgaaatt 63900
tacttctggg taggatgtaa tgtagaattt attttttgtg tgtttgtttg gatagccagt 63960
taactcaaaa ccattgaact ttcccactga tttatgtcac ttccattata gccaagtctc 64020
atatctttga gggtctgtat tagtccattc ttgcactgct atagataaat acctgaaatc 64080
aggtaattta taaagtttaa ttggctcata gttccacagg ctgtatagga agcatggctg 64140
gggaggcctc aggaaactta caatcatggt ggaaggtgaa agggaagcag gcatgtctta 64200
cgtggctgga gcaggaggaa gagagggaag aggtgccaca cgcttttttt tttttttttt 64260
gagacagagt cttgctctgt tacccaggct ggaatgcagt ggcacaatct cagttcactg 64320
taacctccac cctctgggct caagcgatcc tcccacctca gcctcctgag tagctgggac 64380
cacaggcatg catcaccatg ctcggctact atttttgtat tttgtagag atgatgtctc 64440
agcatgttgt ccaggctggt cttgacctcc tgagctcaag tgatcctcct gcctcagcct 64500
cccaaagtgc tgggattata ggcaagagcc actgcacctg gcctccacac acttttaaac 64560
aaccgggtct catgataact tactatcatg agaactgcac ccagtgggaa atccatttcc 64620
atgatccaat cacctcccac cagaccccat ctccaacaaa ggggattaca attcgacatg 64680
agatttggag agggagggac acagatccaa accctattag gatctgattc aaaaaagttt 64740
tgttctgtac tgtggtctat ttatgtgtgc atataccagg actagactgt tttaatcact 64800
ctaagtttca accagtaaac ttgtgacata tggttaggca agggatctca tattcttctg 64860
cttcaagttt tgtttttct tggccattta ctcttccata agaattttaa ggccgggtgt 64920
ggtggctcat gcctataatc ccagcacttg aggaggccga ggcgggcaga tcacgaggtc 64980
aggaattcaa gaccagcctg accaacatgg tgaaaccccg tctgtactaa aaatacaaaa 65040
aaattagccg ggcgtggtgg tgcgcacctg tagtcccagc tactcaggag gctgagccag 65100
gagaatcact tgaacctggg aggtggagct tgcagtgagc cgagatcgcg ccattgcatt 65160
ccagcctcgg taacagagtg agactccgtc tcaaaaaaaa aaagaatttt aagaacagtc 65220
tgtcaagttc cataaaatat ctgattggag ttttgcttag aattgtactg aatttttaga 65280
tttgagaaaa attatcagct ttatatcaaa cttccgtctt tgtgagtagg ggatctcttc 65340
ctattctttt atatcttctt tatgttctct aataaaactt tataattttc tccataaata 65400

tcatacacag actttgttat atttattcct aagtatctta tgatttttgt tatgacaaat 65460
ggtattttct tttctttttt ttttttttg agacggagtc tcgctctgtc gcccaggctg 65520
gagtgcagtg gcgcaatctc ggctcactgc aagctccgcc tactgagttc acgccattct 65580
ccttcctcaa cctccccagt tgctgggact acaggtgcct gccaactcgc ccagctaatt 65640
ttttgtattt ttagtagaga cggggtttca ctgtattagc caggacggtc tcgatctgct 65700
gacctcatga tccgcctgcc ttggcctccg gaagagctgg gattacaggc gtgagccacg 65760
atgcctggcc taaaatgaaa tcttttatct gtttattgct gtggtgtaag gatattgatg 65820
tttgtgtatt ctccttgccc ccggcaagga aattgttatt tctaatagtt ggtaggttct 65880
tgtgaatttt ctttgtatag taagcaatct atgtatagta attgtctatg tatagtgatg 65940
gctttatttc ttctttttct tcttgtgtca cttcagactt cttggcagaa aggtgaatag 66000
aaacagtaaa aactggtatc cttgtcttga ctttggaagt tactttctat tcctagttag 66060
ctagattaaa aagattaaaa aagaaataga caggttctca ctatacagtg gttattcaca 66120
gcctcaatca tagagcacta cagcctcaaa ctcccaggtt caacgaacct taccacctta 66180
gcctcccaag tagctgggac tacaggcaca agccaccaca cctggctaat ttttaaatat 66240
tttgtagttc actacagcac tattcacaat agcaaagaca tggcgtcaac ccaaatgcct 66300
atcaatgaaa gactagataa agaaaatgta gtatgcagcc aggcgtggtg gctcacactt 66360
gtaatcccag cacttaggaa gcctgaggca ggtggatcag gagttcgaga ccagcctgac 66420
cgacatagtg aaaccctgtc tctactaaaa atacaaaaaa attagccagg tgtggtggca 66480
aatgcctgta atcctagcta ctcaggaggc tgaggcagga gaatttcttg aacccgggag 66540
gcagaggttg cagtgagctg agattgtgcc aatgcgctcc agcctgggtg acagagtgag 66600
actctatctc aaaaaaaaaa aaaaaaaaaa aaaaatgccg ggcacgatgg ctcatacctg 66660
taatcccaga actttgggag gccgaggtgg gcagatcaca aggtcagaag ttcacgacca 66720
gcctggccaa catggtgaaa tcctgtctct actaaaaata caaaaattaa cagggtgtgg 66780
tggcactcgc ctatagtccc agctacttgg gaggctgagg cagaagaatc gcttgaaccc 66840
aggaggcaga ggttgcagtg agccgagatt gtgccactgc tctccagcct gggtgacaga 66900
gcaagacacc accttaaaaa aaagaaaaaa gaaaatgtag tatgcgtaca ccatggaata 66960
ctatgcagcc ataaaaggga atgagatcat gtcctttaca gggatgtgga tggaactgga 67020
agccactatc ttcagcaaac taacacagga acagaaaacc aaacaccaca tgttctcact 67080

tataagtggg agctgaacga tgaaaacacc tggacacatg tgggtggggg gaacaataac 67140
ccactgaagt ctgtcagaag gggaagtggc aggagggaga gcatcaggaa gaatagctaa 67200
tggataccgg gtttaatacc taggtgatgg gttgacaggt gcagcaaacc atcatagcac 67260
atgttcacct atgtaacaaa cctgcacatc ctgcacttgt actccggaac ttaaaataaa 67320
agttgaaaaa atattttgta gagacgaagt ctcactatgt tgcccaggct ggtcttgaac 67380
ttctgagctc aagcaatcct cttgccttgg cctcccaaaa ttctgggatt ttagcagtga 67440
gccactgcac ctggcctcca gttctggatc ctgggtcttg cgacattatt ccatgctggc 67500
ttctgaagtc aatgaggcaa aaccagaaag gggaactaaa aagttaaaga gggagagggt 67560
ctcacaggca agatggagga agcctgggat gaaggcaggg ctgtggacct gaatgatgcc 67620
aggaattaag gaggaagtaa cagtgttagg taatagcaca tgggattctt gggctgcagc 67680
tacaggaggg agaatcatgg ccttaattca tcacctctga aatcctgaaa tatctagcct 67740
cctgcccaga tcctgccatg aatgcatcta ttcaaccact atttataaag tatccagtgt 67800
taggtccagg gagtggcaag agtgagcatc tgcagtcata gtccctgccc tcagggagct 67860
tacattcaat ggggcagaca gacatcagtc tgtacataat catcaacaaa tgtagaactc 67920
caacagggcc aagtgcctct gaggaaaggt acaccttagt gtgagcgctc ataacaggag 67980
aatttgaccc tgaaaagaag gtcaggaagg cttccctgag gctattgaga aggtgaggcc 68040
tgaagaatga agaggagcta agaggagagg aacagaggaa ggagccgttc cacagagggt 68100
ccagcctccc tggagactct gtgcaggagg aagcacaagg tggacagcaa ctggagcaca 68160
cgagtgaggg ggtgtggtgg gagatgaggc tggagaggag gggccacccc ttgcagggcc 68220
ttagaggtca ggttaaggac ttgggtcttt aacctaagat caatggaaaa ccactgaata 68280
ggtttaagga ggctgactga aacaatcaga tttgcatttt taaaggatcc ctcttaggtg 68340
tgtgtcatac tgtagagaag gccattccca gcctcttcag ctactgcaat gactcagaga 68400
aggccacatg gtccacatgg tggctagccc agggatgtgg agggatgaaa acgagtggac 68460
agacttgaga tatattagag cagataaaat ggataggact aggtgctgga ttcaatattg 68520
agatgctcat tctaaaccgc ccctacatca aatccttcga aggcaggggc tatgccacat 68580
gactatgata cccaggtccc agaacaactc ctgacacatg gtagactcaa tacacatttc 68640
ccgaaagggt ggaaatgatt agcagtctct caagtaatcc tttgaccact aagcgtattt 68700
aatttcttct agcatgtgtc agtaatcaac ctccttggta catcataggg agaaatattg 68760
ccgagttcca aagagtatac tctggggcaa gttaatacaa gaataaggtc tcacttaccc 68820

tataacatga agagtaacag taaactcgta agaacaggac ctggtgaagc aaggagtagg 68880
cacaggaaga tggctcatca aataggggcg ccgggtgtag tgtgaacacc tgaggcagga 68940
agaacccagt agcctctggg ggttgggtgg gattagttgg gggactagga tttgatggta 69000
gggggttgtc ctggagatct ctggaaatta agttatctat tttttttctg tttttatttt 69060
tataaaagaa ctcttaaggg catttttttc tttgtttgtt ctgttttgag atagaggttc 69120
actctgtcgc ccaggctgga gtgcagtggc gtgatctcgg ccactgcaaa ctccgcctcc 69180
caggttcaag tgattctcat gccttaacct cctgagtagc tgggattaca ggcatgggcc 69240
accacgcctt gctaattttt gtgtttttttg tatagccagg gttttgccag gtttggtcag 69300
gctggtcttg aactcctggc ctcaagtaat ccacctacct tggcttccca cagtgttgga 69360
attacagcca tgagccatca tgcccagcct aagctatcac tttaaaatga cccttcaaga 69420
aagtcccaag gagtctaatc catccacgac aacagcacgt ctgagacagc ctagtgaaat 69480
ttctccgatg gaggacagga gatggaaaag tgccccagga cctcttattt atataaaaaa 69540
caaaattttt taagaccctg agaagtatgg attcttctcc agagaaaagt tcctggcttg 69600
ggagctccct taggacttgg cactttggtc tagagtgaag tggacctggc cattccagct 69660
ttgagtgggt tcaggcgaga cactcgagct accccttatg gttgctctga aggtcaccct 69720
ggctgggagt gggtggatct ctgctctgtt atgcctccca caggtggagg aagtctgtct 69780
ctgaggctgc cctgccaccc tcatagagcg ctgcagattg gggcggtga tgcagccaca 69840
ggggctctga ttccctgcct ccccactgtg ttcatctcat tcctctccac atatgaggcc 69900
tggtgtcatg gcaggtgggt gttccaagtg aaaccacagc cacagaggca agcagtagag 69960
cctgtccctg atgtggccct ggtgatgctg caccagcctg gggctgtatc agaaaccatg 70020
cctgcagcct ttgcttctgc agacttgctg gtgctgcacc agccctgagc aatgtccata 70080
gcccttgcct cactggtcac tgtgatgggg acgcccccag gtatggttct gctgctggtg 70140
ttgcaggttg gagtgggggc cagcaccctc atctaccaca agaaggctta ggtggcacag 70200
acgtaaggct gcttgcctgt gtgtagagtg gcaggctgca acgggctgga gccttgggga 70260
gaggcccttg ccacactggt ggcagtgatt gggttcaggc aggtgtgtgc cgcagtgctg 70320
ggccaggtgt gagctgtggc aagagctgtg gccacacttt ctgcaggagt agggcctatc 70380
acctgtgtga aagtgttcgc acagggtcag gtgggcccct tggctgaagt acttcctata 70440
ttagggggag gagaagggcc ccttgcccag gctgtgctga gagaggtgag agcagtggga 70500

agagactcca cattccaggc aggaaatttg agtggatttg tgggtggaga tgggggtccc 70560
gggtagggga gttggggaag ggttggttgt gacagctggc aggtaaagga gtgccatagt 70620
aggtagaggg ctgggtctgt cctctcttct cctcctcctc tccctttgct cccagcctgc 70680
agggagaggg gagagaggtg agagcagcat gggggaggag gaagcagagg agacagaggt 70740
gggagaggag aggctaagcc ccacacccaa ctgaagaagt ggttggcggt ttcagcggca 70800
cctccctcct cgctgaggtg gactgtgaag gtggagaagc ccagagcaca gcctcctgtg 70860
agcagcttcc cagggtgttg agggctgcca ggcaaggaca gggccctcag agttaggcct 70920
tgcctgggca ggggtctcct gggggaacct tccctcaggg ccctgaggct ctgggctcca 70980
gagctcagct tccgcgttca gatacatggt ctgctctgct ttggagactg agaagcctga 71040
gaggtaaaat cgtgctgggt ggtaggtagg gtaagggaga gggagcttcc ccccatctcc 71100
ctcacccact gcagaaaggc ttctctgttt ccttccccag cccagtccca cctcctctgt 71160
gaggccgctc ctcacctccc tactggcctg cagtgatcct cctgcttggg agtcacagag 71220
cacccctaca cgggcccttt gccattcttt atagtataag gcaaggccgt aacattttgt 71280
ggggcatctt cttcagtgtc ctcagccttg tgtgaacatc agaagcactt gggggcttgg 71340
gaaaatgcaa ttgctggccc catctccagt ttcttaaaca gcagatctgg ggtggggccc 71400
aagaatttgc atttctaaca agttcccagg cgatgctgat gctgctagtc tgggatccac 71460
actgggagag ccactgatct gttaggcgtg taactttact tgtattatct ctcaattctc 71520
agggcaacct gttactctcc cccattttgc aggtgaagaa acagagggaa gacccagtgc 71580
cttgtgcaag gtggctcagt ttgtaagtgg cagagccgat ttgaacccac gtttgtctga 71640
ctctaatact gtatcaaccc tccttcctct accctgttcc tctgcattgt caactattcc 71700
acatgtatct tgttcctcaa caaaatagta acgttttctt ttgcatactt tatatctctc 71760
accttgcctg acccagtaac ataggcttgg ttgggaggga ctgaaggact tggaaaggaa 71820
atgccccagt ggagccagga tggaaatggg ttcctctgtg tctcccacat ggtgtcacct 71880
aaataaacag ccatgtctgt gaggttcagt catggggaga ggagggcaag agagcactcg 71940
caccaagcga tggggttagg gggacacgaa aggaaatgtg ataaatagag agggggggcgt 72000
gtggggcaaa ggtgaggact ggggacccgg gagggggcag gctgtttcag agtgaaggca 72060
ggacgggcag cagagacaga gaagaggctg ccgggtggtt agggctgggg aggacaagga 72120
gggtgaggac agacgatccc ggcagcccct ggagccagaa gagaagtcag gttaactcca 72180
gacttttggc ttccacctct gtatctcacc caactcctcc ccacctgctt ctctaccctg 72240

aaactccagg aacaacgccc acctgcctcc cactacaact tccccttata ccgtacacgt 72300
cctggatgct tggggtccgg gaggagggga gtcaggatca cgggttccag ggaagtgagt 72360
gtagaaaggg gctcctacga gggtagaagt gggtgttgtc tgcttctgca ggaaaggggc 72420
cagccagccc aactggtccg cagtacttgc tggggctgga ggatgcccac ctgcatctac 72480
gggattgagg gtaccggaga ggaggcgaac aaacgagaaa tcccaagggt ggagaaggga 72540
gccatgggat cggcgggcgg gccgcgcagc tgcggaactg ggagggccaa gcgggcttta 72600
gggggaaaca cggccggctc ctctgccagt gagacagaga actgagcatg ggggaggaat 72660
ttggggccag aggggctggg ggtacccaga gcagaggaaa caggaagtga ggggcagaga 72720
ggttacgggt ggtggaagga acagcagtcg gttctggaga ggcgattcct cttccccgaa 72780
tacctgcact gccccggacc gggagtgcag tcgtgcgccc ctgtcccccc agcctcaggc 72840
ttggtgccca gccccgctcg cggaactcga ggccagtaag gcagccgcgg ctttccttga 72900
tgccccccact cctctgctct cagccgctgc ctggacgagc acagaactag tgttcccagg 72960
cgaccctccc tccgctcagc cccgctgccc ggcgggcaaa ggctgtgcac tcacactccg 73020
agtctgcagc ccagcctgcc ctttttttggg aggtggcccg gggactggga tgtcgaacag 73080
ggagcttctc cgcagtcctc tccacctccc tccctctttc cagctataga aaccggctgc 73140
tcgcagtgac ttccaaacat cacggtcatt cccacatcta cacacatccc tttctctttt 73200
gagccatttt cctgctttcc ctttggtgtt gaaaccctgg atttcagcct gggcgactca 73260
ggttttaggg gggaaagttt ggggtctaac ttggcacctc tgttttttat cctctcttcc 73320
agcttcatag atggttccta ttttcataca tatttgctaa tttggagttt ccgacgacat 73380
atctcgaata acccgacatt ttctccccat ttccaaggct gattttacat tatttctagg 73440
cctccctccc caatctattt tcctttcttt ttcttgtaca cccctgataa acagacacac 73500
agaactgcag gtttcaaaga ggaaaaaagc acgtttgttg ttggtaggcg ggcgtgaatg 73560
ggagggttct tcaccttctc caacacctcg tatagatggg acgttgcagg gcaatagaaa 73620
aggcagcaga tgggggcgtg ttttgcccca ctctgtacaa tatagaagaa tctcatatag 73680
atactttgta taaaagccac gtgtcctcat ttgtgtcctc ttattggctg gctgggctgg 73740
gggtactggg ggacaaagaa gctggggtga tagaccagga cacagggata ccccatggtc 73800
cccttcctct ggctgtcccc tggtggggtc actttggatc cttggtggga gcatagcaca 73860
gctccagtgg ccgggacacc ttccagaatt tctcattcac cagctccagg gtcagcgagc 73920

cattcaacgt ctgaggaggg ggtaaaaggg agagagggga aaagagagag gaaagttgga 73980
gaagatggaa aaggaaatga atgagaggca aaaggtgacc aagggaggag gaaagaaaaa 74040
aggaataata aaagcgataa ggcaaaaaag gacatggatg atgggaaagg agaggggaca 74100
tagaaagaga tagaggatgt ataggggaag agtgctgatg agttagacca ggctcatggt 74160
tctatgtggc ccagaccacc ctccctcctc tcccctcttc taccactgcc ctcagaagct 74220
ctcaccgtga acgccccgcc tccaggtgcg atgtagatgg tgtactgctt ttcactgacg 74280
ccctccaggc tgggcaaagc aggctcctca ggaccgtcac ctcctccggc acccccaccc 74340
tccccgccac acccatcagg tcctccggtg tcagaggcgc cccctccagg ccctcctggc 74400
tcccctgctt cctgctgttg cctccgctcg agggcaatgc gcagggccaa gtacttggag 74460
aggtggtcca ctgtggcatt cccagttgtc ttcacatacc tggaatggga gggaggacag 74520
gcttagagcc agagcgcctc agatgaggac tggaggtagg ggctttccaa aatggattct 74580
gggttggggt ggtattttgg gtttgggcaa agcccacttc tgatggaaat ctaggtgatc 74640
tttggtttct cagtggctgg ggaaagacag ggctcctcac ctcgtctggc agtattctcc 74700
cttctccacg agcagggggt ggggccggaa cacgagctca atttctccac ctggctctgg 74760
ggggctgggg gccccaggag ggcttggggg gcccagcgtt ccccctccca gagtccctcc 74820
ccggtcacca gagtcttccg aaccggcacc cccacccacc ccaccagtgc cgcctccccc 74880
tgtccctaca ctgctccccc ctgcgccccc tccacggggt cgcttgggag cagggcctgg 74940
ggcagagtca ggggcggagt ctgagctcac atcttctcca tccccttctc cctccccggg 75000
ctctccttcc cccccactca tcgttgtggt ctgatctgac cctggtatcg gccgcctcac 75060
acgctgggcc ctgtagaagc aagtggttaa ggttagaagg catccaggat aaagggctta 75120
gactttaaac ttcagagaat taagagataa gaaaaatcct aatgacgata cctaatattt 75180
attaaacaca atgtgctaag cactcagtat actaagtgtt ttgcaccaat ccccttacct 75240
aatacaatga atcctatgac acaggtaatt atattacctc tattttatgg ctgaggaaac 75300
tgaggcttag agaggttaag taacttgtta gtgatcatag gctgtcacct ctcaaagtgc 75360
ggtagcagca tacacatccc ctgtgagctt gtgaaatgca gactctcggg cccaactcta 75420
gacctactga atcacagttt gcatctgaag atctacaggt aatctggata cacatgaaag 75480
actgagagat gctggcctag gaggtgattt aacttccagc agtatgaatc cggggcttat 75540
tctcaaaatc gctctactgt tctaacttct agaaagtcat ggggtaaact acatttttcc 75600
ttttttttt tttgtgacag ggccttgttc tgtcgcccag caggctggag tgcagtgtgc 75660

agtggtgtga tcatggttca ctgcaacatc cactttcttc ccaggctcaa gtgatcctat 75720
tacctcagcc tcccaagaag gtaggactac aggcatgtgc caccatgccc agctaaatgt 75780
ttaatttgtt gtagagatga gatttcccta tgttattcaa ggtggtctca aactcctggg 75840
ctcaagtgat cctcccatcc tcacctccca aagtgctggg attacagatg tgagtcactg 75900
tgcccagccc tatatttttc ttaagaagag cagggccagt tgtttactaa gaaactgcaa 75960
tttacaccca tgatttccag ccctaatgat cagtctgtgg tcagctccta ggatgctgag 76020
gcttagaagc tagcaggcaa caaggactga cccacggacc catcccatca gttctgcttc 76080
tctcctgacc ctcacacctg tgcatggcct gcatgcgtag cccctcctca atgctggagc 76140
tcaatgcctg ctggttgtgc aggcggctca ggcggataag cactcggtct tgatgggcct 76200
cgtattcctc ccggctagga tagatcttag agatcagggc atcaaagttg gggtctggcc 76260
gtagggatcg cttggacacc agcttctttc ggcaggtagg acactccttg ttcctggggt 76320
aggagaaggg aagccctgag tggtcagtca agggaactga agcctaggct gaaccaagac 76380
cagtgagagg gccctaactt tgtacccctt catctcatct caaacatgtc tctcctatta 76440
cccgctccgt agggctgtga caatgcagtc agagcagaat ctgtggaggc actccttggt 76500
ggtcatcgta ttcttcagca tgtccaggca gatagggcac atgagttctg aatgcagtga 76560
ccgaggggaa acagcaatct ctgtgccatc cattatggct tcctgaaggc atcagtgagt 76620
agaaaggtgg tgtgggttag agggaaaggg gttttagaaa cagcagccca taaaataaga 76680
attttgagaa atacagtgag aagacccatg ttggtctcta taggagacaa gaattctgag 76740
aaagagaagg actgatgact tggagcaaat ggagaaaaga cagactttag tgtccattaa 76800
ctaagagtag gagctttgga ataggaggat ctgaggtggc taagtggcct aggagtgaag 76860
atggcacaaa tctgtggttg ggaaaaacta ttcaacaaaa ttttttaaag tcctggtgct 76920
acctgtatta gaaccccata gggtgcctgt taaaaattca gattctgaga acccaattca 76980
ctactgaatc agaatgttgg ggggcatgaa gctctgcttt taataaactc ccaaagtgag 77040
tcttatgtgc actgacttat gacactgaat gaggaacagg gaaggtaatt tttgtgtttg 77100
tgatctttca aatgaagata ataatacttg ttccacctct ctggcagggc tgttttgagg 77160
attaaatgag ctaaatgcga aagtgctttg gaaagattaa ggtgattcaa aggtagggcc 77220
aggcaaggca gaatagagca aattacggaa aagaagctgt gtgatgcggt ctgtggatat 77280
aagggtttgg gggagggggt aagcctgaaa gggagaatgc ctgtcacctg cggggtccgg 77340

tgcagctcat acagactcag ttcccacgtt ttgctggcat tctgggcatt cgccggcgtc 77400
gtcatggtga ccgggcgcag acccccccacc agggctccac agccgaggag aaggcgaaga 77460
aggctggggg tgggggaag gggaggaggg cgtcaggggg gccgcccctc gcgtagaccc 77520
cgcccctcca gaagcgcccc tctccgcccc cgctcccgcc cccgcgccgg cactgcccct 77580
cacccagctc cagccgttcg cgctcccgcc gccgccgcgc ctctccctag gcccgggctc 77640
ctgggccaag ttcgctcggt ccgcaaccgc tgctcagaca gcagctcccg ccaccgccgc 77700
cgccatggcc cgggcgctgg ggccctacgt cacttccgcc tgctcctccg ctacacccgc 77760
tcccccgcc ggcggagacg tcacccacca ttcgcggcgc gggtgggacg ggacgtggac 77820
gccgcggttc tcccgcccac gggacgggct cgtcccgcgt gaggatcctg cgtcccggga 77880
gttctgcgcc acttagggaa ccacggtctc cgcccccggc ccagcaccgt cgcgcggcca 77940
acaaccttag cctcggttcc cttccagagg cccccacgcg gggcctcagc tccgggggc 78000
cggggcctgg aatatctggg accccaggag gggacagagg gctcgtgaaa agggctacct 78060
cccccactct gcgtaccctg gcgactctgg ggatcaaccc ccctcacttc tccgtaaccc 78120
ccattccagg atcccggggg aaaaggctgc aaaagatctg ccgctttagc cttctagtcc 78180
tgagaacaca cctaggtccc gatgtgcccg ggattcccct taccaccctg gggactccct 78240
ccaccctctg ctttggctct ccctagaccc tccgacccgc tctcccgctc gaggtttggg 78300
ggcggcggct cgggacgtcc agactcaact ctcggccgga gccatagact cgagaattgc 78360
gtttggacaa tcaggagccg cggcccgggg cggggaaggg agggaggcgc acgggaggaa 78420
agggggaatg gagacaccac gcggaaacag ggacatacac aagatggagc tcccattggg 78480
aagggggggt gcgcgtgtgg gtggggtaca caggggcaga atgtctggag tgggaagtgg 78540
gcagaagcct cttcgaggac tctagagtaa acgaagggga tggggaggaa agagaagagg 78600
aaggggcaga gcgatcccca ttgagtgtag ggaatgagag ggatacaggg cgaggggggtg 78660
gggggcagtt agagaccctg atgggcatct tgtccccgcc cgagctgagg ctcccggact 78720
tgggctgcgg ctgccagggg gtggcctgtc cctgggacaa ggcagcagtg ggtgccctcg 78780
ccccccaccc cagttccccc ctacttccct gtcctctcct tcagtccgtg gccagtcctt 78840
atgtgggcgc cagagtcgat tagcgcagac ccccctcccc ttcctcctcc tcttcccagc 78900
cccctccctc ctgctctctc cagccccttt catcggctgg ttcattcccc taatcctggc 78960
cccctcccct aatccccccc atccgacccc aggccggctg cagctattgt aaggtggaga 79020
gaaaggggag ggggggactc agagaaagga gaggggtggg ggagggccac ctgttccaag 79080

accccctttc aaggccagac tggacaccaa gatggggcca tgaacaaatc acccttgggg 79140
accataagaa cccagggagt tgggggggagg ggactggtgc tgcagaacca gtggaaaggg 79200
gtgacgcacg aacccctccc ttcaaaaaga cccggagtgt cacgcataca cagtgacaca 79260
tactctttcc tctcacaccc ggcggcgggg gttgccctgg gagaccaggc agagaaaggg 79320
aacaatcctt cgggaaaggg aaaggagggg gaggtgggga agggtctgag ggcttggaca 79380
caagaagagc cggaggtggc agggaagagg acttggaatt tattagggtc acaagcaccc 79440
ctgcttccca tattcagcat tcctgaacca tacactgcca ccccttttgt atcctgggaa 79500
cttagagtcc tcatcagcag gaggccaggc agagtggtgg gggggtgagc agagtccagg 79560
gtccttgagg cagttacatg aaaagacctc ctgggagggg cagaaacatt tggacagatg 79620
catgggtgga gacacaaatg ggctaggggg tacccacttc tgtccctggc cttgggaacc 79680
tctgcccttc ccagtggctc cagactcccc ttacttgctt tctcatatag tctgggttct 79740
acttctctgc cctctccctc cccatgctgg cctcatacca gtgacttcca ctgaggtccc 79800
tgtgatgtat ccactatctt cagatgccaa gaatgcgacc acatctgcca catctatgag 79860
aatggtgggg agagggcaga attctgctca ctcccataga cccaaaacag ccataatctc 79920
ccaccaaaga catccctccc cacaaacctc tccacagaca ccaaccacca gtcccctaga 79980
aaatccccaa actttgggga tctcttcatt caggcccccct tcccagggac cccactacat 80040
tcagtgctca ccctcagggt cccccaagtg tcccatcggg atcatttcag taatctgtaa 80100
ggaaatactc atgtgggtga aagcttcttt tatggatttt tttgaagact gagtctgtga 80160
ccctttttttg tcttcatttc tcatacattc attgaatatt taatgtttgc caggaactgt 80220
gcccagtatt ctctctctct ctctctctct ctctctgtga agcccagaga gattgagtct 80280
ctgaatgatt ctgccctcca cccacagcct cctaccttgt ccaccacttt ctgtggcact 80340
ttctgtgtca tgggtgttgc aatgaaccct gggaggacag agttacagcg gatcccatgt 80400
ctgtgggaag gagagtggct gccgattctg gagagggctg aatgctggta cctccctcag 80460
accccccagg tgctccccag caccctcaag catctgacca accgtccaag ctcccgggct 80520
gcggtctggg tcagcccaat cactccagcc ttggatgctg catagtttgt ctgccccacg 80580
ttccccacct ataggtgagt cagagatgtg gaatgagtca ggagtctcaa ggaggggttc 80640
tcctctctat accacttggc tggctgacct cgtccaactc aacctgacct ttcctacgat 80700
gctactgatg ttgatgatgg aaccacgaca accattggac accagggctt gtgctgcagc 80760

ctgagtgact aggaaggtgc cctggcggga aagggtaaca agggagaaaa gatcagctgg 80820
ggtgacagcc ctcctccaac tccctcccca ggctaagggg gccaaacgtc gcaggttcag 80880
agatcgccac cttgaggttg acagctatga ctttgtccca gtcatcctca gacatgtgca 80940
gcagaaactc atcctgggtg atgcccgcac aggacacaac gacagatggt gggcgagaaa 81000
agcaggccta tgggaggggg aggttacgca tcaaaaaccc cccacaaaaa gccggggcag 81060
tggaggcaat atcagagctt tagaggggga aagtggccta gcgttcacct gcacttgttc 81120
cagcaggcac ctggcggccc tggcctcaga cacgtcagcc tggaaggcag catggttccc 81180
tcggggcggc ccctccttgc tccctggccc gcccagcagc cgcaccgtct cctgtgccgc 81240
tgcccggtcc aggtcgcagg cagctacggt ggccccctct ccggccaggc gtacactgac 81300
cgctcggccg atgccgctcc ccgcacctga ggtaggacag aacacgccgg accgggcacg 81360
gggaggagag ggcagggatc agaggtcaca gggcagaaag taaaatgtgg gtaaggggcc 81420
agaggtcaca gcggccgcgc actccaaggg cacgcccctg acctcactcc aataccccaa 81480
accgcccggg gaaagaaccc cctcaacctg tgaccaaggc cagtgcggag cggagtcggt 81540
tctggagctg agacgccatg gcgggctgtg ggtgggtggg aatcccagca ctaagccaat 81600
cccagcgcag aggggcgtgg caagggcaaa gccggggcca atcaaaggcc cgccagcctt 81660
aggccatctg cggccaccag cgcaggccga cctcccaccg agggggcgtg gcccgacact 81720
gctgcttagg ggccccgggt gtggagaaga caaggggccc cattcccccc aaaaagacaa 81780
agactgcatg tggggcggaa gatcggaggt ctttatttcc ggttaccacc cctccccctc 81840
gattcaaaca cagtgatata aaaggaatag aaacaaaaaa aatcaggcca tgagaatacc 81900
aactaaccct ctcccacccc atcccccaa tcactcccaa atcaggacac acgattgatg 81960
tttgatgttt atccccgact cctcctgtta ccctctctgt ccaggactct ggtcttccct 82020
gaagcctcca ccaactggtg ggagaaagga aactggacct tggagaactt cctgtgtagc 82080
aacttggttc tccgagaaca ggtggggtag gggacaccag gtaggctgcc atcacccct 82140
gtcccttcca gccttcttca tggccccatt tagccttcca aatgcaacat ggtcagacag 82200
tgcccaacac tatggtccct ctggcctctc attctcaccg ctccagcctc tgtaagactg 82260
ccagcccttg aaggccctaa tgccaaagta accattctcc caggctacag ttccttcctt 82320
tggcagatgt ccctggccag ggcccccaac ctctacgcac ccctgacctg gagttagggg 82380
tagaggtttg gggcaggggt agtttatcca cccctcactc aaggtgggca atcagcacca 82440
tcatgataac tccccccagc agccccagca cctccagaag tgattgcaat ggtgatgcct 82500

```
ccctcagcag ctcgggcaac acagacactg ttgctacgta gataaagcca cctgcagtaa  82560
atggcaggac ccagccagga cctgcaccac ctgcaatttc actgcccact gctcctcctt  82620
cagtgagaag ggcacaggct gtgcctgcca gtgcccctac tgctgtcagt agttgcagac  82680
gcatcgcctg atgggcagaa aagagaaaaa ggcactcacc aatagctaaa gattatcagt  82740
ggtttagaag tggctggtgg ggtataggta taggggacat ggatttggaa actaaacatc  82800
aaaataaaaa gagaagaggg agcagtctag aatagacaat ttctgaaaaa tcttaagaga  82860
tcagaggtcc tttccacaga ctttctgatt gagtaggcgt gtgtgtgtaa gactccacta  82920
aaccattcag atggtttata gaccagttta agaaatactg agctaaagga caaagtaacc  82980
agaacttctt ccccctcatt tctagaggac ttgtcaatga cagtatgaat tgtttggaac  83040
taataagagg ctgtttgggt ttagggtgag tgaggagaga taaagggttt gaggcagctg  83100
tgtttggcag acatcaccaa cctgcttttt gctgcagcca gactggacca agatggcaaa  83160
gtctccgacc tcgtggggca cttcatgtag caggacagtc attgtggtca ggatccctag  83220
tccccggccc cctcgaaagg aagccccaat ggccagacca tcagtgaagt tgtgtgccaa  83280
gtcagcagcc agattcaggt accccgacac acgcaggtct tgtacaggac aagaagagaa  83340
aatgatcaga ggcttccatc accacttgtt ccagtcttgg tcagctattc tcacacctcc  83400
cctcagagac cactttacac ccagtccttt agtgtttctc agatacctgc cattacccac  83460
agcacatatt ccatgtgatt tgtgatgatg tcccttgccc aaatttatca ccaactctgg  83520
cccttaccta agcctctttt ttcttcttca gcgttctgag gtctcactgg cccatctttg  83580
ggtactgtgc tccctcctcg cctcttctga acccctcttg tttccttttc ttcttcctct  83640
gagctctgct tctccttggt agaacgctct gagggaagaa aagagttggt cagagtggag  83700
aacagatctt ccaacatgtc ctctccctcc tgagggccat aagatgagga gattctgagg  83760
caggcaagtc agctttcagg aaagttgttc ctgggctcac cttgtcttcc atgtccatga  83820
cttccacgtg tatgactgtg agcgtgtcca tgtccatgac tgtgaccatg tcctcctttc  83880
acatgtctca caaatttctc cacgacaaga aaggcaacaa ttccactgag aacccacagt  83940
cccacagaca gaatggggcc ctggcctggg aatggaggca ttgagacatt aagggagatg  84000
tgcattccag actcctcctc ctcaagtgcg ggaggtgagg aatagggaga gacggacctt  84060
ccccagcacc ccaactccca tccccatctg tctcttcctc accactgtgg gagtgtccat  84120
gtccgggttg ctccagagtg tggtgagaat gaggttctgg aagaaggggg gaaaaccaga  84180
```

tgagggaata tctgacacaa tgagtgtgta tttgggggaa tatcgaaagg tcagggatca 84240
taaaggagaa agaacccttt tcctccaaag aacgaaatag gttagatttg aggtagaagt 84300
cagaggttac ttaccaagag catgaggaat gaggtgcagg aaagcatctc ccaggagccc 84360
accggaagca aaactgagca agatctgaag tagagagcga tgccggggag agttcgactc 84420
cacggggata aggaagagga caaaaaatgg agctgctgag atcagcactg tggcccccag 84480
tgcctggtga gggagagtca aggtcaagta ctgtggagtt tcccaacaat ccagaaccag 84540
cccttctctc tcccatcccc tggagactca cataagccca gagagtgaca gcatccaggt 84600
cctgcttgat gcctggagcc ccagactccc catagcctcc atggctatgc tcatggtcat 84660
gtccatgtcc tctgtggtag aggctctcat gggagtagcc atggctatgg ccatggtgta 84720
aatcctcatg tgaatgtcca tggtcgtgat cgtgggtatg tccatgccag atgctctcgt 84780
gagtgtggcc atggccatgg gcatggctgt ggccatggtg gaaatcttca tgtgagtgcc 84840
tgtggctgtg gccatggaag tcctcttgca gatcgtcgtg caggtcgtca tgacccccga 84900
gtccagccac cagaagcccc aaggtcgccc aggtcagcag tcccacggcc acccagtggg 84960
gggcccccag gcctctggcc atcacgctga ccagagggac agaggcagtg actccacttg 85020
actcaactct atacctacac agggtccgct ttactccaat cgcgccctcc gtccacctct 85080
atgggccgct ctcccattcc cttaagttcc cacctacatt gttcccaaga ctagttctct 85140
ttatcccgaa ccacctcccg gatgtagggc cctgggacac taatctttca ccagccactt 85200
tacagactct ctgggccctc gtctctacga ctcattaggt gaggaaaagg cctggaaagg 85260
atggtagaga aaaaacagag aattctcacc ggctccggga tcctctcctt tcccggtttg 85320
cttggacgtg gcagtcccgc ctttagatcc cgcgagaaca ggaagttccg ttccaccttc 85380
gccctaatgc ctttaccaat atggcggcac tccggtagcg acgaatccat cctgcactga 85440
cacgcatgcg cgagtgtgga ggtgatttga ggggagggtg ggcgaggcgg gctaacaggc 85500
cggaggagac cgcctcctcg gtttccaact agacgagggg gcgggaactc gcgccgagac 85560
ttcccgtctg tacaaagatg gctgccacat tggcgctgtc attttggtac tgagcagagc 85620
gacgggctta attcgaccca atccaggcca gagtctttct ctcaggggct tcctcgtgct 85680
cagctaatcc tccgatcaat ccttgggaat ccctgggacc tcttcggtat ccctactctc 85740
agccagggat catgtcttgg gccgctcgcc cgcccttcct ccctcagcgg catgccgcag 85800
ggcagtgtgg gccggtgggg gtgcgaaaag aaatgcattg tggggtcgcg tcccggtggc 85860
ggcggcgacg gccctggctg gatcccgcag cggcggcggc ggcggcggtg gcaggcggag 85920

aacaacaaac cccggagccg gagccagggg aggctggacg ggacgggatg ggcgacagcg 85980
ggcggggtga gtgccccagc agaagggcag gcgagccagt gaccgcttta tctgcgaccc 86040
ctcccccaca ccctcccccg gtgctctttt ccttgcgctc ccgccccctt gtttgtaaac 86100
acgcgtcccc tccctcccga gggccttagc accctccccc ccggggcggg gcggggaagg 86160
ggagcttccg ccctcctagc tgtgaccgct tgaggccttg ggcaccggag gtcgcgggcc 86220
tgggaagggc acgattcctc ttaaacctca ggactttggg cgtttacagg cagatcctgc 86280
gtcttaggag ggtctctcc tgcctgtctc tttcccttgc acgctctggc tacctggctt 86340
tacgctggga atagaggggc tcgatggtct accccacgtg ctgccccacc cacgagacag 86400
gtgtggcttt gggatgacct ggtattcatt caaatggatt ggttggaaaa ttttcctcac 86460
ctgaactatc ctcctctcct tgtcaactcc ctcctctcac cacaatgagg agttacgctg 86520
tttttgggtt tttttagcca gtcaaatata gcagtgggag gttgtatacc aattttagtg 86580
acacaaatgt taataagttc tgataaccca ctaccatcgg accagccgga gttacactgt 86640
tgtttgacag cagggtgtct ctgaacttgg ggatgggtgg agtgggctgg gtccggcacg 86700
gtggggtatt agagaattaa agtctctagt tggatgtttg aagactcatt ttcttttttt 86760
gtctcttctg tctcttgtgt gtctgtgtgc ctgtacatac ccctccctca gactcccgaa 86820
gcccagacag ctcctcccca aatccccttc cccagggagt ccctccccct tctcctcctg 86880
ggccacccct accccccttca acagctccat cccttggagg ctctggggcc ccaccccccac 86940
ccccgatgcc accaccccca ctgggctctc cctttccagt catcagttct tccatggggt 87000
cccctggtct gccccctcca gctcccccag gattctccgg gcctgtcagc agcccccagg 87060
tgagaggttg tgaccaactt actgcctggc cactttcatt ttcttgtttt ccttgtaacc 87120
ccagatcctt gtgtgaactt ccccatggcc cattgacctt ccaatcctct aaccttgtca 87180
ggcctgtggt ctttatgaga ccccttgcct ttcctgaggt gactgatctt tcagtgcatg 87240
cttcctcccc taaaagaact ctaatattct ttgtttttgt gtgtgtgtgt gtgttttttt 87300
tttttttttt ttttgagatg gagtcttgct ctgttgccca ggctggagtg cagtggcaca 87360
atctcagctc actgcaacct ctgcctcctg ggttcaagca attctcctgc ctcagcctcc 87420
tgagtagctg ggattacagg tgcctaccac cacgcccggc taattctgat attcttgatg 87480
tccttttcat ctatctgatt ttctgccctt ttgatcccat ctgctttccc aagacacctg 87540
cccttcaggg tcactgtgtt gcacaatttc agggggggcc tgtttattca ctttggggtt 87600

gtgctccagg gatggccttt cacatagact gcagtgtaaa tgacagcctc tggaatgtgc 87660
attgcagggc cttgcttagt ggtagggaat gatttccatc acttctgtga cattctgctt 87720
cccaataagt cttcctgtga cttccctatt tcccccatcc cagattaact caacagtgtc 87780
actccctggg ggtgggtctg gcccccctga agatgtgaag ccaccagtct taggggtccg 87840
gggcctgcac tgtccacccc ctccaggtgg ccctggggct ggcaaacggc tatgtgcaat 87900
ctgcggggac agaagctcag gtatgtggct cagaggatga acagagaggg agagtctggg 87960
ccatgtatca tcacctgtgg gattcccagg gcttatggag tttggtcaga gcaagtgacc 88020
tgggggaggc ctgatgggag taaagaagct gaagctgaga tgtaggacgc gattgggggg 88080
aaggtcagag ggaaaaggaa gcagcgtgta gggtttctga acagtgagga gactgggact 88140
ggatcatcac tcagctcctg gttccctatc tctgtatttg gcaaacaggg aggactttaa 88200
gttttgctag ggggagattg gaaacagact aaatggtgaa ggtgtctcca tgcaaccttc 88260
ttattgctcc tcccctttcc ctgtaggcaa acactacggg gtttacagct gtgagggttg 88320
caagggcttc ttcaaacgca ccatccgcaa agaccttaca tactcttgcc gggacaacaa 88380
agactgcaca gtggacaagc gccagcggaa ccgctgtcag tactgccgct atcagaagtg 88440
cctggccact ggcatgaaga gggagggtaa ggacccgccc tgcccaggcg tcttagacca 88500
catgcccttc tcccttatcc cactcagact gtgtacctct ctggaagggt ggactgctct 88560
ctctcctcta gagggcgata tttgatttct ctctcccttc gcagctctcc tccctgttac 88620
cctgttctgg cccttgagtt tccttcttaa tggttaggcc tctgtggcct gcacatatga 88680
cccctcccta gactgatcct aggataggat aacttttcct tagccatgat gggctgtctc 88740
cccatttcta acctgagtgg ttcctcctct gattccatgg accttctctg gtctctgtct 88800
cctgttatct gtgatctttc ctctaactcc tgggactggg cactcttaag cttacagact 88860
ttactgaaga cctcagggct tcccttcctt cttctgtgtc tccatcttat cttcttaagt 88920
cagattccta ggattctgag agacacttta gagcatatct tggtcaaaac acccacttta 88980
gagaagatga gcctgaggct tggctttccc aaggtcatgc aacaaaggag tggcaggcag 89040
gtttgggatc agaacccaca tcttcaaaga cctaggccag tgctcctttc actgcctatt 89100
tgttgagtca gaagagcaca agatttgcac tcagacacac ttagagtcag ttgtatgacc 89160
tgccatgtat tatatgaatt taagcaaact aatctctttg aaccccgagt tcttcatctt 89220
aaaaatgaga ataaatgctt ccagcatcat tatgataatt gagtgaatgg taaaatgtgc 89280
ttagcatgtt tcttggcata ttttatttaa aaaaattttt tttttcaatc agctttctca 89340

ggttgaatct tggcatgttt tttttaacca tgtagtaggc tctccattta aattcctccc 89400
taattctttt acctacctca cccttcccga ctgacttacc tgccctttta taacccttcc 89460
ttcagggctg cccccagtcc accctttcta gtgacttccc caattcctat aaatatctcc 89520
caagggccat gagatcctga tgaggccgta aggatatttg tggaacccct tcatggctgg 89580
ctgctgactt tccatttttt ctctccccct tccccctgca gcggtacagg aggagcgtca 89640
gcggggaaag gacaaggatg gggatgggga gggggctggg ggagcccccg aggagatgcc 89700
tgtggacagg atcctggagg cagagcttgc tgtggaacag aagagtgacc agggcgttga 89760
gggtcctggg ggaaccgggg gtagcggcag cagcgtgagt gttggggtca atccactctc 89820
cttcgtgatg gggttggggg gaggcagtct aggtctgttc tacatcccct ccccctcctt 89880
tcccctcata accttcctaa cactacttgg gactggaggt gctgccaaac aaggtctttc 89940
aaacatctga ggtggatgtg atagctcctt ctgtctccac tccccaaaca acccactggc 90000
agaaccatag gcatgtccca aataaataat tgtttgcact aatgccagaa gagaagactc 90060
acttacaggg attggtttgg atggggctca caggaagact atatgtaagg agggggtgtc 90120
aaaagcctct tacaaggggg ctcccagcat atctcaaaat cttccataac tcttaccccc 90180
gtcccctgca gccaaatgac cctgtgacta acatctgtca ggcagctgac aaacagctat 90240
tcacgcttgt tgagtgggcg aagaggatcc cacacttttc ctccttgcct ctggatgatc 90300
aggtcatatt gctgcgggca ggtcagtgac cttggatccc tttgacttct tgacatttga 90360
cccctctttg acttcccgat ctttagtgac cccagtggcc ttaccttgcg tacccaggga 90420
gccaaacttg ctgacctcgc cacctctttt ctccttctct tccactgatg tgctttgaat 90480
cccttggcct gatttctggc tcctgaccct tgctgcccca cccaggctgg aatgaactcc 90540
tcattgcctc cttctcacac cgatccattg atgttcgaga tggcatcctc cttgccacag 90600
gtcttcacgt gcaccgcaac tcagcccatt cagcaggagt aggagccatc tttgatcggt 90660
cagtggccct cggctaggct ggcatgtaga tagaggggt ggggctatag gctggtccgt 90720
gtccaaggct ggctgagctg tgacctttga gtgacctgca ggtccctctc cagggtgctg 90780
acagagctag tgtccaaaat gcgtgacatg aggatggaca agacagagct tggctgcctg 90840
agggcaatca ttctgtttaa tccaggtaag aggaggatta cctttgtctc tcaaggccaa 90900
ggcaaccttg gagcctcctc ttctccgaga ctcctaagtt acccagctat ccccctcagt 90960
aagaccctca acctggaaat ctcccaactg gccgaggaaa aacccacatc cacggatcca 91020

```
tcccacaaac ccagtgggcc ctgcttccct tgccaggcct ctggtaaagg gcaagcaagt   91080
gcagcccaaa aggggcatcc tgtggctcac atctgcatgg ccatcctgat ttggtttgtc   91140
ttcatttctc ttcctcctct cttccctgcc atcccagatg ccaagggcct ctccaaccct   91200
agtgaggtgg aggtcctgcg ggagaaagtg tatgcatcac tggagaccta ctgcaaacag   91260
aagtaccctg agcagcaggg acggtgagat ggggctgggg ggcactgagg ctcccttggg   91320
ggtggggagt gctatgagga tgtgttcagg gcccgtgtgc ttccagctct caatcccctt   91380
ctcccacctc caccccaggt ttgccaagct gctgctacgt cttcctgccc tccggtccat   91440
tggccttaag tgtctagagc atctgttttt cttcaagctc attggtgaca cccccatcga   91500
caccttcctc atggagatgc ttgaggctcc ccatcaactg gcctgagctc agacccagac   91560
gtggtgcttc tcacactgga ggagcacaca tccaagaggg actccaagcc ctggggcagg   91620
gtgggggccc atgttcccag aaccttgatg gggtgagaag tacagggcag aaccaagaac   91680
ataaaccctc caagggatct gcttgatatc ccaagttgga agggacccca gatacctgtg   91740
aggactggtt gtctctcttc ggtggccttg agtctctgaa tttgtcgggt tctcccatga   91800
tttggggtga tttctcaccc tctgtccttc ccccagcaca aagcactggc cttgcctcca   91860
ggaccttgct tccttctcat cttgcctcat tttgcttccc atctgaagag tggaaatggg   91920
gaactccccc agaggtggat actgggggc aggcctccca agctgatgga catgagagta   91980
gggccctgac aggccttcct cctctcaaac ctggcagatg gggcctctc tggaagaggg   92040
aggggccctg tcactgtcca gagtctcttt ttacacttca cctccttctg cagtcagact   92100
gaaatataaa aaaggtggtg gtggtggtga aggggctggt ggagatgtag gaaccgatct   92160
gctattttta atttcctgtg aggatagaga cttgcagtta gactcaaaga agtactgtac   92220
tttcccaggt tgactaagaa atgccagtgg tggaggtggg tgtttgggaa aggcagggcc   92280
ctgaaatggc ctgtccctag ggctctccaa gcactagcct tcccagcttc ccgccgcccc   92340
ccctatctct tcctgtctaa cttggggaag gggcctgggc tgtgaggaca gggcccccac   92400
aggggatggt ttcacgagtg tagtcccgga ggccttccct ttacagctct cctccagccc   92460
tgggcacata gcataggctg gggacacagg atcctggcct gagaattgag gggaggtggc   92520
cagcccgcag aggtggggtg ctggggctgc atgatttttg ccctgcgtcc cttctctttg   92580
gggctccttt cccctctcat acataaaatc gctttcaaat taaaatcgct gttttctgga   92640
ctgaggtgac tgtatgagga tgggcagcgc cgtttcgggc ttgggggggg tatccggttg   92700
ggagctccgg acgcgatccc tggacgccgc cgccgaggtt ccgtggggcc caagaggtgc   92760
```

acgcagggtg ggggacacaa ggcaagcttt gtcggggagg gggggagagg gtgtgccagg 92820
ctgggggcgg ggccggccgg aggaggaagg gggggcggtg gattctcaaa ggcgccttgt 92880
tcgctcgtgc cctctccgcg ccagcgggcg gcggcgcctc ggctcgctgc ggccttccct 92940
ccgggcgcgc tgcgggctcc gggcagaccc ggcgccgtgc ccgctcgtgg gggcgcactg 93000
ggcgccgagc gctgcgtgct tctcaggcgt tgcccgcccg gaggcggccc acgtccccga 93060
gtgaccccat ttccctggac ccttccaacc agagtcgact gctcgctcat cttaccactg 93120
ctccccctct acctcggctc tgggactccc taaccgcgtc ccccatttgc atctccttgg 93180
acctgactat cctgtgtttg tcggtgggtc ccagtctctg gcctcttgct ctccgtagaa 93240
caccttcccc cacatccccc gccccagttc gtcgctatct atagccttgt ctataaatac 93300
ccccgccccg gcccggctct gtaattacac ggggcggggt gagggaagta attatggaga 93360
cctgattatg ggtggggtgg ggactgcgac ccccaggccc gcctctcccc ctcccactgt 93420
gccctaaatc ccgcccaggc ctctgctgaa agggggctct gggcccccaa gagggaggga 93480
atgggaggga gtgtgtgtga ctggacgttt gggtcctaga aaaggaaggg gctagggaag 93540
atattggggt tcccgaaaag agaatcttag ggtacaggcc gttgagacct acaaggggca 93600
ggagagagcg agcgatagag ggagggttcc cgcctccctc cccaggtgga gactgagggt 93660
ggggtttccc ttcggtggct gtgggcgggc ggctggaggc gggggccggg ccgggggcgg 93720
gggcgaggtg ggggctctgg gcgccagggt ggccggggac acacagaagc ggcagccacc 93780
gaggagggag cagtgccggg agccccgacg gcgccttgct gcatggagct gggccgctga 93840
cagctgtcgc tgcccgcagc ctctgacctc cctgggaccc cggcgtctga ggctcatagt 93900
ctgctccctg tcttctgtca gcctcagggc atccagcgtc tcaggccgac ctgggtccct 93960
gggacccggc gtttcggctt ctcagccatg gagcggtgca gccgctgcca tcgcctcctc 94020
ctcctcctac ctctggtgct ggggctgagc gcggccccag gctgggcagg taagaggagt 94080
cctgatgcct gggtcctcgg agtcaggctg gagctctgag tgtctctggg aaggggccag 94140
ctgatgcctg gggcagcagc ttctgagtct aacaaggaga tttggggctt caaggctaca 94200
ggctggaggg caggacacct gtgttccttg gcatcagggt aggaatctct ttgtccttga 94260
agtgactggg ggagggcaga tagggctgaa aggtggagga ccactttaga gaccctgaat 94320
ggggatggat gcttgtcttg agtccttggg aagagctgaa gatggccagg gccagcccgg 94380
tccagtcatt gctggtgtgg ggatggtaga cgccaatgtt gatgggtgag gcgtgtgtgt 94440

cttatggcct ttctgtcgat atctctggga taaggggtga gcacctgtga ttcagagcag 94500
agcccagtaa gcccagaaag aaaggaagcc ttgactacct gtctcattgg cctcctggac 94560
agggtccccc tccccctccc tgtgccctcg atgctgccac gcagctggct ctggggagca 94620
ctggggctgg ctgccaacag gacggccctc cggacaggcc aggatcaggt gtctgaggcc 94680
agagccaact ctttgcattc ctgcctagcc cctcctccct ctggccagcc aggctcccct 94740
gggaccctgg tgcccacaac cctgatcttt tccttctttt tcccaggacc cagaattcct 94800
ggcttctagg aaaggggatt tgatgtcaag gagggggctg tgagcaccag gtcctcagca 94860
ggctggaaaa agcccatttc tggagacact gagggctgat ttatatttaa cttgactatt 94920
caattcttct ctgctttaga gagaaaatgg tcagaatggc agcacaaaag atttagatta 94980
gattacagaa ggaagaactt ccaagatgtg aggacagtga agcccagggc caggaaattg 95040
aggcgtatgg tgcctgtaga ctctaggagt ctttcttagg gggaagaggg atcctctggc 95100
atggaggcag ggggatggct aagatgactg tttcttattt caggggctga gtgggtagag 95160
atgggacttt agggaaatga gttactgtag aattcctaag gttagagaat aaggcatttg 95220
gggggccaga ggtagagtgg tggagggtaa tggggctacc agtggagagc ctgtggggca 95280
gatgaggctg gatacagggg ggcaagtatg tggcctggtg tgggaagatg gtgggcggac 95340
ctgtgggtag gttgtccata tgatcgaatt gtactccctt gggatttggt ggagggtggg 95400
gacctccgag gtctccttgg cctggggtct ctgtgagtct ctgtgtctct gtctcactca 95460
cccttcgtct ctcaaagcgg cccttgtgtg ttggtgtttg cggagctgcc acacgcgcag 95520
gggccaggct taggtggggg ttggggggga aggccgtggt ttcccggctc ctggactcaa 95580
agggcctttt ctctgcctgc ccgccccacc tcacccaccc cacccagctc cactctcctg 95640
attgctctgg cctctgccct gccccacact tctatgaaag ttttgctgga actctctttg 95700
ggatttttcc aatctggagc tgatggtgtg aagagtatgt gtataggggga gcaagtgagg 95760
gcaaggtgat cttttgtttt ccagggagtt tgagccctag gaagctgagt tctagttaga 95820
cagggctggt gggctggatc ttgaatcttt gggtcccagg tggtggggtt ggtgggggtg 95880
gtgagacagg attgaggttc caggtgtggg gagcttgcct gggttctggg caagcactct 95940
gatgggatcc acagatgtgt ggctcagggc cctggacctg ttccggctcc tcgtggcagc 96000
ttcagcccct gccccccatt cctccacccc tccccaactc atctcctaaa ccaggaggca 96060
cagactcctg ggagagcttt cttgtatctt tttcctcagc atgctcaggg ttgttcccac 96120
cccatccccc aaacctggga tctgtagctg cttgaagccc ctctcataga gctgcttctc 96180

tgatctcttc ctcgtggccc ccctgcacct cctgtgtcca ggatgatcga ttgcccagat 96240
gttgtttgtg ctggagacgg gacagatgcc agagacccag agagtgagag atatggaaag 96300
agagtgagag cgagggagag aaaaagatgg acagaatgtg agagaaatgg aggaagacag 96360
aagagacaga gacagaaaga ccaacataaa gagacagaga gagtaagaga gtgagagaga 96420
caggagacag atgaagagtt gagagaagta ttcagagagt acagagagag acagattcat 96480
aagctaaaag acaaagagag agcgtaagag aggcagatgc taggagagac aggggcagga 96540
gtgtcatggg gttgaatgcc agctgatgct gccctgcctc caggtacctt tgcatttccc 96600
atccattcag gtactgttca gaagcaagtg agggtgaggt gacctttccc ttgcagggag 96660
tttgagccct ggaaagctga gttctagatc ccaaatgtcc cctatttatg ccctcctgag 96720
ggcatgtccc ttctcctgat aatcatggac tctcccaggt gcaccccctg tggatgtgct 96780
ccgggccctg aggttcccct ccctccctga tggtgtccgg agagcgaaag gcatctgtcc 96840
agctgatgtg gcctaccgag tggcacgacc tgcccagctc agtgcaccca ctcgccagct 96900
tttcccaggt atgggtgaca tggtggggta ggcctggggg gaggtaatgg gatggggcct 96960
aggatcagac accaggagga aaggggttgt ggcggctccc tttgcctctc actctgtgtg 97020
tatctctctc ggttactagg aggatttccc aaagatttct ctctgctgac tgttgtccgg 97080
acccgccctg gtctccaagc tcccctcctg actctctaca gtgcccaggg tgtccgacag 97140
ctgggcctgg agctgggccg acctgtccgc ttcctgtatg aagaccagac tgggcggcct 97200
caacctccct ctcagccagt cttccgaggc ctcagcctag cagatggcaa gtaagtttgt 97260
ttgctcctct ggtctgcctg gcccacactt tcaggaggaa gtgcccccaa acccctacac 97320
tctaaactgt gaaacccttg agaccctttg ggccacacca cacctaccca ctgcccaaac 97380
ttcagtcact tctagtccag agtttgggct ttagagtgta cagccttctc tgcatgttga 97440
actagcctgt accttgggca agttacttaa aatttttgag cctcagtttc tacatctgta 97500
aaatagacat taaatagaat tggcacaaat aagaaagtga cggcatggtg tctggtgcac 97560
tgtaaacact caataaatgg tagccattgt tattactgct cttatcacca ttggcttcaa 97620
ctcttatcac tgctctccaa ccatgttgac tcccctactt cagtcagcca agagttcact 97680
tgaacctctt ccaccatttc caggtggcac cgtgtggctg tggctgtgaa gggccagtct 97740
gtcaccctca ttgttgactg caagaagcga gtcacccggc ctctcccccg aagtgctcgt 97800
ccagtattgg acacccatgg agtgatcatc tttggtgccc gtattctgga tgaagaagtc 97860

tttgaggtaa ccagagcaat cagaggcagg attgacttct ggtcccctat cttgtgccca 97920
ctacccttct ggccccagca tgtcatcttc ctattcccta ggccttcatt tttttttctt 97980
atgaattttc atttctattt tctatcttca gggctactgt tctctgcagg agaactagat 98040
gcctacctat atggcaggac tgtccacagc cacttactct gtccttcagt cttcatcagt 98100
tccctcatcc atccatcctt tcaacccctc tctctcctca cccacccatc aattcactca 98160
ctcctcagcc aacctatgca cacatccacc acccactccc ccatctattt actcatctat 98220
gcatcacacg cccacctagt catgcatgaa ggcttatatc cacccacctg tcaatcctcc 98280
ccctgcttaa ccactctccc cttcaaacca cccatctatg cttccaccca tctatcctct 98340
tcaactcctc catccagcta ttcactcacc cacccaccct ccccatctac ccatgcacac 98400
aaggatgcat gcatccattc agccaccatc tatccattat ccttccacca actcaaccac 98460
tccctccttc aatatactca ctcacccatc tcctgaccca cttacttccc atccgaccag 98520
tcattcaccc acataagttc acccatctac ctattcaccc atttgcttcc atctactctg 98580
taacccctgt tcatctgccc accagtccac cctcccatac gtacatttat tcattcacta 98640
tttatcaatg ctcccattca ttaatgcatg taagccccca gccacccatc cactcatcta 98700
tccattcatc catccctcca ttcacccatc cctccattca ccctccaagc cagtcaacat 98760
tttctgaata cctgctggac aaggagaaca aagaagagaa tataaagtct ctatcacccc 98820
caagagctcc caggtgggct ctggaaatga gacaacatcc ctaaattacg gtgcaatgga 98880
gagtatgcta agcaccatgt gtgggcaaaa gtaaatgcca ttggaactca ggaatgggtt 98940
ctccccactc catttctaga ggtccagaga attctcatca tcactgccat tggaaggcca 99000
aggcatctaa agctgagccc tgtctctgga agccactgtg gaagggctca tgcattgcca 99060
caaagcgcag aggggaggca aagaattctg gaaggcctgg gatctggtcc tgattctgcc 99120
actctgggtg accttggggc agtcattgcc tttttgggct tttctgaatg gaacgtatct 99180
gaaaaatggg tagaaagaaa gatccttgtt ctgccaactt tcctggttgt tatgaaggtc 99240
ctatgagatc ccttgaaaac tgaaaagggc tatgcatatg gaggactgac agtgaccttc 99300
tcccatctta gggttttacc taggattggc ctcctatact ctttctcccg gatgataccc 99360
tctgcctctc tctgaatctc tccgctccat ctctctcatg tctttgcagg gtgatgtcca 99420
ggagctggcc attgtcccag gggtccaggc agcctatgaa tcatgtgaac agaaggagct 99480
ggaatgcgag gggggccaga gggaaagacc ccaaaaccaa cagcctcaca gagcccagag 99540
atctccacag cagcaaccat caagacttca caggccacaa aatcaggaac cccagagcca 99600

ggtgagggag ctgggagaac ccccaagtgc agcacacccc agagagggaa gacacccagg 99660
catctctcct ccttagttgt cctccccacc ctcatctccc tattcccatc accccctcct 99720
cctagtcctc attcatcagc ctttttattt tcatctaaaa ataaaaagag ttgttatgaa 99780
gaatttgtgg ctgggagctg tgttccctgc tctgcgtctc ctcttccagt cttcctggaa 99840
ctgtgtccct gggttttccc ttccttcttt gaattaagag attgggagac agtgggagag 99900
gggtgagagc tggggaggca ggtaggagag gggactgaag ccaggagaaa gcagtcggga 99960
tggtgagacc aaggaggagg aatgggaagg agtagggatg gagggaatat caaaggaggg 100020
gtgggtctgc agaagtgtgg gagtggggaa cccagtctct ggccccccacc tacctggagg 100080
ggcaggaaaa ggggaggtag taggggggaag ggaagggaag ggaagggaag ggagcctggg 100140
ttggatgggg tctggagagt tagaatacag ataggctctg gggtcagtgg tctaaagatc 100200
agagggtcaa tctcttaatc atgtctgctt ctgccccaca tgtggaaccc cttccctctg 100260
ttccacctct ccccacctcc ctcctgaccc tcaacctctc ctttcatctt caactctctg 100320
tttcccaact ccacacctcc cccgtttccc aactcccaca ccatctttat atctccctct 100380
cccgcccttc accccacacc ttccaattac cccatcccct tcctgtctat gcccaccccc 100440
caatcccagc ccactgagtc tctctactat gactacgagc ccccctatta tgatgtgatg 100500
actacgggga caacccctga ttatcaggta aactcaaggg accccttcat ttcttccctt 100560
tctcccagca cccccagcaa tcatctccta ggacaccttc ctcccctccc ccatgccgtg 100620
gggagaggca cagtgtgttt agattatggg gatttactta gttcctgccc cctccctgtc 100680
tctctgctgc agcgccctgg gagccccccac cacaggcctc cccccttcccc tagggggcat 100740
cggacccctc cctgaaagcc taccccacct gccaagaggt caatgacctg gcaggcgtcc 100800
ccctcaccag gccggaggcc ctctccaact gcaggctttg acccagcatg ggagcagcct 100860
cccctttccc gatgcccagc tcggagggca gcttccccca ctgcctggcc ccgccccagg 100920
accagaggca gcagttaccg gcaggtagag ccggggcatc cagatctctg cccccaaacc 100980
tctatgacct tggaggttca ctctccccaa actatccccc tccaaagggg ctgcaagatc 101040
tgagatttcc cccatccccc agcacagaca gaccctgccc tcgggggaca ggtgctgctt 101100
tgcctccctt ctgctaacct ttctccttcc ttcccacccc gtgctactct tctccttctc 101160
tccttggggt caggacccca ccccaggtga agaggaagaa atcctggagt cgagcctctt 101220
gccacccctt gaggaggtaa ctctatgccc caccctcacc ccatctgggg gcagtgggca 101280

cctgtgactc actcccctca gcccatgacg tcttgatgga ccagcccctc ctctggccgg 101340
agggttcttc ctcatttcca tcgatggcct ctgtctttgg gtcactctag aactatgggc 101400
ctgcttgact ctctaagtcc agcccgttcc cttcagtatc caggcctcct tccatgattc 101460
tttgattcct gagtgacttc gtctccatgt tggtgtctaa tggtcttact ttctatgtcc 101520
tatttctgtt cccgggagcc actttctgtc cagtgttgtc accttttgtc ttccctatct 101580
ctttggtcat tcctggactc ctttgtctgt cattgtgtcc atggactgtc cactctgcat 101640
ccagtcctct ggccaccctt cattttatcc accacttctt cccactggac ccactttctg 101700
gacatctctc cccatttttg gcctttgatg gcccttccat ttatatacaa ccactcccat 101760
ggctattctt gggagaaaaa atccagttct gattaacctt ggaatccttt gggctagaag 101820
ttgtcacacc ctggggtcca gggtaagcct cctctcagcc ctaacatccc catctacccc 101880
tctccctccc catccctggg aaggagcaga cagatctcca ggtccccccc acagccgaca 101940
ggttccaggc agaggaatat ggggagggtg gcacagaccc ccctgaaggg ccctacgatt 102000
acacctatgg ctatggggat gattatcgtg aggagacaga gcttggccct gccctctctg 102060
cggagacagc ccactcagga gccgtaagtg aaaggagctt ctctttcatt cttgactacc 102120
agtgggagct acaagaattg cttcttgttg gtttcgatcc ttggctgttg gctgtgagct 102180
atttcccatc tgtgacttgt cattttctgt tcacatatga tgtatatgtt gcttgaaaag 102240
attactacct gccttccttt gccaaatgtc ttaccttgtg actcttgtgg atacttctga 102300
agctcagggc aattgggaat taaaaaaaaa aaatatgtag ccacttcctg tctttcttga 102360
tcacttcctg tctaactgaa gctggatttc ataaagcttt tggtcaataa actactacct 102420
tgtgtccatt ttcagtgttt ctctatcatt tcttcttggt gaaataattt tttgttcatg 102480
tttctaccaa acgatctaac ttcctatgtc accttttttt aacctacagg ccacttatta 102540
tctgtccctt cctgccttct ccagctgtgg catccatctt ggtaactgtg tcaggttgga 102600
ctgcaaagga tttattattt ctgttctggc tctagttttt catgctatct tgtgacaatt 102660
tcctttgaca ataagccact tcctgtatgt ctcaggtcat ttaaatcata aagtttccat 102720
tgtgggcctc gatatttctc atggctactt cctgtgtgtc ctagccttga tgtttgtgtt 102780
ttctggaaga attacccttc actgtcatga ttccctttgc aggagtcaca ttctacctgt 102840
tttgtgcctt tcttttctct ctgggtcacg gtagccatgt tgttgttgaa ggttcacttc 102900
ctggatcctc tttccttcac tgcttcctgt ctgttcagcc acttcctgtt tgtccagttt 102960
gttttctagc catgttggcc atgtttaatt ttcttcggcc ctggcatcat tcttgcttcc 103020

aagtgtttgt cattttgttc tgtgtccctt agttgttctc atatcttcca gtattccctg 103080
tcagtgggac tgcttcctgt ctactccgct ccgtcttctg cctgccttgc agttctggct 103140
gcctcctcta ccctgctgcc gcatcccata cctcccctct cttgcctccc ttgagcttct 103200
actgcctttc ctttatgctg ctctctgggt atttggggtt gatatgggat ttcagagaat 103260
tttgaaacgg atcattgctg tttggtcaga atgaaggtgg agaggggcat tctgaagatc 103320
tttgttgggg ggagttgggg tggtggtgaa tgtctcccat tctccatgtt cttattttgt 103380
ttctggccct agagttggga acaggtatct gagtggaggt gtgcaaggga gggaactggt 103440
gggcttggaa tgtacttgag ggccagagga agggcaatgc attcagtggg gactgctctc 103500
caattttttt tttttttgag gcagggtctc actctgtcac ccaggttgga gtgcagtggc 103560
aggatcttgg cttactgcaa cctctgcctc ctgggttcaa gcgatcctcc tgcctcagcc 103620
tcccgagtag ctggggttac aggcatgtgc cactgcgccc ggctattttt tgtattttta 103680
gtagagacgg ggtttcacat gttggccagg ctggtctcaa actactgacc tcaaataagc 103740
tgcctgtttc ggccccacaa agtgctggga ttacaggcgt gcgccaccgt gcctggcctc 103800
caaatttact tagagtatgg atgcttaacc tttggggcat atttaaactt gtgaaccctt 103860
tgaaatccta aataaaattc tgtgcataat agcatatact ttttcatttc tggagaaaga 103920
ctcacagctt tcatcagatt cttttttttc tttgagatgg agttttgctc ttgttgccta 103980
ggctggagta cgatggcata atcttggttc acttcaacct ccacctcctg ggttcaagct 104040
attctcctgc ttcagcctcc caagtagctg ggattacagg catgtgccac cacacccagc 104100
taattttgta tttttattag agacggggtt gcaccatgtt ggtcaggctg gtctcaaact 104160
cctgacctca ggtgatctgc ctgcctccgc ctcccaaagt gctgggatta tgggcgtaag 104220
ccacctcgcc cggcctagct tttatcaggt tctcatgtgg gtccatgccc tcctcttacc 104280
ccaaataaaa atctacccac aatcttagag agattctttt atgatgtatt ggggtaaatg 104340
tgtggaaggt ttcttaaggt tctagggagg gagtttggga gatgggatag ggtcctctag 104400
aagagggttt gggaaggtgg gtggtttaaa atccctgaaa ggggccaggt gtggtggctc 104460
acgcctgtaa ttccagcact ttaggaggcc aaggcaggcg gatcacctga ggtcaggact 104520
ttgagaccag cctggccaac acggtgaaac cccatctcta ataaaaatac aaaaattagc 104580
cgggcatggt ggtgcacatc tgtaatccca gtgtaatccc agctacttgg gaggctgagg 104640
caggagaatc acttgaacct ggaaggcaga ggttgtagtg agccgagatc gcgccactat 104700

acttcagcct gggtgacaga gcaagacttc gtctcaaaaa aaaagaaaaa caaacaaaca 104760
aacaaaaaca ctggaagttt ctggggacct tcctgaaaga ggctctcagg gagatggaga 104820
ggttgtgttt gctgaggtgg ggctgggaac tgggggggca gggaaccctg aggtctcctt 104880
ggcaggcttg aaaggtttgt gaagagggag ttttagggga gggctgtggg gctttcgggt 104940
agggctgaag tggtctcgag aggatctaag aatcaaggtt ggagttgaga tggagaaagg 105000
cctagtgtcc tggctgctca cgggccccac tgggggctac atgtgtgtct ccttcaggct 105060
gcccatggac cccgagggct gaagggagag aaaggagagc ctgcagtgtt ggaacctgta 105120
agttatgctg gtcacagggc tgaggcagtg gaaataggag aagcaagtgg ggttgagtgt 105180
gctggtcctg tcgcctctga tttttaacct ttgactccac agggtatgct cgtggagggg 105240
ccccctggcc cagaaggccc tgcggtaagt ctagcagtga cctggtggcc attcttttct 105300
tagaaacccc ttctatgtgc tcatctgagc cttccccaca tatgcccagg cctcctcctc 105360
agaactcggg agcatccctc caatcaacgc ttcccagatt cccagatctg ttctgcacag 105420
accactcctc agccacaggc tgagtgtcca cacctgtctg aatgcccaca cctgacccct 105480
actcttttgt tcctcaggga ttgattggtc cccctggcat ccaggggaac ccaggcccag 105540
ttggagaccc tggagagagg gtaagggggt gtcctccatg tgacggggga gcttcggggg 105600
agctagtggt atccaagcgg ggggctacca tgcccagtaa tttcctgtta ggtggcttgt 105660
taatgaatga ggggatggtt gtgcttccca gcaaccagaa aggagggcag actctggttg 105720
gggagggtgc tgatctggtt tctcaccctc aattttcccc atgggggtgg gagactcagg 105780
aaagagctag agcaatctac acttattctg ttgaagagga tatttcaaaa tcatgctaag 105840
atcttgatgt ccccttatct ttcaccccat cttttgcatt ctcttcccac tccagggccc 105900
ccctggccga gcagggctcc ctggatcaga tggggctcct ggtcctcctg gcacatctct 105960
catgctccca gtgagttgtc tcttgggttt tggaacatgc tgatggggaa gacaaggaat 106020
tgtgtcatgt taccaagaac cagatgggca ggaaagatat ggaggagtct ctaaagatca 106080
tcaaagatca tcactccaaa gggattcgtc tttgggggttg gggagatggg gcctcattag 106140
gtgacctaga acaaaacacc agtcttctag gactgggcag aacagcctat atttcaggag 106200
ctctagaacc aaaagagagt ctttctggag gcagaggctg gacagtggag gagggcagga 106260
gttgagtttc tggaatcctc taagtgatta tctggaagcc atgggaaatg ctggatgggc 106320
agaacctggg gcaaggggca gaaacgctgg agaaagcctc tggctgggag ggtcccatgg 106380
ctttcttgga agagaacctg gaccttctct ccttgccctc agttccggtt tggcagtggt 106440

gggggtgaca agggccctgt ggtggcggcc caggaggctc aggcccaggc gatcctgcag 106500
caggcgaggg tgagtggggc tgcttccctg gaaagggaag gctctggggg gcactggaag 106560
ggttggctgg agtgagtgtg cggcagggga ggcctgggtt ggtttggggt taacagggag 106620
gttggggaga atcctgggca gtggatgagg atcaggagag gaagggtgaa tttggagagt 106680
gctggggctg gggttccca ctgcctgtcc ctcagctctg ctgtcccccct gctcccctag 106740
ctggcgctcc gtggaccccc tggccccatg ggatacacag ggcgccctgg acccttggtg 106800
agtgagcagg gtgctcgggt ggaggatgct ttaattgtgt gtggggtgtg gatagttctg 106860
gaaaggggct tcttttgggg aacactccga ggccactgtt ggctgtgtcc ttccttacgg 106920
ccatcctctc ttctctctcc cagggccaac ctgggagccc tggcctgaaa ggagagtctg 106980
gagacttagg acctcaggtg accactttcc tccactgcac ccccatatct gttcaccagc 107040
tcagtctccc tcactcccct ccctatgcct cctaacccca ccccatctct cctctcacca 107100
gggccccaga ggacctcagg gcctcacagg ccctcctggc aaggctgggc gaagggtgag 107160
tgccccaggg gtggatgggt gttgtgggga gacagggcct gcagggtagg ggactgcggc 107220
cctgcttgtt ctgacacttc ccttgttctc ccagggccgg gcaggtgctg atggagcccg 107280
agggatgcct ggagatcctg gagtgaaggt aacaggcttg ggcccctccc tgaagcctgt 107340
agccttcagc ccacgctggg ctcagttgtt tcttggggat gacctagttc tccaggcttc 107400
cccaggatca gcaccccccc agctcagggt gcagtaggga ggggtgggct tggacagggt 107460
cgtgtcctca ctctggctat ccttcctcct cctagggtga ccgaggtttt gatggactcc 107520
cagggctccc tggagagaag ggccataggg tgagtacatt agtgggtgtg tgttgtaatt 107580
ggggatagat cttctatggg agtgaggata ggtagggaag ggaggctgga ggcttggcag 107640
gagctcagtg aaagtaatgg atgggctaag tgcagaggtt cggtgtctgc ctgtgttggg 107700
ggctctgaag cccctcttat gagttccccc attttgcagg gtgatactgg tgcccagggc 107760
cttcctggtc cccctggtga ggatggagag agggtaagtg tagtggcaaa tgtaggggct 107820
gggtgcaggg gaggtctaga aggcacagca gctttaccgt gtcctcctcc tccagggaga 107880
tgacggggag attgggcctc gagggctgcc tggagagtcg gtgagtgtcc aagggctggt 107940
gctgggggag ggggctctgg ggaagccagg agggggacat gagcactaaa gggacacagt 108000
tttatccatt catgccgtct cctcccctca ccgtgaatgc agggacctcg aggtctcctt 108060
ggccccaaag gcccacctgg tattcctgga ccccctgtaa gtgactccct gacttctgac 108120

cctgaggtga cccccttgacc tctccaactc tccacctttc tagctgcctt cctgctccct 108180
ctcctctctc ggcatgcttc ccattccctg tgagctcccc ctgctctggc cccacactgc 108240
cctgcctcag cattcgctcc ccgtgcacag ggagggccat gtggcgtggt gccctgaggg 108300
ctctgggcat aggaaataaa tcttcaagaa aaacaaaacc aaagcccaca agcccacaga 108360
tccacagtcc cacacactgc aggcctgggc cagctgtgtt tgcttgggtt tgggatggat 108420
tggaggtctg accgtcacac cctcgggaat ggccgggcct ctgaaaacca caggtccctc 108480
cgggccacct tcaggctgct cacacccatc ttgtgtttca gggcgtccga ggcatggatg 108540
gtccccaggg ccccaaaggg agcttggtga gtgatggata ggagatccca cccccattct 108600
tatcccccga ggtccctgcc agcatcctgt tggccgccat tttgacccct gcctgcttcc 108660
tgctcttgcc ttcttggcta tcgctgctgg gactcagctc catgctctaa atgtgttcta 108720
tctccatctc cttctgacct gtgcctggtc acctgtttct agggacccca gggagagcca 108780
ggacctcctg gacaacaggg caccccctggg acccaggtga gcggtgcccc tactccgttc 108840
cccaattttc atgacttccc ttgggcttcc acaatgggtg agaccctgga aggatacagg 108900
aaattaaggg tttcaggccc ttgttgctca tgctttgaat ccgggaggct ggatagaaga 108960
gaccctggga ggcccctgtg acttctcttt ccctgtatgt agggtcttcc cgggccccag 109020
ggtgccatcg gccctcatgg agagaaggta agtgactaag tgatttggag gacagggggt 109080
ttagagtggg gatgtaggga gaaccctaaa tgtctgctgg gattttgtct gtgtccagac 109140
atgacccctc tagtgaccct gagcctcttt gtcttcctgc agggtcctca agggaagcca 109200
gggctccccg gcatgcctgg ctcagacgga cccccggtga gtgggacccc acttccgaat 109260
ccagttcccc ttggccttcc accccagtgc atgacctcag agctccttta gtgacccttg 109320
cactgaaagg tcactggtgg tgcttgtcca tggacagttg gcttcactag gtcacaggaa 109380
atttgggttg gaagaagtgt agggaggggt gcagagaggg tgacaggggc cactggtcac 109440
attttctatc ccttactccc agggtcaccc agggaaggaa ggtccccctg gaaccaaagg 109500
aaaccaggtg agatcttcca tgtctttatc ccctgaggga gtctccactc agactctaga 109560
gcctcaaagt ccttgggatt cccttgtctc attattcata tggctctgcc acattctcaa 109620
ttcctgtgcc ccgtaaccct acccagacag caatgttgtt acttccattt cttccacccc 109680
tggaggcccc cacagtgaca ctctgaggcc cctttataaa tgcctctctt ttatcttcca 109740
gggtccctct ggacctcagg gacctctagg atacccagga cctcgagggg tcaaggtaac 109800
tgaccaccag gctgggagac aaagggctgt ggtcagggga gctatatggg gtcttaggag 109860

cttggtcctt ccaacccacc tccatcccct gatcagtcta cttctatccc ctctctaatt 109920
ctagggtgtg gacggaattc ggggtctgaa gggtcataag ggtgagaagg tgagcaccat 109980
gcccccagct tcccagcacc tcagtcctgc catctcctct cttcactgcc ttccctccag 110040
cctgccccgc actgccgtcc agctcccgag cccctgcttc cacctatgtt ctcagaattc 110100
ccattagaac cccagccctc tgtcatctgt ggtgccctct cacctctatc tttctcaggg 110160
tgaggatggc tttcctgggt tcaaaggtga cataggcgtg aaaggtgaca gggtgagtag 110220
agaccccaca ctggccccaa ttccgtgttc tactgagcct ccactctgga gacgccaagc 110280
accggtttat tctcctgagt ctccagactc cacagttcca gtgctgtgtt cccttgggag 110340
cctgacccct acaggatgat agccccatgg tctgtgtcat gcctgccctc ccatcactac 110400
actatcccta ccctgtgggc ttcctgtcct gcagtggctc ctgggacccc tctagccctc 110460
ctcagcctca ctgttgccca tttctcctct gggcccagag cccacctcca gtccccagca 110520
ctcacctgtc cttccctctc acacagggcg aagttggagt ccctggttcc aggggagagg 110580
atggtcctga ggggccaaag ggacgcactg gaccgactgg agaccctggg cccccagggc 110640
tcatgggcga gaaggtgatg ggatgagggt tctgtgcctg ggtcctctgg gggtgtgggc 110700
actggggtca ggatgggcat gggtgcccgc cgctgaagtc agccagtcag ttgaagatgg 110760
ccatgcatta gtcatacttc tgtctgtgta aatggcatct ctcagctgct gatgggcaga 110820
ggggatatat aagaagtcag ggaaaagtcc ctgacacgaa gagttggacg gagacgaagc 110880
ttagagagta gctgttgctt caaggcagag tgaacatgcg ttcggactcc aagcagcagg 110940
agttctgaga tgagagagtc taatagcagt gaggtctgtg gtctccagag ctgactctcc 111000
ccagcacttg ccctgtgcca ggtgctgtgc tgatgcgtcc tatctatcct atgaggtagg 111060
catgtgacgt actgttttgc tgaggtttta cagaggcaca gggatgcttg ttgattacat 111120
ggccagtgaa gggcagagcc aggggttatg cccaggcagt ctggcttcag aatctctgct 111180
tctaagccct gctctgcagc tgcctgggca ggatgagccc tggagcctct ggggaagggg 111240
gcatgcacgg gagtcggggt gtggtgggca aagggcctgc agggggattg gtcttggtga 111300
agcaggagtg ggtgctgggg tagatcccac ctccctcatg ggtagatgg agctgaggtt 111360
gacatgtggg aagcagctag atcacagtga ccctgaagga cctgatacct ctgtaggcgc 111420
aggggaggtg ggatcccagc actagaattg ggaaaatcc actcacggga aggagagaca 111480
acaaaagtca ggcagctgtg gggcaagcaa tgaggggcta gaggctgggg tgtgtggggt 111540

gtgttgctgg gtgggggctg gctgttatat gtgagggttg aggtctccag gggtggagtg 111600
gcatctgggc ctcctctcac tcctaactgc ttcctgtccc tccacagggc aagctgggtg 111660
ttcctggtct gcctggctat cctggacgtc agggacccaa ggtgatgcca tgccccatac 111720
gtgcccctcc tcctcttccc tcctcctctt cgcttccctg atccctgatt ccagaaagca 111780
cagatgcagg gcagtggggg accccagagg gaatttagct gacccccatt ttacagatga 111840
ccgaaagagg cccagagagc agcaggggtt tgtccaaggc cacttagttc ttggcagagc 111900
tcgggtctcc ctggccactg cctttgtggc ctctcctgac cccctttgcc ccttcctgaa 111960
ccctaccttt cataacttct caatttcccc ccttctccgt tctcaccagg ggtccctagg 112020
atttcctggc tttcctggtg ccagtggaga gaagggagcc cgggtaagct gggggtggg 112080
agggcgtgag aagggaaggg ctggagaggt gtagaggggg tctgtgggga gtctcaccct 112140
gggtaatgtt ctctctgctt cattcccacc agggcctgtc ggggaagtca gggcctcggg 112200
gagaacgggg ccccacggtg agtgcagggg agagaaacag gtggctcaag gtccagaggg 112260
gggcatttgg gtttctctga caactcctct tccctctgta gggtccacgg ggtcagcggg 112320
gaccccgagg tgccactggg aagtctggag ctaaggtgaa tggtctctac aaggctccaa 112380
ctgcccccctg cccactcagc cccagctctc cctcaacatc ctgacctcgg ggacagctgg 112440
aaagcagctc ttcccactcc ttgactctgt ttccccccac agggaacatc tggtggtgat 112500
ggcccccatg ggccccctgg agagagggtg agtgtggatc gaaagtcccc ttcccctgag 112560
gtccccaggg agcctgggtg agccagctcc ctcctcaccc caggaaggaa gctgagttcc 112620
cccactcagc ccctgccccc attctcctga ctccccccac ctctgtctct agggcctccc 112680
tggacctcag ggtcccaacg ggtttcctgg accgaaagga cccccggtaa gttgaccccg 112740
agctctgacc cacctcgctt ctctgagtcc ttcttcccta cctgctttcc agctgagagt 112800
tcacctgaca actgaacccc ttccagggcc cccctgggaa ggatgggctg ccgggacacc 112860
caggccaaag aggagaagtg gtgagtgatg ctcctggcta cccattctca gggggctcca 112920
tcagaccctt taaccccaga gctgtctgga aagcccacag ggacgttctc ctggcagagt 112980
ggagcctgcc ctggtgctgc tcagacccag ggcccagggc ttggtgtgat cctgcctggg 113040
atccttcctc ctgggaatct gccccacagc gcccacacct gctttcactg cattctcctc 113100
tcttcctcgc ctagggtttc caagggaaga ccggcccccc tggtcctcca ggagtggtgg 113160
gacctcaggt aggtttgtcc ctaggagaga atggacccct gaccctagca ttagtctgtc 113220
tgcccctctt ctctcggtca cacgcccctg ccccggtcca cctgacccag actcctgggg 113280

ctggggtcaa tggtgggggc tgggatttca ggcaggggtc ctgaacttca gtctgttttg 113340
gggcgcattc tcatcgtctt gtcccccagg agaggcagtt ccccaacaga gacacaaggt 113400
ggtgtagtta ctccacatgg ggcaggagaa aacccagcag tggcccagga atgccgggga 113460
atttcccagg agctttgggg cagggataaa ggggttctga aggagtgttt tagggtggcc 113520
agggtgggct cttctggcag acagcgagat gaagggcctg agagcaggaa ctctctctgc 113580
ttttgtctag ggagcagcag gagaaaccgg ccctatgggg gagagaggtc acccaggccc 113640
cccggggccc cctggagagc agggactacc tgggacagct ggaaaagaag gaacaaaggt 113700
cagtgagggg ccaggcagag gggaagtggg ggagctgggg attggggtgc tgaaaggaac 113760
aggtagttgg agatttgagg gggggctgga gagctctctg tttaatttgg gagcatggct 113820
gtggctcatc acctctcctt tccttttagg gtgaccctgg tccccctggg gccccaggga 113880
aggatggtcc tgctggtctg aggggattcc caggagagag aggcctccca ggcactgctg 113940
tgagtgtgac tcccagaccc ctgcctgtca caagcaccaa gcatcctgag tccccacccc 114000
gaccctgtcc ctgaccccct ccatgtcact ccccacttcc atcttttgga gcctgtcttc 114060
cctctccagc tctcaccctt ctcttttcgt gaagtctcat cttccccaaa tgtctggttc 114120
atagggtgga cctggtttga aggggaatga aggtccgtct ggccccccctg gccctgcagt 114180
gagtctgggg ttccggaagt ggtaggaagg acccgggaaa ggggtgggtg aggaagggtg 114240
ggagtggcat tctgatacca ccaggggctg tggggctggg cagaggctgt ccagggacag 114300
ccagaatgca tctgattttg ggagcttttg ggaattgtgg tctgaggttc tctggtatgg 114360
gcgggctggt gtctccttcc tgggggtctg accatctgct ttctcagggc tcccctgggg 114420
aacgaggtgc agcaggatca gggggaccca ttggtccgcc agggcgccca ggcccgcagg 114480
gtccccctgg agcagcagga gagaaaggtg tcccagtgag tgtgggggta ttggggaggg 114540
gtactgggga ggggtctgtg agcctagggt tgatgggcta tgacaagttt cctgtggggt 114600
gtttgagggt gagggtcacc tccaggccat gactccttcc tcctgtccca cagggtgaga 114660
agggccccat tggcccgact ggccgagatg gagtgcaggg tcctgtgggg cttcctggtc 114720
ctgctgggcc tccaggtgtg gctggagagg atggagacaa ggtgagggga ccccacagac 114780
cccgaccaat cttctttcca gatgagatgg ctgacctggg catgacccct ggcccctgtc 114840
atgtcatcct tcccactcta cccatcacct ttctctaata tcttgtctgc cttctcctcc 114900
cagggtgagg tgggggaccc cggacagaag ggcaccaaag ggaacaaggg tgaacatgta 114960

agtgtccatg accttgactt ctgacctcct ggcctttaac ctcagtccca cctgatctct 115020
ctccctgttt tggtgtctct gactctcttc tcttacccag ggccctcctg gacccccctgg 115080
acccattggt cctgtggggc agcctggagc agcggtgagt gacccctcca cccccaccga 115140
aggcccagca gcctctggtc ctccctcagc tcccctctgc ccttgtggct gggagtggga 115200
gcaggggctc tgggcctggc acccagctgt gtgtggtatt tgtggataga gaacacctga 115260
cccatgaccc atccctgtgt ccttgtctct gctttccctc aaccctgcag ggagcagatg 115320
gggagcccgg agctcgggga ccccagggac actttggagc caaaggtgat gaaggaacaa 115380
gaggattcaa tgggccccca ggacccattg gcctacaggt gtgttggggg ataggacagg 115440
ggctgagagg tggggtcagg atggggtgat gttttgcccc agactctgcc agcagcctgc 115500
cgatgcagac tggagggcct gcggagtctg aggggagggg actgtggggc ctatctgcct 115560
agggctgtgc tttgggtggg gtcgtcacct gggcccatgt tttgtatact tgcacagggt 115620
ttgccaggcc cctctgggga gaagggagaa acaggagatg tgggtcctat ggtgagtgtg 115680
acccctactg accctagcca ccatactagt caccccccctc cctggccagc ccccacccca 115740
caccccactg ccaactccct ggcctggctg ctcctcctcc tctctccacc attcatgctc 115800
agccatctca cttcccttcc tcatctgtgg tgtatctgtt tcccccaggg accacctggc 115860
cccccaggac ctcgaggtcc agctggaccc aatggcgctg atgtgagtca tctcagcccc 115920
tgtcccttga gaccaatgtg tctggtcttc tgcctccctt tcccagacca taaactctag 115980
tatcgtctca ggggccaaga gaagccctta ctcccgggag gcctccactg cctcactgct 116040
gtaaccttgg gctgctcatc tgggactttg cccctgaaat ggcacctcca tcctaagcaa 116100
tgacacctat ttctgttcct cttccagggc ccacaaggtc cccccaggagg tgttgggaac 116160
ctgggtcccc ctggagagaa ggtaactggg aagggggtga gtggaccagt catggaagtg 116220
ggggatggga gttggatttc tgccaatttt gggtggggag acaaaagaga atgagatgtg 116280
gggaatatca gaattttttg gctggggaag gagaggaggt ttttgactct aatctctttg 116340
caggggggaac caggagagtc aggatctcca gggatccagg gcgagccagg tgtcaaggtg 116400
agtgacagct ccaaggcctt actccccagt ccccgtcacc cgcctcaacc ctgcctccac 116460
ttttcctgtg acctccttga gctggaactc ctctgcagca agagtctctg ctccctgcca 116520
tcctctatga ggcctgatcc ctggtctgag tgagcccttc ttccccctttc ttgaagggaa 116580
ggggactagg gaactcccct tgtaggcttg actttctgtg tatgtcccat tgtcactgag 116640
gggagtaggg gatggctgaa ggtgtctcgg aagcagaggc tgcatccctg atcttcaaga 116700

ttccctgggc catctgtgtc ctccgcttgt tctgcagggt ccacgcgggg aacgtggaga 116760
gaaaggagag tcggggcagc caggagagcc agggccacca gggcctaaag gccccacagg 116820
cgatgatggc cccaaaggga accctgtgag tttggggcag tgggggctga gtcctctcag 116880
gccctgtgaa tgacagacct gggaatatgg gtgtgtgtga gggaggatct tggagtgtgg 116940
ggagcccggg agtttggggg atgctctggg aatggggcat ccagagggat gcctggggtc 117000
taggatccgg agagaaagtg agagatgccc tgcagttaat accttgagga attagacaac 117060
acgcagtgca cgtggtaggg aaaggggtgc aggagacctc ttcctggggg gtactggtgg 117120
gcactgcctc tagagaggca gtggcgtgtg cctgtgggcg tctgtgctgg gtgggcttag 117180
ggagctgtga ccctgactct tattctccat ctggatcagg gtcctgttgg ttttcctggt 117240
gaccctggcc cccctggaga aggtggccct cgggtgagtc ttactcagag aagggaaggg 117300
gcaaaaaggg tggggcttct atggggggt cctctgtgtg gccgctgggc ttgggtattg 117360
ggaagccggg ggtatggcag ggtgggcaag gggatggggt attgacagtt ttggaggtga 117420
tgccagccag gttgggggcc cacctctgac cttgcttcac ttctgcaggg ccaggatggt 117480
gctaagggtg accgaggcga ggatggtgag ccaggacagc ctgtgagtgc ctggtgaccc 117540
caccaccccc ctgagcccaa gcctcatcct ctttacccct cttctgtgcc ccactcctga 117600
ggggtccctt ggctggagga taaacactca gccaccccaa ttcctctctc cctagggatc 117660
ccctggtccc accggggaga atggaccccc agggccactt ggaaagcgag taagtgaggt 117720
ggacccctga gaccttggga ggcagtccct gggctgtgtg ggtggaggct gggcaatggc 117780
aggtgggatg ggtggggagg tgcctggtgt ctgcattgcc ctgggtgtgt gtgtgtgcag 117840
gagctggtgg gtttaagggc gtgtggtatc tcattgcccg gggcagggtg tgtgtgcagg 117900
agctggtggg tttaagggta tgcggtatct cattgcactg ggcgagtgtg tgtgcaggag 117960
ctggtgggtt gatgggtgtg cggtatctcg ggcatgtttg ttcctgggtt ctggtgtgta 118020
tgttttcacc aggggtagtg gtggttactg acaaagcaga atggaaactg gaggaggggc 118080
tggccagctt ttctgtgggc caggggtgaa ccttttttagt ttctggggca ggagacgggc 118140
caccaggtag ggtgtgggca agtggccctt caccaaatgt acagactacc cagtattttc 118200
acaactgtca cagctgtatc tgttctgcac atctgtgaat cggccctcgg cgcgtgtccc 118260
tgtgtatgca cgtgtgtgtg tgcatgtgta tgtgtgtgtc taggacagga agggggaaga 118320
gttgagcctg gctgcccacg gcctcatgtg ctcttccttc ccactccacc tgcagggtcc 118380

tgctggctcg cctggttccg aggggcgaca aggagggaag ggagccaagg tgagggacag 118440
gctgccctag tgctggagca tcccctccgc ctcccacccc tcagatgcct ccgtctccag 118500
atgcccaccc catctccacc tctcccatgt tgggccctta tggggacagg cgttccctgt 118560
acttgttcct gcgctagggg ctcctagagt ttggcccttc ctccctgcct cctgcctccc 118620
accttggacc ctctgccccc tgcccacacc ccactcctct gccattcagg acacattcct 118680
tgtgtgtgtt tcagggagat cctggcgcta taggtgcccc ggggaagaca ggcccggtgg 118740
gtcctgcagg cccagcaggg aaacctggcc ctgatggtct gagggggctc ccaggctcag 118800
tggtgagtca ctgcagggaa gggctgggct ggggtgggag tgaggggcca tgggaggggt 118860
gcagtgtggg gatgctcctc ctgacccctg tggccccctc aaatcttcag ggtcagcaag 118920
gccgacctgg agctacaggc caggctgggc ccccaggtcc tgtggtgagt gactgggatt 118980
gggctgagtg aggggtgagg gcagtgccct gggacagagc tgaagccctg gaggaagtgg 119040
aggctgttgg ggagaacttg gtcccacctc tcccttaaga gaatgacttc ccatctctcc 119100
acagggaccc ccagggctgc ctggtctccg gggcgatgct ggagccaagg gagagaaggt 119160
gagtgacaga cagacacgtg gccaggtgtc tctcccatca ccctcgcccc tgaaacctgt 119220
gggacccaag tgcccactgc tctgcttcag gcctccggca gccagtccca gggagtccct 119280
gctcagtgag ggccactcac ggaccctgtg cccggctccc tctccatgtc ctcttggccc 119340
ttctccccca tccacatgac tcctctttgc attctcttga ctgcctgcac ccttctctag 119400
ggccacccag gtctcattgg actgattggg cccccgggtg agcagggaga gaagggagat 119460
cggggacttc ctgggcctca gggctcccct gggcagaagg gtgagatggt gagtagaggg 119520
catggtcccg gaagttgtgg gggttgagag tggggtggag aggggtggag ttggagttgg 119580
gactcagctg tgggtaagcg agcaggtgct caggagggtg tgggaggagg acccagttga 119640
accagccact acccttccta gggtatccca ggagcatccg gccccattgg tcctggaggt 119700
ccccccggcc tccccgtgag tactgcctct gttcctccac aaaatcccct aaacccctct 119760
gctgcccacc cacagcctcc caacgacttc catggaacta ccagcctaac aagcatagtc 119820
ctcagggtcc cccatttgtc ctgtcaccac cagtacctcc tccccatact tccagggctc 119880
tgaatgtcct agtattccca caagactgcc cctcctgtag taacccgagg ctcctgtgga 119940
cttgggacct tgcctccccg ctcctctgag tcctgtcctt cccctgcagt gaccgtctct 120000
ttcttgttcc tcatttcaca gggacctgct ggccccaaag gagccaaagg agccacagtg 120060
agtgaccccc ttcagctctg acctttgctc cacccccctct ggacttgatg atgtctctcc 120120

aggacaaata gactcccccc aaggagcccc ttattccagc caggaggctg atttgatctt 120180
tctgtgacct tgatccatgc ttgacctctt gacccctcca tgacctgatt tattccttgt 120240
cagggcccag gcggacccaa gggagagaag ggtgtgcagg gccctccagg acacccggtg 120300
agtgaggagt cagggctgct cccagggccg tacccctctg ctccactgct gccacacttc 120360
accctcacac caaccttgtc tcttgccctc tccatctgtc cctgcaccca gggtccccca 120420
ggcgaggtga tccagccact gcccattcag atgcccaaga agactcggcg ctcggtggat 120480
ggaagccgtc tgatgcagga agatgaggcc ataccgaccg ggggagcccc cggcagtcct 120540
ggggggctgg aggagatctt tggctcactc gactccctgc gggaggagat cgagcagatg 120600
aggcggccaa cagggaccca ggacagccct gctcgcacct gccaggacct gaagctgtgc 120660
cacccagagc ttcccgatgg tcagtgctag cctcagggtg cacaaacatc tccccaacag 120720
catgggtact gaacaagcag aatggatgct gggggagtct tggtgaaggt gttgggttaa 120780
gggtgagggc tgaggggctg ggaactgcag ctatttacta accaggtcag aagtatccca 120840
atactttagc aactgatctg ggtcagtgtg taccagttgg ttatcagccc agtgtctgca 120900
ggagcagata aacatacata cgtgcacaca cctgtttgca cctgaacaga tgaatctgtg 120960
tgagcgcctg caaccacatg ttgtcccagt atgaggctgt ccatactcgg acctcctcca 121020
gtcagagggc ctggggttac tgttgggggc agaggtgtca cgtgatgaaa gtgggctgca 121080
gcagtaggat gctgcagtag aatccagcag tagggctaga ttctagggct ctgggaggga 121140
ggaggaccag gagactaaga gcatgatggg gagggctggg agggaagagg gaatgtgggg 121200
gcaaagagca tcttggagcc tggacctagg tggccctgac ctcccctctc cctcacccaa 121260
caggagagta ctgggtcgac cccaaccagg gctgtgctcg ggatgccttc cgagttttct 121320
gcaacttcac agcagggggt gagacctgtg tgacgcctag ggatgacgtc acgcaggtga 121380
gagctggccc ctcgctgccc ctcccctgcc ccatagtgca ccccctcagg gcttagtggt 121440
ttctgggttt tggtgacagt cccctggctg ttgccccccc cggagcctgc caccccttct 121500
ccacttctta gctctttctt cttttccttt tcaccggtcc tctgatgctc actttgcctt 121560
tcgtgtaccc ccagtctcct ggctcccact cagcccctca cttctttccc agcccttgcc 121620
ttccagccat tccctgctca tgactctggc tcagctgtcc tggtgccgta gatgctcctg 121680
aggcccgtct ctgtctccct ctgaggccag ccctctctct ccccctctga ggccagccct 121740
ctctctcctt ccagttctct tacgtggact cagagggctc cccagtgggt gtggtccagc 121800

tcaccttcct gcggctgctc agcgtctcag cccaccagga cgtctcctac ccctgctctg 121860
gagcagcccg tgacggtccc ctgagactcc gtggggccaa tgaggatgag ctgagcccgg 121920
agactagccc ctatgtcaaa gaattcagag atggctgcca ggtgggaaca ggaagagctg 121980
ggttgggggc tgatctcaga ctcaggctgg aggaaggagg tgggaagacc ccttgggcag 122040
ggcacccagg gggagcaggg aggagtccgt cctgctggat tgtcagggat gcctgaggga 122100
gctcaaaagc cggtgaggaa ggtggaaagg atggaagcca tcgggtgagg ttcacttggg 122160
tacaaacacc agcgtcacac aggttaatgc agacgtgtac acagactggc acatggctgc 122220
ccagcagagg cacacggtgg gggagaagca aacacacacg catgggcaca tagacactgc 122280
cagtctgtac atcctgagtc accctgatgg ggccaaggtg ctcagaggag aggtgagcct 122340
gggcctgagg ttagagggtg gggggtgcct ccatcctgct cacactttct tccttgtctc 122400
cccctgaaga cacagcaagg ccggacggtg ctggaggtgc gaacgcctgt gctggagcag 122460
ctgccagtgc tggatgcctc cttctcagac ctgggagccc caccgaggcg gggaggggtg 122520
ctgctggggc ctgtctgctt catgggatag gaccgtctct gtctgatcct gtccattcgg 122580
aaccaggccc acctggaatc ccacaacatc agctctgtgc cacctcccaa gagggctcct 122640
cactatctag ggagccctgg gccagggcgt ggagagccct cagtcggggc aggccagggg 122700
aggggtgaag tggttgcctg gacaccccac gggaggagtg gcatctgggg ctcttggccc 122760
tcccacctgg agcctgttac ccgttagaga gctgagaccc ttatttaaaa ctcacctccc 122820
aatcacccca aacaaatgga agagaagaga aaggacatgg cgtattttgt atttaaaagt 122880
aattgtatta attatttaaa gtgtggaaag caaaataaca aaaaagagaa acgccaacaa 122940
aaatcagcag atgttgaaga caggggtctc gggggtgggc tccggcaccc acatcctgga 123000
gtcaggactt tcctcagtga ctgtgtgtag gggggtttca gggctgaacc ccacctccct 123060
cccaccttcc tcccacctca cctgtcgcac ccactgtgaa agttggaata tgtggtctcc 123120
ctggcctcag ggctctgact ctgccagggt ggggctctct aacccacagg tgttggctgc 123180
ctggcccatg tgcccactgt ctcttccact tggtctgggt ttggcaggca ctgcctgcta 123240
cttgagggcc aggatgctcc cccagggaag aaacggaata gtgtggggtg tgtgcagggc 123300
tgcatcccgc agatggctgg aatattaaaa ttcttctata ttggctggta aattgccatg 123360
gccctgagcc actgagtatg ttcattgcca cccctgtccc tcccctgggc acccctcact 123420
ttccctgatc ctgcaattaa agggttaatg tgtggcatat ggaagggact cccaggaccc 123480
tgtgcccagc ttccatgctg actgatggtt aaataatgtg attgtctcct cccaggtgtc 123540

tgtgtcactg cttgtgttgt tatttcagtc tcccccgaca cccatctgat gcttcctctt 123600
cccagctaag tggttaccag aattgtatgg cttaatccag ataccctgca agccctgtcc 123660
agctggggtg tcagggccca gagtttacaa agcctgggtg actagcataa aattacaaac 123720
aatgccccag ggacaccagg taggggtttg gggtccctat ggctccagtt tctgacacgg 123780
gtgtgcgatt gcttctgggt gtgggtggga cggtgctaca tagcccagtc taatctctgt 123840
aaatggggag gctgaggccc acttccaaga catttactgc atctgtacct ctggtcacct 123900
tatccctccc catcagcctt gtgctgtgct gtcatgaaga cagggctgtg gccaagccct 123960
ccacactcac gtgtagtcac gtgcatactc gttgagtctt tgtgttgggt ggagtcgcac 124020
agtccctgcc tgtgtccccc cactcttgac tgtcccccac ttccctagcc tccctctcct 124080
gggcactcag aagctctgct ccctccgggt taggtgtcca agaagtcctc tcttccaagg 124140
ctggccgcgc agccgggacc ccctagctgg tgcccatggg gcttcacatt cccaagcagt 124200
attgcctggc tcctgcctgt cagggcgctg cattaaaacg tgttccaggg cctcatccca 124260
atttcctctt cctcaaccct cttcctcgcg ggcgccagaa tcaagagtgc gtgcctttgt 124320
gtcccggttt cgcatccgcg gcctcggggc gggcctggca gggctgccct tcaccccgag 124380
cccgagcccg ggtaggctgc gcccccgccc ctcctccccg tacttcttcc tcccagcgtg 124440
tccccgacag catcggcttg gccgtcccgg tcccaaccca ttctcgatcg caggaagccc 124500
cacgtgggcg tctagacggc cactctggcc cgggcgggtg cgcggaaaaa cttctcgagg 124560
gtggaagcct ctcctcggcg ctggcgcctc cgcctctgcc gccccctagt gcccgcgctg 124620
agaacgcggc cccgcaccgg gtcagctgaa taatcccggt ccctcagacg cctctccctt 124680
gtcacccgct gaaccccctg accccgaagg ccgaatggag gaagaagccc ctgatctcat 124740
ctgtcagggg tccggtggtg gataggtttt ctgggatcac ccatgctctc catctcaata 124800
tccaaggccc aggggcttca gacacccccct tccctccctc tcagggtcac attaagagaa 124860
gatcagatct aaataggtgc cccccatctg tttctcccgc acccactccc agcctagccg 124920
ggctggagtt gagatttctt tggcgctctt tctgcagctg gagccaaacc cgcagaggtc 124980
gccccccgaa cagcaagagg actttcccaa aggtcacgtt tggtcagatg ctctgagttc 125040
cgagggcttt tctgcatgga aacaatgctg tacagtcact cgaataacat ttatatacgg 125100
cggctaagtc cccaggccaa tccacaaaag cctatttaat tacacagtgc tgccaaccaa 125160
ccccatctaa cagtttcacg agggagtctg tgccaagttg ctgttgagga ggccaggccc 125220

acagccttct cttggcagct aacttcttcc tcccctccag aaagctagag ccaggctcta 125280
aggaagggtg ggaggggttt aagggcaggg ctgtagaggc ggggttttgg ggtggggctg 125340
ggtgtgaaca gggcaagtgc ccaggcctct gggcctgtgg gtagtggcag tgtctccatt 125400
ggctggtggg caatggaggc ttctgggtgg ggtgggagga gatagggaag agaaaggcag 125460
aggctttagg gaactacttt gggtttgatt ggggcagaac ataaactctt gttggagggg 125520
ccaaattctg aaaagtattg actgcttatg gaaggagttg aacttgaacc caaaaactaa 125580
gagtcataga tggtgtttga aaaggcaagt gatatgatgg aattgtgcgc tggcatggtg 125640
gcgaaggaga ggagggtttg ggatggggcg cacagggaga tcatgttttt ttttgttttt 125700
tgagacagag tctcgctctg tcgcccaggc tggagtacag tggcgccatc tcggctcact 125760
gcagcccctg cctcccaggt tcaagcaatt ctcctgcctc agcctcccgg atagctggga 125820
ctacaggtgt gcactgccac gcctggctaa tttttgtatt tttaatagag acggggtttc 125880
ctcacgttgg ccagtatggt cttgatctct tgacctcatg atcctcccgc ctcggtctcc 125940
caactgctgg ggttacaggc gtgagccacc gcgtgggaga tcatgtatag agggagggct 126000
gtggggtggg tcctctggcc tgtcacaccc ttggagctgc ttttcagctt tgggcttata 126060
tttttagcat ctgtaactta tggttaaaaa ataacttgtg aaataaaata aaaataactt 126120
acagaccact tatatcactg tacctgggag caccacatat cctagaaggc catgggctac 126180
acagagctgt tcttgccctc ccttggctgt cttgcatgct gaggtttgta gttttgaatg 126240
gaccagggca ggtgggggtg gagatgagac agaggaggga gtgagtcaaa acaaggctct 126300
cagcctgggc aacaaagcaa aactccttct ctctctctct ttctctcttt ctttgagaca 126360
gggtctctgt cactcaggct ggagtggagt ggcaagatca aggttcactg cagcctcgac 126420
ctccgtgggt tccagccatc ctcccacctc agcctcccga gtagctgggt ctccaggcat 126480
gtaccactag tttttgtatt ttttgtagag atgggatttt gccctgtttc ccaggctggt 126540
ctagaactcc tgggctcaag agatctgccc atctcggcct cccaaagtgc tgagattaca 126600
ggtatgagcc atcacgcacg gcccttttttc tctacaaaaa aataatttaa aaattagctg 126660
ggcatggtgg catgtgcctg tagttgcagc tactcaggag actcaggcag gaggttcact 126720
tgagcccagg agtttgaggt tgcagtgagc tatgctcttg ccactgcact ccagcctggg 126780
tgacagagag ataccccatc acaaacagac aaacaaacaa acaaagggct ccagctgcct 126840
cagaaataag tttctgtgta gtagctggtg gccaccttgt tttgtggttg tttagcccat 126900
tttcctgcaa gtgtggtgtg gtcagcaggg agcatcaggg ccacactgga gcttctttct 126960

gtcgagctgt gcagttcaca gactgtacat gtgtaggcca tagctatgtt tctcaactca 127020
aacctagagt aagcagaagt caccttaagt gcttataact ggccttctgt tatcttttct 127080
ttcatcttca gagtagtttc atgggaactg gcactgacag cagactgcca tctcagtagg 127140
gaagacctgt cctgctagtc cttgaataaa gaccaaggat atctacatgg gcttctcctt 127200
tagggctctc cccacacggc caggatggta gaatgaacac agctctcagc ttcagcgaga 127260
gccagcatat caccctgtct ggctttccct cagttggcag gagcaggtca atccattttg 127320
tgtacttcgc tctggcctct ctttttttgt ttgtttttg tttttgagat agggtctcac 127380
tccccttgcc taggctagca tgcagtggtg caatcatggc tcactatagg cttgacttct 127440
tatgctcagg tgattcttcc ccctcagcct cctgagtagc tgggactaca ggcgagtgcc 127500
accatgccca gctaattttt tgtattttta gtagagacgt ggtttctcca tgttgccgag 127560
gctggtctca aactcctggg ctcaagagat ccccctgcct cagcctccta aagtgctggg 127620
attatctggc ctctcttttt tttttttgag atggagtctc gctgtgtcgc ccaggctgga 127680
gtgcagtggc acaatctcgg ctcactgcaa cctccgcctc ccgggttcaa gcgattctcc 127740
tgcctcagat tcctgagaag ctgggactac aggtgcccgc caccacgccc ggctattttt 127800
ttttattttt agtagagacg gggtttcacc gtgttagcca ggatggtttc gatctcctga 127860
cctcgtgatc cacccgcctc ggcctcccaa agtgctggga ttacaggcgt aagccaccgc 127920
gcctggccta tctggcctct ctttctatta aaaacgcctt actctaggtt gaaggacttt 127980
tggttcctga ggcatggatg atttcccaaa gtacaacaga taaaagccac actggctgta 128040
cagcagctca tgtaaaggtc atgttccagg tcttggccac ttcacctata acaggcttgg 128100
actccggcag agcctcaggc caaagaatgt gggtcataga gatatcttcc tcattgtgtt 128160
cacatctccc ccgggcctgg gctatattgt tgggtcaggt aatcggcctg ctctcctcat 128220
gaaataaggc atactggtat tactaatgct gggagagatt tcttataatt ccatcccttc 128280
ccagttgtgg ttatttgtat taatacctgc aggttttcct tacttttaga actgcaaatc 128340
tatagaaaca aacaaacccc aaacccaaca aacatgtttc agagacatta gggttaggga 128400
gaagatgaga aatagcaccc tgtttgattg gggccctggg gagagaggga ctcagacaaa 128460
agagtttgag tggcaggtgg tctagttttg ggtgggttga gcagacttag ggccaaaggt 128520
ggcactgatt ctagctggca gcgggcctct ctgtatttcc aactgagagt tctccttccc 128580
ttagcttgtc agggatgctt tgcttctgag ttgcacactt aacctcagca tcagtttctt 128640

cacctgcaaa gtgggataat acttgctctg taaaggaata attccccctt tactgacagc 128700
ctttgcaatg gaaagtgagg ggagggacct gacaagctgt cttggttcca tctccatgtc 128760
ttggttattg ctgacacacg ggtgtctggg gcatgctgct gtcccttttc cacttttgat 128820
tttagtgctg ttaaaactct atacttacta ttattatttt cttgttacaa atcgcaaaat 128880
ctgattcaga atagcttaag ctccaaaagg aattgattgt aaaaatattg ggtttctcag 128940
gatttgaaga cagagttgac tcaataggga agactgagag cagttctggg aacctcaggg 129000
gctcatggac tttgtccctg gagtgtggat actcaccgct cagcagagct gctcttgacc 129060
tctgtgtctc tgggccccat ggttcaaagt catggagaga caatctgatt ggcttagctt 129120
gggttatctc tgtccctgag aaccaatttt ctgtggctga gggcccagtg taccaaccaa 129180
gaggtatgta ggatgccttt tggtggaggt ggaggggtgt tgggggcact gtttccagat 129240
aatgtgggat cctgaggccc ccaggaggtg tccgcttgct ttcccttctt cgcttctgtc 129300
taaacagact cctttgtatg catttgacca gcgtgataca accagcccct tcatgatcac 129360
atttgctctc cctgccaccc aatctcctgg atgggcaacc caaagtcaca ttcagcagct 129420
gtggccatga cctctctaac ctcagaggcc gacatctctg ccagttcata ttttgccact 129480
ttgtgattca gcacataaac tttcatggcc gttatatcca gggctgcatt gtgacttctg 129540
cgggccctat gcacttttct aaacaataaa tttgaaatca tacttatgat tgtgttgata 129600
cacaggcaac tatattaata ttatatatta aaatattttc ttcaacctta attttttttc 129660
tattttaaaa gaaatgtaaa cattgccatg ggagtattgt gggccctagg cactgtggct 129720
actgtacata atggatccat gggccctcct tgtgtctttg ttaaagattt tactggttta 129780
ctcttaaaat gtaatcacaa ttctgttacc acaattttaa taatcattcc tgttgtcatc 129840
aaatatccag tcagtgttca gatttccaag attgttttgt aggttttgtg tgtgtgtgtg 129900
tgtgtgtgtg tgtttgactc atgatccaaa taaagctgat ggtcttctaa ttctttaaag 129960
atctacattt tccgtaattt atttaaagtc ctttttgttt acttttatta tggacaaatt 130020
taagcatata caaaagtggt ttattattgc tttttttttt tttgagatgg tcttgctctg 130080
tcgcccaggc tggagcacag tggcacgatc tcaactcact gcagcaacat ccgcttccca 130140
ggctcaagcg atgctccagc ctcagcctcc caagtagctg ggactacagg cgccagccac 130200
catgcccggc taatttttgt atttttgta gagacagggt tttgccatct tgtccaggct 130260
ggtctccaac tcctgagctc aagcaatctg ctcatcttgg cctcccaaag tgttggaatt 130320
acaggtgtga cccactgagc cttgcctgtt attgctatct taaaaagaat taatacatgt 130380

taaaaatctt tttcagttct aattttgaat acagtaaata tcaataacaa tattgtaagt 130440
aaagcttttc agtgttttca gtaatttaag gggtttatac tcttaaaaca ttgagagatt 130500
tttttcaaat tgagatttaa aaaaattggg aatcactaat ctagaagaac tgttaaaaat 130560
agtcatgccc acctgtgctc acccttagag atctggattc aacccatttg gggcgtgagc 130620
aaagggactt gtgtaaaatt tccccaggca attccaaagt gcaggctgtg ttgagaacct 130680
ctgctctaaa ctggagagat cccgtaagaa ataagcagcc aggtgcagtg gctcatgcct 130740
gtaatcccag cactttgggg gaccaaggca ggtggatcac ttgaggtcag gagttcgaga 130800
tcagcctgtt caacatggtg taaccttgtc tctactaaaa atacaaaaaa ttcgccaggt 130860
gtggtggcac gtgtctgtaa tcccagctac tggggaggct gaggcaggag aatcacttga 130920
accagggaag cagaggttgc agtgaaccga gatcatgcca ctgcactcca gtctggtaga 130980
cagaatgaga ccctgtctca aaaaaaaaag aaaagaaaag aaaagaaaaa aaaggaaata 131040
agcaaagatg gccgggtgtg gtgcctcaca cctgtaatac cagcactttg agaggctgag 131100
gcgggcggat cacctgaggt taggagttcg agaccagcct gaccaacatg gagaagcccc 131160
atctctacta aaaatacaaa attagtcggg catggtggca catgcctgta atcccagcta 131220
cttaggaggc tgaggcagga gaatcgcttg aacttgggag gcagaggttg cggtgagcca 131280
agatcacggc attgcactcc agcctgggca acaagagtga aactctgtca aaaaaaaaaa 131340
aaaaaaaaaa aaaaaaaaag aaataagcaa aggcacagag tgggaaaggc aaccaaatgc 131400
cctaggtctg aaagggcctc ctgggacctg ctgcatccag cagaaatgct tgcaagtgca 131460
ggcccagaca acccaaaaaa caggggctaa aataaacagg aattcatttt tctcataaga 131520
aatctgagga tgacagcttc cagcattggt ttgagcattt aatgatgcca tcagggttcc 131580
agactcttca tatgtttaca ctccaccctc ctacaatgtt acccttacat attttctttg 131640
gctaaactat aagcacgttt ttcataatag ctgttccatt tacatcactg tcaaacatga 131700
cattcaccaa taaagaaaag acgcatgaga cgtgttcggc accccttagc tgtagaaatg 131760
cctgccacca ctttgctgtc atgaaagagc cattttatgg ctattgcaaa tgctttgaag 131820
atactaagac ttgtacctgg aagctataga tgcagtgtgg gaaactggga agtataattg 131880
ccactgaatt gttattgatt tattactgca aataaatcta tgcaggtccc ttctgaaatt 131940
gagatggtcc tgaactgaac aaatgtctta ttgacattca taacagtggc tggtattcct 132000
tctttatcct gtttgtcagt ttttggaatt cactgattag ataaataaaa agaatctcac 132060

ttgctcctta ctaatgagct ccacggaggg gcatgatgtt ggaaaaggca ccatgcaagg 132120
catgattact aagaagacaa agggcttgtc ggggagctgt tagggagggt cctgagccag 132180
ggcttgggaa aaacatctca tcattttact gtatctgatg cttggaagtg acatgtgctt 132240
tgcaaactcc agtttatgtt ttgatgactt tgtgcttgtt ctgcttggtt aggattagct 132300
tcagctgtga gaaacagaaa accctggaaa caacagtgtc tccaccaaga tagaagttca 132360
tttctctctc acatgcaacc ctagggctgg tgtagtgttt aagtgtcaga gactcaggtt 132420
ccttctatct atctttttct tccaccagcc tcaacatagc tcttctctct caggtccaat 132480
atgactgctt gaattccagc tttcatgttt gtgttccagt cagctgaagg agaaagggga 132540
aataaggaca acccctcttt tttttcgttt tttctttttt tttttttttt gagagggagt 132600
cttgctctgt cacccaggct ggagtgcagt agtggatctc agctcactgc aacctccgct 132660
ccccaggttc tagcgattct cctgcctcag cctcctgagt agctgggatt acaggcatgt 132720
gccaccatgc ctggctaatt tttgtatttt tagtagagat ggagtttcac cacgttggtc 132780
aggctggtct caaactcctg acctcaagtg atccacccgc cttggccaat acctttaggc 132840
aaagcattat agcaggagtc taaaaacaaa tgggctgttt tgttttgctc ggaaatctct 132900
ccccgaggga ttgttttgtt ttttgaagta tttgaaggct aagtatctag agcttgtgct 132960
catggagtag ctgccccctt tcctcgtgag gccaccatta gttacctctg catatatgtc 133020
tgcctctgag gtgtctggct gtctgaggag gcctctggct ttgttagtac accactagga 133080
tatttgcaac gtggatcaag actttttaat ttaatttaat tttattttat ttttttgaga 133140
tggagtcttt ctctgtcgcc caggttggag tgcagtggca caatctcagc tcactgcaac 133200
ctctgcctcc caggttcaag ccattcttct gcctcagcct cccaagtagc taggattaca 133260
ggtgcctgcc accatgcctg ttgcgggaag tcagggaccc caaatggagg gaccagctgg 133320
agccgaggca gaggaacata aattatgaag atttcatttt aatatggaca tttatcactt 133380
ccctaataat gctcttataa tttcttacgc ctgtctttac ttcaatctct gaacataaat 133440
tgtgaagatt tcatggacat ttatcagttc ccaagtaata ctcttataat ttcttatgcc 133500
tgtcttactt taatctctta atcctgttat cttcacaagc tgaggatgta cgtcacctca 133560
gggccactat tgtacaaatt gactgtaaaa catgtgtgtt tgaacaatat gaaatcagtg 133620
caccttgaaa atgaacagaa taacagcgat tttagggaac aaggaaagac aaccataagg 133680
tctgactgcc tgtggggttg ggctaaaaga gccatatttt tcttcttgca gagagcctat 133740
aaacagacgt gcaagtagga gagatatcgc taaattcttt tcctagcaag gaatattgat 133800

aattaatact ctgggagaat aattgcattc ctgggggga ggtctataaa cagccgctct 133860
gggagtgtct gtcttatgcg gttgagataa ggactgaaat acgccctggt ctcctgcagt 133920
accctcaggc tcactagggt ggggaaaaac cccaccctgg tgaatttgag gtcagaccgg 133980
ttctctgctc tcgaaccctg ttttctattg tttaagatgt ttatcaagac aatatgtgca 134040
cagctgaaca tagaccctca tcagtaattc taattttgcc ctttgccttg tgatctttgc 134100
tttgcctttt gccttgtgat ctttattgcc ctttgaagca tgtgatcttt gtgacctact 134160
ccctgtttgt acaccccctc cccttttgaa gtccttaata aaaagctgct ggttttgtgg 134220
ctcaggtgga catcatggac ctactgatat gtgatgtcac tcctggcggc ccagctgtaa 134280
aattcctctc tttgtactct ttctctttat ttctcagact ggccgacact tagggaaaat 134340
agaaagaacc tgcattgaaa tattaggggt gggttcccca gatacatgcc cagctaattt 134400
ttttgtgttt ttagtagaga cagggttttg ccacgttggc caggctggtc tcgaattcct 134460
gacctcaggt gatccacccg cctcagcatc ccaaaatgct gggattacag gcattagcca 134520
ctgcacctgg ccatctcaag atggttttaa tggttagaaa tatcagccaa ttgaattttc 134580
agagacatat gccacctaat tttgtattca atacttaagt cattaaactc cagtggtcta 134640
tatacctgtt tggcctctaa gacagtttgg cctctaagtc tgtgagcact ttctgtgatg 134700
atggaaatgt tctacattta tgcaatttaa tgtggtggcc actattcaca tgtggctatt 134760
gagcacttga aacatggcta gtgcactaag aaactcaatt tgaaattcaa ttttaatttt 134820
atttaattta aatggccaca ggtagctagc tagtgctacc atattagaca gcacagctct 134880
gaggctcttt taaggaccca cataattcaa tgaagagacc caagtttaat acagatgtgg 134940
gatatgcagt cttcataatt caaacacgcc aattaatttc tgttcctctt tttcaagttg 135000
ggggctttca agcacaaagg cttttacctg gtcttgggca gaatttttct ccccaaaatt 135060
ttaccaaaat ttgtgctctt ttatagtctg tttcttttaa ctcataaaat taataatgga 135120
agacagaggt ttttgatgca gttgctttaa aaaaaaccag gttcaggcca ggcacagtgg 135180
ctcacacttg taatcccagc actttgggag gctgaggtgg gcggatcttc tgaggtcagg 135240
agtttgagac cagcctggcc agcatggtga aaccccgtct ctactaaaaa tacaaaaatt 135300
agctggatat ggtggtgcgc atctataatc ccagctactt ggggctaagg caggagaatt 135360
gcttgaacct gggaggggga ggttgcagtg agctaagatt gcgccactgc actccagcct 135420
gggtaacaga gtgagactcc atctcaaaaa taaataaata aataaataaa taaacaaata 135480

aataaataaa taaaatcagg tttactgaga tataatttaa atacagtgaa accatagctg 135540
tacagttgtt tatgagtttg acaaacatat tcattgtgta actactacta caatgaagat 135600
aatatttcta ttaccccaaa aagtttcctc atggacacag ctttttttcaa gtcactcctg 135660
gttaatgtgt cattaggata ataaaacaat gttaattctt gaattggatt taggctgttt 135720
aaattttgct caatggattg tacctcggag gtgggagaat ttactctggc aacttagtaa 135780
aggttggggc tgccaagtga aggcagactg ttggtgaata taatttattg ggattgaaaa 135840
ggatacatca gccagattga tgcatgaatg ctaatcaagg gttgatttag caagatcttc 135900
tactaaggaa ataatatcat gtacagtgct aatcacattt ttttcataat tgagttaaat 135960
atttaaagtg attagaatag tcactgccac ttaatactac ttcagaatta gctgcttttc 136020
ttaatatcgg tgtaattcac aattcacttc caagctccac ctccctttttg actggccaaa 136080
atgagttgtt aaagagtcct actctttatc tcagatttct ccaacaatgt tatcataatt 136140
gttattttac atattctcct agggatttgt cactatttca tattacgttg ttttgtgggg 136200
aagtggaatt ctagaggttc ttaagagctt ttccagctcc cacagttgca atcgtaagtt 136260
cagataacat gcccaggggc aggagaaatc accatagaag ctttcttggt tctctgtatc 136320
tcccattaat gtttcataac cttccacctt catactttcc cctccacata ccctggtatt 136380
caaaatagcc cttcttatta gattgggttt tcctaattaa aagttgccat tgatgttata 136440
tcccaaagtt tctggaattc ctaaacattg tcctttgtct aaactgtttt aacgtgagtg 136500
cagagtgtct ccagacacag gttggaacag atctggaggg aagaacattg gggctgtttg 136560
tcccatcgta tgagaggatc tggttttagg gcaaagctct ttaagcttgg agaatgaatc 136620
gaaggtgtgg aagtgccctg tttctgtatg ttctaagaga aatgagctct taacgtttat 136680
aagctttttg tcttggcatt ccccttcggt ttggtcccta gcactgttaa attacatgaa 136740
ttgagggttt gccaaaacta ctgctaaatt tgcagcttct gtatctgcta aaattggact 136800
atttttgata cttcctttaa cctgttttgt aaccttgtta attttctaat gagcccattg 136860
tgctgttggg attccgatat ccccaaatct aatgagccat gaccatacac gttagctcca 136920
ttcccactgg gttctcaaat tctttttcat ttttccaatt ctttagcact ttctcttata 136980
ggcggttctg ccttaatttg aaattaaatg agaacttcag ccccttcgtc tcaccagctg 137040
ctagcatttt aagtgctcaa gagccacctg tggctagtgg cggctcggtt ggatagatac 137100
agaacatttc cctaacagca gaacattgta taggacagca ctgctcaagg gattgctctc 137160
cgatgatcac acccacttta cagatactag gactgtctaa ctatgtacac tttctttctt 137220

gcaaatacga tgtcgaaggc tttccctcag tttgtaactt ttaacaaata ttttagaatc 137280
tttattgatc aatctctcat acatctgtgc aacaaaaata tatgtattca tttaattctt 137340
taattgacaa aaattgaatc tgtttatggt atacaatatg atgtttatat atatatatat 137400
atatatatat atacacacac acacacattg tttaatggct aaatcaagct aattaatatg 137460
tgcattacct tacatatttt ttgtgttggg tgagaacact taaaattatt cacttagcaa 137520
tattcaagta cacagtacat tgttattaac tagagtcacc atgttgtaca gtacatatct 137580
tgaatttatt cctcctgtct aactgaaatt ttgtattcgc tgatgttagt ttttacaatc 137640
aactcattaa aaacataatt aggtcacaga ggttaggagg ttttgaaaaa aacaaaaaac 137700
caaacaaaaa atgtaattag gtttgtttgg caatctagca atgcttagtg tgcaatgctt 137760
tttacagccg ctgctaaaag catagcaaaa ccacagaaat ctatgtgctg ccaaaaccta 137820
tgtatttggt cgtatttatt ttgacttgtg ttttttgtgt gcttaacttt ttctctaact 137880
tttttttgta aagaatgctg gagtattccc aaattgttga caaattccct tgtttcctgt 137940
tctaagccca gatttcagtg ttggccacta ggtggggata ggatcctatt tctctatagc 138000
cttaacatga ttttgaattt cgatgctttg caatggataa tctggctttt tcctcgaaag 138060
ctttaaaaca tttcttttgt gaattcaaaa caacccacag ttttaattct ctgtagttat 138120
ccaataaaaa ggaataggaa acaagtgcta aattatttac ttacagtgtt tctgtatgga 138180
aatccttacc cccaagacag ctggcttcac ggcttgactc aatggtgaga aatctgagtt 138240
gactcacagc tttctgacag tattctgatg gtaccatgtt aggataaatt cttggccaaa 138300
cagaaaatca agggagacct gcttctcctt gtcaaactct aaattaatct actagttctt 138360
ttcataaagc aaaaggtgaa atgttgagga ctctgagttg gcgatcaatc gaaatgactt 138420
ttaaactaag tccctttttaa tgatgactga agacaatatt acattaatca ctgggtatta 138480
taaacactgg tttctcagtc ctctgctgat gagatttgct tgtttgtgta tttctttctt 138540
aaaatcccaa atagtgaagt ctctcttaag caatggaggc ttattcatag tcaaattcct 138600
tgcatgtaaa attctctggg tataaagtag attgcctccc tcttgttttt ctttttcttg 138660
agacagggtc tcactctgtt gtctaggctg gagtgcagtg gcattatcac ggctcactga 138720
agcctttacc tcccaggctc aagtgttcct cctgcctcag cttcctcagt acctgggact 138780
acatttattt tttgtttttt tttatttttt tgtagagaca ggattttgcc atgttgccca 138840
tgctggtcaa gctatctgcc ctccttggcc tcccaaagtg ctgggattat aggcatgagc 138900

caccaggccc agcctgcttt cttctgtttt accaagatgc ttgacagtgg atatataaaa 138960
tgacaacttc cctttctcat atgtggcact gagagatggg cagcctctat ttcctacaag 139020
aaatatagaa tagaaaatgt gtaatttctc atggtgttct gcaaaaatta aaaatttcac 139080
agaacactca tctgaatccc tggtttgtaa ctttggcttt ccaaaacttg cctatatgct 139140
aatatgattc ataaaacatt tgtaataata atttcaaaat aaaaatttta aaatgaattt 139200
agtcctatat cttctataat aaccaatatt tccagaaaag taggatttaa gagtatcttc 139260
tgtaccaata tatgactttc tttttctttt ttttggagac agagtctcac tctgttgccc 139320
aggctgaagt gcagtggtgt gatttcagct cactgcatca ttcagtcggc cgaaggcctg 139380
gctagaacaa aaaggcagaa aaaaggtgaa ttcactctct ctctgttttg gagtagggag 139440
tcctatattc tcctcccctt ggacatcaga tctccaggtt ctctggcctt cacactcagg 139500
gacttgcacc agcagccccc caggtttttg gtcttggact cagagttact ccaccagctc 139560
ccccggttct cagcccttca gacttggact gagccatgct acaggctccc ctgtctcagc 139620
agcttgcaga aggcctaacg tggaactcct cagcctccat agttgaggga gtgaatcccc 139680
agcataaatc cccactcatc tatctaccta tatatatcca ataaattctg tttctctgaa 139740
gaagtctgac ttcttcagcc cccaaaggcc cccaaaggcc cccaaagccc ccaaaggccc 139800
tagtgctggt ctgttacgaa cttcctggta gacaatattt tacacatggc atcccagctc 139860
cttgtgggag gaattaagtg tgtcctgtgt ggctccaggg ggacaggatc cttggaagcc 139920
tgagcctggt ttctcttagg ctttgcccag tgtgcctttc cctctgctga gtttgctttg 139980
catcctttta ctgtaatgaa tcagagccct gagtaggacc atatgatgag ccctgtgagt 140040
cctcctaggg catcatctca cctggggtgg ctctgaggac ccctaacata gctcttcctc 140100
tggcattggt atctggtaat atataaaatc ccccaaaact aacttgtcat cataatttat 140160
gaccatattt catcaaaatt tcatgtagtc acatccaaga agttcagtta agaaagaaaa 140220
actctttaag cttggttaag gctctgccat ttttgaaaat tcgtattaat ctaataatat 140280
tgtagcaact ataagttttc ctgataactt tttttttttt tttgggagtg ggggatggag 140340
ttttgctctt gttgcccagg ctggagtgca atggtgtggt ctcagctcac tgcaacctcc 140400
gcctcctggg ttcaagcgat tctcctgcct cagcctccca agtagttggg attacaggcg 140460
cccaccacca cacccagcta atttttgtat ttttagtaga gacaggattt caccatattg 140520
gtcaggctgg tcttgaactc ccgacctcag gtgatctgcc tgccttggcc tcccaaagtg 140580
ctgggattac aggcgtgagc cactgtgccc agcctcctga ttactattat ataaaatttg 140640

gacttagtgg cacatcataa catgaaatga ctggagacaa tatgacttct ctctcggtat 140700
taaacatcaa ttaagatgaa ttacaaaaat catagtaaac tgtattctaa aaccaagcct 140760
atgtctgaaa aatctggtat acacacatct tttgaaacta aggatgtgtt ttcactgtac 140820
aacctgcaag aggtgatctg gtgaacatag agttatggaa taagtgtgta tctataactt 140880
agtacaaatt atatataaag agaacgataa tgtattatgc ggctgggcat ggtggctcac 140940
ttctgcaatc ccagcatttt gggaggctga ggtgggtgga ttacttgagc tcaggagtcc 141000
aagaccagcc tggccaacat ggtaaaacct catctctacc aaaaacaaaa aattagctct 141060
caggtactct agagactgag atgggaggat cgcctgaacc ctggaggcag aggttgtagt 141120
aagctgagat catgccattg catttcagcc tgggtgatag agtgagactc catctctaaa 141180
taaataaaat aaaataatat attatgctat tatacctatt atttaaaatt gggatcttat 141240
gttgatttct acatttggga agcatattcc taagaactga cctttatcag tcctgtattg 141300
tgttcagtgg caaagccagg cttggtgtct gttccatctg ggaacgccag gagtaatggt 141360
cccactcctt gctgctgagg gtacagcatg aatttgggca gcaccatgaa ataaagcaaa 141420
tgtaagaaga tcctctatca gttgcactcc tatttaataa gaagacttga tcatcatcaa 141480
gaagttaatt tctccatgcc tgggtaaaac cacactgact catcctgagg ctcttcattg 141540
gacccagtga tcaccaaatc cttcaagcat aaaatggcca tgtgattatc tccttcctct 141600
caggatgaaa ggcagacaat aacatgtgat ttatgttcct caggaaagga catgctataa 141660
actagagaac ttgggaaaga ggaagtacat gaattaacat gcaggcacat ggtggcatgt 141720
gcctgtggtg ccagctactg ggcaggctga gtggcagcat tgtttgagcc caggaggtgc 141780
aggctatggt gaactgagat cgtactactg cactccagcc tggttaacag aatgagaccc 141840
tgtctgtctt tctctctctc tctctctctc tcacacacac acacacacac acacacacac 141900
acacacacag aaaataaagg agaaaaagaa attgttctta aacataaaat ttaaagtggg 141960
gttctttgta tcaccacatt cttttttttt tttttttttt gagacggagt tttgctcttg 142020
ttgcccaggc tggactgcaa tggtgtgatc tcggctcact gcaacctcca cctcctgggt 142080
tcaagtaatt ctcctgcctc agcccctcga gtatctggga ttacaggcac gtgccaccat 142140
gcccagctaa tttttgtatt tttagtagag atggggtttc accatgttgg ccaggctggt 142200
ctctaactac tgacctcaga tgatccaccc gcctcagcct cccaaagtgc tgggattaca 142260
ggcatgagcc accatgccca gctgactctt ttaaatttca ttcagctgtg cgcccagcct 142320

caccacattt ttatatgaag aagaaataaa ataataatta ctaattaaat aattaaatat 142380
tatttccttc tccttatatg tttgaaaaca tcaaagattt tctccaaaat tatcatagct 142440
ataaaagagt caatataata tcaaatggta acaaattaat tcctttatga tgcaaagaat 142500
caatgtaaaa ttgtgaagaa atatcatcca ttcacagaag taactacaaa taaaaaatcc 142560
caacttgcgt aatgtatttc ctcattaaga agaaggcgat tatgatgtcc taattttcgt 142620
tttcttcact tacccctaca ctctgcagta gtcaggggct tcctgggcca tgctggtgcc 142680
tgacaattct cccacttcct cccagcagct ctagcatctt ccttccccag ccacagaggc 142740
gtcccttctg atgtattctg gttggttgag ccccatcagg ctgtttcatc tccagccttt 142800
ctccctggac aggggtaggg aaaggagtgc tggattcgga gctcctccgt ccctgcgccg 142860
cctcattttc tccttggggt ctggcgcaca gtggccattg caatacaccc taaagactga 142920
aggagccagc gcagagccag gatcctcctg tgagccatgg ccctggctgc cctgcttagc 142980
ctctcggaac ctgggccatc aggggtgagt gcgcaggtgg gcgagggagg gtgggggctc 143040
tgggcgcttc tggagcccac agatgctgtt ttctttcctg ttctgttttc tctgttcttt 143100
actgttttgc ctgaggctct cttttgcttt ctctcattct aacaataata accaccattt 143160
actgaacacc ccctaagtgc caaacctcat gtccttcctc acaacaccat tttcagaaag 143220
gcttttatta acacaatttt agaaatgaaa agactgagac tcaaagagat tcataaatgt 143280
ctcctttccc ccacgtcctc tcgctgaaga gagaagagcc agggcttggc aacctcctct 143340
ggagattctc tcgtagattg tagaggtcag cttcctcaaa taggagtctg caatgtgggt 143400
ttttttcctt ccttccttcc ttccttttct ccctccccct cctcctcttc ttcttctttt 143460
tcttcttctt cttttctctc tctctctcct ctctctctct cgacagggtc ctgctctgtt 143520
gcccaggctg gagtgcaatc acgtgatcac agttcactgc aaccttgaac tcccaggctc 143580
aagggatcct cacacctcag cctcctgggt aactgggact acaggtgtct gtcactacac 143640
ctggataatt atatatatat atatatatat atatatatat ttttcccctt agagatggag 143700
tctcactgta tttcctaggc tggtctcaaa ctcctggcct caagtgatcc tcctgttcag 143760
cctctcacag cactaagatt acaggtgtgg ggcaccacac ccagcccgtt agtgactctc 143820
tctcatttca ttcagtcttc aacccactgc attctccctt tttcttcccc atgtctctga 143880
tgctgctctc agggtgacct ttatacaacc caaaccagtg atcacttctc tgtctctatc 143940
tcttgtgtgt catgagtgtg tttgtgatgc gtccgctcct ctttcctgga tgttttctcc 144000
tgtctgttgt cttttatgaa cccacttcct ctttgttttc ctcctacttt ctagtcttct 144060

cctttcagc ctcttttttc atgtctgtgt ttgtctggat gcaggagcac acactcagac 144120
ccttgctatt ctgactttat gtacttttct tgagtgacct gatccaaatt tagaacttca 144180
cttataatga gcttccttcc agtctgtgtc actaacctag tgtaccctgg gacccaacca 144240
tttagctcac tatcctcaga ttaactccat ttgttcccac agaaattcaa actaagcatg 144300
ttccaaacag aacatcttac cttctctcga ttatttcatt tttcattacc tttgcagtca 144360
ctgatgcccc agttctccac gttgggtatt ctggactcct tcatatccct acttctggca 144420
catgatttag tgattcctgt ggcttttcgt ttcattattg ccattcaaac ccatccgctg 144480
tccgcctcac cccatgttcc tgcccctgcc tacaccctca tgacttccca tctgatggct 144540
gaagagtcct attggtcttg ctttcaaacc agcctcctcc aacccatcct ccctactgct 144600
gccccctcca aacacaaatc tcatcatgtt actgtcctgc ttaagatgct tcacaacttc 144660
ctatgtcctg tgggataaaa tgcaaacacc tgccatcagc acacaaggca ggttacagtg 144720
ccgcccggcg cacatctccc ctggcttcag tcacactgaa gaactgcagt tctccttgca 144780
acttcatgcc tctctgctcc tgagcctgct tctcctgctg gacatgtcat ttccccttc 144840
tctgttttgc taacctcagg taaacactat ccctaggaac ctttcctaaa tctcattttt 144900
caaaaatgcc ctctttatgc tcctgagtgg ctccagtgta tatcttatgg tactcagtgg 144960
tgatcaaaat aacctatgga aataaaagat taaaaaacac aaaataaaat acacatgtag 145020
caatcttctt ttctttgtct ctttggattt tggatttcaa gcagcttgag gacaaggccc 145080
aggtcttctg agttacatcc ttagtgccta aagagtgaaa attcacaatg agtgtttatt 145140
taacagaatg aaaattaaca tttgaaggtg gaatttgctt taaaatgcac ggacctttag 145200
ttttaggctt tgtgaatata ataaaaacaa agtaacctaa ggtgaacaaa cgacatctct 145260
gtagcacagc ttgtatatca tgtttttcat ctgtttttc tttgggcttc gtttctgtag 145320
gtgagtaatt ctctagccca taaactcagt ggaggaccct caacatccgt gtgtgacact 145380
gaacttgccg tcacttgacg catccaagga gaggactggt tttccccgtg ttgggcttgg 145440
aggggaaatc cttgcttagg accaacaacc atgcagtgtc atttcgcctc acttcctctt 145500
ccaggctgga ctccatgctc tctcatcttt attcttttac tgtaatagta atagactttt 145560
ttgttgtcaa ccttttgctg cgtcttatac atcaggtaaa gctaagatac tatcccatag 145620
gtgtttcaaa tgcaacaact tttatttcca ggtagaacta cacttgaaaa tatacattca 145680
acaagaacat tttgtgaatg ttatgtgcat gacaacattt ttaaatttac tatgacttaa 145740

aatgaaagtg ttttgataaa attatataag gtaccatttt tattctaaaa gtaagaacat 145800
aacttgaagt ctataaatag cactaagact tactctggag gtcttttcat gtccttcatt 145860
atttttttcc ctgaaagatt tttatttcat ctccctactt ccctgccttt ctctgtggct 145920
atatcagtat tttttatgtc tggtcctgta ggggttctga gatcctatgg gaatggggta 145980
gggggactac gcaattttgt ttctaccagg agttgtgtga cttgggtgag ttactttact 146040
tctgtggtca gttccccaat atgtaaaata atttcctctg ttaaatcaag tttagcctaa 146100
agttgcctcc ttacatattt taagtttggc ctaaaggttt ctctgtacat catgaactat 146160
aacaagtgga ggtgtaaaca gaccatagcc tacacttgtg ccaatcactg agttttggcc 146220
aatcatatgt agctaactgt ttgaactgtg ttcaagttag gcaaacattg agctgtaacc 146280
aatctggctg tttctgtacc tcacttctgt tttctgtatg ttacttttct ttttctgtcc 146340
ataaactttc ttccaccacg cagctgtgct ggagactttg agcatactct ggcttgggag 146400
gctgcccgat ttgtgaattg tttattgcgc aattaacctc atttaaaatt tattcggctg 146460
aagtttttct tttatcaggt ggtgtcagaa gtggggttgg aagtagagct tctaatgacc 146520
cccagaagtg ctgagtgacc tagcgaggta ccctccaggc ccattgtgtc tctttctctc 146580
tcggagcagc tggggatcgt ggtaagttct cttgcagatt ccgaagctcc acagatttgt 146640
gtgttgagct ctctgagttt ctttgagtaa atttctgatc ctgattgggt ttgaaagtcg 146700
cgaaagaaat tgaactgagt ccaggatcgg attggctcca gtaattaact ggcttggatc 146760
cggttagaga cctcttacat ctaactggat cagaaagaaa ccagtattaa atggcaatat 146820
tgcaaaggtg taaaatttgg cttttggaaa ttcacagaga tttttatgtt ctactccttt 146880
atttttcttg tgcacttagg taggaaaaaa aatcattggc taagttgaac aagggacctg 146940
agagcaaagc cagtatttga ggtaaaaaat gtgatactga atttctgaag aactgagttc 147000
cttctggatt atacatgcat aagtattagg ccctggaagg agcaacgcct tacagaaatt 147060
gtccaggcgt ggtggctcac acctgtaatc ccagcacttt gggaggccaa ggtgggtaga 147120
ttgcttgagg ctaggagttt gagaccaggc tggccaacac tgtgaaaccc tgtctgtact 147180
aaaaattaaa aaaaaaaatt agccaggcat ggtggcatgt gcctgtaatt ccagctactc 147240
gggaggctga ggcacaagaa tcacttgaac ccaggaggtg gaggctgcca tgagctgaga 147300
tcataatact gcactccagc ctgggtgaca gagactgtgt ctcaaataaa attttttttt 147360
ttttttttac taaagataac ttgcagtaga acattccaaa agaacaacac tgcactgaag 147420
tgcatttgaa aatgagggct cccaaattag tctcatgtac agtctcatgt agggatgcct 147480

attgatatgc agaagcttct aaaaaatttc aatatttgca tttaaagact ttacaaaaaa 147540

gaattaaaaa gcttaaacaa ctaattgatt taaaaaatta aatctgcctt gcgctttttg 147600

ctgatggctg tgtgtgacag gattaggcat gtacaggatc atgggacatg gggaactttt 147660

ttctccccaa agcaggaaac ttgagagctg atgagatggc tggaaaagat ccttcatgac 147720

tgacaagcag ctgcctgaac tttttttttt tttgaaacgg agtctcactc tgtctcccag 147780

gctggagtgc ggtggcacaa tctcagctca ctgcaacctc cgcctcctgg gttcaagtga 147840

ttcttctgct tcagcctccc gagtagctgg gattacgggc gcccaccacc atgcctggct 147900

aatttttttgt gtttagtaga gacagggttt caccatgttg gtcaggctgg tctcgaactc 147960

ctgacttcag gtgatccgcc caccttggcc tcccaaagtg ctgtgattaa aggcatgagc 148020

caccgcacct ggccctgaac ttttaattta gtgtcactgc aataggtggg tccttctctg 148080

tcctccctga actctttgcc ttccccaccc tgctgcagac aatgcttttc tttctctctt 148140

tctcttcttt cctttttcta tctttctttt tttttttttt ttgaaacgga gtcttgctat 148200

gtcacccagc ctggagtgca gtggtgcgat ctcggctcac tgcaagctct gcctccgagg 148260

gtcatgccat tctcctgcct cagcctccgg agtagctggg accacaggcg cccgccacca 148320

cgcctggcta attttttttt tttttgtatt tttagtagag acggagtttc accgtcttag 148380

ccagaatggt ctcgatctcc tgacctcgtg atctgcccac ctcggcctcc caaagtgctg 148440

ggattacagg cgtgagccac cacgcccggc ctcctttttc cagctttcta ttatatcttg 148500

tctagagatc acatgttgaa acttctggct ggaggccatt ccaccccact ttgaatagat 148560

taaagatagc agagccccac caggggcaag tttaagcctt accagttcaa tattgggcac 148620

taagcagagt ggctaatgtc tatgttttgt cacatgtatt ttgctgtggc tggaagggaa 148680

aatgttaatt cagttccctc atgcaatctc ttgggcagca tcttacaaaa ttgagaggct 148740

tttgcctatg gttccctgaa atcagaaaag atgattttcc tttgtgttgt ggcttggccc 148800

ccaggctatg tgcagctaac agggtcgcta gggccactca gaaagaggga acccagaagc 148860

ctggcatgct ggcaaaaggg taagaatttc ttagcagtca ggcttctggc ctctctctgt 148920

gtggaaacgg ttgtaggagt agtaaaaatc actgtctcct ctgcaaagtt ttaattaatg 148980

ataaaaagga tttgtgaggc tgggtgcggt ggctcacgcc tgtaatccca gcactttggg 149040

tggccgaggc gggtggatca cgaggtcagg agatcaagac caacctggct aacacagtga 149100

aacccagtct ctactaaaaa tacaaaaaat tagccgggcg aggtggcggg cgcctgtagt 149160

cccagctact cgggaggctg aggcaggaga atggcgtgaa cccgggaggt ggaacttgca 149220
gtgagccgag atcgcgccac tgtactccag cctgggcgac agcgagactc cgtctcaaaa 149280
aaaaaaaaaa aaaaaaaaag gtatttgtga gccaatctta agctgtagca aacctggtgt 149340
actttgtgct atgaattggt ctttctgtgt catttggtca taaaaggggt taaataggat 149400
aaaatgtggg cctaggactc ccagaagtct gctgttcaag ccagcctggc aaactggtca 149460
gttacaaact gcgggtccct gaaacaaaaa aacactggat aaggtttccc tgtcatcttg 149520
ttttatgtca ttgggagctt gaccttataa tcacatgatg gtactttctt ttggtctcca 149580
ccctctggag gacaggaatt ttggagttca tgtcatagtt agctctaaaa attatcttga 149640
gcagttaaaa gcctttgcaa gctgaaaatt ggctgctctt aggctcctcc ttctgggagg 149700
aacaatggaa accaccaacg gtgtagctta gtggctaagc tttgccatct tatgatggca 149760
gccaggcagg gttcaattcc agctcaggga atgagacctg tttggtttga tatctttctg 149820
atctttgcta tttgctgatt ctcttcccct catgaacaac ttctgactct cgtcttgaat 149880
tttcctttct ctgagctacg tttggaaatt ctagagtttg taaaaattgc ttgctacctt 149940
tttgaaaata cttcgtacac ttgtggttaa gtcataacct tgttaaagct tattggtttc 150000
acctgggaga ttacctttgg taaagttcaa aagccagaca tattggctgt ttggcctggc 150060
taaagtcaag taataagaga tttaaaatga ttattttagg ctgggcgcag tggtccatgc 150120
ctgcaatccc agcgctttgg gaggccaagg aggatggatc acctgaagtc aggagtttga 150180
gaccagcctg accaacatgg agaaaccctg tctctactaa aaatacaaaa attagctgga 150240
tgtggtggct cgcactggta gtcccaccta ttcaggaggc tgaggcagga taatcgcttt 150300
aacctgggag gcggaggttg cagtgagctg agattgaacc actgaactcc agcctgggca 150360
agagagcaag actccgtctc taaaaacaat caaataattt tttttttttt ttagaaagag 150420
cgctatagtt aaaagtcagc ttaattaaaa gtggatatcc aagctatagg tatgtttaaa 150480
gggccttttg gtttttctc ttcttggatc tttgtttttt tttaattatt tttttctttt 150540
tagccaactg aattgttttt ctccattttg tcttcttgcc acttttgttg cacacatgag 150600
aggacctaag gtaacttcta acagcctggg actccttggg aaaaacagag gaggcactac 150660
gatcctgctt tgggatataa ctcggttttc ctcctgaaac accaggaatc aaaagcagat 150720
agatccctct caaaatctaa ggctaaatat ccttttgggg atggccaaca gggtgaaacc 150780
cagtctctac taaaaataca aaaattagcc tggtgcagtg ctgcacacct gcagttccag 150840
ctactggaga gtctgaggca tgagaattgc ttgagcctgg aaggcggagt tgcagtgagc 150900

cgagattgca ccactgcact ccaacatggg tgacagagtg atactctgtc tcaaaataaa 150960
ggaaagaaag aaagagagag agagagtgga aggaaggaag gaaggaagga gggaaggaag 151020
gaaggtcatt tccacagtta attgcttaat gctggtgcaa tttctgaaaa cttcacaagt 151080
aggcacaatt ctagaatatg gtgtctttta ggaggttcct gaaaggatgg aaaggacccc 151140
aaaaagtatt cttgattaca ggtttctgat aactttagaa tcatatcatt tttactgggt 151200
aagaattcct ggagcttttt tttttttttt gagacggagt ctcactctgt cacccaggct 151260
ggagtgcagt ggcacgatct tggctcactg caagctccgc ctcccaggtt cacgccattc 151320
tcctgcttcc tggagcttaa aagactgact ggtttataaa actgccatcc caagtagaac 151380
acaaattaat tgaataccaa gaaaatactt tgcaagtctt tcatgctaaa tcacccaata 151440
ctgaaattgt ttagatacac aatttgaaga aactctgtgg tctaactcaa attacctatg 151500
ataacccatc agttatcagt gctatgcacc tcaattggag aaacaaccgt gtcaagcatg 151560
gactcatgga gaaccaaggt ggctgcctta tccttcctga gcttttaaag cttttataat 151620
ttaaagttct gcattccatg actcatcatg gaaaagataa aatgatccaa gttaaatgca 151680
tattggtgtg gtgacttcta aattgctaaa atagtttatg accaatgttt ggtttgccaa 151740
acccatattc ctgggaagac aatcaaagct tcaggtacat tctgctacct catgagccat 151800
ttaaacattt atgaagagat ttcagtcaat tgtcattttc aatgcatgtt ttctggttgt 151860
gtaaaagctt tcctatacaa gtgaactgat gttataacag tagattatta tttcacagtg 151920
tattttcacc aggtaaagaa agctttttat ggttcactga ctgaggacaa tcaacccctt 151980
cacaatctag aacctgaaga ttggatcttc tgagaacatc agagaaagac tgcccttgcc 152040
atccacactg cagtaaacat cagaaccgtg aactttggct tcatgatctc acaactgaga 152100
aggatccctc cacattcttg gaaatataca cccattggaa cccgtaaggt aaagctaacc 152160
agggaagttt cttcccagaa gaagatggta tccttgatgt gaacagcttt caccaagatc 152220
acagatcaag acttctctgc tatatggagg ctcttatctc tgagtatttt ttcccttgct 152280
gatgcctcta tgaacagtag aagtgaaaag ggggtctgtt gtgtgcactt atggggtagg 152340
tatactttta ttggtgaagg gttttgcagc cagccttata catggataac ctcacacctt 152400
gatagatgaa agatgaaggc ccagtgtagg tgagaaattt taatggtaca taggttgcct 152460
cataatcagt cagaaacaga acattgattc actcccctat tccacatcat gggttaaaga 152520
gaacattgcc aggaggcctt cagtcttcta gaagggcatt tttccttagg tcctttttac 152580

catgatttgg agtaaaacag gcaatgatta gaaatgtatc cctcatgata ggctctaaac 152640
agagtctact gtaagggcga tggttacaca acagatttta cattcttttg tgaaagttat 152700
gcttaatagt aggattgctg tagattactt actggctaaa cagagaagta tctgtgcagc 152760
tgctggcact tgtggcctat ggagaaatac atcacgtcag gtgtacagag attcagttgt 152820
aggtaattaa tgaagagact gcttaaatga gtaaactctt tatttagctc attctttgat 152880
ctatttaatt ttgggtggtt tggtttatgg ggaccctgtg taaggagcat actccaaact 152940
cttggtatta tcctcctgat agtcataata gtagtctacc tggtgcactg tattctctcg 153000
aaagttttaa atgtttgcat gcagccatct ctagaatgtc aaattgtctc tcttcaacta 153060
gaatgacaag attgaaagaa atgtgtgacc atgatggcat tttaacctat gaatgatgtg 153120
ttgagactgg aaacccaaaa tgatggtaac tgagagtggc gctaaggccc taagctttgg 153180
tcacattctc acctaagtga gaacctgacc aaaaagggga catttttaaa caaaatcatg 153240
ggaggccatt gttttggact ttaatgaaag gctaaatgca ctaagcccca acagaccaga 153300
ccaaaccaaa atggagtcac ttgtgctaaa tgtgacatga ttaaactaag actttaagga 153360
aacacataga tcctaaaaca gaccaggttt tgttttttc tcctataaac agaacgttcc 153420
aacaaaagga ggtaaccctt acaaaaataa aattaaaata aaataaaata acctgaaatc 153480
cttgttcccc cttacaaaac tcattgttct gctatttccc aggggggtttc aagaccaaat 153540
aagtacattt acaatggtga tagtggcatc gatgactaaa gttttggtaa atctctaaaa 153600
attgagaaga ttaccaaaag aggaaaattg ttaaatcaag tttagcctaa agctgcctcc 153660
ttacatattt taagtttggc ctaaaggttt ctctgtacat cgtgaactat aacaagtgga 153720
ggtgtaaaca gcctacactt gtgtcaatca ccaagtttcg gccaatcata tgtagccaac 153780
tgttcaaact gtgttcaaat aaggatacac ccagctgtaa ccaattgggt gtttctgtac 153840
ctcacttctg ttttctgtat gtcactttcc tttttctgtc cataaatctt ccaccacatg 153900
cctgcaatgg agtctctgag cctactctgc ctcgggaggc tgcctgattc acgaatcgtt 153960
cattgctcaa tttgactctt ttatattgaa ttcagctgaa gtttttcttt ttttcttttt 154020
tctgtttttt tttttcgaga cagagtctca ctccgtcacc cagtctggag tgcagtggcg 154080
caatctcagc ccactgtaac ctcctcctcc cggttcaagt gagtctcctg actcagcctc 154140
ccaagtagct gggactacag acagcatgcc tggctaattt ttgtatcttt agtagagaca 154200
aagaggttta gtagagacaa aggggtttca ccatgttggc caggctgatc ttgaattcct 154260
gacctcaagt gaaccacctg cctcggcctc ccaaagtgct gggattacag gcatgagcca 154320

ccgtgcctgg ccagaagttt ttctttgaac actttttttt tttggggaag gagtctcgct 154380
ctatctccca gactggagtg cagtggcgcg atctcagctc actgcaagtt ccacctcccg 154440
ggttcacgcc attctcctgc ctcagcctcc tgagtagctg ggactacagg cacctgccac 154500
catgcccggc tgattacagg cgtgagccac cgcgcctggc cttctttgaa cacttctacg 154560
tggaagtgtg aggattcaat gaataagttg agcacatggt acatgttcag tgaatatttt 154620
ctgattgtgt tcagtgttat cattattgga atgatgtgag ggttgtgtaa gtcttcagag 154680
accagggaaa caactgaaat cttctatgag tcatttctat cattgtttat ccccattcct 154740
tttcttttca ttcatttttg catttctcat tttctgtgtt attcccttgc attcttaaaa 154800
actttcccat tacctgcttg cttcttgtat tctgtgtact ttcctatgtg ttcctcattc 154860
tgcagtcttc ttccccttca agcagcagac catgtgtcaa cgtatgcaga gtttgtgcag 154920
acacacagac cctctgggga gtatatgttt gaatctgatg aagatgagca gttatttgtg 154980
cacctggaca agaaggagat ggtctggcat ctgtgagagt ttattcacac tgtgaggggc 155040
agaaaatgaa gggaccaggg atggaaaggg atgggggaca aaacactaag ctggcagtcc 155100
taaccccagt gtgtttattc tactccaggt gtcaaacttt ctcagtcaga agttgacata 155160
aggaagtcac cagcttcggt ctcctttaaa tctccaggcc taactttctt ttgaatctct 155220
gctcttcaac cacaatgact tatgtacccc aagacccctc tacctcctgg ctgcctatga 155280
tcctcctgca cctagcactc agcaaagggt ctgacattta gatagcactc aatcaatatg 155340
aagaatttaa agtgtagtga caaggtggtg cccacactgg atggtctgta tgctgtggtc 155400
tcatcaggga cctttctctg cactgcattt ttcctgagaa atcccagagt tcaataaata 155460
cctgtgtatg tcagagctag acacatctga tgcctttctg tgactcctta tattctcagt 155520
ttcctaggaa tgcatatcct ggacttgtaa ttgtaggata caggtgcatt atgggtgcag 155580
atacctcagt tttgcatcct gcaagagtta gtctgcagat tgttcactcc actctgggta 155640
cggctgacac ctacagcatg cgtccagggg acaaaaatgg aaggagatga ctctgattgg 155700
atagtgagag gaacacgggt agctctggta aactgaccag ggataggcgc ccaggggcac 155760
acaaggccca gaagctgctt gtgagaagtc agggagaagg aagccaacaa ctggatgtat 155820
cagagagcca ctgagtgctc attatgtgta tcaggagtta agaatccatc ctctacacga 155880
aaaggaaagc ttagaccaaa catgtcttct accttgattt acagtaactt aatatgaaat 155940
gcaaaacaca taaaaatttt ggattgtaaa aaaaagatca gtgtttgaaa gagtgccaca 156000

cctattgctt tgcactttca acaatgggga ggcgtttctc actgtatgaa ttattagctc 156060
aggaggtcta ggctggtgaa tgaatgcaga cttagaggat aaatgtcgtc tacttgatgc 156120
attcaaaagg ccaagcaaca tttggttaag gagtatttgt gatggcatgc aaagaaagtg 156180
ttgaaagtgt gttagattat atttatcgtt ttaaattctg atgcttattt aatttcttaa 156240
cagtaatgct aaaacatgct cttagtaagt tatatgttga atcagtgcta tgttaatatg 156300
ggccctataa cacagatttg cagtctgttc accactgaat agctaagaat agtggtttca 156360
gacctaaatg gatgaagaca ccaaacatga ggatggggca gctcactgaa atatagtaaa 156420
tccaggagag gtctatgtcc aacaggccac cttatacaat agtttggatg gattttcatg 156480
gtttcttct ttcttatgtc taaaccacaa tgaacttctt tattaactct tactgtttgc 156540
taaaaccctc aagaaaaaaa agtgatgaga catgacttgg tgtatattct taacagaaaa 156600
caatcatgtt gggttttgat tactcatggc actaaggaga aaggaacttg gttggaggcc 156660
ccatgagaga aatcatgcat tgaatccagg aacagaatga tagaaactgg cttcagtgca 156720
actactcagc agcagcatcc tcttgggagg ctgggggtct gtcctcgaag caggtactgg 156780
gactaaagaa acagaaaatg cttgttccaa ggtacattag tcttttaggc aaggtcagca 156840
accctgtgga ggtaaaaaac agtaatccca ggttgctggc cccccttcctt taagtcccag 156900
gagagaaaaa taacggtgag aggctgaatc tcaggctgat aaagaataga gttgatctga 156960
gcttccttta gcaatggagc catgcaatgc tctttcccca ctcaccccag ggctgaggtt 157020
gaaacagggg ttcaggcctg agactctctt cctagaggag cctcagtgtc acaatgtaca 157080
gaaaggactt atgtcctcct gtaactcccc caacaaggaa atcaaagcag gaatattgcc 157140
tgtttatgca gatccttgtt gaactgaaat gaaaggaaga aagagaatgt attcagctac 157200
tgaaaccacc tcagacaatt agagaaacgt ttccatcata cttccattgt ccatcactca 157260
atggacgctc tgcaggagcc tgagtaactg ggacctcatg catctggctg cattaagatg 157320
ccaggctggg attcctctct ccttagaagc tgtctttgtt tcctgactat gtgatttcag 157380
caagctctct ggcattgctt ttttgtctca gcccctcatc atggaagaag ggtgagcaag 157440
tcaggagaaa ggtgggtatc ggtcacctca caggatttct cacctttctt cctccttctg 157500
tgcgtgaaga tgtccactcc acagatgatg agccccagca tgaagcccct ggctcctgtc 157560
agcatcttgc tctgcacaga atcagactgt gcctctagga agaacaggct caggttcaga 157620
gtcacctcca gcagctgcac ccctgctcat gtcctgtctg agatcctgca gcctgatgca 157680
gaatattggc acgaggagag cgaggggaat acaccttgca tgacaggaaa ctgagaaaca 157740

gtcactctct caattctaga acagagggtg tgacctcaac aacagacaaa ttcagattca 157800
attagttgcc tctcaaaata acttagaata agctaaaagg ctgaaggaag acaacagttc 157860
ccatgtgtaa cgtcagttca ggaaggtgtc tgcatcccta gattcttgga caaggagagg 157920
aactatgaat ccttaggacc tcctaggccg gggcaggca agtctccatt gtccccaggt 157980
taaaactaca ctcagtgtct tctatagaag aaaaatgggg tcggacatgg tcccagggta 158040
tagacacata aaaccagatg agtggacccc agagccagag agtcccctgc tcttcagagg 158100
tggagtctag agggtcatca gaccctcact ccactccacg gtgacaggac tgtccaggct 158160
ggggtgctcc acttggcagg tgtagatgtt tccctgctgg ggggtcattt ccagcatctc 158220
caggatctgg aaggtccagt ctccattacg gatcaggttg gtggacacga ccccagctgt 158280
ttcctcctgt ccattcagga agcatcggac ttgaatgctg tctgggtaga aatctgtcac 158340
gtggtagaca agcaggtcgt ggtgctgctg gggctccttc ttggagggga gatgttcacc 158400
ctaggctgga ctaggagtgg cgaagtggaa aagtggaata gcatttgaga ttattatttc 158460
ttagacatgc tcattatgga attgatttct agatgttgaa aataggaatg atggccaggc 158520
gcagtggctt acacctgtaa tcccagcact ttgggaggct gaggcaggtg gttcgagacc 158580
agcctgacta acatggagaa accctgtctc tactaaaaat acaaaattag ctgggtgtgg 158640
tggtgcatgc ctgtaatccc agctactcag gaggctgagg caggagaatc gcttgaaccc 158700
cggaggcaga ggttgcatga gctaagatcc caccattgca ctccagcctg gacaacaaga 158760
gtgaaactcc atctcaaaaa aaaaaaaaaa aaaaaaaaaa aaagtaggaa tgatgtgatt 158820
aggtcagcag ataaggggag tattgaaact atagaaatag tattgagaag aataggaggc 158880
acacaaactt accagtttgt agcacattta aaagaaatct tatctatcag ccactgatta 158940
acagcctttg gacaaagaat atttaatcag cagcgacact accttatctt ttgagtgctc 159000
tatcctgatg caaaagatga aaaatttgct caaatgattt cttgaaatta atatatatat 159060
aaacaacatt ctcatggtct attaatccaa taactatatt tcataaatat atcaagaaaa 159120
taaagtgagc ttgacgtgac ttattctgca agtaacctat ggatttttg gcctcgccta 159180
caaacactca aaatcgtcct atttacataa tatcctgagc ccctacatgc ttaaatagta 159240
gaaatcccat ccttttccct aagttcctcc tctgcactgt gttgtcacat ccattgaggt 159300
tctcagataa ccaggtgttg gctgtgcctt ggcacctctc tatttttgcc ccctcttttt 159360
ctgccgctta cagatcaatt tccacgctac cacctgcagc ttttcctttg gacctcagcc 159420

actagtaccc ttttcctcca cagaaggcag aaaagggcca actgcccta gagaacagct 159480
caattacagg aatgttccaa atcagagctt ctaggtcctt atttactctg ttcttgagaa 159540
cacaaataca ctgacatggg cctgggccag gtaaatgaac agtctttaac taaccccaa 159600
cccttactcc ctatggcatt tttgccttgt gtccaggatt attcacattc acagcctctt 159660
ccctcgatgt gattttattt cagaggcact tggctctcgt tctacttcaa ccacctccat 159720
aaactcaaca tccatcagac tggtcagccc atgtctgaat cccaactgta gattaagtgg 159780
aacttcttcc ccataggtgt cctgggaagc atgaaagtca tgggatatat ggttgtctgt 159840
tttaaggtcc cagaacattc catgagaagc actttgctcc atcttctttg acgggagctg 159900
gtcagcctgg agcacatcag agacctccag gggcgctgc agctaaggct tgatacccag 159960
tggcctcatt tctgacccag ttcaaatata tttcaccaag gcaaggattc ctgtttgttc 160020
tagttgcctt tgctcagtta attctagatt tgggtgggca tccacactgg aatggccaga 160080
acttctatcc tcctctttcc tccgtctccc ctacccctgt gtttttcttt attctaattc 160140
atgcccttat tatgatttcc ctggacaata atagcagttt cttatctcat ctccctgcct 160200
ctggttcctg ctctctccaa ctgacttcac agtcttctga aaacagattt ccagccttgt 160260
catttttttt tttttttttt tttttttttt ttgagacgga gtctcgctct gtcgcccagg 160320
ccggactgcg gactgcagtg gcgcaatctc ggctcactgc aagctccgcc tcccgggctc 160380
acgccattct cctgcctcag cctcccgagt agctgggact acaggcgccc gccaccgcgc 160440
ccggctaatt ttttgtattt ttagtagaga cggggtttca ccttgttagc caggatggtc 160500
tcgatctcct gacctcatga tccacccgcc tcggcctccc aaagtgctgg gattacaggc 160560
gtgagccacc gcgcccggcc tccagccttg tcattttaag atttctgaag attttcaaaa 160620
atcttcagtg ttctgacact tatctggtta catgggaatc acttgggagc tgattaaaaa 160680
cacagatctc cagacctccc cacctaggga atctgaatct ctaggcagtg agtctggggg 160740
aatctttgct tcacagagga tctcaaggag agtcttctga gcagtgaggt tggggacctg 160800
ctggcctaga ggagaaatcc acactcctta gtctggcatc agggggtctc tgcaatctgg 160860
cagctgatta ccatgaaaga aaatgctgcc atgtcgctga tttgggcctc tatgaagtct 160920
tagtgacaga ttttgcaaat tgtattactt ttatttggac acctctctca accaattacg 160980
ccagcaactt tcctaaaccc atgtcactct cctcatcctt actcaatgtg tgcatcttct 161040
ccctcttatc agatgacctt cctttcactt tcccaggaac agagggatca atggtgtgac 161100
ctcctgagcc tcctcctcct ccccttcat tctttcctct ttctctagga caaaggtcct 161160

tgtttagtga gaggctcact ggtgcttttt ggtctccttc acgggatttc tctcttggcc 161220
aaattaatac acgagttatt aagagattat ttttaggcag ctaaaaaggg taaaagttct 161280
gggtggaatt ttcctttaat aaaaagcagc cccaagccat tttttctcta acagaaagca 161340
gcctgaaaac tcaggcatag atatgcaaac tagaagcttt tatgtaaatg ctggcagctg 161400
ttcctggaag tcaggtaatt caatatggct attctcaccc tctttccctt ctacgtttac 161460
aggtgtcaag gcagcctcca ggttaaagca cgtgtacagg tatcatggcc gccaccaggt 161520
ggaggccgca tttgtataat aaaatactag ggtgggaagg ccagtctttt tgcaggttat 161580
gtaaatgaca cacctggtca aaccaatccc ctgagcccta tgtaaatcaa tcactgcctc 161640
ctcaagcctc tgtacaaaac caattgcttt ccaccagaaa caggagaccc tctcttgggt 161700
gacctgcctt atcagcatta ggaagctttt cctctcactc ctcttttcta ttaaactttc 161760
cgctcctaaa cccactcctt gtgtgtgtcc gtgctgtgaa ttcttttcgg ctatggtaaa 161820
gaaccagggt atataccccta gacagtggag ctgtttcatt ttgggagctc atccgggatc 161880
caaatcagaa tggaagatag aaacatcgga gtggtgagta tagagcaaac gtcaaatctg 161940
ttctttaatc tcaagaatct cttcatacca gtttcctttc atggagaact tcaccatcgc 162000
atgaggctgg gaaagtcttg gggcaactga aaatttctgg ccagggcaca ccctggtgtt 162060
attcaaaggc ttctggactg aacgcagcct ccgacagcct gtccaggtgt tggtaatgga 162120
tctccagcta acccgttgca aaattttcct ttcctttgta tccgtggtca ctatgtctcc 162180
tgtcctctct ctctgtgtgt gcaatttgcg ggaagtttta cagttcaggg aaacactcct 162240
gttagggaag atcggcaaat gccacaggca gtaactgtta ctctctatcc tctctggcga 162300
gcacatggta gtgctaagcc aacagcacca cctagtggaa ataaaaatcc tcttcatcag 162360
gcaccttgtc ggttttttac cgtaacactg ccgcttccaa attctttcgt gccgctagaa 162420
aagcctcttc tgtgaacgag aaagcactgt cttcaacagt ttggagtaaa atgtcctctg 162480
tagtgaaatt ttagttctga tactgtctca tcagcaggaa aaacagccat taggttccta 162540
cgttcatttc cgtctccaat taggatagta cttaattagc aaggggattt taggttcgga 162600
agttaaccag agccattttg ctaagggtaa atgtcttagc atgggccgta atggcaggca 162660
atctagcaca ctgcctccgt taaaggagcc tacccaaaga tgacatagtc tctctggaga 162720
tccatttttc tgggagccag gcagatcaca caaatttagg acgtcaaagg ggatcacata 162780
aggtggataa gctaaggttg tgtgggtaaa gtatggttaa tcccatcact tagtttatcc 162840

agttccacgg cttggaggac cacgcctaca accatgggtg gtacatttaa cacgttgcca 162900
ggacccagga accaaggaga gaaaacagta gggaggacac ttccactgtc ttctcctcca 162960
ccctgggtca caatgaaaga atggggacga aaggatactt ttattctcac ttctttttct 163020
agatgggtga cagaccagct tcagcttgca cccctctgga gtgcactctg aaacactgga 163080
actcctttaa cctcaggact ttgaagagaa aagtgactca ttttcttttg cacaagggca 163140
tggctttttt actaaacctt tgcaagcact gtaagatcag cccagctttt taaataggca 163200
tatcaggtag gcctatagaa aataatcccc cagaattaga aaggcaattt ccaagggaac 163260
catctgagaa ttccccttat ttagggttgc caatatgggg gaagtaaaga gaaatcagac 163320
tgttgctgtg tctatgtaga aaaaggaaga cataagaaac tccattttga tctgtactaa 163380
ggaaaattct tctgccttga catgctgtta atctgtaacc ctagccccaa ccctgtgctc 163440
gcagaaaacc tgtgctgtat tgactcaagg tttaatggat ttagggctgt gcagggtgtg 163500
ctttgttaaa aatgtgtttg taggcagtat gcttggtgaa agtcattgcc attctccagt 163560
ctcgagtacc cagggacaca atgcactgtg gaaggccgca gggacctctg cccaagaaag 163620
cctgggtatt gtccaagggt tccccccact gagagagaca gcctgagaca tggccttgtg 163680
ggaagggacc tgacctgacc gtccccaagg ctgattccca taaagggtct gtgctgagga 163740
ggattagtga aagaggaagg cctctttgca gttgagataa gaggaaggca tctgtctcct 163800
gctcatccct gggaatggaa tgtctcggtg taaaacccga tcatacattc tatttactga 163860
gataggagaa agccgcctta tggctggagg tgagacatgc tggtggtaat actgctgttt 163920
actgcactga gatgtttgtg taaagtcaaa cataaatctg gcctatgtgc acatccaggc 163980
acagcacctt tccttaaact tatttatgac acagagtcct ttgctcacat gttttcctgc 164040
tgaccctctc cccaccatta ccctatagtc ctgccacatc cccctcactg agatgataga 164100
gatagtgatc aataaatact gagggaactc agagaccaga gccggcacag gtcctctgta 164160
tgctgagcac tggtcccttg ggcccactgt tctttctcta tactttgtct ctgtgtctta 164220
tttcttttct cagtctctcg tcccacctga tgagaaatac ccacaggtgt ggaggggctg 164280
gccccccttca acctcaagct ccctttttcat tacaggacct taggcaaaca aaggaagact 164340
tatgctaatt ttctgataac cccaataggt atatagaagc tgtccagaat ttaactcagg 164400
tgtttcacct cacatggaag gatgttatgc tgctcctaaa ccaaactcta accgcagttg 164460
aaaagcaggc agctctgcag gcagcagata attttggaga tgagcaacat atctcctata 164520
atacaccaaa agggaagaaa agagataggg aaagtgaaaa aaataacaga aacaccattc 164580

ccagtaggaa gggaagcagt tcctctcgac aaccccaact gggaccccag tagctctgca 164640
aatgaataga aatggaagca tttttaaaat atgcatatta gagggtctat gaagaactaa 164700
ggcctgacct cttaattcct ctcaactgtc tatgatagac caaaagccag atgggaatcc 164760
tgcagctttt atggaaaggc tgagagaggc actaatagag cacacttcct tagcccctaa 164820
ttcagtcaag ggatggctca ttgtaaagac aagtttatta cacaggcagc tcttgatatt 164880
agaaggaaac tgtagaagca cgctatagga ccagatagca ccttggggaa cctcctgagg 164940
gtggccactt ataataggga ccaggaggag gccccccaga aagagagaaa gctcaggaga 165000
aagacagagg ctctagtagc agctttgcaa gcttgcaaag tccaagattt ctgaggtgca 165060
tccgctagtt gctatcagtg tggcaagcca gggcatttta aaaaggagtg cccaaacagc 165120
aagaggaagc cacctcaacc ctatccagcc tgtggtggag accactggaa atcaaactgc 165180
ccccggagac ggaggtcact ggagtcagaa ccagtctcac agatggtcca gcaggactga 165240
tgggtcctgg ggctcaaacc ccagctccag tggctcaaac tgccattaca gcacgggagc 165300
caccaggtga ttctggaaat tgaaggaagg aaactagacc tccttctaaa cactcgagcc 165360
agtctctctc tcttttctcc tctttaatcc aggcctctct tcttcccata gcgtgagtgt 165420
aaggggtgtc tcaggaaaaa ctctattcca atatttttct caacctccta attgcagtta 165480
ggaggaccta ttgtttacac atgcttccaa gccattgcca cggtggctct actagtcaaa 165540
aaagcctcca aattaaccct aggaaataat ttaactgttt acaccccaca taatgtagca 165600
ggattactgt cctctagggg agactttagc taacaaacag caggtaaagc aagaaataca 165660
taaggcagga caagcaatag tcactctaat gtctctcccc agacacaagc actcaattag 165720
ctgaactaat agttcttgca agtgcactta aattaagcag gggaaagata gctaacattt 165780
ccactgactc caagtatgct ttcttagttc tccatgctca tgctgctatt taaaaggaaa 165840
gacattcttt taccactaaa gcatctccta taaaatatca ccaggaaatt aacaggttat 165900
tatcctcagt tttcctttca cgaaaaatag cagtaatgta ttatagggaa catcaaagag 165960
gaacagatga agtagccaaa ggaaataggt tagctgagca gggagctaag caggcggcag 166020
ggaagcctca aggcattaac acacttcaag cccttttaat ctcggaagcc tccataaaag 166080
aaattaaacc tcagtattcc cctgcagaaa taaaataagc cacttcttaa gggtattcca 166140
gccctgagga tgacaaactc catttactgg cctccagtca atggaaagtc cttaaaatcc 166200
ttcaccaagc tttttcacat aggaaaggat aaaacttatc atcagtgtgc tcagagattg 166260

ttttcaggca gaaaacctct aagttgttta aaaatgtaac ctctctagct cacttccaac 166320

agaaattgac acaactagcc aaaggccaac cccaggaaat tggaccacca gaaaatttgg 166380

tattggtgaa aactcatctc tctctccttc cctaagccag gctgggaagg gccctacaca 166440

attcttcttt caacccccccc agcagtaaaa gttacaagta tcaacctctg aatatatcac 166500

actcaagtca aagcctgaaa agctgaggga gcaacctttg acagcccaga ggaacatcct 166560

gaatatcaat gtggagatat agaagatctt aagctgaaaa tcataaaagg taagtaaatg 166620

agtgagggct actcgcctta gcgccaatcc tacctcacca ggtactcttt atcatttcta 166680

cctttcctct caaaattcac tgctcagtat tagaactttt ttttaatgca tatttgcaga 166740

gagattttaa ttatacatgg gactgcattt gttactttgt aaatccccaa agggaaacat 166800

tatatcttgg caagtaaagt tttaaatgga aattatttac tacgtcactt ttgtgggaat 166860

tgttatcatc atgctgttat ttgcaataga actgtatact gtggcaccca caatgtggaa 166920

ttctggttgt aaaattctaa ttcctgtaat attttgccta attatcatct ttatgacaga 166980

attaataatt gcaggaagga tttggtcaag tttgttttgc ttatagcagg agtaatagtt 167040

acagacaaga agtaagcatg aaaattttac tatcactaag tttgatagga ctttttttatt 167100

gaagattggt aaatggtgca ctctaagcta tggaaagaag gttacaaata aagggatttt 167160

atataagaaa ggatcttgta tagtaaattc ttgtcctaaa aggaaatgac tggttgttta 167220

agacaagtca gaaagttgag tacattgtaa gagggtctgt gaaagtcatg aaagaattta 167280

ataattaaga aatttaataa ttaaaggaaa ggaattgcca agattaacac caaagttatt 167340

ttagccaccc aataacgttt ttctcccaat catatcataa gttataaaga atggcctaaa 167400

ccaaaaatta tgccctaata gcaagtcaag ggggaaacat gttttctcaa aggaaatgat 167460

gcttttatat taacgtttct ggtaatgtac agcgacatct agtggagaca aaccagtatt 167520

acaatccatt ggtgtaacag gtatcaaact ctactgccat agttacagtc tataggtggt 167580

aatcttaata ctcatatggt aaccctatat tttaaacctt cttgtaaaat ttatctcttt 167640

ttgcctagaa gcaatcaaac ttcaaatggt gctgcaaaca aagccacaca tggacatgcc 167700

attcttccaa gaagccttag atcaacctca ggaggagccc caactgcagc cccccgacac 167760

gacgcccctt ttcagcagga agtagccaga aagaatcgtc gtccaacacc ccctaacagc 167820

agttatggtt acgtctcctg agggaggaaa taatacagga gttattaaga aattattttt 167880

aggcagctag aaagggtaaa aattctcagt ggaattttcc tttaataaaa agcagcccca 167940

aaccatttct tctctaacag gaagcagcct gaaaactcag gcatagatat gcaaactaga 168000

agcttttata tgtaaatgct ggcagctgta cctggaagtc aggtacatcc aatatggcgg 168060
ttcccactct cttttccttg tcaccacgtt tacaggtgtc atggcagcct ccaggtaaaa 168120
ccacatgtac aggtatcctg tccaccacca gttggagacc gtatttgaat aataaaagac 168180
tagggtggga gagtcagtct tttcgtgggc tatgtaaatg acacaactgg tcaaaccaat 168240
tccctgagcg ctgtgtaaat caatcaccgc ctcctcaacc tctgtacaaa accgactgca 168300
ttccaccaca aaccgcagac cctcttttgg gcaacccact ttctcagcat gaggaaggat 168360
tttttctctc ttttcttttc tattaaactt tccactccca aacccactcc tcacgtgtgt 168420
ctgtgtcgtg aattttctcg gccatgacaa agaaccaggg tatatacccc agacaatgga 168480
gccgttacaa aatcacaatg tccagttccc aggatccagt cctatgctcc tcccgatggg 168540
tcccctctcc ctgggtggtc tcatgtaggc caggcccctc cccgctgca aactcttctc 168600
tcacctgccc atcagaccac ccatctggcc cctcaagcac ctcaaacccg gccatctccc 168660
tgcaggtttc ttctctgcgt ggcctgctgt gccatctcca ccttcatctc cacaccgcac 168720
ccctgcacga tgtcctgtgg cctcgtttcc cctcacccca catgcagtca gctgccaggc 168780
ctgatgaagt cccaggggtc tctctccctc catcctcctc actccatgct cagcccaatc 168840
tccgtgctca ccctcctgac aagctccggt tgggctcctc ccatcaatcc ccagctcaaa 168900
gtccccttcc ctcctggcac taaggtccct taggccggcc cggctgcccc tactccctgg 168960
gcccggcccc cacggctgcc ccaggagccc tggttcacag ccctcacctc ggcgctgccg 169020
gatgagtggc tccatcagct cgtacttgtg tctgcacacc ttgtccacct cggctcgctt 169080
ccgttccata aagtccttct ggctattgaa gtactcgcct atgggccgcc ccagctccat 169140
cactgctagg aactccccca ctgcgctgtc aaaatgcacg tattcctccc ggttgtagat 169200
gagcccgtcc acaacgcgct gagtcccatt gaacgcatag cattcctgcc gttcctggta 169260
gacggaattc tctgtgaaga gcagggagag atgggggtgg tgccactccc aacaacaccc 169320
ccctcctcaa tattagctat ttcctcaaat cttcccactc aggactggat ttaaaaatac 169380
gatttttcct actaccgagt tctgtggtcc taggcaggtc acagactcca ggcatcagtt 169440
ttctcaccac gcagcgagag gatttactga aaagaatgag actcactgct cagggtggct 169500
gagtgatcat tagggagggg cgcacgctga agggaaagca gccctttctg ctggcggctg 169560
gaggaggagg gggagacatt tcacgtctca gggaggagca gaatctcatc aggggaggg 169620
tccctcaggc agagaaacag tagggatagg aggagttggg gacctggagg gcagagctgc 169680

tccccctacc caactccctg cctgacattt ccatcctgat gtcagtcctg gctcaaatgc 169740
cacctcctcc aggaagccct ccattccttc tttccctttc tacagctagg ttctctctct 169800
tctctgccag tttcttgaga atacctcaag ttgctcttgc tgttctgcat gctggacatc 169860
tctgcatgct ggaaatgcag aaatgcattt ccatgcattc ctccctccct ccctcccttc 169920
cttccttcct tctttcttct ttcaatggag tatccttccc tccctccctt ccttccttcc 169980
tttcttcctt tctttctaca gagtatcact ctgtcgccca ggctggagtc cagtggtgca 170040
atctcagctc actgcaacct ccgcctccag ggttcaagca gttttcctgc ctcagcctcc 170100
tgagtagctg ggattacagg tgtgcgccac cacgcccaga taatttttgt atgtttagta 170160
gagatggggt ttcaccatgt tggccaggct ggtctcgaac tcctgacctc gagatctgcc 170220
ctcctcggcc acccaaagtg ctgagattac aggcgtgagc caccgcactt ggccagaaat 170280
gcatttctta aaacttcttg aagtttgcca taagaaagac tatatagcct gaaagttaat 170340
tttcactttc aaacatggct gtagaaagac ttgatctcaa aacacctgga aaatatctta 170400
atcaatgaaa atcaccagaa caaccagaaa gctaagtgac tggactcctt taggtttaga 170460
ggcattgatg gtgtcatttt tacataaaga aatgtggcag ccttttccac ctgcagttct 170520
tcctagggag caccatgggc agtgtccggc aggtgcatgt gtatacagaa gtagcaatga 170580
cccagcacat gtctgagcct cttcagacct gatctgttcc tggtcacagt ggggaaatta 170640
aggtgctgtg atttgcacac agcaaattaa ggtttgtttt ggacacctgt ttcatgttct 170700
acaaattagc aactcacgtt tattaacctc acccctcata gaagatgcac agctggtggc 170760
agaggacagt agagaagggg tggggctggg cacagcggca ccgtagactc ggcctgtagt 170820
ttccagtgtc atctctaagg caagatccca ggactttaga tatcttaacc cgcctcttct 170880
cctagtgcct tgggctccag ccccctaaac acccaggccc atcgccccca gcccctttag 170940
tctgcccctc cctattcaag tgccccactc tgagtgtgta tcctctcatt ttaatttgtt 171000
agtctttgtt cctcttcctc attcaccaat aagagaacat gttttagtca agagtaagaa 171060
tttcaaaact ttcttttgca acatttaact ttttaaaggg aattttacct gcaaagcaga 171120
tataagtgat gagtatatgg tggaatttga aacttcattt tttttttgta tatggaaaac 171180
tttatcctgt ttgcggaatc tttgacaatt gagttaccca gagcacaatt tgaaaaccaa 171240
tgatctgagt gaattcatct ctcactcaga ataggttata ttaaaatata attgcagaaa 171300
gatataatgg agtccatatg ggtaagaaaa ggaacatgtc acaggtaggg attccagttc 171360
ttgcactacg actgattact aagttatctt ggacaagaaa caaccttcta actctccatt 171420

tcttgaaagg caaaatagta ataatactat ttaccttgca aaactgctgt gagaaccaaa 171480
tgagctcaca tatgtcaaat acatagtatt gtatctgtta ctgctcaatc tatcttagtt 171540
cccttcctct tttcactgtg tgacctgttt gaataggagc aaaattctaa ataaatgcat 171600
atgaaagagg aactggatca gcttatgagc tactggaaaa cctcaagaag actttccatg 171660
ggcaccagat cttaatggtt gaaaagtgtt tgttggctgt ttgctgactg gatcttcccc 171720
cctctctttt gtgctaggga ttttatttga ggagttagat taacagaaga gtgagtaccc 171780
aaagactcaa atgcacaatg agcttctgca agtgcccagc tggtgtgaca ggaggactcg 171840
ggagtgtggc cctaatgctt ggggtagtgg gtggggtgag gtaagaaact cagctgtaaa 171900
aagcccattt taaagcttgt cagagacaaa agggtgttgc ctccaggagt ctggtttttt 171960
cacctgccgt gtcatctcct gctccctagg tgttgtctca gccaagcttt tctccaccct 172020
cccctctctc actttaagcc acagcctggc tccttgtgcc tttcctcttg tgctctgagg 172080
atggagatgt gtgtggagtt ggaagagcat gcagggaatt gtggaattgg ccctgcccac 172140
tctacccact cccctatggc tccagctctc cctccagcag gttttgattg gacattcatt 172200
ctacacgggg agctctggta acccaccctc ggttcctgtc acatggctcc actgcctcat 172260
ctcatttccc ctaccaacct caacccagtc tttgcccgtc cacctgttca ctgcccacca 172320
tcatcacgct ccctcctgtg ctttctgcta ccccgcacct tgagggtttc catggcgttt 172380
cccaacacca cccctcatcc tacaaacaac tctgcctatg gacactgttg ctatggacct 172440
cctgctggac actgttcagt gtcaccagcg ctgctccagc ctcctctctc cccaacctca 172500
cccctctcca gttcccaggg ctgagccatt ctgctggtta gttctcagca cccctgtgac 172560
tacaagtgca gtttgtccac cctttcccgg acaatgaacc tgaggtaata ggtgaggggc 172620
tttggggttt gaggggctgt cctcaggaga ttcgaatact gttaccctgg aaaatgagga 172680
ggtgacatga gaacagcact ttctaggggt gtcctaggtg gatgtggaag ggtctcagag 172740
ggagggtcta tgcagaaagg tggaagtcag tggaaaacta agacacctac tctgcagtcc 172800
ttcctctcag ggtgttggtg taaatttgga ccagaaaagt aagaacatcc tgagagaaaa 172860
acaatgggtc atagaagcca taatattaca ccagccacaa ggagacagca ggagacagag 172920
gtttttccct tggttactgc tttcttggct gtctgataac ctacactcaa tctctttcac 172980
gtactcacac atccttatct cattcttctg acaggtttca acccatccta atactctaac 173040
caagttctgg ggaggctggg agaaatacct tcaacaagag tgcctttagg ggctcgaacc 173100

tgtccccctc cctcccatct tgccttcatt gtccagggag cattggctcc tgctccaccc 173160
tggagaatga gaggcattct ctgtgagcac tgaatcctca gtgatacttg tagtctggac 173220
acaccagcta agggctctct gcctgagtcc cctcaaggtt ggatgcagat gtgagcacac 173280
ccaggagtct gcacttgcca acctctctct ctgaaacctt gtctgtccaa ggttatcctg 173340
aacctcttgg ccccatttcc ccatagacaa gcaacttgac ccctgagcac ctccccttat 173400
ttactgtgtc catgttcctg gagagagaat agacctggtg gatagcaacc tatcctatag 173460
gaggtgagtt tgattctcca gctgtgatag aaggacacta ggccgtggca ggagccccac 173520
atgctgtctc agagtctggt tccataaaga gaaagtcccc taggaattgt tccctgagcc 173580
agaccctcca ggaatagcag ctctgctctt acctggagtg gccctgctct ggaccacaga 173640
tatgagcagc accatcagta atgctgtcag agccactgtc caggggcccc ctgaaacctg 173700
caggatcatc atggagttgg aaaaggttgg cagaatgaag agagctgcag tcaggaaaac 173760
aagaactcat taaagggagc tcctgtctga aatattagag accatgaacc caagcagtct 173820
tctgtgaccc taggattgga cagactctga gaaaagaacc aatgggcact gagctttgta 173880
tgagtcattg ctcactgggc agaaagttag tattaaagat ctgacaatat agagccagtg 173940
atgctgttac gaggacagat ggagaacact gacactcatt ttaaccagtc agagtcatga 174000
gttttgggga gatgatgtgt tttctttgct ctgaaggtga tctcagatat tctgctggcc 174060
cacctacagg gattatcatt tccccaattc tgccacacct cacacaccca caggacatgg 174120
cctggtgtgg aagaaatgct atctcaatgt gtaaaaggtc attcagtggc atgatttaga 174180
gagattagag tatccatccc agaactgaaa atgaggcctg gagtctgttt tgcctttgtt 174240
caaggccgtg cttcagatta gtgcacattc atattttctt cctcccacat gtctgtgagt 174300
cctgagatgt gcgggggata ctggctcctt ccataggact gtcatcaggg tcagcagggc 174360
tcagtctagg ggccttacac ctgggagcat ggacacacca cctacactac catggaagta 174420
tgcagcttga aggacactgc ctgtcttgga cttcagttct ttgtcttcag tatggggatg 174480
atatgacctg cctttacagc agggctctta aggtcaaatt agatcaacgg atctgtaatt 174540
gctttggaaa aatgagacta aaaattatac agtgaatgag gaagaaatga aaaaatgtca 174600
taaaagaccc tacattttcc aaaacatctg attctgtggt gtttatactg aatagtttca 174660
taaactttca aagaatatta ctccttaatt taaagaccta taaatgtgat cctgtacgac 174720
ctcctaatct aataaagaaa atgtaaaagt ggcatcattt gtttatataa atgttaaaat 174780
gtaaatagaa gactagcatg taaaattcaa gagaagaaaa taattatgca gtagaagggg 174840

```
ccagtatagg attgcgggga aaaagctcac gttccctgcc atagtcacca agacagcatg 174900
gtactggtat aaaaataggc acatagacaa agggaacaga atagagaacc cagggataaa 174960
cccaaatact tacagccaac tgatcttcga caaagcgaac aaaaacatat ggtggggaaa 175020
gacaccctttt tcaacaaatg gtgctgggat aattggctat ccacatgtag gagaatgaaa 175080
ccgggtcctc atctctcacc ttatacaaaa atcaactcaa gatggattaa ggacttaaac 175140
ctgagacctg agactataaa agctctagaa gatcacattg gaaaaaccct tctagacatt 175200
ggcttaggca gggatttcat ggccaagaac ccaaaagcaa attcaataaa aacaaagata 175260
aatagttgag acttaattaa actaaagagc ttttgcacgg caaaaggaac catcagcaga 175320
ataaacagac aacccacaga gtagtcacaa tctatacatc tgacaaagga ctaatatcca 175380
gaatctacaa tgaacacagg caaatcagta agaaaaaaca aacaacccca tcaaaaagtg 175440
ggctaaggac atgcatagac aattctcaaa agaagatata caaatggcca agaaacataa 175500
gaaaatgctc aacatcacta atgatcaggg aaatgcaaat caaaaccaca atgtgatacc 175560
accttactcc tgcaagaatg gccatgataa aaaaataaaa aaacagtaga tattggtgtg 175620
gatgtggtga tcagggaact cttctaccct gctggtggga atgtaaacta gtacagccac 175680
tgtggaaaac agtgaggaga ttccttaaag aactaaaagt ggaactataa tttgatc    175737
```

<210> 35
<211> 661
<212> DNA
<213> human

<400> 35

```
tacctggtaa gagttggttt ctttttattt ggaaataact atggtcaact ttcattaatg     60
tggaagttga ctgataatgt taagagtcaa agtgaaaaag aaagccgata cccatattca    120
acagtgcttt cagttacaac attttttgaa tttccttctc taaaacattc acagcagagg    180
tgtccatttc acttgcctgt ttcacctcta gataccacca cagttgctgg ggtcatgtga    240
ggtaaaggaa tatatccaat tgactgttag cccaacatga ctatctgcaa ttctgataga    300
aaagctactt tcagccatgt cttacaaagt gcttaaagct ggacagaaga ctttaaataa    360
agctaaaatc gcttattgcc tgagttgaac aagttacaaa ccacagctct tcctcctccc    420
ctgctgtctc accaccaatt agccttcttg tgaggacaac attagaaatg gaactggttt    480
ttgagcccag aaacataaaa atttaagtgt tcttagccca gcgtggtggc tcatgcctgt    540
aattccagca ctcttggagg ccgaggtggg gtgatcacaa ggtcaggatt taagaccagc    600
```

ctggcaacca tgggaaactc ttgctctact aaaatcccaa aaatagcctg gccttgtggg 660

c 661

<210> 36
<211> 3227
<212> DNA
<213> human

<400> 36
gtttgaggcc ggccagtcgt gactgggcgg caatgaggtc agtgactgcc gggagtcctg 60

caggggcggg gcggcgccaa gcgcagggag cccggctgag tggcagccag attgaagatg 120

gatacgtgac aatcccaggg accgctgcac tgacttcatt tccttagaca agacacagtg 180

tagggcccgg cccggttggc cccaggactc ctttggaata tagctgtgga caatgaatcc 240

tgcgagcgat gggggcacat cagagagcat ttttgacctg gactatgcat cctgggggat 300

ccgctccacg ctgatggtcg cggctttgtc ttctacttgg gcgtctttgt ggtctgccac 360

cagctgtcct cttccctgaa tgccacttac cgttctttgg tggccagaga gaaggtcttc 420

tgggacctgg cggccacgcg tgcagtcttt ggtgttcaga gcacagccgc aggcctgtgg 480

gctctgctgg gggaccctgt gctgcatgcc gacaaggcgc gtggccagca gaactggtgc 540

tggtttcaca tcacgacagc aacgggattc ttttgctttg aaaatgttgc agtccacctg 600

tccaacttga tcttccggac atttgacttg tttctggtta tccaccatct ctttgccttt 660

cttgggtttc ttggctgctt ggtcaatctc caagttggcc actatctagc tatgaccacg 720

ttgctcctgg agatgagtac gccctttacc tgcgtttcct ggatgctctt aaaggcgggc 780

tggtccgagt ctctgttttg gaagctcaac cagtggctga tgattcacat gtttcactgc 840

cgcatggttc taacctacca catgtggtgg gtgtgtttct ggcactggga cggcctggtc 900

agcaacctgt atctgcctca tttgacactg ttccttgtcg gactggctct gcttacgcta 960

atcattaatc catattggac ccataagaag actcagcagc ttctcaatcc ggtggactgg 1020

aacttcgcac agccagaagc caagagcagg ccagaaggca acgggcagct gctgcggaag 1080

aagaggccat agctgctcca gccggggctc cggggcggca gcagagctgg cacaccgatt 1140

ctgggaagcc ccgcgaatga tggcttttga attaatgagg cagtgaatgt tttgtgttta 1200

cttctaaggg aaatactaac tttctttcgc attagtatta attttgaagt agctacaaag 1260

tatttttaag aaattataat tttatgactg tctggcaggc tctgtcagtt tagccgcgcc 1320

ggaccgtgtc aagcatctag gagaggagtc catggtgtcc aggcatcggg gcgtcacacc 1380

tgttgaggag tggggtggct ttgaatgctg gaaatggctt catagtgaag tgcctcccac 1440
agggcgggtg ggtcagcgtt gactctttcc actgcacact catatgccgt gtgtcttatt 1500
cagaagtcac attcttttca gttggagaga attgggctaa gatagaaaat aacatgattt 1560
gttccttatt aaagtttccc agcgtatgaa attctaagct gggtggggtg gctcacgccc 1620
gacgtaatcc cagcacgttg ggaggccgag gcaggtggat cacttgaggc caggagttcg 1680
agaccagcct ggtcaagatg gtgaaacccc atctctacta aaattacaaa aattagccgg 1740
gtgtcgtggc acacacctgt aatcccagct atttgggagg ccaaggcagg agaattgcct 1800
gaacccggga ggcggaggtt gcagtgagct gagatcgcac cactgcactc cagcactcca 1860
gcctgggtga cagagcaaga ctctctctca taaaaaaaaa aaaaaaaaaa attctagacc 1920
gaagtgtacg gcagtgccat ctggtggcaa gtggtacaaa gacaacgctg aggggtagtg 1980
actcctgtag cagcagagac gccttggagt ttaacccccca ccaaccagag cgtgggctgg 2040
taaagatgaa atcttgcaag tttttttttt ttttaaatca tggtacctgt tttaaaatga 2100
gaagttatta ttcatactgt gttgctcatt tgacaaaata aggtaaggat ttcataatca 2160
ggttgtctgg gtttcagagc tgtttttaat tgactgagtt acctacaggc acccaatcta 2220
gaccctaatt ctgtggttgg tgttcctctg tagatttcaa gacaagtagt gatgtcattt 2280
tctccctgag ctgtagggtg gacctggacc cctcagagat gctcgactgc agggtcctgg 2340
ggtccctaga gtggtcctgc agcagcccct ggcaatgagc tgcctgatga tgagtcccaa 2400
ggtacattgt tctactgttt tatacccagg tagaagttac acagcaattt agcaatgttg 2460
ttaacagaca tacagaattg tgtgtgtgtg tgtgtgtgtg tgtgtgtgtg tgagtgtttt 2520
gatataaacg tgcaattaaa attcaaagtt tattttttat cacaagcacc tttgaaatgt 2580
acgtggaaaa cgtcggctgc tcgtgagaat catgtgtgac ttacgttcct tatgttttat 2640
ggaactctcg gaacagtgtt ttgtttttgt ttagtttttg ttttcgtttt tttggacgga 2700
gcctggcttt ggctcccagg ctggagtcca gtggtgtgat ctcggctcac tgcaacctcc 2760
acctcccggg ttccagggat tctcctgcct caccctttca agtagctggg attcaggtac 2820
ctctaccatg cctggctgat tttttgtatt tttagtagag acagggtttc accatgttgg 2880
ccaggctggt ctctgactcc tgactaaaag tgatctgccc acctcggcct cccaaagttc 2940
tgggattacg ggcgtgagcc acttcacccg gcccggagcg gtgtattttg attgctggtg 3000
ctttaactat attgaccaag gtgtatagta acctatttat cacattttac gaagcaattt 3060

taatcttaaa cacatccagc aaatggtgtc cctgctgaaa cttttaactg gaaacctcac 3120

acctggcagg cagcactctc tgcagccgtg cccttgtgga gtcagcagga gctggtgttt 3180

ttcagccaga cagaggcata ataaagtgac tgatgtttac gaaaaaa 3227

<210> 37
<211> 3080
<212> DNA
<213> human

<400> 37
gggggcgggg ccttgccgct gcggaggccg gagacgcggc ggcgctggac gcggaggcgc 60

tgggcgcacg gcgcggagcc ggccggagct cgaggccggc ggcggcggga gagcgacccg 120

ggcggcctcg tagcggggcc ccggatcccc gagtggcggc cggagcctcg aaaagagatt 180

ctcagcgctg attttgagat gatgggcttg ggaaacgggc gtcgcagcat gaagtcgccg 240

cccctcgtgc tggccgccct ggtggcctgc atcatcgtct tgggcttcaa ctactggatt 300

gcgagctccc ggagcgtgga cctccagaca cggatcatgg agctggaagg cagggtccgc 360

agggcggctg cagagagagg cgccgtggag ctgaagaaga acgagttcca gggagagctg 420

gagaagcagc gggagcagct tgacaaaatc cagtccagcc acaacttcca gctggagagc 480

gtcaacaagc tgtaccagga cgaaaaggcg gttttggtga ataacatcac cacaggtgag 540

aggctcatcc gagtgctgca agaccagtta aagaccctgc agaggaatta cggcaggctg 600

cagcaggatg tcctccagtt tcagaagaac cagaccaacc tggagaggaa gttctcctac 660

gacctgagcc agtgcatcaa tcagatgaag gaggtgaagg aacagtgtga ggagcgaata 720

gaagaggtca ccaaaaaggg gaatgaagct gtagcttcca gagacctgag tgaaaacaac 780

gaccagagac agcagctcca agccctcagt gagcctcagc ccaggctgca ggcagcaggc 840

ctgccacaca cagaggtgcc acaagggaag ggaaacgtgc ttggtaacag caagtcccag 900

acaccagccc ccagttccga agtggttttg gattcaaaga gacaagttga gaaagaggaa 960

accaatgaga tccaggtggt gaatgaggag cctcagaggg acaggctgcc gcaggagcca 1020

ggccgggagc aggtggtgga agacagacct gtaggtggaa gaggcttcgg gggagccgga 1080

gaactgggcc agaccccaca ggtgcaggct gccctgtcag tgagccagga aaatccagag 1140

atggagggcc ctgagcgaga ccagcttgtc atccccgacg gacaggagga ggagcaggaa 1200

gctgccgggg aagggagaaa ccagcagaaa ctgagaggag aagatgacta caacatggat 1260

gaaaatgaag cagaatctga gacagacaag caagcagccc tggcagggaa tgacagaaac 1320

atagatgttt ttaatgttga agatcagaaa agagacacca taaatttact tgatcagcgt 1380
gaaaagcgga atcatacact ctgaattgaa ctggaatcac atatttcaca acagggccga 1440
agagatgact ataaaatgtt catgagggac tgaatactga aaactgtgaa atgtactaaa 1500
taaaatgtac atctgaagat gattattgtg aaattttagt atgcactttg tgtaggaaaa 1560
aatggaatgg tcttttaaac agcttttggg gggtactttg gaagtgtcta ataaggtgtc 1620
acaatttttg gtagtaggta tttcgtgaga agttcaacac caaaactgga acatagttct 1680
ccttcaagtg ttggcgacag cggggcttcc tgattctgga atataacttt gtgtaaatta 1740
acagccacct atagaagagt ccatctgctg tgaaggagag acagagaact ctgggttccg 1800
tcgtcctgtc cacgtgctgt accaagtgct ggtgccagcc tgttacctgt tctcactgaa 1860
aagtctggct aatgctcttg tgtagtcact tctgattctg acaatcaatc aatcaatggc 1920
ctagagcact gactgttaac acaaacgtca ctagcaaagt agcaacagct ttaagtctaa 1980
atacaaagct gttctgtgtg agaatttttt aaaaggctac ttgtataata acccttgtca 2040
tttttaatgt acaaaacgct attaagtggc ttagaatttg aacatttgtg gtctttattt 2100
actttgcttc gtgtgtgggc aaagcaacat cttccctaaa tatatattac caagaaaagc 2160
aagaagcaga ttaggttttt gacaaaacaa acaggccaaa aggggggctga cctggagcag 2220
agcatggtga gaggcaaggc atgagagggc aagtttgttg tggacagatc tgtgcctact 2280
ttattactgg agtaaaagaa aacaaagttc attgatgtcg aaggatatat acagtgttag 2340
aaattaggac tgtttagaaa aacaggaata caatggttgt ttttatcata gtgtacacat 2400
ttagcttgtg gtaaatgact cacaaaactg attttaaaat caagttaatg tgaattttga 2460
aaattactac ttaatcctaa ttcacaataa caatggcatt aaggtttgac ttgagttggt 2520
tcttagtatt atttatggta aataggctct taccacttgc aaataactgg ccacatcatt 2580
aatgactgac ttcccagtaa ggctctctaa ggggtaagta ggaggatcca caggatttga 2640
gatgctaagg ccccagagat cgtttgatcc aaccctctta ttttcagagg ggaaaatggg 2700
gcctagaagt tacagagcat ctagctggtg cgctggcacc cctggcctca cacagactcc 2760
cgagtagctg ggactacagg cacacagtca ctgaagcagg ccctgtttgc aattcacgtt 2820
gccacctcca acttaaacat tcttcatatg tgatgtcctt agtcactaag gttaaacttt 2880
cccacccaga aaaggcaact tagataaaat cttagagtac tttcatactc ttctaagtcc 2940
tcttccagcc tcactttgag tcctccttgg ggttgatagg aattttctct tgctttctca 3000
ataaagtctc tattcatctc atgtttaatt tgtacgcata gaattgctga gaaataaaat 3060

gttctgttca acttataaaa 3080

<210> 38
<211> 1078
<212> DNA
<213> human

<400> 38
gtgtcgcgcg gcgcggtggc ctcggtcggt accctgggcg cggacgagct gcctcattag 60

tattcgtacc cacgaggcgg cgcacggggc cctcggggac agcgagcgtc gcgccatggc 120

ttatcactcg ggctacggag cccacggctc caagcacagg gcccgggcag ccccggatcc 180

ccctcccctc ttcgatgaca caagcggtgg ttattccagc cagcccgggg gatacccagc 240

cacaggagca gacgtggcct tcagtgtcaa ccacttgctt ggggacccaa tggccaatgt 300

ggctatggcc tatggcagct ccatcgcatc ccatgggaag gacatggtgc acaaggagct 360

gcaccgtttt gtgtctgtga gcaaactcaa gtattttttt gctgtggaca cagcctacgt 420

ggccaagaag ctagggctgc tggtcttccc ctacacacac cagaactggg aagtgcagta 480

cagtcgtgat gctcctctgc ccccccggca agacctcaac gcccctgacc tctatatccc 540

cacgaaggcc ttcattactt acgtgctcct ggctgggatg gcactgggca ttcagaaaag 600

gttctccccg gaggtgctgg gcctgtgtgc aagcacagcg ctggtgtggg tggtgatgga 660

ggtgctggcc ctgctcctgg gcctctacct ggccaccgtg cgcagtgacc tgagcacctt 720

tcacctgctg gcctacagtg gctacaaata cgtgggaatg atcctcagtg tgctcacggg 780

gctgctgttc ggcagcgatg gctactacgt ggcgctggcc tggacctcat cggcgctcat 840

gtacttcatt gtgcgctctt tgcggacagc agccctgggc cccgacagca tgggggcccc 900

cgtcccccgg cagcgtctcc agctctacct gactctggga gctgcagcct tccagcccct 960

catcatatac tggctgactt tccacctggt ccggtgaccc cctggcccca gatggcactg 1020

agtttttcat tcattgaaga tttgatttcc ttgaaaaaaa aaaaaaaaaa aaaaaaa 1078

<210> 39
<211> 1145
<212> DNA
<213> human

<400> 39
attctctccc cagcttgctg agccctttgc tcccctggcg actgcctgga cagtcagcaa 60

ggaattgtct cccagtgcat tttgccctcc tggctgccaa ctctggctgc taaagcggct 120

gccacctgct gcagtctaca cagcttcggg aagaggaaag gaacctcaga ccttccagat 180
cgcttcctct cgcaacaaac tatttgtcgc aggaataaag atggctgctg aaccagtaga 240
agacaattgc atcaactttg tggcaatgaa atttattgac aatacgcttt actttatagc 300
tgaagatgat gaaaacctgg aatcagatta ctttggcaag cttgaatcta aattatcagt 360
cataagaaat ttgaatgacc aagttctctt cattgaccaa ggaaatcggc ctctatttga 420
agatatgact gattctgact gtagagataa tgcaccccgg accatattta ttataagtat 480
gtataaagat agccagccta gaggtatggc tgtaactatc tctgtgaagt gtgagaaaat 540
ttcaactctc tcctgtgaga acaaaattat ttcctttaag gaaatgaatc ctcctgataa 600
catcaaggat acaaaaagtg acatcatatt ctttcagaga agtgtcccag gacatgataa 660
taagatgcaa tttgaatctt catcatacga aggatacttt ctagcttgtg aaaaagagag 720
agaccttttt aaactcattt tgaaaaaaga ggatgaattg gggggatagat ctataatgtt 780
cactgttcaa aacgaagact agctattaaa atttcatgcc gggcgcagtg gctcacgcct 840
gtaatcccag ccctttggga ggctgaggcg ggcagatcac cagaggtcag gtgttcaaga 900
ccagcctgac caacatggtg aaacctcatc tctactaaaa atacaaaaaa ttagctgagt 960
gtagtgacgc atgccctcaa tcccagctac tcaagaggct gaggcaggag aatcacttgc 1020
actccggagg tagaggttgt ggtgagccga gattgcacca ttgcgctcta gcctgggcaa 1080
caacagcaaa actccatctc aaaaaataaa ataaataaat aaacaaataa aaaattcata 1140
atgtg 1145

<210> 40
<211> 1840
<212> DNA
<213> human

<400> 40
ggaagaagag gctctcccgc gggagccctt gaggaccaag tttgcggcca cttctgcagg 60
cgtcccttct tagctctcgc ccgccccttt ctgcagccta ggcggcccgg gttctcttct 120
cttcctcgcg cgcccagccg cctcggttcc cggcgaccat ggtgacgatg gaggagctgc 180
gggagatgga ctgcagtgtg ctcaaaaggc tgatgaaccg ggacgagaat ggcggcggcg 240
cgggcggcag cggcagccac ggcaccctgg ggctgccgag cggcggcaag tgcctgctgc 300
tggactgcag accgttcctg gcgcacagcg cgggctacat cctaggttcg gtcaacgtgc 360
gctgtaacac catcgtgcgg cggcgggcta agggctccgt gagcctggag cagatcctgc 420

```
ccgccgagga ggaggtacgc gcccgcttgc gctccggcct ctactcggcg gtcatcgtct    480
acgacgagcg cagcccgcgc gccgagagcc tccgcgagga cagcaccgtg tcgctggtgg    540
tgcaggcgct gcgccgcaac gccgagcgca ccgacatctg cctgctcaaa ggcggctatg    600
agaggttttc ctccgagtac ccagaattct gttctaaaac caaggccctg gcagccatcc    660
cacccccggt tccccccagc gccacagagc ccttggacct gggctgcagc tcctgtggga    720
ccccactaca cgaccagggg ggtcctgtgg agatccttcc cttcctctac ctcggcagtg    780
cctaccatgc tgcccggaga gacatgctgg acgccctggg catcacggct ctgttgaatg    840
tctcctcgga ctgcccaaac cactttgaag gacactatca gtacaagtgc atcccagtgg    900
aagataacca caaggccgac atcagctcct ggttcatgga agccatagag tacatcgatg    960
ccgtgaagga ctgccgtggg cgcgtgctgg tgcactgcca ggcgggcatc tcgcggtcgg   1020
ccaccatctg cctggcctac ctgatgatga agaaacgggt gaggctggag gaggccttcg   1080
agttcgttaa gcagcgccgc agcatcatct cgcccaactt cagcttcatg ggcagctgc    1140
tgcagttcga gtcccaggtg ctggccacgt cctgtgctgc ggaggctgct agcccctcgg   1200
gacccctgcg ggagcggggc aagacccccg ccacccccac ctcgcagttc gtcttcagct   1260
ttccggtctc cgtgggcgtg cactcggccc ccagcagcct gccctacctg cacagcccca   1320
tcaccacctc tcccagctgt tagagccgcc ctgggggccc cagaaccaga gctggctccc   1380
agcaagggta ggacgggccg catgcgggca gaaagttggg actgagcagc tgggagcagg   1440
cgaccgagct ccttccccat catttctcct tggccaacga cgaggccagc cagaatggca   1500
ataaggactc cgaatacata ataaaagcaa acagaacact ccaacttaga gcaataacgg   1560
ctgccgcagc agccagggaa gaccttggtt tggtttatgt gtcagtttca cttttccgat   1620
agaaatttct tacctcattt ttttaagcag taaggcttga agtgatgaaa cccacagatc   1680
ctagcaaatg tgcccaacca gctttactaa agggggagga agggagggca aagggatgag   1740
aagacaagtt tcccagaagt gcctggttct gtgtacttgt ccctttgttg tcgttgttgt   1800
agttaaagga atttcatttt ttaaaaaaaa aaaaaaaaaa                         1840
```

<210> 41
<211> 2263
<212> DNA
<213> human

<400> 41

```
aggaagtagg gagcggggtg gcaggggggg gacccgccgc ggctgctgcc accgccgcca     60
```

ccaccgcctc tgctcgtggc gtgggaaagg aggtgtgagt cccgggcgcg agccgcggcg 120
gcgccgctgc gggagggtcg gcggtgggaa ggcgatggcg gatttagata aactcaacat 180
cgacagcatt atccaacggc tgctggaagt gagagggtcc aagcctggta agaatgtcca 240
gcttcaggag aatgaaatca gaggactgtg cttaaagtct cgtgaaatct ttctcagtca 300
gcctatccta ctagaacttg aagcaccact caaaatatgt ggtgacatcc atggacaata 360
ctatgatttg ctgcgacttt ttgagtacgg tggtttccca ccagaaagca actacctgtt 420
tcttggggac tatgtggaca ggggaaagca gtcattggag acgatctgcc tcttactggc 480
ctacaaaata aaatatcctg agaatttttt tcttctcaga gggaaccatg aatgtgccag 540
catcaacaga atttatggat tttatgatga atgtaaaaga agatacaaca ttaaactatg 600
gaaaactttc acagactgtt ttaactgttt accgatagca gccatcgtgg atgagaagat 660
attctgctgt catggaggtt tatcaccaga tcttcaatct atggagcaga ttcggcgaat 720
tatgcgacca actgatgtac cagatcaagg tcttctttgt gatcttttgt ggtctgaccc 780
cgataaagat gtcttaggct ggggtgaaaa tgacagagga gtgtccttca catttggtgc 840
agaagtggtt gcaaaatttc tccataagca tgatttggat cttatatgta gagcccatca 900
ggtggttgaa gatggatatg aatttttttgc aaagaggcag ttggtcactc tgttttctgc 960
gcccaattat tgcggagagt ttgacaatgc aggtgccatg atgagtgtgg atgaaacact 1020
aatgtgttct ttcagatttt taaagcctgc agagaaaaag aagccaaatg ccacgagacc 1080
tgtaacgcct ccaaggggta tgatcacaaa gcaagcaaag aaatagatgt cgttttgaca 1140
ctgcctagtc gggacttgta acatagagta tataaccttc atttttaaga ctgtaatgtg 1200
tactggtcag cttgctcaga tagatctgtg tttgtggggg cccttccttc catttttgat 1260
ttagtgaatg gcatttgctg gttataacag caaatgaaag actcttcact ccaaaaagaa 1320
aagtgttttg ttttttaatt ctctgttcct tttgcaaaca attttaatga tggtgttaaa 1380
gctgtacacc ccaggacagt ttatcctgtc tgaggagtaa gtgtacaatt gatctttttt 1440
aattcagtac aacccataat catgtaaatg ctcattttct ttaggacata aagagagccc 1500
tagggtgctc tgaatctgta catgttcttg tcataaaatg catactgttg atacaaacca 1560
ctgtgaacat tttttatttg agaattttgt ttcaaaggga ttgctttttc ctctcattgt 1620
cttgttatgt acaaactagt ttttatagct atcaacatta ggagtaactt tcaaccttgc 1680
cagcatcact ggtatgatgt atatttaatt aaagcacact tttccccgac cgtatactta 1740
aaatgacaaa gccattcttt taaatatttg tgactctttc ctaaagccaa agtttctgtt 1800

gaattatgtt ttgacacacc cctaagtaca aggtggtatg gttgtataca catgctgcct 1860

tcttggggat tcaaaaacag gtttttgatt ttgaatagca attagtgata tagtgctgtt 1920

taagctacta acgataaaag gtaataacat tttatacaat ttccatatag tctattcatt 1980

aagtaatctt tttacagttg catcaggcct gaacccgtcc attcagaaag cttcaaatta 2040

tagaaacaat actgttctat acgagtgacc gattatgctt tctttggcct acattcttta 2100

ttctgcggtg aagttgaggc ttataagtta aaacaaagga actaacttac tgtccaccag 2160

tttatacaga actcacagta cctatgactt ttttaaacta agatctgtta aaaaagaaat 2220

ctgtttcaac agatgaccgt gtacaatacc gtgtggtgaa aat 2263

<210> 42
<211> 6755
<212> DNA
<213> human

<400> 42
gagagaagcc tgatatgtaa cccaggcggg tgggagcctc agtctgtcgg gctgaggtct 60

ggcatctaca aagcctcttg gccgtgttct gaacttgaag cctggaggag ttctctgctc 120

agcacagcca aggaacagaa ttagaagaaa aggaaccctg gcctgaggca ggtgacaaac 180

attaccaccc cagctgtgca cgatgcagca gatgcaacca gatgttcaca gaaggagagg 240

aaatgtatct tcaaggctcc accgtttggc atcccgactg taagcaatct acgaagaccg 300

aggaaaagct gcggcctacc aggacatcct cggaaagtat ttattctagg ccaggctcca 360

gtattcctgg ctcaccaggt catactatct atgcaaaagt agacaatgag atcctggatt 420

acaaggattt agcagccatt ccgaaggtca aggcaattta tgacattgaa cgtccagatc 480

ttattaccta tgagcctttc tacacttcgg gctatgatga caaacaggag agacagagcc 540

ttggagagtc tccgaggact ttgtctccta ctccatcagc agaagggtac caggatgttc 600

gggatcggat gatccatcgg tccacgagcc agggctccat caactcccct gtgtacagcc 660

gccacagcta cactccaacc acgtcccgct ctccccagca tttccacaga cctggcaatg 720

agccgtccag cggccggaac tcccctctcc cttaccggcc agacagccgc cctctaactc 780

caacttacgc tcaggcccct aaacatttcc atgttccaga tcaaggaatc aacatttacc 840

gaaagccacc catctacaaa cagcatgctg ccttggcagc ccagagcaag tcctcagaag 900

atatcatcaa gttttccaag ttcccagcag cccaggcacc agaccccagc gagacaccag 960

agattgagac ggaccactgg cctggtcccc cctcatttgc tgtcatagga cctgacatga 1020

aacgcagatc tagtggcaga gaggaagatg atgaggaact tctgagacgt cggcagcttc 1080

aagaagagca attaatgaag cttaactcag gcctgggaca gttgatcttg aaagaagaga 1140

tggagaaaga gagccgggaa aggtcatctc tgttagccag tcgctacgat tctcccatca 1200

actcagcttc acatattcca tcatctaaaa ctgcatctct ccctggctat ggaagaaatg 1260

ggcttcaccg gcctgtttct accgacttcg ctcagtataa cagctatggg gatgtcagcg 1320

ggggagtgcg agattaccag acactcccag atggccacat gcctgcaatg agaatggacc 1380

gaggagtgtc tatgcccaac atgttggaac caaagatatt tccatatgaa atgctcatgg 1440

tgaccaacag agggcgaaac aaaatcctca gagaggtgga cagaaccagg ctggagcgcc 1500

acttagcccc tgaagtgttt cgggaaatct ttggaatgtc catacaggag tttgacaggt 1560

tacctctttg gagacgcaac gacatgaaga aaaaagcaaa actcttctaa gtcccactcg 1620

tgaatggcaa ttagagaaag gactgacagt ggcggtgccc cataggatgt catattgagg 1680

cccaaacttg attggagaat ttgcaaacta ccgtcgctca gcaacaccaa aaagagaaag 1740

tctggttaaa acaccatgag tcaaatgtcg ggccagccaa cagtaacact tgccaagaag 1800

catggcgtag aaatttctat gttccgaaac acagagtagt gtccacatgt gaccttttca 1860

cattacctta tgtatacaac aggagctgcg ttgttttctt ctttttcttt tcctttatct 1920

gttgtccaac aacagtgctg actgtccgga taagagctgg caagtgccct taggatgccg 1980

catgggaaaa atcggttatc ataatttcaa agtataaata tatttattat gtagcgctgc 2040

ggctaagaag gaagagtgag ggtctgtcc atggggtggc agtgatttca cacccgcctt 2100

tcttgaatgg cttcgtgtta ctcagccgtg ccctggcagg aatggaactc catcagggaa 2160

cagggcagct ctgtttgaat ggggtgaaaa acagcaaaat taattcttag caagctcctt 2220

gttctctact gttcaccggg acctggctgg caaatgactt agctattagt atatttaaaa 2280

actgttcaaa agcaaaagag aaggaacagg aatgaaagga aaccttccct taactctttc 2340

tgcttcccat agcaggtctg gttcctgctc cccgcctagg acaggagctg tctggagctc 2400

cacctttgca aagaaaggaa aaacacaaat tgcccccaga ttggcccagt ggcatgccat 2460

taggtgattt tgcgtagggg ccatatcatt catggccaaa aaaaaccaaa aagcaaaaaa 2520

caaaaacaag tcccatgtcc tactgactct agttcttcca gtcgttatcc tctgctgtct 2580

ctctggcatc ctatgaatca gatcaagccc atgctgttgt tggtttttaa ggtttctttc 2640

aataataggc taaggaaaga catgtttttc tcttttaaat ctctgcaact ccaaagtaga 2700

ctccttcgaa cgtatttaat ttggcctttt cagctttctt cttgcccagc tctgtcgaat 2760
tcatgggtgt gtgtgcacat gttggtttca ctcacccaga gtaattttgt gagcatgcat 2820
gtgcttttta atttctgctt gaatgtttgt cctgctgtgt tgcagcttct aaagacattg 2880
tcaccgagtg tgtgtgactc aaagatcaag agtgaggcta aactgcgacc ccaacatcac 2940
ttccttcatg gaaaggtgtt ggcgctgatg tagctcatgg aaggatcaga ctgggaacca 3000
cagaggaagg atgaggtggg atgtggaggt cagagcatct cacgagagtc cctcaggctc 3060
tgcaacacta gattcgaata tgagcctagg gtttccccaa cacgtgcgtg catatgaaac 3120
cacatcatga ttcccgacgt gagttagatg agggagagtt tgtgtaactc cacacccagg 3180
agggtaagcc actttattat catgctttgg ggctgtgctt aagaatttaa gctctgtttt 3240
aaagccgaag aagaaaacat ttgaatttac cttcatatgc cactaagaac aaaagctctg 3300
aagttcttcc tgctggcacc aaatattagg attacaagct gtctctctga caccagtggt 3360
aaaaaagcaa gtagaatagt ttctgagtct gtgttgcagc ctctgacacg tggctcactg 3420
ctgtcatttg gggggtggaa tgaacagtca ctgttctata gcatcctcct gtgctcttac 3480
ctaaggaatc taaattgtcc tagccttcca ttgctatgca ctgaatgagg actactctcg 3540
tgtgtgtgtg tgtgtgtgtg tgtgtgtgtg tgtgtgtgtg tgtgtgttgg atctactaac 3600
atgtttagca aatggctctg acagggtggc catgagcaga tgagaccagc tcttgcttgg 3660
ttggaagcca cactgcagtt tgcagtttag cctttggtgt ctcagtggcc tcttgcttat 3720
tccatgcttt ttctatccca tcctctctct gtgccctcct gcaatggcag ccttggcaca 3780
atgcctgggt gtcctcatcc ttgctttact ctggaaagtg tggggatgga gaggatggtg 3840
catttccagt gcttccttac ctgtcaataa caatggtggc cacctgggtc ccagcacttg 3900
gctggtggag agattttttt cattatggag ctaataaagg gtccctacgt tttacattga 3960
ttaatgaatt atcacccaga catgtgcaga tgaaatgtca atataagaaa aatcaaggta 4020
tttgggaaac atgggcataa tcaggtagtt ttgctaaaat tcctcttcct tggtctacat 4080
aaagaagaca tgggtctgtc atagaatggg gacattagac tgaaaactgg gacaaccccc 4140
ttccttattt tatagagact aggagttaag gggcctgcct aagattgcag tgataaagaa 4200
ctgaacccta aatcagctcc cactaatgaa accctgcttt ccttgtatct tttaaactca 4260
gttctgcatc caacttgaga agaaaaaaag cttctttcca tatcaagtac catatgagtt 4320
catttaaact gcaccttgca gaaatgcatt gccagaaagc accagtagcc tcctatctgc 4380
aagcagagta gtgctctgct ctggggaggg gtcattggaa accataatgc agagtgggcc 4440

ccctactcca tttcccagca aaaggctcca gctggaggga tgggttgtgg ggcaacctgg 4500
ttcctgctaa ctgccagatt gaatgtgtgg gctagaatgc ctgcgcattt agttaaactg 4560
ggctcagcat gcttgtcctc aaaatgtcca tcctggtcac agcacacaag atggctattg 4620
gtctgctttt accctaccct gtactataca tgaaaattcc agttattaac acactcaaac 4680
tggtggagct tgttcaccct aggaagggga ttgtatatat ggcaggcttc cctggtgccg 4740
atgtaaaggg ctacatttgg gaacatttga cttccttggg actcttaagt gcatactgat 4800
ggcatgaagt aaaaggggcc tcaatgatga taggaaaatc agttctttta aaatttcttc 4860
aagaaaatcc aggctatcac atagtctttc tgtgtgactt attaggagat aggaagagca 4920
ttgggaaact tgcacagcta gctatgcatc tacattttgg tttggggtta gttatgaaat 4980
gttcttaata tgacgtgttc aataacttca cataaacttc ctgttctcca aaacctcaaa 5040
gagatagagt taatgagttg ttgttttttt ttaaatgggg gtagttttct atctgtcatg 5100
ggctctagca tctactccgc tacccaattc tgtcatctcc aagctgagtt tcctcttctg 5160
aggcagaggc tggagcagtt ctttttcagt tctcatcctc tccatcccaa tccagtatat 5220
caatcaactc taactcggag acgtctagct ggcaatgttt ctaaaacttt cactggattt 5280
ctttagacat tgaagcaaac atttttttct aagaattgct tctcagatga tgatatcaaa 5340
tgtatatgct tttgcaagtt tgaaaagttc aaattaacca cttttgacta ggtaagtctt 5400
tctaaaaacc atttaaagct aactgggtct tagcatcctc ctgtgtatgg aagagacagg 5460
tgaccgctcc aggttgggtg ctcacagaac ccttttcctg actctcatgg aagatggtgg 5520
aaggaaaata gactgtctca tcaaccctcc tgtgtcctct gaagcaatct cagtttttat 5580
taaccacctc ttctgtgttt ctggtagcta tttaacctgt atttaatctg tacttcctat 5640
gccagcctca attttatttg atttttaaaa ttattctctt ctaaccaatg aagtgtttgt 5700
cagtatgccc caaagcttgc tcttttgtgc tccctttttga ataactttct atccagaaaa 5760
agagattatt tgggacttga gatttgcagt gataccaact tatagcaatg atgtacttta 5820
agggaactac ccaactatgt tgtgatagaa gaaagagaaa ccttcacttt ggcatttttt 5880
ttaatcactg tttatttttc tgtttgcggc ccaggaagca gtgggaggtg gtggcagata 5940
tgctttgcat atggattgtt atgtttttat ttgggcaagt ttaatcatgg aaaactcaaa 6000
aagaaggggg gaaatggtca gtttaagcca aaagaaactt tctaaacaat gtataggtac 6060
acagcaaaat taaacaaatc caacaatttc tgaagcttag tgtaattgag tggtggttgt 6120

```
tattcaataa aattattccc aaaagtgttt ctcctaagag tgcagttccc atgagtcact   6180
tcctgaaccc attgaccaaa ggtggacaga gacaatcctg tagaccttga cattcagaaa   6240
gatgtgagct gcttactgat catatatgca tacgtttctt tacagcagag gaaaccattg   6300
tccacaaaac tgatgttctt ttggggtttt atgtacagac ttgtccaatc atgtgtgtgg   6360
ttcctgcgag ttgctgatga ctccgcattg aagctctctg agttctttga ttttaagttg   6420
ggtttatgga attttttcaa atgttggaag gcgtgtggtt cttcctgccc tccctcccct   6480
tttggaaata tgaaagcaaa tgtttagaag aattcctttt gaaaagctgt gtcgtgttcc   6540
ctgtgaaact gagcaggtgt gtgttggcgc gctaagtgcc acatgcttgt gtgtagagga   6600
ggaggtggcc ctgccggctc cgcgctgctg tgcctgtgat ccctacctgc tccccgctcc   6660
tgttgccagc agcactcact gcactccttt gtcatatact ctgcatcact gtcatactca   6720
caacttcgtg aataaagttg tgtgctttat tcgtc                              6755
```

```
<210>  43
<211>  1607
<212>  DNA
<213>  human

<400>  43
cctaattctc ctgaggctga gggagggtgg agggtctcaa ggcaacgctg gccccacgac     60
ggagtgccag gagcactaac agtaccctta gcttgctttc ctcctccctc ctttttattt    120
tcaagttcct ttttatttct ccttgcgtaa caaccttctt cccttctgca ccactgcccg    180
taccccttacc cgccccgcca cctccttgct accccactct tgaaaccaca gctgttggca    240
gggtccccag ctcatgccag cctcatctcc tttcttgcta gcccccaaag ggcctccagg    300
caacatgggg ggcccagtca gagagccggc actctcagtt gccctctggt tgagttgggg    360
ggcagctctg ggggccgtgg cttgtgccat ggctctgctg acccaacaaa cagagctgca    420
gagcctcagg agagaggtga gccggctgca ggggacagga ggcccctccc agaatgggga    480
agggtatccc tggcagagtc tcccggagca gagttccgat gccctggaag cctgggagaa    540
tggggagaga tcccggaaaa ggagagcagt gctcacccaa aaacagaaga agcagcactc    600
tgtcctgcac ctggttccca ttaacgccac ctccaaggat gactccgatg tgacagaggt    660
gatgtggcaa ccagctctta ggcgtgggag aggcctacag gcccaaggat atggtgtccg    720
aatccaggat gctggagttt atctgctgta tagccaggtc ctgtttcaag acgtgacttt    780
caccatgggt caggtggtgt ctcgagaagg ccaaggaagg caggagactc tattccgatg    840
```

```
tataagaagt atgccctccc acccggaccg ggcctacaac agctgctata gcgcaggtgt     900
cttccattta caccaagggg atattctgag tgtcataatt ccccgggcaa gggcgaaact     960
taacctctct ccacatggaa ccttcctggg actttgattt tacggatatc ttgcttctgt    1020
tccccatgga gctccgaatt cttgcgtgtg tgtagatgag gggcgggga cgggcgccag    1080
gcattgttca gacctggtcg ggcccactg gaagcatcca gaacagcacc accatctagc    1140
ggccgctcga gggaagcacc cgccggttgg ccgaagtcca cgaagccgcc ctctgctagg    1200
gaaaacccct ggttctccat gccacacctc tctccaggtg ccctctgcct cttcacccca    1260
caagaagcct tatcctacgt ccttctctcc atctatcgga ccccagtttc catcactatc    1320
tccagagatg tagctattat gcgcccgtct acagggggtg cccgacgatg acggtgcctt    1380
cgcagtcaat ttactcttcg ggtcccaagg tttggctttc acgcgctcca ttgccccggc    1440
gtggcaggcc attccaaggc cttccgggct ggaactggtg tcggaggagc ctcgggtgta    1500
tcgtacgccc tggtgttggt gttgcctcac tcctctgagc tcttctttct gatcaagccc    1560
tgcttaaagt taaataaaat agaatgaatg ataaaaaaaa aaaaaaa                  1607
```

```
<210>  44
<211>  5067
<212>  DNA
<213>  human

<400>  44
ctcctcccca tcctctccct ctgtccctct gtccctctga ccctgcactg tcccagcacc      60
atgggaccca cctcaggtcc cagcctgctg ctcctgctac taacccacct ccccctggct     120
ctggggagtc ccatgtactc tatcatcacc cccaacatct tgcggctgga gagcgaggag     180
accatggtgc tggaggccca cgacgcgcaa ggggatgttc cagtcactgt tactgtccac     240
gacttcccag gcaaaaaact agtgctgtcc agtgagaaga ctgtgctgac ccctgccacc     300
aaccacatgg gcaacgtcac cttcacgatc ccagccaaca gggagttcaa gtcagaaaag     360
gggcgcaaca agttcgtgac cgtgcaggcc accttcggga cccaagtggt ggagaaggtg     420
gtgctggtca gcctgcagag cgggtacctc ttcatccaga cagacaagac catctacacc     480
cctggctcca cagttctcta tcggatcttc accgtcaacc acaagctgct acccgtgggc     540
cggacggtca tggtcaacat tgagaacccg gaaggcatcc cggtcaagca ggactccttg     600
tcttctcaga accagcttgg cgtcttgccc ttgtcttggg acattccgga actcgtcaac     660
atgggccagt ggaagatccg agcctactat gaaaactcac cacagcaggt cttctccact     720
```

```
gagtttgagg tgaaggagta cgtgctgccc agtttcgagg tcatagtgga gcctacagag    780
aaattctact acatctataa cgagaagggc ctggaggtca ccatcaccgc caggttcctc    840
tacgggaaga aagtggaggg aactgccttt gtcatcttcg ggatccagga tggcgaacag    900
aggatttccc tgcctgaatc cctcaagcgc attccgattg aggatggctc ggggggaggtt    960
gtgctgagcc ggaaggtact gctggacggg gtgcagaacc tccgagcaga agacctggtg   1020
gggaagtctt tgtacgtgtc tgccaccgtc atcttgcact caggcagtga catggtgcag   1080
gcagagcgca gcgggatccc catcgtgacc tctccctacc agatccactt caccaagaca   1140
cccaagtact tcaaaccagg aatgcccttt gacctcatgg tgttcgtgac gaaccctgat   1200
ggctctccag cctaccgagt ccccgtggca gtccagggcg aggacactgt gcagtctcta   1260
acccagggag atggcgtggc caaactcagc atcaacacac accccagcca gaagcccttg   1320
agcatcacgg tgcgcacgaa gaagcaggag ctctcggagg cagagcaggc taccaggacc   1380
atgcaggctc tgccctacag caccgtgggc aactccaaca attacctgca tctctcagtg   1440
ctacgtacag agctcagacc cggggagacc ctcaacgtca acttcctcct gcgaatggac   1500
cgcgcccacg aggccaagat ccgctactac acctacctga tcatgaacaa gggcaggctg   1560
ttgaaggcgg gacgccaggt gcgagagccc ggccaggacc tggtggtgct gcccctgtcc   1620
atcaccaccg acttcatccc ttccttccgc ctggtggcgt actacacgct gatcggtgcc   1680
agcggccaga gggaggtggt ggccgactcc gtgtgggtgg acgtcaagga ctcctgcgtg   1740
ggctcgctgg tggtaaaaag cggccagtca gaagaccggc agcctgtacc tgggcagcag   1800
atgaccctga agatagaggg tgaccacggg gcccgggtgg tactggtggc cgtggacaag   1860
ggcgtgttcg tgctgaataa gaagaacaaa ctgacgcaga gtaagatctg ggacgtggtg   1920
gagaaggcag acatcggctg caccccgggc agtgggaagg attacgccgg tgtcttctcc   1980
gacgcagggc tgaccttcac gagcagcagt ggccagcaga ccgcccagag ggcagaactt   2040
cagtgcccgc agccagccgc ccgccgacgc cgttccgtgc agctcacgga gaagcgaatg   2100
gacaaagtcg gcaagtaccc caaggagctg cgcaagtgct gcgaggacgg catgcgggag   2160
aaccccatga ggttctcgtg ccagcgccgg acccgtttca tctccctggg cgaggcgtgc   2220
aagaaggtct tcctggactg ctgcaactac atcacagagc tgcggcggca gcacgcgcgg   2280
gccagccacc tgggcctggc caggagtaac ctggatgagg acatcattgc agaagagaac   2340
atcgtttccc gaagtgagtt cccagagagc tggctgtgga acgttgagga cttgaaagag   2400
ccaccgaaaa atggaatctc tacgaagctc atgaatatat ttttgaaaga ctccatcacc   2460
```

acgtgggaga ttctggctgt cagcatgtcg gacaagaaag ggatctgtgt ggcagacccc 2520
ttcgaggtca cagtaatgca ggacttcttc atcgacctgc ggctacccta ctctgttgtt 2580
cgaaacgagc aggtggaaat ccgagccgtt ctctacaatt accggcagaa ccaagagctc 2640
aaggtgaggg tggaactact ccacaatcca gccttctgca gcctggccac caccaagagg 2700
cgtcaccagc agaccgtaac catccccccc aagtcctcgt tgtccgttcc atatgtcatc 2760
gtgccgctaa agaccggcct gcaggaagtg gaagtcaagg ctgccgtcta ccatcatttc 2820
atcagtgacg gtgtcaggaa gtccctgaag gtcgtgccgg aaggaatcag aatgaacaaa 2880
actgtggctg ttcgcaccct ggatccagaa cgcctgggcc gtgaaggagt gcagaaagag 2940
gacatcccac ctgcagacct cagtgaccaa gtcccggaca ccgagtctga gaccagaatt 3000
ctcctgcaag ggaccccagt ggcccagatg acagaggatg ccgtcgacgc ggaacggctg 3060
aagcacctca ttgtgacccc ctcgggctgc ggggaacaga acatgatcgg catgacgccc 3120
acggtcatcg ctgtgcatta cctggatgaa acggagcagt gggagaagtt cggcctagag 3180
aagcggcagg gggccttgga gctcatcaag aaggggtaca cccagcagct ggccttcaga 3240
caacccagct ctgcctttgc ggccttcgtg aaacgggcac ccagcacctg gctgaccgcc 3300
tacgtggtca aggtcttctc tctggctgtc aacctcatcg ccatcgactc ccaagtcctc 3360
tgcggggctg ttaaatggct gatcctggag aagcagaagc ccgacggggt cttccaggag 3420
gatgcgcccg tgatacacca agaaatgatt ggtggattac ggaacaacaa cgagaaagac 3480
atggccctca cggcctttgt tctcatctcg ctgcaggagg ctaaagatat ttgcgaggag 3540
caggtcaaca gcctgccagg cagcatcact aaagcaggag acttccttga agccaactac 3600
atgaacctac agagatccta cactgtggcc attgctggct atgctctggc ccagatgggc 3660
aggctgaagg ggcctcttct taacaaattt ctgaccacag ccaaagataa gaaccgctgg 3720
gaggaccctg gtaagcagct ctacaacgtg gaggccacat cctatgccct cttggcccta 3780
ctgcagctaa aagactttga ctttgtgcct cccgtcgtgc gttggctcaa tgaacagaga 3840
tactacggtg gtggctatgg ctctacccag gccaccttca tggtgttcca agccttggct 3900
caataccaaa aggacgcccc tgaccaccag gaactgaacc ttgatgtgtc cctccaactg 3960
cccagccgca gctccaagat cacccaccgt atccactggg aatctgccag cctcctgcga 4020
tcagaagaga ccaaggaaaa tgagggtttc acagtcacag ctgaaggaaa aggccaaggc 4080
accttgtcgg tggtgacaat gtaccatgct aaggccaaag atcaactcac ctgtaataaa 4140

ttcgacctca aggtcaccat aaaaccagca ccggaaacag aaaagaggcc tcaggatgcc 4200

aagaacacta tgatccttga gatctgtacc aggtaccggg gagaccagga tgccactatg 4260

tctatattgg acatatccat gatgactggc tttgctccag acacagatga cctgaagcag 4320

ctggccaatg gtgttgacag atacatctcc aagtatgagc tggacaaagc cttctccgat 4380

aggaacaccc tcatcatcta cctggacaag gtctcacact ctgaggatga ctgtctagct 4440

ttcaaagttc accaatactt taatgtagag cttatccagc ctggagcagt caaggtctac 4500

gcctattaca acctggagga aagctgtacc cggttctacc atccggaaaa ggaggatgga 4560

aagctgaaca agctctgccg tgatgaactg tgccgctgtg ctgaggagaa ttgcttcata 4620

caaaagtcgg atgacaaggt caccctggaa gaacggctgg acaaggcctg tgagccagga 4680

gtggactatg tgtacaagac ccgactggtc aaggttcagc tgtccaatga ctttgacgag 4740

tacatcatgg ccattgagca gaccatcaag tcaggctcgg atgaggtgca ggttggacag 4800

cagcgcacgt tcatcagccc catcaagtgc agagaagccc tgaagctgga ggagaagaaa 4860

cactacctca tgtggggtct ctcctccgat ttctggggag agaagcccaa cctcagctac 4920

atcatcggga aggacacttg ggtggagcac tggcctgagg aggacgaatg ccaagacgaa 4980

gagaaccaga aacaatgcca ggacctcggc gccttcaccg agagcatggt tgtctttggg 5040

tgccccaact gaccacaccc ccattcc 5067

<210> 45
<211> 3887
<212> DNA
<213> human

<400> 45
gcgtttgaat ctggtccgag cgcgggaaac ggcgggtccc cgagcccagg gttacaaaat 60

aaatgccatt tgaacagtgc catctgtcat ggaaaaacgt gagacgtttg tacaagccgt 120

gtctaaggag ctggttggag agtttttgca atttgttcaa cttgataaag aggcctctga 180

tcctttcagc ctaaatgaat tactagatga attatcaagg aaacagaaag aagaattatg 240

gcaaaggctg aagaatttat tgacagatgt gttgttagaa agcccagtgg atgggtggca 300

ggtagtggaa gcccagggtg aagacaatat ggaaaccgaa catggctcaa aaatgagaaa 360

aagcatagaa ataatttatg caattacatc tgtgattctt gcttctgtgt ctgtaataaa 420

tgaaagtgag aactacgaag ccctactgga atgtgttatt atattaaatg gtattttata 480

tgcattacct gagtctgaac gaaaactaca gagttctatt caggatttgt gtgttacctg 540

```
gtgggagaaa ggcctgcctg ccaaggaaga cacaggaaag acagcctttg tcatgttact    600
aaggaggagt ctggagacta agacaggtgc agacgtatgt cggctttggc gtatccatca    660
agctttatat tgctttgatt atgatttgga ggaaagtgga gaaattaaag atatgttact    720
tgagtgcttc ataaatatta attatatcaa gaaagaagag ggaagaagat ttcttagttg    780
tctcttcaac tggaatatca acttcatcaa aatgatccac gggaccatta aaaaccagtt    840
acagggatta caaaagtctt tgatggtata cattgcagaa atttatttca gagcttggaa    900
aaaggcttca gggaaaatac tggaggcgat tgaaaatgat tgcatccagg acttcatgtt    960
ccacgggata caccttccga ggaggtctcc agtgcattcc aaagtgcggg aggttctgag   1020
ttactttcac catcaaaaga aagttcggca gggagtggaa gagatgcttt atagattata   1080
taagcccatc ctttggagag gattaaaggc cagaaactct gaagttcgat caaatgctgc   1140
attgttgttt gttgaagcat ttcctattag ggatccaaac cttcatgcta ttgaaatgga   1200
tagtgaaatc cagaaacagt ttgaagagct ctatagcctt ttagaagatc cttacccgat   1260
ggtccgttcc acagggatcc ttggtgtttg taaaataact tctaagtact gggaaatgat   1320
gcccccgacc attcttattg acctcctgaa gaaggtgact ggggaactgg catttgacac   1380
gagctcagct gatgttcgtt gttctgtctt taagtgtctg ccaatgattt tggacaacaa   1440
actgagccac ccattgttag agcagctcct tccagctctc agatacagtc tccacgacaa   1500
ttcggagaaa gtgagggtag cttttgtgga catgctgttg aagatcaaag ctgtgagggc   1560
tgctaagttt tggaaaatat gtcccatgga gcacattctg gttcgtctgg aaactgattc   1620
tcgacctgtg tctcggcgcc tggtgagcct catctttaat tctttcctgc ctgtgaatca   1680
gccggaggag gtctggtgcg agcgctgtgt caccctggtg cagatgaacc acgccgctgc   1740
caggaggttc tatcagtacg cccacgaaca caccgcctgc accaacatag caaagctgat   1800
tcacgttatt cgtcattgct taaatgcctg tatccagagg gcagtgagag agcctccaga   1860
ggacgaggag gaagaggacg gaagggagaa ggagaatgtg actgttctgg acaaaacact   1920
gtcagtaaac gatgttgcat gcatggcagg tttactagaa atcattgtga ttctctggaa   1980
aagtattgac agaagtatgg aaaataataa agaggccaaa ctttacacga ttaacaagtt   2040
tgcctctgtg cttccagagt atctgaaagt atttaaggat gatcgctgca agatcccttt   2100
attcatgcta atgtccttta tgccggcctc tgctgtcccc ccattcagct gtggtgtgat   2160
ttccacgctg agaagccggg aggagggcgc tgtggacaag agctactgca ctttgttgga   2220
ttgcctctgc tcctgggggc aggtggggca cattctggag cttgttgaca actggctgcc   2280
```

cacagagcat gcccaggcca agagcaacac agcttctaaa ggtagggtgc agatccatga 2340

cacacgccca gtcaaacctg aattggcatt ggtctacatt gagtatctgc tgactcatcc 2400

aaagaaccgc gagtgcttgc tctctgctcc tcggaagaaa cttaaccatc ttttgaaagc 2460

ccttgaaacg tcaaaggcag atctggagtc acttctgcag acaccgggtg ggaagcctcg 2520

tggcttcagt gaagcagctg ccccgcgagc ctttggtctc cactgtcgcc tgagcatcca 2580

tcttcagcac aagttctgct cagaaggaaa ggtgtatttg tccatgttgg aagacactgg 2640

cttttggtta gaaagcaaaa ttttatcttt tattcaagat caagaagaag actacctgaa 2700

gcttcatagg gtcatttatc agcaaattat ccagacctac ctgactgtgt gtaaagatgt 2760

tgttatggta ggccttggtg accatcagtt tcagatgcaa ctcttacagc ggagtcttgg 2820

aatcatgcaa acagtgaagg gatttttta tgtttcatta cttcttgaca ttctgaaaga 2880

gataactgga agttccttga ttcagaaaac agattcagat gaagaagttg caatgctgtt 2940

ggacacagtc cagaaagtat ttcagaaaat gttggaatgt attgcacgga gcttcaggaa 3000

gcagccggaa gaaggcctgc ggctgcttta ttctgttcag aggcctcttc atgagttcat 3060

tactgctgtt cagtctcggc acacagacac ccctgtgcac cggggtgtac tttctactct 3120

gatcgctggg cctgtggttg agataagtca ccagctacgg aaggtttctg acgtagaaga 3180

gcttacccct ccagagcatc tttctgatct tccaccattt tcaaggtgtt taataggaat 3240

aataataaag tcttcgaatg tggtcaggtc atttttggat gaattaaagg catgtgtggc 3300

ttctaatgat attgaaggca ttgtgtgcct cacggctgct gtgcatatta tcctggttat 3360

taatgcaggt aaacataaaa gctcaaaagt gagggaggtt gcagccactg ttcacagaaa 3420

actaaagaca ttcatggaaa ttactttgga agaggatagc attgaaagat ttctctatga 3480

atcatcatca agaactctgg gagaactttt gaattcataa ccaagccaac atctccagac 3540

atgtaaaaat agggaaaagt gattcaaatt gaaatgcctg tgtattttcc tattgttttt 3600

aatgttaata acccatataa tagggaaagg gtgggatttt tttgtgggaa tgtgggaagg 3660

tgggggttat ggaggagata actcaaaact tcttcaattt tgcctagtgc ctgcgtaaat 3720

aatatattta atataaagga ctccaggtat gaatggtgta gaaatccatg attccaagaa 3780

aaaacacttt tctagcaaac ctggttgttt ttaaaatgac ttttatatat gtaatattgc 3840

ttggaaacta tgagtaataa agcaatgaca acaaaaaaaa aaaaaaa 3887

<210> 46

<211> 5745
<212> DNA
<213> human

<400> 46
```
cacagggcct gcagagaggg gacccaccgg gcaggatgca ctggcttcca gcaggccacg      60

acatcaacgg tgccctggag ccctccaaca tagacaccag catcctggag gagtacatca     120

gcaaggagga tgcctccgac ctctgcttcc ctgacatctc tgctccagcc agctcggcct     180

cctactccca cgggcagcct gcgatgcctg gctccagcgg ggtccaccac ctgagccccc     240

ctgggggtgg accctccccg gggcgccatg gtcccctccc acccccgggc tacggcaccc     300

cgctgaactg caacaacaac aacggcatgg gcgctgcccc caagcccttc ccggggggca     360

ccgggccccc catcaaggct gagcccaagg ctccctatgc cccaggcaca ctgccggact     420

ctcccccaga ctcgggctcc gaggcctact ccccccagca ggtgaatgag ccccacctcc     480

tgcgcacgat aacccctgag acactgtgcc acgtgggagt gccctcccgc ctggagcatc     540

cgcccccacc tccagcccac ttgccaggcc ccccgccacc cccaccaccc ccacctcact     600

accctgtcct gcagcgggat ctgtacatga aggccgagcc cccgatcccc cactacgctg     660

ccatggggca ggggctggtg cccactgatc ttcaccacac ccagcagtcc cagatgctgc     720

accagctcct gcagcagcac ggagctgagc tccctacaca cccctccaag aagaggaagc     780

actctgaatc ccccccgagc accctcaatg cccagatgct gaatggaatg atcaaacagg     840

agcctgggac cgtgacagcc ctgcctctgc accccactcg agccccatcg ccaccctggc     900

ctccccaggg tccgctctcc ccgggccctg gttccttgcc tctcagcatt gcccgtgtcc     960

agacaccgcc ttggcacccg ccaggtgccc cctccccagg cctcctgcag gacagtgaca    1020

gcctcagtgg ctcctacctg gaccccaact accagtccat caagtggcag cctcatcagc    1080

agaacaagtg ggcgaccctg tacgatgcta actacaagga gctgcccatg ctcacctacc    1140

gcgtggatgc ggacaagggc ttcaactttt cggtgggcga cgacgccttt gtgtgccaga    1200

agaagaacca cttccaggtg acagtgtaca tcggcatgct gggcgagccc aagtacgtca    1260

agacgcccga gggcctcaag cccctcgact gcttctatct gaagctgcac ggagtgaagc    1320

tggaggccct gaaccagtcc attaacatcg agcagtccca gtcagaccgg agcaagcggc    1380

ccttcaaccc ggtcacggtc aatctgcccc ctgagcaggt cacgaaggtg actgtggggc    1440

ggctgcactt cagcgagacc accgctaaca acatgcgtaa gaagggcaag cccaacccgg    1500

accagaggta cttcatgctg gtggtggccc tccaggctca tgcacagaac cagaactaca    1560
```

cgctggccgc ccagatctca gagcgcatca ttgtgcgggc ctccaaccca ggccagttcg 1620
agagcgacag cgatgtgttg tggcagcggg cacaggtgcc cgacaccgtc ttccaccacg 1680
gccgcgtggg catcaacaca gaccggccgg atgaggcgct ggttgtgcac gggaatgtca 1740
aggtcatggg ctcgcttatg caccccctccg acctgcgcgc caaggaacac gtgcaggagg 1800
tggacaccac cgagcaattg aagaggatct cgcgcatgcg gctggtgcac tacagataca 1860
agcccgagtt cgccgccagc gcgggcatcg aggccaccgc gccagagaca ggtgtcatcg 1920
ctcaggaggt gaaggagatc ttgcctgagg ctgtgaaaga caccggagac atggtctttg 1980
ccaatgggaa aaccatagag aacttcctgg tggtgaacaa ggagcgcatc ttcatggaga 2040
acgtaggggc cgtgaaggag ctgtgcaagc tgacagacaa cctggagacg cgcattgatg 2100
agctggagcg ctggagccac aagctggcca agctgcggcg gctcgacagc ctcaagtcca 2160
ccggcagctc gggcgccttc agccatgcag ggagccagtt cagtcgggcg ggcagcgtcc 2220
cccacaagaa gaggcccccc aaggtggcca gcaagtcatc gtccgtggtt ccggaccagg 2280
cctgcatcag ccagcgcttc ctgcagggaa ccatcattgc cctggtggtg gtcatggcct 2340
tcagcgtggt gtccatgtcc acactgtacg tgctgagcct gcgcacagag gaggacctgg 2400
tagacactga tggcaggtcc agccagagct ttgggaccac gcagctccga cagtcccccct 2460
tgaccacggg gctaccaggc atacagccct ctttgctgct ggtgaccacc agcctcacca 2520
gctcggcccc aggttctgct gtccgcacct tggacatgtg ttccagccac ccctgccctg 2580
tcatctgctg ttcctcaccc actaccaacc ctaccactgg tcctagtctt ggccccagct 2640
ttaaccctgg ccatgttctc agcccaagtc ccagccccag caccaaccgc tcaggcccca 2700
gccagatggc ccttctgcca gtcaccaaca tcagagccaa gtcctggggt ctttcagtca 2760
atggcattgg ccactccaag catcacaaga gtctggagcc tctggccagc cctgcagtcc 2820
ccttccctgg ggggcagggc aaagccaaga acagtcccag ccttggtttc catggccggg 2880
cccgccgagg ggccctccag tccagcgtgg gccctgctga gcccacctgg gcccagggcc 2940
agtcagagcc agtgccctcc ctgacctcca tccaggtgct ggagaattcg atgtccatca 3000
cctcccagta ctgtgctcca ggggatgcct gcaggcctgg gaacttcacc taccacatcc 3060
ctgtcagtag tggcacccca ctgcacctca gcctgactct gcagatgaac tcctcctccc 3120
ccgtgtctgt ggtgctgtgc agcctgaggt caaaggagga accatgtgag gaggggagcc 3180
ttccacagag tctccacacc caccaggaca cccagggcac ctctcaccgg tggccaataa 3240
ccatcctgtc cttccgtgaa ttcacctacc acttccgggt ggcactgctg ggtcaggcca 3300

actgcagttc agaggctctc gcccagccag ccacagacta ccacttccac ttctaccgcc 3360
tgtgtgactg agctgccctc ctgaggcagc accacaccag ggaccagggg tgcccaggca 3420
ccccccaaca ctggatgcaa tggtgttaca ctggagcccg ctgcaggcca gctctgctgt 3480
tcactggccc tacccgagac tggtgaaact ggaagtcttc acactggagt tgctgttcca 3540
gctggtcgcc cctcacggca cagagggaac ctgagagcca gagacttctt gggccttcct 3600
gcctgccacc ccctaggggc caggacagga ccagtttacc tctttccaga tatggtggtt 3660
ggagggctgg ttcaggtgcc ctggagggaa ggggaagcct gtggccctga tttgttcaga 3720
gcccattctc ccttgcctcc ccttttgaga ctggagccaa ccccttttgga gagaggacct 3780
gcccaccttt gagatcagca gggggctcgg atccagccct aagagacttg ggtggacccc 3840
catgagtcaa tggagggcag acggctctcc cccttaaagc tgttccctgg gggatggctt 3900
ggtagtggac tttctggggt ttgcctgtta cgccagactc ggacttctaa gctttaagtg 3960
tggcccagga ggtttcttct ccctgggagg gcttggctcc caagaagtcc cagggcagcc 4020
gaggccagcc ctgcctgggt tggagaaact gactttgtgc cttaagtcta ctcagtgcct 4080
ggtgaagcca ccctcagccc ttcacaggcc tgaaccagta ggggccagtg ggccaggtaa 4140
gccctagagc cttgaaccag gaatatccag gaagaggaaa ttccctttga gcccccagat 4200
ggtattgcag cttcactgcc tgcgttcctg ggagcgtctg gagctcacag tgatcagtga 4260
ccacatcatt ctctctgagc agaggagcag gaatccctca agcagcagcc tggtcttggc 4320
tggtgggcag atgcaaatag cttttgctgt tattaatgaa gtaattacta aatgcactta 4380
aaccagggca ggaaggaatg gaaggatgga gctagaaagc tcagagtggg ccagagcagg 4440
ggtgtgacac ttgcaaagac agggctctga ctctgatccc tcccagggag cctccgacac 4500
ccatcccact cccaaccacc aagaccctgg gttagggaag aagttgtatc ttaagtgcca 4560
ccttcaagtt tcttagtggt gcctggtgca ttccgaggct acatccaggc tcatggaagg 4620
agtgtagtat tcatttagcc atgtctgcca tgggtccaga aatgggaaag ggaattgctg 4680
tccttgccct gtggtatgct gccacctctt tgggaagcag gccttgcccc tgtcccacca 4740
ctcattctca gctttgaatg ggaggccttt ctatagtgga ggcctttcct tgaagcctat 4800
gaactgcagg ccccctttttg ccattgatct caaagcactt gtcctcagga tagggaagag 4860
cagggggatg caggaatagc agggatagct tgctcccagc cccctcccca atttggttcc 4920
gttgacatag gaattttacg attcccaaac catgcagggg ctgagccttc cttatgatga 4980

ctttgttctc cctcccactg ggggaatcct ccctatgcct taaaactgcc gagccccact 5040
ccatgtaata ggattcctgg gcttcctcaa tgggggttca tgttcttgga ctgcgggccc 5100
tcagtcctta actggaaagt gaccgtccac tgccccatgg agcccatctg gacacagcac 5160
agccccaaaa ccgttagcag ctggctctgt ttccaagcct ggggaggggt tcctcagtgc 5220
aggagttggg gacaggctgg ggatccaagc tgcttgaggg ggtcaacctt ggaccaaagt 5280
tgccttaagc ctgtggtaaa agggcttcag ggaaggtaag tgggccacct gctggaagct 5340
gccagctgcc cggctggcaa tggtgtgagt gtcttggccc tgtccctgcc ctggggtcca 5400
gcaggtcatc cctcccttct tctctctcct ttggcgtttg ttcctgtagt cactgggcta 5460
atctccccct agcttcaagc tgtacatagg gcctcccagt gcaaatcctc ctgcccatac 5520
cgtgcaccct tagaagcctg cgtgtgcata gagcgccccc tacttcccag ttaactccca 5580
gttcttctcc ctgagcttgg tatttgtcat gtgccaactc tgactctgag gtgggcagtg 5640
agggaagcag ccccgggcct gcttgcttcc tgtccccgaa atgttcgttt cttctgaagt 5700
aaatatacat atataaataa atgtataaat actgctttgt atctg 5745

<210> 47
<211> 5261
<212> DNA
<213> human

<400> 47
ctcaggcacg tctagatttt cccacttaca taggtcttgt taagaaacca gtcctgcctt 60
cttgccactc gtgtctttcg atggctccct tcccgaagtc ccgctgcctc taagcggagt 120
gttaagcggg gctctcggaa gccggtggac cgagatttcc ccgggtgggg cgggcgcggt 180
gtagcggccc gcgggctgac ttgctcccgg ctgtccccccg gccccagcga ccatgccccg 240
taaaggcacc cagccctcca ctgcccggcg cagagaggaa gggccgccgc cgccgtcccc 300
tgacggcgcc agcagcgacg cggagcctga gccgccgtcc ggccgcacgg agagcccagc 360
caccgccgca gagactgcaa gtgaggaact tgataataga agtttagaag agattttgaa 420
cagcattcct cctcccccgc ctccagcaat gaccaatgaa gctggagctc ctcggcttat 480
gataactcat attgtaaacc agaacttcaa atcctatgct ggggagaaaa ttctgggacc 540
tttccataag cgcttttcct gtattatcgg gccaaatggc agtggcaaat ccaatgttat 600
tgattctatg ctttttgtgt ttggctatcg agcacaaaaa ataagatcta aaaaactctc 660
agtattaata cataattctg atgaacacaa ggacattcag agttgtacag tagaagttca 720

ttttcaaaag ataattgata aggaagggga tgattatgaa gtcattccta acagtaattt 780
ctatgtatcc agaacggcct gcagagataa tacttctgtc tatcacataa gtggaaagaa 840
aaagacattt aaggatgttg gaaatcttct tcgaagccat ggaattgact tggaccataa 900
tagattttta attttacagg gtgaagttga acaaattgct atgatgaaac caaaaggcca 960
gactgaacac gatgagggta tgcttgaata tttagaagat ataattggtt gtggacggct 1020
aaatgaacct attaaagtct tgtgtcggag agttgaaata ttaaatgaac acagaggaga 1080
taagttaaac agggtaaaga tggtggaaaa ggaaaaggat gccttagaag gagagaaaaa 1140
catagctatc gaatttctta ccttggaaaa tgaaatattt agaaaaaaga atcatgtttg 1200
tcaatattat atttatgagt tgcagaaacg aattgctgaa atggaaactc aaaaggaaaa 1260
aattcatgaa gataccaaag aaattaatga gaagagcaat atactatcaa atgaaatgaa 1320
agctaagaat aaagatgtaa aagatacaga aaagaaactg aataaaatta caaaatttat 1380
tgaggagaat aaagaaaaat ttacacagct agatttggaa gatgttcaag ttagagaaaa 1440
gttaaaacat gccacgagta aagccaaaaa actggagaaa caacttcaaa aagataaaga 1500
aaaggttgaa gaatttaaaa gtatacctgc caagagtaac aatatcatta atgaaacaac 1560
aaccagaaac aatgccctcg agaaggaaaa agagaaagaa gaaaaaaaat taaaggaagt 1620
tatggatagc cttaaacagg aaacacaagg gcttcagaaa gaaaaagaaa gtcgagagaa 1680
agaacttatg ggtttcagca aatcggtaaa tgaagcacgt tcaaagatgg atgtagccca 1740
gtcagaactt gatatctatc tcagtcgtca taatactgca gtgtctcaat taactaaggc 1800
taaggaagct ctaattgcag cttctgagac tctcaaagaa aggaaagctg caatcagaga 1860
tatagaagga aaactccctc aaactgaaca agaattaaag gagaaagaaa aagaacttca 1920
aaaacttaca caagaagaaa caaactttaa aagtttggtt catgatctct ttcaaaaagt 1980
tgaagaagca aagagctcat tagcaatgaa tcgaagtagg gggaaagtcc ttgatgcaat 2040
aattcaagaa aaaaaatctg gcaggattcc aggaatatat ggaagattgg gggacttagg 2100
agccattgat gaaaaatacg acgtggctat atcatcctgt tgtcatgcac tggactacat 2160
tgttgttgat tctattgata tagcccaaga atgtgtaaac ttccttaaaa gacaaaatat 2220
tggagttgca acctttatag gtttagataa gatggctgta tgggcgaaaa agatgaccga 2280
aattcaaact cctgaaaata ctcctcgttt atttgattta gtaaaagtaa aagatgagaa 2340
aattcgccaa gctttttatt ttgctttacg agatacctta gtagctgaca acttggatca 2400
agccacaaga gtagcatatc aaaaagatag aagatggaga gtggtaactt tacaggggaca 2460

```
aatcatagaa cagtcaggta caatgactgg tggtggaagc aaagtaatga aaggaagaat   2520
gggttcctca cttgttattg aaatctctga agaagaggta aacaaaatgg aatcacagtt   2580
gcaaaacgac tctaaaaaag caatgcaaat ccaagaacag aaagtacaac ttgaagaaag   2640
agtagttaag ttacggcata gtgaacgaga aatgaggaac acactagaaa aatttactgc   2700
aagcatccag cgtttaatag agcaagaaga atatttgaat gtccaagtta aggaacttga   2760
agctaatgta cttgctacag cccctgacaa aaaaaagcag aaattgctag aagaaaacgt   2820
tagtgctttc aaaacagaat atgatgctgt ggctgagaaa gctggtaaag tagaagctga   2880
ggttaaacgc ttacacaata ccatcgtaga aatcaataat cataaactca aggcccaaca   2940
agacaaactt gataaaataa ataagcaatt agatgaatgt gcttctgcta ttactaaagc   3000
ccaagtagca atcaagactg ctgacagaaa ccttcaaaag gcacaagact ctgtcttgcg   3060
tacagagaaa gaaataaaag atactgagaa agaggtggat gacctaacag cagagctgaa   3120
aagtcttgag gacaaagcag cagaggtcgt aaagaataca aatgctgcag aggaatcctt   3180
accagagatc cagaaagaac atcgcaatct gcttcaagaa ttaaaagtta ttcaagaaaa   3240
tgaacatgct cttcaaaaag atgcacttag tattaagttg aaacttgaac aaatagatgg   3300
tcacattgct gaacataatt ctaaaataaa atattggcac aaagagattt caaaaatatc   3360
actgcatcct atagaagata atcctattga agagatttcg gttctaagcc cagaggatct   3420
tgaagcgatc aagaatccag attctataac aaatcaaatt gcacttttgg aagcccggtg   3480
tcatgaaatg aaaccaaacc tcggtgccat cgcagagtat aaaaagaagg aagaattgta   3540
tttgcaacgg gtagcagaat tggacaaaat tacttatgaa agagacagtt ttagacaggc   3600
atatgaagat cttcggaaac aaaggcttaa tgaatttatg gcaggttttt atataataac   3660
aaataaatta aaggaaaatt accaaatgct tactttggga ggggacgccg aactcgagct   3720
tgtagacagc ttggatcctt tctctgaagg aatcatgttc agtgttcgac cacctaagaa   3780
aagttggaaa aagatcttca acctttcggg aggagagaaa acacttagtt cattggcttt   3840
agtatttgct cttcaccact acaagcccac tccccttac ttcatggatg agattgatgc   3900
agcccttgat tttaaaaatg tgtccattgt tgcattttat atatatgaac aaacaaaaaa   3960
tgcacagttc ataataattt ctcttcgaaa taatatgttt gagatttcgg atagacttat   4020
tggaatttac aagacataca acataacaaa aagtgttgct gtaaatccaa aagaaattgc   4080
atctaaggga ctttgttgaa ctttatgctg aagattcttc aagttgattc agtgtattac   4140
```

tgattttttt ctatttgtaa aggattatga gttgtataaa atacatactc cctaaactag 4200

atcatgaaac tggtttctgt tttatgcagt tgtcatttgt aaagtctaat aaaatattct 4260

ctataattgc ttctagatta caaaaatatg acaatcttgt aagtagcaga ctatggagaa 4320

aaatgagtta cctggagggt caggtaactt gccaaactaa aaagtatgtt agttgaggca 4380

aagtcctaag caaggttgtg ctatcaaggc tcagcatacc ttcgtgggcc tttgatttac 4440

caacactgga aatgcctgcc aactaatctt ggatagattc tttaaggcat tccacttagc 4500

ttgccagttg agacaatcac cacagttatt acccaaatac tatgaacata tttttgtaaa 4560

ccagtcattc tgaattatag tgatgagaat ttaaatatat gcttttctag aatttgatgt 4620

ttgaccattt atgacttaat taccagagag ccagtaaatt aggacagtgt ttcaacaagc 4680

ctaggctatc tcgtaagttg aaaaatatcc cactatagtt gcttcatgag tatgaagtaa 4740

gatggcctct gatttacact ggttcaattt acaaattttc aactttatga taggtttatc 4800

cgggtactaa atgcatttca acttgatagt ttcaacttat gataggttta ccaggatgta 4860

gtcccactgt tgaggagcat ctatttaggg gttaattact ttagtaataa gtggaaagta 4920

agataccttg agtaatgttt gcctataaaa ttgtcagcgt atttttacac tattggctca 4980

agaatgttat aatgctaagg gacataagtt ggcaaccact tggtttttgg aaggactttc 5040

ggtattgtat tagaagtctg ccctagctgt taaatttctg ggtatttatc ctaaggaatt 5100

aattaaagag ttaattgttc ctttcttcag tgggccattg ttttagatat ttaaaaaatc 5160

caacagtttc tatcataatg taactgtaaa aatgtaaaca cattattagc atggactttt 5220

aaataaagat ttaaagaaag caaaaaaaaa aaaaaaaaaa a 5261

<210> 48
<211> 924
<212> DNA
<213> human

<400> 48
atgctgaata caacctcagt caccgaattt ctcctcttgg gagtgacaga cattcaagaa 60

ctgcagcctt ttctcttcgt ggttttcctc accatctact tcatcagtgt gactgggaat 120

ggagccgttc tgatgattgt catctccgat cctagactcc attcccttat gtatttcttc 180

ctgggaaacc tgtcctacct ggatatctgt tactctacgg tgacactgcc aaaaatgctg 240

cagaactttc tctctacaca caaagcaatt tctttcttgg gatgcataag ccagcttcat 300

ttcttccact ccctgggcag cacggagtcc atgttgttcg ccgtgatggc atttgacctc 360

tctgtggcta tctgcaagcc acttcgctac actgtcatca tgaaccctca gctctgtacc 420

cagatggcca tcacaatctg ggtcattggt tttttccatg ccctgctgca ctccgtaatg 480

acttctcgct tgaacttctg tggttccaac cgtatccatc attttctctg tgatattaag 540

ccattgctaa agctggcctg tgggaacact gagcttaatc agtggctact cagtactgtc 600

acggggacaa ttgccatggg ccccttcttt ctgacacttc tctcctattt ctacattatc 660

acttatctct tcttcaagac ccgttcttgt agcatgctct gtaaagcact gtccacttgt 720

gcctcccact tcatggtagt tattcttttc tatgcacctg ttcttttcac ctatatccat 780

cctgcgttag agagcttcat ggaccaggac cggattgttg ccatcatgta cactgtggtc 840

actcctgtac taaacccact gatctatact ttgaggaaca aggaagtgaa gggggccttg 900

ggtagagtga tcagaaggct ttga 924

<210> 49
<211> 2496
<212> DNA
<213> human

<400> 49
cgagtgcatc tggaatacgc agagtcagta agaccatggc tacgtcctcg atgtctaagg 60

gttgctttgt ttttaagcca aactccaaaa agagaaagat ctctctgcca atagaggact 120

attttaacaa agggaaaaat gagcctgagg acagtaagct tcgattcgaa acttatcagt 180

tgatatggca gcagatgaaa tctgaaaatg agcgactaca agaggaatta aataaaaact 240

tgtttgacaa tctgattgaa tttctgcaaa aatcacattc tggattccag aagaattcaa 300

gagacttggg cggtcaaata aaactcagag aaattccaac tgctgctctt gttcttggtg 360

tgaatgtcac agatcatgat ttgacattcg gaagtctaac agaggccctt cagaataatg 420

tcacaccata tgtagtctca ttgcaagcta aagattgtcc agatatgaaa catttttttgc 480

aaaagttgat ctcacagttg atggactgct gtgtagatat aaaatccaaa gaggaggaaa 540

gtgttcacgt cacccaaaga aagacacatt attcaatgga ttcactttcc agttggtata 600

tgactgtcac acagaagacg gacccaaaaa tgctaagcaa aaaaaggact acttctagcc 660

aatggcagtc tcctcctgtt gtcgttatct tgaaggatat ggaaagcttt gccacaaaag 720

tactacaaga cttcataatt atcagcagtc aacatctcca tgaatttcca ctaatactca 780

tttttggaat agccacatct cctattatca tccaccgatt gcttcctcat gcagtatcat 840

ctctattgtg catagaactg ttccaatctt tgtcttgtaa ggagcacctg actacggtac 900

tcgataagct acttcttaca actcagtttc cctttaaaat aaatgaaaaa gtattacagg 960
ttctgaccaa catctttttg tatcatgatt tctcagttca aaactttata aaaggacttc 1020
agctttctct attagagcat ttctattccc agcccttaag tgtcctgtgc tgtaatcttc 1080
cagaagccaa aagaagaata aattttttat caaataatca atgtgaaaac atccgacgtc 1140
taccatcttt taggaggtac gtggaaaagc aagcttcaga aaagcaagtt gcgctcttga 1200
ccaatgagag atatttgaag gaggaaacac aattattact agaaaacctg catgtttatc 1260
atatgaatta cttcctggtt ttgagatgtc ttcataagtt cacctcttct cttcccaagt 1320
atccactagg tcgacagatc agagagttgt actgtacatg tttagaaaag aacatatggg 1380
attcagagga gtatgcatca gtcttgcagc tgctgaggat gttggcaaag gatgaactga 1440
tgaccatact tgagaaatgt ttcaaggttt ttaagtctta ttgtgaaaac caccttggca 1500
gcacagctaa gagaatagag gagttcctgg cccagtttca gagcctcgat gaaaccaaag 1560
aggaagaaga tgcttctggg tcacagccaa aggggcttca gaagacagac ctctatcatc 1620
ttcagaagtc cttattggaa atgaaggagt taagaagaag taagaagcaa accaaatttg 1680
aagtactcag agaaaatgtt gtgaacttca ttgactgtct agtgagagaa taccttctgc 1740
ctcctgagac acagcctctc catgaggtgg tgtacttcag tgctgcccat gcccttcgtg 1800
agcatttaaa tgctgctccg cgaattgccc tccatactgc actcaacaat ccttactatt 1860
atctcaagaa tgaagcactg aaaagcgaag aaggctgcat tccgaatatc gccccagaca 1920
tctgcatagc atacaaactg cacctagagt gtagcaggct catcaacctc gtggactggt 1980
cagaggcttt tgcaacagtt gtgacagctg ctgaaaaaat ggatgcaaat tctgcaacct 2040
cagaagaaat gaatgaaatt atccatgctc ggtttattag agctgtttct gaactagaac 2100
ttttaggatt tataaaacct accaaacaga agactgacca tgtggcaaga ctaacatggg 2160
gaggctgcta gaaagcaaat aagcaaagcc agaactatca catttagctt aagagaaaaa 2220
ggtgaccagt catatttaca tatattagag gagcctgttt tgttgagaag ataaatgtgt 2280
aacccccatt gatgtttaac cagaaaagta cattgctaac cccaaacagg catgtatcaa 2340
aacacctgtg gagtacttta gactccaaca aataataatg taactaaaac tgctcacaca 2400
ttttactgta ctttccaaag tcattactaa attgtgagta aatcattctt gaacttagag 2460
tatgtaaatg taataaattc cgttatccag gagtat 2496

<210> 50
<211> 4916

<212> DNA
<213> human

<400> 50
agaattcgga gcgcggaaga gccagagctg cgagcgcctg gagctggatc tctctccggt 60

cgcgcacgcc gaggccagta gggagagaag atggtggttc tccgcagcag cttggagctg 120

cacaaccact ccgcggcctc ggccacgggc tccttggacc tgtccagtga cttcctcagt 180

ctggagcaca tcggccggag gcggctccgc tcggccggcg cggcgcagaa gaaacccgcg 240

gcgaccacag ccaaagcggg cgatgggtca tcagttaagg aagttgaaac ctaccaccgg 300

acacgtgctt taagatcttt gagaaaagat gcacagaatt cttcagattc tagttttgag 360

aagaatgtgg aaataacgga gcaacttgct aatggcaggc attttacaag gcagttggcc 420

agacagcagg ctgataaaaa aaaagaagag cacagagaag acaaagtgat tccagttact 480

cggtcattga gggctagaaa catcgttcaa agtacagaac acttacatga agataatggt 540

gatgttgaag tgcgtcgaag ttgtaggatt agaagtcgtt atagtggtgt aaaccagtcc 600

atgctgtttg acaaacttat aactaacact gctgaagctg tacttcaaaa aatggatgac 660

atgaagaaga tgcgtagaca gcgaatgaga gaacttgaag acttgggagt gtttaatgaa 720

acagaagaaa gcaatcttaa tatgtacaca agaggaaaac agaaagatat tcaaagaact 780

gatgaagaaa caactgataa tcaagaaggc agtgtggagt catctgaaga gggtgaagac 840

caagaacatg aagatgatgg tgaagatgaa gatgatgaag atgatgatga tgatgacgat 900

gatgatgatg atgatgatga tgaagatgat gaagatgaag aagatggaga agaagagaat 960

cagaagcgat attatcttag acagagaaaa gctactgttt actatcaggc tccattggaa 1020

aaacctcgtc accagagaaa gcccaacata ttttatagtg gcccagcttc tcctgcaaga 1080

ccaagatacc gattatcttc cgcaggacca agaagtcctt actgtaaacg aatgaacagg 1140

cgaaggcatg caatccacag tagtgactcg acttcatctt cctcctctga agatgaacag 1200

cactttgaga ggcggaggaa aaggagtcgt aatagggcta tcaataggtg cctccccacta 1260

aattttcgga aagatgaatt aaaaggcatt tataaagatc gaatgaaaat tggagcaagc 1320

cttgccgatg ttgatccaat gcaactagat tcttcagtac gatttgatag tgttggtggc 1380

ctgtctaatc atatagcagc tctaaaagag atggtggtgt ttccattact ttatccagaa 1440

gtctttgaaa aatttaaaat tcaacccccca agaggttgtt tgttttatgg gccacctgga 1500

actggaaaga ctctggttgc cagagcactt gccaatgagt gcagtcaagg ggataaaaga 1560

gtagcatttt tcatgaggaa aggtgctgat tgtctaagta aatgggtagg agaatctgaa 1620

agacagctac gattgctgtt tgatcaggcc tatcagatgc gcccatcaat tatttttttt 1680
gacgaaattg atggtctggc tccagtacgg tcaagcaggc aagatcagat tcacagttct 1740
attgtttcca ccctgctagc tcttatggat ggattggaca gcagagggga aattgtggtc 1800
attggtgcta cgaacaggct agattctata gatcctgctt tacgaaggcc tggtcgcttt 1860
gatagagaat tcctctttag cctgcctgat aaagaggctc gaaaagagat tctaaagatt 1920
cacaccaggg attggaatcc caaaccactg gacacatttt tagaagagct agcagaaaac 1980
tgtgttggat actgtggagc agatattaaa tcaatatgtg ctgaagctgc tttatgtgct 2040
ttacgacgac gctacccaca gatctatacc actagtgaga aactgcagtt ggatctctct 2100
tcaattaata tctcagctaa ggatttcgag gtagctatgc aaaagatgat accagcctcc 2160
caaagagctg tgacatcacc tgggcaggca ctgtccaccg ttgtgaaacc actcctgcaa 2220
aacactgttg acaagatttt agaagccctg cagagagtat ttccacatgc agaattcaga 2280
acaaataaaa cattagactc agatatttct tgtcctctgc tagaaagtga cttggcttac 2340
agtgatgatg atgttccatc agtttatgaa aatggacttt ctcagaaatc ttctcataag 2400
gcaaaagaca attttaattt tcttcatttg aatagaaatg cttgttacca acctatgtct 2460
tttcgaccaa gaatattgat agtaggagaa ccaggatttg ggcaaggttc tcacttggca 2520
ccagctgtca ttcatgcttt ggaaaagttt actgtatata cattagacat tcctgttctt 2580
tttggagtta gtactacatc ccctgaagaa acatgtgccc aggtgattcg tgaagctaag 2640
agaacagcac caagtatagt gtatgttcct catatccacg tgtggtggga aatagttgga 2700
ccgacactta aagccacatt taccacatta ttacagaata ttccttcatt tgctccagtt 2760
ttactacttg caacttctga caaaccccat tccgctttgc cagaagaggt gcaagaattg 2820
tttatccgtg attatggaga gatttttaat gtccagttac cggataaaga agaacggaca 2880
aaatttttg aagatttaat tctaaaacaa gctgctaagc ctcctatatc aaaaaagaaa 2940
gcagttttgc aggctttgga ggtactccca gtagcaccac cacctgagcc aagatcactg 3000
acagcagaag aagtgaaacg actagaagaa caagaagaag atacatttag agaactgagg 3060
atttcttaa gaaatgttac acataggctt gctattgaca agcgattccg agtgtttact 3120
aagcctgttg accctgatga ggttcctgat tatgtcactg taataaagca accaatggac 3180
ctttcatctg taatcagtaa aattgatcta cacaagtatc tgactgtgaa agactatttg 3240
agagatattg atctaatctg tagtaatgcc ttagaataca atccagatag agatcctgga 3300

gatcgtctta ttaggcatag agcctgtgct ttaagagata ctgcctatgc cataattaaa 3360
gaagaacttg atgaagactt tgagcagctc tgtgaagaaa ttcaggaatc tagaaagaaa 3420
agaggttgta gctcctccaa atatgccccg tcttactacc atgtgatgcc aaagcaaaat 3480
tccactcttg ttggtgataa aagatcagac ccagagcaga atgaaaagct aaagacaccg 3540
agtactcctg tggcttgcag cactcctgct cagttgaaga ggaaaattcg caaaaagtca 3600
aactggtact taggcaccat aaaaaagcga aggaagattt cacaggcaaa ggatgatagc 3660
cagaatgcca tagatcacaa aattgagagt gatacagagg aaactcaaga cacaagtgta 3720
gatcataatg agaccggaaa cacaggagag tcttcggtgg aagaaaatga aaaacagcaa 3780
aatgcctctg aaagcaaact ggaattgaga aataattcaa atacttgtaa tatagagaat 3840
gagcttgaag actctaggaa gactacagca tgtacagaat tgagagacaa gattgcttgt 3900
aatggagatg cttctagctc tcagataata catatttctg atgaaaatga aggaaaagaa 3960
atgtgtgttc tgcgaatgac tcgagctaga cgttcccagg tagaacagca gcagctcatc 4020
actgttgaaa aggctttggc aattctttct cagcctacac cctcacttgt tgtggatcat 4080
gagcgattaa aaaatctttt gaagactgtt gttaaaaaaa gtcaaaacta caacatattt 4140
cagttggaaa atttgtatgc agtaatcagc caatgtattt atcggcatcg caaggaccat 4200
gataaaacat cacttattca gaaaatggag caagaggtag aaaacttcag ttgttccaga 4260
tgatgatgtc atggtatcga gtattcttta tattcagttc ctatttaagt catttttgtc 4320
atgtccgcct aattgatgta gtatgaaacc ctgcatcttt aaggaaaaga ttaaaatagt 4380
aaaataaaag tatttaaact ttcctgatat ttatgtacat attaagataa atgtcatgtg 4440
taagataact gataaatatt ggaactttgc tagaacaaga ccctgtagta atagtaataa 4500
tagttgaagt ttggccaact cttaataaag ttattttggt aactaatgtt ttatggcact 4560
taagaataat tagcagcgtt aaattttgtt tgtattaagc acttttaatt ttatccttcc 4620
taaaaatagt ttattgtatc tgacaagaaa cttacttaac cattgtgtcc ttcccatctt 4680
ttttgtcatc tttgttttct tcaaatgccc tcctcccatc tgccttgaga ttcccttgtc 4740
ttcacttaaa agccagagtg caagtcatga tttgcgggag ggctcttgaa ccacttctgg 4800
ctgcaccaca attctgtact tgagtatcac agtcattgtt tttgagacaa acatttttat 4860
aattctaatt tgggttaata aagattttaa atatttaaaa aaaaaaaaaa aaaaaa 4916

<210> 51
<211> 4183

<212> DNA
<213> human

<400> 51
atgagaccac agtcataaga gtaaaacaaa aattctgatg gaaataaatg cttatttggt 60

aggaagcaaa aagcacaaaa acaagattgt ctttcttttt ctttttcttt ttaaatgaga 120

aggatccacc cttgtgggaa aggaactctc aaaagacagc agtgggcctg acatcttggg 180

cactttccag gttcttggga ggaagggaga aaagaagcag agagagagaa gaaatatcta 240

ctatttttat atacggtgta ttcctctcat atcttaaaga aaagctttat gtagaaaaaa 300

agattcatga cgcagcttat gtaagaataa acttgtggaa tggaattagt tgcagggagg 360

aggtctgatt tcgttcagtt ctcacagtga tctagtgtct gacttttcta gggaatccaa 420

tcaataaaat tgaagactgg ctgcaggatt gcggatactc tgaagaagga ttttctgagg 480

aagcaggaca gtttatctac aatggcttct gcagccatgg gaccagcttt gaagatgact 540

tgaccctggg agcggaggcc acactactgg ccgccaatgg caaactcttc tccaggagtt 600

tcctcgagac agccaggcct tgccagctac ttgatcttgg ctgtagtttg gcttccagca 660

gcatgactgg ggggaccaac aagactagtt caagcatctc agaaattctg gacaaagtgc 720

aagaagatgc agaagatgtc ctcttcagtc tgggctttgg ccaagaggat cacaaagaca 780

cttctcggat acccgcccga tttttcacca ccccctctca ggccaagggc attgatttcc 840

agctcttcct gaagtcccag gtgcggagga ttgaaatgga agacccctgc ctcatgctgg 900

ccagcaggtt taagcaggtg caaacactgg ctgtcactgc tgatgccttc ttctgtctct 960

actcctatgt gtctaagaca cctgtccaaa agttcacacc atcccacatg ttctggaatt 1020

gcaaccatcc aactgatgtg ccatccatca ggattctgtc ccgagagcct gaaccccagt 1080

cacccagaga ccgccttcgg aaagccatct ccaagatgtg tctgtacaca tgcccccgag 1140

accggccacc accaccccac aacacccccca aaaggaacag tttggaccaa gttgtgttgg 1200

aagtgatgga caaagtgaaa gaagagaagc agttcctcca gcaagactct gatttggggc 1260

aattctcaca ggaagatcct gtgccccctg ctgagggtaa gaagttgccc acttctccct 1320

atccatgtgt cttctgctgt gaagaggaaa cacagcagag gatgtccaca gtgctagcac 1380

catcccaaac tctggattcg aaccccaagg taccctgttg cacacattct ctgcccatag 1440

aagatccaca gtggagcaca gacccagctc agataaggag agagctgtgt agtctaccag 1500

ccaccaatac ggaaacccat ccagccaagg atgagacttt ctggaaaaga aagagcagag 1560

caagaaagag cctcttccag aagaatctca tgggcaggaa ggttaagtcc ttggacctgt 1620

ccatcaccca gcagaaatgg aagcagagtg tagacagacc agaactgcgt cggtctttaa 1680

gccagcagcc acaggacact tttgacttgg aggaggtgca aagcaacagt gaggaggagc 1740

agagtcagag tcgctggccc agcagaccca ggcaccccca ccaccaccag acttttgctg 1800

gcaaagactc gtgaagcttt ctccaccacc acaacagcat gtggtctgac agaattggct 1860

tcaaagaaga aactaaaagc cacctcttgc aggaaactgc actgccctca acatccagcc 1920

cctactcctt ggtcctccaa acccccaaac tctgcaggag aaaggcagca tggtacatgg 1980

ggaggaagac cagactgagc gatttggaat ccacatccta atgctgccac aagctgcatg 2040

cacaaagacc ttaggcacat ctcttcattt ctctgtacac tggtttctct actatgtgtg 2100

tattaaaata tataatgtgg atgatagtaa actgaacaaa gccttaattt tctcccaagc 2160

tttgacattg ccaagggcag ttaggagact tcaggatcaa gtttagggga caagtttttt 2220

tctaatactt tcaaaaggcc caagtgaaga gaggaaggac acctcacttt ctggctctaa 2280

aagcatggta catctcacac taggattccc tctgcacctc tgttcatccc tgtattccta 2340

actaaattcc agaaaatctt ccacaacact ggagtcctat ttttcttctt gagacctcct 2400

ttgaagaaat caacttggag actcctcctt aaatccagtt tctttctcta cccaaaattt 2460

tgcatcttta tagaaaaaga gataagtagg attcagagtc tcttctccta gccattgtgg 2520

ggggaaaaaa tgctatttct gttggcagaa aacaatacaa atattttttt taaatcatgg 2580

catgttttta aagttaacta acagctgtag tttgctttgg aagttaaatg ttttgtcaat 2640

aaacagggct tgagcacatg cagtgctctc ttaagcagta tgtggtttca gagcatcttg 2700

gacacatttc tcccctgaga aaatattagt attaatagaa tgggagaagt aagatgcacg 2760

catttccaga agtatattat ttggctgcct aataaattgt gagacaaaag aaaaaaaaaa 2820

aacaggaaga gagggacttg agggaatttg agggaaatca taaccaaaag tttaaaggaa 2880

aaaattacac cagacatgat aaacagaagt tgattttgtt ctggtgctat tgccagcatt 2940

aggggagaag gcacaaactg agtctgatac gtagtgaggc agagacaaaa gggctagggt 3000

ggaaaaattt ttaaaccatc tgtgtttact aattccccat accaaaagaa caaaatgaac 3060

tttctcctat ttcatgataa aagatagttt cacaatttgg aaaaagcccc ccactccaag 3120

tttcctaccc ttcccaccca cagaaaccag gagacagggg aactatcttc cttgatgtta 3180

acatttcaaa gaggtggttc tcaggtccta gagaaagata ttcctgagtt gtaaaactgg 3240

gaagaggctg gaagaagatt tagatctcaa gaaatagaaa gaactacagc aaattttcta 3300

aagcaaatat tcttaaggga ggtcaagggc ctgaagtcag gaagaaacct atttggtcaa 3360

gctgagggga aagttaaggc tatcttgtca caagagaaca gaaatatctg acaatatttc 3420

atttaaagaa ctgtattagt ccattctcac actgctataa ataaatacct gagactgggt 3480

aatttataca gaaaagaggt ttaattggct cacagttccg caggctgtac aggaagcatg 3540

gcaacatcag tttctgggga ggcctcaggg agctttaact catggtggaa agcaaagtgg 3600

gagcaggcat cttacctggt atgagcagga ggaacaaagg aaaggggagg tgctaacact 3660

tttaaatgac cagatcttgt aagaactcac tcattataca gtaccaaggg gagatggtgc 3720

taaacgatta atgaaaactc tacccagtaa tccaatcacc acccaccagg ccccacctcc 3780

aacaatgggg attacagttt gacatgagat ttgggtgagg acacagatcc aaactgtcag 3840

gcctctgagc ccaagctaag ccatcatatc ccctgtgacc tgcatgtata catccagatg 3900

gcctgaagca agtggagaat cacaaaagaa gtgaaaatgg cctgttgctg ccttaacaga 3960

tgacattact ttgtgaaatt ccttcttctg gctcagaagc tcccccactg agcaccttgt 4020

gacccccacc cctgcctgcc agagaataat cccctttgac tgtaattttc cactacctac 4080

ctaaatccta taaaacagcc ccacccctat ctccctttgc tgactctctt tttggactca 4140

gcctgcctgc acccaggtga ttaaaaagct ttattgctca cac 4183

<210> 52
<211> 2505
<212> DNA
<213> human

<400> 52
gctgagcgcc ggaggagcgt aggcagggca gcgctggcgc cagcggcgac aggagccgcg 60

cgaccggcaa aaatacacgg gaggccgtcg ccgaaaagag tccgcggtcc tctctcgtaa 120

acacactctc ctccaccggc gcctccccct ccgctctgcg cgccgcccgg ctgggcgccc 180

gaggccgctc cgactgctat gtgaccgcga ggctgcggga ggaaggggac agggaagaag 240

aggctctccc gcgggagccc ttgaggacca agtttgcggc cacttctgca ggcgtccctt 300

cttagctctc gcctgcccct ttctgcagcc taggcggccc aggttctctt ctcttcctcg 360

cgcgcccagc cgcctcggtt cccggcgacc atggtgacga tggaggagct gcgggagatg 420

gactgcagtg tgctcaaaag gctgatgaac cgggacgaga atggcggcgg cgcgggcggc 480

agcggcagcc acggcaccct ggggctgccg agcggcggca agtgcctgct gctggactgc 540

agaccgttcc tggcgcacag cgcgggctac atcctaggtt cggtcaacgt gcgctgtaac 600

accatcgtgc ggcggcgggc taagggctcc gtgagcctgg agcagatcct gcccgccgag 660
gaggaggtac gcgcccgctt gcgctccggc ctctactcgg cggtcatcgt ctacgacgag 720
cgcagcccgc gcgccgagag cctccgcgag gacagcaccg tgtcgctggt ggtgcaggcg 780
ctgcgccgca acgccgagcg caccgacatc tgcctgctca aaggcggcta tgagaggttt 840
tcctccgagt acccagaatt ctgttctaaa accaaggccc tggcagccat cccacccccg 900
gttcccccca gcgccacaga gcccttggac ctgggctgca gctcctgtgg gaccccacta 960
cacgaccagg ggggtcctgt ggagatcctt cccttcctct acctcggcag tgcctaccat 1020
gctgcccgga gagacatgct ggacgccctg ggcatcacgg ctctgttgaa tgtctcctcg 1080
gactgcccaa accactttga aggacactat cagtacaagt gcatcccagt ggaagataac 1140
cacaaggccg acatcagctc ctggttcatg gaagccatag agtacatcga tgccgtgaag 1200
gactgccgtg ggcgcgtgct ggtgcactgc caggcgggca tctcgcggtc ggccaccatc 1260
tgcctggcct acctgatgat gaagaaacgg gtgaggctgg aggaggcctt cgagttcgtt 1320
aagcagcgcc gcagcatcat ctcgcccaac ttcagcttca tggggcagct gctgcagttc 1380
gagtcccagg tgctggccac gtcctgtgct gcggaggctg ctagcccctc gggacccctg 1440
cgggagcggg gcaagacccc cgccaccccc acctcgcagt tcgtcttcag ctttccggtc 1500
tccgtgggcg tgcactcggc ccccagcagc ctgccctacc tgcacagccc catcaccacc 1560
tctcccagct gttagagccg ccctgggggc cccagaacca gagctggctc ccagcaaggg 1620
taggacgggc cgcatgcggc agaaagttgg gactgagcag ctgggagcag gcgaccgagc 1680
tccttcccca tcatttctcc ttggccaacg acgaggccag ccagaatggc aataaggact 1740
ccgaatacat aataaaagca aacagaacac tccaacttag agcaataacc ggtgccgcag 1800
cagccaggga agaccttggt ttggtttatg tgtcagtttc acttttccga tagaaatttc 1860
ttacctcatt tttttaagca gtaaggcttg aagtgatgaa acccacagat cctagcaaat 1920
gtgcccaacc agctttacta aaggggggagg aagggagggc aaagggatga gaagacaagt 1980
ttcccagaag tgcctggttc tgggtacttg tccctttgtt gtcgttgttg tagttaaagg 2040
aatttcattt ttaaaagaaa tcttcgaagg tgtggttttc atttctcagt caccaacaga 2100
tgaataatta tgcttaataa taaagtattt attaagactt tcttcagagt atgaaagtac 2160
aaaaagtcta gttacagtgg atttagaata tatttatgtt gatgtcaaac agctgagcac 2220
cgtagcatgc agatgtcaag gcagttagga agtaaatggt gtcttgtaga tatgtgcaag 2280
gtagcatgat gagcaacttg agtttgttgc cactgagaag caggcgggtt gggtgggagg 2340

aggaagaaag ggaagaatta ggtttgaatt gctttttaaa aaaaaaagaa aagaaaaaga 2400

cagcatctca ctatgttgcc aaggctcatc ttgagaagca ggcgggttgg gtgggaggag 2460

gaagaaaggg aagaattagg tttgaattgc tttttaaaa aaaaa 2505

<210> 53
<211> 2204
<212> DNA
<213> human

<400> 53
gagcggtgcg gaggctctgc tcggatcgag gtctgcagcg cagcttcggg agcatgagtg 60

ctgcagtgac tgcagggaag ctggcacggg caccggccga ccctgggaaa gccggggtcc 120

ccggagttgc agctcccgga gctccggcgg cggctccacc ggcgaaagag atcccggagg 180

tcctagtgga cccacgcagc cggcggcgct atgtgcgggg ccgctttttg ggcaagggcg 240

gctttgccaa gtgcttcgag atctcggacg cggacaccaa ggaggtgttc gcgggcaaga 300

ttgtgcctaa gtctctgctg ctcaagccgc accagaggga gaagatgtcc atggaaatat 360

ccattcaccg cagcctcgcc caccagcacg tcgtaggatt ccacggcttt ttcgaggaca 420

acgacttcgt gttcgtggtg ttggagctct gccgccggag gtctctcctg gagctgcaca 480

agaggaggaa agccctgact gagcctgagg cccgatacta cctacggcaa attgtgcttg 540

gctgccagta cctgcaccga aaccgagtta ttcatcgaga cctcaagctg ggcaaccttt 600

tcctgaatga agatctggag gtgaaaatag gggattttgg actggcaacc aaagtcgaat 660

atgacgggga gaggaagaag accctgtgtg ggactcctaa ttacatagct cccgaggtgc 720

tgagcaagaa agggcacagt ttcgaggtgg atgtgtggtc cattgggtgt atcatgtata 780

ccttgttagt gggcaaacca ccttttgaga cttcttgcct aaaagagacc tacctccgga 840

tcaagaagaa tgaatacagt attcccaagc acatcaaccc cgtggccgcc tccctcatcc 900

agaagatgct tcagacagat cccactgccc gcccaaccat taacgagctg cttaatgacg 960

agttctttac ttctggctat atccctgccc gtctccccat cacctgcctg accattccac 1020

caaggttttc gattgctccc agcagcctgg accccagcaa ccggaagccc ctcacagtcc 1080

tcaataaagg cttggagaac cccctgcctg agcgtccccg ggaaaaagaa gaaccagtgg 1140

ttcgagagac aggtgaggtg gtcgactgcc acctcagtga catgctgcag cagctgcaca 1200

gtgtcaatgc ctccaagccc tcggagcgtg ggctggtcag gcaagaggag gctgaggatc 1260

ctgcctgcat ccccatcttc tgggtcagca agtgggtgga ctattcggac aagtacggcc 1320

ttgggtatca gctctgtgat aacagcgtgg gggtgctctt caatgactca acacgcctca 1380

tcctctacaa tgatggtgac agcctgcagt acatagagcg tgacggcact gagtcctacc 1440

tcaccgtgag ttcccatccc aactccttga tgaagaagat caccctcctt aaatatttcc 1500

gcaattacat gagcgagcac ttgctgaagg caggtgccaa catcacgccg cgcgaaggtg 1560

atgagctcgc ccggctgccc tacctacgga cctggttccg cacccgcagc gccatcatcc 1620

tgcacctcag caacggcagc gtgcagatca acttcttcca ggatcacacc aagctcatct 1680

tgtgcccact gatggcagcc gtgacctaca tcgacgagaa gcgggacttc cgcacatacc 1740

gcctgagtct cctggaggag tacggctgct gcaaggagct ggccagccgg ctccgctacg 1800

cccgcactat ggtggacaag ctgctgagct cacgctcggc cagcaaccgt ctcaaggcct 1860

cctaatagct gccctcccct ccggactggt gccctcctca ctcccacctg catctggggc 1920

ccatactggt tggctcccgc ggtgccatgt ctgcagtgtg ccccccagcc ccggtggctg 1980

ggcagagctg catcatcctt gcaggtgggg gttgctgtgt aagttatttt tgtacatgtt 2040

cgggtgtggg ttctacagcc ttgtcccccct cccccctcaac cccaccatat gaattgtaca 2100

gaatatttct attgaattcg gaactgtcct ttccttggct ttatgcacat taaacagatg 2160

tgaatattca aaaaaaaaaa aaaaaaaaaa aaaaaaaaaa aaaa 2204

<210> 54
<211> 2530
<212> DNA
<213> human

<400> 54
aaaacaggag ctgattcgag ctggcagagc tgggccatgg aaccggtaga gacctggacc 60

cccggaaagg tggcaacttg gctgagaggt cttgacgact ccctgcagga ctatcccttt 120

gaggactggc agctgcctgg caagaacctg ctccagctct gcccccaaag cctcgaggct 180

ctggctgtgc ggtctctggg acaccaggag ctcatcctgg gcggggtgga acagctccag 240

gccctgagct ccaggctaca gacagagaac ctgcaaagcc tgacagaggg acttctgggg 300

gcaacccatg acttccagag catagtccaa ggctgcctgg gggactgtgc caagacccct 360

attgatgtcc tctgtgcagc tgtggagctg ttgcatgaag ctgacgccct cctcttctgg 420

ctcagcaggt acctcttctc ccacttaaat gatttctcag catgccagga gatccgagac 480

ttgttggagg agctgagcca ggtcttgcat gaggatggtc cagcggctga gaaggagggc 540

acagtcctga ggatctgcag ccacgtggct gggatctgcc acaacatcct ggtctgctgc 600

cccaaggagc tgctggaaca gaaggccgtg ctcgagcagg tgcagctgga cagtccattg 660
ggcctagaaa ttcacaccac cagcaattgc cagcactttg tgtcccaagt ggacacccag 720
gttcccactg actcccgact gcagatccag cctggagacg aggttgtcca gatcaacgag 780
caggtggtgg tgggatggcc ccgtaagaac atggtgaggg aactgctgcg ggagccagcc 840
ggactcagct tagtgctgaa gaagatcccg ataccggaga cccccccaca gacgccccct 900
caggtcctgg actccccgca ccagaggagc ccatcactgt ctctggcccc actgtctccc 960
agggccccat ctgaagacgt ctttgccttt gacctgtctt caaaccccag tcccggaccc 1020
agccctgcct ggacagactc tgcctccctt ggccctgagc cctgcccat cccccggaa 1080
cccccagcca tactcccagc aggggtagca gggactccag ggctccctga atcccctgac 1140
aagagtcctg ttggtcggaa gaaatcaaaa ggcctggcga cccggctgag ccgccggcgg 1200
gtgtcatgcc gtgagctggg ccggccggac tgtgacggct ggctcctgtt gcgaaaggca 1260
ccgggcggct tcatgggccc gcgctggcgc cgccgctggt ttgtgctcaa gggacacacg 1320
ctctactggt accgccagcc ccaggatgag aaggctgagg gcctcatcaa tgtctccaac 1380
tatagtctgg aaagtggaca tgatcagaag aagaaatatg tgtttcagct cacccatgat 1440
gtgtacaaac ccttcatctt cgctgctgat accctgacag atctgagcat gtgggtgcgt 1500
catctcatta cctgcatctc caagtaccag tctccaggcc gggccccccc accccgagag 1560
gaagactgct acagtgagac cgaagcagag gacccggacg atgaggctgg gtcccactca 1620
gcctcgccca gccctgctca agctgggagt cccctccatg gagacacatc acctgcagcc 1680
acccccacac agcgcagccc acggacctcc tttggctctc tgacagacag cagtgaagag 1740
gcactggaag gaatggtacg gggggctgagg cagggtggcg tgtccctcct aggccagcca 1800
cagcccctga cccaggaaca gtggcggagc tctttcatgc ggcgcaaccg agaccctcag 1860
ctcaatgagc gagtgcaccg tgtgcgggcg ctacagagca cactcaaggc aaagctgcag 1920
gagctgcagg tcctagaaga agtgctgggt gaccctgagc tgacaggaga gaagttccgc 1980
cagtggaagg agcagaaccg ggagctgtac tcagagggcc tgggggcctg gggagtggca 2040
caggctgaag gcagctccca catcttgacc tctgactcca cagaacagtc cccccaactcc 2100
ctgccctctg accctgaaga gcactcccat ctctgccccc tgacctcaga gagcagcctc 2160
cgacctcctg acctctgacc ctggccagca ctctagctcc tgacctttga cccgagggcc 2220
acctcaaccc cagcttctga cgtgtccagg acagagcatc cctggattct gttcagggtg 2280

ggaagtagta ctgctagtca tggtctcacc ccgagctgac ccctctgcct gggctttgtg 2340

ccaccctctc ccttgccaaa gaagaaactc tccccccaaa tcctccaacc tctggggcca 2400

cagccctgcc cctccagttc cttggcagtt ctcccccaaa ccaggtctgt acaggtgttc 2460

tttattttac atgagggcta ctttccaacc aaataaagtc aatttttcta agaaaaaaaa 2520

aaaaaaaaaa 2530

<210> 55
<211> 374
<212> DNA
<213> human

<400> 55
atgtctggcc gtggtaaagg tggaaaaggt ttgggtaagg gaggagctaa gcgtcatcgc 60

aaggttttgc gcgataacat ccagggcatc actaagccag ctatccggcg ccttgctcgt 120

cgcggcggtg tcaagcgaat ttctggcctt atctatgagg agactcgtgg tgttctgaag 180

gtgttcctgg agaacgtgat tcgtgacgct gtcacttaca cagagcacgc caaacgcaag 240

accgtgacag caatggatgt ggtctacgcg ctgaagcgac agggacgcac tctttacggc 300

ttcggtggct aaggctcctg cttgctgcac tcttattttc attttcaacc aaaggccctt 360

ttcagggccg ccca 374

<210> 56
<211> 2265
<212> DNA
<213> human

<400> 56
agtcctgcga tttcgggtgt agagggagca ggggcctgcg gggacctggt gtgggtggag 60

tggggacaag cggtggagaa gggtacgcca gggtcgctga gagactctgt tctccctgga 120

gggactggtt gccatgagag cagccgtctg aggggacgca gcctgcacta cgcgccccaa 180

gaggctgtgc gtggcgagca ggtcacgtga cgggagcgcg ggctttggaa ggcggctgaa 240

cgtcaggcca cccgccgcta agctgagaag ggagagcgag cttaggaccg cctgcccggg 300

gcaaccccga accaagcttt agccgccgag gccgcgtgtc ccaaaggcca gtcatccctc 360

ctctgtgttg ccatgggaat tcaaggcctg gccaaactaa ttgctgatgt ggcccccagt 420

gccatccggg agaatgacat caagagctac tttggccgta aggtggccat tgatgcctct 480

atgagcattt atcagttcct gattgctgtt cgccagggtg gggatgtgct gcagaatgag 540

gagggtgaga ccaccagcca cctgatgggc atgttctacc gcaccattcg catgatggag 600

aacggcatca agcccgtgta tgtctttgat ggcaagccgc cacagctcaa gtcaggcgag 660
ctggccaaac gcagtgagcg gcgggctgag gcagagaagc agctgcagca ggctcaggct 720
gctggggccg agcaggaggt ggaaaaattc actaagcggc tggtgaaggt cactaagcag 780
cacaatgatg agtgcaaaca tctgctgagc ctcatgggca tcccttatct tgatgcaccc 840
agtgaggcag aggccagctg tgctgccctg gtgaaggctg gcaaagtcta tgctgcggct 900
accgaggaca tggactgcct caccttcggc agccctgtgc taatgcgaca cctgactgcc 960
agtgaagcca aaaagctgcc aatccaggaa ttccacctga gccggattct gcaggagctg 1020
ggcctgaacc aggaacagtt tgtggatctg tgcatcctgc taggcagtga ctactgtgag 1080
agtatccggg gtattgggcc caagcgggct gtggacctca tccagaagca caagagcatc 1140
gaggagatcg tgcggcgact tgaccccaac aagtaccctg tgccagaaaa ttggctccac 1200
aaggaggctc accagctctt cttggaacct gaggtgctgg acccagagtc tgtggagctg 1260
aagtggagcg agccaaatga agaagagctg atcaagttca tgtgtggtga aaagcagttc 1320
tctgaggagc gaatccgcag tggggtcaag aggctgagta agagccgcca aggcagcacc 1380
cagggccgcc tggatgattt cttcaaggtg accggctcac tctcttcagc taagcgcaag 1440
gagccagaac ccaagggatc cactaagaag aaggcaaaga ctggggcagc agggaagttt 1500
aaaaggggaa aataaatgtg tttccccatt atacctcctt caccccagaa tatttgccgt 1560
cttgtaccct taagagctac agctagagaa accttcacgg ggtggagaga ggattctaag 1620
gcttttctag cgtgaccctt ttcagtagtg ctagtccctt ttttacttga tcttaatggc 1680
aagaaggcca cagaggtact tttccttttt tagctcagga aaatatgtca ggctcaaacc 1740
acttctcagg cagtttaatg gacactaagt ccattgttac atgaaagtga tagatagcaa 1800
caagttttgg agaagagaga gggagataaa agggggagac aaaagatgta cagaaatgat 1860
ttcctggctg gccaactggt ggccagtggg aggtgatggt ggacctagac tgtgcttttc 1920
tgtcttgttc agccttgacc caccttgaga gagagccacc aggaaggcgc atcttagcag 1980
atgggaggaa ctgctgagag aagatgggca gaaagctgga gcccctggag ttggctgtgt 2040
ctgtgtttgt gactgattac tggctgtgtc ttgggtgggc agaaactcga acttgctatg 2100
taatttgtgt ctagttattc agaggagtaa gatggtgatg ttcacctggc aatcagctga 2160
gttgagactt tggaataaga cactggtttt catgcgctgt ttttgtttta aagttatgaa 2220
gaaaaaagtc aataaaattc taaaagtaaa aaaaaaaaaa aaaaa 2265

<210> 57
<211> 808
<212> DNA
<213> human

<400> 57
gccggaagtg acgcagggca gcggcgtcgc gggggcgggg ctcgggaaag acccgtgcca 60

gcgggcgtgt ggccgcgggt ttcgcacggt ccaataaggg agggcggcgt ggcccggcct 120

ggtagcgacg aggacgcgcc tgcgcagagg cggcagcacc accggggttg actccggggg 180

cgcggcgagg agagacatga ggctgagctg gttccgggtc ctgacagtac tgtccatctg 240

cctgagcgcc gtggccacgg ccacgggggc cgagggcaaa aggaagctgc agatcggggt 300

caagaagcgg gtggaccact gtcccatcaa atcgcgcaaa ggggatgtcc tgcacatgca 360

ctacacgggg aagctggaag atgggacaga gtttgacagc agcctgcccc agaaccagcc 420

ctttgtcttc tcccttggca caggccaggt catcaagggc tgggaccagg ggctgctggg 480

gatgtgtgag gggggaaagc gcaagctggt gatcccatcc gagctagggt atggagagcg 540

gggagctccc ccaaagattc caggcggtgc aaccctggtg ttcgaggtgg agctgctcaa 600

aatagagcga cgaactgagc tgtaaccaga ctggggaggg gcagggggag aggcccccat 660

cagggaccag actgttccaa aaaaaaaaca aaaaacaaaa acaaacaaaa aaacacttaa 720

aagcccaagg agtaagcctg tgtgtttgtg ggccctgaga gactcagaga cctcagctcc 780

agcatacccc accaccttct cctttccc 808

<210> 58
<211> 1782
<212> DNA
<213> human

<400> 58
acacggtctt tgagctgagt cgaggtggac cctttgaacg cagtcgccct acagccgctg 60

attccccccg catcgcctcc cgtggaagcc caggcccgct tcgcagcttt ctccctttgt 120

ctcataacca tgtccaccaa cgagaatgct aatacaccag ctgcccgtct tcacagattc 180

aagaacaagg gaaaagacag tacagaaatg aggcgtcgca gaatagaggt caatgtggag 240

ctgaggaaag ctaagaagga tgaccagatg ctgaagagga gaaatgtaag ctcatttcct 300

gatgatgcta cttctccgct gcaggaaaac cgcaacaacc agggcactgt aaattggtct 360

gttgatgaca ttgtcaaagg cataaatagc agcaatgtgg aaaatcagct ccaagctact 420

caagctgcca ggaaactact ttccagagaa aaacagcccc ccatagacaa cataatccgg 480

gctggtttga ttccgaaatt tgtgtccttc ttgggcagaa ctgattgtag tcccattcag 540
tttgaatctg cttgggcact cactaacatt gcttctggga catcagaaca aaccaaggtt 600
gtggtagatg gaggtgccat cccagcattc atttctctgt tggcatctcc ccatgctcac 660
atcagtgaac aagctgtctg ggctctagga aacattgcag gtgatggctc agtgttccga 720
gacttggtta ttaagtacgg tgcagttgac ccactgttgg ctctccttgc agttcctgat 780
atgtcatctt tagcatgtgg ctacttacgt aatcttacct ggacactttc taatctttgc 840
cgcaacaaga atcctgcacc cccgatagat gctgttgagc agattcttcc taccttagtt 900
cggctcctgc atcatgatga tccagaagta ttagcagata cctgctgggc tatttcctac 960
cttactgatg gtccaaatga acgaattggc atggtggtga aaacaggagt tgtgccccaa 1020
cttgtgaagc ttctaggagc ttctgaattg ccaattgtga ctcctgccct aagagccata 1080
gggaatattg tcactggtac agatgaacag actcaggttg tgattgatgc aggagcactc 1140
gccgtctttc ccagcctgct caccaacccc aaaactaaca ttcagaagga agctacgtgg 1200
acaatgtcaa acatcacagc cggccgccag gaccagatac agcaagttgt gaatcatgga 1260
ttagtcccat tccttgtcag cgttctctct aaggcagatt ttaagacaca aaaggaagct 1320
gtgtgggccg tgaccaacta taccagtggt ggaacagttg aacagattgt gtaccttgtt 1380
cactgtggca taatagaacc gttgatgaac ctcttaactg caaaagatac caagattatt 1440
ctggttatcc tggatgccat ttcaaatatc tttcaggctg ctgagaacct aggtgaaact 1500
gagaaactta gtataatgat tgaagaatgt ggaggcttag acaaaattga agctctacaa 1560
aaccatgaaa atgagtctgt gtataaggct tcgttaagct taattgagaa gtatttctct 1620
gtagaggaag aggaagatca aaacgttgta ccagaaacta cctctgaagg ctacactttc 1680
caagttcagg atggggctcc tgggaccttt aacttttaga tcatgtagct gagacataaa 1740
tttgttgtgt gcaaaaaaaa aaaaaaaaaa aaaaaaaaaa aa 1782

<210> 59
<211> 2258
<212> DNA
<213> human

<400> 59
cccgggaagg caggcgcgcg ggttagaacg cgccagaggt cggcgcgcgc acacccgcac 60
cgcccccgaac ccaggtagtg aggccagtga ttccgagtgt gtgaggagcg gcagtggcgg 120
cggaggaggg ggcggcgtgg gtgggggcgg gggcgggatg cgctccccgg cccctctagc 180

```
cccgtggtgg tacaacgcga aggtgtggga aggccgcgat aaaccggaac tgcagcccgc    240
cggacacctc cggcttcact tccgtaagag gagaggagtg tacggcaagg ggcgggaact    300
ggaacttggc ccgcctcgtt gtgagctgag ctcagcggca cgcttttgtg gcgtcactgc    360
actgttaccc cgccctacgt gtctctgacg ctgacacctt ctcactgtga aacgtcgcga    420
cctgtgacgt ctgggggcg cctcaaatct tccactccag catcggatcc cggaaaggca    480
gcgtcggaga ctggacccaa aactcttcct gttctgcctg cagagttgag ccccgtccgg    540
gtcctggacc cgcgtagtac tgaccctgga tccctgttca ctgcgttctc gctccccgcg    600
ctccctgctg gaccccggga tgccgggcat ctccgcccga ggcctctctc atgaggggag    660
gaagcagcta gctgttaacc tcacccgtgt cctggcactc taccgttcca tcttggatgc    720
ctacatcatc gaatttttca cagacaacct atgggacaca ctcccttgct catggcagga    780
agcattggat ggactgaaac caccacagct ggccacaatg ctgctgggga tgcctgggga    840
aggggaggtc gtcaggtaca ggtcagtgtg gccactcacc ctgctggccc tgaagtccac    900
ggcgtgtgcc ctggccttta cccggatgcc tggctttcag accccctcag aattcctgga    960
gaaccccagc cagagctccc gactaacagc tccattccgg aaacatgtca ggcccaagaa   1020
gcagcatgag atccggaggc tgggagagtt ggtgaagaag ctgagtgatt tcacaggctg   1080
cacccaggtt gtagacgtgg gctcaggcca gggccatctc tcccgcttca tggctcttgg   1140
cctggggttg atggtgaaga gcatcgaagg ggatcagaga ctggtggaga gagcccagcg   1200
cctggaccag gagcttctgc aggctctgga gaaagaggag aagaggaacc cgcaggtggt   1260
ccaaaccagc cctcgtcact ccccacacca cgtggttagg tgggtagacc ccacagccct   1320
gtgtgaggag cttctgcttc cactggagaa cccgtgtcag ggcagggccc gcttgctgct   1380
cacaggcctc cacgcctgtg gggatctgag tgttgccttg ctgagacact tctcctgctg   1440
tcctgaggtg gtggccctgg cctcagtggg ctgctgctac atgaagctga gtgaccctgg   1500
cggctaccca ctgagtcagt gggtggctgg gctgcctggc tatgaactgc cctaccggct   1560
tcgggagggg gcctgccatg ccctggagga atatgctgag cggctacaga aagctggccc   1620
tggccttcga actcactgct accgtgcagc actggagaca gtcatccgac gggcccggcc   1680
cgagctccgt cggccaggcg tgcagggtat ccccagggtc cacgagctca agattgaaga   1740
atatgtgcag cgggggctac agcgagtggg gctagatccc cagctgccac tgaatctggc   1800
tgcccttcag gcccacctgg cccaggagaa ccgtgtggtg gccttcttca gcctggctct   1860
```

actgcttgcc ccactggtgg agacgcttat tctactggac cggctgctgt accttcagga 1920
acagcttttc catgctgagc tcctgcccat cttcagtcct gaactctctc ccagaaacct 1980
ggttctggtg gccaccaaga tgcccctggg tcaggctctt tctgttctgg agactgaaga 2040
cagctgatgc agcctgagga gacatctcag accccatcat ctgaaagtgc ccagagagca 2100
cagtggcaga gtacatctca tccagagaaa cagcaccctg catcctccag agtcctggtt 2160
ccttcagttt catccccttt ctctccttcc atggattatg taatacattg taaagtttta 2220
attaattaaa aattggatat ctgaaaaaaa aaaaaaa 2258

<210> 60
<211> 2118
<212> DNA
<213> human

<400> 60
aaaaaaaaga aaccaggcgg cgcgttcccg gtggcggcgc cctggactcc cgggcccgcg 60
catccccgcc agccttcctt aaggcggatg ggtggccccc gagaccccgt cggacccatg 120
gtttccagtg cagcgcggag tgggcggtgc cagcgtgcca ggagccatgt ctgaccagga 180
cgtttggaag atcatatcca tgccagaggc tcttgtgggg agatgagttg gtaaagagag 240
aggctgggat gaagatgctg ccaggagtgg acgtgtttgg gactggcagc tccgcccgag 300
ttctggtccc actgctgagg gcagaagggt tcactgttga ggccctgtgg gggaagactg 360
aggaggaggc gaagcagctt gctgaggaga tgaacatcgc cttctacacc agccggactg 420
atgacatctt gctgcatcaa gatgtggatc tggtgtgcat cagcatcccc cctccactca 480
cccggcagat atccgtgaag gctctaggta ttgggaagaa tgtggtttgc gagaaggcag 540
caacatcggt ggatgccttc cggatggtga cagcctcgcg ctactacccg cagctcatga 600
gcctggtagg gaacgtgctg cgcttcctgc ctgccttcgt gcgcatgaaa cagctgattt 660
cggaacacta tgtgggagcg gtgatgatct gtgatgcccg catctactca ggcagcctgc 720
tgagccccag ctatggctgg atctgtgatg agctcatggg cggcgggggc ttgcacacca 780
tggggaccta cattgtggac ctgctgaccc acctgaccgg ccggagagcc gagaaggtgc 840
acgggctgct caagacattc gtgaggcaga acgctgccat ccgtggcatc cggcacgtca 900
ctagcgatga cttctgtttc ttccagatgc tcatgggtgg gggtgtgtgt agcacagtga 960
cactcaactt caacatgcca ggcgcctttg tgcatgaagt catggtggta ggctctgcag 1020
gacgcctcgt cgcccgggga gccgacctct atgggcagaa gaactctgcc acgcaagagg 1080

```
agctgctctt gagggactcg ctggcagtgg gcgcaggact gcctgagcag gggccccagg   1140
atgtcccgct gctgtacctg aagggcatgg tctacatggt gcaggccttg cgccagtcct   1200
tccaggggca gggcgaccgc cgcacctggg accgcacccc tgtctccatg gccgcctcct   1260
tcgaggatgg gctgtacatg cagagcgtgg tggatgccat caagaggtcg agccgatccg   1320
gggagtggga ggctgtggag gtgctgacgg aggagcccga caccaaccag aacctgtgtg   1380
aggcacttca gcggaacaac ctatgagcct gcacctgggc tccttgccac agggcagagg   1440
gaccagggag gggaacagga gccagacatg acagggactt ggccctagca gagacagtgt   1500
ctttcatttg atgagtctgg gtgaagccag aggtggctct gggacttcgc cccctccagt   1560
ggctcacctg ctcctcagac ttgcagggtg gcaaatgttc ctgttgccag gttaccacgg   1620
tgtgcacagg gcagagctgc cgccaggcct cctcatggtg atgccagtga gtcggaccag   1680
gaaaggctca ccctggggag cccttcagcc tgatccgcag tcctaacagg gtgagaaagc   1740
acaagagggg ccagctcggc tgaggcctga ggagaaatga tgagaagttt tgtttcgttc   1800
ttgggctggg gcatctcagg aacagtgagg gcaacaagct gtccctcagg gaccctcacc   1860
tgccctccca tgtgaccagg cccttcccag gtggggactg gggcttgctt tattaagtga   1920
gctcttgggc ttttgaagta ggacagaggg ccctggtttg gcagagagga ggcaagggag   1980
ggctttagaa catctgagaa atgttaataa ataattcaga aaataaaaaa aaaaaaaaaa   2040
aaaaaaaaaa aaaaaaaaaa aaaaaaaaaa aaaaaaaaaa aaaaaaaaaa aaaaaaaaaa   2100
aaaaaaaaaa aaaaaaaa                                                 2118
```

<210> 61
<211> 3275
<212> DNA
<213> human

<400> 61

```
ctgggaccct gaggcggccg tggttaggcg gctccccggc ggctcctccg cggcggtgac     60
ggcgaccgca ctccccgctt cccgctcccc gcgctcctcc gcccgggtcc gccagccgag    120
gccgctcccg agcgtcggaa gatgccggcc gtgtccaagg gggacgggat gcggggcctg    180
gcggtcttca tctcggatat ccgcaactgt aaaagtaaag aagcagaaat aaaaaggata    240
aacaaggaac tggcaaatat cagatcaaaa tttaaaggtg acaaggctct tgatggctat    300
agtaaaaaaa agtacgtctg caagttgctc ttcatctttc tccttggtca tgacattgac    360
tttggacaca tggaggctgt gaacctgctg agttcaaaca gatacacgga aaagcagatc    420
```

```
ggctaccttt tcatctctgt gttggtgaac tcaaacagtg agctgatccg cctgatcaac    480
aacgccatca agaatgacct ggccagccgc aaccccacct tcatgggcct ggccctgcac    540
tgcatcgcca gcgtgggcag ccgggagatg gccgaggcct tcgccgggga gatccctaag    600
gtcctcgtag ccggagacac tatggacagc gtgaagcaga gcgcggccct gtgcttgctg    660
cgcctgtaca ggacgtcccc cgatcttgtc cccatgggcg actggacatc ccgagtggtg    720
cacctgctca atgaccagca cttgggtgtg gtaactgcag ccacaagtct gatcaccact    780
ttagcacaga agaacccaga agagtttaaa acctccgtgt ctctggctgt ctctaggcta    840
agcagaatcg tgacgtctgc atccacagat ctccaggatt acacttacta ttttgtcccg    900
gctccctggc tgtctgtcaa actgctgaga ctgctgcagt gctacccacc cccagaccct    960
gcagtgcgag gccgcctgac tgagtgcctg gagaccatcc tgaacaaagc ccaagaaccg   1020
cccaagtcga agaaggtcca gcactccaac gcgaagaatg ccgtgctctt cgaggccatc   1080
agcttaatca ttcaccatga cagtgagccg aacctgctcg tccgtgcctg caaccagttg   1140
ggccagtttc tgcagcaccg cgagaccaac ctgcgctacc tggccctgga gagcatgtgc   1200
acgctggcca gctctgagtt ctcccatgag gctgtcaaga cgcacatcga gacggtcatc   1260
aacgccctga agactgagcg ggacgtgagc gtgcggcagc gggccgtgga cctcctctac   1320
gccatgtgcg accgcagcaa cgccccacag atcgtggccg agatgctgag ctatctggag   1380
acagctgact actccatccg agaagagatt gtgctgaagg tcgccatcct ggctgagaag   1440
tacgcggtgg actacacctg gtatgtggat accatcttga acttgatccg aattgctggt   1500
gattacgtga gtgaagaggt gtggtaccga gtcattcaga tcgtcatcaa ccgggacgac   1560
gtgcagggct acgcggccaa gactgtgttc gaggctcttc aggctcccgc gtgccacgag   1620
aacctggtca aagtgggcgg ctacatcctg gggdagtttg gaaacttgat agctggagac   1680
ccgagatcca gcccgctgat ccagttccac ctgctgcact ccaagttcca cctgtgcagc   1740
gtccccaccc gcgcgctgct cctgtccacc tacatcaagt tcgtgaacct cttcccggag   1800
gtgaagccca ccatccagga cgtgctgcgc agcgacagcc agctcaggaa cgcagacgtg   1860
gagctgcagc agcgtgctgt ggagtacctg cggctcagca ccgtggccag caccgacatt   1920
ctggcgaccg tgctggagga gatgccccca ttcccggagc gggagtcctc catcttggca   1980
aagctcaaga agaagaaggg ccccagcacg gtgacagacc tggaggacac caagcgggac   2040
aggagtgtgg acgtgaacgg gggtcctgag cctgccccag ccagtaccag cgccgtgtct   2100
acgccttctc cgtcggcaga cctgctgggt ctcggggctg ccccccctgc cccgcgggc    2160
```

cccccaccct cctccggcgg cagcgggctg ctcgtggacg tgttctcaga ctcggcctct 2220
gtggtcgcgc ctctcgctcc tggctccgaa gacaactttg ccaggtttgt ttgtaaaaac 2280
aatggtgtgt tgtttgaaaa ccagctgctt caaattggac ttaagtctga atttcggcag 2340
aatttaggtc ggatgtttat cttttatggt aataagacct ccacgcagtt cctaaacttt 2400
accccaacac taatctgttc agacgacctt cagcctaacc tgaacctgca gaccaagccc 2460
gtggacccga ccgtggaggg gggcgcgcag gtgcagcagg tggtcaacat agagtgcgtg 2520
tccgacttca cggaggcgcc agtcctcaac attcagttca ggtatggggg caccttccag 2580
aacgtgtctg tgcagctgcc catcactctc aacaaattct tccagccgac agaaatggct 2640
tctcaggatt tctttcaacg ttggaagcag ttgagcaatc cacagcagga agtgcagaac 2700
atcttcaaag caaagcaccc aatggacaca gaagtcacca aagccaagat cattggattt 2760
ggttctgcac ttcttgaaga agttgatcct aatcctgcga atttcgtggg agctggaatc 2820
atccacacga aaaccaccca gattggatgc ctgctgcgct tggagccgaa cctgcaagcc 2880
cagatgtacc ggctcacgct gcgcacaagt aaggaagccg tttctcagag attatgtgaa 2940
ttgctctcag cgcagtttta gtcctgagga tggaagacca ggcttgtgtg tcttgtgttg 3000
tcttcgtctg tgccgtttgt cttcgtggcc atcctgcaga tgagcaccgt gtccagtgcc 3060
acagcacaag gcgcctcccc gccccgccgc cccacacctc tcccctttgg gctggacggg 3120
aacacacgtg tgtggctcag gaggaaaagc tcagcctgga ctgtggcagc cacggcagaa 3180
ggtggatctt gggatcaatt tttataaaaa tcgagacagt tctgtggtta aatctacaaa 3240
ttaaagggaa attagaaaaa aaaaaaaaaa aaaaa 3275

<210> 62
<211> 1482
<212> DNA
<213> human

<400> 62
gttgacgtgt tcgactttcc tgataattct gatgtctcaa gcattggcag gctgggtgaa 60
aatgagaaag atgaagaaac ttatgagacc tttgatcctc ctttacatag cacagctata 120
tatgctgatg aagaagaatt ctccaaacat tgtggactgt ctctctcttc aactcctcca 180
ggaaaagaag caaaaagaag ttcagacact tctggaaatg aagcaagtga aatcgaatct 240
gtaaaaatta gtgcaaaaaa gccaggaaga aagctcaggc ccattagtga tgactctgaa 300
agcattgaag aaagtgatac aaggagaaaa gttaaatcag cagagaaaat aagtacacaa 360

```
cgtcatgagg ttattcgaac cacagcgtct tcagaacttt cagagaaacc agctgagtct     420
gtcacttcta aaaagacagg acccettagt gcccagccct ctgttgaaaa agagaacttg     480
gcaatagaaa gtcaatcgaa aactcagaaa aaagggaaga tatctcatga caaaaggaag     540
aaatcaagaa gtaaagccat aggctcagat acttctgaca ttgtgcacat ttggtgtcca     600
gaaggaatga aaccagtga catcaaggag ttgaatattg ttttgcctga atttgagaaa     660
acccacctag agcatcaaca aagaatagaa tctaaagttt gtaaggcagc catcgccaca     720
ttttatgtta atgttaaaga acaattcatc aaaatgctta aagaaagcca gatgttgaca     780
aatctgaaaa ggaagaatgc taagatgatt tcagatatcg aaaagaaaag gcagcgtatg     840
attgaagtcc aggatgaact gcttcggtta gagccacagc tgaaacaact acaaacaaaa     900
tatgatgaac ttaaagagag aaagtcctcc cttaggaatg cagcatattt cttatctaat     960
ttaaaacagc tttatcaaga ttattcagat gttcaagctc aagaaccaaa cgtaaaggaa    1020
acgtatgatt catccagcct tccagctctg ttatttaaag caagaacact tctgggagcc    1080
gaaagccatc tgcgaaatat caaccatcag ttagagaagc tccttgacca gggatgagaa    1140
gagcagtcta ctaaaatgtg cctataggaa gactagtctc atgctgttac cttctgaaac    1200
tgtaccttta taaatcaatt gttttgcaaa gaagttatgg cctacttaga atctaaaatt    1260
tgttattcaa attaaatggc tgtgaacaat gttaaatagc atcagtttgt ccaatagttt    1320
taaaggccat aatcatcttt tctggttaat atcttgagta attttaaaat gttgacacct    1380
taatcggtcc caggtatgag ctataataaa cttgtaaaat taaaaaaaaa aaaaaaaaaa    1440
aaaaaaaaaa aaaaaaaaaa aaaaaaaaaa aaaaaaaaaa aa                       1482
```

```
<210>  63
<211>  4696
<212>  DNA
<213>  human

<400>  63
ggcagaccgt cacgtgacga cgtcgattcg cgtgcggcag tggcgaagtt gacaaacccc      60
gcgaaaatcg actctttgca tcgcacattt tgttgatttt ccctcgtttt tctttctctc     120
tttcccccgt ccatccgaaa gagggttgga aaaaaacaaa acaaacaaac aaacaaaaaa     180
aaaacctaac gctgttggga cccggaagcg gaaagggcat ctttgaggtc gatacttccg     240
ggtcattggg agagtgcggg attcctgggc cgagagcggg tggctgagcc gggacctcgc     300
gtgattctcg gaacccgagg agaagcggcg tccggggcta tggctgtgac tctggacaaa     360
```

```
gacgcttatt atcggcgagt gaagagactg tacagcaatt ggcggaaagg agaagatgag    420
tatgccaacg ttgatgccat tgttgtatca gtgggtgttg atgaagaaat tgtttatgcc    480
aaatcaactg ccttacagac atggctcttt ggttatgaac taactgatac tatcatggtc    540
ttttgtgatg acaaaatcat ctttatggcc agcaagaaaa aagtggagtt cttgaaacag    600
attgccaaca ctaagggcaa tgagaatgct aatggagccc ctgccatcac actgctaata    660
cgagaaaaga atgaaagtaa taagagtagc tttgacaaaa tgattgaagc cattaaagaa    720
agcaagaatg gcaagaagat tggagtgttc agcaaagaca aattccctgg agagttcatg    780
aagagctgga atgactgcct caacaaagaa ggctttgaca aaatagatat cagtgcagtt    840
gtggcatata ccatcgctgt aaaggaggat ggggagctca acctaatgaa gaaagcagcc    900
agcatcactt ctgaagtctt caacaaattc ttcaaggaaa gagtcatgga aatagttgat    960
gcagatgaga aagttcgaca cagcaaactg gctgagtctg tggaaaaggc cattgaagag   1020
aaaaaatacc ttgctggggc agacccttct actgtggaaa tgtgttaccc tcctatcatt   1080
cagagtggtg gcaactataa tctcaagttc agtgtggtga gtgacaagaa tcatatgcac   1140
tttggggcta tcacttgtgc catgggtatt cgcttcaagt cttactgctc caaccttgtt   1200
cgcactttga tggttgatcc ttctcaagaa gttcaagaaa attataactt tttgctccag   1260
cttcaagagg agctgctgaa ggaattaaga catggtgtga agatatgtga cgtgtataac   1320
gctgtcatgg acgtggttaa aaagcagaag ccagaactgc tgaacaaaat taccaaaaac   1380
ctagggtttg ggatgggaat tgaattccgt gaaggctccc tagtaatcaa tagcaaaaat   1440
caatacaaac tgaagaaagg aatggttttc agcatcaatt taggattctc agacctgact   1500
aacaaggagg ggaaaaagcc agaagagaaa acctatgccc tgttcattgg tgacacagtg   1560
cttgtggatg aggatggccc agctactgtt ctcacttctg tgaagaagaa agtgaagaat   1620
gtggggattt tcctaaagaa tgaagatgag gaagaagagg aggaggagaa agatgaggca   1680
gaggaccttt tgggaagagg ttctcgggca gcattactta cagaaagaac aagaaatgaa   1740
atgactgcag aagagaagcg aagagcacat cagaaagaac tagcggctca actcaatgaa   1800
gaagcaaaga ggcgattgac tgaacaaaag ggagaacagc agattcagaa agctcgcaag   1860
tctaatgtgt cctataaaaa cccatctctg atgcctaagg aaccgcatat tcgggaaatg   1920
aagatctaca tcgataagaa atatgagact gtaataatgc ccgtgtttgg cattgcaaca   1980
ccgtttcaca ttgccacaat caagaatata agtatgtccg tggaaggaga ttatacttac   2040
```

```
ttgcgaatca acttttattg cccaggcagt gctctgggca ggaatgaagg caacatcttt   2100
cctaaccctg aagcgacttt tgtcaaggaa attacatacc gagcatcaaa tattaaggca   2160
cccggagaac agacagtacc agccttgaac cttcagaatg ctttccgaat tattaaagaa   2220
gtacagaaac gttataaaac tcgagaagct gaagagaaag agaaggaggg gattgtaaaa   2280
caagactcac tggtgatcaa tctaaaccgg agtaatccga aactgaaaga tctatacatt   2340
cgcccaaata ttgcccaaaa gaggatgcaa ggctcactgg aggcccatgt caatggcttc   2400
cgcttcacat ctgttcgagg agacaaagtg gatattttgt acaataatat taagcatgct   2460
ttgttccagc cctgtgatgg agaaatgatt attgtcttgc actttcacct caagaatgcc   2520
atcatgtttg ggaagaagcg gcacacggat gtgcagttct acacagaagt gggagagata   2580
accacggact tggggaaaca tcagcatatg catgaccgag atgacctcta tgctgagcag   2640
atggaacgag aaatgaggca caaactgaaa acagccttta aaaatttcat tgagaaagta   2700
gaggctctaa ctaaggagga actggaattt gaagtgcctt ttagggactt gggatttaac   2760
ggagctccct ataggagtac ctgcctcctt cagcccacta gtagtgcgct ggtaaatgct   2820
acggaatggc caccttttgt ggtgacattg gatgaggtag agctgatcca ctttgagcgg   2880
gtccagtttc acctgaagaa ctttgatatg gtaatcgtct acaaggacta cagcaagaaa   2940
gtgaccatga tcaacgccat tcctgtagcc tctcttgacc ccatcaagga atggttgaat   3000
tcctgcgacc tgaaatacac agaaggagta cagtccctca actggactaa aatcatgaag   3060
accattgttg atgaccctga gggcttcttc gaacaaggtg gctggtcttt cctggagcct   3120
gagggtgagg ggagtgatgc tgaagaaggg gattcagagt ctgaaattga agatgagact   3180
tttaatcctt cagaagatga ctatgaagag gaagaggagg acagtgatga agattattca   3240
tcagaagcag aagagtcaga ctattctaag gagtcattgg gtagtgaaga agagagtgga   3300
aaggattggg atgaactgga ggaagaagcc cgaaaagcgg accgagaaag tcgttacgag   3360
gaagaagaag aacaaagtcg aagtatgagc cggaagagga aggcatctgt gcacagttcg   3420
ggccgtggct ctaaccgtgg ttccagacac agctctgcac cccccaagaa aaagaggaag   3480
taacttctga actttggccc tgagctccat tcttcctcca gccaacccct gaaaatttta   3540
catgacatag aaactgtatt tttcctttcg ttttcatttg aagttttgcc atttgtgttt   3600
atgggtttag gggccatttt gtgtggacca atctactcgg ggaattccag gcccaccagg   3660
acacgtgcca atggccccat tcagatggca agggaggagg tgttcttgaa gacaggagga   3720
ggctcccgct gttaataaat attgtttcat tcttctctct tcctgtcacc ttctgccaag   3780
```

acattgatgg cttctgacat cttatttggt gtctcaaagc tgtatttcca agacagtggt 3840

acaaggtgac ccttaattac ccgtatcatg gttcttgacc agcacattca atcctccaac 3900

ctaccctact gccatgacct tccgcacatc tctaagtttt atctttgcaa tactcaaggt 3960

tctcggaaat ttgctaatgg ttgtgataaa ccatacagct tgagccagtg aggcagattg 4020

ggctggtgcc ttcgtctgag ttttcctgct ttcctgcctc gtgcagattc tgaggtatat 4080

ctgctgcctt ggaagacata agaagcagtg atactccctg gctcggttat tttctccata 4140

caatgcacac atggtacaat gatagaaggc aaaattgcca ctgtcttctt ttttttctca 4200

tatatctaag gaagatatat caggttgtgc ctcatgtacc gcttctagtg aaatgtagag 4260

gaaggctcaa aggagtcaac atttagatct ggaagggaca agtcatgcct tgggcctaga 4320

ataccctgat gagaaaagag aagaggaagg gaggccatat ctacaacaca gcctctcggc 4380

actgctgctc cttattttaa ctttgtcttg cattgtcctg tatttatcac agtttctgtt 4440

gaacagcttt tcaagtattt ggggagttta tcttgccatc ctccccttct ggttctctgc 4500

acccacctgt cccactgcag ttccttccgt gctctgtgac tttaagagaa gaagggggga 4560

ggggtcccgg attttatgtt tgtttgtttt ttctccttag cagtaggact tgatattttc 4620

aattttggaa gaactaaaag atgaataaac tgggtttttt ttgttgtttg tttttgtaaa 4680

aaaaaaaaaa aaaaaa 4696

<210> 64
<211> 1249
<212> DNA
<213> human

<400> 64
ggcacgagcc agggtttcct cttcaagtag gtctaaaaca tttttttctc cattgacttc 60

cttcctgttc taactgccag tactcagaag tcagagttga gagacagagg caccccggac 120

agagacgtga agcactgaat aaatagatca gaatgactga aaaagcccca gagccacatg 180

tggaggagga tgacgatgat gagctggaca gcaagctcaa ttataagcct ccaccacaga 240

agtccctgaa agagctgcag gaaatggaca aagatgatga gagtctaatt aagtacaaga 300

aaacgctgct gggagatggt cctgtggtga cagatccgaa agcccccaat gtcgttgtca 360

cccggctcac cctggtttgt gagagtgccc cgggaccaat caccatggac cttactggag 420

atctggaagc cctcaaaaag gaaaccattg tgttaaagga aggttctgaa tatagagtca 480

aaattcactt caaagtgaac agggatattg tgtcaggcct gaaatacgtt cagcacacct 540

acaggactgg ggtgaaagtg gataaagcaa catttatggt tggcagctat ggacctcggc 600

ctgaggagta tgagttcctc actccagttg aggaggctcc caagggcatg ctggcccgag 660

gcacgtacca caacaagtcc ttcttcaccg acgatgacaa gcaagaccac ctcagctggg 720

agtggaacct gtcgattaag aaggagtgga cagaatgaat gcatccaccc cgttccccac 780

ccttgccacc tggaagaatt ctctcaggcg tgttcagcac cctgtccctc ctccctgtcc 840

acagctgggt ccctcttcaa cactgccaca tttccttatt gatcgatctt ttcccaccct 900

gtcactcaac gtggtcccta gaacaagagg cttaaaaccg ggctttcacc caacctgctc 960

cctctgatcc tccatcaggg ccagatcttc cacgtctcca tctcagtaca caatcattta 1020

atatttccct gtcttacccc tattcaagca actagaggcc agaaaatggg caaattatca 1080

ctaacaggtc tttgactcag gttccagtag ttcattctaa tgcctagatt cttttgtggt 1140

tgttgctggc ccaatgagtc cctagtcaca tcccctgcca gagggagttc ttcttttgtg 1200

agagacactg taaacgacac aagagaacaa gaataaaaca ataactgtg 1249

<210> 65
<211> 1545
<212> DNA
<213> human

<400> 65
gaagacacca ccggaagcaa ggaaggtgct gtgtaatcat taaggagcgg aggcttttgg 60

agctgctaaa atgccggatt acctcggtgc cgatcagcgg aagaccaaag aggatgagaa 120

ggacgacaag cccatccgag ctctggatga gggggatatt gccttgttga aaacttatgg 180

tcagagcact tactctaggc agatcaagca agttgaagat gacattcagc aacttctcaa 240

gaaaattaat gagctcactg gtattaaaga atctgacact ggcctggccc caccagcact 300

ctgggatttg gctgcagata agcagacact ccagagtgaa cagcctttac aggttgccag 360

gtgtacaaag ataatcaatg ctgattcgga ggacccaaaa tacattatca acgtaaagca 420

gtttgccaag tttgtggtgg accttagtga tcaggtggca cctactgaca ttgaagaagg 480

gatgagagtg ggcgtggata gaaataaata tcaaattcac attccattgc ctcctaagat 540

tgacccaaca gttaccatga tgcaggtgga agagaaacct gatgtcacat acagtgatgt 600

tggtggctgt aaggaacaga ttgagaaact gcgagaagta gttgaaaccc cattacttca 660

tccagagagg tttgtgaacc ttggcattga gcctcccaag ggcgtgctgc tctttggtcc 720

acccggtaca ggcaagacac tctgtgcgcg ggcagttgct aatcggactg atgcgtgctt 780

```
cattcgagtt attggatctg agcttgtaca gaaatacgtc ggtgaggggg ctcgaatggt    840
tcgtgaactc tttgaaatgg ccagaacaaa aaaagcctgc cttatcttct ttgatgaaat    900
tgatgctatt ggaggggctc gttttgatga tggtgctgga ggtgacaatg aagtgcagag    960
aacaatgttg gaactgatca atcagcttga tggttttgat cctcgaggca atattaaagt   1020
gctgatggcc actaacagac ctgatacttt ggatccagca ctgatgaggc cagggagatt   1080
ggatagaaaa attgaattta gcttgcccga tctagagggt cggacccaca tatttaagat   1140
tcacgctcgt tcaatgagtg ttgaaagaga tatcagattt gaactgttag cacgactgtg   1200
tccaaatagc actggtgctg agattagaag cgtctgcaca gaggctggta tgtttgccat   1260
cagagcacgg cgaaaaattg ctaccgagaa ggatttcttg gaagctgtaa ataaggtcat   1320
taagtcttat gccaaattca gtgctactcc tcgttacatg acatacaact gaaccctgaa   1380
ggctttcaag tgaaaacttt aaattggaat cctaacctta tatagacttg ttaataacca   1440
attcataaac aaataaatgg cttcaaaatt gtatgctttt ttccatatct cttcttgtaa   1500
tataataaaa ggtgatttct aatgttaaaa aaaaaaaaaa aaaaa                   1545
```

```
<210>  66
<211>  4421
<212>  DNA
<213>  human

<400>  66
ggcagcgcca ggcgtggggc cggggcagcg ccgggcgagg gcgagggcgc gggcggaagt     60
tccaggaggg ccagaattcc aagtgatttc tgtcacccgc cggcggaccg agagtctagg    120
gttcggggcg cgcaggctgc ctcggcgcag acctcttccc ctcagggagg ccgtctcctg    180
accagaccca ctcctgcctc ccacccgggt gccgtgttta gggttatttt aggctgctca    240
cctgacagat tgtacacaag ccccttaattg cccccctctg tttgaggtcg tggtgaggtt   300
tcatatttcg cagccacgct caaattgtgc cgtcatgtta cagttgtagg tgatgaaata    360
ccggctgctt agacgcggga tcggttttta taccggggca aacgctagtg gtcccagaat    420
gcagagttgg gggcctcaag gaatccgggt gtgctgagat aattggttaa ttgcttgatt    480
aaaattgggg attaatttta gagttcttcc actcggagct cgcttctgtg ctgaagatgc    540
tttgcaacgc cttgaggaaa acgcgccaaa gaagtttctg ttagacttga gttacaagat    600
tgcagatttt tcttgatttg ctagtatgtt tttttttacg ttcgtagagt tttataggta    660
gatagataac tttggttcgg gtagttttcc ttttaaaatt tgtagagaac tgaaagtggc    720
```

```
tgatatgtta gctttttttag ggggacgggg tcggggaatg gagtctcagt ctgttgccca    780
ggctggagtg cagtggcgcg atctcggctc actgcaacct ccgcctcccg ggttcaagcg    840
attctcctgc ctcagcctcc cgaatagctg ggattacagg cgcccgccac ggcgcccagt    900
cagtatatca gtttttaagg tttgtgaagc gcgatttatt ttttcagaat gtcgtcttcc    960
tccacattgt gaggaaaatt ttcagaatac aaaaagttga ggccgggcgc tgtggctcaa   1020
gcctgtaatc ccagcacttt gggaggccga ggcgggtgga tcatgaggtc aggaggatca   1080
tgaggatcag gtggatcatg aggatcagga ggtcagcctg gccaatatgg tgaaaccccg   1140
tctctactaa aaatacaaaa ttagccgggt gtggtggcgc gtgcttgtaa tcccagctac   1200
tcaggaggct gaggcaggag aatcccttga acctgggagg cggaggttgc agtgagccaa   1260
gatcacacca ttgcactcca gcctgggcga cagggtgaga ctgtgtctca aaaaaaaaaa   1320
aaaaaagttg aaaggattct acagtggaca tccatagatt tattccaaat tctaaagttg   1380
ttaacagctc gttatactgc attctcttta tcacatagtg gttgttgaaa ggtttgacag   1440
gaacactgat gcttcaataa tggttttggt tcagtttgtt agtctgttga acttcaggat   1500
cgtgtcttag aatatatgtt cgaaaggata aaaaccgtac tttaattttg ggtttcaaaa   1560
gcctacccct gcaacaaaat agaagctcaa taagtgtgtc atcctcaaaa tatccgtggg   1620
tcttttgtct ttataatatt ctgacaccaa aagtgagaga agtagtagtt gaattttatg   1680
atctgcattg ttaaatgagg gcttatcttc cttaacaaat aataacatca agaacttaaa   1740
cgaaaattga acattctgac tcgaccgagt ggaatattgg tgaacgatac taagttagat   1800
ggacctatat tacagattct attcatgaac aatgctattt caaagcaata tcatcaagaa   1860
atagaggaat ttgtatcaaa tttagtaaaa agatttgagg aacagcagaa aaatgatgtg   1920
gaaaagactt cctttaatct tttgccccag ccatccagta ttgtgctaga ggaggaccac   1980
aaagtggaag agtcctgtgc cattaaaaac aacaaggaag ctttcagtgt tgtaggaagt   2040
gtcctgtatt ttactaattt ttgccttgat aaattggggc aaccgcttct aaatgaaaac   2100
cctcagcttt ccgaaggatg ggaaataccc aagtatccta catagtgaat gaagatcttt   2160
tatgcatctt gccactaagt tcacctttaa aacaatttcc aaattttgca gaccatgata   2220
actattcctt aataaaattc actaaaggta ccatcaagtc ttcagccaca ttgtttctct   2280
agaagggcaa gaaatacaag taaaggcaaa aaggccaaag cctcactgtt tcaattgtgg   2340
ttctgaagaa caccaaatga aagattgccc aatgcctcgg aatgctgctc gaataagtga   2400
```

```
aaagagaaaa gagtatatgg atgcctgtgg agaagcaaac aatcagaatt tccagcagcg   2460
ataccacgca gaagaagtag aagaaagatt tggaagattc aagccaggag ttattagtga   2520
ggaacttcaa gatgcactag gtgtgacaga caagagtctt ccacctttta tctatcggat   2580
gcgccagcta gggtacccac cagggtggct caaagaggct gaattggaga attcggggct   2640
tgcactctat gatggaaaag atggcactga tggggaaaca gaagttggag aaatacaaca   2700
gaataaaagt gtcacttacg atctctcaaa attggtcaac tatcctggtt ttaatatatc   2760
tactcccaga ggaattccag acgaatggag gatctttggt tccataccaa tgcaggcatg   2820
tcagcagaag gatgtgtttg ccaattacct tacttctaac ttccaagcgc caggtgtgaa   2880
gtctggcaac aagaggtctt catctcactc tagcccaggt agtccaaaga agcagaagaa   2940
tgaaagcaac tcagcgggat ctcccgccga catggagctc gattcagata tggaggtacc   3000
acatggttct cagagcagcg aaagttttca gtttcaacca ccattacctc ctgacactcc   3060
tccactcccc cggggaactc ctccacccgt cttcacccct ccactcccaa agggcacccc   3120
gccgctgact cccagtgact caccccagac cagaacagca tctggagctg tggatgagga   3180
cgcactgact ctagaagaac ttgaagaaca gcagaggcgg atctgggcag ctcttgagca   3240
ggccgagagc gtaaacagcg actccgacgt tcctgtggac acacctttaa ctggcaattc   3300
cgttgcctca tcaccttgtc caaatgagct agacctccct gtcccggagg gaaaaacatc   3360
tgaaaagcag acgctggatg agcctgaggt accagagatt tttacaaaga aatcagaagc   3420
tggacatgcc tccagtccag actctgaggt gacatcactt tgtcagaagg aaaaagcaga   3480
gttggctccg gtaaacactg aaggtgccct tcttgataat ggcagtgtcg taccaaactg   3540
tgacatcagc aatgggggca gccagaagct ctttcctgca gacaccagtc cttcaacggc   3600
cactaaaatt catagcccta tacctgacat gagcaaattt gcaactggaa tcacgccatt   3660
tgaatttgag aatatggcag aatctactgg aatgtacctc aggataagaa gcttgttaaa   3720
gaactcaccc cgaaaccagc agaaaaacaa aaaggcctct gaataatggc ttgacttagc   3780
actgagagct atttaataac tttgttccgt taattagtac taattaagtg gatagataga   3840
atggttttcc tgtttgtccc tcccatgttt aaaaatctat ccaaggttca tgttccaaag   3900
tcaagcctat tttaaagaaa gactgagctc actagttcag tatattttat tctcactgac   3960
aaaacttggg gggagatgtg aatatgacct ggtttagaga gggtttgtta aggtttatac   4020
tatttttgga ttgtgactat ccgtcgagag tgatggtttt tatctgtctt ttgtacattg   4080
ttttcccttt ctacattttg ctaattatcc tgtatataag tttaatatat cactttttaa   4140
```

aagaaaaaat tctaccattt taaattcatg tttcaactcc tacaaccaaa tgagaaaaat 4200

cagggatgag cagctttatc ccatttgggg tatttttgta agtgatttac atgtgtcaat 4260

tttagtaata cttttacttt tttgtaactt catccttcat atatgcttgc tatacaggta 4320

tgttcatctt tgtgtacaga ggtttaataa attagttttc atataaaaaa aaaaaaaaaa 4380

aaaaaaaaaa aaaaaaaaaa aaaaaaaaaa aaaaaaaaaa a 4421

<210> 67
<211> 2387
<212> DNA
<213> human

<400> 67
cctcgtgccg cggaccccag cctctgccag gttcggtccg ccatcctcgt cccgtcctcc 60

gccggcccct gccccgcgcc cagggatcct ccagctcctt tcgcccgcgc cctccgttcg 120

ctccggacac catggacaag ttttggtggc acgcagcctg gggactctgc ctcgtgccgc 180

tgagcctggc gcagatcgat ttgaatataa cctgccgctt tgcaggtgta ttccacgtgg 240

agaaaaatgg tcgctacagc atctctcgga cggaggccgc tgacctctgc aaggctttca 300

atagcacctt gcccacaatg gcccagatgg agaaagctct gagcatcgga tttgagacct 360

gcaggtatgg gttcatagaa gggcatgtgg tgattccccg gatccacccc aactccatct 420

gtgcagcaaa caacacaggg gtgtacatcc tcacatccaa cacctcccag tatgacacat 480

attgcttcaa tgcttcagct ccacctgaag aagattgtac atcagtcaca gacctgccca 540

atgcctttga tggaccaatt accataacta ttgttaaccg tgatggcacc cgctatgtcc 600

agaaaggaga atacagaacg aatcctgaag acatctaccc cagcaaccct actgatgatg 660

acgtgagcag cggctcctcc agtgaaagga gcagcacttc aggaggttac atcttttaca 720

ccttttctac tgtacacccc atcccagacg aagacagtcc ctggatcacc gacagcacag 780

acagaatccc tgctaccagt acgtcttcaa ataccatctc agcaggctgg gagccaaatg 840

aagaaaatga agatgaaaga gacagacacc tcagtttttc tggatcaggc attgatgatg 900

atgaagattt tatctccagc accatttcaa ccacaccacg ggcttttgac cacacaaaac 960

agaaccagga ctggacccag tggaacccaa gccattcaaa tccggaagtg ctacttcaga 1020

caaccacaag gatgactgat gtagacagaa atggcaccac tgcttatgaa ggaaactgga 1080

acccagaagc acaccctccc ctcattcacc atgagcatca tgaggaagaa gagaccccac 1140

attctacaag cacaatccag gcaactccta gtagtacaac ggaagaaaca gctacccaga 1200

aggaacagtg gtttggcaac agatggcatg agggatatcg ccaaacaccc agagaagact 1260

cccattcgac aacagggaca gctgcagcct cagctcatac cagccatcca atgcaaggaa 1320

ggacaacacc aagcccagag gacagttcct ggactgattt cttcaaccca atctcacacc 1380

ccatgggacg aggtcatcaa gcaggaagaa ggatggatat ggactccagt catagtacaa 1440

cgcttcagcc tactgcaaat ccaaacacag gtttggtgga agatttggac aggacaggac 1500

ctctttcaat gacaacgcag cagagtaatt ctcagagctt ctctacatca catgaaggct 1560

tggaagaaga taaagaccat ccaacaactt ctactctgac atcaagcaat aggaatgatg 1620

tcacaggtgg aagaagagac ccaaatcatt ctgaaggctc aactacttta ctggaaggtt 1680

atacctctca ttacccacac acgaaggaaa gcaggacctt catcccagtg acctcagcta 1740

agactgggtc ctttggagtt actgcagtta ctgttggaga ttccaactct aatgtcaatc 1800

gttccttatc aggagaccaa gacacattcc accccagtgg ggggtcccat accactcatg 1860

gatctgaatc agatggacac tcacatggga gtcaagaagg tggagcaaac acaacctctg 1920

gtcctataag gacaccccaa attccagaat ggctgatcat cttggcatcc ctcttggcct 1980

tggctttgat tcttgcagtt tgcattgcag tcaacagtcg aagaaggtgt gggcagaaga 2040

aaaagctagt gatcaacagt ggcaatggag ctgtggagga cagaaagcca agtggactca 2100

acggagaggc cagcaagtct caggaaatgg tgcatttggt gaacaaggag tcgtcagaaa 2160

ctccagacca gtttatgaca gctgatgaga caaggaacct gcagaatgtg gacatgaaga 2220

ttggggtgta acacctacac cattatcttg gaaagaaaca accgttggaa acataaccat 2280

tacagggagc tgggacactt aacagatgca atgtgctact gattgtttca ttgcgaatct 2340

tttttagcat aaaattttct actcttaaaa aaaaaaaaaa aaaaaaa 2387

<210> 68
<211> 2641
<212> DNA
<213> human

<400> 68
acggtccgct gcctccatgt ggagggagat acgggaagct gcatgtcacc acggagactt 60

ccttgttcgt gtctatatgg ttcatacatg gccttcctct taattaaaga tgtcttccga 120

ggaacatttt gtctaagaat aacccagtgg aaatctctca cataagaagt gatggaacta 180

cacatctgag tatgagcctg catggcatag tgagtctgca actgtgaact cgggaaatcc 240

tgtaccttcc cgtgcagtcg ctccccagcc atgcaggtat ttccagagcc tggagatccc 300

```
agactacctt tcaaaagaat gaattccaga cccagagagg cccctggagg agattcatgg    360
caagcagccg tacgaaataa cagaagcccc agagagggga agcaatttcc tgcctaagtc    420
gtccatctgt cagtggcaaa gctaggactg gaactcagga ttctccatcc ctcttcccaa    480
acctggcact atcttcctgg ctttctgggt ctggagtccc atgttagagg ccctctgttt    540
tcagagcagg actcattgaa tatgtgtgct ctttctactc aagtgcttca aggatccttc    600
ctggaaaagc ctcggttccg gaccagtgct gtcatggcct caccatgtca tagaattccc    660
atcatttcac ttgtgactcc cttcttccta ggacccgggc ataaattctt gaagagttga    720
tctgagtgac agactcccat gctatgccaa aaacggcctc ttatttccca atgattgaga    780
ccttttattt gatcaatctc atgtgctcac tggaccccag tgataccttc acatacctga    840
cacctcaggg aaacacagct tccgttcaag tcaagatcct tgctatactg caaactctta    900
tgaaacgtct aggagaattt tcaggctcct ttgtatatgg aaaaaggtca gaatgaagct    960
ccaaattctt cagcatgtgt aaaaacttaa gacaaccata ttttattttt gttttatttt   1020
ttattttttt cattgttttt ttttttgaga cagccttgct ctgttgccta ggctggagtg   1080
cagtggcgcg atctcggctc attgcaactt ccacctcctg ggttcaagcg attcttgtgc   1140
ctcagcctcc caagtagctg gaattacagg catgcaccac cattcacagc taatttttgt   1200
atttttagta gagatggggt ttcaccatgt tggccaggct ggtctcgacc tcctgacctc   1260
aggtgatcca gtcgtcttgg cctcccaaag tgctgggatt acaggcatga gcccctgtgc   1320
ccagccaaca actacatttt aaagcaactt tattctcaac tgaatgacat tttaatcact   1380
ttcttgccca tgtggatatg gataaggata ttttgccaat attcttgttt gtgtttgact   1440
gtgatgctat tgttatacaa agaatgggca ggtggagcta agcagaaaga gacagtcggg   1500
tacctgtacc atggaaaatg gaagagagcc aagcagactg catccttagg gggcagggag   1560
gtcagtgcaa gttgggacaa agcctagctg agtcggtgag cacttcatgg cagactagtc   1620
aaattatatc tcttggacta agatcttcat taaaggcctg cacctcagag acatttcagc   1680
ctaccaaggt gcatcattat ggaagactgg gcatgataat agtaatttaa atgaagttaa   1740
aagagcagtc taaattagaa caggtattca cattccaatt tctcttgaga agagcctctg   1800
ttggataaag aaaactcgct gagcaaaata aatcgattgg aggtaaactt taccagccct   1860
ggagtgcaag gtttagaaac atgcacacat cccacggtgt taagggtcct gctgttttct   1920
tccatgtcat ttcatcacag aacaattttc tacagcaacc tgatctacct tgtggcccag   1980
```

taaggcatgt tttgttttga gatggagtct cactctgtcg cccaggctgt agtgcagtgg 2040

cgtgatctcc gctcactgca agcactgcct cccgggttca cgccattctc ctgcctcagc 2100

ctcccaagta gctgggacta caggcgcccg ccaccatgcc cggctaattt tctgtatttt 2160

tattagagac ggggtttcac catgttagcc aggatggtct cgatctcctg acctctggga 2220

tctgcccgtt tcggcctccc aaagtgctgg gattacaggt gtgagccacc gctcccagcc 2280

aagcatgtga gagttttaaa tcaaatgtta ccttgtccct attagcctta ccactgctaa 2340

atatctacac ggttccctgt taaggagagt ttctaaatga ctctcttgag aaatcctgga 2400

taaatggaga atggagcatt tttcctgcct ctaagctttg ctccactgca atctttcaca 2460

tgctgtgtat ccagagggtg actgccagat agcttcttga agaaagggag gaggaaacgg 2520

tcattttctt gctgcgcttt ctaacaccca acagaggaaa tgcacagcat aggtgtgtgg 2580

gagtttggct gacttatggt aaaaaataaa catggtttcc tgttaaaaaa aaaaaaaaaa 2640

a 2641

<210> 69
<211> 1883
<212> DNA
<213> human

<400> 69
agttccgcgc ggggctgtcg gggaaccatg gctgccccga gagacaatgt cactttatta 60

ttcaagttat actgcttggc agtgatgacc ctgatggctg cagtctatac catagcttta 120

agatacacaa ggacatcaga caaagaactc tacttttcaa ccacagccgt gtgtatcaca 180

gaagttataa agttattgct aagtgtggga attttagcta aagaaactgg tagtctgggt 240

agattcaaag catctttaag agaaaatgtc ttggggagcc ccaaggaact gttgaagtta 300

agtgtgccat cgttagtgta tgctgttcag aacaacatgg ctttcctagc tcttagcaat 360

ctggatgcag cagtgtacca ggtgacctac cagttgaaga ttccgtgtac tgctttatgc 420

actgttttaa tgttaaaccg gacactcagc aaattacagt gggtttcagt ttttatgctg 480

tgtgctggag ttacgcttgt acagtggaaa ccagcccaag ctacaaaagt ggtggtggaa 540

caaaatccat tattagggtt tggcgctata gctattgctg tattgtgctc aggatttgca 600

ggagtatatt ttgaaaaagt tttaaagagt tcagatactt ctctttgggt gagaaacatt 660

caaatgtatc tatcagggat tattgtgaca ttagctggcg tctacttgtc agatggagct 720

gaaattaaag aaaaaggatt tttctatggt tacacatatt atgtctggtt tgtcatcttt 780

```
cttgcaagtg ttggtggcct ctacacttct gttgtggtta agtacacaga caacatcatg     840

aaaggctttt ctgcagcagc ggccattgtc ctttccacca ttgcttcagt aatgctgttt     900

ggattacaga taacactcac ctttgccctg ggtactcttc ttgtatgtgt ttccatatat     960

ctctatggat tacccagaca agacactaca tccatccaac aaggagaaac agcttcaaag    1020

gagagagtta ttggtgtgtg attttagcct cacgtgagac tccttttaag actaaaccat    1080

ttgcattaaa ctagagcctt aagtcaatct cagaaggtag cataaacaaa taaaaattaa    1140

ctgtatggca tgatcagtgc ggttatgtgg aaacaacaac aaacaaacga agctatctga    1200

gtgaactgct aatacagaaa cttaatgtag acctgtttgg ggtctactat tgttttagaa    1260

tgaaggaatt gtattattgt gtgtatatat aatttgtaaa taaaaagtat ggagatgata    1320

cggtgttaaa aaaaatcatg gtgaggctac aatactcaag taacaaggtt tgggacaatg    1380

tctaagggtt aaagtgccaa agccatttct gtactaactg ttctcttgtt ccggtaccgg    1440

ggagaaggat gacccctcct tattctccaa ttcatgtaca gtattttgtc ctagcagcat    1500

aaagacctag ctcttttctt acaagaggca gaaacaagac aggctagttc ataaacaaac    1560

tgaatgtgta actaacttct caaaatgaat ctatttcata actcggacaa tttctgggtg    1620

gtgactgagt accccttag tgagtacccc tttagtgcta tatttgtgcc attcattatc     1680

tggttcatat ttctttgctg ttagatgata cacatttctt caaaaaaatt tctaatgtca    1740

cttttgtact tttttaaata aagtatgttt aactgttggg ctctcaataa tttgtgaaat    1800

ttcagtgttt tctataatgt taatggggaa attcagcaat aaactttatt tgcaaaaaaa    1860

aaaaaaaaaa aaaaaaaaaa aaa                                            1883


<210>  70
<211>  2536
<212>  DNA
<213>  human

<400>  70
agcgggtgcg gggcgggacc ggcccggcct atatattggg ttggcgccgg cgccagctga      60

gccgagcggt agctggtctg gcgaggtttt atacacctga aagaagagaa tgtcaagacg     120

aagtagccgt ttacaagcta agcagcagcc ccagcccagc cagacggaat ccccccaaga     180

agcccagata atccaggcca agaagaggaa aactacccag gatgtcaaaa gaagtctggc     240

taaacatgtt aaaaaaggag agcagatatg ttcatgacaa acattttgaa gttctgcatt     300

ctgacttgga accacagatg aggtccatac ttctagactg gcttttagag gtatgtgaag     360
```

```
tatacacact tcatagggaa acattttatc ttgcacaaga ctttttgat agatttatgt   420
tgacacaaaa ggatataaat aaaaatatgc ttcaactcat tggaattacc tcattattca   480
ttgcttccaa acttgaggaa atctatgctc ctaaactcca agagtttgct tacgtcactg   540
atggtgcttg cagtgaagag gatatcttaa ggatggaact cattatatta aaggctttaa   600
aatgggaact ttgtcctgta acaatcatct cctggctaaa tctctttctc caagttgatg   660
ctcttaaaga tgctcctaaa gttcttctac ctcagtattc tcaggaaaca ttcattcaaa   720
tagctcagct tttagatctg tgtattctag ccattgattc attagagttc cagtacagaa   780
tactgactgc tgctgccttg tgccatttta cctccattga agtggttaag aaagcctcag   840
gtttggagtg ggacagtatt tcagaatgtg tagattggat ggtacctttt gtcaatgtag   900
taaaaagtac tagtccagtg aagctgaaga cttttaagaa gattcctatg gaagacagac   960
ataatatcca gacacataca aactatttgg ctatgctgga ggaagtaaat tacataaaca  1020
ccttcagaaa aggggacag ttgtcaccag tgtgcaatgg aggcattatg acaccaccga  1080
agagcactga aaaaccacca ggaaaacact aaagaagata actaagcaaa caagttggaa  1140
ttcaccaaga ttgggtagaa ctggtatcac tgaactacta aagttttaca gaaagtagtg  1200
ctgtgattga ttgccctagc caattcacaa gttacactgc cattctgatt ttaaaactta  1260
caattggcac taaagaatac atttaattat ttcctatgtt agctgttaaa gaaacagcag  1320
gacttgttta caaagatgtc ttcattccca aggttactgg atagaagcca accacagtct  1380
ataccatagc aatgtttttc ctttaatcca gtgttactgt gtttatcttg ataaactagg  1440
aattttgtca ctggagtttt ggactggata agtgctacct taaagggtat actaagtgat  1500
acagtacttt gaatctagtt gttagattct caaaattcct acactcttga ctagtgcaat  1560
ttggttcttg aaaattaaat ttaaacttgt ttacaaaggt ttagttttgt aataaggtga  1620
ctaatttatc tatagctgct atagcaagct attataaaac ttgaatttct acaaatggtg  1680
aaatttaatg tttttaaac tagtttattt gccttgccat aacacatttt ttaactaata  1740
aggcttagat gaacatggtg ttcaacctgt gctctaaaca gtgggagtac caaagaaatt  1800
ataaacaaga taaatgctgt ggctccttcc taactggggc tttcttgaca tgtaggttgc  1860
ttggtaataa cctttttgta tatcacaatt tgggtgaaaa acttaagtac cctttcaaac  1920
tatttatatg aggaagtcac tttactactc taagatatcc ctaaggaatt ttttttttta  1980
atttagtgtg actaaggctt tatttatgtt tgtgaaactg ttaaggtcct ttctaaattc  2040
ctccattgtg agataaggac agtgtcaaag tgataaagct taacacttga cctaaacttc  2100
```

tattttctta aggaagaaga gtattaaata tatactgact cctagaaatc tatttattaa 2160

aaaaagacat gaaaacttgc tgtacatagg ctagctattt ctaaatattt taaattagct 2220

tttctaaaaa aaaaatccag cctcataaag tagattagaa aactagattg ctagtttatt 2280

ttgttatcag atatgtgaat ctcttctccc tttgaagaaa ctatacattt attgttacgg 2340

tatgaagtct tctgtatagt ttgtttttaa actaatattt gtttcagtat tttgtctgaa 2400

aagaaaacac cactaattgt gtacatatgt attatataaa cttaaccttt taatactgtt 2460

tatttttagc ccattgttta aaaaataaaa gttaaaaaaa tttaactgct taaaagtaaa 2520

aaaaaaaaaa aaaaaa 2536

<210> 71
<211> 9061
<212> DNA
<213> human

<400> 71
atgaatggtc accgttttac gttcagaact cgttcgcttc tacctttaca aaagggacaa 60

cacagatgca ttactaagct cttggctcat aacgatccct ggggtatatt cagagcccct 120

tcctcgcggc gttcatatag tgtctcaggg gtctgtcagc cagcaatccc caattcctca 180

ttacatattc ctcacaatgc atgctggcta aaatggaaat tgtgttcaga aatagccctg 240

ccctaccatc atatactagg agaaaggagg aaattatgct ctattagaaa gtttgaacac 300

tggcgggaag atgtccgcag ctgttgccag gccagggttc tcccgagagg gaggacgctg 360

ggactgtggc ttgccctgat cggccgagaa gagtttgcca tgaatcttct gcgtcggagt 420

gggaaacggc ggcgttcaga atcaggctca gattcgttct cgggaagcgg cggtgacagc 480

agtgccagcc cccagttcct ctccgggtcc gtgctgagcc cgccgcccgg ccttggtcgc 540

tgcctgaagg ccgcagctgc aggagaatgc aagcctacag ttcctgacta cgaaatagac 600

aagctactat tggcaaactg gggacttcct aaagcagttc tggaaaaata ccacagtttt 660

ggtgtaaaaa agatgtttga atggcaggca gagtgccttt tgcttggaca agtcctggaa 720

ggaaagaatt tagtttattc agctcctaca agtgctggga agactcttgt ggcagaatta 780

cttattttga agcgggtttt ggaaatgcgg aagaaagctt tgtttattct tccctttgtt 840

tctgtggcta aagagaagaa atactacctc cagagtctgt ttcaggaagt aggaataaaa 900

gtagacggtt atatgggcag cacctctcca tcaaggcatt tctcttcatt ggatattgca 960

gtctgcacaa ttgagagagc caatggtctg atcaatcgcc tcatagagga aaataagatg 1020

gatctgttag gaatggtggt tgtggatgaa ttacatatgc tgggagactc tcaccgaggg 1080
tatctgctgg aacttttgct gaccaagatt tgctatatta ctcggaaatc agcatcttgt 1140
caggcagatc tagccagttc tctgtctaat gctgtgcaaa tcgttggcat gagtgctacc 1200
cttcctaatt tggagcttgt ggcttcctgg ttgaatgctg aactctacca taccgacttt 1260
cgccctgtac cgcttttgga gtcagtaaaa gttggaaatt ccatatatga ctcttcaatg 1320
aaacttgtga gggaatttga gcccatgcta caagtgaagg gagatgagga ccatgttgtt 1380
agtttatgtt atgagacgat ttgtgataac cattcagtat actttttttg tccatcaaag 1440
aaatggtgtg agaagctggc agatatcatt gctcgagagt tttataatct acatcatcaa 1500
gctgagggtg agggattggt gaaaccctct gaatgcccac cagtaattct ggaacaaaaa 1560
gaactcctgg aagtgatgga tcagttaaga cgtttgcctt caggactgga ctctgtatta 1620
cagaaaactg taccatgggg agtagcattt catcatgcag gtcttacttt tgaggagagg 1680
gatatcattg aaggagcctt tcgtcaaggt ctcattcggg tcttggcggc aacttctact 1740
ctttcttctg ggtgaattt acctgcacgt cgtgtgatta ttcgaacccc tatttttggt 1800
ggtcgacctc tagatattct tacttataag cagatggttg gccgtgctgg caggaaagga 1860
gtggacacag tagggagtat cttaatttgt aagaactctg agaaatcaaa aggcatagct 1920
ctccttcagg gttctctaaa gcctgttcgc agctgtctgc aaagacgaga aggagaagaa 1980
gtaactggca gcatgatacg agctattctg gagataatag ttggtggagt ggcaagtaca 2040
tcacaagata tgcatactta tgctgcctgc acatttttgg ctgcaagtat gaaagaaggg 2100
aagcaaggaa ttcagagaaa tcaagagtct gttcagcttg gagcgattga ggcctgtgtg 2160
atgtggctac tagaaaatga attcatccag agtacagaag ccagtgatgg aacagaagga 2220
aaggtgtatc atccaacaca tcttggttcg gccactcttt cttcttcact ttctccagct 2280
gatactttag atatttttgc tgacctgcaa agagcaatga agggctttgt tttagagaat 2340
gatcttcata ttctctatct ggttacacct atgtttgagg attggactac tattgattgg 2400
tatcgatttt tctgtttatg ggagaagttg ccaacttcaa tgaaaagggt ggcagagcta 2460
gtgggagttg aagaggggtt cttggcccgt tgtgtgaaag gaaaagtagt agccagaact 2520
gagagacagc atcgacaaat ggccatccat aaaaggtttt tcaccagtct tgtgctatta 2580
gatttaatca gtgaagttcc cttaagggaa ataaatcaga aatatggatg caatcgtggg 2640
cagattcaat ctttgcaaca gtcagctgct gtttatgcag ggatgattac agtattttcc 2700

aaccgtctgg gctggcacaa catggaacta ctactttccc aatttcagaa gcgtcttacg 2760

tttggcatcc agagggagct gtgtgacctg gttcgggtat ccttactaaa tgctcagaga 2820

gccagggttc tctatgcttc tggctttcat actgtggcag accttgctag agcaaatatt 2880

gtggaggtgg aggtgattct gaaaaatgct gtgcctttca aaagtgcccg gaaggcagtg 2940

gatgaggaag aggaagcagt tgaagaacgt cgcaatatgc gaactatctg ggtgactggc 3000

agaaaaggtt taactgaaag ggaagcagca gcccttatag tggaagaagc cagaatgatt 3060

ctgcagcagg acttagttga aatgggagtg caatggaatc catgtgccct gttacattct 3120

agtacatgct cattgactca tagtgagtcc gaagtaaagg aacacacatt tatatcccaa 3180

actaagagtt cttataaaaa attaacatca aagaacaaaa gtaacacaat atttagtgat 3240

tcttatatta agcattcacc aaatatagtg caagacttaa ataaaagtag agagcataca 3300

agttccttta attgtaattt ccagaatggg aatcaagaac atcagacatg ttccattttc 3360

agagcaagaa aacgggcctc tttagatata aataaagaga agccaggagc ctctcagaat 3420

gaggggaaaa caagtgataa gaaagttgtt cagacttttt cacagaaaac aaaaaaggca 3480

cctttgaatt tcaattcaga aaagatgagc agaagttttc gatcttggaa acgtagaaag 3540

catctaaagc gatctaggga cagcagcccc ctgaaagact ctggagcgtg tagaatccat 3600

ttacaaggac agactctgtc taatcctagt ctttgtgaag acccgtttac cttagatgag 3660

aagaaaacgg aatttagaaa ttcagggcca tttgctaaaa atgtatcttt gagtggtaag 3720

gaaaaagata ataaaacatc attcccatta caaataaagc aaaattgttc atggaacata 3780

acactaacta atgataattt tgtggagcat attgtcacag gatctcagag taaaaatgtg 3840

acttgtcagg ccactagtgt ggttagtgaa aagggcagag gagtagctgt tgaggcagaa 3900

aaaataaatg aagtgctgat acaaaatggt tcaaaaaacc agaatgttta tatgaaacac 3960

catgacatcc atccaattaa ccagtacctg cgaaagcaat ctcatgaaca gacaagcact 4020

attaccaaac agaaaaatat aatagagaga caaatgccct gtgaagcagt cagtagttac 4080

ataaatagag actcaaatgt tactatcaat tgtgaaagga taaagcttaa tacagaggaa 4140

aataaaccaa gtcattttca ggcattagga gatgatataa gcagaactgt gatacccagt 4200

gaagtacttc catcagctgg agcatttagc aaatcagaag gccagcatga gaattttcta 4260

aatatttcta gactacaaga aaaaacaggt acttatacaa caaacaaaac taaaaataat 4320

catgtttctg acttaggttt agtcctctgt gattttgaag atagtttcta tctggatact 4380

cagtcagaga aaataataca acagatggca actgaaaatg ccaaactagg agcaaaggac 4440

```
accaacctgg cagcagggat aatgcagaag agcttagtcc aacagaactc aatgaactct   4500
tttcagaagg agtgtcacat tccttttcct gctgaacagc accctctagg agcgactaag   4560
atagatcatt tggaccttaa gactgtaggt actatgaaac aaagcagtga ttcacatggg   4620
gttgatatcc tgactccaga aagcccgatt ttccattctc caatactatt ggaggaaaat   4680
ggtctttttt taaaaaagaa tgaagtttct gttactgatt cacaattaaa tagttttctt   4740
caaggttatc aaacacaaga aactgtgaaa ccagttatac ttctgattcc tcaaaagaga   4800
actcccactg gtgtagaagg agaatgtctt ccagttcctg aaacaagttt gaatatgagt   4860
gatagtttac tatttgatag cttcagtgat gactatctag taaaagaaca attacctgat   4920
atgcaaatga aagaacccct tccttcagaa gtaacatcaa accattttag tgattctctg   4980
tgtctacaag aagacctaat taaaaaatca aatgtaaatg agaatcaaga tacccaccag   5040
cagttgactt gttccaatga tgaatctatt atattttcag aaatggattc tgttcagatg   5100
gttgaagctt tggacaatgt ggatatattt cctgtccaag agaagaatca tactgtagta   5160
tctcctagag cattagaact aagtgatcca gtacttgatg agcaccacca aggtgatcaa   5220
gatggaggag atcaagatga aagggctgaa aaatcaaaat taactgggac caggcaaaat   5280
cattcattca tatggtcagg ggcatcattt gatctaagtc caggactgca aaggatttta   5340
gataaagtat ccagtcctct agaaaatgaa aagctaaaat caatgactat aaacttttcc   5400
agtttgaata gaaaaaatac agagttaaat gaagaacaag aagttatttc aaacttggag   5460
acaaaacaag tgcagggaat ttcattttct tctaataatg aagtaaaaag caagattgag   5520
atgctagaaa acaatgccaa tcatgatgaa acctcatccc tcttacctcg taaagaaagt   5580
aatatagttg atgataatgg tctcattcct cctacaccca ttccaacatc tgcttctaag   5640
ctgacatttc cagggattct tgaaacacct gtaaaccctt ggaaaactaa taatgtttta   5700
caacctggtg aaagttattt atttggctca ccttcagata ttaaaaacca cgatttaagt   5760
ccagggagta gaaatgggtt caaagacaac agccctatta gtgacacaag cttttcactt   5820
cagttatcac aggatggatt acagttaact ccagcctcaa gcagttcaga aagtttgtcc   5880
ataattgatg tagcaagtga ccaaaatctt ttccaaacat tcattaagga gtggcggtgc   5940
aaaaagcgat tttccatctc actggcttgt gaaaagatta gaagtttgac atcttctaaa   6000
actgctacta ttggcagtag gtttaagcaa gctagctcac ctcaggaaat tcctattaga   6060
gatgatggat ttcccattaa aggttgtgat gacaccttgg tggttggact ggcagtatgc   6120
```

tggggtggaa gggatgccta ttatttttca ctgcagaagg aacaaaagca ttctgaaatt 6180
agtgccagtt tggttccacc ttctttagat ccaagcctga ctttgaaaga caggatgtgg 6240
taccttcaat cttgcttgcg aaaggaatct gataaagaat gttctgttgt catctatgac 6300
ttcatccaga gctataaaat tcttcttctt tcttgtggca tctccttgga gcaaagttat 6360
gaagatccta aggtggcatg ctggttacta gatccagatt ctcaggagcc gactcttcat 6420
agcatagtta ccagttttct tcctcatgag cttccactcc tagaagggat ggagaccagc 6480
caagggattc aaagcctggg gctaaatgct ggcagtgagc attctgggcg atacagagca 6540
tctgtggagt ccattctcat cttcaactct atgaatcagc tcaactcttt gttgcagaag 6600
gaaaaccttc aagatgtttt ccgtaaggtg gaaatgccct ctcagtactg cttggccttg 6660
ctagaactaa atggaattgg ctttagtact gcagaatgtg aaagtcagaa acatataatg 6720
caagccaagc tggatgcaat tgagacccag gcctatcaac tagctggcca cagtttttct 6780
ttcaccagtt cagatgacat cgctgaggtt ttatttttgg aattgaagtt gcccccaaat 6840
agagagatga aaaaccaagg cagcaagaaa actctgggtt ctaccagaag agggattgac 6900
aatggacgca agctaaggct gggaagacag ttcagcacta gtaaggacgt tttaaataaa 6960
ttaaaggcat tacatccttt accaggcttg atattagaat ggagaagaat cactaatgct 7020
attaccaaag tggtctttcc ccttcagcgg gaaaagtgtc ttaatccttt tcttggaatg 7080
gaaagaatct atcctgtatc acagtcgcac actgctacag gacgaataac ctttacagaa 7140
ccaaatattc agaatgtgcc aagagatttt gaaatcaaaa tgccaacact agtaggagaa 7200
agcccacctt ctcaagctgt aggcaaaggc ctacttccca tgggcagagg aaaatataag 7260
aagggtttca gcgtgaatcc tagatgccag gcacagatgg aggagagagc tgcagacaga 7320
ggaatgccat tttcaattag catgcgacat gcctttgtgc ctttcccagg tggttcaata 7380
ctggctgctg actactctca gcttgaactg aggatcttgg ctcatttatc ccatgatcgt 7440
cgtctcattc aagtgttaaa cactggagct gatgttttca ggagcattgc agcagagtgg 7500
aagatgattg agccagagtc tgttggggat gatctgaggc agcaggcaaa acagatttgc 7560
tatgggatca tttatggaat gggagctaaa tctttgggag agcagatggg cattaaagaa 7620
aatgatgctg catgctatat tgactccttc aaatccagat acacagggat taatcaattc 7680
atgacagaga cagtgaagaa ttgtaaaaga gacggatttg ttcagaccat tttgggaagg 7740
cgtagatatt tgccaggaat caaagacaac aacccttatc gtaaagctca cgctgagcgt 7800
caagctatca acacaatagt ccaaggatca gcagctgata ttgtcaaaat agccacagtt 7860

aacattcaga agcaattaga gaccttccac tcaaccttca aatcccatgg tcatcgagag 7920

ggtatgctcc aaagtgaccg aacaggattg tcacgaaaga gaaaactgca agggatgttc 7980

tgcccaatca gaggaggctt cttcatcctt caactccatg atgaactcct atatgaagtg 8040

gtagaagaag atgttgttca ggtagctcag attgtcaaga atgaaatgga aagtgctgta 8100

aaactgtctg tgaaattgaa agtgaaagtg aaaataggcg ccagctgggg agagctaaag 8160

gactttgatg tgtaactgtg ctgttgatga agtcctccca gggaagcctg tgcagatgca 8220

gtcacctgga aagaacagag attacccttt cacctacctc agcaaaacaa actttcaagt 8280

cttgatagac ttagcctagt aattttatag tgagagtttc aaactatata tcagtgtcta 8340

tagcatcaaa aacttctggg ggcgtggggg aagtagaata ccaagtataa tagttacatt 8400

cactttcaaa gagcatctat gaatttgcct tttgtaactt actgtggctt taaacatatt 8460

cagaacagat gcttgaaata tgcacttagc actttggttc cacatctgtc tgggtaaacc 8520

atgaagaaaa tgaagctgct gcctcaatcg acccagacag cagccatagg cagataaaga 8580

tttggtttca ccctggtggt ggtaggcatc gtgtgtgact ttttttcctc taatatcaat 8640

tttacagtac ggaaatagta ttttaaaata gtattggcta ataaattatg aattctataa 8700

agtagtaaga cttggtatgg ttggagtgta ggaatgaata ttcatgaaat gtttcttatt 8760

gcttttcctt ccctaattca tacaatgaat gtatttggaa tacttacata ttataaaata 8820

aactatacct cttcaagagg tatcctgttc tgtaagatca gatgttttta ttgcaggtca 8880

atataatact gccagagaca gaaaataccc ccttatcagt cccttagtgc ctctttctgt 8940

ttgtggcatg gtgagaaaac ccatgctgaa aagattgtac tttgtgatcc caatcagagg 9000

gatggagcta atctttttgc tgttgaaata aaatgaattt atgagaaaaa aaaaaaaaaa 9060

a 9061

<210> 72
<211> 569
<212> DNA
<213> human

<400> 72
tggtcctttg gcgtcgtcct caagttatat tagaatcgtg tcctcccggc tttggccaac 60

ttactattct aggacttgat tccttcattc agtcacaatt tattgagcac cgactttgca 120

tcaacctctt gctgaagata acagtgctga caatatacag ccctgccctc agagcttata 180

tagtagagga gaaaaagtga acccataata tacagtcagt agcgagtatt tactaagtac 240

tttctatttg cgaggccctg ataaaagtac tgtcctggcc aggcgcggtg gctcacgcct 300

gtaattccag cactttggga ggtcgaggtg ggcagatcac ctaaggtcag gagttcgaga 360

tcagcctggc taacatgggg aaaccccgtc tctactaaaa atggaaaaat tagctgggca 420

tggtggcggg cgcctgtaat cccagctact cgggaggctg agacaggaga atgacttgaa 480

cccaggagtt gcagtggcca agataagata gcgccattgt actccagcct gggtaacaca 540

gcgagactgt gtctcaaaaa aaaaaaaaa 569

<210> 73
<211> 3213
<212> DNA
<213> human

<400> 73
aatcatcgct cgcagcggcg gcgcccgcag tggccgcagc agcgcgccgg gccctggccg 60

cgccccagcc gagcgcagcg cggagtcgcc ccgacctttc tctgcgcagt acggccgccg 120

ggaccgcagc atggcgggca tcgcggccaa gctggcgaag gaccgggagg cggccgaggg 180

gctgggctcc cacgagaggg ccatcaagta cctcaaccag gactacgagg cgctgcggaa 240

cgagtgcctg gaggccggga cgctcttcca ggacccgtcc ttcccggcca tcccctcggc 300

cctgggcttc aaggagttgg ggccctactc cagcaaaacc cggggcatga gatggaagcg 360

ccccacggag atctgcgctg acccccagtt tatcattgga ggagccaccc gcacagacat 420

ctgccaagga gccctaggtg actgctggct gctggcagcc attgcctccc tcaccttgaa 480

tgaagaaatc ctggctcgag tcgtccccct aaaccagagc ttccaggaaa actatgcagg 540

gatctttcac ttccagttct ggcaatacgg cgagtgggtg gaggtggtgg tggatgacag 600

gctgcccacc aaggacgggg agctgctctt tgtgcattca gccgaaggga gcgagttctg 660

gagcgccctg ctggagaagg catacgccaa gatcaacgga tgctatgaag ctctatcagg 720

gggtgccacc actgagggct tcgaagactt caccggaggc attgctgagt ggtatgagtt 780

gaagaagccc cctcccaacc tgttcaagat catccagaaa gctctgcaaa aaggctctct 840

ccttggctgc tccatcgaca tcaccagcgc cgcggactcg gaggccatca cgtttcagaa 900

gctggtgaag ggccacgcgt actcggtcac cggagccgag gaggttgaaa gtaacggaag 960

cctacagaaa ctgatccgca tccgaaatcc ctggggagaa gtggagtgga cagggcggtg 1020

gaatgacaac tgcccaagct ggaacactat agacccagag gagagggaaa ggctgaccag 1080

acggcatgaa gatggagaat tctggatgtc tttcagtgac ttcctgaggc actattcccg 1140

cctggagatc tgtaacctga ccccagacac tctcaccagc gatacctaca agaagtggaa 1200
actcaccaaa atggatggga actggaggcg gggctccacc gcgggaggtt gcaggaacta 1260
cccgaacaca ttctggatga accctcagta cctgatcaag ctggaggagg aggatgagga 1320
cgaggaggat ggggagagcg gctgcacctt cctggtgggg ctcattcaga agcaccgacg 1380
gcggcagagg aagatgggcg aggacatgca caccatcggc tttggcatct atgaggttcc 1440
agaggagtta agtgggcaga ccaacatcca cctcagcaaa aacttcttcc tgacgaatcg 1500
cgccagggag cgctcagaca ccttcatcaa cctccgggag gtgctcaacc gcttcaagct 1560
gccgccagga gagtacattc tcgtgccttc caccttcgaa cccaacaagg atggggattt 1620
ctgcatccgg gtcttttctg aaaagaaagc tgactaccaa gctgtcgatg atgaaatcga 1680
ggccaatctt gaagagttcg acatcagcga ggatgacatt gatgatggag tcaggagact 1740
gtttgcccag ttggcaggag aggatgcgga gatctctgcc tttgagctgc agaccatcct 1800
gagaagggtt ctagcaaagc gccaagatat caagtcagat ggcttcagca tcgagacatg 1860
caaaattatg gttgacatgc tagattcgga cgggagtggc aagctggggc tgaaggagtt 1920
ctacattctc tggacgaaga ttcaaaaata ccaaaaaatt taccgagaaa tcgacgttga 1980
caggtctggt accatgaatt cctatgaaat gcggaaggca ttagaagaag caggtttcaa 2040
gatgccctgt caactccacc aagtcatcgt tgctcggttt gcagatgacc agctcatcat 2100
cgattttgat aattttgttc ggtgtttggt tcggctggaa acgctattca agatatttaa 2160
gcagctggat cccgagaata ctggaacaat agagctcgac cttatctctt ggctctgttt 2220
ctcagtactt tgaagttata actaatctgc ctgaagactt ctcatgatgg aaaatcagcc 2280
aaggactaag cttccataga aatacacttt gtatctggac ctcaaaatta tgggaacatt 2340
tacttaaacg gatgatcata gctgaaaata atgatactgt caatttgaga tagcagaagt 2400
ttcacacatc aaagtaaaag atttgcatat cattatacta aatgcaaatg agtcgcttaa 2460
cccttgacaa ggtcaaagaa agctttaaat ctgtaaatag tatacacttt ttacttttac 2520
acactttcct gttcatagca atattaaatc aggaaaaaaa aatgcaggga ggtatttaac 2580
agctgagcaa aaacattgag tcgctctcaa aggacacgag gcccttggca gggaatattt 2640
aaagcaactt caagtttaaa atgcagctgt tgattctacc aaacaacagt ccaagattac 2700
catttcccat gagccaactg ggaaacatgg tatatcatga agtaatcttg tcaaggcatc 2760
tggagagtcc aggagaggag actcacctct gtcgcttggg ttaaacaaga gacaggtttt 2820

gtagaatatt gattggtaat agtaaatcgt tctccttaca atcaagttct tgaccctatt 2880

cggccttata catctggtct tacaaagacc aaagggatcc tgcgcttgat caactgaacc 2940

agtatgccaa aaccaggcat ccaatttgta aaccaattat gataaaggac aaaataagct 3000

gtttgccacc tcaaaacttt atgaacttca ccaccactag tgtctgtcca tggagttaga 3060

ggggacatca cttagaagtt cttatagaaa ggacacaagt ttgtttcctg gctttacctt 3120

gggaaaatgc tagcaacatt atagaaattt tgccttgttg ccttatcttc ttccaaatgt 3180

actgttaaat aaaaataaag ggttacccca tcg 3213

<210> 74
<211> 1847
<212> DNA
<213> human

<400> 74
ttgcgcgccg cccggccagg cccgcaaaga ggcctccgag cgccatggct gcgcccccgg 60

cccgcgcgga cgctgatcct tcgcccacgt cgccacctac ggcccgagac acaccaggcc 120

ggcaggctga gaaaagcgag accgcgtgcg aggaccgcag caatgcagag tccctggaca 180

ggctcctgcc acctgtgggc actgggcgct ctccccggaa gcggaccacc agccagtgca 240

agtcagagcc tcccctgctg cgtacaagca agcgtaccat ctacaccgcc gggcggccgc 300

cctggtacaa tgaacacggc acgcaatcca aagaggcctt cgccatcggc ttgggaggcg 360

gcagtgcctc tgggaagacc actgtggcca gaatgatcat cgaggccctg gatgtgccct 420

gggtggtctt gctgtccatg gactccttct acaaggtgct gactgagcag cagcaggaac 480

aggccgcaca caacaacttc aacttcgacc acccagatgc ctttgacttc gacctcatca 540

tttccaccct caagaagctg aagcagggga agagtgtcaa ggtgcccatt tatgacttca 600

ccacgcacag ccggaagaag gactggaaaa cactgtatgg tgcaaacgtc atcatctttg 660

agggcatcat ggcctttgct gacaagacac tgttggagct cctggacatg aagatctttg 720

tggacacaga ctccgacatc cgcctggtac ggcggctgcg ccgggacatc agtgagcgcg 780

gccgggacat cgagggtgtc atcaagcagt acaacaagtt tgtcaagccc tccttcgacc 840

agtacatcca gcccaccatg cgcctggcag acatcgtggt ccccagaggg agcggcaaca 900

cggtggccat caacctgatt gtgcagcacg tgcacagcca gctggaggag cgtgaactca 960

gcgtcagggc tgcgctggcc tcggcacacc agtgccaccc gctgccccgg acgctgagcg 1020

tcctgaagag cacgccgcag gtacggggca tgcacaccat catcagggac aaggagacca 1080

gtcgcgacga gttcatcttc tactccaaga gactgatgcg gctgctcatc gagcacgcgc 1140

tctccttcct gccctttcag gactgcgtcg tacagacccc gcaggggcag gactatgcgg 1200

gcaagtgcta tgcggggaag cagatcaccg gtgtgtccat tctgcgcgcc ggtgaaacca 1260

tggagcccgc gctgcgcgct gtgtgcaaag acgtgcgcat cggcaccatc ctcatccaga 1320

ccaaccagct taccggggag cccgagctcc actacctgag gctgcccaag gacatcagcg 1380

atgaccacgt gatcctcatg gactgcaccg tgtccacggg cgcggcggcc atgatggcag 1440

tgcgcgtgct cctggaccac gacgtgcctg aggacaagat ctttttgctg tcgctgctca 1500

tggcagagat gggcgtgcac tcagtggcct atgcatttcc gcgagtgaga atcatcacca 1560

cggcggtgga caagcgggtc aatgaccttt tccgcatcat cccaggcatt gggaactttg 1620

gcgaccgcta ctttgggaca gacgcggtcc ccgatggcag tgacgaggag gaagtggcct 1680

acacgggtta gctgcccagt gagccatccc gtccccacca ccctcctcct gcctcctgac 1740

ccaggactgt tgaatacaaa gatgttaatt tttaaaatgt tactagtata atttattcta 1800

tgcattttat aaaataaata aagctttaga aaaaaaaaaa aaaaaaa 1847

<210> 75
<211> 812
<212> DNA
<213> human

<400> 75
cagcggaggt ggcgctggct aatctcaaga acaaatatga aaatgaaaaa gcaatggtga 60

ctgaaaccat gacgaagctt agaaatgaac tgaaggcttt gaaagaagat gctgcaacct 120

tctcatccct gagagcaatg tttgcaacaa gatgtgatga atatgtcacc cagttggatg 180

agatgcagag acagttagca gctgcagagg atgagaagaa gactctgaac actttgttac 240

gaatggctat ccagcaaaaa ctcgccctga cccagaggct ggagggctta gagtttgacc 300

atgagcagtc ccgacgcagc aaaggcaaac ttggaaagag caagatcggc agccctaaag 360

taagtgggga ggcatcagtc accgtgccca ccatagacac ttacctcctg catagtcagg 420

gcccacagac acccaacatt cgggtcagca gtggcactca gaggaaaagg tatgcatgca 480

gcgatcttca tagtacggtg cagtggccag attttagtta actgcaaaaa taaatgtgct 540

cttgttgtgg aggatggagg aggggaagca aaagaaaaaa tgggagctgg catataaatg 600

gtcttgctaa tgtggctctt tccagatcaa aacctttttg ataattgtgt ttatgtagtc 660

ctttctaacc ccctgcccaa tccctcctct tgattaatcg taatataatt ttcaaggtct 720

gttaatattt ttgctactct ttgtatgtgt aaatatgcct ctcccttacc tccaacctga 780

gaattggtag ataaaggtgg atattaagaa tg 812

<210> 76
<211> 1841
<212> DNA
<213> human

<400> 76
actgcgccgc caccgtcaat aggtggaccc cctcccggag ataaaaccgc cggcgccggc 60

gccgccagtc cctctggctg agacctcggc tccggaatca ctgcagcccc cctcgccctg 120

agccagagca ccccgggtcc cgccagcccc tcacactccc agcaaaatgg gcaaggagaa 180

gacccacatc aacatcgtgg tcatcggcca cgtggactcc ggaaagtcca ccaccacggg 240

ccacctcatc tacaaatgcg gaggtattga caaaaggacc attgagaagt tcgagaagga 300

ggcggctgag atggggaagg gatccttcaa gtatgcctgg gtgctggaca agctgaaggc 360

ggagcgtgag cgcggcatca ccatcgacat ctccctctgg aagttcgaga ccaccaagta 420

ctacatcacc atcatcgatg cccccggcca ccgcgacttc atcaagaaca tgatcacggg 480

tacatcccag gcggactgcg cagtgctgat cgtggcggcg ggcgtgggcg agttcgaggc 540

gggcatctcc aagaatgggc agacgcggga gcatgccctg ctggcctaca cgctgggtgt 600

gaagcagctc atcgtgggcg tgaacaaaat ggactccaca gagccggcct acagcgagaa 660

gcgctacgac gagatcgtca aggaagtcag cgcctacatc aagaagatcg gctacaaccc 720

ggccaccgtg ccctttgtgc ccatctccgg ctggcacggt gacaacatgc tggagccctc 780

cccaacatg ccgtggttca agggctggaa ggtggagcgt aaggagggca acgcaagcgg 840

cgtgtccctg ctggaggccc tggacaccat cctgcccccc acgcgcccca cggacaagcc 900

cctgcgcctg ccgctgcagg acgtgtacaa gattggcggc attggcacgg tgcccgtggg 960

ccgggtggag accggcatcc tgcggccggg catggtggtg acctttgcgc cagtgaacat 1020

caccactgag gtgaagtcag tggagatgca ccacgaggct ctgagcgaag ctctgcccgg 1080

cgacaacgtc ggcttcaatg tgaagaacgt gtcggtgaag gacatccggc ggggcaacgt 1140

gtgtggggac agcaagtctg acccgccgca ggaggctgct cagttcacct cccaggtcat 1200

catcctgaac cacccggggc agattagcgc cggctactcc ccggtcatcg actgccacac 1260

agcccacatc gcctgcaagt ttgcggagct gaaggagaag attgaccggc gctctggcaa 1320

gaagctggag gacaacccca agtccctgaa gtctggagac gcggccatcg tggagatggt 1380

gccgggaaag cccatgtgtg tggagagctt ctcccagtac ccgcctctcg gccgcttcgc 1440

cgtgcgcgac atgaggcaga cggtggccgt aggcgtcatc aagaacgtgg agaagaagag 1500

cggcggcgcc ggcaaggtca ccaagtcggc gcagaaggcg cagaaggcgg gcaagtgaag 1560

cgcgggcgcc cgcggcgcga ccctccccgg cggcgccgcg ctccgaaccc cggcccggcc 1620

cccgccccgc ccccgccccg cgcgccgctc cggcgccccg cacccccgcc aggcgcatgt 1680

ctgcacctcc gcttgccaga ggccctcggt cagcgactgg atgctcgcca tcaaggtcca 1740

gtggaagttc ttcaagagga aaggcgcccc cgccccaggc ttccgcgccc agcgctcgcc 1800

acgctcagtg cccgttttac caataaactg agcgacccca g 1841

<210> 77
<211> 2837
<212> DNA
<213> human

<400> 77
tgaaaagatc cttacggctt ctgttgtttc atagcttctg cttacagtgt tctgggtatc 60

cttcagtttt gctaccaccc tatagcctct cagtgtgcct cacatttaga tacctccaga 120

gagaatccga ttggattctt cagccattat ctaatatagg ggtgcatggc aaccagtgca 180

taaggaacca cttgtggcta cttactcaga ggagacttca gatctaggag acagtcagag 240

taccatgtcc agaatatata ccatgatgaa ttagcaagat gcagttttca atacatagat 300

gcaaagtgat tatggtatga gaattttta atctcagatt ttgtaatttg cttttacaaa 360

gcacccattt gacagcacct tgtttaaaga ccacaaaaag catgtcctta aaactatagt 420

cacattcttg gtgctccctt cactttgttt ggggtattaa tactactctg aggctgccat 480

gggaccacct gttccaaaaa tattctttga tgtggattga gtagttttta ctgtgattat 540

tctaccaaga taaacataga atttattgcc tttcacagta ataataagaa aacattgtta 600

aagaaggttt cggccaggca cggtggctca cgcctgtaat cccagcactt tgggaggccg 660

ccgagccagg cagatcacaa ggtcaggagt ttgagaccag cctgaccaac gtggtgaaat 720

cccatctcta ctaaaaatac aaaaattagc cgggcatggt agcacacacc tgtaatccca 780

gctactcagg aggctgaggc aggagaatca cttgaaccgg ggaggtggag gttgcagtga 840

gctcacatca cgccactgta ctccagccta ggtgacagag cgagactctg tctcaaaaaa 900

aaaaaaaaaa aaaaactttc tttggttgac taacaatttt tcattgttct aatgggtgtc 960

ttcctccttc acaagggagg aagatcctcc aaagtatctg tgagtataat cttataactg 1020

gtgtttttgt taagaaataa ttaggagtca ccaggcactc catttttttaa gttcacaaat 1080
ttcaaacttt ttttttaaag atttacttcc tcattttaat agggacagaa ggataaaaag 1140
tatatttctg ccatcttagg gggatggaag ttttggtaac tctctctcct ttgaaggtcc 1200
tggaatcgtt tattataata tataccattt agtatacatg ttgggctgtc tgaatgagaa 1260
catacccatc agtgtgcaca tgtgcttaca aacatgttca ttcatttagt gaaattaaga 1320
gtggaagcag gctgttttca gaaaaaatat ttgagggctg agttccctct ttcattcatc 1380
ccatgagggt attactaggg ggtttaatat atttgggtta atgatgaggg ggaaaaaata 1440
gcatcctttt ggggaaatag aaccagacac aatttaaagt gggacccaaa tagaaacata 1500
attcttttat gaaacaaatg ttttattttc tgtttgtcac acggatgcca tctaaatttt 1560
aaaagaaatc taggcttcct cttattcttt atgttttttt taaaagcaag aagtcagaga 1620
ctttgtacat catgattaac ttctacattt gtttgagcta tgtgtcacaa gtaattaagt 1680
tataaatatt ctgaagatta attggtgcac actctaagtg ctttttaaat tttacacagc 1740
ttacctttct ttctgaagaa tgtcctcaca aagatagatt atatcaattt ctccactgat 1800
gatgtattcc agcaataaac atagtcctag aattgtagaa tgtgcactgc tgcactctgt 1860
cctaacagac agtagacaag gtttataatt tttttctatg ggacactaaa aataccctag 1920
gatttccttt aactttttgc actgacaggt ctaaagcaaa gccacagaag ctgctcttgc 1980
tcagtaagta ttgttaatgc tgcagtgcat attaaaattt cagaaaaccc aattatgtac 2040
tatctaggtg tggatagctc tcattgttag tatcctaaat catctaaagg cttgttttct 2100
ttcttttttt ttttttttaca gacctaacta ccataatgaa tgctgcatat taagagaaac 2160
cacaagaagg ttatatgttt ggttgtctaa tattcttgga tttgatatga accaacacat 2220
agtccttgtt gtcattgaca gaaccccagt ttgtatgtac attattcata ttcctctctg 2280
ttgtgtttcg gggggaaaag acattttagc ctttttttaaa agttactgat ttaatttcat 2340
gttatttggt tgcatgaagt tgcccttaac cactaaggat tatcaagatt tttgcgcaga 2400
cttatacatg tctaggatcc ttttatcaag gcagttatga tcatcgtttt cctgccttga 2460
ccccaccatc atcaaacact cagttaaata taaattaaca ttttttagat gaccactcaa 2520
cataatgctt aagaatggaa tttcctctct gtgacagaac ccaggaatta attcctaaat 2580
acataacgtt ggtatattga agacgaaatt aaaattgtcc ttcagttttg aggccatgtg 2640
taaagtttaa ccatattgta aaatatctat tccgtattag aaatagctag ttgacagctt 2700
atacttctca aaattcatat tgttatgtac acaaactaag tttctatatg tgaagttagt 2760

gagtcttttt gtgttactcc aaaataaagg caatgattta ttttttaaaa aaaaaaaaaa 2820

aaaaaaaaaa aaaaaag 2837

<210> 78
<211> 1684
<212> DNA
<213> human

<400> 78
cgacggagtg ccaggagcac taacagtacc cttagcttgc tttcctcctc cctccttttt 60

attttcaagt tccttttat ttctccttgc gtaacaacct tcttcccttc tgcaccactg 120

cccgtaccct tacccgcccc gccacctcct tgctacccca ctcttgaaac cacagctgtt 180

ggcagggtcc ccagctcatg ccagcctcat ctcctttctt gctagccccc aaagggcctc 240

caggcaacat gggggcccca gtcagagagc cggcactctc agttgccctc tggttgagtt 300

ggggggcagc tctgggggcc gtggcttgtg ccatggctct gctgacccaa caaacagagc 360

tgcagagcct caggagagag gtgagccggc tgcaggggac aggaggcccc tcccagaatg 420

gggaagggta tccctggcag agtctcccgg agcagagttc cgatgccctg gaagcctggg 480

agaatgggga gagatcccgg aaaaggagag cagtgctcac ccaaaaacag aagaatgact 540

ccgatgtgac agaggtgatg tggcaaccag ctcttaggcg tgggagaggc ctacaggccc 600

aaggatatgg tgtccgaatc caggatgctg gagtttatct gctgtatagc caggtcctgt 660

ttcaagacgt gactttcacc atgggtcagg tggtgtctcg agaaggccaa ggaaggcagg 720

agactctatt ccgatgtata agaagtatgc cctcccaccc ggaccgggcc tacaacagct 780

gctatagcgc aggtgtcttc catttacacc aaggggatat tctgagtgtc ataattcccc 840

gggcaagggc gaaacttaac ctctctccac atggaacctt cctggggttt gtgaaactgt 900

gattgtgtta taaaaagtgg ctcccagctt ggaagaccag ggtgggtaca tactggagac 960

agccaagagc tgagtatata aaggagaggg aatgtgcagg aacagaggcg tcttcctggg 1020

tttggctccc cgttcctcac ttttcccttt tcattcccac cccctagact ttgattttac 1080

ggatatcttg cttctgttcc ccatggagct ccgaattctt gcgtgtgtgt agatgagggg 1140

cgggggacgg gcgccaggca ttgttcagac ctggtcgggg cccactggaa gcatccagaa 1200

cagcaccacc atctagcggc cgctcgaggg aagcacccgc cggttggccg aagtccacga 1260

agccgccctc tgctagggaa aacccctggt tctccatgcc acacctctct ccaggtgccc 1320

tctgcctctt caccccacaa gaagccttat cctacgtcct tctctccatc tatcggaccc 1380

cagtttccat cactatctcc agagatgtag ctattatgcg cccgtctaca gggggtgccc 1440

gacgatgacg gtgccttcgc agtcaaatta ctcttcgggt cccaaggttt ggctttcacg 1500

cgctccattg ccccggcgtg gcaggccatt ccaagccctt ccgggctgga actggtgtcg 1560

gaggagcctc gggtgtatcg tacgccctgg tgttggtgtt gcctcactcc tctgagctct 1620

tctttctgat caagccctgc ttaaagttaa ataaaataga atgaatgata aaaaaaaaaa 1680

aaaa 1684

<210> 79
<211> 1973
<212> DNA
<213> human

<400> 79
agccgccgcc tcgccgcttc ccctcgtcgg agcggccgct cgtccgcccg gcttgaggcc 60

cgcggggagc gcggcgcaat tcgtcggccc gcgggggggc ggcctcccgg catcttcgcg 120

gcgaccaagg actaccagga aggggagcgg ctgggatggc gcgtccgcgg ccccgcgagt 180

acaaagcggg cgacctggtc ttcgccaaga tgaagggcta cccgcactgg ccggcccgga 240

ttgatgaact cccagagggc gctgtgaagc ctccagcaaa caagtatcct atcttctttt 300

ttggcaccca tgaaactgca tttctaggtc ccaaagacct tttccatat aaggagtaca 360

aagacaagtt tggaaagtca aacaaacgga aaggatttaa cgaaggattg tgggaaatag 420

aaaataaccc aggagtaaag tttactggct accaggcaat tcagcaacag agctcttcag 480

aaactgaggg agaaggtgga aatactgcag atgcaagcag tgaggaagaa ggtgatagag 540

tagaagaaga tggaaaaggc aaaagaaaga atgaaaaagc aggctcaaaa cggaaaaagt 600

catatacttc aaagaaatcc tctaaacagt cccggaaatc tccaggagat gaagatgaca 660

aagactgcaa agaagaggaa aacaaaagca gctctgaggg tggagatgcg ggcaacgaca 720

caagaaacac aacttcagac ttgcagaaaa ccagtgaagg gacctaacta ccataatgaa 780

tgctgcatat taagagaaac cacaagaagg ttatatgttt ggttgtctaa tattcttgga 840

tttgatatga accaacacat agtccttgtt gtcattgaca gaaccccagt ttgtatgtac 900

attattcata ttcctctctg ttgtgtttcg gggggaaaag acattttagc ctttttttaaa 960

agttactgat ttaatttcat gttatttggt tgcatgaagt tgcccttaac cactaaggat 1020

tatcaagatt tttgcgcaga cttatacatg tctaggatcc ttttatcaag gcagttatga 1080

tcatcgtttt cctgccttga ccccaccatc atcaaacact cagttaaata taaattaaca 1140

```
tttttagat gaccactcaa cataatgctt aagaatggaa tttcctctct gtgacagaac    1200
ccaggaatta attcctaaat acataacgtt ggtatattga agacgaaatt aaaattgtcc    1260
ttcagttttg aggccatgtg taaagtttaa ccatattgta aaatatctat tccgtattag    1320
aaatagctag ttgacagctt atacttctca aaattcatat tgttatgtac acaaactaag    1380
tttctatatg tgaagttagt gagtcttttt gtgttactcc aaaataaagg caatgattta    1440
ttttttccc agtgccaata caattttgag ctaagcactc aaggtggata ctttacattt    1500
taaagctgga atcagcaaca gccctatggg aaaccagaca aagcattgac ttttaaatgt    1560
agacttttaa aataaactgt tttcttttgg aactacaatt agaatagtta atattcatcc    1620
ttaaaccatt attatgtgta cattattgtt gctattgtga taatagagaa ttttatttat    1680
ttttatgcca gcttatattg tgagaacaca tttagtcagt ttgggtttta tcaatcctgt    1740
taatgcttgt ccttggaaca tctttcgcgt attcacggtt tgtagttgaa aagtttactg    1800
taaaaaaatc aaaaacaaaa aaatgtattg tttttacaga ataaatttat tgggatgtgt    1860
actgggagta agatttgagg ttgtaagcaa actaagttag tgtaatttgg cttcatatat    1920
gtaacgtgag gtattaatgt aattcatata ttaaagcaaa aattgttcgc agc          1973
```

```
<210>  80
<211>  1230
<212>  DNA
<213>  human

<400>  80
catagcaagt ctgccatggg ccgcggggcc cgtgtcccct cggaggcccc gggggcaggc      60
gtcgagcgcc gctggcttgg agccgcgctg gtcgccctgt gcctcctccc cgcgctggtg     120
ctgctggccc ggctgggggc cccggcggtg ccggcctgga gcgcagcgca gggagacgtc     180
gctgcgctgg gcctctcggc ggtgcccccc acccgggtcc cgggcccact ggcccccgc      240
agacgccgct acacactgac tccagccagg ctgcgctggg accacctcaa cctcacctac     300
aggatcctct ccttcccgcg gaacctgctg agcccgcggg agacgcggcg ggccctagct     360
gccgccttcc gcatgtggag cgacgtgtcc cccttcagct tccgcgaggt ggcccccgag     420
cagcccagcg acctccggat aggcttctac ccgatcaacc acacggactg cctggtctcc     480
gcgctgcacc actgcttcga cggccccacg ggggagctgg cccacgcctt cttccccccg     540
cacggcggca tccacttcga cgacagcgag tactgggtcc tgggccccac gcgctacagc     600
tggaagaaag gcgtgtggct cacggacctg gtgcacgtgg cggcccacga gatcggccac     660
```

gcgctgggcc tgatgcactc acaacacggc cgggcgctca tgcacctgaa cgccacgctg 720

cgcggctgga aggcgttgtc ccaggacgag ctgtgggggc tgcaccggct ctacggatgc 780

ctcgacaggc tgttcgtgtg cgcgtcctgg gcgcggaggg gcttctgcga cgctcgccgg 840

cggctcatga agaggctctg ccccagcagc tgcgacttct gctacgaatt ccccttcccc 900

acggtggcca ccaccccacc gccccccagg accaaaacca ggctggtgcc cgagggcagg 960

aacgtgacct tccgttgcgg ccagaagatc ctccacaaga aagggaaagt gtactggtac 1020

aaggaccagg agcccctgga gttctcctac cccggctacc tggccctggg cgaggcgcac 1080

ctgagcatca tcgccaacgc cgtcaatgag ggcacctaca cctgcgtggt gcgccgccag 1140

cagcgcgtgc tgaccaccta ctcctggcga gtccgtgtgc ggggctgagc ccggctgata 1200

aagcactttc tctctgaaaa aaaaaaaaaa 1230

<210> 81
<211> 494
<212> DNA
<213> human

<400> 81
cagacagtcc gggagcacca gctcgctatc aagcagctgg acgccccgct gaggccgtag 60

acgtccacca gggcccagag cgggtcggcc gtgcggaccc gcatgaagaa cagcataaca 120

gccgagtcgt tgatgcggtg gaagacacgg cccttcttgt ccacccagaa tgcgatgatg 180

ttgccctcat tggcaaactc ctcaggcagc gcttggccca gaagccactc tgggacacca 240

ggtcggggca ggcgtacttg ggcagcgagt cagggtggat gcgggacggg tccttgctgg 300

tgaagcccag ccgcaggccc cgctccagca gcactgcttc ttggtgatct tcagcctgac 360

ttgctcgtag atgaggaccg ggcggttgct gaaggtgatg gcgttgcaga agctggcctg 420

cctcttgaca gccttgtggc tgaggtccat gaggatctgg gagccttggt gtgcgggtgg 480

aagagcagcg cgtg 494

<210> 82
<211> 3311
<212> DNA
<213> human

<400> 82
cgcccgggca ggtgaaaggt tttgtctatt tctgttcctt gttgaatctc tagtacctag 60

aaccatgtct ggtcctgatt aggcacttgg tgaatgtttt tgaatgaata ctctgagctg 120

ttctttaatc ggtcctcatg atcctcattg tacagatgag ggaactctgg gcgaacctgg 180
cctgggtctt gagctaatta cgggtaggtc aggttctgta cccaagtagt acacacagtg 240
atgggcgggg tggcctgggg ccgtggtttg tcaacccta cgctggaggg tgatgttttg 300
gtacaagagg agaggtgccc caatgcgtcc tgtgtctgta attgatggcg ttgtctgtgt 360
ttccccagga tgtggttctg agacagtccc tgtccctgat ggcccacgga gcgactcggt 420
ggaaggaagt cccttccgtc ccccgtcaca ctccttctct gccgtcttcg atgaagacaa 480
gccgatagcc agcagtggga cttacaactt ggactttgac aacattgagc ttgtggatac 540
ctttcagacc ttggagcctc gtgcctcaga cgctaagaat caggagggca aagtgaacac 600
acggaggaag tccacggatt ccgtccccat ctctaagtct acactgtccc ggtcgctcag 660
cctgcaagcc agtgactttg atggtgcttc ttcctcaggc aatcccgagg ccgtggccct 720
tgccccagat gcatatagca cgggttccag cagtgcttct agtaccctta agcgaactaa 780
aaaaccgagg ccgccttcct taaaaaagaa acagaccacc aagaaaccca cagagacccc 840
cccagtgaag gagacgcaac aggagccaga tgaagagagc cttgtcccca gtggggagaa 900
tctagcatct gagacgaaaa cggaatctgc caagacggaa ggtcctagcc cagccttatt 960
ggaggagacg ccccttgagc ccgctgtggg gcccaaagct gcctgccctc tggactcaga 1020
gagtgcagaa ggggttgtcc ccccggcttc tggaggtggc agagtgcaga actcaccccc 1080
tgtcgggagg aaaacgctgc ctcttaccac ggccccggag gcaggggagg taaccccatc 1140
ggatagcggg gggcaagagg actctccagc caaagggctc tccgtaaggc tggagtttga 1200
ctattctgag gacaagagta gttgggacaa ccagcaggaa aacccccctc ctaccaaaaa 1260
gataggcaaa aagccagttg ccaaaatgcc cctgaggagg ccaaagatga aaaagacacc 1320
cgagaaactt gacaacactc ctgcctcacc tcccagatcc cctgctgaac ccaatgacat 1380
ccccattgct aaaggtactt acacctttga tattgacaag tgggatgacc ccaattttaa 1440
cccttttct tccacctcaa aaatgcagga gtctcccaaa ctgccccaac aatcatacaa 1500
ctttgaccca gacacctgtg atgagtccgt tgaccccttt aagacatcct ctaagacccc 1560
cagctcacct tctaaatccc cagcctcctt tgagatccca gccagtgcta tggaagccaa 1620
tggagtggac ggggatgggc taaacaagcc cgccaagaag aagaagacgc ccctaaagac 1680
tgacacattt agggtgaaaa agtcgccaaa acggtctcct ctctctgatc caccttccca 1740
ggaccccacc ccagctgcta caccagaaac accaccagtg atctctgcgg tggtccacgc 1800
cacagatgag gaaaagctgg cggtcaccaa ccagaagtgg acgtgcatga cagtggacct 1860

agaggctgac aaacaggact acccgcagcc ctcggacctg tccacctttg taaacgagac 1920

caaattcagt tcacccactg aggagttgga ttacagaaac tcctatgaaa ttgaatatat 1980

ggagaaaatt ggctcctcct tacctcagga cgacgatgcc ccgaagaagc aggccttgta 2040

ccttatgttt gacacttctc aggagagccc tgtcaagtca tctcccgtcc gcatgtcaga 2100

gtccccgacg ccgtgttcag ggtcaagttt tgaagagact gaagcccttg tgaacactgc 2160

tgcgaaaaac cagcatcctg tcccacgagg actggcccct aaccaagagt cacacttgca 2220

ggtgccagag aaatcctccc agaaggagct ggaggccatg ggcttgggca ccccttcaga 2280

agcgattgaa attagagagg ctgctcaccc aacagacgtc tccatctcca aaacagcctt 2340

gtactcccgc atcgggaccg ctgaggtgga gaaacctgca ggccttctgt tccagcagcc 2400

cgacctggac tctgccctcc agatcgccag agcagagatc ataaccaagg agagagaggt 2460

ctcagaatgg aaagataaat atgaagaaag caggcgggaa gtgatggaaa tgaggaaaat 2520

agtggccgag tatgagaaga ccatcgctca gatgatagag gacgaacaga gagagaagtc 2580

agtctcccac cagacggtgc agcagctggt tctggagaag gagcaagccc tggccgacct 2640

gaactccgtg gagaagtctc tggccgacct cttcagaaga tatgagaaga tgaaggaggt 2700

cctagaaggc ttccgcaaga atgaagaggt gttgaagaga tgtgcgcagg agtacctgtc 2760

ccgggtgaag aaggaggagc agaggtacca ggccctgaag gtgcacgcgg aggagaaact 2820

ggacagggcc aatgctgaga ttgctcaggt tcgaggcaag gcccagcagg agcaagccgc 2880

ccaccaggcc agcctgcgga aggagcagct gcgagtggac gccctggaaa ggacgctgga 2940

gcagaagaat aaagaaatag aagaactcac caagatttgt gacgaactga ttgccaaaat 3000

ggggaaaagc taactctgaa ccgaatgttt tggacttaac tgttgcgtgc aatatgaccg 3060

tcggcacact gctgttcctc cagttccatg gacaggttct gttttcactt tttcgtatgc 3120

actactgtat ttcctttcta aataaaattg atttgattgt atgcagtact aaggagacta 3180

tcagaatttc ttgctattgg tttgcatttt cctagtataa ttcatagcaa gttgacctca 3240

gagttcctgt atcagggaga ttgtctgatt ctctaataaa agacacattg ctgaaaaaaa 3300

aaaaaaaaaa a 3311

<210> 83
<211> 599
<212> DNA
<213> human

<220>
<221> misc_feature
<222> (415)..(415)
<223> n is a, c, g, or t

<400> 83
tttttcctt caaaaaatag tttattctgc acatttccta gtaggctctc tgcccaccgt 60

tccagggtag cagctactca taacttgtct ttctctccaa aaccaagagg gccttcccaa 120

cagaaaaacc ttcagttccc aaagcagcat cgattcttcc cctcacccca gcaaacctcg 180

gggtggaata atgaatcatt caccttctcc cacccctcac tgccccgccc caccttcatt 240

tgccaggact gtcactggct gatggtcaga ttttgtcttt ttcaacttgg ataccctcaa 300

gcatcccagc ttttggaggc ttccctgggc aacctcatgg gcttccacat tgaccaggca 360

ttacattcag catccttgga caaaccaccc atcagagcag tgggttgtgg ccagntccct 420

ccacccccga ctccgcccca atggaagaaa ccagaggatc acagggcccc aggctgtgcc 480

gcctgcagag ccaggagctg ctgggtgcat tgcaacgttc tccctgagca aatcggcatg 540

gtcaccacag tccagtcctc agtcaccgac agctctgccc ctcggggtca gatcccacc 599

<210> 84
<211> 666
<212> DNA
<213> human

<220>
<221> misc_feature
<222> (517)..(517)
<223> n is a, c, g, or t

<220>
<221> misc_feature
<222> (555)..(555)
<223> n is a, c, g, or t

<220>
<221> misc_feature
<222> (635)..(635)
<223> n is a, c, g, or t

<220>
<221> misc_feature
<222> (638)..(638)
<223> n is a, c, g, or t

<220>
<221> misc_feature
<222> (663)..(663)

<223> n is a, c, g, or t

<400> 84
cggggcgccc gagggcgtgg aggccgcgga ggccgaggaa caggaggagg cggaggagga 60

gcaggacgag gccgaagagc aggagggcgg cggcggcgtg cgcgacgtgg ggctgtcgag 120

cagcagcggc gactcgaggc cgcccggggg ctgatggtgg ccagacggga agcccgagaa 180

gctgaggctg tggtgcaact tgggccgcgg ctcccgcggg aagcccaggt gcaacgcatc 240

gcgcgtgcca aaggcacgca ggtccccgga ggcgcccccc gacggcgcgg gccgccgctc 300

gtcagcgttg tggatgaagt ggcagcgcgg cccatagggg cagaagccga tggtatgaaa 360

ggtgcggcac agctcggtct tgtactttcg attgcaagtc aggctgcgca gctcgtggaa 420

gccatgcgcg aactggcact tttcgccgta cttgcacgtg ccgctctcct cgaagggccg 480

gcacagctcg gtcttgtagc gcgtggagtt gatctgngag ccgccgcccc ccttctgctg 540

ctgctgcagg tgcangaggt gctggctgcg atcgccgttc tcgctaaacg agcggtcccg 600

gaatttgttc tcctttgtga gcaaggctgt gccgncgncc cccgacggct tctttagggt 660

gcngta 666

<210> 85
<211> 694
<212> DNA
<213> human

<220>
<221> misc_feature
<222> (640)..(640)
<223> n is a, c, g, or t

<220>
<221> misc_feature
<222> (686)..(686)
<223> n is a, c, g, or t

<400> 85
tttttttttt tttttttttt ttttttgagg gtatctaggc caatttattc agctggaaat 60

caatctgtcc agggctggac caggcgcctc cccctcgata cagctcctgg ctgaagaggt 120

catcaaagca gcagctgtcc ccaggccagt gtcacgtcac tgcgtacagt tcctcccgat 180

cgttcttgca aatctgatag tggcagggga atttttgcga gcaggcccag ggctcggcgg 240

gctggtcagg tggcggcagc agcaaggctg cgctcccggc cagcaggatg tgccaaatgc 300

tgtgggtgta gtagtagttg tcgctagtca tcatggaggt gtagatggcg atgcccacag 360

```
aggccataaa gaccccgggc aggaggtaaa aggcccagcg ctgccacaag gtggggtagc     420

actggcgccg gtgcccgcag cggtaagcgt atttcaggac tgtcttgagc cgtgccatgc     480

acaggatggt gacccagatg gccgccccgg agcccaagaa gtcgcagtac tgcagcgtgt     540

cgtagctgag gatgcacagc accgcctccc cgggctggtc gcaggcgtgg tagaacgtgg     600

agaagaacat ggtgtaggcg tagaccgagg ccttcaccan gaaaaatcgc cgcactgaga     660

ccgcgatggg ggccagaaac atgagngtgc taac                                 694
```

<210> 86
<211> 3091
<212> DNA
<213> human

<400> 86

```
gggagaccca agcttctaga gatccctcga cctcgagatc cattgtgctc taaagagcgg      60

accccagcct ctgccaggtt cggtccgcca tcctcgtccc gtcctccgcc ggcccctgcc     120

ccgcgcccag ggatcctcca gctcctttcg cccgcgccct ccgttcgctc cggacaccat     180

ggacaagttt tggtggcacg cagcctgggg actctgcctc gtgccgctga gcctggcgca     240

gatcgatttg aatataacct gccgctttgc aggtgtattc cacgtggaga aaaatggtcg     300

ctacagcatc tctcggacgg aggccgctga cctctgcaag gctttcaata gcaccttgcc     360

cacaatggcc cagatggaga aagctctgag catcggattt gagacctgca ggtatgggtt     420

catagaaggg catgtggtga ttccccggat ccaccccaac tccatctgtg cagcaaacaa     480

cacaggggtg tacatcctca catacaacac ctcccagtat gacacatatt gcttcaatgc     540

ttcagctcca cctgaagaag attgtacatc agtcacagac ctgcccaatg cctttgatgg     600

accaattacc ataactattg ttaaccgtga tggcacccgc tatgtccaga aaggagaata     660

cagaacgaat cctgaagaca tctaccccag caaccctact gatgatgacg tgagcagcgg     720

ctcctccagt gaaaggagca gcacttcagg aggttacatc ttttacacct tttctactgt     780

acaccccatc ccagacgaag acagtccctg gatcaccgac agcacagaca gaatccctgc     840

taccactttg atgagcacta gtgctacagc aactgagaca gcaaccaaga ggcaagaagc     900

ctgggattgg ttttcatggt tgtttctacc atcagagtca aagaatcatc ttcacacaac     960

aacacaaatg gctggtacgt cttcaaatac catctcagca ggctgggagc caaatgaaga    1020

aaatgaagat gaaagagaca gacacctcag tttttctgga tcaggcattg atgatgatga    1080

agattttatc tccagcacca tttcaaccac accacgggcc tttgaccaca caaaacagaa    1140
```

```
ccaggactgg acccagtgga acccaagcca ttcaaatccg gaagtgctac ttcagacaac   1200
cacaaggatg actgatgtag acagaaatgg caccactgct tatgaaggaa actggaaccc   1260
agaagcacac cctcccctca ttcaccatga gcatcatgag gaagaagaga ccccacattc   1320
tacaagcaca atccaggcaa ctcctagtag tacaacggaa gaaacagcta cccagaagga   1380
acagtggttt ggcaacagat ggcatgaggg atatcgccaa acacccagag aagactccca   1440
ttcgacaaca gggacagctg cagcctcagc tcataccagc catccaatgc aaggaaggac   1500
aacaccaagc ccagaggaca gttcctggac tgatttcttc aacccaatct cacaccccat   1560
gggacgaggt catcaagcag gaagaaggat ggatatggac tccagtcata gtacaacgct   1620
tcagcctact gcaaatccaa acacaggttt ggtggaaaat ttggacagga caggacctct   1680
ttcaatgaca acgcagcaga gtaattctca gagcttctct acatcacatg aaggcttgga   1740
agaagataaa gaccatccaa caacttctac tctgacatca agcaatagga atgatgtcac   1800
aggtggaaga agagacccaa atcattctga aggctcaact actttactgg aaggttatac   1860
ctctcattac ccacacacga aggaaagcag gaccttcatc ccagtgacct cagctaagac   1920
tgggtccttt ggagttactg cagttactgt tggagattcc aactctaatg tcaatcgttc   1980
cttatcagga gaccaagaca cattccaccc cagtgggggg tcccatacca ctcatggatc   2040
tgaatcagat ggacactcac atgggagtca agaaggtgga gcaaacacaa cctctggtcc   2100
tataaggaca ccccaaattc cagaatggct gatcatcttg gcatccctct tggccttggc   2160
tttgattctt gcagtttgca ttgcagtcaa cagtcgaaga aggtgtgggc agaagaaaaa   2220
gctagtgatc aacagtggca atggagctgt ggaggacaga aagccaagtg gactcaacgg   2280
agaggccagc aagtctcagg aaatggtgca tttggtgaac aaggagtcgt cagaaactcc   2340
agaccagttt atgacagctg atgagacaag gaacctgcag aatgtggaca tgaagattgg   2400
ggtgtaacac ctacaccatt atcttggaaa gaaacaaccg ttggaaacat aaccattaca   2460
gggagctggg acacttaaca gatgcaatgt gctactgatt gtttcattgc gaatcttttt   2520
tagcataaaa ttttctactc tttttgtttt ttgtgttttg ttctttaaag tcaggtccaa   2580
tttgtaaaaa cagcattgct ttgtaaatta gggcccaatt aataatcagc aagaatttga   2640
tcgttcagtt ccacttggag gccttcatcc tcgggtgtgc tatggatggc ttctaacaaa   2700
aactacacat atgtattcct gatcgccaac ctttccccca ccagctaagg acatttccca   2760
gggttaatag ggcctggtcc ctgggaggaa atttgaatgg gtccattttg cccttccata   2820
gcctaatccc tgggcattgc tttccactga ggttggggtg tactagttac acatcttcaa   2880
```

cagaccccct ctagaaattt ttcagatgct tctgggagac accaaagggt gaagctattt 2940

atctgtagta aactatttat ctgtgttttt gaaatattaa accctggatc agtcctttga 3000

tcagtataat tttttaaagt tactttgtca gaggcacaaa agggtttaaa ctgattcata 3060

ataaatatct gtacttcttc gatcttcaaa a 3091

<210> 87
<211> 5261
<212> DNA
<213> human

<400> 87
ctcaggcacg tctagatttt cccacttaca taggtcttgt taagaaacca gtcctgcctt 60

cttgccactc gtgtctttcg atggctccct tcccgaagtc ccgctgcctc taagcggagt 120

gttaagcggg gctctcggaa gccggtggac cgagatttcc ccgggtgggg cgggcgcggt 180

gtagcggccc gcgggctgac ttgctcccgg ctgtcccccg gccccagcga ccatgccccg 240

taaaggcacc cagccctcca ctgcccggcg cagagaggaa gggccgccgc cgccgtcccc 300

tgacggcgcc agcagcgacg cggagcctga gccgccgtcc ggccgcacgg agagcccagc 360

caccgccgca gagactgcaa gtgaggaact tgataataga agtttagaag agattttgaa 420

cagcattcct cctcccccgc ctccagcaat gaccaatgaa gctggagctc ctcggcttat 480

gataactcat attgtaaacc agaacttcaa atcctatgct ggggagaaaa ttctgggacc 540

tttccataag cgcttttcct gtattatcgg gccaaatggc agtggcaaat ccaatgttat 600

tgattctatg ctttttgtgt ttggctatcg agcacaaaaa ataagatcta aaaaactctc 660

agtattaata cataattctg atgaacacaa ggacattcag agttgtacag tagaagttca 720

ttttcaaaag ataattgata aggaagggga tgattatgaa gtcattccta acagtaattt 780

ctatgtatcc agaacggcct gcagagataa tacttctgtc tatcacataa gtggaaagaa 840

aaagacattt aaggatgttg gaaatcttct tcgaagccat ggaattgact tggaccataa 900

tagattttta attttacagg gtgaagttga acaaattgct atgatgaaac caaaaggcca 960

gactgaacac gatgagggta tgcttgaata tttagaagat ataattggtt gtggacggct 1020

aaatgaacct attaaagtct tgtgtcggag agttgaaata ttaaatgaac acagaggaga 1080

taagttaaac agggtaaaga tggtggaaaa ggaaaaggat gccttagaag gagagaaaaa 1140

catagctatc gaatttctta ccttggaaaa tgaaatattt agaaaaaaga atcatgtttg 1200

tcaatattat atttatgagt tgcagaaacg aattgctgaa atggaaactc aaaaggaaaa 1260

aattcatgaa gataccaaag aaattaatga gaagagcaat atactatcaa atgaaatgaa 1320
agctaagaat aaagatgtaa aagatacaga aaagaaactg aataaaatta caaaatttat 1380
tgaggagaat aaagaaaaat ttacacagct agatttggaa gatgttcaag ttagagaaaa 1440
gttaaaacat gccacgagta aagccaaaaa actggagaaa caacttcaaa aagataaaga 1500
aaaggttgaa gaatttaaaa gtatacctgc caagagtaac aatatcatta atgaaacaac 1560
aaccagaaac aatgccctcg agaaggaaaa agagaaagaa gaaaaaaaat taaaggaagt 1620
tatggatagc cttaaacagg aaacacaagg gcttcagaaa gaaaaagaaa gtcgagagaa 1680
agaacttatg ggtttcagca aatcggtaaa tgaagcacgt tcaaagatgg atgtagccca 1740
gtcagaactt gatatctatc tcagtcgtca taatactgca gtgtctcaat taactaaggc 1800
taaggaagct ctaattgcag cttctgagac tctcaaagaa aggaaagctg caatcagaga 1860
tatagaagga aaactccctc aaactgaaca agaattaaag gagaaagaaa aagaacttca 1920
aaaacttaca caagaagaaa caaactttaa aagtttggtt catgatctct ttcaaaaagt 1980
tgaagaagca aagagctcat tagcaatgaa tcgaagtagg gggaaagtcc ttgatgcaat 2040
aattcaagaa aaaaaatctg gcaggattcc aggaatatat ggaagattgg gggacttagg 2100
agccattgat gaaaaatacg acgtggctat atcatcctgt tgtcatgcac tggactacat 2160
tgttgttgat tctattgata tagcccaaga atgtgtaaac ttccttaaaa gacaaaatat 2220
tggagttgca acctttatag gtttagataa gatggctgta tgggcgaaaa agatgaccga 2280
aattcaaact cctgaaaata ctcctcgttt atttgattta gtaaaagtaa aagatgagaa 2340
aattcgccaa gctttttatt ttgctttacg agatacctta gtagctgaca acttggatca 2400
agccacaaga gtagcatatc aaaaagatag aagatggaga gtggtaactt tacagggaca 2460
aatcatagaa cagtcaggta caatgactgg tggtggaagc aaagtaatga aaggaagaat 2520
gggttcctca cttgttattg aaatctctga agaagaggta aacaaaatgg aatcacagtt 2580
gcaaaacgac tctaaaaaag caatgcaaat ccaagaacag aaagtacaac ttgaagaaag 2640
agtagttaag ttacggcata gtgaacgaga aatgaggaac acactagaaa aatttactgc 2700
aagcatccag cgtttaatag agcaagaaga atatttgaat gtccaagtta aggaacttga 2760
agctaatgta cttgctacag cccctgacaa aaaaaagcag aaattgctag aagaaaacgt 2820
tagtgctttc aaaacagaat atgatgctgt ggctgagaaa gctggtaaag tagaagctga 2880
ggttaaacgc ttacacaata ccatcgtaga aatcaataat cataaactca aggcccaaca 2940

agacaaactt gataaaataa ataagcaatt agatgaatgt gcttctgcta ttactaaagc 3000
ccaagtagca atcaagactg ctgacagaaa ccttcaaaag gcacaagact ctgtcttgcg 3060
tacagagaaa gaaataaaag atactgagaa agaggtggat gacctaacag cagagctgaa 3120
aagtcttgag gacaaagcag cagaggtcgt aaagaataca aatgctgcag aggaatcctt 3180
accagagatc cagaaagaac atcgcaatct gcttcaagaa ttaaaagtta ttcaagaaaa 3240
tgaacatgct cttcaaaaag atgcacttag tattaagttg aaacttgaac aaatagatgg 3300
tcacattgct gaacataatt ctaaaataaa atattggcac aaagagattt caaaaatatc 3360
actgcatcct atagaagata atcctattga agagatttcg gttctaagcc cagaggatct 3420
tgaagcgatc aagaatccag attctataac aaatcaaatt gcacttttgg aagcccggtg 3480
tcatgaaatg aaaccaaacc tcggtgccat cgcagagtat aaaaagaagg aagaattgta 3540
tttgcaacgg gtagcagaat tggacaaaat tacttatgaa agagacagtt ttagacaggc 3600
atatgaagat cttcggaaac aaaggcttaa tgaatttatg gcaggttttt atataataac 3660
aaataaatta aaggaaaatt accaaatgct tactttggga ggggacgccg aactcgagct 3720
tgtagacagc ttggatcctt tctctgaagg aatcatgttc agtgttcgac cacctaagaa 3780
aagttggaaa aagatcttca acctttcggg aggagagaaa acacttagtt cattggcttt 3840
agtatttgct cttcaccact acaagcccac tccccttac ttcatggatg agattgatgc 3900
agcccttgat tttaaaaatg tgtccattgt tgcattttat atatatgaac aaacaaaaaa 3960
tgcacagttc ataataattt ctcttcgaaa taatatgttt gagatttcgg atagacttat 4020
tggaatttac aagacataca acataacaaa aagtgttgct gtaaatccaa aagaaattgc 4080
atctaaggga ctttgttgaa ctttatgctg aagattcttc aagttgattc agtgtattac 4140
tgattttttt ctatttgtaa aggattatga gttgtataaa atacatactc cctaaactag 4200
atcatgaaac tggtttctgt tttatgcagt tgtcatttgt aaagtctaat aaaatattct 4260
ctataattgc ttctagatta caaaaatatg acaatcttgt aagtagcaga ctatggagaa 4320
aaatgagtta cctggagggt caggtaactt gccaaactaa aaagtatgtt agttgaggca 4380
aagtcctaag caaggttgtg ctatcaaggc tcagcatacc ttcgtgggcc tttgatttac 4440
caacactgga aatgcctgcc aactaatctt ggatagattc tttaaggcat tccacttagc 4500
ttgccagttg agacaatcac cacagttatt acccaaatac tatgaacata tttttgtaaa 4560
ccagtcattc tgaattatag tgatgagaat ttaaatatat gcttttctag aatttgatgt 4620
ttgaccattt atgacttaat taccagagag ccagtaaatt aggacagtgt ttcaacaagc 4680

```
ctaggctatc tcgtaagttg aaaaatatcc cactatagtt gcttcatgag tatgaagtaa   4740

gatggcctct gatttacact ggttcaattt acaaattttc aactttatga taggtttatc   4800

cgggtactaa atgcatttca acttgatagt ttcaacttat gataggttta ccaggatgta   4860

gtcccactgt tgaggagcat ctatttaggg gttaattact ttagtaataa gtggaaagta   4920

agataccttg agtaatgttt gcctataaaa ttgtcagcgt atttttacac tattggctca   4980

agaatgttat aatgctaagg gacataagtt ggcaaccact tggtttttgg aaggactttc   5040

ggtattgtat tagaagtctg ccctagctgt taaatttctg ggtatttatc ctaaggaatt   5100

aattaaagag ttaattgttc ctttcttcag tgggccattg ttttagatat ttaaaaaatc   5160

caacagtttc tatcataatg taactgtaaa aatgtaaaca cattattagc atggactttt   5220

aaataaagat ttaaagaaag caaaaaaaaa aaaaaaaaaa a                        5261


<210>  88
<211>  812
<212>  DNA
<213>  human

<400>  88
cagcggaggt ggcgctggct aatctcaaga acaaatatga aaatgaaaaa gcaatggtga     60

ctgaaaccat gacgaagctt agaaatgaac tgaaggcttt gaaagaagat gctgcaacct    120

tctcatccct gagagcaatg tttgcaacaa gatgtgatga atatgtcacc cagttggatg    180

agatgcagag acagttagca gctgcagagg atgagaagaa gactctgaac actttgttac    240

gaatggctat ccagcaaaaa ctcgccctga cccagaggct ggagggctta gagtttgacc    300

atgagcagtc ccgacgcagc aaaggcaaac ttggaaagag caagatcggc agccctaaag    360

taagtgggga ggcatcagtc accgtgccca ccatagacac ttacctcctg catagtcagg    420

gcccacagac acccaacatt cgggtcagca gtggcactca gaggaaaagg tatgcatgca    480

gcgatcttca tagtacggtg cagtggccag attttagtta actgcaaaaa taaatgtgct    540

cttgttgtgg aggatggagg aggggaagca aaagaaaaaa tgggagctgg catataaatg    600

gtcttgctaa tgtggctctt tccagatcaa aacctttttg ataattgtgt ttatgtagtc    660

ctttctaacc ccctgcccaa tccctcctct tgattaatcg taatataatt ttcaaggtct    720

gttaatattt ttgctactct ttgtatgtgt aaatatgcct ctcccttacc tccaacctga    780

gaattggtag ataaaggtgg atattaagaa tg                                 812
```

<210> 89
<211> 748
<212> DNA
<213> human

<220>
<221> misc_feature
<222> (136)..(137)
<223> n is a, c, g, or t

<400> 89
gcgacatggt gcgcaagagc gggggcgaa agtacacgta ccgcttcggg ggccgcgtgc 60

ccagcctagc ctatccggac tgtgcgggag gcggacgggg agcagagaca caataaaaat 120

tcccggtcaa acctcnnaaa aaaaaaaaa aaaaaaaaa aaaaaaaaa ggggaaaaaa 180

aaaaaaaaaa aaaaaaaaa aaaaaaaaa aaaaaaaaa aaaaaaaacc ctgggggggg 240

ggcccggtcc caaattcccc ctatagggag tcgtttaaaa attaacgggc cggggtttaa 300

aaacgtcgga acgggaaaaa ccctggggta accaaactaa atcgcttgga agaaaatccc 360

cttttcgcaa gttggcgtaa tagcaaaaag cgcccccca atcgcccttc caaaaattgg 420

cccaccctaa atggcaaagg gaaaattgaa acgctaaata tttggtaaaa atccgggtaa 480

aattttggga aaataaccta atttttaaac caataggcca aaatcggaaa aaccctttttt 540

aaataaaaaa aataaaccga aatagggtta gatggttgtc caagttggaa acaaaatccc 600

attataaaaa acgggaactc aaacgtaaaa gggcaaaaaa ccgtatataa gggaatggcc 660

catacggaaa caatccccct aataaatttt ttggggtcaa ggggccccct taaatcgaaa 720

ccctaagggg acccccattt ccttcggg 748

<210> 90
<211> 683
<212> DNA
<213> human

<400> 90
tgtccatcct gcaggccaca agctctggat gaggaacttg aggcaagtca ccagcccctg 60

atcatttcgc ctaaaagagc aaggactaga gttcctgacc tccaggccag tccctgatcc 120

ctgacctaat gttatcgcgg aatgatgagg ctgccagtgc ccagcagtga gggacctgac 180

ctcaggggca gagacaagag aggcaccaag gaggatccaa gagctgacta tgcctgcatt 240

gctgagaaca aacccacctg agcaccccag acaccttcct caacccaggc gggtggacag 300

ggtcccctg tggtccagcc agtaaaaacc atggtccccc caaaaaaaaa aaaaaaaaaa 360

aaaaaaaaaa aaaaaaaaaa aaaaaaaaaa aaaaaaaaaa ataataaaaa tattatattt 420

ttataataaa acatatttaa ttttattttt ttatactgct ataaaaaatt aaaataaata 480

aaaattttgg cccccccttgg ccccgtttgt ttttttctcg ggccagtttt ttcttcgggc 540

ccaaatttgg agctcctttg cccatttccc cttaaggggg gcgatttaaa ttcacgggcg 600

gggttttaaa aggggggggt gggaaaaccc ggggtgtccc cccttttttc tctgggaaaa 660

aaccccctttt gcaagtggtg aaa 683

<210> 91
<211> 2917
<212> DNA
<213> human

<400> 91
ggcacgaggg cggcggccgc gatgagcctt cagcctgccg ggcaggggac gtgaacatgg 60

atgaccctaa gaaggaagac attcttcttt tggccgatga aaaatttgac ttcgatcttt 120

cattgtcttc ttcgagtgca aatgaagatg atgaagtctt cttcggaccc tttggacata 180

aagaaagatg tattgctgcc agcttggaat taaataatcc ggttcccgaa cagcctccgt 240

tgcccacatc tgagagtccc tttgcctgga gccctctggc cggggagaag ttcgtggagg 300

tgtacaaaga agctcactta ctggctttac acattgagag cagcagccgg aaccaggcag 360

cccaagctgc caagcctgaa gaccctcgga gccagggcgt ggaaagattc atacaggagt 420

caaaattaaa aataaacctc tttgagaaag aaaaggaaat gaagaaaagc cccacgtctc 480

ttaaaaggga gacatactac ctgtcagaca gccccttgct ggggcccccct gtgggtgagc 540

ctcggctctt ggcctcctcc ccggccctgc ccagctctgg tgcccaggcc cgcctcaccc 600

gggcgccggg gcctccgcac tctgctcatg ctttgcccag ggaatcatgc actgctcatg 660

ctgcaagtca ggcagcgact cagaggaagc ccgggaccaa attgctgctg cctcgagcgg 720

cctctgttag aggaagaagc atccctgggg ctgcggagaa gcccaagaaa gagattccag 780

ctagtccttc caggacaaaa atcccagctg agaaggaatc ccaccgggat gttctccctg 840

acaaacctgc cccgggtgct gtcaatgtgc cggccgccgg aagccacttg ggccagggca 900

agcgggcgat ccctgttcca aacaagttgg ggctgaagaa gaccctgtta aaagcacccg 960

gctctaccag caatctcgca aggaagtcct cctcggggcc tgtttggagc ggggcatcca 1020

gtgcgtgcac atccccagca gtgggcaaag ctaaatcaag tgaatttgca agtattcctg 1080

caaatagctc ccggcctctg tcaaacatca gcaagtcagg cagaatggga cccgccatgc 1140

```
tgcggccagc tctgcctgca ggccctgtgg gggcatcctc ctggcaggcc aagcgggtcg   1200
atgtttctga gctggcagcg gagcagctca cggcaccccc ctcagcatcc cccacccaac   1260
cccagactcc ggaaggtggc ggccagtggc tgaactccag ttgcgcttgg tcagaatctt   1320
ctcaattgaa taagactaga agtatcagac ggcgagattc ctgtctaaat tccaagacaa   1380
aggttatgcc tactcctaca aatcaattta aaattcctaa gttttctatt ggtgactccc   1440
cggacagctc aacaccaaag ctttcgcggg cacagcggcc gcagtcgtgc acgtcagttg   1500
gcagggtcac tgtccacagc accccggtta gacgctcatc tgggccagca ccacaaagcc   1560
tgctgagcgc acggcgtgtg tcagccttgc ccacacccgc cagccggcgc tgctctggcc   1620
ttccaccgat gacccccaaa acgatgccca gggccgtggg ctctcccctg tgtgtgccag   1680
ctcggagacg ttcctctgag ccccgcaaga actctgcaat gagaactgaa ccaacaaggg   1740
agagcaacag aaagacagat tccaggctgg tggatgtgtc ccctgacagg ggttctcctc   1800
cttcccgtgt gcctcaggca cttaactttt ctccagagga aagcgattct actttctcca   1860
aaagtactgc cacagaagta gctcgggagg aagccaagcc gggtggagat gcagccccta   1920
gtgaggctct tcttgtagat atcaaactgg aaccactcgc ggtcactcca gatgctgcaa   1980
gccagcccct cattgacctt cctctcatcg acttctgcga taccccagaa gcacacgtgg   2040
ctgtaggatc tgaaagcagg cctctgatcg acctcatgac aaacactcca gacatgaata   2100
aaaatgtggc caaaccttca ccggtggtgg gacagctcat agacctgagc tcccctctga   2160
tccagctgag ccctgaggct gacaaggaga acgtggattc cccactcctc aagttctaag   2220
ccgaaccaaa tcctttgcct tgaaagaaca gccctaaagt ggttttcaac cctcagaaac   2280
aagctttagg ctggtcgcag tggcttacac ttgtaaccct agaacttggg aggctgaggt   2340
gggcggatta cttgagccca ggagttcggg accagcctgg gaaatatagt gaaactcctg   2400
tccctacaaa aagtacaaaa attagccggg tgtggtagtg catgcctgta gtcccagcta   2460
cttgggaggc tgaagtggga ggatggcctg agctcaagga gatgcaggct gcagtgggct   2520
gtgattgtgc cactgcactc cagcctgggc accaatgtga gaacctgtct tggaaaaaaa   2580
aaaaaaaaga aacatgtttt agtagaagtt ttatttgaaa aagaaaaata agcataaata   2640
tattcccagt gctggagagg gtgggctgag ggactggggc cagcacggac cacccaaggc   2700
ctctgcttcc cgccgccacc ctcctcgctg ccattctctg ggctggaatg tgaagcctca   2760
gtcactctaa atgaagaatt ttcttttgaa tgttttgtat gtaaaatagc aagtggctat   2820
ttttaaagtt aagtttgtat aaatagttag atattctaga tttacattaa attgtaaaat   2880
```

aaatggactt attgaagcat aaaaaaaaaa aaaaaaa 2917

<210> 92
<211> 1134
<212> DNA
<213> human

<400> 92
agcaactaca gaaagctttt atttatttgt tcaaagtaac cagtgctatg gatgcaaaaa 60

acccccaaca actcttccaa cgctactttc aaaacaaaca tatcaaactt gattttcatt 120

aggatgtagt tattttgtca cactagtaaa tcaaaatcta agacccaatt tttatatata 180

tatataaata tatataaaag aacaatgtga acaattattg agcttcatct tctggacaag 240

aatgccaagt tagtctctct tcataaaaag caattacaat ttgaggacac ttcatacttt 300

gcctctttcg ccagcaccaa gctctgcctc agtctgaatc tttccatttc atgagaaaca 360

tcaattctcc actgactgtc tgtggcacca attattctat caggatcaag acctctggca 420

aatcctcttg gtttgtcagc agcatctctt tatcttcttt gatttgctag tcatcagatt 480

cactgtcaga taaagatgtc tattatatgt accatctaga cacttatgcc agctgtctga 540

gagtgaagaa acgcttcaat caattctgga caatctacat ataccttcag gttcccaaag 600

tatcgtcagc atctgtaaaa tccgaacaca ttcagagaaa tatacacttg ccattcacta 660

cacgtcgatc tagtactgat ccacgacaaa tactatcagc tctgcgacaa aactactaac 720

gtcattctcc gtctggtaca aggccaacta cctagacaaa gtagaatata gatgggagga 780

caatgagaat aggcggactg tagaagagct aggatacaac gaagtacgaa ctgacccagg 840

gtcacgagat agaaagagcc agcgagacaa gacagaaata cgggacatac tgtcaggatc 900

aacgccaaca ggaatacttc gaaaagagta ggacatccac agctagcaga gcagacacag 960

acgcgccgac ctggagcaca gcttatcggc acagagagca agcgcaatag agcgaataaa 1020

aataagggaa gaagtaagga taggggccaa gcacgaacac ggcaccggac actaacccca 1080

ccgccgacgc agcacacaca gcatagacag agagagagac agctcagtcc gacc 1134

<210> 93
<211> 544
<212> DNA
<213> human

<400> 93
ttttaggttt tttactattt tattatggca cacaggatag aggatggtac agttttctta 60

cttcaaccaa gtaattctca aagcatccag ctatttccat ttggctaaag ttactttttg 120

cacatagctt gcatctgttt gagacttacc atgtacatca acccaggtct agtaagcaga 180

aatgtgaaaa gttttgtttc tgaggagacg cctcatcttt acagaagcca atacactgag 240

agccttcata gttccaatcc attaccatca tggcaaggaa gcactttacc tatttgcata 300

gcaacatata tttaactaga aataggtggt acaaagggat taagtaactt taaatggaga 360

ccactttggt ttcaggttaa attaataact tatagagatc gtctaaaaaa caaatattga 420

atgaaattag ctgacaaagc aattgtttca gaacaaaggc agaatagcag atagtaatat 480

catctatatt tattccacat caaatgcaag agcgttctta actttacgac agaaaggata 540

catg 544

<210> 94
<211> 704
<212> DNA
<213> human

<220>
<221> misc_feature
<222> (572)..(572)
<223> n is a, c, g, or t

<400> 94
ttttagtata atccaaattt attatatcta agaggctatc atgggctgta agtagaatca 60

aaggttaaga acattttatg cacttattcc acaaacattt actgagcata ctaggtgctg 120

ggaatgtgac agtgagcaaa aaacacaaga gtgtgcaaag agtgatcaaa atgtcaacca 180

gtaagaacag ggaagagctg ctgaggccaa gaggagacga ctagttctaa agatgtgtgg 240

atttcacagc ctggtctctg gttttgagcc tgattgcaat ttgacatgac tcttggctta 300

ggaaacggta atccctaata agacagggat gatattgatg tagaacttga cataagatct 360

ctattttctt gaaaagagca caaggtttgt tttaagtcct gaagtagctc aatctcttcc 420

ttcaactgct gccacctttg ttgagatgca tgaatttttt tctctattgc tgcttgaaaa 480

tttacaaatg tgtcttcgaa caatctacag tcaaaaagtg cactcccgat ttcttcatag 540

tctgattctg aagtagtctc atccatgagt tnttctggaa ttgaatgaac tttgtctaat 600

atttcttcaa gtaagtaatt tagaagtcct gcttccttgc ttttcttaag aaaaagaact 660

ttcacagttg catctgtgtg tctggcatgc tcttctttca cttg 704

<210> 95

<211> 402
<212> DNA
<213> human

<220>
<221> misc_feature
<222> (74)..(74)
<223> n is a, c, g, or t

<220>
<221> misc_feature
<222> (205)..(205)
<223> n is a, c, g, or t

<220>
<221> misc_feature
<222> (271)..(271)
<223> n is a, c, g, or t

<220>
<221> misc_feature
<222> (274)..(274)
<223> n is a, c, g, or t

<220>
<221> misc_feature
<222> (330)..(330)
<223> n is a, c, g, or t

<220>
<221> misc_feature
<222> (353)..(353)
<223> n is a, c, g, or t

<220>
<221> misc_feature
<222> (371)..(371)
<223> n is a, c, g, or t

<220>
<221> misc_feature
<222> (383)..(383)
<223> n is a, c, g, or t

<400> 95
tttttttttt ttttttttct ttgaatgttt actaagcagt cagaaggtga cttactccat 60

tccccagttg caanccctga agcatagagg gtttagggtt aggaggagta ccacagactg 120

aaagaggaag taagaagtta agggcttggc aaagttgcca ctcctatccc tggtctagct 180

gcctcagaac taaaggaggc ctggnactaa caagtcactc ccttccagat tagatgaggc 240

cagctgcttt tggatgcccc aggaagaggt ngtngcaaca gtggcatgag ggatccctct 300

gtaactccac atgccaggtc atcttcactn gattagatgt ccatttcaaa ttntgcgtca 360

tcgggtatct nttcctctgt ctnctgcttc cttcagcttc tt 402

<210> 96
<211> 3473
<212> DNA
<213> human

<400> 96
tggggcagaa aagtaataca gacggagcac tgcagaaacc ttcaaatgaa ggtgtcattg 60

aaataaaagc aactaaggtc tgtgaccaga ggaccaaatg taaaagtcgc tgtaatgaaa 120

tgctgccagg cacgtcaaca ggcaataatc aaagcactat cactctatca gttgcttctc 180

agaacttaac tttcaccagc agcagctcac cacctaatgg tgactcaatc aataaagacc 240

ctaaattatg cactaaaagc ccaagaaaac gactgtcttc tacattgcaa gagacccagg 300

tgcctcctgt aaagaaacca attgtggaac agctttcagc agctaccata gaagggcaga 360

aacaaggcag tgttaagaag gaccaaaagg ttccacattc agggaaaaca gaaggttcaa 420

cagcaggtgc tcagattcct agcaaggtat cagtaaatgt cagttcacac ataggagcaa 480

atcaaccctt gaattcctct gcccttgtta tcagtgattc agctttggaa cagcaaacaa 540

ccccatcatc atctccagat ataaaagtaa aacttgaagg aagtgtcttt ctcttggaca 600

gtgattcaaa gtcagttggc agctttaatc caaatggatg gcaacaaatc actaaagatt 660

ctgagtttat atctgccagt tgtgaacaac agcaagatat cagtgttatg acaattcctg 720

agcactctga tatcaatgac ttagagaaat ctgtttggga attagaagga atgccacagg 780

acacatatag ccagcagcta catagccaga tacaggaatc ttctttaaat caaatacaag 840

cacattcttc agatcagtta cctctgcaat ctgaactgaa ggagtttgag ccttctgttt 900

cccagacaaa tgaaagctac tttccttttg atgatgaact tacacaagat agtattgtgg 960

aagagctggt gcttatggag cagcaaatgt caatgaacaa ttctcattct tacggcaact 1020

gtttgggaat gacccttcag agtcagtcag taactccagg agctccaatg tcatctcaca 1080

cttccagcac ccacttctat catccaatcc acagcaatgg cactccaatc cacacaccca 1140

cacccacacc cacacccact cctactccaa ccccaacccc aaccccgaca tctgaaatga 1200

ttgctggatc tcagagtctg tcacgggaga gcccttgctc caggctagcc cagactacac 1260

ctgtggatag tgctttagga agtagccgac atacacccat tggtactcca cattctaact 1320

gcagcagtag tgtccccccc agccctgttg aatgcaggaa tccgtttgca ttcactccaa 1380

taagctccag tatggcatat catgacgcca gcattgtctc aagtagtcct gtgaaaccga 1440

tgcaaagacc catggccaca caccctgaca aaaccaagct tgaatggatg aataatgggt 1500
atagtggggt tggtaattca tcagtttctg gccatggtat tctcccaagc tatcaggaac 1560
tagtggaaga ccgtttcagg aaacctcatg cttttgctgt gcctggacag tcttatcagt 1620
ctcaatccag acatcatgac actcattttg gtcgtttgac tcctgtctct cctgtgcagc 1680
atcaaggtgc cactgtaaat aacaccaaca aacaggaggg ttttgcagtc cctgcccctc 1740
ttgataataa aggaactaat tcatctgcca gcagcaactt cagatgccgg agtgtgagcc 1800
ctgctgttca tcgccaacgt aatcttagtg gaagcaccct ctatccagta tctaatatcc 1860
cacgatctaa tgtgaccccc tttggaagtc cagttacccc agaagttcat gttttcacaa 1920
atgttcacac agacgcatgt gccaacaaca tagctcaaag aagccaatca gttccattga 1980
cagtcatgat gcagacagcc ttcccaaacg ctcttcagaa gcaagcaaac agtaaaaaaa 2040
taaccaatgt tttgttgagt aaacttgatt ccgacaatga tgatgcagtg agaggtttgg 2100
gaatgaacaa cctgccctct aattatacag cccggatgaa tctcactcag attttggaac 2160
cttccactgt ttttcctagt gccaacccac aaaatatgat cgattccagc acttctgttt 2220
atgagttcca aacaccatct tacctcacca aaagtaatag caccggtcag atcaattttt 2280
ctcctggaga taatcaagca caatcagaaa ttggagagca acaattagat ttcaatagca 2340
ctgttaaaga cctgttgagt ggagacagct tgcaaaccaa ccagcagctg gtaggtcagg 2400
gagcatctga tctcactaat actgcatctg atttctctag cgatatcagg ttgtcttctg 2460
agctctcagg cagcatcaat gatttgaaca ctttagaccc aaatctactg tttgatccag 2520
gtcgtcagca gggacaagat gatgaagcta cactggaaga attaaagaat gacccattat 2580
ttcaacaaat ttgcagtgaa tccatgaatt ctatgacttc atcaggtttt gaatggatag 2640
aaagcaagga ccatcctact gttgaaatgt tgggttaaat tgtgttttat aacatgtagc 2700
acactgtatc taaagacata tgtattgtat ttgtcttaat ggaagtgcct cccgcagcag 2760
aaatactatt aattgtgaca ttttaaaagc gtttgctgca gaagtggttt cttcagtagg 2820
aaatggttag acttgcctca gttcttaaca agtgtctaaa gacagtaggc tgtagaagaa 2880
caagtattag gttttaaatt ttataatgtg ccccatttat tcacattgat tgactatcca 2940
gctgttacaa gagcagtcga tctttatttt gtgtaagtta gtttcatttc atcagatgat 3000
ctgcacagaa ctcagaataa accatcaatt ttaggtatag gctgttttgt tgttggcatt 3060
atcttttaga tataaaatca tagctagggt gaaccgtaga caagaaagtc ttccttgaaa 3120

cagggataat attcaggtta ttataaatat ttaggcttga agcatagagc tactgtaggg 3180

tgaaaaatct ctgtaggact ttctggggct tcagtaccat ttacacccac gatgaagggc 3240

ttaaatagaa aataatggaa aattaaatat tactttaagg aaagcaaagc tgcactaaaa 3300

tttttaaggg aaaaaaaaca agtagccatt tatatttgtg accttgcatt aatatttaaa 3360

tgttgatttt aatatggagc tagcacacat agatgcgcag gcacgcacac acaaaacaag 3420

caaatgtaac tccaggcatt tgtaatttaa tataataaat acaagcatgt tag 3473

<210> 97
<211> 2001
<212> DNA
<213> human

<400> 97
agtctgtata aaaagcaatt gctcttttgt ttccaaaaca ggtattacaa gtggataaat 60

aatgtgcact gctctgagcc caaaggtacg cagtggacct ggcctctctg atatgcatca 120

gtatagccaa tggctagcca gcagacatga agctaatttg ctaccaatga aagaagatct 180

ggccttgtgg ttaaccaatc tattagggaa ggagattaca gcagaaactt ttatggagaa 240

gttggacaat ggtgccttgc tctgtcaact tgcagaaact atgcaggaga aattcaagga 300

gagcatggat gctaacaagc ccacaaagaa tctaccgttg aagaagatcc catgcaaaac 360

cagtgcaccc tcgggctcct tttttgccag agacaataca gcaaatttct tatcctggtg 420

ccgagattta ggggtggatg aaacgtgtct atttgaatcg gaaggtttgg tcctccacaa 480

gcaacccaga gaagtgtgtc tctgtctgct agagcttggc cggattgcag ccaggtatgg 540

tgtggagcct cctggtttga taaagctgga aaaagagatt gaacaagaag aaacactttc 600

tgccccttct ccttcacctt ctccttcatc aaagtcttct ggaaaaaaga gtacaggaaa 660

cttactggat gatgcagtga aacgaatttc tgaagatcct ccttgcaaat gcccaaacaa 720

gttctgtgtg gagcggctct cccaaggaag ataccgagtg ggagaaaaga tcctcttcat 780

taggatgctg cacaacaaac atgtcatggt ccgtgtggga ggaggctggg aaacttttgc 840

agggtatttg ttgaaacacg acccctgccg aatgctgcag atctcccgtg tggatggcaa 900

aacatcccct atccaaagca aatctccaac tctaaaggac atgaatccag ataactactt 960

ggtggtctct gccagttata aggctaagaa ggaaattaag tgaaacaaat tggtcatgac 1020

aaggggaccc tcataatggc ctgtatccac ttctccagta tagtcagttt agttcatatg 1080

ttctgaaaac tgttttggag aaagatagac agaaaaatgt catcatattg aaaaatgttc 1140

aaagagtagc actatttatt tttaatgctt ctgtagaata tgacctatta aaagaaaatc 1200

taaactcaaa tttaaattat ccaaatttta ggcaaattat tatttctcaa tatgcgaaca 1260

cagtatttag aacacaatat tagaaacaca attctaacta aagataaata aggagaaaac 1320

atttaggatt tgcagataag tcaacagaaa gtgcctgctt tacactcatg agtaaaagca 1380

ctccagacat ttttataatt gcaagatttt tatgcttttt tttttttttt ttacaaaatg 1440

atgattagtg tgataatgtg ctgcaaaaaa tatccaacag cactacacat aagtacagag 1500

tattcacaat agtaatatgt tactggaaag gccacttccg aaaagtttac attcacttgg 1560

aaggctgcct taaagtatat ctcttatcta tgaccaaata tctggggtac ttttagaagt 1620

tttcagtaca ccccccttaga gaatagctta tgagttattt cacacattcc tgagcacatg 1680

gctgtgttta gaatgatcta gtgtaattca tacatgagct gacatacgtt gatactttga 1740

cgagtaaatt gtacagtcaa atgttcattg atttcatgtt atttcaaagc attttcttat 1800

taaaaatata tctttagtta atcctatttt gctatgcttt ctagtaaagt aaattcactg 1860

tagctaatga tgttacagac ttacgtatac ccttgtatac ctgggacagg gctgttttat 1920

accaataaac agcaaaatat tgtccttact cattcaagaa ttaaaaggat aaattttaaa 1980

aatgttaaaa aaaaaaaaaa a 2001

<210> 98
<211> 1612
<212> DNA
<213> human

<400> 98
cttagacgct ccccagatga ttctaatgag cagcctggtt gagaactact tcacgtggta 60

gcgcctcctg cccagaaccg tgggatatag ccctgggcaa accctcacca ggatcaggcc 120

tgtcttccct atgaggtgta acctatgctc caggctctgt aatgttcccc caggcaagag 180

cttcctcctc cagatttaag ggtgcccagg ctgggagact gtcccctgtc tttcccaggt 240

ggtcctgtgc ttcttgggct catcacagga aagacctgtc ccaggtgcaa ggtctctctg 300

atgtagaagg cctcccctga gatggacagt ctgggggatc ttcccacgtg aacctgtctc 360

ttgctccgca ggtgtaaggt atgcccactg accttttca ccaagtcaga gatgcagatc 420

cactccaagt cgcacacaga ggccaagccc cacaagtgcc cgcactgctc caagtccttt 480

gccaacgcct cctacctggc ccagcacctg cgcatccacc tgggcgtcaa gccctaccac 540

tgctcctact gtgataagtc cttccggcag ctctcccacc tccagcagca caccaggtga 600

gtggcctgcc tgctgccctg ctgcagcccg actcagctca gcacccgtgg cctggcacat 660
ggagccagtg caaggagggg caaggacctt ctccaggtgc ccattgccct cggggtcacg 720
gcccttgtgg acctcactgg cctcagctgt tgttactgca ccccgtcccc actgtttctc 780
atctctaggt ctttgcacat gctgtgtcct ttaccagaga gacacccctc tcccgctttt 840
ccctgtttct ctggccatcc ccttgtcctc tctcagtcta gcttcctcca ggaagccctc 900
cctgattgct ctagctgggt gagggacccg acctctgtgt ccccacaggg ctctgtgcat 960
gtccatcata gcagtggtag gctgtcacgc ccgtgtctct ttatctgccc acctcactca 1020
atctactggg agttcttgaa ggcaagagct cagtctcctc catttccata tccccagttt 1080
gtggcacaaa ataagcaatt attaaatatt ttggaataaa tataactggt ttttacagag 1140
catatgattc atccatttaa caaatactta tagaaacttt tactctatgc cagacactat 1200
tctagatgtt gaggatacag caaggaagaa aacagacgaa aatctctgcc ttcgtggggc 1260
atacagtcta gttgagtgtg tgaaagggag actcaaagct ataaatgctt taagaaaaat 1320
ttgtggctgg gtgcggtggc tcacacctgt aatcccagca ctttaggagg ctgaggcggg 1380
tggatcacct gaggtcagga gttcatgacc agcctggcca acgtggcgaa accccatctc 1440
tactaaaaat acaaaaatta gctgggtgtg gtggtgggcg tctgtaatcc cagctacttg 1500
ggaggcttag gcaggagact cgcttgaacc caggagatgg aggctgcagt gggccgagat 1560
tgcgccattg cactccagcc tggatgacaa gagtgaaact ctgtctcaag ag 1612

<210> 99
<211> 1086
<212> DNA
<213> human

<400> 99
atggatcccc tgggtgcagc caagccacaa tggccatggc gccgctgtct ggccgcacgg 60
ctatttcagc tgctggtggc tgtgtgtttc ttctcctacc tgcgtgtgtc ccgagacgat 120
gccactggat cccctagggc tcccagtggg tcctcccgac aggacaccac tcccacccgc 180
cccaccctcc tgatcctgct atggacatgg cctttccaca tccctgtggc tctgtcccgc 240
tgttcagaga tggtgcccgg cacagccgac tgccacatca ctgccgaccg caaggtgtac 300
ccacaggcag acacggtcat cgtgcaccac tgggatatca tgtccaaccc taagtcacgc 360
ctcccacctt ccccgaggcc gcaggggcag cgctggatct ggttcaactt ggagccaccc 420
cctaactgcc agcacctgga agccctggac agatacttca atctcaccat gtcctaccgc 480

agcgactccg acatcttcac gccctacagc tggctggagc cgtggtccgg ccagcctgcc 540

cacccaccgc tcaacctctc ggccaagacc gagctggtgg cctgggcggt gtccaactgg 600

aagccggact cagccagggt gcgctactac cagagcctgc aggctcatct caaggtggac 660

gtgtacggac gctcccacaa gcccctgccc aaggggacca tgatggagac gctgtcccgg 720

tacaagttct acctggcctt cgagaactcc ttgcaccccg actacatcac cgagaagctg 780

tggaggaacg ccctggaggc ctgggccgtg cccgtggtgc tgggccccag cagaagcaac 840

tacgagaggt tcctgccacc cgacgccttc atccacgtgg acgacttcca gagccccaag 900

gacctggccc ggtacctgca ggagctggac aaggaccacg cccgctacct gagctacttt 960

cgctggcggg agacgctgcg gcctcgctcc ttcagctggg cactggattt ctgcaaggcc 1020

tgctggaaac tgcagcagga atccaggtac cagacggtgc gcagcatagc ggcttggttc 1080

acctga 1086

<210> 100
<211> 5956
<212> DNA
<213> human

<400> 100
atgagttctg actcagaatt ggctgttttt ggggaggctg ctcctttcct ccgaaagtct 60

gaaagggaac gcattgaggc ccagaatagg ccctttgatg ccaaaacatc tgtctttgtg 120

gcggagccca aagaatcctt tgtcaaaggg accatccaga gcagagaagg aggaaaagtg 180

acggtgaaga ctgagggagg agcgactctg acagtgaagg atgatcaggt cttccccatg 240

aaccctccca aatatgacaa gatcgaggat atggccatga tgactcatct gcatgagcct 300

gctgtgctgt acaacctcaa agaacgttat gcagcctgga tgatctacac ctattcaggt 360

ctcttctgtg tcactgtcaa cccctacaag tggctgcctg tgtataagcc cgaggtggtg 420

acagcctacc gaggcaaaaa gcgccaggag gccccgcccc acatcttctc catctctgac 480

aacgcctatc agttcatgct gactgaccga gagaatcagt caatcctgat cactggagaa 540

tctggtgcag ggaagactgt gaacaccaag cgtgtcatcc agtactttgc aacaattgca 600

gttactggtg agaagaagaa ggaagaaatt acttctggca aaatacaggg gactctggaa 660

gatcaaatca tcagtgccaa ccccctactg gaggcctttg gcaacgccaa gaccgtgagg 720

aatgacaact cctctcgctt tggtaaattc atcagaatcc actttggcac tactggaaaa 780

ctggcatctg ctgatattga aacatatctg ctagagaagt ctagagttgt tttccagctt 840

aaggctgaga gaagttatca tattttttac cagattacat cgaataagaa accagaactt 900
attgaaatgc ttctgattac cacgaaccca tatgattacc catttgtcag tcaaggggag 960
atcagtgtgg ccagcatcga tgatcaggaa gaactgatgg ccacagatag tgctattgat 1020
attttgggct ttactaatga agaaaaggtc tccatttaca agctcacggg ggctgtgatg 1080
cattatggga acctaaaatt taagcaaaag cagcgtgagg agcaagcaga gccagatggc 1140
acagaagttg ctgacaaggc ggcctacctc cagagtctga actctgcaga tctgctcaaa 1200
gctctctgct accccagggt caaggtcggc aatgagtatg tcaccaaagg ccagactgta 1260
gaacaggtgt ccaacgcagt aggtgctctg gccaaagccg tctacgagaa gatgttcctg 1320
tggatggttg cccgcatcaa ccagcagctg gacaccaagc agcccaggca gtacttcatc 1380
ggggtcttgg acattgctgg ttttgagatt tttgatttca acagcctgga gcagctgtgc 1440
atcaatttca ccaatgagaa actgcaacag tttttcaacc accacatgtt cgtgctggag 1500
caggaggagt acaagaagga aggcatcgag tggacgttca tcgacttcgg gatggacctg 1560
gctgcctgca tcgagctcat cgagaagcct atgggcatct tctccatcct ggaagaggag 1620
tgcatgttcc ctaaggcaac agacacctcc ttcaagaaca agctgtatga ccagcacctg 1680
ggcaagtctg ccaacttcca gaagcccaag gtggtcaaag gcaaggccga ggcccacttc 1740
gctctgattc actatgctgg tgttgtggac tacaacatta ctggctggct ggagaagaac 1800
aaggaccccc tgaatgagac cgtggttgga ctgtaccaga agtctgcaat gaaaactcta 1860
gctcagctct tctctggggc tcaaactgct gaaggagagg gagctggtgg aggggccaag 1920
aaaggtggta agaagaaggg ctcttctttc cagacagtgt ctgccctttt cagagagaat 1980
ttgaacaagc tgatgaccaa cctcaggagt acccatcctc actttgtgag gtgtatcatc 2040
cccaatgaga caaaaactcc tggtgccatg gagcatgagc ttgtcctcca ccagctgagg 2100
tgtaacggtg tgctggaagg catccgcatc tgtaggaaag gatttccaag cagaatcctt 2160
tatgcagact tcaaacagag atacaaggta ttaaatgcaa gtgcaatccc tgaagggcaa 2220
ttcattgata gcaagaaggc ctctgagaag ctccttgcat ccatcgacat tgaccacacc 2280
cagtataaat ttgggcacac caaggtcttt ttcaaagctg gtcttctggg gctcctagag 2340
gagatgcgag atgacaagct ggcccagctg attacccgaa cccaggccag gtgcagaggg 2400
ttcttggcaa gagtggagta ccagaggatg gtggagagaa gggaggccat cttctgtatc 2460
cagtacaata tcagatcctt catgaatgtc aagcactggc cctggatgaa actcttcttc 2520
aagatcaagc ctctgttgaa gagtgcagaa actgagaagg agatggccac catgaaggaa 2580

gaatttcaga aaattaaaga cgaacttgcc aagtcagagg caaaaaggaa ggaactggaa 2640
gaaaagatgg tgacgctgtt gaaagaaaaa aatgacttgc agctccaagt tcaggctgaa 2700
gccgaaggct tggctgatgc agaggaaagg tgtgaccagc taatcaaaac caaaatccag 2760
ctagaagcca aaatcaaaga ggtgactgag agagctgagg atgaggaaga gatcaatgct 2820
gagctgacag ccaagaagag gaaactggag gatgaatgtt cagaactcaa gaaagacatt 2880
gatgaccttg agctgacact ggccaaggtt gagaaggaga aacatgccac agaaaacaag 2940
gtgaaaaacc tcacagaaga gatggcaggt ctggatgaaa ccattgctaa gctgaccaag 3000
gagaagaagg ctctccagga ggcccaccag cagaccctgg atgacctgca ggcagaggag 3060
gacaaagtca acaccctgac caaagctaaa atcaaacttg aacaacaagt ggatgatctt 3120
gaagggtcct tggagcaaga aaagaaactt cgcatggacc tagaaagggc taagaggaaa 3180
cttgagggtg acttgaagtt ggcccaagaa tccataatgg acattgaaaa tgagaaacag 3240
caacttgatg aaaagctcaa aaagaaagag tttgaaatca gcaatctgca aagcaagatt 3300
gaagatgaac aggcacttgg cattcaattg cagaagaaaa ttaaagaatt gcaagcccgc 3360
attgaggagc tggaggagga aatcgaggcg gagcgggcct cccgggccaa agcagagaag 3420
cagcgctctg acctctcccg ggagctggag gagatcagcg agaggctgga agaagccggt 3480
ggggccactt cagcccagat tgagatgaac aagaagcggg aggctgagtt ccagaaaatg 3540
cgcagggacc tggaggaggc caccctacag catgaagcca cagcggccac cctgaggaag 3600
aagcatgcag atagtgtggc cgagcttggg gagcagattg acaacctgca gcgagtgaag 3660
cagaagctgg agaaggagaa gagtgagatg aagatggaga ttgatgacct tgctagtaat 3720
gtagaaacgg tctccaaagc caagggaaac ctagagaaaa tgtgccggac tctagaggac 3780
caactgagtg aactgaaatc aaaggaagag gagcagcagc ggctgatcaa tgacctgact 3840
gcgcagaggg ggcgcctgca gactgaatct ggtgagtttt cacgccagct tgatgaaaag 3900
gaagctctgg tgtctcagtt atcaagaggc aaacaagcct ttactcaaca gattgaagaa 3960
ttaaagaggc aacttgaaga ggagataaaa gccaagaacg ccctggcgca tgccctgcag 4020
tcttcccgcc acgactgtga cctgctgcgg gaacagtatg aggaggagca ggaatccaag 4080
gccgagctgc agagagcact gtccaaggcc aacaccgagg ttgcccaatg gaggaccaaa 4140
tacgagacgg acgccatcca gcgcacagag gagctggagg aggccaagaa gaagctggcc 4200
cagcggctgc aggcagctga ggaacatgta gaagctgtga acgccaaatg tgcttccctc 4260

gaaaagacga agcagcggct gcagaatgag gtcgaggacc tcatgcttga tgtggagagg 4320

acaaatgccg cctgtgccgc ccttgacaaa aagcaaagga acttcgataa gatcctggca 4380

gaatggaaac agaaatgtga ggaaacgcat gctgagcttg aggcctccca gaaggaggcc 4440

cgttcccttg gcactgagct gttcaagata aagaatgcct atgaggaatc tttggatcag 4500

ctagaaaccc tgaagcgaga gaacaaaaac ttacagcagg agatttctga cctcacggaa 4560

cagattgcag aaggagggaa acgtatccat gaactggaga aaataaagaa acaagtggaa 4620

caagaaaagt gtgaacttca ggctgcttta gaagaagcag aggcatctct tgaacatgaa 4680

gagggaaaga tcctgcgcat ccagcttgag ttgaaccaag tcaagtctga ggttgatagg 4740

aaaattgctg aaaaagatga ggaaattgac cagctgaaga gaaaccacat tagaatcgtg 4800

gagtccatgc agagcacgct ggatgctgag atcaggagta ggaatgatgc cattaggctc 4860

aagaagaaga tggagggaga cctcaatgaa atggaaatcc agctgaacca tgccaaccgc 4920

atggctgctg aggccctgag gaactacagg aacacccaag gcatcctcaa ggatacccag 4980

atccacctgg atgatgctct ccggagccag gaggacctga aggaacagct ggccatggtg 5040

gagcgcagag ccaacctgct gcaggctgag atcgaggagc tgcgggccac tctggaacag 5100

acagagagga gcagaaaaat cgcagaacag gagctcctgg atgccagtga gcgtgttcag 5160

ctactgcaca cccagaacac cagcctgatc aacaccaaga agaagctgga gacagatatt 5220

tcccaaatgc aaggagagat ggaggacatt ctccaggaag cccgcaatgc agaagaaaag 5280

gccaagaagg ccatcactga tgccgccatg atggctgagg agctgaagaa ggagcaggac 5340

accagcgccc acctggagcg gatgaagaag aacatggagc agaccgtgaa ggatctgcag 5400

ctccgtctgg atgaggctga gcagctggcc ctgaagggtg ggaagaagca gatccagaaa 5460

ctggaggcca gggtacggga gctggaagga gaggttgaga gtgagcaaaa gcgtaatgct 5520

gaggctgtca aaggtctgcg caaacatgag aggcgagtga aggaactcac ttaccagacg 5580

gaagaagata gaaagaatat tctcaggctt caagatttgg tagataaact tcaggcaaaa 5640

gtgaaatctt ataagagaca agctgaggag gctgaggaac aatccaacac caatctagct 5700

aaattccgca agctccagca tgagctggag gaggccgagg aacgggctga cattgctgag 5760

tcccaggtga acaaactgcg ggtgaagagc cgggaggttc acacaaaagt cataagtgaa 5820

gagtgatcat gtcctgatgc catggaatga ctgaagacag gcacaaaatg tgacatcttt 5880

ggtcatttcc ctctgtaatt attgtgtatt ctaccctgtt gcaaaggaaa taaagcatag 5940

ggtagtttgc aaacaa 5956

<210> 101
<211> 1042
<212> DNA
<213> human

<400> 101
tttcctcact gactataaaa gaatagagaa ggaagggctt cagtgaccgg ctgcctggct 60

gacttacagc agtcagactc tgacaggatc atggctatga tggaggtcca gggggggaccc 120

agcctgggac agacctgcgt gctgatcgtg atcttcacag tgctcctgca gtctctctgt 180

gtggctgtaa cttacgtgta ctttaccaac gagctgaagc agatgcagga caagtactcc 240

aaaagtggca ttgcttgttt cttaaaagaa gatgacagtt attgggaccc caatgacgaa 300

gagagtatga acagcccctg ctggcaagtc aagtggcaac tccgtcagct cgttagaaag 360

atgattttga gaacctctga ggaaaccatt tctacagttc aagaaaagca acaaaatatt 420

tctcccctag tgagagaaag aggtcctcag agagtagcag ctcacataac tgggaccaga 480

ggaagaagca acacattgtc ttctccaaac tccaagaatg aaaaggctct gggccgcaaa 540

ataaactcct gggaatcatc aaggagtggg cattcattcc tgagcaactt gcacttgagg 600

aatggtgaac tggtcatcca tgaaaaaggg ttttactaca tctattccca aacatacttt 660

cgatttcagg aggaaataaa agaaaacaca aagaacgaca aacaaatggt ccaatatatt 720

tacaaataca caagttatcc tgaccctata ttgttgatga aaagtgctag aaatagttgt 780

tggtctaaag atgcagaata tggactctat tccatctatc aagggggaat atttgagctt 840

aaggaaaatg acagaatttt tgtttctgta acaaatgagc acttgataga catggaccat 900

gaagccagtt ttttcggggc ctttttagtt ggctaactga cctggaaaga aaaagcaata 960

acctcaaagt gactattcag ttttcaggat gatacactat gaagatgttt caaaaaatct 1020

gaccaaaaca aacaaacaga aa 1042

<210> 102
<211> 969
<212> DNA
<213> human

<400> 102
agggtggtac tgaggcttca gcgggtgccg cccgcctaga gggagtggag cgagagccta 60

cgactagatt tgcatcttta cgtcctgcgc ggaggctgct acacacatgc agaagtcatg 120

ctggtggcct ggacattgaa gggagagaag tggatttggg agacatttag gagatggcac 180

cgaaagcgaa ggaagctcct gctcctccta aagccgaagc caaagcaaag gctttaaagg 240

ccaagaaggc agtgttgaaa ggtgtccgca gccacacgca aaaaagagga tccgcatgtc 300

actcaccttc aggcggccca agacactgcg actccggagg cagcccagat atcctcagaa 360

gagcaccccc aggagaaaca agcttggcca ctatgctatc atcaagtttc cgctgaccac 420

tgagtcggcc gtgaagaaga tagaagaaaa caacacgctt gtgttcactg tggatgttaa 480

agccaacaag caccagatca gacaggctgt gaagaagctc tatgacagtg atgtggccaa 540

ggtcaccacc ctgatttgtc ctgataaaga gaagaaggca tatgttcgac ttgctcctga 600

ttatgatgct ttcaatgttg taacaaaatt gggatcacct aaactgagtc cagctggcta 660

actctaaata tatgtgtatc ttttcagcat aaaaaataat gtttttcata agaatgacaa 720

cttaattaga atcaaatcta taagctttaa gattttacgt ttctagtaag tataatatta 780

gcttatttga ctagaactca agcagaatag gaatttatgc ttgttttata ttcaataatg 840

ataattttga agatatagtt gttttattac accaaaaata ctatattaat cttatttaac 900

taagttttat ccaaatcatg ttaacttaag aaacatttga tcagttccta taaaaaaaaa 960

aaaaaaaaa 969

<210> 103
<211> 2000
<212> DNA
<213> human

<400> 103
gcgcccagaa cgccccggcc atgggcatcc gaggcatgct gcgagccgca gtgatcctgc 60

tgctcatcag gacctggctc gcggagggca actaccccag tcccatcccg aaattccact 120

tcgagttctc ctctgctgtg cccgaagtcg tcctgaacct cttcaactgc aaaaattgtg 180

caaatgaagc tgtggttcaa aagattttgg acagggtgct gtcaagatac gatgtccgcc 240

tgagaccgaa ttttggaggt gcccctgtgc ctgtgagaat atctatttat gtcacgagca 300

ttgaacagat ctcagaaatg aatatggact acacgatcac gatgtttttt catcagactt 360

ggaaagattc acgcttagca tactatgaga ccaccctgaa cttgaccctg gactatcgga 420

tgcatgagaa gttgtgggtc cctgactgct actttctgaa cagcaaggat gctttcgtgc 480

atgatgtgac tgtggagaat cgcgtgtttc agcttcaccc agatggaacg gtgcggtacg 540

gcatccgact caccactaca gcagcttgtt ccctggatct gcataaattc cctatggaca 600

agcaggcctg caacctggtg gtagagagct atggttacac ggttgaagac atcatattat 660

tctgggatga caatgggaac gccatccaca tgactgagga gctgcatatc cctcagttca 720

ctttcctggg aaggacgatt actagcaagg aggtgtattt ctacacaggt tcctacatac 780

gcctgatact gaagttccag gttcagaggg aagttaacag ctaccttgtg caagtctact 840

ggcctactgt cctcaccact attacctctt ggatatcgtt ttggatgaac tatgattcct 900

ctgcagccag ggtgacaatt ggcttaactt caatgctcat cctgaccacc atcgactcac 960

atctgcggga taagctcccc aacatttcct gtatcaaggc cattgatatc tatatcctcg 1020

tgtgcttgtt ctttgtgttc ctgtccttgc tggagtatgt ctacatcaac tatcttttct 1080

acagtcgagg acctcggcgc cagcctaggc gacacaggag accccgaaga gtcattgccc 1140

gctaccgcta ccagcaagtg gtggtaggaa acgtgcagga tggcctgatt aacgtggaag 1200

acggagtcag ctctctcccc atcaccccag cgcaggcccc cctggcaagc ccggaaagcc 1260

tcggttcttt gacgtccacc tccgagcagg cccagctggc cacctcggaa agcctcagcc 1320

cactcacttc tctctcaggc caggcccccc tggccactgg agaaagcctg agcgatctcc 1380

cctccacctc agagcaggcc cggcacagct atggtgttcg ctttaatggt ttccaggctg 1440

atgacagtat tattcctacc gaaatccgca accgtgtcga agcccatggc catggtgtta 1500

cccatgacca tgaagattcc aatgagagct tgagctcgga tgagcgccat ggccatggcc 1560

ccagtgggaa gcccatgctt caccatggcg agaagggtgt gcaagaagca ggctgggacc 1620

ttgatgacaa caatgacaag agcgactgcc ttgccattaa ggagcaattc aagtgtgata 1680

ctaacagtac ctggggcctt aatgatgatg agctcatggc ccatggccaa gagaaggaca 1740

gtagctcaga gtctgaggat agttgccccc caagccctgg gtgctccttc actgaagggt 1800

tctccttcga tctctttaat cctgactacg tcccaaaggt cgacaagtgg tcccggttcc 1860

tcttccctct ggcctttggg ttgttcaaca ttgtttactg ggtataccat atgtattagt 1920

cccccagtgc tccagaacag cgggagcact gtgctgtgct cctttcagtt tcttttgggt 1980

ttgtttttcc ctctttcctt 2000

<210> 104
<211> 5468
<212> DNA
<213> human

<400> 104
cgcccgccca gccccggggg cagggaaagc ctaaattacg gaattaccgc gagcaaggag 60

cgcggaatcg gggagcgtcc ggagctagct ggatcctcta ggcaggatgg tgatgggaat 120

```
ctttgcaaat tgtatcttct gtttgaaagt gaagtactta cctcagcagc agaagaaaaa    180
gctacaaact gacattaagg aaaatggcgg aaagttttcc ttttcgttaa atcctcagtg    240
cacacatata atcttagata atgctgatgt tctgagtcag taccaactga attctatcca    300
aaagaaccac gttcatattg caaacccaga ttttatatgg aaatctatca gagaaaagag    360
actcttggat gtaaagaatt atgatcctta taagcccctg gacatcacac cacctcctga    420
tcagaaggcg agcagttctg aagtgaaaac agaaggtcta tgcccggaca gtgccacaga    480
ggaggaagac actgtggaac tcactgagtt tggtatgcag aatgttgaaa ttcctcatct    540
tcctcaagat tttgaagttg caaaatataa caccttggag aaagtgggaa tggagggagg    600
ccaggaagct gtggtggtgg agcttcagtg ttcgcgggac tccagggact gtcctttcct    660
gatatcctca cacttcctcc tggatgatgg catggagact agaagacagt ttgctataaa    720
gaaaacctct gaagatgcaa gtgaatactt tgaaaattac attgaagaac tgaagaaaca    780
aggatttcta ctaagagaac atttcacacc tgaagcaacc caattagcat ctgaacaatt    840
gcaagcattg cttttggagg aagtcatgaa ttcaagcact ctgagccaag aggtgagcga    900
tttagtagag atgatttggg cagaggccct gggccacctg gaacacatgc ttctcaagcc    960
agtgaacagg attagcctca acgatgtgag caaggcagag ggattctcc ttctagtaaa    1020
ggcagcactg aaaaatggag aaacagcaga gcaattgcaa aagatgatga cagagtttta   1080
cagactgata cctcacaaag gcacaatgcc caaagaagtg aacctgggac tattggctaa   1140
gaaagcagac ctctgccagc taataagaga catggttaat gtctgtgaaa ctaatttgtc   1200
caaacccaac ccaccatccc tggccaaata ccgagctttg aggtgcaaaa ttgagcatgt   1260
tgaacagaat actgaagaat ttctcagggt tagaaaagag gttttgcaga atcatcacag   1320
taagagccca gtggatgtct tgcagatatt tagagttggc agagtgaatg aaaccacaga   1380
gtttttgagc aaacttggta atgtgaggcc cttgttgcat ggttctcctg tacaaaacat   1440
cgtgggaatc ttgtgtcgag ggttgctttt acccaaagta gtggaagatc gtggtgtgca   1500
aagaacagac gtcggaaacc ttggaagtgg gatttatttc agtgattcgc tcagtacaag   1560
tatcaagtac tcacacccgg gagagacaga tggcaccaga ctcctgctca tttgtgacgt   1620
agccctcgga aagtgtatgg acttacatga gaaggacttt tccttaactg aagcaccacc   1680
aggctacgac agtgtgcatg gagtttcaca aacagcctct gtcaccacag actttgagga   1740
tgatgaattt gttgtctata aaaccaatca ggttaaaatg aaatatatta ttaaattttc   1800
catgcctgga gatcagataa aggactttca tcctagtgat catactgaat tagaggaata   1860
```

cagacctgag ttttcaaatt tttcaaaggt tgaagattac cagttaccag atgccaaaac 1920
ttccagcagc accaaggccg gcctccagga tgcttctggg aacttggttc ctctggagga 1980
tgtccacatc aaagggagaa tcatagacac tgtagcccag gtcattgttt ttcagacata 2040
cacaaataaa agtcacgtgc ccattgaggc aaaatatatc tttcctttgg atgacaaggc 2100
cgctgtgtgt ggcttcgaag ccttcatcaa tgggaagcac atagttggag agattaaaga 2160
gaaggaagaa gcccagcaag agtacctaga agccgtgacc cagggccatg gcgcttacct 2220
gatgagtcag gatgctccgg acgtttttac tgtaagtgtt ggaaacttac cccctaaggc 2280
taaggttctt ataaaaatta cctacatcac agaactcagc atcctgggca ctgttggtgt 2340
ctttttcatg cccgccaccg tagcaccctg gcaacaggac aaggctttga atgaaaacct 2400
tcaggataca gtagagaaga tttgtataaa agaaatagga acaaagcaaa gcttctcttt 2460
gactatgtct attgagatgc cgtacgtgat tgaattcatt ttcagtgata ctcatgaact 2520
gaaacaaaag cgcacagact gcaaagctgt cattagcacc atggaaggca gctccttaga 2580
cagcagtgga ttttctctcc acatcggttt gtctgctgcc tatctcccaa gaatgtgggt 2640
tgaaaaacat ccagaaaaag aaagcgaggc ttgcatgctt gtctttcaac ccgatctcga 2700
tgtcgacctc cctgacctag ccaatgagag cgaagtgatt atttgtcttg actgctccag 2760
ttccatggag ggtgtgacat tcttgcaagc caaggaaatc gccttgcatg cgctgtcctt 2820
ggtgggtgag aagcagaaag taaatattat ccagttcggc acaggttaca aggagctatt 2880
ttcgtatcct aagcatatca caagcaatac cgcggcagca gagttcatca tgtctgccac 2940
acctaccatg gggaacacag acttctggaa aacactccga tatcttagct tattgtaccc 3000
tgctcgaggg tcacggaaca tcctcctggt gtctgatggg cacctccagg atgagagcct 3060
gacattacag ctcgtgaaga ggagccgccc gcacaccagg ttattcgcct gcggtatcgg 3120
ttctacagca aatcgtcacg tcttaaggat tttgtcccag tgtggtgccg gagtatttga 3180
atattttaat gcaaaatcca agcatagttg gagaaaacag atagaagacc aaatgaccag 3240
gctatgttct ccgagttgcc actctgtctc cgtcaaatgg cagcaactca atccagatgc 3300
gcccgaggcc ctgcaggccc cagcccaggt gccatccttg tttcgcaatg atcgactcct 3360
tgtctatgga ttcattcctc actgcacaca ggcaactctg tgtgcactaa ttcaagagaa 3420
agaattttgt acaatggtgt cgactactga gcttcagaag acaactggaa ctatgatcca 3480
caagctggca gcccgagctc taatcagaga ttatgaagat ggcattcttc acgaaaatga 3540

aaccagtcat gagatgaaaa aacaaacctt gaaatctctg attattaaac tcagtaaaga 3600
aaactctctc ataacacaat ttacaagctt tgtggcagtt gagaaaaggg atgagaatga 3660
gtcacctttt cctgatattc caaaagtttc tgaacttatt gccaaagaag atgtagactt 3720
cctgccctac atgagctggc aggggaacc ccaagaagcc gtcaggaacc agtctctttt 3780
agcatcctct gagtggccag aattacgttt atccaaacga aaacatagga aaattccatt 3840
ttccaaaaga aaaatggaat tatctcagcc agaagtttct gaagattttg aagaggatgc 3900
cttaggtgta ctaccagctt tcacatcaaa tttggaacgt ggacgtgtgg aaaagctatt 3960
ggatttaagt tggacagagt catgtaaacc aacagcaact gaaccactat ttaagaaagt 4020
cagtccatgg gaaacatcta cttctagctt ttttcctatt ttggctccgg ccgttggttc 4080
ctatcttacc ccgactaccc gcgctcacag tcctgcttcc ttgtcttttg cctcatatcg 4140
tcaggtagct agtttcggtt cagctgctcc tcccagacag tttgatgcat ctcaattcag 4200
ccaaggccct gtgcctggca cttgtgctga ctggatccca cagtcggcgt cttgtcccac 4260
aggacctccc cagaacccac cttctgcacc ctattgtggc attgtttttt cagggagctc 4320
attaagctct gcacagtctg ctccactgca acatcctgga ggctttacta ccaggccttc 4380
tgctggcacc ttccctgagc tggattctcc ccagcttcat ttctctcttc ctacagaccc 4440
tgatcccatc agaggttttg ggtcttatca tccctctgct tactctcctt ttcattttca 4500
accttccgca gcctctttga ctgccaacct taggctgcca atggcctctg ctttacctga 4560
ggctctttgc agtcagtccc ggactacccc agtagatctc tgtcttctag aagaatcagt 4620
aggcagtctc gaaggaagtc gatgtcctgt ctttgctttt caaagttctg acacagaaag 4680
tgatgagcta tcagaagtac ttcaagacag ctgcttttta caaataaaat gtgatacaaa 4740
agatgacagt atcccgtgct ttctggaagt aaaagaagag gatgaaatag tgtgcacaca 4800
acactggcag gatgctgtgc cttggacaga actcctcagt ctacagacag aggatggctt 4860
ctggaaactt acaccagaac tgggacttat attaaatctt aatacaaatg gtttgcacag 4920
ctttcttaaa caaaaaggca ttcaatctct aggtgtaaaa ggaagagaat gtctcctgga 4980
cctaattgcc acaatgctgg tactacagtt tattcgcacc aggttggaaa aagagggaat 5040
agtgttcaaa tcactgatga aaatggatga cccttctatt tccaggaata ttccctgggc 5100
ttttgaggca ataaagcaag caagtgaatg ggtaagaaga actgaaggac agtacccatc 5160
tatctgccca cggcttgaac tggggaacga ctgggactct gccaccaagc agttgctggg 5220
actccagccc ataagcactg tgtcccctct tcatagagtc ctccattaca gtcaaggcta 5280

agtcaaatga aactgaattt taaacttttt gcatgcttct atgtagaaaa taatcaaatg 5340

ataatagata cttataatga aacttcatta aggtttcatt cagtgtagca attactgtct 5400

ttaaaaatta agtggaagaa gaattacttt aatcaactaa caagcaataa taaaatgaaa 5460

cttaaaat 5468

<210> 105
<211> 902
<212> DNA
<213> human

<400> 105
gggggggagc ggggaaaaca aaaggagacg aaggacgcat gcgtttggtg agtcccggat 60

tctggtgggt tcttccgctc aggctgggtg aagcgcttcc gggtcgccgc cggcagcagc 120

ctcccggcgc gatgaagaca ctgaggctca gagaggttaa gtgactcagc caaggtcaaa 180

cagctagtaa gtggtggagc caggactcaa agccagtcta ggagccatgt ccactttgtt 240

cccctcactc ttccctcgtg tgactgagac tctgtggttt aatctggatc gaccctgtgt 300

ggaagagaca gagctgcagc agcaggaaca gcagcatcag gcctggctcc aaagcatcgc 360

ggagaaagac aacaacctgg ttcctattgg caagccagcc tcagagcact atgatgacga 420

ggaagaagag gatgatgaag atgatgagga tagtgaagag gactcagagg atgatgagga 480

tatgcaggac atggacgaga tgaatgacta caatgagtca ccggatgatg gagaggtcaa 540

tgaggtggac atggaaggca acgaacagga tcaggaccag tggatgatct aggtagacaa 600

ggcagggtgg cctcagggag attccaggcc agcccaaact accctgcatc ccaacccccca 660

acccctgccc acagaaccag ctgatggccc cagtgcctga aagtgccctt gggcacctcc 720

tcagctgctg ccaggatctg gtctctttgg cccctcccag gccatcagtc tgcacttgaa 780

atccccaggg cctgaaacct actccacctt cctggccagt acctcacccc ttgattgcca 840

ggtctggtct aagtttcttt aataaagaca aaggagtgat tttcaaaaaa aaaaaaaaaa 900

aa 902

<210> 106
<211> 2039
<212> DNA
<213> human

<400> 106
aatgacatga cagccattcc gtggccaggg acaccactgc ccaagctgga gaccacgagg 60

```
attcagggac tgaagccagc atgggaattc ctggtttgag atcagagtcc tgagtacctc    120
gtgggaactt gggcactcat ccgcaggagg tctagacccc cagagaattc cttgagtcta    180
aggcacaggc ccaacatgtg tagcaccagt gggtgtgacc tggaagaaat ccccctagat    240
gatgatgacc taaacaccat agaattcaaa atcctcgcct actacaccag acatcatgtc    300
ttcaagagca cccctgctct cttctcacca aagctgctga gaacaagaag tttgtcccag    360
aggggcctgg ggaattgttc agcaaatgag tcatggacag aggtgtcatg gccttgcaga    420
aattcccaat ccagtgagaa ggccataaac cttggcaaga aaaagtcttc ttggaaagca    480
ttctttggag tagtggagaa ggaagattcg cagagcacgc ctgccaaggt ctctgctcag    540
ggtcaaagga cgttggaata ccaagattcg cacagccagc agtggtccag gtgtctttct    600
aacgtggagc agtgcttgga gcatgaagct gtggacccca aagtcatttc cattgccaac    660
cgagtagctg aaattgttta ctcctggcca ccaccacaag cgacccaggc aggaggcttc    720
aagtccaaag agatttttgt aactgagggt ctctccttcc agctccaagg ccacgtgcct    780
gtagcttcaa gttctaagaa agatgaagaa gaacaaatac tagccaaaat tgttgagctg    840
ctgaaatatt caggagatca gttggaaaga aaggacactg ccttcatccc cattcccttg    900
gttgacacca gcatccaggg ttttccacag gatggtttga tggcctgcat ttgagctaaa    960
gaatgaactt ctgtctgcct cgtggagcca agctactgta ctgagtgctt attcttttgt   1020
acacagctga agaaagataa ggctttgatg ggccacttcc aggatgggct gtcctactct   1080
gttttcaaga ccatcacaga ccaggtccta atgggtgtgg accccagggg agaatcagag   1140
gtcaaagctc agggctttaa ggctgccctt gtaatagacg tcacggccaa gctcacagct   1200
attgacaacc acccgatgaa cagggtcctg ggctttggca ccaagtacct gaaagagaac   1260
ttctcgccat ggatccagca gcacggtgga tgggaaaaaa tacttgggat atcacatgaa   1320
gaagtagact gaaatatcag atttgtcatc aggaatactc tttgtctact gtggtcctgt   1380
gcacgttggc ctcagatgga ctacaggaga ttacaacgta caaggcagat ggagcattga   1440
cgttttcaaa accattattc ctgtgactgg agaggcatca ggagaggtct cgttcgtctc   1500
cagctcataa aatgtagcag catcatcctt gacagtgatg tttttcaggc cctccattga   1560
gaacctgagg aaatctgtaa agataagtgg tgatgttgtt tcaaacgttc agaacagata   1620
ccatcatcct gcctttgtta gctgctgtag ggaaagtgcg ttacagatgt ctgctgacct   1680
cacaagagtg aaaagataaa ctgtgcatgt gtttccactt ccgtttctag tactatttat   1740
ttttaaacta cacttggggt ggcctaatac ctaggaagat gttgctattc acgttagtaa   1800
```

acagcctaaa gaaactctta ggtttactgc tacatccatt tgtttggaga ggtaactgtt 1860

gtctgtgcct ttttgaaaaa cttccatttg gtacaaaatt tttactccaa caccccctca 1920

acccttttct cagggaccac acctcttctt cccaaggtcc ctgggacttc ctcattcttt 1980

gtggtagtac aatgattggt agcaggtaaa ataaatacat agaaagacta ctgtcaaaa 2039

<210> 107
<211> 2030
<212> DNA
<213> human

<400> 107
gtccgttagg acgtgttgcc ctttctgtgt aagctgtgag cgtaggcggc cctgagcggg 60

tgtgttgcag gggtttccaa gcccagcacc agcacccttg ccctttttcca tcaggggttc 120

agcctagggt ccccgctggt gggcggctcc cgagtcttgg agaagagcac gagaacctag 180

accgcccccg aagtgcggag accccctggg caggctgaaa gatggcggcg gcgtctgtct 240

ctgcggcttc tggttctcac ttgtcgaaca gctttgctga gccatcaagg tctaatggaa 300

gcatggttcg gcattcttca tctccatatg tagtatatcc ttcggataag cctttcctta 360

atagtgatct acgacgctcc ccaagtaagc ctacacttgc ctatccagaa agcaacagca 420

gagccatatt ttctgctctt aagaatcttc aagataagat tcgacgcttg gaacttgaga 480

ggattcaggc agaagaaagt gtgaaaacct tgtctagaga aacaattgaa tataagaaag 540

tactggatga acagatacaa gaaagggaga attcaaagaa tgaggaatca aagcacaatc 600

aagaactgac atctcagttg ttagctgcag aaaataaatg caatctatta gaaaaacaat 660

tggaatacat gcgaaatatg ataaagcatg ccgaaatgga gaggacatct gtcttagaga 720

aacaagtttc cctagaaaga gaacgacaac atgatcaaac acatgttcag agccaacttg 780

aaaaattgga tcttcttgaa caggagtata acaaacttac cacaatgcag gcccttgcag 840

aaaaaaaaat gcaagagttg gaagcaaaac tccatgaaga agaacaggaa aggaaacgca 900

tgcaagctaa ggcagctgag ttgcagactg gtctagaaac aaatagactt atctttgaag 960

ataaggcaac tccgtgtgtt cccaatgcaa gaagaattaa aaaaaagaag tcaaaaccac 1020

cagaaaagaa aagttctagg aactattttg gtgcacaacc acattataga ttatgcttgg 1080

gtgatatgcc atttgtagct gggaagtcca caagccctag ccatgccgtg gtagccaatg 1140

ttcagcttgt cttgcatcta atgaagcaac acagtaaagc tttgtgcaat gatcgagtca 1200

tcaacagtat tcctttggca aagcaagtat cttcacgagg tggtaaaagt aagaagttgt 1260

cagtaacacc tccctcctcc aacggtatta atgaggagtt gtcagaagtc ttacagactt 1320

tacaggatga atttgggcaa atgagctttg atcaccagca gcttgcaaaa cttatccagg 1380

agtcgccaac cgttgaactg aaagacaagt tggagtgtga attggaggca ttagtgggaa 1440

ggatggaagc aaaagccaac caaataacta aagttcgaaa ataccaagcc cagctggaga 1500

aacagaagtt agagaagcag aagaaggaat taaaagctac caaaaagact cttgatgaag 1560

aaagaaacag cagcagccgt tctggaatca cagggaccac aaataagaaa gattttatga 1620

aactgagacc tggagaaaaa aggagaaaaa atcttcagtt attgaaggac atgcaaagca 1680

tacagaattc attacaaagc agtagtttgt gttgggatta ctgactcata accaggtcag 1740

aaattttatt cagataatct gtacctcatc aatcagatga tgacaattta cttcccaggt 1800

ctcatactca cttatgttgg aattaattaa tagcaggtgt taaaggaccc aggcttcatt 1860

acacaggctt ttcatgtatg caggatgact caatgttaaa gcatttaaat ggaaaccagg 1920

ggagttttaa agcccgagaa accacacata atcttttgtt gagatgagtt tgctgtactg 1980

acgctgcact ttgtaaacag attaccagtt ttttaaaaaa aaaaaaaaaa 2030

<210> 108
<211> 57759
<212> DNA
<213> human

<400> 108
ccgagtgtga tggtgccctc ttggagtccc agctactcag gaggctaagg cgggaagatc 60

atttaagccc aggagatgga ggctgcagtg agctgtgatc taccactgca ttccagcctg 120

ggtgacagag caagaccctg tctaaaaaaa aaaaaaagaa aaaaaagaaa agaaaagaat 180

gagtgggagg gcgaggcgcg gtggctcacg cctgtaatcc cagcactttg ggagtgggag 240

gtgggtggat tacctgaggt cgggagttcg agctcagcct ggctaacatg gtgaaacccc 300

gtctctacta aatacacaaa attagctggg cgtggtggca cacgcctgta atcccagcta 360

ctccggaggc tgaggcagga gaatcgcttg aacccaggag gcggaggttg cagtgagccg 420

ggattgtgcc actgcactcc aacctgggcg acagtgagac tccgtctcaa aaaaaaaatg 480

aagtactggt acctgcaaca gacacaaaag ccacatatta tatgattccc tgtgtgtgaa 540

atgtccagaa gaggcaaatc catagagctg acaagtagaa tggtgattac caagagctta 600

ggagaggaag ggctaatggc cttgaggttc tttttaaggt gatgagcatg tttatttgga 660

gacagagtct cgttgtgttg cccaggctgg agtgcagtgg tgtgttcaca gctcactgca 720

gcctcaacct cctaggctca agtgctcctg ccatctcagc ctcccgagca gcttggacca 780
cagggctgaa ccatcacccc cagctaattt tttctgtttt ttgtaaaggc aaggtctcac 840
aaaatcccaa catgctggga ttacaggcgt gagccaccat gcctggcccc tactcattct 900
tttttttttt taagatggag tcttgctgtg tcacccaggc tggagtgcag tggcacaatc 960
ttggctcact gcaacctcca cctcctgagt tcaagcaatt ctccctgcct cagcctcccg 1020
agtacctggg attacaggcg cctgccacca cgcccagcta atttttgtat ttttagtaga 1080
gatggcgttt caccatgttg gccaggttgg tctcgaaatc ctgacctcag gtgatccacc 1140
caccttggcc tcccaaagtg ctgggattac aggcatgagc caccgcgccc agccgcccct 1200
actcattctt aatcacacca cctgactttc tccacattga tcacattagg atattatctt 1260
gtttatttac agaaaatctg tgtccctctt cagtgcactg gactcttcgg tgcactggat 1320
gctcaatgca ggcactaagt tttgtctgac ttaccactgg ttgtatgccc tgctcacagc 1380
tggtgaatga gtgattggaa acacacctgc tcccttgatt taggcctctg ggaaacaagg 1440
gcaaggtaag ttcaggaaga agaaagacct cgtaatttcc aggtaggaaa aatcctttgg 1500
tgtggccgtg agacactgca ttcttcttgg ccacagctcc ctctgctttg agaatcacat 1560
aatcaacttt atctttctct gaatcgttca cataaaaggg aggtgttgga acacaaggtg 1620
gaaatgggtg tgttggcggt ggctgaggaa gtcggggcag gaggagccac tgtttctggg 1680
aatgaattcc tgaaacttgc tggtcccacc aagatgcaaa tcttgcggca gtttttacag 1740
ttatcctgtg gaagaagtga ggatcccact ccaagcccag gtcagaatgc tgataatccc 1800
ctctgatcag gttttttaa ttttattttt attttttgtg gtttggggaa gatttgccaa 1860
ctgtatcctc ttttctggca aggaaaattt atatctgccc aatgaaatca ggaatttaac 1920
ttcattcatt caacagttat ttaattctat cctctactag attcctacag ctgctgtaac 1980
cctaaagtac cacaaactgg gtggcttgaa acaacagaca cgtagccagg cgcagtggct 2040
cacgcctgta atcccagcac tttgggaggc tgaggtgggc agatcatctg aggtcaggag 2100
ttcaagacca gcctggccaa aacggtgaaa ccctgtctct gctaaaaata caaaaaaaat 2160
tagccgggca tggtggtggc gcacggctgc cattccagct actcggaatg ctgaggcagg 2220
agaaccgctt gaacctgtga ggcggaggtt gcagtgagag cgagactcta tctcaaaaaa 2280
acaaaacaaa aaaccccaac aacaacagac atgtatcctc tcacggttct ggaggccaga 2340
agtctcaaat caaacgatta gcagggccat gttctccctc cacaggctct tggggagaat 2400

```
gtttctctgc ctttctcttc gctgctggtg ttgctggcag ctcttggcac tttttgggtg   2460
taggctcatc actccagcac ctgccctggg gtcacatggc tctctcctca ctgtgcctct   2520
tcttttttta tttttgagac agggtctcat tctgtggccc aggctggagt gcagtggcac   2580
agtctcagct cactgcaacc tcctcctccc caggctcaag tgatcctcct acgtcagcct   2640
cccaagaagc tgggacttca ggctcatgcc accaagcccg gctaatgttt tgtattttta   2700
gtagagagag cagtttacca tgttgcctag gctagtctcg aatacctgag ttcaagtgat   2760
ctacttgtct cagcctccca cagtgctgag attacaggtg tgagccactg ggccgggccc   2820
tcctcttttt ttaaaaaaac aaaacaaaac aaaaacattt attttaagtt caggggtaca   2880
tgtgcagctt tgttatacag gtaaacttgt gtcacggggg tttgttttac agattattaa   2940
tttcatcacc caggtattaa gctagtatcc attagttatt ttttcttttt tgtttttttt   3000
ttttttttga gacagagttt cactcttgtt gcccaggctg gagtgcaatg gtgccatctc   3060
ggatcactgc aacctccgcc tcccgggttc aagtgattct cctgtctcag cctcccgagt   3120
agctgggatt acaggcatga gccaccgtgg ccagcctagt tatttttttct gatcgtctcc   3180
ttcctcccac cctccaccct tggccagacc ccaatgtgtg ttgttcccct ctatgtgtcc   3240
atgtgttctc atcatttagc tcccatttgt gagaatatgc agtatttggt tttctgttcc   3300
tgcattagtt tgctaaggat aatgacttct agctccatcc atgtccctgc aaaggacatg   3360
atatcattcc tttttatggc tgcatggtat tccatggtct atatgtagca cattttcttt   3420
atccagtcta tcactgatgg gcattgaggt tgattccatg tctttgctat tgcgaatagt   3480
gctgcagtcc ttttcttcta aggataccgg tcatattaga ttatgggtcc actctcatcc   3540
agtataacct catcttaaat gatatcttaa ttatatcttc aaagaagtaa ggtcacactc   3600
acaggtactg gggttaggac ttcaacatag gtttttgggg ggaaactatt caatgcctaa   3660
cacaacctct ccaagaaaaa aatcttactg aaattttaat tggaattgta ttcaatttat   3720
atcataattg ggggattttg acttttgtgt tttttttttt tttttttttt tgagacagag   3780
tctcgctctg tcgcccaggc tggagtgcag tggcacgatc tcagctcact acaacctctg   3840
cctcctgggt tcatgccatt ctcctgcctc agcctcccat gtagctggga ttacaggtgc   3900
ccgccaccat gcctggctaa tttttgtatt tttagcagag acggggtttc accatgttgg   3960
tcaggctggt ctcaaactcc tgacctcagg tgaactgcac gcctcagcct tccaaagtgc   4020
tgggattaca tgcgtgagcc accatgcccg gcaacttttt ctttttcttt ttctttttct   4080
tttttttct tttttttttt ttgagatggc gtcttgctct gtcacccaga ctggagggta   4140
```

gtggcgccat ctcagctcac tgcaacatct gcccccccgg gttcaagcga ttctcctgcc 4200
tcagcctcca agtagctggg attataggca cccgccacca tgcctggcta atttttgtat 4260
ttttagtaga gatggggttt tgccatgttg gccaggctga tctcgaactc ctgacctcag 4320
gtgattcacc cactccgcct cccaaagtgc taggattaca ggcatgagcc attgcgccca 4380
gccaaattta tttctaagtg tttcatagtt tttgtctgca tgctgaatga aaatgtttct 4440
tatttaaatt tctaggtgtt tattgctaca gtgtgagaaa actattgtgt ggtttttttt 4500
gtttgttttt tgtttttttt taagatggag tcttgctcta tcgcccaggc tggagggcag 4560
tggcttgttc tgggctcact gcaacctccg cctcctgggt tcaagtgatt ctcctgcttc 4620
agcctcacta gtagctggga ttacaggtac ccaccaccag gcctgtctaa tttttgtatt 4680
tttcgtagag atggggtttt gctatgttgg ccaggctggt ctggaattcc tgacctcagg 4740
cgatccaccc acctccgcct cccaaagtgc tggcattaca gaagtgagcc accacgccca 4800
gccttgtttg atgtatattt atcctgagac tagccatcta accaaacttt cttatttatt 4860
ccagtgtttt gaaaaaataa ctagtatgtt ttgagttttc taagcacaca gtcctataaa 4920
tagagaaatg attgaatgtt gcatcttaga atattttgca ggataggaaa aataacctag 4980
ggctatctat cagctggcca tgacagcttt ccacaaggta gtgttaacta aggaaagcaa 5040
gatgtaagaa agaatgctta atataatctc agttttgaaa aacaatgagt gggccgggtg 5100
cggtggctct gccctgtaat cccagcattt tgggaggctg aggccagcgg atcacgaggt 5160
caggggtttg agaccagcct ggccaacatg gtgaaacccc gtctcttcta aaaatacaaa 5220
aattagctgg tgtggtggca cgctcctgta gccccagcta cttgggaggc tgaggcaaga 5280
gaatcacttg aacctggcag gcggaggttg cagtgagcca agctgcaccg ttgcactcca 5340
gcctgagcga cagagcagga ctctgtctca aaaaaaaaaa aaaaaaaaat cagtaaaagg 5400
caatatgtgt gtattaggat atatggtttt ttctttggtt tttttgtttt tgttttgttt 5460
tttgagacag agtttcgctc tcgttgctca ggctggagtg cgatgcgcaa tctcggctca 5520
ctgcaacctc ccgctcccgg gttcaaacga ttctcctgcc tcagcctccc tagtagctgg 5580
gattacaggc atgcaccacc acgcccagct aatttttttgt atttttagta gagacagggt 5640
tttgccatgt ttgtcaggct ggtcttgaac tcctgacctc aggtaatccg ctcacctcgg 5700
cctcccaaag tgctgggatt acaggagtga gccactgcac tcggccagga tatatgtttt 5760
gtatatgatt tcacattcat agagaaaata cagaatttta ggttaacatg agccactttg 5820

```
tgtaggtggg gaactaacat gggggcagaa aggagaagag aaggagataa gcagaaagtg   5880
aaattaaaca acctgccaaa agatccaaaa tcattatgac atggtcacat ttatgcatgt   5940
atgtaattta tgtgtgtgtg tatacatata tatttgtata tgttcataga aatttaaaat   6000
aatttctttt tttttttct tttggagatg gggtcttgtt atgttgccca ggctggtctc   6060
aaactcctgg gctcaagcag tgctcccacc ttggcctcac aaagtgctgg gattacagga   6120
gtgagccact gcacctggcc taaacatttt tttttttttc agatggggtt ttatgctgtt   6180
acgcaggttg gagtgcagtg ctaccatttc agctcactgc aacctctgcc tcccaggctc   6240
aagtgatcct gccacctcag cctcctgagt agctgggacc acaggcatgc ccccaccatat  6300
gtggctaatt ttttgtattt ttggtagaga cggtttcacc atgttgccta ggctggtctt   6360
gaacttctga gctcaggcca tccgcccacc tcagcctccc aaagtgctgg gactacaggc   6420
gtgagccacc atgactggcc ctaaaataat ttttaaatga acacaaaaac aagcaaacaa   6480
gcaaaatgta gataaagtgg ctgttgaaga caggccttgg ctgggttctg gaatagccga   6540
gatgaggaag gctgcaccct caaggacacc ccacagagaa cctctgactg ctggtgacag   6600
gaggaatgca acgacaacgg agggtcccac tcaccagcat ccgtgccctg tgttgtcaca   6660
gcagcccagt tttcctttgg ggaaccacct catctcccca tcccttctct atcacctgca   6720
ctgtgtatca tcttggtggg accatcaagc aagacactgc ccagtggccc cgtgcggtgt   6780
ggctcacgcc tgtaatccca gcactttggg aggccaaggc gtgcagatca cttgaggtca   6840
ggagttcgtg accagcctgg ccaacatggt gaaacgccgt gtctactaaa aatacaaaaa   6900
ttagccgggc atggtggtgg gcacctgtaa tgccagctgc tcgagaggct gaggcaggag   6960
aatctcttga acctgggagg cagaggttgc agtgagccga gatcacgcca ttgcactcta   7020
gtctgggcaa caagagcaaa actctgtctc aaaaaaaaag aacactatgc tcagccccag   7080
cagaggtggc tgagtcttca gggcagccag ccagatgctc ctctggaaat gtgactcagg   7140
aatgaagggc caagcacagt tgagcccaag ctctcaacat gaaggcctct tggtcttgct   7200
ccttaaaacc aggaggccgg gcacagtggc ccacgcctgt aatcccagca ctttgggaag   7260
ctgaggcagg tggatcacct gaggtcagga gttcgagacc agcctgacta acatggtgaa   7320
accttgtttc tactaaaaat acaaaaagtt agccaggtgt ggtggtgcac acctacaatc   7380
ccagctactc gggaggctga ggcagaagaa tcacttgaac ccaggaggcg gaggttgcag   7440
tgagctgaga ttgcaccagt gcagtccagc gtgggtgaca gagtgagact ccgtctcaaa   7500
caaacaaaca aacaaacaaa ccacactaaa taattatgag aatgcaaagt gaaagctgga   7560
```

gcaagcagaa ttaaggctca ggaggagggt gggtctggat ctgggaggat cacaaaagca 7620
agccaactcc cccaggccta agggagacca gcttgcagtt agacacaact gagggaaatg 7680
ggctgacatt tcctgtatct tccaggtgta agccccagag aaagcaagtc actggatcag 7740
gaaccatggt ctcctggatc caaatctagg cttttctccc agaaaggaca tctgccaggg 7800
atgcctggtc caggtggtgg aggctgggtg gctcaccgtt ctgtggtttc cgtcttcgtg 7860
gagagtttat tccctcacgt tgatgatgac ggaagggcag ggcttcttca aggtggctgc 7920
ttcactggtt tgtgatccac ccagtgtact cggagagctt ctcgccacac tttttctgta 7980
atttgccaga aatagtaaat attttaggct tcatgggccg tctgttctct gccacggtgc 8040
tagactttgt tgtagtgaca gcagccatac tgtttacata ttatctgtgg cagcttttga 8100
caccatatgg agtagaaagc ctacattttg ctatctggtt ctttacagaa gaagtttgcc 8160
aaccctgtca tatagtatga acatctttct taccaccatc tttttttga gatgaagtct 8220
tgttatattg cccaggctgg aaacaaactc ctgggaccaa gagatcctcc ccattcagcc 8280
tcccaagtag ctgggactaa aggcacatca ccaagccctg ctgcttacca ccaatttttt 8340
tttttttttt ttgagatgga gtctctctct cgcccaggct ggagtgcaat ggtgcgatct 8400
cagctcactg caacctctgc catccgagtt gaagcaattc tcctgcctca gcctcccgag 8460
tagctgggat tacaggggtg cgccaccacg cccagctgat ttttgtattt ttagtagaga 8520
cggggtttca ccatgttggc caggctgtta ccaccatttt taatggctgc attttgtttc 8580
ccttccacca atgcttccaa agctgcttag taatgacatt caaaacagaa tctgccaaga 8640
taagacacct tttgaatagc aataggaaaa gtatctgcaa atcctgactt ctcaaccagc 8700
cctgccctag tgtctaagct ccgacgggct gaaaaatggg cattttctaa gtcatttctc 8760
ctgatccaag atcttgaaac tcttcaaact tataaattag gccacctgct gagcaatcct 8820
gtaggccctg tcctaagatg actcagggca gtctcttctt ttccctgtaa cctggtccta 8880
ttcacgtgaa gctatttttc agctatttgc aaaaagctcc ttaaaatcta tttttccctt 8940
taattatagt ttgcagctgg tctttctgaa agcaaagaat ttattgcttg ccaagggcca 9000
ttaaagatca agctgggtta gggggtctcc ttgctgaatc cagggaggag ggttcaagac 9060
agaggctatt tccgaaccag tttttctagt tcccaggcac tctccggatt cttctctggg 9120
tttacttttc ttaggtggaa accctaccta gatttgcaaa tgacgtggag ctctttgtca 9180
cccaagatcc cttggggctc atgggtcagt cattgtttgg aaagtgttgc ctcttaggct 9240

tttctatttt tataaaagag ggattactgt ctttgctccc tgtaaccttg gactactgcc 9300
aagttttctt ggtggcattt tcacttgata aatatgagcc tttctgctcc tgacccagaa 9360
gagtgcttct cctagcagtc tcaggagttt cagacctagt tatacccctg catttatcgc 9420
ctcagacatg tcaaatgcag atgtcacctc ccaaccaact tttagacaca ttggtggagc 9480
cagaaagata taaaagatta tcattatgct aatttttaa atgtacagaa ctgccctttt 9540
taatctttaa ttttggagtt ttagccacaa ctcctttaaa ataaaataat ataataattt 9600
tttttttttt tttgagatgg cgttcgctct tgttgcccag gctggagtgc aatggcgtga 9660
tctcagctca ccacaacctc cgcctcccgg gttcaagcga ttctcctgcc tcagcctccc 9720
tagtagctgg gattacaggc atgtgccacc acacccagct aattttgtat ttttagtaga 9780
ggcggggttt ctccatgttg gtcaggctgg tctcaaactc ccaacctcag gtgatccacc 9840
cacctcggcc tcccagtgtt gggattacag gtgtgagcca ctgcgcccgg cctataattt 9900
tttattatta aatgatcatt agagaataaa taacactgta ggacaaattt tccccttcca 9960
tttttttttt tttttttaga tggagtcttg ctctgtcacc caggctggag tgcagtggta 10020
tcacctcggc tcactgcatc ctctgcctcc tgggttcaag caattctcat gccttggcct 10080
cccagtaggt aggattacag gtgtgcaaca ggcccagcta atttttataa tttagtagag 10140
gcaggatatc actatgttgg ccagactggt cttgaactcc tgacctcaag tgatcaaccc 10200
gcctcagcct tccaaagtgc taggattaca ggcatgagcc actgtgcccg gccccccgc 10260
tttttttttt tttttttttt tttgagagtg tcttcttctg tcgcccaggc tggagtgcat 10320
tgttccatca tggctcactg cagcctcgac ctccaggact caagcaatcc tcccaccgca 10380
gcctccccag tagctgggat tacaggagtg cacaacccct cacccagcta atttttgtat 10440
tttttgtaga gacagggttt tgccatgttg cccaggctgg tcttgaactc ctaggctcag 10500
gtgatccttc tgcctgggcc tcccaaagtg ctgggattac aggcatgagc caccgcacct 10560
ggctaaatcc aaggattttt gtatccacgg gtggatcctg gaaccactgc gccagggata 10620
ccgaggaatg attatacaga aaaaggaaat tttctgtata caaggagaac ccagttggaa 10680
acacctcagc atttgcctta tttgaacaca gtttaaacat ttggctgcct gcaactaact 10740
gaagctcagc tagctattgg ttaaaagaat atactcctaa tttagcctct cagttaattt 10800
atgtgttatg ttaggttaca gttcattaca taaaggccaa agtatggaag cctcctcagg 10860
ccaaatttag tttaatttaa caattcctcc cttttggtcg gtctcaattt tgagagactg 10920
accaaaacct tgacactact ctctgtcacc atcatccgac taaatacgga acttacttag 10980

```
tctctgtata caattcacaa gtcacaacag ttgaacaatt ctttatcttc tggcagatct  11040
agttcaaatg agaccgttca acatttgacg aatgattgta tacaaacatt taagatgtga  11100
gaggatagga tacggtgcac caggaggact attataatgg ctgtcaggag ggttacacca  11160
aaaagactga agtgtactcc ttaacaaaag cttgtaggaa ctgaacccat ccaaatcaaa  11220
gttcaggcaa tatagacagc tcaataatat ttggccatgg tctgtattct ggatttgatg  11280
gcaattaagg actatagttt ggtgtaaaat ggcctgattt aaagaggtat ctgtttttgt  11340
tgttagcctg ataatacagg ctgtagcaat ctagagactt accaagagaa caagatgaga  11400
aaagcttgga aacaccaagc tggccatcca ccgctgagga ggcccgtgga ccaactggtg  11460
gctgctccca taaacacatc atctgttcct tttccttgag aaattctctt ttttattgtt  11520
gttgttacat tggcaatttt tttttttttt tttttttttt ttagctagag tctcgctctg  11580
tcacccaggc tggagtgcag tgacgcgatc ttggctcact gcaagctctg ccttccgggt  11640
taaagtgatt cccgtgcctc agctgcccaa gcagctggga ttacggatgc acaccaccat  11700
gcctggctag tttttgtatt tttagtagag acaaggtttt tgccacgttg gccaggctgg  11760
tctcgaactc ctggtcttaa gtgatcctcc caactcggcc tcccaaagtg ctgggattac  11820
aggcatgagt cactgcactc agccatgtct tgagaaattt tcttagtgta tttggtagca  11880
gtgtctaaag aaacaacagt atcagccacc tttttttttt tttttttgga ggggtggaca  11940
gagtcttgct ctgtcaccca ggctggagtg tgcaatctca gctcactgca acttctgcct  12000
ctggggttca agcaattatc ctgcctcagc ctcttgagta gctgggatta caggtgcacg  12060
ccaccatgcc cagataattt ttgtattttt agtagagacg gggtttcacc atgttggtca  12120
ggctggtctc gaactcctga cgtcatgatc cgcccactca gcctcccaaa gtgctgggat  12180
cacaggcgtg agccactgtg ccctgccttc agccaccttt ttaattaagc tttctgtagt  12240
aggaaaatca aaagaaaaat agatacaata ttccgttttc ttaaaaacca tatacaaggc  12300
tccgacttga gacagaagga gatctaaagc aggatgctct gctaggtgtt tttgttgaac  12360
acatgtatta tcacccactt tttagaaagt ggcttctacc cctctgaaca gcagggaggt  12420
ctggctgact gcaaggtcca aggtgtttcc ccagtttaca gatgagtgtc aaattctata  12480
ctactagtgt cccacaactg ccaagagtga gcctccagaa acctcactgg aatctttcct  12540
cgatgaaact aacttgtctt cataaacccc ataattgctc aggtttttgc taaagcataa  12600
accttagcta aggccattca taaactatgg ttccatggat tttcctggag agtaaaggaa  12660
```

aggattaaga cagcaaaaaa tgaagaaaag aaaaagaatg ctgggtgtgg tggctcacgc 12720
ctgtaatccc agcactttgg gaggccgagg tggatggatc acctgaggtc gggggagtt 12780
cgagaccagc ctgaccaaca tggagaaacc ccctgtctac taaaaaaatt agctgagcat 12840
ggtggtgcac gcctgtaatt ccagctactt gggaggctga ggcaggagaa ttgcttgaat 12900
ccgggaggcg gaggttgcag tgagctgaga tcatgccatt gcactccagc ctgggcaacc 12960
aagattgaaa ctccgtcaaa aaaaaaaaaa aaagaaagag agagagagag agaaggaggg 13020
agggagggag ggagggaggg aagtaaggaa ggaaggatgg aaggaagaaa ggaaggaagg 13080
aagggaagga gggaaggaag gaaggaagga tggaaggaag aaaggaaggg agggagggag 13140
ggaaggaagg gagggagaga gggagggaag gaaggaaggg agggagggag ggaaggaagg 13200
gagggaggga gggagggagg gaataaggct ttcctgatgg tagaggagcc ttgatcttgg 13260
aaaagccgtc caggtctaga acaccatctg cttctgggga gaaattttcc ccagctagct 13320
ttacattaaa gtctctaacg gaggccagga gcagtggctc acacctgtaa tcccagcact 13380
ttgagaggcc aagatgggtg gataacttga gggtcaggtg tttgagacca gcctgaccaa 13440
catggtaaaa ccccactcct ggtatcaatt ttctgtgtta gtttgttctc acactgctat 13500
aaagaactac ttgagactgg gtaatttatg cagaaaatta atttaactaa ctcacagttc 13560
tgcaaactta atgggaagca tgactgtgag gccttgggac actgacaatc agcagaaggt 13620
gaaggggaag caagcacctt cttcacaatg gcggcaggag aaagatatag tgaaggggga 13680
actgccacac acttttattt actttttttt ttaatttatt tttttgagac ggagtttcac 13740
tcttcttgtc caggctagag tgcaatgaca caatctcggc tcaccgcaac ctccgcctcc 13800
ccggttcaag cgattctcct gtgtcagcct cccaagtagc tggggttaca ggcacatgcc 13860
accacaccca gctaattttt gtatttttag tagagacggg gtttcaccat gttggtcagg 13920
ctggtctcga actcctgacc tcaacctccc aaagtgctgg gattacaggc atgagccact 13980
gcactgggcc tgaatttact ttaatttaac agaatgcaat tatgggcaat gagataaaga 14040
ggaaaatgtt ctgacatttc tagaaatttg ctcacaccta tatgaaatct tactttcttt 14100
cattatctca cctgatactc ataaaaactt tgtgcaaggg attttgagga gtactaatat 14160
ttaggagagt tgggcagatg aacaactaac aaaggtaaac aatcgaaaga tccaagaaac 14220
caagtgacgt tattgtttca aagaggcacc gctccacaat ttcaaaggct gctgagaaat 14280
taagaataag aaggtgacct gtgtgtggct gatagatgaa caaaatggaa gctattagtg 14340
agcttggtac ctttcttgag ccgtattgct tattcatatc tcattccatt tgtcttgata 14400

ttctgcccag aagctgggac catgctttgc acatggcaag tgcccatttt atgtctgtta 14460
gtagcagttt tgctgagtga ctgaaaccag agacttagca gagatgggtg tgctcagagt 14520
tggcctccac tctattccca ttacaaagca aaatacaata caaagcaaaa tacaaataca 14580
ataccaatac aaagcaaaat tcccattaca aaaactcatc gccagacagt tctacttaca 14640
ggaatttgtc ctaaagacag ttcccccaaa gcctgagatg ctctacttac aaggatatta 14700
attgcaatgc tatttgtaat agcaaaaggc gggaaaccac ctaaattcca ccaactagag 14760
attgtgcaat aagctgcaaa gcatccagtc agtggggcaa taggatgccc ctttaagaaa 14820
gaacaaggct ggccgggtgt ggcggctcac acctgtaatc ccagcactct gggaggctga 14880
ggcaggcaga tcacctgagg ttcaggggtt caagatcagc tgggccaaca tggtgaaacc 14940
ccatctctaa taaaaataca aagattagcc tgatgttgtg atgcgtgtct gtaatgccag 15000
ctacttggga ggctgagaca tgagaattac ttgaacctgg gaggtggagg gtgcagtgag 15060
ctgagatcat accactgcat tccagcctga gggtgacaca gtgagaatct gtcaaaaaaa 15120
aaaaaaaagg acggaaagaa agagcgaacg aggctgatgc ctctgcactg agatggtctc 15180
cacgatgtat ttgaaaagtg gagagaaaag agggcagaac cttacggagg gaaaaaaaga 15240
caattaaaag aacagctggg ccagatgcag tggctcatgc ccataatctc agcactttgg 15300
gaggccaagg caggtagaca tcttgagctc agaagttcaa gaccagcctg ggcaacatag 15360
taagaccccc tccccccaac catctcaaca aaaaatacaa aaaatagcca agcatagtgg 15420
catgcctgta gtcccaactg cttggaaggc tgagacagga ggattgcttg agcctgattg 15480
aggcttcagt gagccatgat cgcgccactg cactccagcc tgggcaacag agcgagacct 15540
tgtttcaaaa aaaaaaaaaa aaaaaaaaag gaattatttc aatggatctt gcagtattga 15600
ctggatagaa ggctggaagc caataaagca aagagaatac aaatagactg actgttctag 15660
aaattctatg ggtaaaggaa gaagggatta attagaaagc atacatttat ttatgtattt 15720
atttattgag atggagtctc gctctgtcac ccaggctgga gggcagtggc gcaatctcgg 15780
ctcactgaaa cttttgcctc ctaagttcaa gtgattctcc tgccccagcc tcctgagtag 15840
ctggaattac aggcacccac caccctgcct agctaatttt tgtattttta gtagagatgg 15900
ggtttcacca tgttggccag gctggtctcc ggacctcaag tgatccgcct cccaaagtgc 15960
tagaattaca ggtatgagcc accgcgcccg gccagaaagc atacattttt tatttagatt 16020
gtatgcttat ctggcgtggc ttgatacggg ggtgctgatg gggattggga ggtagagaaa 16080

tacccteccca ataataatag ccaaacgcaa catagcactt gtgatatttt ctgtgaattt 16140
tattagtatg aactcatttc aacctcctaa caaccctgtg gggcaggtcc ttttcatacc 16200
cccatttgtg aggacacagt ggcctttcta atatcacaca ccagcaagtg tcaaagcttc 16260
aaacccaggc agcctggtca cagagagtca cataactgct ctgcaaacct gcctccctct 16320
gcttcagagg ggctcagatt cggggtttac ctctcactag ctgggcaagc ttaggcactg 16380
gccttctcag agccttggtt ttctcgccgc agaatgaggg taactgcaga gttgttgaga 16440
agcatttgaa gcacctgatt cttggtgctc aatacaggga acgtgtgata gttactgccg 16500
acttagagca gagcttaggc aggcactgca ccaggatttg actattgatg catctccaag 16560
ctgagcctcg gtaggcctgt ggtcccatgc tagagagcag ctgccagtgc ctccatctgc 16620
tgcaaagctt ggctgtgtgt ggatgggtgg gacaagtcac tccagaagct tctcccctta 16680
agacatcctg acattaaggg atcttcttcc tttgctcctc tttctgtcct ccctgccccc 16740
cttcctccct ccctttcttc cttaaatgcc caaagtcagt tgatacettt tcttaagtaa 16800
ctcaataagc aatacaggca gtttgctcat ggttggcagt gggcaggatt aagggaggat 16860
tagggccctt tgtcaatgtc tcatctcctg tctgcagaga caagacctga aggattgcta 16920
aagaatgccc ttggtaacaa ctgtttctcc aggaccttgt ttggcttccc cctggctggg 16980
tggaacccgc tgtgtgcagc cttcgcaggg aggtgggccg aaggctgcca acgttggagc 17040
aggtgagcta tggcatttgc attgggctgg gtctccattc ttgggccaaa tcaactcacc 17100
tcttgggcga gttgcttcag acttcagaaa caagccacat catgccgaga ggctttcccc 17160
ccagttccag ggcccaaggg tgcaggcaca tagccagccc agccttctct tccctgtgtg 17220
ttcccttctc ccctgggttt ctcctccctt gtgaagaaac agcacacctg cttttacaca 17280
aggccagtcc tccacgggcg ccctcatccc acccctttg accttctcaa ggactacaag 17340
tctctaatga tcctctccct ctcctgactc atccatctct ccctctccct tgtgactcag 17400
cagtgtacag acctgcttct agattaaaac aaaaacagaa acccctccga ccaccccctc 17460
cacctttttt cctgcttttc ctcagagaca aacttctcag cacccctgc ctcctttcct 17520
caattcccgt ccattctcca gcccacttcc agctggcttt ggcacccact gtccctcctc 17580
tcagattcac tcttgtcaag gtcaccagga atacccggtg cacagtacaa caggcacttc 17640
catgtcctcg cgggatgggt cttcccagct gcacttgacg cagctgaccc agggtgctcc 17700
cctgcttccc ctcctggggc acgctcatgg ctttcccgcc tcctccctct gaccctcctg 17760
cctcctccct ctgatcctcc tgcctccttc ctcggaccca gggcgctctc ctccctcctc 17820

tcctggaaca cgctcatggc tttcccacct cctccctggc catctcttct cagtcttcat 17880
ttccaacttt tcctctttct gttctctgaa tgttggcttt ctgcatggct aagccctggg 17940
tccccctcta gctagactct catgggatgg tctcatccat ggccaaggtt tttaacataa 18000
tctagaagct gagactccca tattgcatct ctggtcttgc tgtcaactca catgtccgtg 18060
gcctgctgga tacccctgtt ctgtctcttg ggcattccac acttagtgtt tcctaaatca 18120
gactgtccag ggccttccac attggtttgc tcccagtctc ccttggagcc ggcactggtg 18180
ccacccttcc acctggtgcc tggctgccaa gcttgattcc ctcctcctcc tccctaaacc 18240
ctcccctcct cagacctcac tttttccatc ctcacattgc caccctaggc caggccacca 18300
tcagctcctg cctggacact gtgacagcct cctaagtgat cgacagcccc catttttcac 18360
gctgctgcca ttgagatctt tctagaataa aaatcaaatc aagccagtcc cttgcttaaa 18420
accatttcac gctccctatt gctcttcagg tgaagctaac ctccttaact tggtttacac 18480
agcccttccc agccagcccc tccctgcctc tcccacctcc agtgccaccc ccagacctca 18540
agtgatccac ccacctcagc ctctcaaagt gctgggtggt ggctcacacc tggcgtcgtc 18600
ctcttcctcc cttcctcttc ccctgcccct tccccttgcc ctcctcctcc tcctactcct 18660
ccttcttcct cctccttctt ctatgagtgg tcagttattc caatgataga ttacagaatt 18720
ggcttctgcc tttcaaaaat taaatcataa cccatctcct gaagtttgaa cacagccttg 18780
cctaattttg tttcatttat gttattgtta tcactacagt tatttggggt ggcctgagga 18840
cacggctgga gctggagtgt ggctgtgacc tcctcttgac atctttctgc accactatta 18900
atggtatccc aagtgctggg cacagaggag gagatagatc aatatttgat gaataaatgg 18960
ctgggcgtgg tggctcacgc ctgtaatccc agaactttgg gaggcctagg caggtgaatc 19020
acctgaggtc aggagttcaa gaccagcctg gccaacatag tgaaacccca tctctactaa 19080
aaatgcaaaa attaggccag gcggggtggc tcacgcctgt aatcccaaca ctttaggagg 19140
ctgaggtggg cagatcacct gaggtcagga gttcgagacc agcctgacta acatggagaa 19200
atcccatctc cactaaaaat acaaaattag ccaggcgtag tggcacatgc ctgtagtccc 19260
agctactcag gaggctgagg caggaaaatc gcttgaacct gggaggcgga ggttgcagta 19320
agccgagatt gcgccattgc actccagcct gggcaacaag agcaaaactc cgtctcaaaa 19380
aaaaaaaaaa aattagctgg gcatggtggg gcatgcctgt agtcccagct acttgggagg 19440
ctgaggcagg agaattgctt ggacccagga gacggaggtt gcagtgagcc aagatcacgc 19500

cattgcactc cagcctgggc gacaagagca aaactcagtc tcaaaaaaaa aaaaaaagac 19560
tttgatgaat aaatgaatga acgaatgaaa tcactctcaa accagagctt caagtttcat 19620
gttattggcc tggagcggtt aattttatca gcatacacaa gactctcagt ctccaaatca 19680
tggaactata ggatggcagg atgggagaat ccctggacca cccagggccc aacctccaaa 19740
tttatcagtt gagccagctg agccattgct cacccacagt tgtcccggag caggcaggct 19800
gcctgcagga tggaagaacg tggaggagga agggtgtgca ggggccaggt cttacctgga 19860
tttgttgatt ttattctcaa tatgatgaga agtcccgaga gactttaaca taatccctga 19920
tcttatttta ttttatttta ttttatttta aagatcattg caatccctgc aaaaccagat 19980
tcaggactgg agagagaaaa taaggtatgg gctggggagt caacacacat ttgtgcctct 20040
ttgatggcaa ggcctaggaa cacatgcctg gggtcagctt gccatcatgt ggccaaacct 20100
aaggactgca catctcccat cttgctttcc cagggctccg tgtacttgtg gagacaggat 20160
ttggacccag gtctctcctc ccaccaaagt tcagggtcta taggcctgag ttcaagtccc 20220
ggttcctgca ctcccagcca tatgaccaaa gaggattcag gttctgtatc cgactcatgg 20280
ggacagcaac acactcatct ctcacagtga agacccagta agaaatggca gctgtcagcc 20340
aggtgcagtg gctcacgcct gtaatcccag cactttggga ggccgaggca ggcggatcac 20400
gaggtcagga gatcgagacc atcctggcta acacggtgaa accccgtctc tactaaaaat 20460
acaaaaaatt agccaggcat ggtggcgggc acctgtaatc ctagctactt gggaggctga 20520
ggcaggaaaa tggcatgaac ccaggaggtg gaggttgtag tgagctgaga tcgcaccact 20580
gcactccagc ctggcgacag agtgagactc tgtctcaaaa aaataataaa taaaaggcca 20640
ggcgcagtgg ctcaagcctg taattccagc attttgggag gccgaggcgg gcaggttgcc 20700
tgagctcagg agtttgagac cagcctgggc aatatggtga aaccccatct ctaaaaaaaa 20760
aaaaaaaaaa attagctgag tgtggcggcg tgtgcctgaa gtcccagcta ctcaagaggc 20820
tgaggcagga gaattgcttg aacccgggag gcagagattg cagtgagccg agatcgcacc 20880
actgcactcc agcctgggtg acagagtgag actccgtttc aaacaaacaa acaaacaaaa 20940
aaaatagggg ctgggtacag tggctcacac ttgtaatccc agcactttgg gagaggattg 21000
cttgaggcca ggagtttgag accagtctgg gcatcatggt gagacctcca tttctattaa 21060
aaaaaaaaaa ttataataag taaatgtcta accaccttat cttagggagg aagaaactga 21120
ggctcagaga aaagcaggta gaaagcaaga tttctcgggc caggcacagt ggctcacacc 21180
tgtaatccca gcactctggg aggccaaggc aggcggatca ccggtcggga gtttgagacc 21240

agcctgacca acatggagaa gccccgtgtc tactaaaaac acaaaattag ctgggcatag 21300
tggtgcatgc ctgtagtccc agctacttgg gaggctgtgg caggagaatc acttgaaccc 21360
gggaggcgga ggttgtggtg agctgagatt gtgccattgt actccagcct gggcaacaac 21420
agcgaaaccc cgtctcaaaa aaataataat aataaaagaa agaaagcaag aattctcaag 21480
gctctgttca aaatgcagga cttccactcc ccaggtcagc agtgagccca aggctctagc 21540
tccagcggaa ggctggacag tgttagggcc ggaggtgcca ggcaggcacc ccagaatgag 21600
atgcagaaac ggccagtgag gacagaagcc atggtgcttt ggaagcccag ctccttcttc 21660
ctgctgatga acaggcatcc agagagcctg tgagcggggc tgtgtgctcc aggtcatgtc 21720
ccaggtgtac agaatttcag gaatttagtg gagctctcgc ccagcaattt tttgctttac 21780
aggagaggaa gctgagatcc tgaaagaggt ggggcccggc ccagggtcac tcagcaattt 21840
tcagtagctg ctccctactg ggaactgtg tgtctgaggt cgtggccatc tgtccaaaga 21900
gcttcttctg accaaagtgc tcattttcat agtgtgacca cagaggtatg tattttataa 21960
ccttacctca tttcatcatc ccagcagcct tgcagggagg tttatttcca ttttgcaaat 22020
gaggactctg aggctcaggg aggtgacgtg acatgcgcaa ggctgggagg ggtgggtgag 22080
gacttgccct gtctccactg gcctccagac cctgcgctca cagctggtcc cgtgtggcat 22140
tacaagaaca tttgagcttc tcaggagggt taataaaggg tgtaagaaag gacagatctt 22200
taggagggcc gtcttcagag tttacttctc tgtgttctag agagaaccaa cgaccctcat 22260
gcattgcttt ttggcaaggg aaatgaagga ttcaggacag tgggcactgg gcacccacag 22320
ggtttattca agcagccgaa gctcctgctt cagaaccttg gcacggttag ggcccaaata 22380
agggtctttt ccttctcact ttgaacacag cctcgcctaa ttttgtttca tttatattat 22440
tgttatcact gcagttattt ggggtggcca cccccatccc acagctgctc ccacccttct 22500
ctgctgtaga aatggaggag ggaaggccca gtggctccag tgtgagggga agagacctga 22560
ctgcacctgg gctgtgtctt ctccgtccac agtacacccg tgggaagagg agtcggggca 22620
tcccttgctg gaaatacccc agccctgaag gttgctggca aaaccaccat ttaattcaga 22680
acaaaaagtc tgctccagga gcccctgaga gcgtcaaaaa gaacaaggtg ggtgggttgg 22740
ggggaaacat cactgagagg ctacctaggc ctggatcccc ggcagatgca ggtccacaac 22800
ccgacgccga ccactggccg gccctcccga gtgtctgact gcgggactac ctggcatcca 22860
ctcgaagaga ccagcctggg gcatccctcc ctgcgcccca acccctgtgg acactccctc 22920

gggagcattt ctaactaggc ctgggtatgt gaaaaaagag gaatcctttc gcagaaggca 22980
ggctggacct gggaaaacag ttctccatgg atggagaagt gaggggataa aggatgatga 23040
acttagctgt tttctttcct gtccacattg tttctaggtt ctttggaacc aggggtcatc 23100
ttttatctcc cacagtaact catgtctgcc ttagacttat ttccaacccc tcaagatcag 23160
ttgaagatct ttgtaggatc tacacattca tactttactt actgacaccc cacaacctgt 23220
aaaccataac aaaatgcacc tgctgcacac atctgaaagg ttttcttttt ttttgttttg 23280
ttttgtttga gacagaatct tgctctattg cccaggctgg agtgcaatgg cgtgatctca 23340
gctcactgca acctctgcct cccgggttca agtgattttc ccccctcagc ctcccaagta 23400
gctaggatta caggcatgca ccaccatgcc cagctaattt ttgtattttt agtagagaca 23460
gggtttcacc atgttggccg ggctggtgtc gaactcctga cctcaggtga tctgcccgcc 23520
ttggcctccc aaagtgcttg gattacaagc gtgagtacca tgcccagcca acattttctt 23580
tgctataaaa tgttttaaaa tgtataactg agttttgcca gctgaatata gttcaatttg 23640
ttcttgtgat tcaaacttta ttatgttttt atataaagct atatcaagaa tccaaaaaca 23700
ttctatacat ttggaagttt aaagacaagc ctctaaatag gtcacaatca aagaggaaat 23760
cacaatgtaa atgggaaaat acctagaact gaaggacaat gaaaacccta cctagttaaa 23820
tgcgtgggag gcaggtaagg ttgtacctaa gagaaaatat atggccttaa ataaatgcac 23880
atatataatc aggaagaaag accaaaaaat aatgagttaa agtttcaact caacaaatta 23940
gaaaaggaca ccagagtaaa tccaaagaaa atgaaagaga atgataaaaa taatagtaga 24000
aattaattag aatgcactca gagtatcaac aaaaccagaa gttggctctt tgaaaatgaa 24060
gacaaataac taagactaat aactaagaca aataactaag atattattta tgaaaaaaaa 24120
gaaaattata cattttatga atggaaacag aggcactaca gacatggtag agattaaaaa 24180
ataaaaggac aagccaagca cactggcatg tgcctctaat cccagctact tgggagactg 24240
agacaggagg atcacttgag actaggagtt tgaggctgca gtgagctgtg atcatgtcac 24300
tgcactccag tgtgggtgac agagggcgac cctgtctctg aaaagtaaat acacagagat 24360
aagagaacac tttgaagcag ctatatacca atgaatttga aaatttagaa gaaaacgaca 24420
atttcctggg aaaacacaac ctaccaaaat cacttttgag gactgcagta cacctacatt 24480
cattatattg aatcagtagt tttacatgca gccaagagac tctacaaaac ctctgtgatt 24540
ttgcaagaaa gtcctaacaa ataaaaaaca gataatcctt gtattgttcc agagaataaa 24600
aaatgagaga aaactccctg tgctagtctg ttttcacact gctataaaca acctctctgg 24660

ccgggcgcgg tggctcacgc ctgtaatcct agcactttgg gaggctgagg caagcggatc 24720
acctgaggtc gggagtttga gaccagcctg accaacatgg agaaaccctg tctgtactaa 24780
aaatacaaaa ttagccagac atggtggtcc atgcctgtaa tcccagctac tcgggaggct 24840
gaggcaggag aatcacttga acccaggagg cggagattgc agtgagccaa gatcacgcca 24900
ttgcactcca gcctgggcaa cagagcaaaa ttccgtctca ataaaaaaaa aaaagaactt 24960
ccctgagact ggataattta taaaggaaag agatttaact gactcacagt tccacatggc 25020
ttgggatgcc tcaggaaact tacaaacatg gcagaagggg aagcagacac atcctacatg 25080
gcagaaggcg agagagagcg tgtgtgaagg aggaactgtc aaacacttag aaaaccatca 25140
gatctcctga gaacagcatg ggggaagcca cagccatgat ccaatttcct cctcccctga 25200
cacgtgggga ttacaagttt ctcccttccc atgtggggat tacaatttga gatgagattt 25260
ggatggggac acaaagtcaa accatttcac tccccatctc atttgttttt cttttgagac 25320
tgagtgtcac tctgtcaccc aggctggagt gcagtggcgt gatcttggct cactgcaacc 25380
tctgccttct ggtgtcaagc aattctcctg ctttagtctc ccaagtagct gggattacag 25440
gcatatgcca ccacatctgg ctaatttttg tatttttagt agagatgggg cttcaccatg 25500
ttggtcaggc tggtctccaa cttctgggct catgtaaccc acccacctta gcctcccaaa 25560
gtgctgggat tacaggtgtg agccacctca cccagcaccc atctcattta gtgaggctag 25620
attcatctta gtacaatacc agagaggaga gaaacaaaca ccatggccag tattgcttag 25680
gaacacagtt caaaaatact acgtaaagta tcattaagcc aaatggaact aaatatggag 25740
aacatatatt ttgtgtgccc tctgtggaag tccctgggac agggtggggc agcaacctgg 25800
cagggagctg gtgctcatat tgtcagggtg cctgggagat ccaatggccc agtagttgag 25860
gagatttgct gcacacaggg tgacggagaa aatgagtcaa tatatggagg atttcaggga 25920
tccaagtttc ttactttctt tttttttttt aagagacaga gtctcactct gttgcccagg 25980
ctagagtgta gtggcgcgat ctcagctcac tgcaagctct gtctcctgag ttcacgccat 26040
tctcctgcct cagcctcctg agttgctggg actacaggtg cccaccacca cgcctggcta 26100
attttttttt tttttgtat ttttagtaaa gacggggttt caccgtgtta gccaggatgg 26160
tctcgatctc ctgacctcgt gatctgcccg ccttggcctc ccaaagtgct gggattacag 26220
gcataagcca ccacatccgg ctcaagtttc ttattttcag agaaaaaaaa agtccaatta 26280
tggaaggagg aaatgctaga atgaatcctg ggagctttt tggaatggga agaattaata 26340

tgaatgtgtt tttccatata aatatataaa acaatcaatg taaatgtgta cacacacaca 26400
cccataccccc agtagtaatg agcacactta gtagtcagat ctttgtttct aaacaccacc 26460
ttccactaaa agaaaccaga cctttgagag gtcaaggcgg gataatccct tgaagctaga 26520
aatttgagac cagcctgacc aacatggtga aaccccgtct ctaccaaaaa tacaaaaaaa 26580
ttagctgggc aaggtggcgg gcgcctgtaa tctcagctac tcaggaggct gaggcaggag 26640
aattgcttga acccgggagg tggaggttgc agtgagccga gatcgcacca ctgcactcca 26700
gcctgggcaa cagaacaaga ctctgtctca aaaaaaaaaa aaagaaaaaa gccactaggt 26760
tttgggtaat ttgtttacac aatagataac taatgtgcgt ggaaatacaa gtgaccaagg 26820
aattgcttat tgctttttgt atgatcttca tctctcccac tgctcattcc gcatcatccc 26880
ccgactttgg ccatctgcac cccagttgcc ctcaaccaaa gtcaagctcc actctctaag 26940
ctgggcttgt actgtcgggg ttcccttgct gacggctctc ctcatgagac tctgattttc 27000
cccaagacag accttccccc tccttttcct ttatatgttg agcagcaggg tccctagtgt 27060
tgggcaaaga ccctggcaga cggtgggcgg ttaggaattg tcagtgggag gagttatacc 27120
cagtcataat tgaggaccca gtgatggttg gatttatgat gactgccagg caccgtgctg 27180
agttctgcaa accctaaatt ctagaccagg ggttctcaaa gtgcactccc cacccgagca 27240
gcatcagtat cccaaaattc ctgggcctca ccccagaccc actgaatcag aaactctaag 27300
ggttgatcct agcaatccat ggcttaacaa tttctctggg tggttctggt ccacatttac 27360
atttgagagc caccgttttg gagtttgaga agacaatgaa ttagggcaaa tgggtcaatt 27420
cccagcagag agaagcaaga atcttttctg aggcccacac tgaattccag ctctttcctg 27480
ggctgatgtt accattgtcc ttcatggggt gggtccacat ttccagtcgc ccacctgggg 27540
agctggtgca gcagagaatg tgcttaaggc tcaaatccca gctttttttcc tgttttaaat 27600
ctcggcttta tttactttta tttctccaag tgggacttca tgtgacttat gggggagcta 27660
ttttgtttgt ttgtttgttt gtttgttttt tgagacggag tctcgctctg tcgcccaggc 27720
tggagtgcag tggtgcaatc tcggttcact gcaagctccg cctcctgggt tcatgccatt 27780
ctcctgcctc agcctcccga gtagctggga ctacaggcgc ctgcaccaca cctggctaat 27840
tttttgtat ttttagtaga gacagggttt caccgtgtta gccaggatgg tctcgatctc 27900
ctgacctggt gatccgcctg cgttggcctc ccaaagtgct gggattacag gcgtgagcca 27960
cggcgcccgg cctgaataag taaatttaaa aaaaaagaaa aaaaaaagag gccgggcgcg 28020
gcggctcacg cctgtaatcc cagcactttg ggaggcgtag gcaagtggat cacgaggtca 28080

ggaattcaag atccgcctgg ccaagatggt gaaaccccgt ctctactaaa aatacaaaaa 28140
attagccagg cgtcgtcatg ggctcctgta atcccagcta ctagggaggc tcaggcagag 28200
aactgcttga acccgggagc tggaggttgc agtcagccga gatcgcgcca ctgcactcca 28260
gcctggcgac agagggagag actctgtctc aaaaaaaaaa aaaaaaaaag agccaggtgt 28320
tggtggctca ggcctgtaat cccagcactt gggaggccga ggtgggtgga tcacctgatg 28380
tcagaagttc aagaccaacc tggtcaacat ggtaaaactc tgtctctact aataatacaa 28440
aaattagctg ggcatagtag tgaacacctg taatcccagt tacttgggag gctgaggcag 28500
gagaatcact tggacccagg aggtggaggt cacaatgagc cgagatcact ccattgcact 28560
ccagcctggg tgacagagag agactctgcc tcaaaaaatt aaataaataa ataaatatct 28620
tatctagagg cagggctctg tgtgtgtgtg tgtgtgtgtg tgtgtgtgtg tgtgagaagg 28680
aggaagggac ataggccagg cacgatggct tacgcctgta atcccagcac tttgggagtc 28740
caaggcagac ggatcacctg aggtcaggag ttcaagatca gcctggccaa catggtgaaa 28800
ccctgtctcc actaaaaaat tagctgagtg tggtagcatg tgcctgtaat cgcagctact 28860
cgggaggctg ggcaggagaa tcacttgaac ctgggaagca gaggttgcag tgtgtgagat 28920
ggggccattg cactccagcc tgggcgacag agcaaggctc tgtctgaaaa aaaaaaaaaa 28980
aaaaaaaaaa aaaaaaggag tggggggcgg gggacagaga gaggcaggct actttataga 29040
attccctttc tctgtcaggt gtttggagct catttttcat tttattttat ttacttattt 29100
atttattttt gagatggagt cttgctcttg ttgctcaggc tggagtacaa tgatgcgatc 29160
tcagctcact ggaacttcca cctcctgggt tcaagtgatt ctcctgctcc agcctctgga 29220
gtggctggga ttacagacgt gcgccgccac ccccggctaa ttgttttgta tttttggtag 29280
agatggggtt tcaccatgtt ggccaggctg gtctcaaact cctgacatca ggtgattcac 29340
ccacctcagc ctcccaaagt gctgggatta caggtgtgag ccactgtgcc caggctgttt 29400
tttattttat tttattatta ttgttttttt gagaaggagt tttgttcctg tggcatgatc 29460
tcagctcact gcaatctccg cctcccgggt tcaagtgatt ctcttgcctc agcctactga 29520
gtaactggga ttacaggcat gtgccaccac gcctagctaa ttgtttgtat ttttagtaga 29580
gatggggttt catcttgttg gccaggctgg ccttgaactc ctcacctcag gtgatccgcc 29640
tcggcctccc aaagtgcagg gattacaggt gtgagccact gcgcccggct ggtctttaga 29700
gcttattttc aagcccatca caaagtccac tcctcctcac caccccatga actttctcgt 29760

tgttcttgct gctatctaga gtgccattcc tgtctttctc cttctcctag aagttttatg 29820
caatccatag aacaatgcag aagtgacggg gggttggggg aggattagga ggaggaggag 29880
gaggaggagg aagggagaat gaactgctcc cagttctctg gcatgtatcc tgccccttgc 29940
ttcatgctca tgaaaaacat tttatagcat atttacagct gcttagatgt aggtgcaatg 30000
tatccatgga aataatcctt gcaggatata caatttattg tgtaagttaa ggaataaaat 30060
gtcagaagga tgaattatgt gcccagtgac atatagcaac ggcatggctg agattagcct 30120
ggttatccca atcactcctg ctctttccca aaaaaatgag tgaggaatgt gccccagctc 30180
ctccatgtct aacctccgtg ctgagatgtg ctgagcacct actatgtgcc aggcaccgca 30240
ctgagctctt tatacaatta gatctcattg tttcaccacc acattgcaag gcagttactg 30300
tatcatcctg attctataaa tggaaacagt aaagctcaga gagatacttt aataactcaa 30360
ccattgttac ccagcctggg tgtggcaggg aagcgatctg aacccaggta acctagcttc 30420
gagactgtgg gcttcaccac tgctttctat tggcaacctt cagatagttt ctttctcttg 30480
aatatttagt atattcgttt ctattgctgc ttcaacaaat aagcacaaac aacacagatt 30540
tattctctta catttctaga aggcagaggt ttaaaatgga ttggtgggac cgggcgcctg 30600
taatcccagc actttgggag gctgaggcgg gcggatcacg aggtcaggag atggagacca 30660
tcctggccaa catggtgaaa cccagtctct actaaaataa aaaacttagc tgggcatgat 30720
ggcacgcgcc tgtagtccca gctacttggg aggctgaggc aggggaattg cttgaacctg 30780
ggaggcggag gttacagtga gctgagatca tgccactgca ctccagccta gcaacagagc 30840
aagactccat ctcaaaagaa aaaaaaaaag gattggtggg actatattcc ttctggggtg 30900
ttagagcagg atccatttcc ttgccatttc cagctttttt ttttgtattt ttagtagaga 30960
cggggtttta ccttgttagc caggatggtc tggatctcct gacctcgtga tccgcccgcc 31020
tcagcctccc aaagtgctgg gattacaggc gtgagccacc atgcccggcc ccatttccag 31080
cttttaaaag ccgcctgcat tccttggctc ctggtggtca cctccatctt tccagacatc 31140
actccagcct ctgcttccat cttcagctct cctcctctga ctcagatcct cctgcctctc 31200
ccttctggaa ggcgccctgt gattaccttg ggcctgccca gataatctgg gataatcttt 31260
ctatctcaag atccttaatt tactcctatc tgtaagatcc cttcgccatg ttaggtaata 31320
tatccatagg ttgcagggat ttggatgtgg acagcttttg gggctatta ttggttctac 31380
cacatttggc tgtttctatt ttgtagacta ataacaaaaa acatttttga atgccttcat 31440
agactttaat tccattagct taattccacc tgaaaaacct gttgatcgag caaaactagg 31500

```
ttatttggac ttcctgcagt aaaggagaac accgtcaaca gattcaaatt agggaaatta 31560
gggaatggta tttatgggtt tttagggccc tggctgggtg atgttaaagc aagtcttgca 31620
aggcaggaaa ctggttgcga ttggggagtt tctgacataa gagctttgga atggtgggcc 31680
caggaagaca gggttttgaa ctcacatctg atgcataatc ttatcttcca gagcaggtat 31740
ttttttggag caagaagtga agtgactttt gcctgtcttg atattgctta acccaaggat 31800
tatgttggtt ttagcccttg ctaattacaa taatagtatt tttttgagac tgagtctcac 31860
tctgtcacca cgctggagtg cagtggtgcg atctcggctc actgtaacct ccgactccct 31920
ggttccagtg attctcctgc ctcagcctcc caagtagcta ggattacagg cacatgccac 31980
catgcccagc taattttttg tatttttagt agagatgggg tttcaccatg ttggacagga 32040
tggtctcgat ctcctgacct tgtgatgcac ctgctttggc ctaccaaaat gctgggatta 32100
caagcgtgag ccaccatgcc aggccataat ttccattttt tagagcacct cctacaggcc 32160
aggcaatttt gaatacattt tgacaattac aacaacccta caaggcaggt atttcaactt 32220
aatctcagta tggcgcaata actcccctct tggattttgg agtcaaaaag tcttggataa 32280
aaatccgggc tcctccacct acctgccagg tgaataggca ggtaagttct ctgggtgagt 32340
ctccatgtct tcatctgtaa aatcaggata atgcttccca cttcgagtgt gtaggagtgt 32400
gcacactgtg tgcccacatg agtaagggga ccatgcttcc ctgtcattgt ttttgttttg 32460
ttttgcttta agacagagtc ttgctctatc gccagactgg agtgcagtgg cgcaatctca 32520
gctcactgca acctctgcct ctcaggttca agcgattctc ctgccttagc ctgtctagtg 32580
gctgggatta caggtgcatg ccaccaggcc cagttaattt tttattttta ttttttttga 32640
gacagagttt cgcgctgttg ccaggttgga gtgcagtggt gcgaacttgg ctcactgcaa 32700
cctcggcctc ccgagttcaa gtgattctcc tgcctcagcc tcccgagtag ttgggactac 32760
aggcacacgc catcacgccc agctaatttt tgtattttta gtagagatgg ggtttcacca 32820
tgttggccag gatggtctcg agctcttgac ctcatgatct gcctgcctca gcctcctaaa 32880
gtgctgagat tacaggcatg agcgactgca cgaggccatt tttttttttt tttgtatctt 32940
tactagagac ggggtttcac catgttggcc aggctggtct tgaactcctg ggctcaagtg 33000
atccacctgc ctcagcctcc caaattgctg ggattacagg tgtgaaccag tgcacctggc 33060
cccctgtcat tgttatcatt gttagtagtg tcctttttta tttttatttt ttttgaggca 33120
gaatcttgct ctgttgctta ggctggagta cagtggtgca atctcggttt actgcaaact 33180
```

ccgccgccca gggttcaagc gattctcccg cctcagcctc ccgagtagct aggataacag 33240
acgcatgcca ccacacccgg ctaatttttg tatttttagt agagacaggg tttcagcatc 33300
ttggccaggc tggtcttgaa ctcttgacct cgtgatccaa ccccccttggc ctcccaaagt 33360
gctgggatta caggcgggaa ccaccacgtc cgaccatgtc cttttattat tccccaaaga 33420
gtccttcctt agatgatgaa ttatatgggc acagttccaa tatggtgaca ctatgcttat 33480
tacttttact cagtaggtga aaggggatgg atcagtgagc ttaggtgaca tgcttagggc 33540
tgcacggcta attgggaaga attctccaag ctaaagctgc atagcagacc aggtgctatc 33600
cttctgcctg gaacaactca acacaatgcc agactttgta actatgtatt tgtgtgattt 33660
gtttcgattt tgatgttttt aattgagaca gaatcttgct ctgttgccca ggctggagtg 33720
cagtgattgg ttcactgcag cctcaacttc ctaggctcaa gcaatcctac tcctcaagcc 33780
tcccaagtaa ttggaactac aggcatgcat caccatgccc agctaatgtt tgtatttttt 33840
gtagagacag ggttttacca tgttgcttag gctggtctca actcctggcc tcaagcaatc 33900
cacccacctc agcgtcccaa agtgctgtga tcacagggca aagccactgt gcccggccca 33960
tttatgttat ttttaaatga aacctgtctt ccaccaggct cagtggctca cgcctgcaat 34020
ctcagcactt tgggaggctg aggtggtcag gagttcaaga ccagcttggc caacatgttg 34080
aaaccccatc tctactaaaa atacaaaaat tagctgggcg tggtggcgtg cacctgtagt 34140
cccagctact caggaggttg aggcaggaga attgcttgaa cccgggaggc agaagttgca 34200
gtgagccatc acgccactgc actccagcct ggtgacagag ctagactctg tctcaaaaaa 34260
aaaaaaaaaa aaattggtga ggaagagatc attcatcatt gtcactgtat ttgataaaca 34320
tccatctccc acagctgggc agacataaga gcggggagaa attctggcca tgcactgggc 34380
gctctcaggt atggtttata tgcctggcta gggcctggct ccaaatgtcc cccagcaaac 34440
aggtttgttt tttttttttg taattaccat actttgggct tggccctcac aagattatct 34500
cccttgaatt agataggatt tttttggaca cgatgaaaaa ccgtgcctcc tcttaacagt 34560
tactatatta taatgctatg ccttggtctt tactgcgggg gtctcctagt tccaggagga 34620
tattaaccca cacatatttg tgtatttaga tgaaggtaat atatggtcct ggtattaaaa 34680
cttcaatcac tatagaagag tttttaacaa gatttaaaat atgtgcagac cccatgactc 34740
agcagtttga actccaggaa cctcatcttt aaaaacatgc aaccagcagg tgtgcagagg 34800
atacttcttt tatctctctt tgtaaaagca ggaaaaactg gaataaccta agggtctatt 34860
gaggagagag gggttgctga cttacacatc agggtctatc catacaatgg aatgcctctc 34920

aggtgtcaaa aataaggagg ctgagctgaa gatggtatca aggatttggc attagagaac 34980
aaagcaaaca ttaacattat gtatgcttgc acagagatag atactcatat aactgcctag 35040
acaagggcct aaaaatgcaa agtacttaaa tctgttacct ctttggcatt ttaaaaagat 35100
tgtatttaga taatattgag gtcattttag aagacacaca cttactgcat taaaacaagc 35160
aaaagttgaa caaaaaggta ataacgttaa gcaggttaca attccattgt aagtaaatgg 35220
tggtgttaat agttcaagtg cttccttggt cactttaaga ttgtttttcc agagctgggc 35280
agcaggagaa ataagttaaa atttcattcc gacatccccc tgccaaaaaa tagcctgaaa 35340
attcagcctc agctctggtc tgaatccggt gacaggactg actaccagga gatctctcag 35400
cctctgcgct gctgactttg gggtctgagt cattcttggt tctgggaggc tgccctgtgc 35460
attgcacggt atacagaagc ttccttggcc tctacccatt cgatacctgt atcacccccct 35520
cccagttctg acgaccaaaa atgtcttcag acccagatat cccctgaagt gcaaattgcc 35580
catcccatcc ccacctgtgg ttgagaatca ctggagtagg atgatggacg tcagggtttc 35640
ctgggatcca cctggttggt gtctcatgaa ctgaggaaat tactaactgc gccccctttc 35700
cttctgtaat aaaccacatg gtcactctgg ctccggccct ttttccctag aaaaatgtac 35760
ccatgcttgt ggcatcataa tccccatgtc tgtcattctc gtcaacactt ctcatggttt 35820
ttgtcacgcg cgtccgtgtg aagagaccac caaacacgct ttgtgtgagc aataaaactt 35880
tttaatcacc tgggtgcagg cgggctgagt cttgtatatg tgcaggtcac acaggggata 35940
agatgactta gcttgggctc agaggcctga cagttttggt cgtagccatc ttcgtggatg 36000
catgatatac ccttttgaaa cttcttccag aacttttttt ctttttcttt tttttttgga 36060
gacggactct tgctcggtca accaggctag agtgcagtgg catagtcttg gctcactgca 36120
acctccgcct cctgcgttca aacgattctc ctgcctcagc ctcccgagta gctgggacgg 36180
ctgacacaca ccaccacgcc cagctaattt tcgtagattt tgtagagaca gggtttcacc 36240
atgttgccca ggcttgtctg gaacttctgg gatcaagcaa tcctcccacc ttggcctccc 36300
gaagtgctgg gattacaggt gagagcaact gcgcccggcc ttcttgtctt agatttgttt 36360
tactcaaagc cctgtggctt acctttatat gaagttccaa agaaggacat tttaacactc 36420
ctgtacttaa atcactacag aagcatgttt gggtgaggaa actctatgaa cttggtttct 36480
aaagaaggcc ttccagccac gggcacgcta cacaatgtgg ggccatttat tttgcaaatc 36540
agtaagactt caaaatttaa aaaataatag tataaaaatg aggatggacc gggcgtgatg 36600

gctcatgcct gtaatcccag tactttggga ggctgaggtg ggtggatcac ccgaggtcag 36660
gacttcgaga ccagcctggc catcatggtg aaaccctgtt tctattaaaa atacaaaaag 36720
agcttgaacc tgggaggcag aggttgcagt gagtggagat cgtgccactg cactccaacc 36780
tgggcgacag agtgagtctc cgtctcaaaa aaataaaaaa taaaagaaaa tttaaaaata 36840
aaaaataagt taaaatttgg atgaattgct gggggaatgg agaaatcaat ttctattttt 36900
ggtccttctg ccctgttttt gtttgtcttc tgggccatgc agatgtcttg tttttataat 36960
taaaaaacta ggcggggtgc agtggctcac atctggaatc ccagcactta gggaggctga 37020
ggcaggagga ttccttgagc acaggaattc aagaccagcc tgggcaacat agccagatcc 37080
catctctaca aaaaatttta aaagttagcc aggtatggta atgcacacct atggtcccag 37140
ctactgggga ggctgaggca ggaggattgc ttgagtctgg gagatcgagg ctgcagtgag 37200
ctacgatacc agcatggccc tttagcctgg gtgacaaaga gagaccctgt ctcaaaacaa 37260
aaacaaaaca aagtatttta ttatacgtaa gtccaaacac attagcacag gccctgaaga 37320
acagcagaaa ttgcacctaa atctccttct ccccatgtac atatccaacc ccaccccttg 37380
ttcccatttg tgctcagctg gggtcaaggt ctcaccatca gagctggctt ccgaaagggc 37440
tcctttccct acaggtttcc cccactggac tttacctcca gatgcccagg acatgggaca 37500
tcatgcaaag acctagcacg tggtgagcat ttagaaaata tttacagaat tattcacagt 37560
atttgttgag cacctactgc ttattcatat cagttatgat ctgctgtgca ctgctgagca 37620
ctggcagaaa gagaagaccc tgagcatctt aaattcacgg cagttgccag ctggagttcc 37680
catctgaatt tcctggagga aggcagaccc catcgcgact ttctttcttt cttttttttt 37740
tctccttgag gctgagtctc gctctgtcgc ccaggctgga gtgcagtggt gctatctcag 37800
ctcactgcaa ctgccacctc ccaggttcaa gcaattctcc tgcctcagcc tccagagtag 37860
ctgggactac aggcgtgtgc caccacgcct ggctaatttt tgtattttta gtagagacgg 37920
ggtttcgcta tgttggccag gctggtctcg aactcctgac ctcaggtgat tcaccagccc 37980
cagcctccca aagtggtggg attacagcca taagccactg cgcctggcct cttcattttt 38040
ttgagaccaa gtctttctgt tgcccaggct ggagtgcagt ggcacgatct tggctcactg 38100
cagcctccac cttctgagtt caagcgattc tcctgcattg gcctcctgag tagctgggac 38160
tacagatgcc tgtcaccacg cccagctaat ttttgtattt ttagtagaaa cgaggtttcg 38220
ccatgttggc caggctggtc ttaaactcct gacctcaagt gatccaccgg cctcggcctc 38280
ctaaagtgct gagattacag gcatgagcca ctgtgcccat cccccaccgg gactttctaa 38340

ttcaggaatt ctagagtggg accctgtaat ttgcatttct ttttaaaatt attttttgtt 38400
cttttcttcc ccgcccaccc caccccgcgc cccccaccca cccagctttt cctgactgga 38460
gaagaacggg gacagaaggc ctgatttgca tttctactat ttcccaggag atgttgacgt 38520
tgcaacaaag agaaccactc aaagagaata gctctgtgag aaccaatgtt ctagagttta 38580
ggaagaccat caattggggt aaatgagcca attcccagga gaaaagggga aggacctttt 38640
ctgaggcatg atctgaattt ccagggctga tgttgccctt tgttatttcc acaggggatc 38700
tgcgttttca atcgctcacc tgcagagccg ccagattctg gccaatctcg gctttttttcc 38760
ctctcactgt gggttcttca gagccccatg gtttagacac aacggtggat tctcttgtgc 38820
cccaattacc acatgcgtca tggggagaag agcttgaacc ccccagaggg tcaggtccag 38880
gtgagaattg agggtgacac tgagagaatt tagctaggtg ggcgctgtgt accgcatcat 38940
tgcctgctcc tttcgcagtt ttgcatttgt gtgttgtaca gctacgtgtc tcaacctaga 39000
aggatctcca ggatgtatcc ttgggtggaa aaatttaggt actagaatct aggccgggcg 39060
cggtagctca cgcctgtaat cccagcactt tgagaggctg aggtgagtgg atcatgaggt 39120
caggagatcg agaccatcct ggctaacacg gtgaaacccc atctgtacta aaaatacgaa 39180
aattagctgg gcacagtggc atgcactcgt agtcctagct actcgggagg ctgagccagg 39240
agaatcgctt gtacccggga ggcggaggtt gcagtgagcc gagattgcgc cattgcactc 39300
cagcctgggc gacagagact cctccatctc aaaaaaaaaa aaaaaaaatt agccgggtgt 39360
ggtggtgggc gcctgtaatc ccagctgcta ctcgggaggc tgaggcagga gaatcgcttg 39420
aacccaggag gtggaggttg cagtaagcca ggattgcacc actgctctcc agcctggatg 39480
acagagcaag actgtctcaa aaaaataaat aaataaataa ataaataaat ataaaaatta 39540
aaaaaatttt aaaaaagaaa tctatattga gttttgtacc tacctatgat tcatggatac 39600
ctaccaggga aaaagcagaa taaaaacatg cacagaagga aatattccaa attgtgcttg 39660
cctgggtggt aacttgctat aatgttttac ttctgtaatg ggagggagta cacacgtggc 39720
tattctatta ttcctcccat gttttcctat ctgaaatatt tcatatttta aatgtttatg 39780
taataaattt tgtcaacctc tttcttcaag tcttggtgtt aaaatcatgt gtattactaa 39840
atcttttttt tttttttaaa tttaagaggc aggatctcac cctgtcaccc aggctagagt 39900
acagtggtgt gatcatagct cagtgcagct tcaacctttt ggactcaaat aattctccca 39960
cctctgcctt cccagtagct gggagtacac gtgtgagcca tcatggctgg ctaattattt 40020

ttatttcttg tagagatagg atctcaccac gttgcccagg ctgctctcaa actcctgagc 40080
tcaagcaatc ctcctgcctt ggccacccaa agcgctgcga ttacaggcat gagccactgc 40140
acctggcctt aaacactttt tcttcctaat atatttttac accttcagct gtttgctata 40200
tctaacggcc agccctccat atccatgggg tcctcattca tggattcaag caactgcagg 40260
tggaaattat tagaaataaa aaaggatggt tgtgtttgta ctgaacatgg acagtttttg 40320
ttattattct ctaaacaata cagtataaca actatttaca tagcatttac attgtattag 40380
gtattaaagc taaagactgc acagcaggtc ctcaaataac tttttttttt tttgatggag 40440
tctttttttt tgatggagtc ttgctctgtc acccaagctg gagtgcagtg gtgtgatctt 40500
ggctcactgc aacctccgcc tgctgggttc aagcgattct cctgcctcag cctcccaagt 40560
agttgggatt acaggtgtcc accatcatgc ccagctaatt attgtatttt tattagtgac 40620
gggatttcac catgttggcc aggctggtct ggaactcctg acctcaggtg atccgcccgc 40680
ctcagcctct taaagtgctg ggattgcagg catgagccac tgtgcctggc caacattctt 40740
tcatttaaca ttgtttcatt atatcattga tgagaaaaca aattggtttt gtttatacat 40800
cctttccctt taagtcaaag tttctaagaa tcttaactgt atacagaagg atgtatggtt 40860
ggttacatgc aaattctcca tcattttatg caagggactt gagcatccat ggattttggt 40920
gcctgaggag agtcctggaa ccaatccccc atggatactg agggacaact gtaatctttt 40980
ttggaataag gaattaaaaa tttccccaaa atgcttctcc ttaatgtcat ttattgaata 41040
acttgttatt tctccactga tgggaaataa taccttttac catttaccaa acccccatat 41100
atatgtggat ggaattctaa acaccttatt ctattttatt aagttttctg attaccctat 41160
accagtacca gatcacttaa ttaaaattgg tattattatc aagaggacac gtgcttctgg 41220
taaatgatgt tgagacaaca cctagagagt ttaatttccc acctctccta accgcacttc 41280
ccagagacaa agctctgggt tagggttcgc agcaggatta aggagcaaga gcccagctgg 41340
ggtgctgggg agcatgcagg gccttaggac cagcgaaggg aggttgcaat tatttcccca 41400
gggttctggg tggtccctgt tcattagcta gaaatgatgc acctttgggg ctagcaatgc 41460
gctgtggttg ttagtacttt attgatgaaa aaaatgaggt catgagtggc tgtctaatcc 41520
aaaatcatac tgctagggag taggtgaaat tcctcaagtt aaagttgcat agcaagacac 41580
atgctgtttc ttttatttgg aatatcttcc ctcctccatc actctttcac atgttgtagc 41640
acatatatgt gtatatatat ggtgtgtgta tatatatagt ataaatatat gtatttattt 41700
tatatattat atatacacat atattatata tatgtatgtg tatatatata atatatatca 41760

catatatttt atatatatgt atgtgtgtat atatatatgt atgtgatata tatatatata 41820
tgtatgtgat atatatatat atatgtatgt gatatatata tatatgtttg agtcagggtc 41880
tcactttgtc acctaggctg gagtgcagtg gcacaatcac ggctcactgc agcctcaacc 41940
tcctgggctc aagtgatcct ccccactcag ccttctgagt agccacgact aaggcatgca 42000
ataccacata gatatttgat tgctgtgtcc ccagtagacc agaaactcca aagtgttggg 42060
cactaggtct attttgttca ctgctctatg cgcagtgcct gtaattgtga gagtgtatgg 42120
taggtgctca atggatgttt gtggatttac aggaaaaccc ttatcctggt gcctaggaaa 42180
gggttaagac atactaataa tttcgggcct agtggctcat gcctgtaatc ccagcacttt 42240
gggaggctga ggtgggtgga tcacctgaga tcaggagttt gagaccagcc tggccaacat 42300
ggcaaaaccc cgtctctact aaaaatacaa aaagtagctg ggcgtggtgg tgggtgcctg 42360
taatcctagc tactcaggag gctgaggcag gagaattgca tgaacctggg aggcggaggt 42420
tgcagtgagc cgagattgcg ccattgcact ccagcctggg tgacaagagt gaagctcagt 42480
ttcaaaaggg gggaaaaaaa aaaaggctgg gtgcagtggc tcacgcctgt aatctcagca 42540
ctttgggagg ccgaggcagg cggatcacct gaggtcagga gtttgagacc agcctggcca 42600
acatggtaaa accccaactc tactaaaaat acaaaaatta cccgggcatg gtggcacgca 42660
cctataatcc cagctactcc agctgaggca ggagaattgc ttgaacctgg gaggtggagg 42720
ttgcagtgag ccgagattgc accattgcac tccagcccgg gcgacagagc aagactccat 42780
ctcaaaaaaa aagaaaaaga aaaaaagaaa aaaagacatg cgaataatta tgtgccagag 42840
ggcttttgat cacagacagg ctcagtctcc aaatgtctag tacagaatcc tctaaagatg 42900
ttcgcaagtc cagcgacatg gtgtgcattc cagtaacaga aaagcctctg cttccatcaa 42960
ttatctgtgt aggtgctgtg accccgtgcc tgagtaaccc agagtctgtg agtaacagag 43020
cagtcctcat tttggttgct tttatttaaa cattggtacc aagccacctt tcaataagtt 43080
agacccggcc agtgtggtgg ttcatgcctg taatcccaga attttgggag gctgaggcgg 43140
gcagatcatg aggtcaggag ctcaagacca acctggccaa tatggtgaaa ctccatcttt 43200
actaaaaata caaaaaaatt agccgagcat gagggcacgc gcctgtagtc ccagctactc 43260
gggaggctga ggcaggagaa tcacttgaac ccgggaggcg gaggttgcag tgagcgagat 43320
tgtgccactg cgctccagcc tgggtgacag gaggctccgt gtctaaagat aaaaataaaa 43380
ataaaaatgt aaataagcta gacccaggaa gcagaaatca tgcccatgcc ctgggttgtc 43440

ttggttatgg tttatgtgcc agtgcttttg ttacctgtct cctggatggg ctattcaggc 43500
ttacaagact ttcccttaat caccacacct ggggagtggg ggtgtctcat agatttgctg 43560
ccacctgata aaatgaaaat tctaaattct cctggaatat tttcttgttt actagaataa 43620
catatcctac aaatcttggt ctcctaccac aggagaaaat tattgttttt atgtatcaac 43680
taacttacat tccttaaggt ggacttttcc ccagatgtta aaaaatgcat gctgggcgtg 43740
gtggctcaag cctgtaattc cagatacttg ggggactgag gtgggaggat cttttgagcc 43800
tagtagttgg aggctgcagt gaactatgat ggtgccactg tactccagcc tgggaaacag 43860
agcaagacct tttctgtatt tacaaaaaaa agagaaaaga ccgggcatgg tggcttatgc 43920
ctgaaatccc agcactttgg gaggctgagg tgggaggata gcttgagccc aggagttcaa 43980
gactggcctg ggcaacatag agagaccccg actctgcaaa atataaaaat tagggcccca 44040
atgtctacgc acaccggaag acagaggtcc tctttccttg cctaatgcag ccatggctcg 44100
tggtcccaag aagcatctga agcaggtagc agctccaaag cattggatgc tgaataaatt 44160
gactggtgtg tttgctcctc atccatccac cagtccccac aatttgagag agtgtctccc 44220
cctcatcatt ttcctaagga acagacttaa gtatgtcctc actggaaatg aagtaaagaa 44280
gatttgcatg cagcggttca ttaagatcaa tggcaaggtc cgtactgata taacctactc 44340
tgctggattc atggatgtca acagcattga caagtcggga gagaatttcc gtctgatcta 44400
tgacaccaag ggtcgctttg ctgtacatcg tattacacct gaggaggcca agtacaagtt 44460
tgtgcaaact gagaaaaatc tttgtgggca caaaaggaat ccttcaaata tatatatatg 44520
tgtgtgtgtg tgtatatatg tatatatatt tatatatatg tgtgtataca tatgcatata 44580
tatttatgtg tgtatatatg tatatatatt tatatatatg tatgtgtgta tatatatttt 44640
tttcccaaag gaaaaagaag acgtgagcca ccgtgcccag cctccacaat tgtattatac 44700
atatatcaaa acatgttgta cactgtaaat atacataact tttatttgtc agtttcagct 44760
taacaaaact agaggaaaag gctagtagag aacaaaacga gatacgggca tagataataa 44820
tgcagaagaa tggacaagta tattgccagg tggtagattt atataaaatt gcaaagaata 44880
agatcctcgt tataaaagct gtatgtgagg ccaggcgtgg tggctcacgc ctgtaatccc 44940
agcactttgg gaggccgagg tgggtggatc acctgaggtc aggagttcaa gaccagcctg 45000
gccaatatgg tgaaccctca tctctactac aaaatacaaa aattagccgg atgtggtggc 45060
gggcacctgt aatctcagct actagagggg gctgaggcag gagaatcact tgaactcggg 45120
aagtggaggt tgcagtgagc cgagatgatg ccaccgcact ccagccaggg tgacagagcg 45180

```
agactctacc tcaaaaaaaa aaaagctgca tgcagatata tgtacttaaa tatatagaaa  45240
agggactcac aggatattta ccatgtttcc ttctggggag aagcgaggca attcctactt  45300
gttggtatgg gaagagcacc taccagggca gagtctgaga gaaatatcct attaacctca  45360
ggaataatgt tcaattctgg gaaaaagcca ggaagatgga catttatgtg ttccatggca  45420
atacacgtat acatatttta agagtatttt ataaccaaaa tgtacgtatg ccttcctgga  45480
ttttaaaaat acattaatac caaaagctaa caaagttatc aagtggaaaa aaatgcaagt  45540
ctctgtcttt acccacccac cactcacctg ccttccccac ttccaggcag ccaccataaa  45600
caatgttccc ttagatgtgg aattgttcct ttgtaagtaa atattgttgt taagagaaaa  45660
tgctaatagc aagggttttc tgcagcgaat tttgatggct atgcattggg tgtggtcacg  45720
ggaggagaaa gaaattaaac catccctcca atggaggggg aaaaaaggaa agaaaggaag  45780
aaaggaaaga gaagacgatg acccacaaaa tgagtacctc tgagtctgat ccgaaaccct  45840
tctacacata gtgtacaggc acaacagttg accaccaggc cttaattatt attattatta  45900
tttttttttt ttttaaagca gagtttcgcc cttgttaccc aggctggagt acaatggcac  45960
aatagcggct caccgcaacc tctgcctccc agattcaagc gattctcctg cctcagcctc  46020
ccgagtagct gggattacaa gcatgtgcca ccacgcccgg ctaattttgt atttctagta  46080
gagacatggt ttctccatgt aggccaggct ggtctcgaac tcccgacctc aagtgatctg  46140
cccgcctcgg cctcccaaag tgctgggatt acaggaatga gccactgcac ccggccaatt  46200
tttattattt taaaattgta tgtaaagatg gagactcact atgttgccca ggttgaactc  46260
ctgggttcaa gcattcttcc tgcctgccta ggcctcccaa gtgctgggat tacaagtggg  46320
agctactgca cccaaccctg ccctttaaat aatattgatg ttaattagaa taagagcagc  46380
agctattgtg cttgatcctg tttctttttc tttctttttc ttcttcttct tttttttttt  46440
ttttttttga gacagagtct ccctctattg tccaggttgg agtgcagtgg cacgatctct  46500
gctcactgca acctccgcct cctgggttca agttattctc ctgcctcaac ctctggatta  46560
gctgggatta cagacgtgcc tcatgcccgg ctaatttttg tatttttagt ggagacgggg  46620
tttcaccatg tttgccaggc tggtgttgaa cccctgacct caagtgatcc acctgcctgg  46680
gcctcccaaa gtgctgagat tacaggtgta agccaccatg cctggccctt tttctttttt  46740
ataaattgag acagggtctc gctttgtcac ccaggctgga gtgcagtggc acaaactctg  46800
cttgctacag cctcaacctc ctgggtttga gcaatcctcc tgcctcagcc tctcgtgtag  46860
```

ctgggaccac aggcacatgc caccatggct ggttaatttt taaatttcct gtaaagacgg 46920
ggtcttgtag agaacagcct aggctggtct ggaactcttg ggctcaatca atcctcctgc 46980
ctcagcctct caaagcgctt ggattacaga cgtaagccac tgtgcctggc ctacccatgg 47040
gggtcccaac tgccacattt tctgccgctt gggttcaccc atccctctcc tccacccgtc 47100
cctcctttct cagtattgta aaacacaccc cgacctcata tcattttacc cagaaatata 47160
tcagtgtcca tctaagcaga taaaggcgtt cttgcttgtt tgtttttcta acgcccagtg 47220
tgatcgtcac tcccagtaca cgttaataaa aatttctggg ctgggtgcag tggcttgtgc 47280
ctgtaatctt agcactttgg gaggccaaga caggggaatg gcttgaggca aggaatttga 47340
gaccagcctg gacaacagag tgagaccccc atctccacac acacacacac acacacacac 47400
acacacacaa attagcgcgg cgtggtggtg gaaggctgtg gtctcagtta cttgggaggc 47460
tgaggtggga ggattgcttg agcccaggag gttgaggctg cagtgagcca tgattgtgcc 47520
actgcactcc agcctgagtg acagagtgag accctatctc aaaataaata aataagccag 47580
gcacttatta attctgtgac tcccatctgt aatcatagca cttggggagg ctgagacagg 47640
aaaattgctc aaacctgaga gggggaggtt gcagtgagca gagatcatgc cactgcactc 47700
cagcccgggc aacagagtga gtgagacttc atctcaaaaa ataataataa taaatgaata 47760
ataatttctt accatctaac aacaggtccc cttttcaatt ctgtgctttt aaatgagacc 47820
agaggaagag ctggaaagct ttggaaactt gccaaacatt gtaaagcact taaatgaatg 47880
tcttcaaggg ctgtgctcaa agtgtgatcc ccaggcaggg ccaccaaacc accctggagc 47940
ttgctagaaa tgcaaaatcc caggcccagg gccccagatc cagtggaact gaactaccgc 48000
tggaccaaaa ccaatggatc attaaagttt aaggttggct atctccactg tgtggaaggc 48060
agtggctggt cctctctgaa ttaatctact ttttcttgag gcctggatct tgctggttga 48120
tagcagcttc actgagtctt cccaccagca gtgagagcct gccatgtgtt aaacatcaac 48180
caggcctatg gggacagttg aggtcactgc cgttgacagg ctcctattct agtgcaagat 48240
acagacaaga agctaataaa tgataaagaa atagagtaat cccaggttgt gattattaac 48300
atatgagttg tgcgaggcct ccccaacaag cctattgaaa ttgcaccagc agagccaggt 48360
gcggtggctc acacccgtaa tccctgcact tcaggaggct gaggtgggcg gatcacctaa 48420
ggtcaggagt tggagaacag ctggccaaca tggtgaaact ccgtctctac taaaagtaca 48480
aatattagct gggaatagta gcacctgcct gtaatctcag ctactaggga gactgaggca 48540
tgagaatctt ttgaacctgg gaggtggagg ttgcagtgag ccgagatcgc accactgcac 48600

tctagcctgg ttgacagagc aagcaagact cagtctcaaa aaaaaaaaga aaagaaaaga 48660
aaaagaaatg gttccagcgc ctaaggtgtc ctccccctct ggctttactt tgccctgtgg 48720
ccctgtttcc agatgctggg ccatgcatct tactacttat ttgctcattg cttttctccc 48780
aggccaggaa gtaatcatca cacacagact gccatatacc caccccttagg acacagaagg 48840
agtaggagaa atacttgttg aatgaaagct actatgactt ttcaaacaat gagagtgcct 48900
aaaagtgatc aatacctgat tagcgattgt tttttaatac atctgattcc tacccatggg 48960
aagagttcct atgtggaatg aggctacaat tctttcactg aaaataaatt gcaccctatc 49020
ctgggtgctg ctccaacctc acctcagggc tgggagcctg gtgctgccac acccttgccc 49080
tgagcagaag aatccggaag aagcctttgg ccagagccgc aggtgagtca tttgaggact 49140
ctatgccctt gtggccatcc ttcggtcacc tgtctggaat tttattttt ttagcccccg 49200
ggctctgcac caaagagctg gctttttttg ttgttgttcc agctatacct aaaagggacc 49260
agcctcattc atataaagat ctcagatctc tcaaaaggca tctacaaatt aagagtaatt 49320
ctgctcaaaa ttaaaggaaa aacatttttt tggcggagga gggaattgtg tagagttgaa 49380
gaaatccaag agattgtaaa gttcttgtgg agggtggagt cagtgagagg aggcagagaa 49440
acaaggcgaa ggcctttgcg ctccatctgg tatctgtgtc caatagatgg tttcacccac 49500
agcatggcag agtccagcaa ggatccaaga atatgcagga atcagggaaa tgatgaagac 49560
agcactttaa atgggaggga tttttaagta aatgctatta ggccaatcaa ctggaagtct 49620
attcatttgc aaacagtaac atgaggcttc ctcctcaaac cccaaacaca tatacacgca 49680
cattccacag ttccacacgg gctcaagacc caaaccaaaa aaacaaattc tatgagaatg 49740
cttgatacaa gactaataaa aagaagactg ggccaggtgc ggtggtggct catgcccata 49800
ataccaacac cttgggaggc ctaggcagga ggatcactta aggccaggag tttgagacca 49860
gcctgggcag cacggtgaga cccccgtcac tacaaaaaat acaaaaatta gctgggtgtg 49920
ctcgcatgct cctgtggtcc cagctactca ccgggctgag gtgggaggat cgcttgagcc 49980
caggaaatgg aggctgcagt gagccccgat tgagtgattg aacctgggtg acagagcatg 50040
accccgtctc aaaaaagaaa aaaaaaaaga aactacactt tcagggataa tttctatagt 50100
tggttactag agaagtttct ttaaatatgt agagcaccat ttaaacagaa taataaaaag 50160
gctgtgagca gtggctcatg cctgtaatcc cagcactttg ggaggccgag gtgggcaaat 50220
cgcttgaggc caggagttcg agaccagcct ggccaacatg gcaaaacccc atctctatga 50280

aaaatccaaa aattaagccg ggtgtggtag ctcatgcctg taatctcagc actttgggag 50340
gccaaggcag acagatcacc tgaggttggg agttcaagac cagcctggcc aaaatggtga 50400
aacctcgtct ctactaaaaa tacaaaaatt agccaggtgt ggtggtgcat gtctgtaatc 50460
acagctactc aggaggctga ggcaggagaa ttgcttgaac ctgggacgcg gaggttgcag 50520
tgagctgaga ttgtgccact gcactccagc ctgggtgaca gagcaagact ctgtctcaaa 50580
aaaaaaaaaa aaaaaaaaga gaaagaaaaa tacaaaaatt agccggactt ggtggtccac 50640
acctgtaatc tcagctgctc ggaggctgaa gcgggagaat cacttggacc agaccctgga 50700
gacagaggtt gcagcgagca gagattgcat cactgcactc tagcctgggc aacagagtga 50760
gactccattt taaaataata agaagaagaa gaagaagaaa aagagaaaga acactaaaaa 50820
gatagtgcaa tggtaacatg agcaaagagc ttaaacaggt agttcactga agaaggaata 50880
caagtaaata cagtaaatac cagaagagtt gtcatcacca taccatttct accatcaggg 50940
cgcacagata ctaaaatgac aaacaaacag aaaatgagac atagctggag ataccgtcga 51000
agaagggcca agaatgttgc ttggcaaatt catgtaaaag agcagtaaga tcttctttaa 51060
aaaagctgtg tgcagaaata tgtacttaaa tacagtatat agaaaaagga ctcaatggct 51120
acagacttct taatcctggt tatttctgtg gagaagctag ggagttccta cttgttatag 51180
tgtaccaagt ggaacatcca ttgcaggtga acttaccagg acacaggctc tcttcttcgc 51240
tgtcggcagg ggtctaattc aattgatggg agcatttgag aacctaaatg tagcattagc 51300
taaaaactct tggacactac accctcccctt cttgcattta ccctatagca tgtacctgta 51360
tgattctgta agtcctatag atactatggg ggatggatcc attacattct gtagcattga 51420
acttcccaaa tgctcacagc ccttaggtcc taaacagcct gtggggtggc ttttgcttgg 51480
gccacaggct atattaaaat atattgcaat caagcagggg aatagtttaa tcttaaagca 51540
agtggtttag cacctggtgt gagaatctcg cttgagccgc ctgcctggcc ctaaagggga 51600
catctctaaa catctgcctc tggggttctg aagtgtgcag tggggagtca ccttcacaga 51660
gaggacttgc tgtgacctgt aagacaccat gcctcttctc tttccttccg agtgctgcct 51720
gacagtgtcc taagtatttg tttgtggtgg tttatcctcc ccaatcatgg taagctttgt 51780
gagatcaggg gctttgttgc ttttgttccc cggggtcccc aagccaggcc tggcatctgg 51840
gaggtgctca gagtgtatct gttacaggaa caagaaagtc ttatccaccg cccatgcagc 51900
ttaagtaaag cattgtattt actaaacact cgctgtggtc caaatgcttt gttgagcaag 51960
gtagataaga ggatggatgt ttgtggactg aaagaaccag ctcctgaccc caccccttagt 52020

gctccaattc tggctgagct ctgggtgccc cagggccttg ctatcccttc ccctccccct 52080
gcttcctcta agaatcttct ccatccttca agaccctcag gattgctgcg aagctctctc 52140
acccctggac tggaccagac cacccgtagc ctcccatagc accctcctgt atcacctctg 52200
taaatgctgg gctggttatg gaactgtcca ccaagtgcga aggtttatct cccactgctg 52260
atgggaagcc ccctgaagat ggggattttt ggcctctttt tttttttttt ctttgagaca 52320
gggtctcgct gtcgcccagg ctggggtgca gctgcacaat catggctcac cacagccttg 52380
acctctcagg ctcaagtaat tctcccacct cggcctcctg ggtagctggg agcacaggcg 52440
cgtgccacca tgcctagcta atgtttctat tatttgtaaa gactgagtct tcttatgttg 52500
cccaggctgc tctggaactc ctgggctcaa gcgatcctcc ctcctcagcc tcccaaattg 52560
ttgggattac aggcgtgagc catcacgcta gccagatttt ggcctctttg gttgagtgtc 52620
agctccccag cacagggcct gggaatctgt ggaatgaatg cacgagtagt accagactga 52680
caactggggc agaggtgggg aatcccagca tggagggacc tgggcagacc tgggtggcca 52740
gtctgaaagg tgatgcatgt gattcaacac ctggcccgag aggggaccac ttaggccttc 52800
ccgggtgctc atgccacaga cacatgcaga ccctgggcac agcctgaatg aggcctttgt 52860
taggatgaag ggtttggctt tccaagcccc ccaagcgctc tgcagacatt acttttcact 52920
tatctctccc ccatatccca agaggttgag actatcattt tcattttttt tttttttttt 52980
tttttgagac agagtcttgc tctgtcaccc aggctgaagt gcagtggctc gatttcggct 53040
cactgcaacc tccgcctctt gggttcaagc gattctcctg cctcagcctc ccaagtagtt 53100
ggaattgcag gcgggtgtca ccctgtcccg ctaatttttg ttattttttt tttagtagag 53160
atggggtttc accatgttgg ccaggctggt cttgaactct tggcctcaag tgatccaccc 53220
acctcagcct cccaaagtgc tgggattaca ggtatgagcc accacgcccg gctatcattt 53280
ttattttaaa agtgtggaaa ctgaggcacg gagaggttag gtcgcttcca gtgagggatg 53340
aaggtgggtt gaacctggag ccctgggacc ccaggtcagt gcagcctcac atggagccag 53400
ctgcctcggt cctggttgca cttgaggcct tgctggctct gacactgaca aagtttgaaa 53460
gttccctcct aacctccctg agcctcagtt tcttcgtctg taaatagaaa tatgcaaagg 53520
acccatttca cagggcatcg tgaagctcag atgacctcgt gcacagggcc caggtcaggt 53580
ggggttcctg ctgtgcgggg ctggccattc gcatccctga cctcctggcg gtgcctgttc 53640
catgcccagc gagccccaga ggaacaaatc tttcactaat gctcattacg ggttaagctg 53700

aaccccacct ttcacatgca agaggccttc cctttcctgt gggaggcagg agggcatcct 53760
gattagcacc aagctctgga cccaaggttt gaggtttggc tctggggcct tggacagtct 53820
tccaacttcc ctgtgggtga aatagtcaaa acccatgttt ccatctcagg gatgtgagga 53880
ggatcttatc tagatcaatg cccagaaatc ataatgcact gtgcctggca cagagtcggt 53940
gcccaggcag ggtctcagcc tcattatcga tgccactcag ccctcctgcc ctcctctgtg 54000
gcctggctta aagtcttcct ggtggaatta ggcgcccaag ctcagaagtt tcacctgttc 54060
caccaaggac tccagagatc ttcataggga tcccccaagt cttcgcttgg acattatgat 54120
gtttcacctt aggtgtcaag atgaaggcac acgtggtgcc cagacattag tcaaacatta 54180
ttctggtcca ggcatggtgg ctcacaccta taaacccaac acttaatctg tgcattttat 54240
tttattttat tttattttat ttattttatt gtttgagatg gagtcttgct ctgtcaccca 54300
ggctggagtg caatggtgcg atcttgactc actgcaacct ccgcctccca ggttcaagtg 54360
attctcctgc ctcggcctcc tgagtagctg ggattacaag cgcccaccac catggctggc 54420
taatttttct attttaagta gagacagagt ttcaccatgt tggccaggct ggtcttaaaa 54480
tcctggcctc aagtgattca cccacctcag cttcccaaag tgttaggatt acaggagtga 54540
gccactgcac ccagtcaatc tgtgcatttt atactttgta aactagatct ctataaggtt 54600
gatttaaaat gttaaaaaag aaagtaagca gctgggcacg gtggctcatg cctgtaatcc 54660
cagcactgtg ggaggccgag gcaggtggat cacctgaggt caggagttca agaccatcct 54720
ggccaacatg gtgatacccc gtctctacta aaaatacaaa aattagctgg gcgtggtggc 54780
aggtgcctgt aatcccagct actcaggagg ctgaggcagg agaatcactg aaacccagga 54840
ggcggaggtt gcagttagct gagacccagc ctgggcaaca agagcaaaac tccatctcaa 54900
aaaaaaaaaa agaaagaaag aaagaaagta aggccgggcg cggtggctca cgcctgtaat 54960
cccaacactt tgagaggctg cggcaagcgg atcacgaggt caggagatcg agaccatcct 55020
ggctaacacg gtgaaatccc gtctttacta aaaatacaaa aaattaactg ggcgtggtgg 55080
cgggcgcctg tagttccagc tactccagag gctgaggcag gagaattgct tgaacctggg 55140
aggcggaggt tgcagtgagc tgagattgca ccactgcact ccagcctggg cgacagagcg 55200
agactccgtc tcaaaaaaaa aaaaaaaaag aacgaaagaa agtaaatata tatccatata 55260
tggaaaaggc taattttatg gaggtcgttg tgattctaac cattgtagag ggtacctaag 55320
aggaaaggga gactgatcct aacaactcgg acctgaccct ggcatgctgc tcagcctcac 55380
tcagggtggg aagcctgggc actcccacac tgtgtcctgt gctgccttca ttgaaagaag 55440

tcagaagaag ccttaggcca gaggcacagg taagtcattt gagaactcta tgcccttgtg 55500
gccatcctct gggccacctg tttggatttt gatttgataa cccagggttt ctcaacctca 55560
gcactgttga cattttggac cagataatgc tttgttgtgg ggactgtgct tgtaggatgt 55620
ggagcagcat tgctggcctc tacccactag atgccagggg caccctccct agtcatgaca 55680
accaaaacat atccccgaac ctagctgaat gtcccctggg gggtcggggg cactaaatcg 55740
ccttggttga gctactgctt taatataaga cttctgcccc agggcttgcc ctttctagac 55800
tgaaaagaga ccttatctca gcctcattag tagaaagata tccagtcttt aaatctaccc 55860
attctgagta attccacaca aaattagagc aagatttgtt tatgtgttgg aaggtgggga 55920
agaacaggac acattggttc tataacctta ctggaagact tgaacgaggg agatgaggca 55980
gaaattgaaa caaggctgaa agctacaata atcaaaacag tctggcctca acataggaat 56040
agactcagtg ggacagaatc ataggaatag aatcaactaa acaggtaaga gggtgcagaa 56100
gaaggcacat ggatagaggt cagctcatta tatgattaaa ggtgacattt ccaggccagg 56160
tgccatggct cttgcctgta atcccagcac tttgggaggc tgaggtgggc gaatcacctg 56220
aggtcaggag tttgagacca gcctgaccaa catggcgaaa ccctgtctct actaaaaata 56280
caaaaattag ccgggcgtga tggtgggcac ctacaatcca aactactcag gaggctgagg 56340
caggagaatt gcttgaaccc gggagatgga ggttgccatg agccgagatt gcgccactgc 56400
acttcagcct gggcgacagt gtgtgactcc gtctcaaaaa aagaaaaaaa aatgcatatc 56460
aaatgtgcac tttgttttgg agtggattat cttgttggca aaaaattgag gactggtaaa 56520
gtaactctcc tccctacaac atctggattt agcacttttc ctgcattgct gtagggaaga 56580
tgagggagtg ggacctttta gagactgtag tctcctacag ccttaccctg gcagcccccca 56640
ggccctaaat agcctgtgga tgtgtttgtt tgtgccacag gttactttaa aaggcagctt 56700
gactcagctt gttagggtgt gtgctcagca cctagtgttc acacattttt gacacctgcc 56760
tggccctgaa gggtgagtta ctaaaccact atgttccctg gttccttcaa atgcaaaatg 56820
aacatataat ccttcctacc tcacagggtt catacaagga ttaaatgaac tagtacatgc 56880
agagggctgg tgaggaacat gcggtaggcc tgttagccat gcggcatatg aaaaaagcct 56940
aacaaaagtg aaaaaaaaaa aaaaggccag gctcagtggt ccccacctgg aatcctgtca 57000
ctttgggagg ctgaagtggg agaactgctc gagggccagg ggttcaaaac cagcctgggc 57060
aacatactgt gacctcgtct ttgaaaaata ataataataa taataacata acaaaaaaaa 57120

tcctaacatc ctaacacggc agttattatc ttttttttt ttaagatgag atcacggcac 57180

tgcactccag cctgagcaac aagagcgaaa tctccgtctc aaagaaaaaa aaaaaaattt 57240

atgttgctat gttgcccagg ctgatgactc cactattgtt attaaaaaag aatttggctg 57300

ggtgcggtgg ctcatgtctg taatcccagc actttgggag gctgaggcgg gaggatcacc 57360

tgaggtcggg agttcgagac cagcctgacc aacatggaga aaccccatct ctactaaaaa 57420

tacaaaaatt agccaggtgt gttggtacat tcctataatc ccagctaccc aggaagcagg 57480

agaattgctt gaacccggga ggcggaggtt gcagtgagcc aagatcatgc cattgcactc 57540

cagactgggc aacaagagca aaactctgtc tcaaaaaaaa aaaaaaatac aattcattat 57600

gagccagatg tgatagctca tgcctataat cccagcactt cgggaggcca atgcaggtgg 57660

atcatttgaa cccaggagtt ggaaaccagc ctgggcacca tgggggaaac cccctctctg 57720

cataaaatac aaaaatcaac cgggcaaggt ggcatgcac 57759

<210> 109
<211> 978
<212> DNA
<213> human

<400> 109
cgaaaatggc taaattcgtg atccgcccag ccactgccgc cgactgcagt gacatactgc 60

ggctgatcaa ggagctggct aaatatgaat acatggaaga acaagtaatc ttaactgaaa 120

aagatctgct agaagatggt tttggagagc accccttta ccactgcctg gttgcagaag 180

tgccgaaaga gcactggact ccggaaggac acagcattgt tggttttgcc atgtactatt 240

ttacctatga cccgtggatt ggcaagttat tgtatcttga ggacttcttc gtgatgagtg 300

attatagagg ctttggcata ggatcagaaa ttctgaagaa tctaagccag gttgcaatga 360

ggtgtcgctg cagcagcatg cacttcttgg tagcagaatg gaatgaacca tccatcaact 420

tctataaaag aagaggtgct tctgatctgt ccagtgaaga gggttggaga ctgttcaaga 480

tcgacaagga gtacttgcta aaaatggcaa cagaggagtg aggagtgctg ctgtagatga 540

caacctccat tctattttag aataaattcc caacttctct tgctttctat gctgtttgta 600

gtgaaataat agaatgagca cccattccaa agctttatta ccagtggcgt tgttgcatgt 660

ttgaaatgag ggaattccct ctgttccttc atctccgagg ccgagagcct ctgccagaac 720

tggcggggat ggcgcaaaca gtcagcgggg cccaattccc ccactggcgg cggtggcgga 780

ggtggcagtg gcggtaccag aatgcgagat ggactggtga tcccattggt ggagctgtca 840

gcaaagcagg tggcatttca tatcccattt gaagtggtgg agaaagttta cccaccagtg 900

cctgagcagc tacagctccg aattgctttt tggagcttcc ctgagaatga agaggacatt 960

cggctgtatt cgtgcctg 978

<210> 110
<211> 6359
<212> DNA
<213> human

<400> 110
ggcctggctg ttgctcaggt gaccagcttg tgtctctggg agggcgctgc tttccccggc 60

cacccggcgc gatgatccag aatgtcggaa atcacctgcg acggggcttg gcctctgtgt 120

tctccaaccg cacatcccgg aagtcagcct tacgtgcggg gaacgacagt gccatggcag 180

acggcgaggg ataccggaac cccacggagg tgcagatgag ccagctggtg ctgccctgcc 240

acaccaacca acgtggtgag ctgagcgtcg ggcagctgct caagtggatt gacaccacgg 300

cttgcctgtc cgcggagagg cacgctggct gcccctgtgt cacagcttcc atggatgaca 360

tctattttga gcacaccatt agtgttggac aagtggtgaa tatcaaggcc aaggtgaacc 420

gggccttcaa ctccagcatg gaggtgggca tccaggtggc ctcggaggac ctgtgctctg 480

agaagcagtg gaatgtgtgc aaggccttgg ccaccttcgt ggcccgccga gagatcacca 540

aggtgaagct gaagcagatc acgccgcgga cagaagagga gaagatggag cacagtgtgg 600

cggctgagcg ccggcgcatg cgccttgtct atgcagacac catcaaggac ctcctggcca 660

actgcgccat tcagggcgat ctggagagca gagactgtag ccgcatggtg ccggctgaga 720

agacccgtgt ggagagtgtg gagctggtcc tgcctcccca cgccaatcac cagggcaaca 780

cctttggggg ccagatcatg gcctggatgg agaatgtggc caccattgca gccagccggc 840

tctgccgtgc ccaccctacg ctgaaggcca ttgaaatgtt ccacttccga ggcccgtccc 900

aggtcggcga ccgtctggtg ctcaaagcca tcgtgaacaa tgccttcaaa catagcatgg 960

aggtgggcgt gtgcgtggag gcctatcgcc aggaggctga gacccaccgg cgccacatca 1020

acagtgcctt tatgaccttt gtggtcctgg acgcagatga ccagccccag ttgctgccct 1080

ggattcggcc ccagcccggc gatggtgagc ggcggtaccg agaggccagt gccagaaaga 1140

agatccgcct ggacaggaag tacatcgtgt cctgtaagca gacagaggtg cccctctccg 1200

tcccctggga ccctagcaac caggtgtacc tgagctacaa taacgtctcc tccttgaaga 1260

tgcttgtggc caaggacaac tgggtgctgt cctcggagat cagtcaggtc cgcctgtaca 1320

```
ctctggagga tgacaagttc ctctccttcc acatggagat ggtggtgcat gtggatgcag   1380
cccaggcctt cctgctgctc tcggacctgc gtcagaggcc agagtgggac aagcactacc   1440
ggagcgtgga gctagtgcag caggtagacg aggacgacgc catctaccac gtcaccagcc   1500
ctgccctcgg aggtcacaca aagccccagg acttcgtgat cctggcctcg aggcggaagc   1560
cttgtgacaa tggggacccc tatgtcatcg cgctgaggtc ggtcacgctg cccacacacc   1620
gagagacgcc agagtacaga cgcggagaga ccctctgctc aggcttctgc ctctggcgcg   1680
agggggacca gctgaccaag tgctgctggg ttagggtctc cctgactgag ctggtctcgg   1740
caagtggctt ctattcctgg gggctcgaat ccaggtcaaa gggtcgcagg agcgacggtt   1800
ggaatggaaa actagctgga ggacacctga gtactcttaa agcaatcccc gtggccaaaa   1860
tcaacagccg atttggatac cttcaagaca cctgaaacct tatcatgagc cagatgccaa   1920
ggaagagact ctgggaggat ccagaggacc ccctggttgc agccacgtag agactgacgc   1980
tgaggaggac tccaactatc atgagcaaca cccgtcgaac acagccaccc cgctaagaac   2040
agatcaagaa gctgtcacag atggcggaag aaaacctgag gaaagcggga caaccagtca   2100
caatgaataa tttaatggta gctatgatag cagttatcac cactgccgtg agtattcctt   2160
caataagggc tggtaataat gcctggatgc aatcattcta tgacacagtt acacatgctt   2220
tctgatctca gtatttacca taataaatcg gctcctataa ttgaggcata ccaccctcaa   2280
aaacctattt gtaaacagga ttggacccag ttagaaaaaa tgaacgtact tatttaggaa   2340
gattgctttg cagaataggc agagatgctg cacaacgatt cctatggaat cattactaat   2400
tggtccccca aggggatgtt tagcttgaat tgcacctctc agtctgcatg ccacggtcac   2460
actatgttca gatgatctga acaaaacagt cagatggtag aaatgttaag tacggcaaaa   2520
gttcctatta tctggaacca tggcggtata gtggcacctc aacctcaaat aatatggccc   2580
gctctaggag ctaaacataa ggatttgtaa aaactattaa atgctcttaa taagatcaaa   2640
atttgggaaa gaataaaaga gtatctagaa ggacactcta caaacttgtt tttggatata   2700
gcaaaattaa aagaacaaat atgtaaagca tcccgggcaa cctgacctta atgccaggaa   2760
ccagagtgct taaaggagct gcagaaaaat tagcagctag taacccattt aaatgggtaa   2820
aaacacttgg aagctctgtg atttcaatga tgactatgct ttttaatctg ttgtttgcct   2880
ttgtatagtc tgcagatgcg gatcccgact cctgcgagag tctctttgca aatcgaagaa   2940
gggagacatg ttgggagcaa gccccccaga gtctggccat aaactggccc caaaactggc   3000
cataagcaaa acctctgcag cactaaaaca tgtccataat ggccctaacg cccaatctgg   3060
```

aaggttgtgg gtttatggga atgagagcaa ggaacacctg gcctgcccag ggcggaaaac 3120
cgcttaaagg cattcttaag ccacaaacaa aagcatgagc gatctgtgtc ttacgggtgt 3180
gttcctgctg caattaattc agcccatccc tttgtttccc ataagggata cttttagtta 3240
atttaatatc tatagaaaca atgctaatga ctggtttgct gttaaatgaa ggggtgggtt 3300
gcccctccac acctgtgggt gtttctcgtt aggtggaacg agagacttgg aaaagagaca 3360
cagagacaaa gtatagagaa agaaaagtgg gcccagggga ccagcattca gcatacagag 3420
gatccacact ggcaccggcc tctgagttcc cttagtattt attgatcatt atcgagcatg 3480
gcaggataat aggataatag tggagagaag gtcagaaggt aaacacatga acaaaggtct 3540
ctgcatcata aacaaggtaa agaattaagt gctgtgcttt agatatgtat acacataaac 3600
atctcaatgc cttgaagagc agtattgctg cccgcatgtc atacctacag ccctaaggcg 3660
gttttcccct atctcagtag atggaagtat attccatgta aagtaaatcg gctttacacc 3720
cagacattcc attgcccaga gacgagcagg agacagaagc cttcctctta tctcaactgc 3780
aaagaggtgt tccttcctct tttactaatc ctcctcagca cagacccttt atgggtgtcg 3840
ggctggggga tggtcaggtc tttcccttcc cacgaggcca tatttcagac tatcacatgg 3900
ggagaaacct tggacaatac ctggctttcc taggcagagg tccctgcggc ctttgcagta 3960
ttttgcgtct ctgggtactt gagattaggg agtggtttga gattagggag tggtgatgac 4020
tcttaaggag catgctgcct tcaagcattt gtttaacaaa gcacatcttg cacagccctt 4080
aatccattta accctgagtt gacacagcac atgtttcagg gagcacaggg ttgggggtaa 4140
ggttacagat taacggcatc tcaaggcaga agaatttttc ttaatacaga acaaaatgga 4200
gtctcctatg tctacttctt tctacacaga cacagtaaca atctgatctc tcttttcccc 4260
acagttaata aatatgtggg taaatctctg ttgggggctc tcagctctga aggctgtgag 4320
acccctgatt ttctacttca cacctctata tttttgtgtg tgtgtcttta attcctctag 4380
cgctgctgag ttagtgaccg agctggtctc ggcaggggtg ggcgggtctt ttgagttcag 4440
gagttcagga gcagcctggc caacatggtg aggccccttc tctactggaa atatgaagat 4500
tgtccgggca tggtggtgtg cctctgtact ttcagctact cgggaagctg ggcacaaga 4560
attgctggag catgggaggt gggggctgca gtgggctgag atcgtgccac tgcactccag 4620
cccgggcgat agagtaagac tctgtctcaa aacaaaaaga gttttaggcc aggtgtggtg 4680
gctcacgcct gtaatcccag cactttggga ggctgaggtg ggcagatcac ctgaggtcag 4740

gagttcgaga ccagtctggc caacatggcg aaaccccatc tctctctact aaaaatacaa 4800
aatttagcca ggtgtggtgg tgggtgcctg tggtcacagc tgcttgggag gctgaggcag 4860
gagaattggt tgaacccagg aggcagaggt tacagtgagc ggagatcgtg ccactgcatt 4920
ccagccgggg tgagagagcg agactctgcc tcaaaaaaag aaggcttagt gtgcaactca 4980
tcagagttgc acagggcaga gaaagaatgg gaaaaaaaac aatttctaga aaacttttcg 5040
aattttctga tcaacaccaa atattccaaa taggaaaaat acaaaaaaat ccatacctat 5100
atgtggcata atatgattgt agagcaccaa agtaaaagat cttatttttt attaaaatta 5160
aaaaaaaatt aaaatagagg gtctcactat gctgcccagg ctggtcttga gctcctggct 5220
tcaggctatc ctcccaccat ggcatcctaa agtgctggga ttgcaggcat gagctgctgc 5280
atctggccca aagtaaaaga tcttagaagc agccagaaaa aatagatttg ggctgggcat 5340
gaatagattg atcaccaaaa aggtggcaga ctaacttctc gacagaatcc acagaagcca 5400
gaaggcagtg gaacattagc tttgatgtac taaaataaaa taccacgcta ggctcctaca 5460
tacagtgaaa atgttttgag aatgagaatt ataatttttt ggcaaacaaa actaaaggag 5520
tttacaacta agaaactctt ttgtgagacg gccttgctct gttgcccagg ctggagtgca 5580
gtgacgaaat ctcagctcac tgcaacctcg gcctcccggg ttcaggcaat tcttgtgtct 5640
cagccttcca agtagctggg attacagatg tgcgccacca ggcccagcta ctttttatat 5700
ttttagtgga gatggggttt cactgtgttg gccaggctgg tctcaaactc ctgacttcaa 5760
gtgatctgcc cgccttggcc tgctaaagtg ctgggattac aggcatgagc gactgtgcct 5820
ctccagtccc tgtgtttttt tttgtttttg gaggttttat ttgtttgttt ttggaggcac 5880
tttcactgtg ttgcccaggc ctgtctgttg gcctcaagtg atcctcctgc cttggatctc 5940
ccagggtgct gggattacag gtgtgaacca ctgtgctcag cccatttttt atttgtttct 6000
tgaaggaagg tagcagtatt aattaatttt ggactttgct cactaaagtt tgaatgttaa 6060
gaatagatgt atacagtctg gatgcagtgg tgcatgcttg taattttagc actttggggg 6120
gctgaagcgg gaggatcgct tgggctcaag agtttgagac cagcctgggc aacatggtga 6180
aaccttgtct ctacaaaaaa attttaaaaa attagccaga catgatggca tgcacctata 6240
atcccagcta cttgggaggc tgaggtggga ggattgcttg agtccaggga ggtcgaggct 6300
gcagtgagcc atgatcacac cactgcactc cagcctgggt gacagagtga tactgtctc 6359

<210> 111
<211> 4257

<212> DNA
<213> human

<400> 111
atgattcgcc tcggtgctcc ccagtcgctg gtactgctga cgctgctcgt cgccgctgtc 60

cttcggtgtc agggccagga tgtccggcaa ccaggaccaa agggacagaa aggagaacct 120

ggagacatca aggatattgt aggacccaaa ggacctcctg ggcctcaggg acctgcaggg 180

gaacaaggac ccagagggga tcgtggtgac aaaggtgaaa aaggtgcccc tggacctcgt 240

ggcagagatg gagaacctgg gacccttgga aatcctggcc cccctggtcc tcccggcccc 300

cctggtcccc ctggtcttgg tggaaacttt gctgcccaga tggctggagg atttgatgaa 360

aaggctggtg gcgcccagtt gggagtaatg caaggaccaa tgggccccat gggacctcga 420

ggacctccag gccctgcagg tgctcctggg cctcaaggat ttcaaggcaa tcctggtgaa 480

cctggtgaac ctggtgtctc tggtcccatg ggtccccgtg gtcctcctgg tcccccctgga 540

aagcctggtg atgatggtga agctggaaaa cctggaaaag ctggtgaaag gggtccgcct 600

ggtcctcagg gtgctcgtgg tttcccagga accccaggcc ttcctggtgt caaaggtcac 660

agaggttatc caggcctgga cggtgctaag ggagaggcgg gtgctcctgg tgtgaagggt 720

gagagtggtt ccccgggtga gaacggatct ccgggcccaa tgggtcctcg tggcctgcct 780

ggtgaaagag gacggactgg ccctgctggc gctgcgggtg cccgaggcaa cgatggtcag 840

ccaggccccg cagggcctcc gggtcctgtc ggtcctgctg gtggtcctgg cttccctggt 900

gctcctggag ccaagggtga agccggcccc actggtgccc gtggtcctga aggtgctcaa 960

ggtcctcgcg gtgaacctgg tactcctggg tcccctgggc ctgctggtgc ctccggtaac 1020

cctggaacag atggaattcc tggagccaaa ggatctgctg gtgctcctgg cattgctggt 1080

gctcctggct tccctgggcc acggggccct cctgaccctc aaggtgcaac tggtcctctg 1140

ggcccgaaag gtcagacggg taaacctggt attgctggct tcaaaggtga acaaggcccc 1200

aagggagaac ctggccctgc tggcccccag ggagcccctg gacccgctgg tgaagaaggc 1260

aagagaggtg cccgtggaga gcctggtggc gttgggccca tcggtccccc tggagaaaga 1320

ggtgctcccg gcaaccgcgg tttcccaggt caagatggtc tggcaggtcc caagggagcc 1380

cctggagagc gagggcccag tggtcttgct ggccccaagg gagccaacgg tgaccctggc 1440

cgtcctggag aacctggcct tcctggagcc cggggtctca ctggccgccc tggtgatgct 1500

ggtcctcaag gcaaagttgg cccttctgga gcccctggtg aagatggtcg tcctggacct 1560

ccaggtcctc agggggctcg tgggcagcct ggtgtcatgg gtttccctgg ccccaaaggt 1620

```
gccaacggtg agcctggcaa agctggtgag aagggactgc ctggtgctcc tggtctgagg   1680
ggtcttcctg gcaaagatgg tgagacaggt gctgaaggcc cccctggccc tgctggacct   1740
gctggtgaac gaggcgagca gggtgctcct gggccatctg ggttccaggg acttcctggc   1800
cctcctggtc cccaggtgga aggtggaaaa ccaggtgacc agggtgttcc cggtgaagct   1860
ggagcccctg gactagtggg tcccaggggt gaacgaggtt tcccaggtga acgtggctct   1920
cccggtgccc agggcctcca gggtccccgt ggcctccccg gcactcctgg cactgatggt   1980
cccaaaggtg catctggccc agcaggcccc cctggggctc agggccctcc aggtcttcag   2040
ggaatgcctg gcgagagggg agcagctggt atcgctgggc ccaaaggcga caggggtgac   2100
gttggtgaga aaggccctga gggagcccct ggaaaggatg gtggacgagg cctgacaggt   2160
cccattggcc cccctggccc agctggtgct aatggcgaga agggagaagt tggacctcct   2220
ggtcctgcag gaagtgctgg tgctcgtggc gctccgggtg aacgtggaga gactgggccc   2280
cccgggacca gcgggattgc tgggcctcct ggtgctgatg gccagcctgg ggccaagggt   2340
gagcaaggag aggccggcca gaaaggcgat gctggtgccc ctggtcctca gggcccctct   2400
ggagcacctg ggcctcaggg tcctactgga gtgactggtc ctaaaggagc ccgaggtgcc   2460
caaggccccc cgggagccac tggattccct ggagctgctg gccgcgttgg acccccaggc   2520
tccaatggca accctggacc ccctggtccc cctggtcctt ctggaaaaga tggtcccaaa   2580
ggtgctcgag gagacagcgg cccccctggc cgagctggtg aacccggcct ccaaggtcct   2640
gctggacccc ctggcgagaa gggagagcct ggagatgacg gtccctctgg tgccgaaggt   2700
ccaccaggtc cccagggtct ggctggtcag agaggcatcg tcggtctgcc tgggcaacgt   2760
ggtgagagag gattccctgg cttgcctggc ccgtcgggtg agcccggcca gcagggtgct   2820
cctggagcat ctggagacag aggtcctcct ggccccgtgg gtcctcctgg cctgacgggt   2880
cctgcaggtg aacctggacg agagggaagc cccggtgctg atggcccccc tggcagagat   2940
ggcgctgctg gagtcaaggg tgatcgtggt gagactggtg ctgtgggagc tcctggagcc   3000
cctgggcccc ctggctcccc tggccccgct ggtccaactg gcaagcaagg agacagagga   3060
gaagctggtg cacaaggccc catgggaccc tcaggaccag ctggagcccg gggaatccag   3120
ggtcctcaag gccccagagg tgacaaagga gaggctggag agcctggcga gagaggcctg   3180
aagggacacc gtggcttcac tggtctgcag ggtctgcccg gccctcctgg tccttctgga   3240
gaccaaggtg cttctggtcc tgctggtcct tctggcccta gaggtcctcc tggccccgtc   3300
```

ggtccctctg gcaaagatgg tgctaatgga atccctggcc ccattgggcc tcctggtccc 3360

cgtggacgat caggcgaaac cggccctgct ggtcctcctg gaaatcctgg acccccctggt 3420

cctccaggtc cccctggccc tggcatcgac atgtccgcct ttgctggctt aggcccgaga 3480

gagaagggcc ccgacccccct gcagtacatg cgggccgacc aggcagccgg tggcctgaga 3540

cagcatgacg ccgaggtgga tgccacactc aagtccctca acaaccagat tgagagcatc 3600

cgcagccccg agggctcccg caagaaccct gctcgcacct gcagagacct gaaactctgc 3660

caccctgagt ggaagagtgg agactactgg attgacccca accaaggctg caccttggac 3720

gccatgaagg ttttctgcaa catggagact ggcgagactt gcgtctaccc caatccagca 3780

aacgttccca agaagaactg gtggagcagc aagagcaagg agaagaaaca catctggttt 3840

ggagaaacca tcaatggtgg cttccatttc agctatggag atgacaatct ggctcccaac 3900

actgccaacg tccagatgac cttcctacgc ctgctgtcca cggaaggctc ccagaacatc 3960

acctaccact gcaagaacag cattgcctat ctggacgaag cagctggcaa cctcaagaag 4020

gccctgctca tccagggctc caatgacgtg gagatccggg cagagggcaa tagcaggttc 4080

acgtacactg ccctgaagga tggctgcacg aaacataccg gtaagtgggg caagactgtt 4140

atcgagtacc ggtcacagaa gacctcacgc ctccccatca ttgacattgc acccatggac 4200

ataggagggc ccgagcagga attcggtgtg gacatagggc cggtctgctt cttgtaa 4257

<210> 112
<211> 335
<212> DNA
<213> human

<400> 112
agacagagac gtgtacagtg gcccccgtg aaagacagaa ttgtggtttt cctggtgtca 60

cgccctccca gtgtgcaaat aagggctgct gtttcgacga caccgttcgt ggggtcccct 120

ggtgcttcta tcctaatacc atcgacgtcc ctccagaaga ggagtgtgaa ttttagacac 180

ttctgcaggg atctgcctgc atcctgacgg ggtgccgtcc ccagcacggt gattagtccc 240

agagctcggc tgccacctcc accggacacc tcagacacgc ttctgcagct gtgcctcggc 300

tcacaacaca gattgactgc tctgactttg actac 335

<210> 113
<211> 329
<212> DNA
<213> human

<400> 113
tgctgaggag tacgtgggcc tgtctgcaaa ccagtgtgcc gtgccggcca aggacagggt 60

ggactgcggc tacccccatg tcacccccaa ggagtgcaac aaccggggct gctgctttga 120

ctccaggatc cctggagtgc cttggtgttt caagcccctg actaggaaga cagaatgcac 180

cttctgaggc acctccagct gcccctggga tgcaggctga gcacccttgc ccggctgtga 240

ttgctgccag gcactgttca tctcagtttt tctgtccctt tgctcccggc aagctttctg 300

ctgaaagttc atatctggag cctgatgtc 329

<210> 114
<211> 348
<212> DNA
<213> human

<400> 114
catcacttgg atgagtgccc acacagtcaa gctttaaaga aagtgtttgc tgaaaataaa 60

gaaatccaga aattggcaga gcagtttgtc ctcctcaatc tggtttatga aacaactgac 120

aaacaccttt ctcctgatgg ccagtatgtc cccaggatta tgtttgttga cccatctctg 180

acagttagag ccgatatcac tggaagatat tcaaatcgtc tctatgctta cgaacctgca 240

gatacagctc tgttgcttga caacatgaag aaagctctca agttgctgaa gactgaattg 300

taaagaaaaa aaatctccaa gcccttctgt ctgtcaggcc ttgagact 348

<210> 115
<211> 496
<212> DNA
<213> human

<400> 115
gatggggttc actgtttggt gggcttcacc ctcacccata ggagattcaa ttataaggac 60

aatacagatc taatagagtt caagactctg agtgaggaag aaatagaaaa agtgctgaaa 120

aatatattta atatttcctt gcagagaaag cttgtgccca aacatggtga tagatttttt 180

actatttaga ataaggagta aaacaatctt gtctatttgt catccagctc accagttatc 240

aactgacgac ctatcatgta tcttctgtac ccttacctta ttttgaagaa aatcctagac 300

atcaaatcat ttcacctata aaaatgtcat catatataat taaacagctt tttaaagaaa 360

cataaccaca aacctttca aataataata ataataataa taataaatgt cttttaaaga 420

tggcctgtgg ttatcttgga aattggtgat ttatgctaga aagcttttaa tgttggttta 480

ttgttgaatt cctaga 496

<210>  116
<211>  193
<212>  DNA
<213>  human

<400>  116
gtcccaagtg caacaaggag gtgtacttcg ccgagagggt gacctctctg ggcaaggact    60

ggcatcggcc ctgcctgaag tgcgagaaat gtgggaagac gctgacctct gggggccacg   120

ctgagcacga aggcaaaccc tactgcaacc acccctgcta cgcagccatg tttgggccta   180

aaggctttgg gcg                                                        193

<210>  117
<211>  526
<212>  DNA
<213>  human

<220>
<221>  misc_feature
<222>  (41)..(41)
<223>  n is a, c, g, or t

<220>
<221>  misc_feature
<222>  (54)..(62)
<223>  n is a, c, g, or t

<220>
<221>  misc_feature
<222>  (76)..(76)
<223>  n is a, c, g, or t

<220>
<221>  misc_feature
<222>  (82)..(82)
<223>  n is a, c, g, or t

<220>
<221>  misc_feature
<222>  (96)..(96)
<223>  n is a, c, g, or t

<220>
<221>  misc_feature
<222>  (105)..(106)
<223>  n is a, c, g, or t

<220>
<221>  misc_feature
<222>  (120)..(120)
<223>  n is a, c, g, or t

<220>
<221> misc_feature
<222> (123)..(123)
<223> n is a, c, g, or t

<220>
<221> misc_feature
<222> (239)..(239)
<223> n is a, c, g, or t

<220>
<221> misc_feature
<222> (500)..(500)
<223> n is a, c, g, or t

<400> 117
gttgtaacaa tatctatgac cactgttagc ccattatatt ncattccaat tagnnnnnnn 60

nnaatgtgaa tactanattc cngtgttttg agtgancaag tttcnnaaaa taaaaacacn 120

gtnttttttaa aagggaaatg cacttaaatg aaaacagtta ttacaaaagt taagatttaa 180

aaagaaaaag caagagtttt tattatgatg taataccagt agaatattta aaaggcacnc 240

cacatctgaa taatcaatgt aaatattttc tttcaaagtt gtaagttttc atatcatgtg 300

ctgtaaagtt ttcctaaatg aggctttaac gtaaacactg gtgacataaa ccattcattg 360

ctacgttgct tattgtgttt ttatgctgtt ttatactttt ttatgagtta tgatagcagc 420

aattaagttg tttgtatttt gcttaactaa aacaaaaatg cttttatctt gctatagaat 480

aaacacattt cagtaaaaan tgtggactgt attttgatgc aacaac 526

<210> 118
<211> 521
<212> DNA
<213> human

<220>
<221> misc_feature
<222> (417)..(417)
<223> n is a, c, g, or t

<400> 118
gaaggtagaa acgatgtctc tccacagtcg aatagattgt ttccacaaac tgtctgtagg 60

tgcatacaat ggaataggat taatccgtga aaaggaagaa attctgacac atgctccggc 120

gtggaggaac cttgaggacg acgctgagtg aaagaagcca gacacgaaag gacagatacg 180

gcaagactcg acttatgtga ggtccctaga ggagtcggat tcatagagac tgaaaggatg 240

gcgggcacca ggggcggtgg agaggggaaa tggggagtta ctgtttaatg ggacagagt 300

ttcagtttta caagatgaaa agggtcctgg agctggatgg gggtgacggc cgcacaacag 360

catgaatgga ctgaacgcca ctgaaaactg aactcttgaa aatggttaaa ctggcanatt 420

ttgttatgca tattttgcca caaaaagcaa acaactcttc tccccaaaaa tatctggggt 480

gtacttacac taaatatgta gggcatattt actctcaaca t 521

<210> 119
<211> 509
<212> DNA
<213> human

<400> 119
gacgactgtg gggactatct ggtgatgcgg aaaacctctt catccaattc tgtgacaaca 60

tatgaaacct gccagaccta cgaacgcccc atcgccttca cctccaggtc aaagaagctg 120

tggattcagt tcaagtccaa tgaagggaac agcgctagag ggttccaggt cccatacgtg 180

acatatgatg aggactacca ggaactcatt gaagacatag ttcgagatgg caggctctat 240

gcatctgaga accatcagga aatacttaag gataagaaac ttatcaaggc tctgtttgat 300

gtcctggccc atccccagaa ctatttcaag tacacagccc aggagtcccg agagatgttt 360

ccaagatcgt tcatccgatt gctacgttcc aaagtgtcca ggtttttgag accttacaaa 420

tgactcagcc cacgtgccac tcaatacaaa tgttctgcta tagggttggt gggacagagc 480

tgtcttcctt ctgcatgtca gcacagtcg 509

<210> 120
<211> 552
<212> DNA
<213> human

<220>
<221> misc_feature
<222> (35)..(35)
<223> n is a, c, g, or t

<220>
<221> misc_feature
<222> (45)..(45)
<223> n is a, c, g, or t

<220>
<221> misc_feature
<222> (50)..(51)
<223> n is a, c, g, or t

<220>
<221> misc_feature

<222> (58)..(59)
<223> n is a, c, g, or t

<220>
<221> misc_feature
<222> (63)..(63)
<223> n is a, c, g, or t

<220>
<221> misc_feature
<222> (74)..(76)
<223> n is a, c, g, or t

<220>
<221> misc_feature
<222> (85)..(86)
<223> n is a, c, g, or t

<220>
<221> misc_feature
<222> (91)..(91)
<223> n is a, c, g, or t

<220>
<221> misc_feature
<222> (95)..(95)
<223> n is a, c, g, or t

<220>
<221> misc_feature
<222> (100)..(100)
<223> n is a, c, g, or t

<220>
<221> misc_feature
<222> (105)..(105)
<223> n is a, c, g, or t

<220>
<221> misc_feature
<222> (109)..(109)
<223> n is a, c, g, or t

<220>
<221> misc_feature
<222> (124)..(124)
<223> n is a, c, g, or t

<220>
<221> misc_feature
<222> (129)..(130)
<223> n is a, c, g, or t

<220>
<221> misc_feature
<222> (152)..(152)
<223> n is a, c, g, or t

<220>
<221> misc_feature
<222> (160)..(160)
<223> n is a, c, g, or t

<220>
<221> misc_feature
<222> (174)..(174)
<223> n is a, c, g, or t

<220>
<221> misc_feature
<222> (190)..(190)
<223> n is a, c, g, or t

<220>
<221> misc_feature
<222> (201)..(201)
<223> n is a, c, g, or t

<220>
<221> misc_feature
<222> (212)..(212)
<223> n is a, c, g, or t

<220>
<221> misc_feature
<222> (222)..(222)
<223> n is a, c, g, or t

<220>
<221> misc_feature
<222> (236)..(236)
<223> n is a, c, g, or t

<400> 120
tctccaaatc aaaccacagt atatgttgta acaantatct atgancactn ntagcccnnt 60

atnttcattc caannngaag aaaannaatg ntgantactn tattnccgng ttttgagtga 120

caantttcnn aaaataaaaa cactgtattt tnaaaagggn aaatgcactt aaangaaaac 180

agttattacn aaaagttaag natttaaaaa gnaaaagcaa gnagtttttta ttatgnatgt 240

aataccagta gaatatttaa aaggcacacc acatctgaat aatcaatgta aatattttct 300

ttcaaagttg taagttttca tatcatgtgc tgtaaagttt tcctaaatga ggctttaacg 360

taaacactgg tgacataaac cattcattgc tacgttgctt attgtgtttt tatgctgttt 420

tatacttttt tatgagttat gatagcagca attaagttgt ttgtattttg cttaactaaa 480

acaaaaatgc ttttatcttg ctatagaata aacacatttc agtaaaaact gtggactgta 540

ttttgttgca ac 552

<210> 121
<211> 184
<212> DNA
<213> human

<400> 121
gctatttttg aggttcgtgc ctgttgtaga ccacagtcac acactgctgt agtcttcccg 60

agtcctcatt cccagctgcc tcttcctact gcttccgtct atcaaaaagc ccccttggcc 120

caggttccct gagctgtggg attctgcact ggtgctttgg attccctgat atgttccttc 180

aaat 184

<210> 122
<211> 382
<212> DNA
<213> human

<400> 122
ggaggcagca acggagagtg gggaagtgga gcggcagctc ccttgggggc ttagcccagg 60

tgcttcgtaa ctgcaatcgg aagtgcagga gctggtcaga gccaatgaga aggaaacctc 120

atctttgcat agcccatgcc tcatggagag gtgacatcat acattcacat gcttctcacc 180

taagtcccca gggtccaagg gagaagcccc agaccccctt ctcttgcaga gtgtgggggt 240

ggtggtgctg caggggcagg gctgggtggg ggtcaccaga ctttttctgc ccttagggta 300

gtacagctgg catttgtttt atagactctt gtctttggaa ttggggggag gggggagtg 360

tttcaatctg ttatatgttc tg 382

<210> 123
<211> 564
<212> DNA
<213> human

<400> 123
cccctacgca ccaggatgga cgtggtgcac ctccaggagc agttagactt aaagctgcag 60

cagcggcagg ccagggaaac aggcatctgc cctgtccgca ggaactctac tcacagtgtt 120

ttgatgagtt gatccgggag tcaccatcaa ctgtgcggag agggggctgc tgctgctgca 180

gtcgggacga gatccgcatg accatcgctg cctaccagac cctgtacgag agcagcgtgg 240

cgtttggcat gaggaaggca ctgcaggctg agcaggggaa gtcagacatg gagaggaaaa 300

tcgcagaatt ggagacggaa aagagagacc tggagaggca agtgaacgag cagaaggcaa 360

aatgtgaagc cactgagaag cgggagagcg agaggcggca ggtggaggag aagaagcaca 420

atgaggagat tcagttcctg aagcgaacaa atcagcagct gaaggcccaa ctggaaggca 480

ttattgcacc aaagaagtga taatttccac atgattaatt tccaacaaga cacttgggag 540

ttatttactg tgttcctctg gcag 564

<210> 124
<211> 515
<212> DNA
<213> human

<400> 124
aagatttcac ttcattgtct agcccagaat cttgagcaag ctaaagaaac catcataatc 60

taaaattgct tcatttaaca ctaacaattt agacttttta aaccaagcat tgaataatgg 120

ctggataact gccgaagtaa gcgccgctcc atgaagtctg cttacttatt taaaaattgt 180

gtatcagttt taaatactgt tcattgtgtg cagatataag gggaataggg cattctgtag 240

aattatacat gtctagtttg taaagtgtgt cctgtgtact gcagatgtgt gttctctggg 300

ctttatgtat ctgtacagta gctttcacat taaaaaaatt gtggacaaac ttgtccgggg 360

ggtttgaggg gagaatggtg gtttatatca ataacgatgc tgtactatag tccatgtaac 420

aaaagatctg gaagtcaccc tcctctggcc cacggaaaat tttggtaatc ttctaggttc 480

taaaatgaag atgtatgggt actctggcag actgc 515

<210> 125
<211> 466
<212> DNA
<213> human

<400> 125
atatctatga ccactgttag cccattatat tcattccaat tagaagaaaa gaaatgtgaa 60

tactatattc cgtgttttga gtgacaagtt tcgaaaaata aaaacactgt atttttaaaa 120

gggaaatgca cttaaatgaa aacagttatt acaaaagtta agatttaaaa agaaaaagca 180

agagttttta ttatgatgta ataccagtag aatatttaaa aggcacacca catctgaata 240

atcaatgtaa atattttctt tcaaagttgt aagttttcat atcatgtgct gtaaagtttt 300

cctaaatgag gctttaacgt aaacactggt gacataaacc attcattgct acgttgctta 360

ttgtgttttt atgctgtttt atactttttt atgagttatg atagcagcaa ttaagttgtt 420

tgtattttgc ttaactaaaa caaaaatgct tttatcttgc tataga 466

<210> 126
<211> 495

<212> DNA
<213> human

<400> 126
gccggtgaga tgctctatct gccggctctg tggttccacc acgtccagca gtcccagggc 60

tgcatcgcag tgaatttctg gtatgacatg gaatacgacc tcaagtatag ttacttccag 120

ctgctcgact ccctcaccaa ggcttcaggc cttgactgat ggagcactgg tgaacaccac 180

caagcacgcc tcgggggacg gagccagccc ccccctggcc aggtcgagag agcctggagt 240

gtgcatgctg gctgctggcc ccgggtccag catggcttga gatcagcttt ggaggatctt 300

ggaatgtggt cataaggact caaggtgcca ggcaggtctg ggtgagggtt ctcaggaagt 360

tgccacacag gtgagcagag tggggatcag gtgcagcggc acctctcccc agcgctgtga 420

tgttgggcga gtcactgcgt ctcgggcatt ggtgtcctgt cagtaaagag ataataatgg 480

ctgtacctcg cgggg 495

<210> 127
<211> 499
<212> DNA
<213> human

<400> 127
tccgaagctc tggaagcgca agccctggcg cgggggtgg cagagctgag tctggccgac 60

tatgccttca ccagctactc cccttccctc ctggcgatct gctgcctggc gctggcggac 120

cgcatgctgc gggtctcgcg gcccgtggac ttgcgactgg gagaccaccc ggaggcggcg 180

ctggaggact gtatgggcaa gttgcagctg ctggtggcca taaacagtac ttccttgact 240

cacatgctgc ccgttcagat ctgcgagaag tgcagcctgc ccccgagctc gaaataaaac 300

agatccttcg tttcctttta gtccctggcc cggctgctgg acctctcccg tagcctcaga 360

agagtgcagt actggtccac agagaaggct tcaggacctg cttggtcagc tgcaggttgt 420

aaatagtgta cgatactagc atctggtatt ttatttattt tgcagcgagc acatgaggaa 480

gctgagtctt tcaccaata 499

<210> 128
<211> 417
<212> DNA
<213> human

<400> 128
tggtgacaca aacagccttt tcccaggtac ttggtacctg gagcgagtgg acgagcagca 60

tcgccgaaag tatgcccggc gtcccgtcta aaggtgttct gcagatccat ggaaagcttc 120

```
ctgggaaacg tatgctagca gagcttctcc ccgtgaatca tatttttaag atcccactct    180

tagctggtaa atgaatttga atcgacatag tagccccata agcatcagcc ctgtagagtg    240

aggagccatc tctagcgggc ccttcattcc tctccatgct gcaatcactg tcctgggctt    300

atggtgccta tggactaggg gtcctttgtg aaagagcaag atggagcaat ggagagaaga    360

cctcttcctg aatcactgga ctccagaaat gtgcatgcag atcagctgtt gccttca       417


<210>  129
<211>  468
<212>  DNA
<213>  human


<220>
<221>  misc_feature
<222>  (91)..(91)
<223>  n is a, c, g, or t

<220>
<221>  misc_feature
<222>  (96)..(96)
<223>  n is a, c, g, or t

<400>  129
gtggcaagct accaactaag ttgtatttta ataaagattc catgggttga acaagccacg     60

ttgcagaaaa agagcttccc ctaacctggg nttgtngcag agtaaatccc acgacataag    120

ctggtatcag tggttcgggg gaaatagttc cattctatga ctcttgtctc ctcctccagg    180

aggactgttc taactagtaa tcttggccct attcattaca tcctctgctt gtcattctgc    240

taatttatga agatagttta ttatagtctg tacttcagtt ctcatcttgt aaataatgct    300

taacataaac ttgtacttac actgaaatcc aaaatagtca tgtttctgca gtattctgta    360

gccaacttaa acctgtgctt tcatgtttaa gaaatgagaa attgtgccaa agatagcaga    420

agagtagata agtgctcagt attgacgacc tacatctgaa atctacaa                 468


<210>  130
<211>  213
<212>  DNA
<213>  human

<400>  130
attccagtct acttgagtta gcataataca gaagtcccct ctactttaac ttttacaaaa     60

aagtaacctg aactaatctg atgttaacca atgtatttat ttctgtggtt ctgtttcctt    120

gttccaattt gacaaaaccc actgttcttg tattgtattg cccaggggga gctatcactg    180
```

tacttgtaga gtggtgctgc tttaattcat aaa 213

<210> 131
<211> 397
<212> DNA
<213> human

<220>
<221> misc_feature
<222> (86)..(89)
<223> n is a, c, g, or t

<220>
<221> misc_feature
<222> (109)..(109)
<223> n is a, c, g, or t

<220>
<221> misc_feature
<222> (112)..(112)
<223> n is a, c, g, or t

<220>
<221> misc_feature
<222> (149)..(149)
<223> n is a, c, g, or t

<400> 131
ccaatatatt tctctctcca caaattaaga tattgttttc ttaagagcat agccttgcac 60

ccacagtctt gtttttggag tgtgtnnnng ctacttatat gatgacctng cnttgaacgt 120

ctctcacctg gctcaatgcc aggctgcana ggtatcactc attgctttca actcaggatg 180

aagagcgcga gcttcactgt gtacagagtc atctccgagg gattacacag agtacaacaa 240

aaggagaagg ctcagtcctt gacataagca aagatatctg cggacagttg actgtaggat 300

tgtaaagtct cataactgcc aggcatagtc gtcacgcctc taatcccagc acttagagag 360

gcagaggcaa gaagatagcg taagcccagc agttcaa 397

<210> 132
<211> 553
<212> DNA
<213> human

<400> 132
tacaccacaa tggctgagca cttcctgacg ttgctggtag tgcctgccat caagaaagat 60

tatggttccc aggaagactt cactcaagtg tggaacacca ccatgaaagg gctcaagtgc 120

tgtggcttca ccaactatac ggattttgag gactcaccct acttcaaaga gaacagtgcc 180

tttcccccat tctgttgcaa tgacaacgtc accaacacag ccaatgaaac ctgcaccaag 240

caaaaggctc acgaccaaaa agtagagggt tgcttcaatc agcttttgta tgacatccga 300

actaatgcag tcaccgtggg tggtgtggca gctggaattg ggggcctcga gctggctgcc 360

atgattgttt ccatgtatct gtactgcaat ctacaataag tccacttctg cctctgccac 420

tactgctgcc acatgggaaa ctgtgaagag gcaccctggg caagcagcag tgattggggg 480

aggggacagg atctaacaat gtcacttggg ccagaatgga cctggccttt ctgctcccag 540

acttgggggc tag 553

<210> 133
<211> 495
<212> DNA
<213> human

<400> 133
gttgagcctg aaaatgtcac catccaatgt gattctggct atggtgtggt tggtccccaa 60

agtatcactt gctctgggaa cagaacctgg tacccagagg tgcccaagtg tgagtgggag 120

acccccgaag gctgtgaaca agtgctcaca ggcaaaagac tcatgcagtg tctcccaaac 180

ccagaggatg tgaaaatggc cctggaggta tataagctgt ctctggaaat tgaacaactg 240

gaactacaga gagacagcgc aagacaatcc actttggata aagaactata atttttctca 300

aaagaaggag gaaaaggtgt cttgctggct tgcctcttgc aattcaatac agatcagttt 360

agcaaatcta ctgtcaattt ggcagtgata ttcatcataa taaatatcta gaaatgataa 420

tttgctaaag tttagtgctt tgagattgtg aaattattaa tcatcctctg tgtggctcat 480

gtttttgctt ttcaa 495

<210> 134
<211> 541
<212> DNA
<213> human

<220>
<221> misc_feature
<222> (93)..(93)
<223> n is a, c, g, or t

<220>
<221> misc_feature
<222> (106)..(106)
<223> n is a, c, g, or t

```
<220>
<221>  misc_feature
<222>  (116)..(116)
<223>  n is a, c, g, or t

<220>
<221>  misc_feature
<222>  (147)..(147)
<223>  n is a, c, g, or t

<220>
<221>  misc_feature
<222>  (150)..(150)
<223>  n is a, c, g, or t

<220>
<221>  misc_feature
<222>  (161)..(256)
<223>  n is a, c, g, or t

<220>
<221>  misc_feature
<222>  (258)..(258)
<223>  n is a, c, g, or t

<220>
<221>  misc_feature
<222>  (267)..(267)
<223>  n is a, c, g, or t

<220>
<221>  misc_feature
<222>  (276)..(305)
<223>  n is a, c, g, or t

<220>
<221>  misc_feature
<222>  (367)..(367)
<223>  n is a, c, g, or t

<220>
<221>  misc_feature
<222>  (393)..(393)
<223>  n is a, c, g, or t

<220>
<221>  misc_feature
<222>  (470)..(470)
<223>  n is a, c, g, or t

<400>  134
tctatctggt ccagggcacc caggtatatg tcttcctgac aaagggaggc tataccctag     60

taagcggtta tccgaagcgg ctggagaagg aantcgggac ccctcnatgg gattancctg    120

gactctgtgg atgcggcctt tatctgnccn tgggtcttct nnnnnnnnnn nnnnnnnnnn    180
```

nnnnnnnnnn nnnnnnnnnn nnnnnnnnnn nnnnnnnnnn nnnnnnnnnn nnnnnnnnnn 240

nnnnnnnnnn nnnnnntnag aaggtanacg gagccnnnnn nnnnnnnnnn nnnnnnnnnn 300

nnnnnactca tgttccgcca atggtcccgg cttgtacctc atccatggtc ccaatttgta 360

ctgctanagt gatgtggaga aactgaatgc agncaaggcc cttccgcaac cccagaatgt 420

gaccagtctc ctgggctgca ctcactgagg ggccttctga catgagtctn gcctggcccc 480

acctcctagt tcctcataat aaagacagat tgcttcttcg cttctcactg aggggccttc 540

t 541

<210> 135
<211> 563
<212> DNA
<213> human

<220>
<221> misc_feature
<222> (121)..(122)
<223> n is a, c, g, or t

<220>
<221> misc_feature
<222> (133)..(133)
<223> n is a, c, g, or t

<220>
<221> misc_feature
<222> (149)..(149)
<223> n is a, c, g, or t

<220>
<221> misc_feature
<222> (340)..(340)
<223> n is a, c, g, or t

<400> 135
ggttggctgg tcactgatgg ttataatgac tgtgggacag gattaacttc agaataaatg 60

aacaggagac acagatgtga agaaagtttc tgattgatat ggtctgaagt actcctggta 120

nngcaagtca ttngctctaa ttctcaatng taggcaaact gatttgtaaa tttgcttctt 180

cagccttctt tcctgtagcc tagcatggag aatctgacca gaccccattt tgagaaggtc 240

agcctacact ggaatgaact ttttacatta gggcatttgt atttccctca caatacttgc 300

cacattactt ggcataggag agatgcttag tgtaattatn agttaacaag cctttggatc 360

agggcttgac tcatgataga caaagtatat gcctgctgga tggaagaatc tcttgggcga 420

gcaccatttt tctttccatc acctttcctt gaaaatatat cttcagcttt gggtaggagg 480

aatcttggtg tatgaaatca ttgcaaattt acttcatctt ttctggagtt tgaagttgtg 540

actctcctgc taccaattaa ata 563

<210> 136
<211> 515
<212> DNA
<213> human

<400> 136
gttttaattc aacccagcca tgcaatgcca aataatagaa ttgctcccta ccagctgaac 60

agggaggagt ctgtgcagtt tctgacactt gttgttgaac atggctaaat acaatgggta 120

tcgctgagac taagttgtag aaattaacaa atgtgctgct tggttaaaat ggctacactc 180

atctgactca ttctttattc tattttagtt ggtttgtatc ttgcctaagg tgcgtagtcc 240

aactcttggt attaccctcc taatagtcat actagtagtc atactccctg gtgtagtgta 300

ttctctaaaa gctttaaatg tctgcatgca gccagccatc aaatagtgaa tggtctctct 360

ttggctggaa ttacaaaact cagagaaatg tgtcatcagg agaacatcat aacccatgaa 420

ggataaaagc cccaaatggt ggtaactgat aatagcacta atgctttaag atttggtcac 480

actctcacct aggtgagcgc attgagccag tggtg 515

<210> 137
<211> 378
<212> DNA
<213> human

<400> 137
satttactta gtccttaggc caaccaattt aactgcagtg tcatgtttca caggccttcc 60

tacatttaga aatcgtcaca cagctgtgat aagagtagat tattttacta tgaaataatt 120

ctgaatagat gaaagcataa aatgtgagaa actgaatgta ttattcagga agaatactga 180

gtgccttcat ttaactaaag ttgaatgtaa aagtcaattt gcacttcttt ataatcctct 240

ggtttagaat tataaattgt taaaaccttg ataattgtca tttaattata tttcaggtgt 300

cctgaacagg tcactagact ctacattggg cagcctttaa atatgattct ttgtaatgct 360

aaatagcctt tttttctc 378

<210> 138
<211> 398
<212> DNA
<213> human

<400> 138
atgcagcagc ctcaccatga agttgctgat ggtcctcatg ctggcggccc tctcccagca 60

ctgctacgca ggctctggct gccccttatt ggagaatgtg atttccaaga caatcaatcc 120

acaagtgtct aagactgaat acaaagaact tcttcaagag ttcatagacg acaatgccac 180

tacaaatgcc atagatgaat tgaaggaatg ttttcttaac caaacggatg aaactctgag 240

caatgttgag gtgtttatgc aattaatata tgacagcagt ctttgtgatt tattttaact 300

ttctgcaaga cctttggctc acagaactgc agggtatggt gagaaaccaa ctacggattg 360

ctgcaaacca caccttctct ttcttatgtc tttttact 398

<210> 139
<211> 159
<212> DNA
<213> human

<400> 139
tcactgagca ccacattctc tagcttcttg ttgaggctgg aactgtttct ttaaaatccc 60

ttaattttcc catctcaaaa ttatatctgt acctgggtca tccagctcct tcttgggtgt 120

ggggaaatga gttttctttg atagtttctg cctcactca 159

<210> 140
<211> 284
<212> DNA
<213> human

<400> 140
aggcagtggc aagtaagtta tagaagagaa atgctgctag aaggaattaa gcgttgtagt 60

aaacgcgtgc tcatcctcta agcttgaaga agggagacga aaatccattt gtttaaattc 120

acatctcaag gagggagaac ccgggctgtg ttgggtggtt gccaatttcc tagaacggaa 180

tgtgtggggt atagaaaaag gaatgaataa gcgttgtttt tcaaataggg tccttgtaag 240

ttattgatga gagggaaaag attgactggg gagggcttaa aatg 284

<210> 141
<211> 450
<212> DNA
<213> human

<400> 141
agctctatgg ttaccaggct cagcattatg tctgtatgaa gcatgttgac ggcacctgca 60

gctggtaccg gggccacctg cctctcagga aggagtttgt tgacatcgtt cagccctagt 120

agggaccagt gaccatcaca tcccttcaag agtcctgaag atcaagccag ttctccttcc 180

ctgcagagct ttggccatta ccacctgacc tcttgctgcc agctaataag aagtgccaag 240

tggacagtct ggccactgtc aaggcaggga aggggccatg acttttctgc cctgccctca 300

gcctgttgcc ctgcctccca aaccccatta gtctagcctt gtagctgtta ctgcaagtgt 360

ttcttctggc ttagtctgtt ttctaaagcc aggactattc cctttcctcc ccaggaatat 420

gtgttttcct ttgtcttaat cgatctggta 450

<210> 142
<211> 448
<212> DNA
<213> human

<400> 142
tttcctataa acagaatcac acaatatttg attttttttt taaaactaag ccttgctctg 60

tctcccaggc tggagtgctg tggcgtgatt ttggttcact gcaacctccg ccttccaagt 120

tcaagagatt ctcctgcctc agcttccaag tagctgggat tacaggcatg tggtaccacg 180

cctggctaat ttcttgtat ttttagtagg gacatgttgg ccaggctggt tgtgagctcc 240

tggcctcagg tgatccacac gcctcagtgt cccagagtgc tgatattaca ggcgtaatat 300

gtgatctttt gtgtctggtt cctttcacgt tgaacgctat ttttgaggtt cgtgcctgtt 360

gtagaccaca gtcacacact gctgtagtct tcccccatcc tcattcccag ctgcctcctc 420

ctactgtttc cctctatcaa aaagcctc 448

<210> 143
<211> 344
<212> DNA
<213> human

<400> 143
ctcatgcgaa acattgtcag ggtggtcgtc ctggacaaag tcacagacct gctgctgttc 60

tttgggaagc tgctggtggt cggaggcgtg ggggtcctgt ccttcttttt tttctccggt 120

cgcatcccgg ggctgggtaa agactttaag agcccccacc tcaactatta ctggctgccc 180

atcatgacct ccatcctggg ggcctatgtc atcgccagcg gcttcttcag cgttttcggc 240

atgtgtgtgg acacgctctt cctctgcttc ctggaagacc tggagcggaa caacggctcc 300

ctggaccggc cctactacat gtccaagagc cttctaaaga ttct 344

<210> 144
<211> 523
<212> DNA

<213> human

<400> 144
cggagaggac cagcgactac taccgcgtga gcgcggacct gccgggcagg ttcaacaacc 60

cggggtggtt ccggggctac aggacccaga aggctgtctc cgtgtacagg accagtaacc 120

aggcttacgg gagcagagcc cccaccgtgc acgagatgcc taaagtattt tatccaaatt 180

cgaataaatt ttcccaacaa cttgcagcgg gtggaatgtt ccggaacaat actctcaatg 240

tttacctgga gaaaagcatc gtgactggcc ccgataactg catcacctcc tgtgaccggc 300

tcaacttcca ccccagttac aacatcaaca ggccatccat ctgcgattga ggggctggaa 360

actccctcta ggagtcctct gaccccaggg cctcctggaa aatcatcatc cctcatcacc 420

cacagtcgcc cgattgtgac ggcacagact gcgctgcctt ccctgaaaat attattcgtc 480

tgcggagtag gaaaatatgc aaacggggcc gtgaagtcct gcc 523

<210> 145
<211> 311
<212> DNA
<213> human

<400> 145
aatgaatgtt ctcctgggca gcgttgtgat ctttgccacc ttcgtgactt tatgcaatgc 60

atcatgctat ttcataccta atgagggagt tccaggagat tcaaccagga tgtttctaca 120

cctgtgggtt atgacaaaga caactgccaa agaatcttca agaaggagga ctgcaagtat 180

atcgtggtgg agaagaagga cccaaaaaag acctgttctg tcagtgaatg gataatctaa 240

tgtgcttcta gtaggcacag ggctcccagg ccaggcctca ttctccctgg cctctaatag 300

tcaatgattg t 311

<210> 146
<211> 390
<212> DNA
<213> human

<400> 146
gaaggtgtgg ttttcatttc tcagtcacca acagatgaat aattatgctt aataataaag 60

tatttattaa gactttcttc agagtatgaa agtacaaaaa gtctagttac agtggattta 120

gaatatattt atgttgatgt caaacagctg agcaccgtag catgcagatg tcaaggcagt 180

taggaagtaa atggtgtctt gtagatatgt gcaaggtagc atgatgagca acttgagttt 240

gttgccactg agaagcaggc gggttgggtg ggaggaggaa gaaagggaag aattaggttt 300

gaattgcttt ttaaaaaaaa aagaaaagaa aaagacagca tctcactatg ttgccaaggc 360

tcatcttgag aagcaggcgg gttgggtggg 390

<210> 147
<211> 396
<212> DNA
<213> human

<400> 147
attcttctaa ttgctgtgtg tcccaggcag ggagacggtt tccagggagg ggccggccct 60

gtgtgcaggt tccgatgtta ttagatgtta caagtttata tatatctata tatataattt 120

attgagtttt tacaagatgt atttgttgta gacttaacac ttcttacgca atgcttctag 180

agttttatag cctggactgc tacctttcaa agcttggagg gaagccgtga attcagttgg 240

ttcgttctgt actgttactg ggccctgagt ctgggcagct gtcccttgct tgcctgcagg 300

gccatggctc agggtggtct cttcttgggg cccagtgcat ggtggccaga ggtgtcaccc 360

aaaccggcag gtgcgatttt gttaacccag cgacga 396

<210> 148
<211> 523
<212> DNA
<213> human

<400> 148
aacagagatg tcccccaggg agcacatcaa gggcaaagag accaccccct ctagcctagc 60

agtgacccag accatggcca ccaaagctcc cgagtgtgtg gaggacccag atatggcaaa 120

ccagaggaag actgccctgg agttctgtgg agagacttgg agctctctct gcacattctt 180

cctcagcata gtgcaggaca cgtcatgcta atgaggtcaa aagagaacgg gttcctttaa 240

gagatgtcat gtcgtaagtc cctctgtata ctttaaagct ctctacagtc cccccaaaat 300

atgaactttt gtgcttagtg agtgcaacga aatatttaaa caagttttgt attttttgct 360

tttgtgtttt ggaatttgcc ttatttttct tggatgcgat gttcagaggc tgtttcctgc 420

agcatgtatt tccatggccc acacagctat gtgtttgagc agcgaagagt ctttgagctg 480

aatgagccag agtgataatt tcagtgcaac gaactttctg ctg 523

<210> 149
<211> 568
<212> DNA
<213> human

<400> 149

tgcatgttca tctgaggtgc cacatgggcc agttgatcag aagtttcaat ccatagtaat 60

tggctgtgct cttgaagatc agaagaaaat taagagaaga ttagagactc tgcttagaaa 120

tattgaaaac tctgacaagg ccatcaagct attagagcat tctaaaggag ctggttccaa 180

aactctgcaa caaaatgctg aaagcagatt caattagtct tcaaacctaa gagcatttac 240

acaatacaca aggtgtaaaa atgataaaat actattttaa ttgataacta gttctttgtt 300

aggtataacc acttagttga cactgatagt tgtttcagat gaggaaaata ttccatcaag 360

tatcttcagt tttgtgaata acaaaactag caatatttta attatctatc tagagatttt 420

ttagattgaa ttcttgtctt gtactaggat ctagcatatt tcactattct gtggatgaat 480

acatagtttg tggggaaaac aaacgttcag ctaggggcaa aaagcatgac tgctttttcc 540

tgtctggcat ggaatcacgc agtcacct 568

<210> 150
<211> 494
<212> DNA
<213> human

<400> 150
agaattcccg ccggaacgtg atgcttcgca atgatccgat gatgtccttt gctgcccagc 60

ctaaccaggg aagtgtggtc aatcagaact tgctccacca ccagcaccaa acccagggcg 120

ctcttggtgg cagccgtgcc ttgtcgaatt ctgtcagcaa catgggcttg agtgagtcca 180

gcagccttgg gtcagccaaa caccagcagc agtctcctgt cagccagtct atgcaaaccc 240

tctcggactc tctctcaggc tcctccttgt actcaactag tgcaaacctg cccgtcatgg 300

gccatgagaa gttccccagc gacttggacc tggacatgtt caatgggagc ttggaatgtg 360

acatggagtc cattatccgt agtgaactca tggatgctga tgggttggat tttaactttg 420

attccctcat ctccacacag aatgttgttg gtttgaacgt ggggaacttc actggtgcta 480

agcaggcctc atct 494

<210> 151
<211> 541
<212> DNA
<213> human

<400> 151
gacgttgaaa cgcagcattt ttgaaaaggg agaaagactc ggacaggtgc tatcgaaaaa 60

taagatccat tctctattcc cagtataagg gacgaaactg cgaactcctt aaagctctat 120

ctagccaaac cgcttacgac cttgtatata tttaatttca ggtaaggaaa acacatacgt 180

gtagcgatct ctatttgctg gacattttta ttaatctcct ttattattat tgttataatt 240

attataatta ttataattat tttatggccc tcccccaccg cctcgctgcc cccgcccagt 300

ttcgttttcg ttgccttttt catttgaatg tcattgcttc tccggtgcct cccgacccgc 360

atcgccggcc ctggtttctc tgggactttt ctttgtgtgc gagagtgtgt ttcctttcgt 420

gtctgcccac ctcttctccc ccacctcccg ggtcccttct gtcggtctgt ctgttctgcc 480

ccccttttcgt tttccggaga cttgttgaga aatacgaccc cacagactgc gagactgaac 540

c 541

<210> 152
<211> 337
<212> DNA
<213> human

<400> 152
agagcagccg cagtgaggtg acggagctcc ggagggtgct ccagggcctg gagattgagc 60

tgcagtccca gctcagcatg aaagcatccc tggagaacag cctggaggag accaaaggcc 120

gctactgcat gcagctgtcc cagatccagg gactgattgg cagtgtggag gagcagctgg 180

cccagctacg ctgtgagatg gagcagcaga gccaggagta ccagatcttg ctggatgtga 240

agacgcggct ggagcaggag attgccacct accgccgcct gctggagggc gaggatgccc 300

acctttcctc ccagcaagca tctggccaat cctattc 337

<210> 153
<211> 479
<212> DNA
<213> human

<400> 153
ctcctccatg agtctgacat ctcggaaact gagcagctgc cggacgcctg ggtcaggaat 60

ccaagacccc acctcttaag gactggttcc tcagaaagca ccctcaggga aaaaggtgaa 120

aacattacat ccgtggattc tcctgccaca accgcattgg aagaaaaggc tgccgcaaca 180

tctcagcgag gagtgaagga cccatgtccc aggaaccgcg ctgcgccacc tgcactcacc 240

cccctcacat tctcttaagc acccggtggc cctccgaggc ctggcggaat ggtggtgccc 300

acggggttgg gcaagggctc accaggacct caacgggcaa agttgtgcac actaaaatat 360

caaatcaagg tgcttggttt taaagtaaat gtttttctaa agaaagctgt gttcttctgt 420

tgacccagac gaatagggca cagccctgta actgcacgtg ccttctgtca ttgggaatg 479

<210> 154
<211> 379
<212> DNA
<213> human

<220>
<221> misc_feature
<222> (210)..(210)
<223> n is a, c, g, or t

<220>
<221> misc_feature
<222> (331)..(331)
<223> n is a, c, g, or t

<400> 154
cctcggacac cagagacaat aactgagcgc ggaggacacg cctgccctgc ctgccatctg 60

tggcccgaag ccattgccat ccactgcaga cgcctggaga gggacaggcc gcttccgagt 120

gcagtcctgg cgcagcaccg actcccacgc acccggggaa ggacaccctc actcccacac 180

cccgggaaga acactagaac atcagcagan gggccctgcc cctccgcctg cagccgtgaa 240

aggaagctgg gtcatcagcc cagccccgcc caccccagcc cctatgtgtg tttccctcaa 300

taaggagatg ccttgttctt ttcaccatgc naataacatg cccagcaaaa acttgcttta 360

tgggtctgcc tggagaaaa 379

<210> 155
<211> 259
<212> DNA
<213> human

<400> 155
ctgttgtgtc tgtggccatg ggacagacaa tcaagatcac atgccaagga gacagcctca 60

aaaagtttta tgcaacctgg taccagcaga agccagggca ggcccctgtt cttgtcgtct 120

atggtaaaaa caaccggccc tcaggaatcc cagaccgatt ctctggctcc agctcaggga 180

acacagcttc cttgaccatc actggggctc aggcggaaga tgaggctgac tattactgta 240

attctcggga cagcagtgg 259

<210> 156
<211> 506
<212> DNA
<213> human

<400> 156
tacacagtac gcctgggaga ccacagccta cagaataaag atggcccaga gcaagaaata 60

cctgtggttc agtccatccc acacccctgc tacaacagca gcgatgtgga ggaccacaac 120

catgatctga tgcttcttca actgcgtgac caggcatccc tggggtccaa agtgaagccc 180

atcagcctgg cagatcattg cacccagcct ggccagaagt gcaccgtctc aggctggggc 240

actgtcacca gtccccgaga gaattttcct gacactctca actgtgcaga agtaaaaatc 300

tttccccaga agaagtgtga ggatgcttac ccggggcaga tcacagatgg catggtctgt 360

gcaggcagca gcaaaggggc tgacacgtgc cagggcgatt ctggaggccc cctggtgtgt 420

gatggtgcac tccagggcat cacatcctgg ggctcagacc cctgtgggag gtccgacaaa 480

cctggcgtct ataccaacat ctgccg 506

<210> 157
<211> 281
<212> DNA
<213> human

<400> 157
ggactggtga agccttcgga gaccctgtcc ctcacctgca gtgtctctgg tggctccatc 60

agtagttact actggagctg gatccggcag cccccaggga agggactgga gtggattggg 120

tatatcaatt acagtgggag caccaactat aacccctccc tcaagagtcg agtcaccata 180

tcagtagaca cgtccaagaa ccagttctcc ctgaagctga gctctgtgac cgctgcggac 240

acggccgtgt attactgtgc gagaacgatt tcgggggtc g 281

<210> 158
<211> 385
<212> DNA
<213> human

<400> 158
gccgccattg ctgatgctga gcagcgtggg gagatggccc tcaaggatgc taagaacaag 60

ctggaagggc tggaggatgc cctgcagaag gccaagcagg acctggcccg gctgctgaag 120

gagtaccagg agctgatgaa cgtcaagctg gccctggacg tggagatcgc cacctaccgc 180

aagctgctgg agggcgagga gtgcaggctg aatggcgaag gcgttggaca agtcaacatc 240

tctgtagtgc agtccaccgt ctccagtggc tatggcggtg ccagcggtgt cggcagtggc 300

ttaggcctgg gtggaggaag cagctactcc tatggcagtg gtcttggcgt tggaggcggc 360

tttagttcca gcagcggcag agcca 385

<210> 159

<211> 470
<212> DNA
<213> human

<400> 159
gcgtgggttt ttgtatccag agctgtttgg atacagctgc tttgagctac aggacaaagg 60

ctgacagact cactgggaag ctcccacccc actcagggga ccccactccc ctcacacacc 120

ccccccccaca aggaaccctc aggccaccct ccacgaggtg tgactaacta tgcaataatc 180

caccccaggt gcagccccag ggcctgcgga ggcggtggca gactagagtt tagatgcccc 240

gagcccaggc agctatttca gcctcctgtt tggtggggtg gcacctgttt cccgggcaat 300

ttaacaatgt ctgaaaaggg actgtgagta atggctgtca cttgtcgggg gcccaagtgg 360

ggtgctctgg tctgaccgat gtgtctccca gaactattct gggggcccga caggtgggcc 420

tgggaggaaa atgtttacat ttttaaaggc acactggtat ttatatttca 470

<210> 160
<211> 330
<212> DNA
<213> human

<400> 160
taaaatgggg atcgcctccc aacagcagat ttcgacatta cctgagagtc ttgattttag 60

gcttgttttt tgtaaaccca tgtgtttgta gagattttag gcgtcttcgg atatcttctc 120

acctatgttc cctggctaag aagtcagagg tagccaatgt ttccttaaat tcatttttaa 180

acttaccatt ggtgcatatg ttccagatgg cagatgctgt caataatctc accattgatg 240

acctttgtgt atgtagttct tgcatcctat actggataag cctgttttaa cctgctatga 300

tgggtgcttc cattgcttca taatcttcat 330

<210> 161
<211> 429
<212> DNA
<213> human

<400> 161
ctggaggtgc gcgtgagcga gctgggcctg ggctacgcgt ccgacgagac ggtgctgttc 60

cgctactgcg caggcgcctg cgaggctgcc gcgcgcgtct acgacctcgg gctgcgacga 120

ctgcgccagc ggcggcgcct gcggcgggag cgggtgcgcg cgcagccctg ctgccgcccg 180

acggcctacg aggacgaggt gtccttcctg gacgcgcaca gccgctacca cacggtgcac 240

gagctgtcgg cgcgcgagtg cgcctgcgtg tgaccctacc tcactcggcc ggcgcggcgg 300

ccactccccc cgcctcgacg gcaccactgg ccggccccgc gaaagactgc gcgtgcgtag 360

agcacgccgg cgcggccccg ggactctcgc gataactgta ctgagataaa gtgtggcaac 420

tcgaaaaaa 429

<210> 162
<211> 337
<212> DNA
<213> human

<400> 162
tcaggcccaa attctcccag attgtcaata ccctggacaa gctcatccgc aatgctgcca 60

gcctcaaggt cattgccagc gctcagtctg gcatgtcaca gcccctcctg gaccgcacgg 120

tcccagatta cacaaccttc acgacagttg gtgattggct ggatgccatc aagatggggc 180

ggtacaagga gagcttcgtc agtgcggggt ttgcatcttt tgacctggtg gcccagatga 240

cggcagaaga cctgctccgt attggggtca ccctggccgg ccaccagaag aagatcctga 300

gcagtatcca ggacatgcgg ctgcagatga accagac 337

<210> 163
<211> 338
<212> DNA
<213> human

<400> 163
gcttttgcag tcgtgtaatg ggcccaagtc attgtttttc tcgcctcact ttccaccaag 60

tgtctagagt catgtgagcc tcgtgtcatc tccggggtgg ccacaggcta gatccccggt 120

ggttttgtgc tcaaaataaa aagcctcagt gacccatgag acggagatct cgccggcttt 180

acgttcacct cggtgtctgc agcaccctcc gcttcctctc ctaggcgacg agacccagtg 240

gctagaagtt caccatgtct attctcaaga tccatgccag ggagatcttt gactctcgcg 300

ggaatcccac tgttgaggtt gatctcttca cctcaaaa 338

<210> 164
<211> 383
<212> DNA
<213> human

<220>
<221> misc_feature
<222> (147)..(147)
<223> n is a, c, g, or t

<220>

<221> misc_feature
<222> (154)..(155)
<223> n is a, c, g, or t

<220>
<221> misc_feature
<222> (159)..(159)
<223> n is a, c, g, or t

<220>
<221> misc_feature
<222> (172)..(172)
<223> n is a, c, g, or t

<220>
<221> misc_feature
<222> (228)..(228)
<223> n is a, c, g, or t

<400> 164
acctcagccc taacggtggt ggagaaccca aaggggagtt gctggaagcc atcaaacgtg 60

actttggttc ctttgacaag tttaaggaga agctgacggc tgcatctgtt ggtgtccaag 120

gctcaggttg gggttggctt ggtttcnaat aagnnaacng gggacactta cnaaattgct 180

gcttgtccaa atcaggatcc actgcaagga acaacaggcc ttattccnac tgctggggat 240

tgatgtgtgg gagcacgctt actaccttca gtataaaaat gtcaggcctg attatctaaa 300

agctatttgg aatgtaatca actgggagaa tgtaactgaa agatacatgg cttgcaaaaa 360

gtaaaccacg atcgttatgc tga 383

<210> 165
<211> 342
<212> DNA
<213> human

<400> 165
tggagtctgg gggaggcttg gtacagcctg gggggtccct gagactctcc tgtacagcct 60

ctggattcac ctttagcacc tatggcatga gctgggtccg ccaggctcca gggaaggggc 120

tggagtgggt ctcagctatt agtggtagtg gtggtagcac atattacgca gactccgtga 180

agggccggtt caccatctcc agagacaatt ccaagaacac gctgtatctg caaatgaaca 240

gcctgagagc cgaggacacg gccgtctatt actgtgcggc cgccccaaga catgcgggga 300

gtccccccta tgactactgg ggccagggaa ccctggtcac cg 342

<210> 166
<211> 514
<212> DNA

<213> human

<400> 166
gaaagttttc agagtccgtc tcggccacta ctccctgtca ccagtttatg aatctgggca 60

gcagatgttc caggggggtca aatccatccc ccaccctggc tactcccacc ctggccactc 120

taacgacctc atgctcatca aactgaacag aagaattcgt cccactaaag atgtcagacc 180

catcaacgtc tcctctcatt gtccctctgc tgggacaaag tgcttggtgt ctggctgggg 240

gacaaccaag agcccccaag tgcacttccc taaggtcctc cagtgcttga atatcagcgt 300

gctaagtcag aaaaggtgcg aggatgctta cccgagacag atagatgaca ccatgttctg 360

cgccggtgac aaagcaggta gagactcctg ccagggtgat tctgggggc ctgtggtctg 420

caatggctcc ctgcagggac tcgtgtcctg ggagattac ccttgtgccc ggcccaacag 480

accgggtgtc tacacgaacc tctgcaagtt cacc 514

<210> 167
<211> 499
<212> DNA
<213> human

<400> 167
gctggccgag gtgcaggagg gccgcgcgtg gattaatcca aaagagggat gtaaagttca 60

cgtggtcttc agcacagagc gctacaaccc agagtcttta cttcaggaag gtgagggacg 120

tttggggaaa tgttctgctc gagtgttttt caagaatcag aaacccagac caaccatcaa 180

tgtaacttgt acacggctca tcgagaaaaa gaaaagacaa caagaggatt acctgcttta 240

caagcaaatg aagcaactga aaaacccctt ggaaatagtc agcatacctg ataatcatgg 300

acatattgat ccctctctga gactcatctg ggatttggct ttccttggaa gctcttacgt 360

gatgtgggaa atgacaacac aggtgtcaca ctactacttg gcacagctca ctagtgtgag 420

gcagtgggta agaaaaacct gaaaattaac ttgtgccaca agagttacaa tcaaagtggt 480

ctccttagac tgaattcat 499

<210> 168
<211> 482
<212> DNA
<213> human

<400> 168
ccacccagtg acgactacga tgacacagac gatgacgacc ctttcaaccc tcaggaatcc 60

aagcgctttg tgcagtggca gtcgtctatc tgagcccctc ctcccggctg gactggagcc 120

tggctcccct cttcgttcct tccctggctg ccggaggaga ccccactaac ccagcctgcc 180

tgggctctga ccactaacac tcttggccat ggacagcctg cacaggaccg cctccctgct 240

cttggccact gtgctcccat ttctgtcctt ggccttggga gtagctgagg gggcagacta 300

gggagtaggg ctggcagggg agggggcaga cagcctcgcc tcgcaccctt catccctggc 360

tgccggtccc atccttggag ggactaagct gggggtgggg acatgagtcc ccctgctgcc 420

cctgccacat cccagtgggc tctgaccccc tgatctcaac tcgtggcact aacttggaaa 480

ag 482

<210> 169
<211> 346
<212> DNA
<213> human

<400> 169
cgcgcaccga cggaggggac atgggcagag caatggtggc caggctcggg ctggggctgc 60

tgctgctggc actgctccta cccacgcaga tttattccag tgaaacaaca actggaactt 120

caagtaactc ctcccagagt acttccaact ctgggttggc cccaaatcca actaatgcca 180

ccaccaaggt ggctggtggt gccctgcagt caacagccag tctcttcgtg gtctcactct 240

ctcttctgca tctctactct taagagactc aggccaagaa acgtcttcta aatttcccca 300

tcttctaaac ccaatccaaa tggcgtctgg aagtccaatg tggcaa 346

<210> 170
<211> 235
<212> DNA
<213> human

<400> 170
cagcctctgg attcaccttt agtagcaatt ggatgagctg ggtccgccag gctccaggga 60

aggggctgga gtgggtggcc aacataaagc aagatgaaag tgagaaatac tatgtagact 120

ctgtgaaggg ccgattcacc atctccagag acaacgccaa gaactcactg tatctgcaaa 180

tgaacagcct gagagtcgag gacacggctg tgtattactg tgcgagagcc gaccc 235

<210> 171
<211> 547
<212> DNA
<213> human

<400> 171
cttcgttcgc agagcttttc agattgtgga atgttggata aggaattata gacctctagt 60

agctgaaatg caagacccca agaggaagtt cagatcttaa tataaattca ctttcatttt 120

tgatagctgt cccatctggt catgtggttg gcactagact ggtggcaggg gcttctagct 180

gactcgcaca gggattctca caatagccga tatcagaatt tgtgttgaag gaacttgtct 240

cttcatctaa tatgatagcg ggaaaaggag aggaaactac tgcctttaga aaatataagt 300

aaagtgatta aagtgctcac gttaccttga cacatagttt ttcagtctat gggtttagtt 360

actttagatg gcaagcatgt aacttatatt aatagtaatt tgtaaagttg ggtggataag 420

ctatccctgt tgccggttca tggattactt ctctataaaa aatatatatt taccaaaaaa 480

ttttgtgaca ttccttctcc catctcttcc ttgacatgca ttgtaaatag gttcttcttg 540

ttctgag 547

<210> 172
<211> 502
<212> DNA
<213> human

<400> 172
agcccatgat gccgtggcac aggaaggcca atgtcgggta gatgacaagg tgaatttcca 60

ttttattctg tttaacaacg tggatggcca cctctatgaa cttgatggac gaatgccttt 120

tccggtgaac catggcgcca gttcagagga caccctgctg aaggacgctg ccaaggtgtg 180

cagagaattc accgagcgtg agcaaggaga agtccgcttc tctgccgtgg ctctctgcaa 240

ggcagcctaa tgctctgtgg gagggacttt gctgatttcc cctcttccct tcaacatgaa 300

aatatatacc ccccatgcag tctaaaatgc ttcagtactt gtgaaacaca gctgttcttc 360

tgttctgcag acacgccttc ccctcagcca cacccaggca cttaagcaca agcagagtgc 420

acagctgtcc actgggccat tgtggtgtga gcttcagatg gtgaagcatt ctccccagtg 480

tatgtcttgt atccgatatc ta 502

<210> 173
<211> 411
<212> DNA
<213> human

<400> 173
gggcagagct aacacctgag ctgttaaaga tcctgcattc tcaggttgct ggcagactga 60

tcatccgtgc agaggagctg gcccagatgt ggaaagtggt gaatctccca acagatctgt 120

ttaatagtgt gatgaatgtg ggtcgcttca cggaggagat cgagtggctg aagtttttag 180

cccttgcttg cagcgctctg ggagttacta ttaccaaaac tctcaagata gtgtgtgagg 240

tcttatcatg tgaccacaat ggtgggttgc cccgaatccc attcagcacc ttccagtttc 300

tctacacgta tattgccgaa gtggatgggg agatctgtgc atcacatgtc agcaggatgc 360

taaactacat tgaacaggaa gtaattggtc ctgatggttt aatcacggtg a 411

<210> 174
<211> 532
<212> DNA
<213> human

<400> 174
cccgctactg cttacacagg ccgggttccc acgcagaggg gaggctgctc cacccctact 60

ctcctccctt gctcccagca gcggaagcgc ctctgaccct tggcttgagt cccacgtggg 120

ggaggaggag gcaggcagca ccagcagggg tccaccaaga gcccagacca gcccctctgc 180

cctcctaccc gggcctcgaa gggtgtggca caggctacgt gttgagcgtg gcctacgtga 240

gccaacaaga agcaggggcc tctgagtgcc aagcgacgtg gcgggctcca cgttagccca 300

gctctgagat gccagcccag gggcggcgct gctcagcttg ggctggtcca gggcctgccc 360

aggctggggc acctttgcct cctgagggca gcgcactcct cccctgccaa gcctactgcc 420

tcccgtgccg ccagtacccc ctccagcccc acacctggcc tcccccgcca ctcccctccc 480

ttgctcccct ctgtccccag gggatcaaac agaagccagc cgtgggcaaa at 532

<210> 175
<211> 363
<212> DNA
<213> human

<220>
<221> misc_feature
<222> (37)..(37)
<223> n is a, c, g, or t

<220>
<221> misc_feature
<222> (55)..(55)
<223> n is a, c, g, or t

<220>
<221> misc_feature
<222> (100)..(100)
<223> n is a, c, g, or t

<220>
<221> misc_feature
<222> (105)..(105)

<223> n is a, c, g, or t

<220>
<221> misc_feature
<222> (120)..(120)
<223> n is a, c, g, or t

<220>
<221> misc_feature
<222> (133)..(133)
<223> n is a, c, g, or t

<220>
<221> misc_feature
<222> (155)..(155)
<223> n is a, c, g, or t

<220>
<221> misc_feature
<222> (171)..(171)
<223> n is a, c, g, or t

<220>
<221> misc_feature
<222> (175)..(175)
<223> n is a, c, g, or t

<220>
<221> misc_feature
<222> (182)..(182)
<223> n is a, c, g, or t

<400> 175
ctacactcac ctgcactaac agagtggccg tcctaanctc gggcctgctg cgcanacgtc 60

catcacgtta gctgtcccac atcacaagac tatgccattn ggggnagttg tgtttcaacn 120

ggaaagtgct gtncttaaac taaatgtgca atagnaaggt gatgttgcca ncctnaccgt 180

cntttcctgt ttcctagctg tgtgaatacc tgctcacgtc aaatgcatac aagtttcatt 240

ctccctttca ctaaaacaca caggtgcaac agacttgaat gctagttata cttatttgta 300

tatggtattt attttttctt ttctttacaa accattttgt tattgactaa caggccaaag 360

agt 363

<210> 176
<211> 474
<212> DNA
<213> human

<220>
<221> misc_feature
<222> (281)..(281)

<223> n is a, c, g, or t

<220>
<221> misc_feature
<222> (319)..(319)
<223> n is a, c, g, or t

<220>
<221> misc_feature
<222> (326)..(326)
<223> n is a, c, g, or t

<220>
<221> misc_feature
<222> (428)..(429)
<223> n is a, c, g, or t

<400> 176
gtggagccgg gaataactgg gccaagggcc actacacaga gggagccgag ctggtcgact 60

cggtcctgga tgtggtgagg aaggagtcag agagctgtga ctgtctccag ggcttccagc 120

tgacccactc tctgggggc ggcacggggt ccgggatggg caccctgctc atcagcaaga 180

tccgggaaga gtacccagac cgcatcatga acaccttcag cgtcatgccc tcacccaagg 240

tgtcagacac ggtggtggag ccctacaacg ccaccctctc ngtccaccag ctggtggaaa 300

acacagatga aacctactnc attganaacg aggccctgta tgacatctgc ttccgcaccc 360

tgaagctgac caccccacc tacggggacc tcaaccacct ggtgtcggcc accatgagcg 420

gggtcacnnc acctgcctgc gcttcccggg ccagctgaac gcagacctgc gcaa 474

<210> 177
<211> 489
<212> DNA
<213> human

<400> 177
ccccgattgg ctgcaatgct gaactttaat cttcagcaac tttgtggccc caagtgccgt 60

gacctgaaag ttgaaaaccc tgagaaatac ggctttgaac caaagaagct gttggaccaa 120

ctgacggata tttacttaca gctggactgt gctcggttcg cgaaagccat tgctgacgac 180

cagagatcct acagtaagga attgtttgaa gaagttattt caaagatgcg gaaggcaggg 240

atcaaatcca caatagcaat agaaaaattt aagctgctcg ccgagaaagt ggaggagata 300

gtggccaaga acgcacgcgc agaaatcgac tacagcgacg ctcctgatga gttcagagac 360

cctctgatgg acaccctcat gacagacccc gtgcggctgc cctctggcac catcatggac 420

cgctccatca tcctgcggca cctgctcaac tcccccacgg accccttcaa ccggcagacg 480

ctgacagag 489

<210> 178
<211> 320
<212> DNA
<213> human

<400> 178
aggtgtccag tgtgaggtgc agttggtgga gtccggggga ggcttagttc agcctggggg 60

gtccctgaga ctctcctgtg cagcctctgg attcaccttc agtacctaca ggatgcactg 120

ggtccgccaa gctccaggga aggggctggt gtgggtctca cgtattaata gtgatgggag 180

tagcacagac tacgcggact ccgtggaggg ccgattcacc atctccagag acaacgccaa 240

gaacacgctg tatctgcaaa tgaacagtct gagagccgaa gacacggctg tgtattactg 300

tgcaagatcc aattactact 320

<210> 179
<211> 504
<212> DNA
<213> human

<400> 179
ggacgccatg aaggttttct gcaacatgga gactggcgag acttgcgtct accccaatcc 60

agcaaacgtt cccaagaaga actggtggag cagcaagagc aaggagaaga aacacatctg 120

gtttggagaa accatcaatg gtggcttcca tttcagctat ggagatgaca atctggctcc 180

caacactgcc aacgtccaga tgaccttcct acgcctgctg tccacggaag gctcccagaa 240

catcacctac cactgcaaga acagcattgc ctatctggac gaagcagctg gcaacctcaa 300

gaaggccctg ctcatccagg gctccaatga cgtggagatc cgggcagagg gcaatagcag 360

gttcacgtac actgccctga aggatggctg cacgaaacat accggtaagt ggggcaagac 420

tgttatcgag taccggtcac agaagacctc acgcctcccc atcattgaca ttgcacccat 480

ggacatagga gggcccgagc agga 504

<210> 180
<211> 387
<212> DNA
<213> human

<400> 180
gtgatagaca cttcgggtgg acccctcgac ctcatggctt gcagagtggt tgcggccagc 60

acccgggaga tggcgatgct catagctcag gccttacaga cgattaacta tgggcgggat 120

gatgagaagt gactgcggct gaggcaaagc tgctcccaag gcctccctgg gctgctgtgg 180

gctcctgggg aggtggccct cgtggcccac gctccatgcc agtggctcac gctctgctcc 240

tggctacccc agagggagtt gtcacgctac agtgagtggc tggcctttta aatcgacgtc 300

tctctcacca ggatttggtg tttagctgtt tctctcttta atctcacgta gcctttttca 360

ggttagtacg tgttcttctg tcagggc 387

<210> 181
<211> 208
<212> DNA
<213> human

<220>
<221> misc_feature
<222> (81)..(81)
<223> n is a, c, g, or t

<400> 181
atacttgttc tgataaggtt caatcttatg atccagaaac caattcttgg ctacttcgtg 60

cagctatccc aattgccaaa naggtgtata acagctgtat ccctaaacaa cctgatctat 120

gttgccggtg gactgaccaa ggcaatatac tgttacgatc cagttgaaga ttactggatg 180

cacgtacaga atacattcag ccgtcagg 208

<210> 182
<211> 535
<212> DNA
<213> human

<400> 182
gccaggctgt catccatggt gaagaaagtc aggctgccct cccgctgcga acccccctgga 60

accacctgta ctgtctccgg ctggggcact accacgagcc cagatgtgac ctttccctct 120

gacctcatgt gcgtggatgt caagctcatc tccccccagg actgcacgaa ggtttacaag 180

gacttactgg aaaattccat gctgtgcgct ggcatccccg actccaagaa aaacgcctgc 240

aatggtgact caggggggacc gttggtgtgc agaggtaccc tgcaaggtct ggtgtcctgg 300

ggaactttcc cttgcggcca acccaatgac ccaggagtct acactcaagt gtgcaagttc 360

accaagtgga taaatgacac catgaaaaag catcgctaac gccacactga gttaattaac 420

tgtgtgcttc caacagaaaa tgcacaggag tgaggacgcc gatgacctat gaagtcaaat 480

ttgactttac ctttcctcaa agatatattt aaacctcatg ccctgttgat aaacc 535

<210> 183
<211> 511
<212> DNA
<213> human

<400> 183
cacgatgacc ccagacatga gatccatcac taataatagc tcagatcctt tcctcaatgg 60

agggccatat cattcgaggg agcagagcac tgacagtggc ctggggttag ggtgctacag 120

tgtccccaca actccggagg acttcctcag caatgtggat gagatggata caggagaaaa 180

cgcaggacaa acacccatga acatcaatcc ccaacagacc cgtttccctg atttccttga 240

ctgtcttcca ggaacaaacg ttgacttagg aactttggaa tctgaagacc tgatccccct 300

cttcaatgat gtagagtctg ctctgaacaa aagtgagccc tttctaacct ggctgtaatc 360

actaccattg taacttggat gtagccatga ccttacattt cctgggcctc ttggaaaaag 420

tgatggagca gagcaagtct gcaggtgcac cacttcccgc ctccatgact cgtgctccct 480

cctttttatg ttgccagttt aatcattgcc t 511

<210> 184
<211> 321
<212> DNA
<213> human

<400> 184
acttcttttc ttatcatcag ttcatccatc tattatgccg agttgcaatc aactgtgaaa 60

aatttactga aacattagtt aagctgagtg tcctagttgc ctatgaaggt ttgccacttc 120

atcttgcact gttccccaaa ctttggactg agctatgcca gactcagtct gctatgtcaa 180

aaaactgcat caagcttttg tgtgaagatc ctgttttcgc agaatatatt aaatgtatcc 240

taatggatga aagaactttt ttaaacaaca acattgtcta cacgttcatg acacatttcc 300

ttctaaaggt tcaaagtcaa g 321

<210> 185
<211> 527
<212> DNA
<213> human

<400> 185
acctgctctc cacaataaat cacaaacact aaaataaaat tacttccata taaatattat 60

tttctctttt ggtgtgggag atcaaaggtt taaagtctaa cttctaagat atatttgcag 120

aaagaagcaa catgacaata gagagagtta tgctacaatt atttcttggt ttccacttgc 180

aatggttaat taagtccaaa aacagctgtc agaacctcga gagcagaaca tgagaaactc 240

agagctctgg accgaaagca gaaagtttgc cgggaaaaaa aaagacaaca ttattaccat 300

cgattcagtg cctggataaa gaggaaagct tacttgttta atggcagcca catgcacgaa 360

gatgctaaga agaaaaagaa ttccaaatcc tcaacttttg aggtttcggc tctccaattt 420

aactctttgg caacaggaaa caggttttgc aagttcaagg ttcactccct atatgtgatt 480

ataggaattg tttgtggaaa tggattaaca tacccgtcta tgcctaa 527

<210> 186
<211> 345
<212> DNA
<213> human

<400> 186
gtaagacttt ctgacatgta acattagttc cgtagttttg agacctggta gaactgactt 60

tcatatttgg ataacctgga aaacacccaa acacaaactt caagtcttct ttctcttttt 120

tcattatctt ttttagtctg aggtgacacc atcattaagg attcgacacc cgtttgtaaa 180

taaaatgaca tcagcaatta ctctgaaatg tttctagttt gcaaagattt agcaatgtga 240

tgttattaac ccttcctccc ttcagagacc tgtcctaagc tctgaaccac tcattccttc 300

cactcttctt accccaggtg gttgatgagc agtggtccct ggtgt 345

<210> 187
<211> 531
<212> DNA
<213> human

<400> 187
gtagccccgt cggacatgat ggatggacgc gtggaagcca tcaaagaggc cctgatggca 60

catggacttg gcaacagggt atcggtgatg agctacagtg ccaaatttgc ttcctgtttc 120

tatggccctt tccgggatgc agctaagtca agcccagctt ttggggaccg ccgctgctac 180

cagctgcccc ctggagcacg aggcctggct ctccgagctg tggaccggga tgtacgggaa 240

ggagctgaca tgctcatggt gaagccggga atgccctacc tggacatcgt gcgggaggta 300

aaggacaagc accctgacct ccctctcgcc gtgtaccacg tctctggaga gtttgccatg 360

ctgtggcatg gagcccaggc cggggcattt gatctcaagg ctgccgtact ggaggccatg 420

actgccttcc gcagagcagg tgctgacatc atcatcacct actacacacc gcagctgctg 480

cagtggctga aggaggaatg atggaggaca gtgccaggcc caagaactag a 531

<210> 188

<211> 242
<212> DNA
<213> human

<400> 188
agcttcaaga agcagtcgtt tgtgctgaag gagggtgtgg agtaccggat aaaaatctct 60

ttccgggtta accgagagat agtgtccggc atgaagtaca tccagcatac gtacaggaaa 120

ggcgtcaaga ttgacaagac tgactacatg gtaggcagct acagcatcaa gtcccgcttc 180

acagacgacg acaagaccga ccacctgtcc tgggagtgga atctcaccat caagaaggac 240

tg 242

<210> 189
<211> 524
<212> DNA
<213> human

<220>
<221> misc_feature
<222> (185)..(185)
<223> n is a, c, g, or t

<400> 189
atgaggtggg gaagcgtcgg caggaggccc gggggccct gcaaaaccga ctgagggtct 60

tcctgtccct catggagact ggctggtttg ataaccttct cctggacata gacaaagctg 120

atgccattgt caagatgctg gatgcagccg tgattaagat ggaaggaggc acggagaatg 180

atctncgcat cctggagcag gaggaggagg aggagcaggc aggaaagcct ggggagccca 240

gcaagaaaga agaaggacgg gctggagcag gcctagggga cggggagcgc aaaaccaacg 300

acaaggatga gaagaaggaa gacggcaagc aggctgagaa tgacagttct aatgatgaca 360

aaacaaagaa gtcggagggt gatggggaca aggaagagaa gaaagaagac tccgagaagg 420

aagccaaaaa gagtagcaag aagcggaacc ggaagcacag tggtgacgac agctttgacg 480

agggcagcgt gtcagagtct gagtcggagt cagagagcgg ccag 524

<210> 190
<211> 452
<212> DNA
<213> human

<400> 190
gatgaaatct cactgctaat gctcagagat ctttttttcac tgtaagaggt aacctttaac 60

aatatgggta ttacctttgt ctcttcatac cggttttatg acaaaggtct attgaattta 120

tttgtttgta agtttctact cccatcaaag cagctttcta agttattgcc ttggttatta 180

tggatgatag ttatagccct tataatgcct taactaagga agaaaagatg ttattctgag 240

tttgttttaa tacatatatg aacatatagt tttattcaat taaaccaaag aagaggtcag 300

cagggagata ctaacctttg gaaatgatta gctggctctg tttttggtt aaataagagt 360

ctttaatcct ttctccatca agagttactt accaagggca ggggaagggg gatatagagg 420

tcacaaggaa ataaaaatca tctttcatct tt 452

<210> 191
<211> 523
<212> DNA
<213> human

<400> 191
agtggcacat tacatatcgg atctaagaga ttaataccat tagaagttac acagttttag 60

ttgtttggag atagttttgg tttgtacaga acaaaataat atgtagcagc ttcattgcta 120

ttggaaaaat cagttattgg aatttccact taaatggcta tacaacaata taactggtag 180

ttctataata aaaatgagca tatgttctgt tgtgaagagc taaatgcaat aaagtttctg 240

tatggttgtt tgattctatc aacaattgaa agtgttgtat atgacccaca tttacctagt 300

ttgtgtcaaa ttatagttac agtgagttgt ttgcttaaat tatagattcc tttaaggaca 360

tgccttgttc ataaaatcac tggattatat tgcagcatat tttacatttg aatacaagga 420

taatgggttt tatcaaaaca aaatgatgta cagatttttt ttcaagtttt tatagttgct 480

ttatgccaga gtggtttacc ccattcacaa aatttcttat gca 523

<210> 192
<211> 507
<212> DNA
<213> human

<400> 192
ccttaaactt catcgcacct aataaatatg agtactgcat ctggattgac ggcctcagtg 60

cccttctggg gaaggacatg tccagtgagc tgaccaagag tgacctggac accctgctga 120

gcatggagat gaagctgcgg ctcctggacc tggagaacat ccagattccc gaagccccac 180

cccccatccc caaggagccc agcagctatg actttgtcta tcactatggc tgagcctgga 240

gccagaaacg acggtaccca ggagaaggga ttttgggccc aggagagaca cttacattct 300

ggtgccttgt cttttgcttg tacagaatct gtagtgattt tggtggccag taaatgccag 360

ccatttctca aacccacctc ggaccaccca gagtttcctc ttggtccctg tctactaaga 420

gtcatgaagg cagggtgctc tgcccactcc atcaccatga agcctgggat tgggccacga 480

ggaacaaaca gcagatgccc ttgcctt 507

<210> 193
<211> 366
<212> DNA
<213> human

<400> 193
atgagcatgc cattattctc tgtctatgaa acaaaaatgg catttttcaa tggatttgtt 60

ttggatatat aattagttca tttgctgttt agaagccttg ccaaaagtgt ttagattttg 120

gtactgcaac tgctttcctc ttgcccagaa atgttttgcc tcttcttttc ctacaagtta 180

aatgttctaa atataaaggg gtatgtgtgt gtgtgtgtaa ttctaatgtg aaaggcacta 240

gctgtctaat agtttcatgt atcattacta ttactatatg tatcttaatg tagtctatgt 300

aggtttttat cagaaagtgt acctttctat ggtttattat tttatattct ggtgcctttt 360

atctca 366

<210> 194
<211> 337
<212> DNA
<213> human

<400> 194
gaaccctgct cctgagctag gtaggaaaca tgagcggcac caacttggat gggaacgatg 60

agtttgatga gcagttgcga atgcaagaat tgtacggaga cggcaaggat ggtgacaccc 120

agaccgatgc cggcggagaa cccgattctc tcgggcagca gccgacggac actccctacg 180

agtgggacct ggacaaaaag gcttggttcc ccaagattac tgaagatttc attgctacat 240

atcaggccaa ttatggcttc tctaacgatg gcgcatctag ttctaccgca aatgttgaag 300

atgtccatgc taggactgca gaggaacctc cacaaga 337

<210> 195
<211> 245
<212> DNA
<213> human

<400> 195
aagacatttc gggatgcatt tggccgatat atcacctgca attaccgggc cacaaagtca 60

gtaaaaactc tcagaaaacg ctccagtaag atctacttcc ggaatccaat ggcagagaac 120

tctaagtttt tcaagaaaca tggaattcga aatgggatta accctgccat gtaccagagt 180

ccaatgaggc tccgaagttc aaccattcag tcttcatcaa tcattctact agatacgctt 240

ctcct 245

<210> 196
<211> 399
<212> DNA
<213> human

<400> 196
gccacacctt cgcgaaacct gtggtggccc accagtccta acgggacagg acagagagac 60

agagcagccc tgcactgttt tccctccacc acagccatcc tgtccctcat tggctctgtg 120

ctttccacta tacacagtca ccgtcccaat gagaaacaag aaggagcacc ctccacatgg 180

actcccacct gcaagtggac agcgacattc agtcctgcac tgctcacctg ggtttactga 240

tgactcctgg ctgccccacc atcctctctg atctgtgaga aacagctaag ctgctgtgac 300

ttccctttag gacaatgttg tgtaaatctt tgaaggacac accgaagacc tttatactgt 360

gatcttttac ccctttcact cttggctttc ttatgttgc 399

<210> 197
<211> 424
<212> DNA
<213> human

<400> 197
gactctggaa caaatcacag cccattttga ggggcgatga cagccactca ttaggggatg 60

gagcaagcct gtgactccaa gttgggccca agcccagagc ccctgcctgc cccaggggag 120

ccagaatcca gccccttgga gccttggtct gcagggtccc tccttcctgt catgctccct 180

ccagcccatg acccggggct aggaggctca ctgcctcctg ttccagctcc tgctgctgct 240

ctgaggactc aggaacacct tcgagctttg cagacctgcg gtcagccctc catgcgcaag 300

actaaagcag cggaagagga ggtgggcctc taggatcttt gtcttctggc tggaggtgct 360

tttggaggtt gggtgctggg cattcggtcg ctcctctcac gcggctgcct tatcgggaag 420

gaaa 424

<210> 198
<211> 390
<212> DNA
<213> human

<400> 198
ggtgggtgtc accggaaccg gattgagaac aggtttccag atgaagctac ttgtatgggc 60

```
ttctgcgcac caaagaaaat tccatcattt tgctacagtc caaaagatga gggactgtgc    120
tctgccaatg tgactcgcta ttattttaat ccaagataca gaacctgtga tgctttcacc    180
tatactggct gtggagggaa tgacaataac tttgttagca gggaggattg caaacgtgca    240
tgtgcaaaag ctttgaaaaa gaaaaagaag atgccaaagc ttcgctttgc cagtagaatc    300
cggaaaattc ggaagaagca attttaaaca ttcttaatat gtcatcttgt ttgtctttat    360
ggcttatttg cctttatggt tgtatctgaa                                      390
```